(19) Europäisches Patentamt
European Patent Office
Office européen des brevets

(11) EP 4 692 059 A1

(12) EUROPEAN PATENT APPLICATION
published in accordance with Art. 153(4) EPC

(43) Date of publication:
**11.02.2026 Bulletin 2026/07**

(21) Application number: **24779702.0**

(22) Date of filing: **19.03.2024**

(51) International Patent Classification (IPC):
***C07D 211/22*** (2006.01) ***A61K 9/127*** (2025.01)
***A61K 31/7088*** (2006.01) ***A61K 47/22*** (2006.01)
***A61K 48/00*** (2006.01) ***A61P 43/00*** (2006.01)
***C07F 7/10*** (2006.01)

(52) Cooperative Patent Classification (CPC):
**A61K 9/127; A61K 31/7088; A61K 47/22; A61K 48/00; A61P 43/00; C07D 211/22; C07F 7/10**

(86) International application number:
**PCT/JP2024/010667**

(87) International publication number:
**WO 2024/203577 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 JP 2023053860**

(71) Applicants:
- **Tohoku University**
  **Sendai-shi, Miyagi 980-8577 (JP)**
- **National University Corporation Chiba University**
  **Chiba-shi, Chiba 263-8522 (JP)**
- **NOF Corporation**
  **Shibuya-ku**
  **Tokyo 150-6019 (JP)**

(72) Inventors:
- **AKITA, Hidetaka**
  **Sendai-shi, Miyagi 980-8577 (JP)**
- **SAKURAI, Yu**
  **Sendai-shi, Miyagi 980-8577 (JP)**
- **DOI, Mizuki**
  **Sendai-shi, Miyagi 980-8577 (JP)**
- **NISHIUMI, Naomasa**
  **Sendai-shi, Miyagi 980-8577 (JP)**
- **NAKABAYASHI, Tomoya**
  **Sendai-shi, Miyagi 980-8577 (JP)**
- **TANAKA, Hiroki**
  **Chiba-shi, Chiba 260-8675 (JP)**
- **TANAKA, Tokihiro**
  **Kawasaki-shi, Kanagawa 210-0865 (JP)**
- **MATSUNO, Yuki**
  **Kawasaki-shi, Kanagawa 210-0865 (JP)**
- **MORIYAMA, Yuya**
  **Kawasaki-shi, Kanagawa 210-0865 (JP)**
- **MOTOUSU, Ryosuke**
  **Kawasaki-shi, Kanagawa 210-0865 (JP)**
- **ONIZUKA, Yuhei**
  **Kawasaki-shi, Kanagawa 210-0865 (JP)**
- **TANGE, Kota**
  **Kawasaki-shi, Kanagawa 210-0865 (JP)**
- **NAKAI, Yuta**
  **Kawasaki-shi, Kanagawa 210-0865 (JP)**
- **ITO, Kasumi**
  **Kawasaki-shi, Kanagawa 210-0865 (JP)**

(74) Representative: **Weickmann & Weickmann PartmbB**
**Postfach 860 820**
**81635 München (DE)**

# (54) CATIONIC LIPID HAVING DISULFIDE BOND

(57) The present invention provides a cationic lipid represented by the following formula (1) (the symbols in the following formula (1) are as defined in the specification).

EP 4 692 059 A1

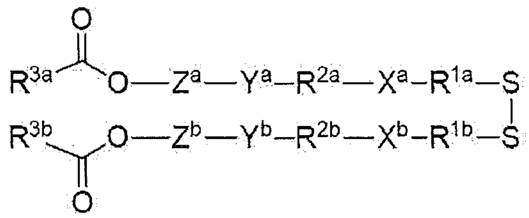

(1)

## Description

[Technical Field]

The present invention relates to is a cationic lipid having a disulfide bond, a lipid membrane structure containing same, a nucleic acid-introducing agent and a pharmaceutical composition containing any of these, a method for introducing a nucleic acid into a cell, and a production method of cellular medicines.

[Background Art]

For practicalization of nucleic acid therapy, an effective and safe nucleic acid delivery carrier is demanded. While virus vectors are nucleic acid delivery carriers with good expression efficiency, the development of non-viral nucleic acid delivery carriers that can be used more safely is ongoing. Among them, carriers using a cationic lipid are non-viral nucleic acid delivery carriers most generally used at present.

Cationic lipids are largely composed of an amine moiety and a lipid moiety. In cationic lipids, the amine moiety showing cationicity and a polyanion nucleic acid electrostatically interact to form a liposome or lipid membrane structure, which promotes uptake into cells and delivers the nucleic acid into cells.

As known cationic lipids generally and widely used, 1,2-dioleoyloxy-3-trimethylammonium-propane (DOTAP) and 1,2-dioleoyloxy-3-dimethylaminopropane (DODAP) can be mentioned. These known cationic lipids form a positively-charged liposome or lipid membrane structure when combined with a phospholipid, which electrostatically interacts with a nucleic acid to be able to deliver the nucleic acid to the target cells (non-Patent Literature 1).

On the other hand, for a lipid membrane structure using a cationic lipid to exhibit a practical effect in vivo as a nucleic acid delivery carrier, it needs to show specific pharmacokinetics. To be precise, requirements such as high stability in blood, property to highly accumulate in the target tissues such as liver, tumor, and the like need to be fulfilled. To address this problem, it is known that a lipid membrane structure in which pKa of the surface of the lipid membrane structure is adjusted to near neutral and PEG-lipid is introduced exhibits a long life in blood after intravenous injection and accumulates at tumor sites. Furthermore, there is an example of improving pharmacokinetics by adjusting surface pKa of lipid membrane structure.

For example, Non-Patent Literature 2 and non-Patent Literature 3 show that pharmacokinetics and distribution in each cell in the liver can be controlled by adjusting the surface pKa of lipid membrane structures. These literatures show that escape of lipid membrane structures from endosomes is promoted and nucleic acids can be efficiently delivered into the cytoplasm by adjusting the surface pKa of lipid membrane structures for endosome escape.

[Citation List]

[Patent Literature]

[Patent Literature 1]
US Patent No. 9708628 specification
[Patent Literature 2]
WO 2016/121942
[Patent Literature 3]
WO 2019/188867
[Patent Literature 4]
WO 2021/193397

[Non Patent Literature]

[Non Patent Literature 1]
Biomaterials 29(24-25): 3477-3496, 2008
[Non Patent Literature 2]
Molecular Therapy 24(4): 788-795, 2016
[Non Patent Literature 3]
Angewante Chemie International Edition 51: 8529-8533, 2012

[Non Patent Literature 4]
Molecular Therapy 13(4): 786-794, 2006

[Summary of Invention]

[Technical Problem]

While cationic lipids having improved pharmacokinetics have been developed as mentioned above, in view of the property of the nucleic acid delivery carriers that they generally introduce exogenous substances into cells, a large effect output from a small uptake amount is desired. That is, when a lipid membrane structure is used as a delivery carrier of an expression vector into cells, it is desired to increase the expression level per lipid membrane structure incorporated into the cells and enhance intracellular expression efficiency. That is, to enhance the intracellular expression efficiency, it is necessary to also improve, besides pharmacokinetics, intracellular kinetics such as uptake process into cells, escape from endosome, nuclear membrane permeation, and the like (non-Patent Literature 4).

In order to improve expression efficiency, a method is available that imparts biodegradability to cationic lipids (e.g., Patent Literatures 1 to 4). Patent Literatures 1 to 4 show a cationic lipid having a structure linked by a biodegradable disulfide bond, and describe that the cationic lipid can improve intracellular dynamics by dissociating nucleic acid from a lipid membrane structure by utilizing intracellular cleavage of a disulfide bond. Such cationic lipid shows high nucleic acid delivery efficiency as compared with known cationic lipids, DOTAP and DODAP. That is, it has been clarified that the cationic lipids described in Patent Literatures 1 to 4 can improve intracellular dynamics such as increased delivery efficiency of nucleic acid into the cytoplasm and the like, and further that the effect of reducing toxicity is also expected by imparting degradability.

However, nucleic acid therapy targets a wide variety of diseases. In order to establish a treatment method suitable for each disease, further improvement of intracellular dynamics is desired.

The present invention has been made in view of the above-mentioned problems, and aims to provide a cationic lipid that shows good intracellular dynamics and can be used as a carrier for nucleic acid delivery.

[Solution to Problem]

The present inventors have conducted intensive studies in view of the above-mentioned problems and found that a nucleic acid can be efficiently delivered to the target cell by using a cationic lipid represented by the following formula (1).

The present invention based on the above-mentioned finding is as follows.

[1] A cationic lipid represented by the formula (1):

[Chem. 1]

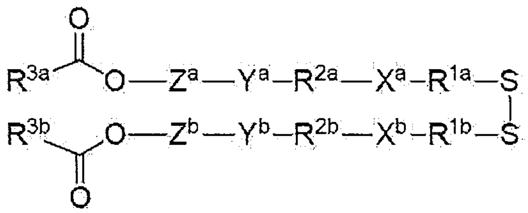

(1)

(in the formula (1), $R^{1a}$ and $R^{1b}$ are each independently an alkylene group having 1 to 6 carbon atoms,

$X^a$ and $X^b$ are each independently an acyclic alkyl tertiary amino group having 1 to 6 carbon atoms and 1 tertiary amino group, or a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms or an oxydialkylene group having 2 to 8 carbon atoms,
$Y^a$ and $Y^b$ are each independently an ester bond, an amide bond, a carbamate bond, an ether bond, or a urea bond,
$Z^a$ and $Z^b$ are each independently a divalent group derived from an aromatic compound having 3 to 16 carbon atoms, at least one aromatic ring, and optionally having a heteroatom,
$R^{3a}$ is

(ia) a monovalent group having 10 to 50 carbon atoms, one carbonyl group, and at least one unsaturated bond selected from the group consisting of an olefinic carbon-carbon double bond and a carbon-carbon triple

bond (excluding a monovalent group containing a residue of a liposoluble vitamin having a hydroxy group and a residue of a sterol derivative having a hydroxy group),
(iia) a monovalent group having 10 to 50 carbon atoms and at least two carbonyl groups (excluding a monovalent group containing a residue of a liposoluble vitamin having a hydroxy group and a residue of a sterol derivative having a hydroxy group),
(iiia) a monovalent group represented by the formula (2):

$$*\text{-}R^4\text{-}X^1\text{-}R^5 \qquad (2)$$

(in the formula (2), * is a bonding position,

$R^4$ is an alkylene group having 1 to 10 carbon atoms,
$X^1$ is a carbamate bond, a carbonate bond, or an amide bond, and
$R^5$ is an alkyl group having 1 to 25 carbon atoms, and $R^5$ is optionally substituted by a substituent selected from the group consisting of a halogen atom and a hydroxy group),

(iva) a monovalent group represented by the formula (3):

$$*\text{-}R^6\text{-CO-O-}R^7 \qquad (3)$$

(in the formula (3), * is a bonding position,

$R^6$ is an alkylene group having 1 to 10 carbon atoms, and
$R^7$ is an alkyl group having 1 to 25 carbon atoms and substituted by at least one halogen atom),

(va) a monovalent group represented by the formula (4):

[Chem. 2]

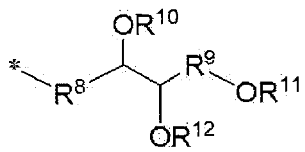

(4)

(in the formula (4), * is a bonding position,

$R^8$ and $R^9$ are each independently an alkylene group having 1 to 10 carbon atoms, an alkenediyl group having 2 to 10 carbon atoms, or an alkynediyl group having 2 to 10 carbon atoms,
$R^{10}$ to $R^{12}$ are each independently a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group)),

(via) a monovalent group represented by the formula (5):

[Chem. 3]

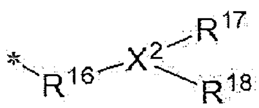

(5)

(in the formula (5), * is a bonding position,
$X^2$ is a nitrogen atom or a trivalent group represented by the formula (6):

[Chem. 4]

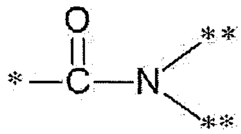

(6)

(in the formula (6), * is a bonding position with $R^{16}$, and

** is a bonding position with $R^{17}$ or $R^{18}$),
when $X^2$ is a nitrogen atom, $R^{16}$ is an alkylene group having 1 to 10 carbon atoms, an alkenediyl group having 2 to 10 carbon atoms, or an alkynediyl group having 2 to 10 carbon atoms, and $R^{16}$ is optionally substituted by a substituent selected from the group consisting of a halogen atom and a hydroxy group,
when $X^2$ is a trivalent group represented by the formula (6), $R^{16}$ is an alkylene group having 1 to 10 carbon atoms, and $R^{16}$ is optionally substituted by a substituent selected from the group consisting of a halogen atom and a hydroxy group,
when $X^2$ is a nitrogen atom, $R^{17}$ and $R^{18}$ are each independently an alkyl group having 1 to 10 carbon atoms, an alkenyl group having 2 to 10 carbon atoms, or an alkynyl group having 2 to 10 carbon atoms, and $R^{17}$ and $R^{18}$ are each independently optionally substituted by a substituent selected from the group consisting of a halogen atom and a hydroxy group, and
when $X^2$ is a trivalent group represented by the formula (6), $R^{17}$ and $R^{18}$ are each independently an alkyl group having 1 to 10 carbon atoms, and $R^{17}$ and $R^{18}$ are each independently optionally substituted by a substituent selected from the group consisting of a halogen atom and a hydroxy group),

(viia) a monovalent group represented by the formula (7):

[Chem. 5]

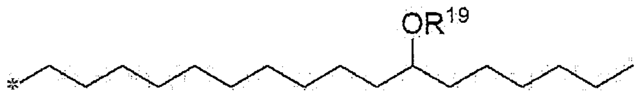

(7)

(in the formula (7), * is a bonding position, and
$R^{19}$ is a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group), or a *-CO-$R^{20}$ group (wherein * is a bonding position, and $R^{20}$ is an alkyl group having 1 to 9 carbon atoms)),
(viiia) a monovalent group represented by the formula (8):

[Chem. 6]

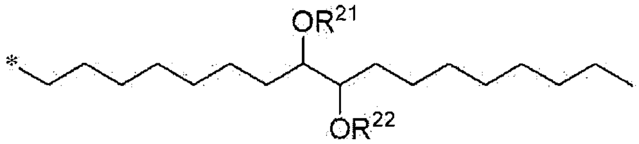

(8)

(in the formula (8), * is a bonding position, and
$R^{21}$ and $R^{22}$ are each independently a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group)),
(ixa) a monovalent group represented by the formula (9):

[Chem. 7]

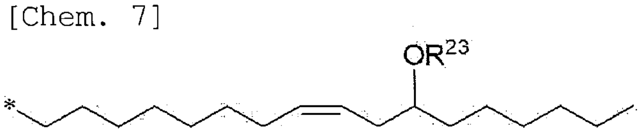

(9)

(in the formula (9), * is a bonding position, and
$R^{23}$ is a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group)),

(xa) a monovalent group represented by the formula (10):

[Chem. 8]

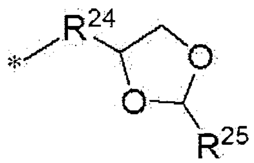

(10)

(in the formula (10), * is a bonding position,

$R^{24}$ is an alkylene group having 1 to 10 carbon atoms, an alkenediyl group having 2 to 10 carbon atoms, or an alkynediyl group having 2 to 10 carbon atoms, and
$R^{25}$ is an alkyl group having 1 to 30 carbon atoms, an alkenyl group having 2 to 30 carbon atoms, or an alkynyl group having 2 to 30 carbon atoms),

(xia) a monovalent group represented by the formula (11):

[Chem. 9]

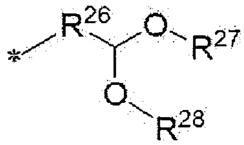

(11)

(in the formula (11), * is a bonding position, and

$R^{26}$ is an alkylene group having 1 to 10 carbon atoms, an alkenediyl group having 2 to 10 carbon atoms, or an alkynediyl group having 2 to 10 carbon atoms, and
$R^{27}$ and $R^{28}$ are each independently an alkyl group having 1 to 10 carbon atoms, an alkenyl group having 2 to 10 carbon atoms, or an alkynyl group having 2 to 10 carbon atoms), or (xiia) a monovalent group represented by the formula (12):

[Chem. 10]

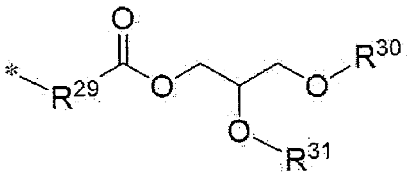

(12)

(in the formula (12), * is a bonding position,
$R^{29}$ is an alkylene group having 1 to 10 carbon atoms, and
$R^{30}$ and $R^{31}$ are each independently an alkyl group having 1 to 10 carbon atoms),
$R^{3b}$ is

(ib) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group),
(iib) a monovalent group having 10 to 50 carbon atoms and at least two carbonyl groups (excluding a monovalent group containing a residue of a liposoluble vitamin having a hydroxy group and a residue of a sterol derivative having a hydroxy group),
(iiib) a monovalent group represented by the aforementioned formula (2),
(ivb) a monovalent group represented by the aforementioned formula (3),
(vb) a monovalent group represented by the aforementioned formula (4),
(vib) a monovalent group represented by the aforementioned formula (5),

(viib) a monovalent group represented by the aforementioned formula (7),
(viiib) a monovalent group represented by the aforementioned formula (8),
(ixb) a monovalent group represented by the aforementioned formula (9),
(xb) a monovalent group represented by the aforementioned formula (10),
(xib) a monovalent group represented by the aforementioned formula (11),
(xiib) a monovalent group represented by the aforementioned formula (12),
(xiiib) a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
(xivb) an $R^{3c}$-CO-$(CH_2)_p$- group (wherein $R^{3c}$ is a residue of a liposoluble vitamin having a hydroxy group or a residue of a sterol derivative having a hydroxy group, and p is an integer of 1 to 8), and $R^{3a}$ and $R^{3b}$ may be the same or different) .

[2] The cationic lipid of the aforementioned [1], wherein $R^{3a}$ is

(ia) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group), or
(iia) a monovalent group having 10 to 50 carbon atoms and at least two carbonyl groups (excluding a monovalent group containing a residue of a liposoluble vitamin having a hydroxy group and a residue of a sterol derivative having a hydroxy group).

[3] The cationic lipid of the aforementioned [1], wherein $R^{3a}$ is

(ia) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group), or
(iia) a monovalent group having 10 to 50 carbon atoms and at least two carbonyl groups (excluding a monovalent group containing a residue of a liposoluble vitamin having a hydroxy group and a residue of a sterol derivative having a hydroxy group),
$R^{3b}$ is
(ib) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group),
(iib) a monovalent group having 10 to 50 carbon atoms and at least two carbonyl groups (excluding a monovalent group containing a residue of a liposoluble vitamin having a hydroxy group and a residue of a sterol derivative having a hydroxy group),
(xiiib) a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
(xivb) a $R^{3c}$-CO-$(CH_2)_p$- group (wherein $R^{3c}$ is a residue of a liposoluble vitamin having a hydroxy group or a residue of a sterol derivative having a hydroxy group, and p is an integer of 1 to 8), and
$R^{3a}$ and $R^{3b}$ may be the same or different.

[4] The cationic lipid of the aforementioned [1], wherein $R^{3a}$ is

(iiia) a monovalent group represented by the aforementioned formula (2),
(iva) a monovalent group represented by the aforementioned formula (3),
(va) a monovalent group represented by the aforementioned formula (4),
(via) a monovalent group represented by the aforementioned formula (5),
(viia) a monovalent group represented by the aforementioned formula (7),
(viiia) a monovalent group represented by the aforementioned formula (8),
(ixa) a monovalent group represented by the aforementioned formula (9),
(xa) a monovalent group represented by the aforementioned formula (10),
(xia) a monovalent group represented by the aforementioned formula (11), or

(xiia) a monovalent group represented by the aforementioned formula (12),
$R^{3b}$ is
(iiib) a monovalent group represented by the aforementioned formula (2),
(ivb) a monovalent group represented by the aforementioned formula (3),
(vb) a monovalent group represented by the aforementioned formula (4),
(vib) a monovalent group represented by the aforementioned formula (5),
(viib) a monovalent group represented by the aforementioned formula (7),
(viiib) a monovalent group represented by the aforementioned formula (8),
(ixb) a monovalent group represented by the aforementioned formula (9),
(xb) a monovalent group represented by the aforementioned formula (10),
(xib) a monovalent group represented by the aforementioned formula (11),
(xiib) a monovalent group represented by the aforementioned formula (12),
(xiiib) a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
(xivb) a $R^{3c}$-CO-$(CH_2)_p$- group (wherein $R^{3c}$ is a residue of a liposoluble vitamin having a hydroxy group or a residue of a sterol derivative having a hydroxy group, and p is an integer of 1 to 8), and
$R^{3a}$ and $R^{3b}$ may be the same or different.

[5] The cationic lipid of any one of the aforementioned [1] to [4], wherein $Z^a$ and $Z^b$ are each independently a divalent group represented by the formula (13):

[Chem. 11]

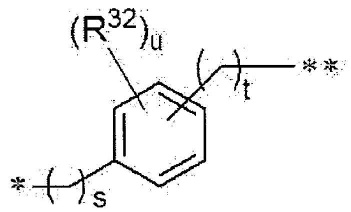

(13)

(in the formula (13), * is a bonding position with O in the formula (1),

** is a bonding position with $Y^a$ or $Y^b$ in the formula (1),
s is an integer of 0 to 3,
t is an integer of 0 to 3,
u is an integer of 0 to 4, and
$R^{32}$ in the number of u are each independently a substituent).

[6] The cationic lipid of the aforementioned [5], wherein s is 0.
[7] The cationic lipid of any one of the aforementioned [1] to [5], wherein $X^a$ and $X^b$ are each independently a cyclic alkylene tertiary amino group having 2 to 5 carbon atoms and 1 or 2 tertiary amino groups.
[8] The cationic lipid of the aforementioned [1] or [2], wherein $R^{3a}$ is

(ia-1) a monovalent group having 10 to 50 carbon atoms and represented by the formula (14):

$$\text{*-}R^{33}\text{-CO-}X^3\text{-}R^{34} \qquad (14)$$

(in the formula (14), * is a bonding position,

$R^{33}$ is an alkylene group having 2 to 10 carbon atoms, an alkenediyl group having 2 to 10 carbon atoms, or an alkynediyl group having 2 to 10 carbon atoms, and $R^{33}$ is optionally substituted by a substituent selected from the group consisting of a halogen atom, a hydroxy group, and a hydrocarbon ring group having 3 to 12 carbon atoms,
$R^{34}$ is an alkyl group having 1 to 40 carbon atoms, an alkenyl group having 2 to 40 carbon atoms, or an alkynyl group having 2 to 40 carbon atoms, and $R^{34}$ is optionally substituted by a substituent selected from the group consisting of a halogen atom, a hydroxy group, and a hydrocarbon ring group having 3 to 12 carbon atoms,

at least one of $R^{33}$ and $R^{34}$ has at least one unsaturated bond selected from the group consisting of an olefinic carbon-carbon double bond and a carbon-carbon triple bond, and
$X^3$ is an oxygen atom, NH, or a sulfur atom),

(ia-2) a monovalent group having 50 or less carbon atoms and represented by the formula (15):

[Chem. 12]

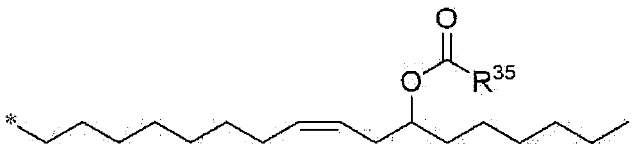

(15)

(in the formula (15), * is a bonding position, and
$R^{35}$ is an alkyl group having 1 to 20 carbon atoms, an alkenyl group having 2 to 20 carbon atoms, an alkynyl group having 2 to 20 carbon atoms, or hydrocarbon ring group having 3 to 12 carbon atoms, at least one ethylene group or at least one trimethylene group in $R^{35}$ is optionally replaced by at least one bond selected from the group consisting of an ester bond, an amide bond, a carbamate bond, and a carbonate bond, and $R^{35}$ is optionally substituted by a substituent selected from the group consisting of an alkoxy group having 1 to 4 carbon atoms, a 3- to 14-membered heterocyclic group, and a hydrocarbon ring group having 3 to 12 carbon atoms (preferably, $R^{35}$ has an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and $R^{35}$ is optionally substituted by a substituent selected from the group consisting of a 3- to 14-membered heterocyclic group and a hydrocarbon ring group having 3 to 12 carbon atoms)),
(iia-1) a monovalent group having 50 or less carbon atoms and represented by the formula (16):

$$*\text{-}R^{36}\text{-CO-}X^4\text{-}R^{37} \qquad (16)$$

(in the formula (16), * is a bonding position,

$R^{36}$ is an alkylene group having 2 to 9 carbon atoms, an alkenediyl group having 2 to 9 carbon atoms, or an alkynediyl group having 2 to 9 carbon atoms, and $R^{36}$ is optionally substituted by a substituent selected from the group consisting of a halogen atom, a hydroxy group, and a hydrocarbon ring group having 3 to 12 carbon atoms,
$R^{37}$ is an alkyl group having 7 to 45 carbon atoms, an alkenyl group having 7 to 45 carbon atoms, or an alkynyl group having 7 to 45 carbon atoms, at least one ethylene group or at least one trimethylene group in $R^{37}$ is replaced by at least one bond selected from the group consisting of an ester bond, an amide bond, a carbamate bond, and a carbonate bond, and $R^{37}$ is optionally substituted by a substituent selected from the group consisting of a halogen atom, a hydroxy group, and a hydrocarbon ring group having 3 to 12 carbon atoms, and
$X^4$ is an oxygen atom, NH, or a sulfur atom),

(iia-2) a monovalent group having 10 to 50 carbon atoms and represented by the formula (17):

$$*\text{-}R^{38}\text{-O-}R^{39} \qquad (17)$$

(in the formula (17), * is a bonding position, and

$R^{38}$ is an alkylene group having 2 to 10 carbon atoms, an alkenediyl group having 2 to 10 carbon atoms, or an alkynediyl group having 2 to 10 carbon atoms, at least one ethylene group or at least one trimethylene group in $R^{38}$ is optionally replaced by at least one bond selected from the group consisting of an ester bond, an amide bond, a carbamate bond, and a carbonate bond, and $R^{38}$ is optionally substituted by a substituent selected from the group consisting of a halogen atom, a hydroxy group, and a hydrocarbon ring group having 3 to 12 carbon atoms, and
$R^{39}$ is an alkyl group having 3 to 30 carbon atoms, an alkenyl group having 4 to 30 carbon atoms, or an alkenyl group having 4 to 30 carbon atoms, at least two methylene groups in $R^{39}$ are replaced by at least two carbonyl groups, and at least one methylene group in $R^{39}$ is optionally replaced by at least one ether bond),

(iia-3) a monovalent group having 50 or less carbon atoms and represented by the formula (18):

[Chem. 13]

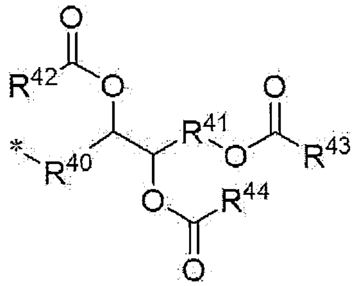

(18)

(in the formula (18), * is a bonding position,

$R^{40}$ and $R^{41}$ are each independently an alkylene group having 3 to 10 carbon atoms, and
$R^{42}$ to $R^{44}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{42}$ is optionally replaced by at least one bond selected from the group consisting of an ester bond, an amide bond, a carbamate bond, and a carbonate bond, at least one ethylene group or at least one trimethylene group in $R^{43}$ is optionally replaced by at least one bond selected from the group consisting of an ester bond, an amide bond, a carbamate bond, and a carbonate bond, at least one ethylene group or at least one trimethylene group in $R^{44}$ is optionally replaced by at least one bond selected from the group consisting of an ester bond, an amide bond, a carbamate bond, and a carbonate bond, and $R^{42}$ to $R^{44}$ are each independently optionally substituted by a substituent selected from the group consisting of a halogen atom, a hydroxy group, and a hydrocarbon ring group having 3 to 12 carbon atoms), or

(iia-4) a monovalent group having 50 or less carbon atoms and represented by the formula (19):

[Chem. 14]

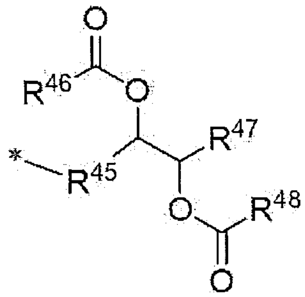

(19)

(in the formula (19), * is a bonding position,

$R^{45}$ is an alkylene group having 5 to 10 carbon atoms, and
$R^{46}$ to $R^{48}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{46}$ is optionally replaced by at least one bond selected from the group consisting of an ester bond, an amide bond, a carbamate bond, and a carbonate bond, at least one ethylene group or at least one trimethylene group in $R^{47}$ is optionally replaced by at least one bond selected from the group consisting of an ester bond, an amide bond, a carbamate bond, and a carbonate bond, at least one ethylene group or at least one trimethylene group in $R^{48}$ is optionally replaced by at least one bond selected from the group consisting of an ester bond, an amide bond, a carbamate bond, and a carbonate bond, and $R^{46}$ to $R^{48}$ are each independently optionally substituted by a substituent selected from the group consisting of a halogen atom, a hydroxy group, and a hydrocarbon ring group having 3 to 12 carbon atoms),
$R^{3b}$ is

(ib-1) a monovalent group having 10 to 50 carbon atoms and represented by the aforementioned formula

(14),
(ib-2) a monovalent group having 50 or less carbon atoms and represented by the aforementioned formula (15),
(iib-1) a monovalent group having 50 or less carbon atoms and represented by the aforementioned formula (16),
(iib-2) a monovalent group having 10 to 50 carbon atoms and represented by the aforementioned formula (17),
(iib-3) a monovalent group having 50 or less carbon atoms and represented by the aforementioned formula (18),
(iib-4) a monovalent group having 50 or less carbon atoms and represented by the aforementioned formula (19),
(iiib) a monovalent group represented by the aforementioned formula (2),
(ivb) a monovalent group represented by the aforementioned formula (3),
(vb) a monovalent group represented by the aforementioned formula (4),
(vib) a monovalent group represented by the aforementioned formula (5),
(viib) a monovalent group represented by the aforementioned formula (7),
(viiib) a monovalent group represented by the aforementioned formula (8),
(ixb) a monovalent group represented by the aforementioned formula (9),
(xb) a monovalent group represented by the aforementioned formula (10),
(xib) a monovalent group represented by the aforementioned formula (11),
(xiib) a monovalent group represented by the aforementioned formula (12),
(xiiib) a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond, or
(xivb) $R^{3c}$-CO-$(CH_2)_p$- group (wherein $R^{3c}$ is a residue of a liposoluble vitamin having a hydroxy group or a residue of a
sterol derivative having a hydroxy group, and p is an integer of 1 to 8), and

$R^{3a}$ and $R^{3b}$ may be the same or different.

[9] The cationic lipid of the aforementioned [8], wherein $R^{35}$ in the formula (15) is an alkyl group having 1 to 20 carbon atoms, an unsubstituted alkenyl group having 2 to 20 carbon atoms, an unsubstituted alkynyl group having 2 to 20 carbon atoms, or a hydrocarbon ring group having 3 to 12 carbon atoms, and the aforementioned alkyl group is optionally substituted by a hydrocarbon ring group having 3 to 12 carbon atoms.
[10] The cationic lipid of the aforementioned [8] or [9], wherein $R^{37}$ in the formula (16) is

(iia-1-1) a monovalent group represented by the formula (20):

[Chem. 15]

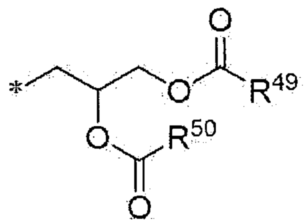

(20)

(in the formula (20), * is a bonding position, and
$R^{49}$ and $R^{50}$ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in $R^{49}$ is optionally replaced by at least one bond selected from the group consisting of an ester bond, an amide bond, a carbamate bond, and a carbonate bond, at least one ethylene group or at least one trimethylene group in $R^{50}$ is optionally replaced by at least one bond selected from the group consisting of an ester bond, an amide bond, a carbamate bond, and a carbonate bond, and $R^{49}$ and $R^{50}$ are each independently optionally substituted by a substituent selected from the group consisting of a halogen atom, a hydroxy group, and a hydrocarbon ring group having 3 to 12 carbon atoms),
(iia-1-2) a monovalent group represented by the formula (21):

[Chem. 16]

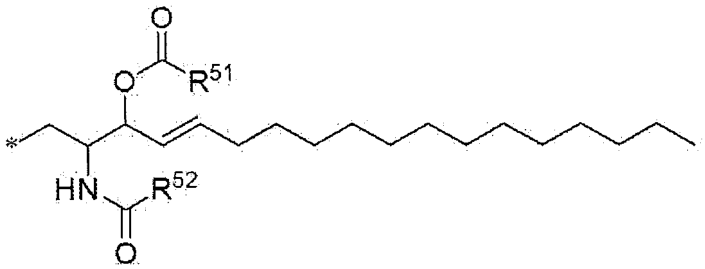

(21)

(in the formula (21), * is a bonding position, and

$R^{51}$ and $R^{52}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{51}$ is optionally replaced by at least one bond selected from the group consisting of an ester bond, an amide bond, a carbamate bond, and a carbonate bond, at least one ethylene group or at least one trimethylene group in $R^{52}$ is optionally replaced by at least one bond selected from the group consisting of an ester bond, an amide bond, a carbamate bond, and a carbonate bond, and $R^{51}$ and $R^{52}$ are each independently optionally substituted by a substituent selected from the group consisting of a halogen atom, a hydroxy group, and a hydrocarbon ring group having 3 to 12 carbon atoms), or

(iia-1-3) a monovalent group represented by the formula (22):

[Chem. 17]

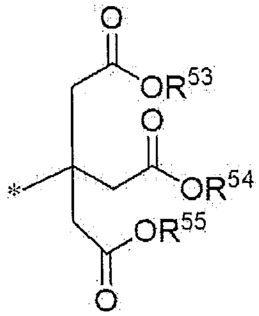

(22)

(in the formula (22), * is a bonding position, and

$R^{53}$ to $R^{55}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{53}$ is optionally replaced by at least one bond selected from the group consisting of an ester bond, an amide bond, a carbamate bond, and a carbonate bond, at least one ethylene group or at least one trimethylene group in $R^{54}$ is optionally replaced by at least one bond selected from the group consisting of an ester bond, an amide bond, a carbamate bond, and a carbonate bond, at least one ethylene group or at least one trimethylene group in $R^{55}$ is optionally replaced by at least one bond selected from the group consisting of an ester bond, an amide bond, a carbamate bond, and a carbonate bond, and $R^{53}$ to $R^{55}$ are each independently optionally substituted by a substituent selected from the group consisting of a halogen atom, a hydroxy group, and a hydrocarbon ring group having 3 to 12 carbon atoms).

[11] The cationic lipid of any one of the aforementioned [8] to [10], wherein $R^{39}$ in the formula (17) is

(iia-2-1) a monovalent group represented by the formula (23):

[Chem. 18]

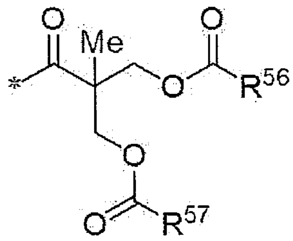

(23)

(in the formula (23), * is a bonding position,

Me is a methyl group, and
$R^{56}$ and $R^{57}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{56}$ is optionally replaced by at least one bond selected from the group consisting of an ester bond, an amide bond, a carbamate bond, and a carbonate bond, at least one ethylene group or at least one trimethylene group in $R^{57}$ is optionally replaced by at least one bond selected from the group consisting of an ester bond, an amide bond, a carbamate bond, and a carbonate bond, and $R^{56}$ and $R^{57}$ are each independently optionally substituted by a substituent selected from the group consisting of a halogen atom, a hydroxy group, and a hydrocarbon ring group having 3 to 12 carbon atoms), or

(iia-2-2) a monovalent group represented by the formula (24):

[Chem. 19]

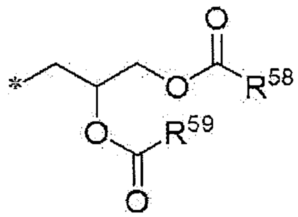

(24)

(in the formula (24), * is a bonding position, and
$R^{58}$ and $R^{59}$ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in $R^{58}$ is optionally replaced by at least one bond selected from the group consisting of an ester bond, an amide bond, a carbamate bond, and a carbonate bond, at least one ethylene group or at least one trimethylene group in $R^{59}$ is optionally replaced by at least one bond selected from the group consisting of an ester bond, an amide bond, a carbamate bond, and a carbonate bond, and $R^{58}$ and $R^{59}$ are each independently optionally substituted by a substituent selected from the group consisting of a halogen atom, a hydroxy group, and a hydrocarbon ring group having 3 to 12 carbon atoms).

[12] A lipid membrane structure comprising the cationic lipid of any one of the aforementioned [1] to [11] as a constituent lipid of the membrane.
[13] The lipid membrane structure of the aforementioned [12], further comprising a nucleic acid.
[14] A nucleic acid-introducing agent comprising the cationic lipid of any one of the aforementioned [1] to [11].
[15] The nucleic acid-introducing agent of the aforementioned [14], further comprising a nucleic acid.
[16] A pharmaceutical composition comprising the cationic lipid of any one of the aforementioned [1] to [11].
[17] The pharmaceutical composition of the aforementioned [16], further comprising a nucleic acid.
[18] A method for introducing a nucleic acid in a nucleic acid-introducing agent into a cell in vitro, comprising bringing the nucleic acid-introducing agent of the aforementioned [15] into contact with the cell.
[19] A method for introducing a nucleic acid in a nucleic acid-introducing agent into a target cell in a living organism, comprising administering the nucleic acid-introducing agent of the aforementioned [15] to the living organism.
[20] A method for producing a cellular medicine comprising a cell expressing a gene in a nucleic acid, comprising introducing the nucleic acid in a nucleic acid-introducing agent into a cell by bringing the nucleic acid-introducing agent of the aforementioned [15] into contact with the cell.

[Advantageous Effects of Invention]

The cationic lipid of the present invention can form a lipid membrane structure. The disulfide bond contained in the cationic lipid of the present invention is cleaved in the intracellular reductive environment, thus promoting release of materials (nucleic acid) enclosed therein. Hence, a nucleic acid-introducing agent using the cationic lipid of the present invention can achieve high efficiency of nucleic acid delivery into the cytoplasm. Therefore, the cationic lipid of the present invention is useful for introducing nucleic acid into cells and living organisms, and particularly useful as a pharmaceutical composition. In addition, since the aforementioned nucleic acid-introducing agent is superior in gene expression efficiency in cells, it can highly efficiently produce cells expressing a specific gene and is useful in the production of cellular medicines containing cells expressing a specific gene.

[Brief Description of Drawings]

[Fig. 1]
Fig. 1 is a graph showing the hemolysis activity of lipid nanoparticles (LNPs) at pH 7.4, calculated in Experimental Example 5.
[Fig. 2]
Fig. 2 is a graph showing the hemolysis activity of lipid nanoparticles (LNPs) at pH 5.5, calculated in Experimental Example 5.
[Fig. 3]
Fig. 3 is a graph showing the hemolysis activity of lipid nanoparticles (LNPs) at pH 7.4, calculated in Experimental Example 8.
[Fig. 4]
Fig. 4 is a graph showing the hemolysis activity of lipid nanoparticles (LNPs) at pH 5.5, calculated in Experimental Example 8.
[Fig. 5]
Fig. 5 is a graph showing the gene expression activity of lipid nanoparticles (LNPs) with an L/R ratio of 33 nmol/μg in vivo, evaluated in Experimental Example 9.
[Fig. 6]
Fig. 6 is a graph showing the gene expression activity of lipid nanoparticles (LNPs) with an L/R ratio of 50 nmol/μg in vivo, evaluated in Experimental Example 9.
[Fig. 7]
Fig. 7 is a graph showing the gene expression activity of lipid nanoparticles (LNPs) with an L/R ratio of 100 nmol/μg in vivo, evaluated in Experimental Example 9.

[Description of Embodiments]

The cationic lipid of the present invention is a cationic lipid represented by the following formula (1) :

[Chem. 20]

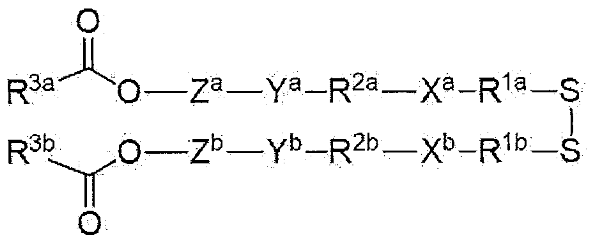

(1)

In the present specification and claims (hereinafter abbreviated as "the present specification"), the "cationic lipid represented by the formula (1)" is sometimes to be abbreviated as cationic lipid (1). Cationic lipids or compounds represented by other formulas are also sometimes to be abbreviated in the same manner as the "cationic lipids represented by the formula (1)".
First, alkylene groups and the like included in the formula (1) are explained.
In the present specification, the alkylene group may be linear or branched. Examples of the alkylene group include methylene group, ethylene group, trimethylene group ($-(CH_2)_3-$), propylene group ($-CH(CH_3)CH_2-$,

$-CH_2CH(CH_3)-$), tetramethylene group ($-(CH_2)_4-$), butylene group ($-CH(C_2H_5)CH_2-$, $-CH_2CH(C_2H_5)-$), pentamethylene group ($-(CH_2)_5-$), hexamethylene group ($-(CH_2)_6-$), heptamethylene group ($-(CH_2)_7-$), octamethylene group ($-(CH_2)_8-$), nonamethylene group ($-(CH_2)_9-$), and decamethylene group ($-(CH_2)_9-$) (in the aforementioned formulas, "-" is a single bond).

In the present specification, the "alkenediyl group" refers to a divalent group having a structure obtained by removing two hydrogen atoms from an alkene. In the present specification, the alkenediyl group may be linear or branched. The number of olefinic carbon-carbon double bonds in the alkene or alkenediyl group may be one or two or more. Examples of the alkenediyl group include ethenediyl group, propenediyl group, butenediyl group, pentenediyl group, hexenediyl group, heptenediyl group, octenediyl group, nonenediyl group, and decenediyl group. In the present specification, "compound name + diyl group (e.g., ethenediyl group)" refers to a divalent group having a structure obtained by removing two hydrogen atoms from the aforementioned compound.

In the present specification, the "alkynediyl group" means a divalent group having a structure obtained by removing two hydrogen atoms from an alkyne. In the present specification, the alkynediyl group may be linear or branched. In the present specification, the number of carbon-carbon triple bonds in the alkyne or alkynediyl group may be one or two or more. Examples of the alkynediyl group include ethynediyl group, propynediyl group, butynediyl group, pentynediyl group, hexynediyl group, heptynediyl group, octynediyl group, noninediyl group, and decynediyl group.

In the present specification, the "oxydialkylene group" means a divalent group having a structure in which two alkylene groups are bonded via an oxy group (-O-) (in the aforementioned formulas, "-"' is a single bond). The alkylene group in the "oxydialkylene group" is as explained above.

In the present specification, the "ester bond" means -COO- or -O-CO- (in the aforementioned formulas, "-" is a single bond).

In the present specification, the "amide bond" means -CO-NH- or -NH-CO- (in the aforementioned formulas, "-" is a single bond).

In the present specification, the "carbamate bond" means - O-CO-NH- or -NH-CO-O- (in the aforementioned formulas, "-" is a single bond).

In the present specification, the "ether bond" means -O-(in the aforementioned formulas, "-" is a single bond).

In the present specification, "urea bond" means -NH-CO-NH-(in the aforementioned formulas, "-"' is a single bond).

In the present specification, "carbonate bond" means -O-CO-O- (in the aforementioned formulas, "-"' is a single bond).

In the present specification, the "residue of a liposoluble vitamin having a hydroxy group" means a monovalent group having a structure obtained by removing a hydrogen atom from the hydroxy group of the aforementioned liposoluble vitamin. Examples of the liposoluble vitamin having a hydroxy group include retinol, ergosterol, 7-dehydrocholesterol, calciferol, cholecalciferol, dihydroergocalciferol, dihydrotachysterol, tocopherol, and tocotrienol.

In the present specification, the "sterol derivative having a hydroxy group residue" means a monovalent group having a structure obtained by removing a hydrogen atom from the hydroxy group of the aforementioned sterol derivative. Examples of the sterol derivative having a hydroxy group include cholesterol, cholestanol, stigmasterol, β-sitosterol, lanosterol, and ergosterol.

In the present specification, the alkyl group may be linear or branched. Examples of the alkyl group include methyl group, ethyl group, propyl group, isopropyl group, butyl group, isobutyl group, sec-butyl group, tert-butyl group, a pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, hentriacontyl group, dotriacontyl group, tritriacontyl group, tetratriacontyl group, pentatriacontyl group, hexatriacontyl group, tetracontyl group, hentetracontyl group, dotetracontyl group, tritetracontyl group, and tetratetracontyl group.

In the present specification, the alkenyl group may be linear or branched. In the present specification, the number of the olefinic carbon-carbon double bond in the alkenyl group may be only one, or two or more. Examples of the alkenyl group include ethenyl group, propenyl group, butenyl group, pentenyl group, hexenyl group, heptenyl group, octenyl group, nonenyl group, decenyl group, undecenyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, tricosenyl group, tetracosenyl group, pentacosenyl group, hexacosenyl group, heptacosenyl group, octacosenyl group, nonacosenyl group, triacontenyl group, hentriacontenyl group, and dotriacontenyl group.

In the present specification, the alkynyl group may be linear or branched. In the present specification, the number of the carbon-carbon triple bond of the alkynyl group may be only one, or two or more. Examples of the alkynyl group include ethynyl group, propynyl group, butynyl group, pentynyl group, hexynyl group, heptynyl group, octynyl group, nonynyl group, decynyl group, undecynyl group, dodecynyl group, tridecynyl group, tetradecynyl group, pentadecynyl

group, hexadecynyl group, heptadecynyl group, octadecynyl group, nonadecynyl group, icosynyl group, henicosynyl group, docosynyl group, tricosynyl group, tetracosinyl group, pentacosinyl group, hexacosinyl group, heptacosinyl group, octacosinyl group, nonacosinyl group, triacontinyl group, hentriacontinyl group, and dotriacontinyl group.

In the present specification, examples of the halogen atom include fluorine atom, chlorine atom, bromine atom, and iodine atom.

In the present specification, the "hydrocarbon ring group having 3 to 12 carbon atoms" means a cyclic group wherein the ring thereof is composed of 3 to 12 carbon atoms. Examples of the hydrocarbon ring group having 3 to 12 carbon atoms include cycloalkyl group having 3 to 8 carbon atoms, phenyl group, naphthyl group, and adamantyl group. Examples of the cycloalkyl group having 3 to 8 carbon atoms include cyclopropyl group, cyclobutyl group, cyclopentyl group, cyclohexyl group, cycloheptyl group, and cyclooctyl group. The hydrocarbon ring group having 3 to 12 carbon atoms is preferably a non-aromatic hydrocarbon ring group having 3 to 12 carbon atoms, more preferably a cycloalkyl group having 3 to 8 carbon atoms or an adamantyl group, further preferably a cyclohexyl group or an adamantyl group.

In the present specification, the "3- to 14-membered heterocyclic group" means a heterocyclic group wherein the ring thereof is composed of 3 to 14 carbon atoms. Other expressions similar to "3- to 14-membered" also have the same meaning as "3-to 14-membered". Examples of the 3- to 14-membered heterocyclic group include 5 to 14-membered aromatic heterocyclic group, and 3- to 14-membered non-aromatic heterocyclic group.

In the present specification, examples of the 5 to 14-membered aromatic heterocyclic group include the following:

(i) 5 to 6-membered monocyclic aromatic heterocyclic groups such as thienyl group, furyl group, pyrrolyl group, imidazolyl group, pyrazolyl group, thiazolyl group, isothiazolyl group, oxazolyl group, isoxazolyl group, pyridyl group, pyrazinyl group, pyrimidinyl group, pyridazinyl group, 1,2,4-oxadiazolyl group, 1,3,4-oxadiazolyl group, 1,2,4-thiadiazolyl group, 1,3,4-thiadiazolyl group, triazolyl group, tetrazolyl group, triazinyl group, and the like;

(ii) 8 to 14-membered fused polycyclic aromatic heterocyclic groups such as benzothiophenyl group, benzofuranyl group, benzoimidazolyl group, benzoxazolyl group, benzoisoxazolyl group, benzothiazolyl group, benzoisothiazolyl group, benzotriazolyl group, imidazopyridinyl group, thienopyridinyl group, furopyridinyl group, pyrrolopyridinyl group, pyrazolopyridinyl group, oxazolopyridinyl group, thiazolopyridinyl group, imidazopyrazinyl group, imidazopyrimidinyl group, thienopyrimidinyl group, furopyrimidinyl group, pyrrolopyrimidinyl group, pyrazolopyrimidinyl group, oxazolopyrimidinyl group, thiazolopyrimidinyl group, pyrazolotriazinyl group, naphtho[2,3-b]thienyl group, phenoxathiinyl group, indolyl group, isoindolyl group, 1H-indazolyl group, purinyl group, isoquinolyl group, quinolyl group, phthalazinyl group, naphthyridinyl group, quinoxalinyl group, quinazolinyl group, cinnolinyl group, carbazolyl group, β-carbolinyl group, phenanthridinyl group, acrydinyl group, phenazinyl group, phenothiazinyl group, phenoxazinyl group, and the like.

In the present specification, examples of the 3- to 14-membered non-aromatic heterocyclic group include the following:

(i) 3 to 8-membered monocyclic non-aromatic heterocyclic groups such as aziridinyl group, oxiranyl group, thiiranyl group, azetidinyl group, oxetanyl group, thietanyl group, tetrahydrothienyl group, tetrahydrofuranyl group, pyrrolinyl group, pyrrolidinyl group, imidazolinyl group, imidazolidinyl group, oxazolinyl group, oxazolidinyl group, pyrazolinyl group, pyrazolidinyl group, thiazolinyl group, thiazolidinyl group, tetrahydroisothiazolyl group, tetrahydroxazolyl group, tetrahydroisoxazolyl group, piperidinyl group, piperazinyl group, tetrahydropyridinyl group, dihydropyridinyl group, dihydrothiopyranyl group, tetrahydropyrimidinyl group, tetrahydropyridazinyl group, dihydropyranyl group, tetrahydropyranyl group, tetrahydrothiopyranyl group, morpholinyl group, thiomorpholinyl group, azepanyl group, diazepanyl group, azepinyl group, oxepanyl group, azocanyl group, diazocanyl group, dithioranyl group (e.g., 1,2-dithiolan-3-yl group), and the like;

(ii) 9 to 14-membered fused polycyclic non-aromatic heterocyclic groups such as dihydrobenzofuranyl group, dihydrobenzoimidazolyl group, dihydrobenzoxazolyl group, dihydrobenzothiazolyl group, dihydrobenzoisothiazolyl group, dihydronaphto[2,3-b]thienyl group, tetrahydroisoquinolyl group, tetrahydroquinolyl group, 4H-quinolizinyl group, indolinyl group, isoindolinyl group, tetrahydrothieno[2,3-c]pyridinyl group, tetrahydrobenzoazepinyl group, tetrahydroquinoxalinyl group, tetrahydrophenanthridinyl group, hexahydrophenothiazinyl group, hexahydrophenoxazinyl group, tetrahydrophthalazinyl group, tetrahydronaphthyridinyl group, tetrahydroquinazolinyl group, tetrahydrocinnolinyl group, tetrahydrocarbazolyl group, tetrahydro-β-carbolinyl group, tetrahydroacrydinyl group, tetrahydrophenazinyl group, tetrahydrothioxanthenyl group, octahydroisoquinolyl group, and the like.

$R^{1a}$, $R^{1b}$ and the like in the formula (1) are explained below. The explanations and preferred embodiments of the following $R^{1a}$, $R^{1b}$ and the like can be combined with each other.

In the formula (1), $R^{1a}$ and $R^{1b}$ are each independently an alkylene group having 1 to 6 carbon atoms. $R^{1a}$ and $R^{1b}$ may be the same or different, and $R^{1a}$ and $R^{1b}$ are preferably the same groups. $R^{1a}$ and $R^{1b}$ are preferably each

independently an alkylene group having 1 to 3 carbon atoms, more preferably each an alkylene group having 1 to 3 carbon atoms, further preferably an ethylene group. In the present specification, "$R^{1a}$ and $R^{1b}$ are each an alkylene group having 1 to 3 carbon atoms " means that $R^{1a}$ and $R^{1b}$ are the same and each is an alkylene group having 1 to 3 carbon atoms. Other expressions similar to "$R^{1a}$ and $R^{1b}$ are each an alkylene group having 1 to 3 carbon atoms" also have the same meaning as "$R^{1a}$ and $R^{1b}$ are each an alkylene group having 1 to 3 carbon atoms".

In the formula (1), $X^a$ and $X^b$ are each independently an acyclic alkyl tertiary amino group having 1 to 6 carbon atoms and 1 tertiary amino group, or a cyclic alkylene tertiary amino group having 2 to 5 carbon atoms and 1 or 2 tertiary amino groups.

In the present specification, the "acyclic alkyl tertiary amino group having 1 to 6 carbon atoms and 1 tertiary amino group" means a divalent group represented by the formula (25):

[Chem. 21]

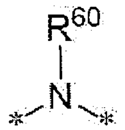

(25)

(in the formula (25), * is a bonding position, and
$R^{60}$ is an alkyl group having 1 to 6 carbon atoms). In the present specification, "*", etc. shows bonding positions, not carbon atoms, as described above.

$R^{60}$ in the formula (25) is preferably a methyl group, an ethyl group, a propyl group, or an isopropyl group, more preferably a methyl group.

In the present specification, the "cyclic alkylene tertiary amino group having 2 to 5 carbon atoms and 1 or 2 tertiary amino groups" means a divalent group in which an alkylene group having 2 to 5 carbon atoms and a tertiary amino group form a cyclic structure, and 1 or 2 tertiary amino groups are contained in the aforementioned cyclic structure. The aforementioned carbon number is preferably 4 or 5.

Examples of the a cyclic alkylene tertiary amino group having 2 to 5 carbon atoms and 1 or 2 tertiary amino groups include aziridinediyl group, azetidinediyl group, pyrrolidinediyl group, piperidinediyl group, imidazolidinediyl group, and piperazinediyl group.

The cyclic alkylene tertiary amino group having 2 to 5 carbon atoms and 1 tertiary amino group is preferably a divalent group represented by the formula (26):

[Chem. 22]

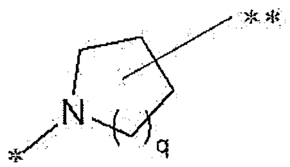

(26)

(in the formula (26), * is a bonding position with $R^{1a}$ or $R^{1b}$ in the formula (1),

** is a bonding position with $R^{2a}$ or $R^{2b}$ in the formula (1), and
q is 1 or 2).

Hereinafter the "divalent group represented by the formula (26)" is sometimes to be referred to as "group (26)". Groups represented by other formulas are also sometimes to be abbreviated in the same manner as the "divalent group represented by the formula (26)". When q is 1, group (26) is a pyrrolidinediyl group, and when q is 2, group (26) is a piperidinediyl group.

A cyclic alkylene tertiary amino group having 2 to 5 carbon atoms and 2 tertiary amino groups is preferably a divalent group represented by the formula (27):

[Chem. 23]

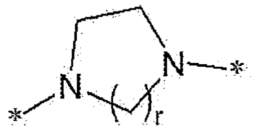

(27)

(in the formula (27), * is a bonding position, and

r is 1 or 2). When r is is 1, group (27) is an imidazolidinediyl group, When r is 2, group (27) is a piperazinediyl group.

$X^a$ and $X^b$ may be the same or different, and $X^a$ and $X^b$ are preferably the same groups. In the formula (1), $X^a$ and $X^b$ are preferably each independently a cyclic alkylene tertiary amino group having 2 to 5 carbon atoms and 1 or 2 tertiary amino groups, more preferably each independently group (26) or group (27), further preferably each independently group (26), particularly preferably each group (26) wherein q is 2 (i.e., piperidinediyl group).

In the formula (1), $R^{2a}$ and $R^{2b}$ are each independently an alkylene group having 1 to 8 carbon atoms or an oxydialkylene group having 2 to 8 carbon atoms. The oxydialkylene group having 2 to 8 carbon atoms is preferably an oxydimethylene group, an oxydiethylene group, an oxydipropylene group, more preferably an oxydiethylene group.

In the formula (1), $R^{2a}$ and $R^{2b}$ are preferably each independently an alkylene group having 1 to 8 carbon atoms, more preferably each independently an alkylene group having 1 to 4 carbon atoms, further preferably each an ethylene group.

In the formula (1), $Y^a$ and $Y^b$ are each independently an ester bond, an amide bond, a carbamate bond, an ether bond, or a urea bond. $Y^a$ and $Y^b$ may be the same or different, preferably $Y^a$ and $Y^b$ are the same bonds. $Y^a$ and $Y^b$ are preferably each independently an ester bond or an amide bond, more preferably each an ester bond, further preferably each *-CO-O-** (wherein * is a bonding position with $Z^a$ or $Z^b$ in the formula (1), and ** is a bonding position with $R^{2a}$ or $R^{2b}$ in the formula (1)).

In the formula (1), $Z^2$ and $Z^b$ are each independently a divalent group derived from an aromatic compound having 3 to 16 carbon atoms, at least one aromatic ring, and optionally having a heteroatom. In the following, the aforementioned aromatic ring and the aforementioned aromatic compound are sometimes to be abbreviated as "aromatic ring of $Z^a$ and $Z^b$".

The aromatic ring of $Z^a$ and $Z^b$ may be either an aromatic hydrocarbon ring or an aromatic heterocycle. Examples of the aromatic hydrocarbon ring include benzene ring, naphthalene ring, and anthracene ring. Examples of the aromatic heterocycle include imidazole ring, pyrazole ring, oxazole ring, isoxazole ring, thiazole ring, isothiazole ring, triazine ring, pyrrole ring, furanthiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxathiine ring, phenothiazine ring, and acridine ring. The aromatic ring of $Z^a$ and $Z^b$ is preferably an aromatic hydrocarbon ring, more preferably a benzene ring.

The aromatic ring of $Z^a$ and $Z^b$ may be substituted by a substituent. Examples of the substituent thereof include acyl group having 2 to 4 carbon atoms, alkoxycarbonyl group having 2 to 4 carbon atoms, alkylcarbamoyl group having 2 to 4 carbon atoms, acyloxy group having 2 to 4 carbon atoms, acylamino group having 2 to 4 carbon atoms, alkoxycarbonylamino group having 2 to 4 carbon atoms, halogen atom (i.e., fluorine atom, chlorine atom, bromine atom, iodine atom), alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group, alkoxy group having 1 to 4 carbon atoms, aryl group having 6 to 10 carbons, and aryloxy group having 6 to 10 carbon atoms. Preferred examples of the aforementioned substituent include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, and phenoxy group.

$Z^a$ and $Z^b$ are preferably each independently a divalent group represented by the formula (13):

[Chem. 24]

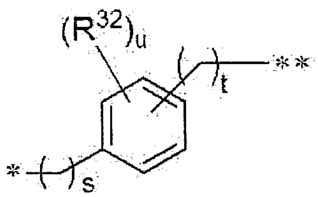

(13)

(in the formula (13), * is a bonding position with O in the formula (1),

** is a bonding position with $Y^a$ or $Y^b$ in the formula (1),
s is an integer of 0 to 3,
t is an integer of 0 to 3,
u is an integer of 0 to 4, and

$R^{32}$ in the number of u are each independently a substituent). The aforementioned "s and t" shows the number of methylene groups ($CH_2$), and "s or t is is 0" means that the corresponding methylene group is not present. The aforementioned "u" shows the number of $R^{32}$, and "u is is 0" means that $R^{32}$ is not present.

s is preferably 0 or 1, more preferably 0.
t is preferably an integer of 0 to 2, more preferably 1.
u is preferably an integer of 0 to 2, more preferably 0.
$R^{32}$ is

preferably an acyl group having 2 to 4 carbon atoms, an alkoxycarbonyl group having 2 to 4 carbon atoms, an alkylcarbamoyl group having 2 to 4 carbon atoms, an acyloxy group having 2 to 4 carbon atoms, an acylamino group having 2 to 4 carbon atoms, an alkoxycarbonylamino group having 2 to 4 carbon atoms, a halogen atom (i.e., fluorine atom, chlorine atom, bromine atom, iodine atom), an alkylsulfanyl group having 1 to 4 carbon atoms, an alkylsulfonyl group having 1 to 4 carbon atoms, an arylsulfonyl group having 6 to 10 carbon atoms, a nitro group, a trifluoromethyl group, a cyano group, an alkyl group having 1 to 4 carbon atoms, a ureido group, an alkoxy group having 1 to 4 carbon atoms, an aryl group having 6 to 10 carbon atoms, or an aryloxy group having 6 to 10 carbon atoms,
more preferably an acetyl group, a methoxycarbonyl group, a methylcarbamoyl group, an acetoxy group, an acetamido group, a methoxycarbonylamino group, a halogen atom (i.e., fluorine atom, chlorine atom, bromine atom, iodine atom), a methylsulfanyl group, a phenylsulfonyl group, a nitro group, a trifluoromethyl group, a cyano group, an alkyl group having 1 to 4 carbon atoms (e.g., methyl group, ethyl group, propyl group, isopropyl group, tert-butyl group), a ureido group, an alkoxy group having 1 to 4 carbon atoms (e.g., methoxy group, ethoxy group, propoxy group, isopropoxy group, tert-butoxy group), a phenyl group, or a phenoxy group,
further preferably a halogen atom (i.e., fluorine atom, chlorine atom, bromine atom, iodine atom), an alkyl group having 1 to 4 carbon atoms (e.g., methyl group, ethyl group, propyl group, isopropyl group, tert-butyl group), or an alkoxy group having 1 to 4 carbon atoms (e.g., methoxy group, ethoxy group, propoxy group, isopropoxy group, tert-butoxy group),
particularly preferably a halogen atom (i.e., fluorine atom, chlorine atom, bromine atom, iodine atom) or an alkoxy group having 1 to 4 carbon atoms (e.g., methoxy group, ethoxy group).

When $R^{32}$ is present in plurality, the $R^{32}$ in plurality may be the same with or different from each other.
$Z^a$ and $Z^b$ may be the same or different, and $Z^a$ and $Z^b$ are preferably the same. $Z^a$ and $Z^b$ in the formula (1) are preferably each independently group (13), more preferably each independently group (13) in which s is 0 or 1, t is an integer of 0 to 2, and u is an integer of 0 to 2, more preferably each group (13) in which s is 0, t is 1, and u is 0.
$R^{3a}$ in the formula (1) is a monovalent group recited below: (ia) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group) (hereinafter sometimes to be abbreviated as "group (ia)"),

(iia) a monovalent group having 10 to 50 carbon atoms and at least two carbonyl groups (excluding a monovalent group containing a residue of a liposoluble vitamin having a hydroxy group and a residue of a sterol derivative having a hydroxy group) (hereinafter sometimes to be abbreviated as "group (iia)"),
(iiia) a monovalent group represented by the formula (2):

$$*\text{-}R^4\text{-}X^1\text{-}R^5 \quad (2)$$

(in the formula (2), * is a bonding position,

$R^4$ is an alkylene group having 1 to 10 carbon atoms,
$X^1$ is a carbamate bond, a carbonate bond, or an amide bond, and
$R^5$ is an alkyl group having 1 to 25 carbon atoms, and $R^5$ is optionally substituted by a substituent selected from the group consisting of a halogen atom and a hydroxy group)

(hereinafter sometimes to be abbreviated as "group (iiia)"),

(iva) a monovalent group represented by the formula (3):

$$*\text{-}R^6\text{-CO-O-}R^7 \quad (3)$$

(in the formula (3), * is a bonding position,

$R^6$ is an alkylene group having 1 to 10 carbon atoms, and
$R^7$ is an alkyl group having 1 to 25 carbon atoms and substituted by at least one halogen atom)

(hereinafter sometimes to be abbreviated as "group (iva)"),

(va) a monovalent group represented by the formula (4):

[Chem. 25]

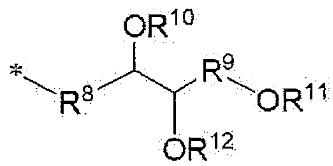

(4)

(in the formula (4), * is a bonding position,

$R^8$ and $R^9$ are each independently an alkylene group having 1 to 10 carbon atoms, an alkenediyl group having 2 to 10 carbon atoms, or an alkynediyl group having 2 to 10 carbon atoms,
$R^{10}$ to $R^{12}$ are each independently a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group))

(hereinafter sometimes to be abbreviated as "group (va)"),

(via) a monovalent group represented by the formula (5):

[Chem. 26]

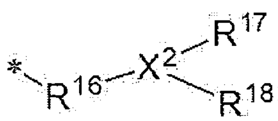

(5)

(in the formula (5), * is a bonding position,
$X^2$ is a nitrogen atom or a trivalent group represented by the formula (6):

[Chem. 27]

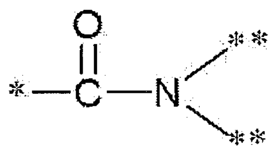

(6)

(in the formula (6), * is a bonding position with $R^{16}$, and

** is a bonding position with $R^{17}$ or $R^{18}$),
when $X^2$ is a nitrogen atom, $R^{16}$ is an alkylene group having 1 to 10 carbon atoms, an alkenediyl group having 2 to 10 carbon atoms, or an alkynediyl group having 2 to 10 carbon atoms, and $R^{16}$ is optionally substituted by a substituent selected from the group consisting of a halogen atom and a hydroxy group,
when $X^2$ is a trivalent group represented by the formula (6), $R^{16}$ is an alkylene group having 1 to 10 carbon atoms, and $R^{16}$ is optionally substituted by a substituent selected from the group consisting of a halogen atom and a hydroxy group,
when $X^2$ is a nitrogen atom, $R^{17}$ and $R^{18}$ are each independently an alkyl group having 1 to 10 carbon atoms, an alkenyl group having 2 to 10 carbon atoms, or an alkynyl group having 2 to 10 carbon atoms, and $R^{17}$ and

$R^{18}$ are each independently optionally substituted by a substituent selected from the group consisting of a halogen atom and a hydroxy group, and
when $X^2$ is a trivalent group represented by the formula (6), $R^{17}$ and $R^{18}$ are each independently an alkyl group having 1 to 10 carbon atoms, and $R^{17}$ and $R^{18}$ are each independently optionally substituted by a substituent selected from the group consisting of a halogen atom and a hydroxy group)

(hereinafter sometimes to be abbreviated as "group (via)"),

(viia) a monovalent group represented by the formula (7): [0131]

[Chem. 28]

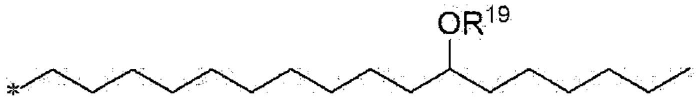
(7)

(in the formula (7), * is a bonding position, and
$R^{19}$ is a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group), or a *-CO-$R^{20}$ group (wherein * is a bonding position, and $R^{20}$ is an alkyl group having 1 to 9 carbon atoms))
(hereinafter sometimes to be abbreviated as "group (viia)"),

(viiia) a monovalent group represented by the formula (8):

[Chem. 29]

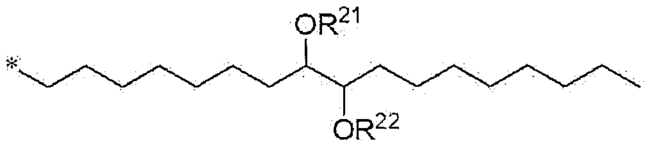
(8)

(in the formula (8), * is a bonding position, and
$R^{21}$ and $R^{22}$ are each independently a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group))
(hereinafter sometimes to be abbreviated as "group (viiia)"),

(ixa) a monovalent group represented by the formula (9):

[Chem. 30]

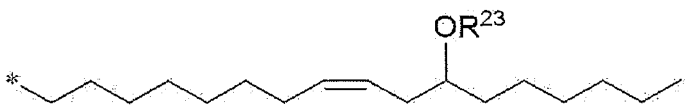
(9)

(in the formula (9), * is a bonding position, and
$R^{23}$ is a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group))
(hereinafter sometimes to be abbreviated as "group (ixa)"),

(xa) a monovalent group represented by the formula (10):

[Chem. 31]

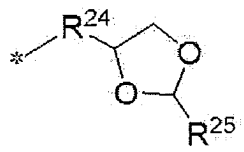

(10)

(in the formula (10), * is a bonding position,

$R^{24}$ is an alkylene group having 1 to 10 carbon atoms, an alkenediyl group having 2 to 10 carbon atoms, or an alkynediyl group having 2 to 10 carbon atoms, and
$R^{25}$ is an alkyl group having 1 to 30 carbon atoms, an alkenyl group having 2 to 30 carbon atoms, or an alkynyl group having 2 to 30 carbon atoms)

(hereinafter sometimes to be abbreviated as "group (xa)"),

(xia) a monovalent group represented by the formula (11):

[Chem. 32]

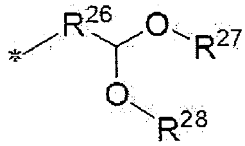

(11)

(in the formula (11), * is a bonding position, and

$R^{26}$ is an alkylene group having 1 to 10 carbon atoms, an alkenediyl group having 2 to 10 carbon atoms, or an alkynediyl group having 2 to 10 carbon atoms, and
$R^{27}$ and $R^{28}$ are each independently an alkyl group having 1 to 10 carbon atoms, an alkenyl having 2 to 10 carbon atoms, or an alkynyl group having 2 to 10 carbon atoms)

(hereinafter sometimes to be abbreviated as "group (xia)"), or

(xiia) a monovalent group represented by the formula (12):

[Chem. 33]

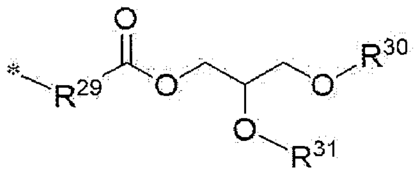

(12)

(in the formula (12), * is a bonding position,

$R^{29}$ is an alkylene group having 1 to 10 carbon atoms, and
$R^{30}$ and $R^{31}$ are each independently an alkyl group having 1 to 10 carbon atoms)

(hereinafter sometimes to be abbreviated as "group (xiia)").

$R^{3b}$ in the formula (1) is a monovalent group recited below:

(ib) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group) (hereinafter sometimes to be abbreviated as "group (1b)"),
(iib) a monovalent group having 10 to 50 carbon atoms and at least two carbonyl groups (excluding a monovalent

group containing a residue of a liposoluble vitamin having a hydroxy group and a residue of a sterol derivative having a hydroxy group) (hereinafter sometimes to be abbreviated as "group (iib)"),
(iiib) a monovalent group represented by the aforementioned formula (2) (hereinafter sometimes to be abbreviated as "group (iiib)"),
(ivb) a monovalent group represented by the aforementioned formula (3) (hereinafter sometimes to be abbreviated as "group (ivb)"),
(vb) a monovalent group represented by the aforementioned formula (4) (hereinafter sometimes to be abbreviated as "group (vb) "),
(vib) a monovalent group represented by the aforementioned formula (5) (hereinafter sometimes to be abbreviated as "group (vib) "),
(viib) a monovalent group represented by the aforementioned formula (7) (hereinafter sometimes to be abbreviated as "group (viib)"),
(viiib) a monovalent group represented by the aforementioned formula (8) (hereinafter sometimes to be abbreviated as "group (viiib)"),
(ixb) a monovalent group represented by the aforementioned formula (9) (hereinafter sometimes to be abbreviated as "group (ixb)"),
(xb) a monovalent group represented by the aforementioned formula (10) (hereinafter sometimes to be abbreviated as "group (xb) "),
(xib) a monovalent group represented by the aforementioned formula (11) (hereinafter sometimes to be abbreviated as "group (xib)"),
(xiib) a monovalent group represented by the aforementioned formula (12) (hereinafter sometimes to be abbreviated as "group (xiib)"),
(xiiib) a monovalent group which is an alkyl group having 1 to 30 carbon atoms, an alkenyl group having 2 to 20 carbon atoms, or an alkynyl group having 2 to 20 carbon atoms, in which one ethylene group in the aforementioned alkyl group is optionally replaced by one ester bond (hereinafter sometimes to be abbreviated as "group (xiiib)"), or
(xivb) a $R^{3c}$-CO-$(CH_2)_p$- group (wherein $R^{3c}$ is a residue of a liposoluble vitamin having a hydroxy group or a residue of a sterol derivative having a hydroxy group, and p is an integer of 1 to 8) (hereinafter sometimes to be abbreviated as "group (xivb)").

In the formula (1), $R^{3a}$ and $R^{3b}$ may be the same or different. In the following, "group (ia) and group (ib)" and the like may be collectively referred to as "group (i)" and the like.

In a preferred embodiment of the present invention, one or both of $R^{3a}$ and $R^{3b}$ are group (i) or group (ii). In other words, in a preferred embodiment of the present invention, $R^{3a}$ is group (ia) or group (iia), and $R^{3b}$ is any of group (ib) to group (xivb). In the aforementioned embodiment, $R^{3b}$ is preferably group (ib), group (iib), group (xiiib), or group (xivb). In the aforementioned embodiment, $R^{3a}$ and $R^{3b}$ may be the same or different.

In another embodiment of the present invention, $R^{3a}$ is any of group (iiia) to group (xiia), and $R^{3b}$ is any of group (iiib) to group (xivb). In the aforementioned embodiment, $R^{3b}$ is preferably group (xiiib) or group (xivb), more preferably group (xiiib). In the aforementioned embodiment, $R^{3a}$ and $R^{3b}$ may be the same or different.

$R^{3a}$ is preferably a monovalent group recited below:

(ia-1) a monovalent group having 10 to 50 carbon atoms and represented by the formula (14):

$$*\text{-}R^{33}\text{-CO-}X^{3}\text{-}R^{34} \qquad (14)$$

(in the formula (14), * is a bonding position,

$R^{33}$ is an alkylene group having 2 to 10 carbon atoms, an alkenediyl group having 2 to 10 carbon atoms, or an alkynediyl group having 2 to 10 carbon atoms, and $R^{33}$ is optionally substituted by a substituent selected from the group consisting of a halogen atom, a hydroxy group, and a hydrocarbon ring group having 3 to 12 carbon atoms,
$R^{34}$ is an alkyl group having 1 to 40 carbon atoms, an alkenyl group having 2 to 40 carbon atoms, or an alkynyl group having 2 to 40 carbon atoms, and $R^{34}$ is optionally substituted by a substituent selected from the group consisting of a halogen atom, a hydroxy group, and a hydrocarbon ring group having 3 to 12 carbon atoms,
at least one of $R^{33}$ and $R^{34}$ has at least one unsaturated bond selected from the group consisting of an olefinic carbon-carbon double bond and a carbon-carbon triple bond, and
$X^3$ is an oxygen atom, NH, or a sulfur atom)

(hereinafter sometimes to be abbreviated as "group (ia-1)"),

(ia-2) a monovalent group having 50 or less carbon atoms and represented by the formula (15):

[Chem. 34]

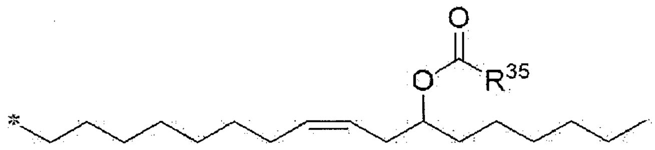

(15)

(in the formula (15), * is a bonding position, and
$R^{35}$ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, at least one ethylene group or at least one trimethylene group in $R^{35}$ is optionally replaced by at least one bond selected from the group consisting of an ester bond, an amide bond, a carbamate bond, and a carbonate bond, and $R^{35}$ is optionally substituted by a substituent selected from the group consisting of an alkoxy group having 1 to 4 carbon atoms, a 3- to 14-membered heterocyclic group, and a hydrocarbon ring group having 3 to 12 carbon atoms)
(hereinafter sometimes to be abbreviated as "group (ia-2)"),

(iia-1) a monovalent group having 50 or less carbon atoms and represented by the formula (16):

$$*\text{-}R^{36}\text{-CO-}X^4\text{-}R^{37} \qquad (16)$$

(in the formula (16), * is a bonding position,

$R^{36}$ is an alkylene group having 2 to 9 carbon atoms, an alkenediyl group having 2 to 9 carbon atoms, or an alkynediyl group having 2 to 9 carbon atoms, and $R^{36}$ is optionally substituted by a substituent selected from the group consisting of a halogen atom, a hydroxy group, and a hydrocarbon ring group having 3 to 12 carbon atoms,
$R^{37}$ is an alkyl group having 7 to 45 carbon atoms, an alkenyl group having 7 to 45 carbon atoms, or an alkynyl group having 7 to 45 carbon atoms, at least one ethylene group or at least one trimethylene group in $R^{37}$ is replaced by at least one bond selected from the group consisting of an ester bond, an amide bond, a carbamate bond, and a carbonate bond, and $R^{37}$ is optionally substituted by a substituent selected from the group consisting of a halogen atom, a hydroxy group, and a hydrocarbon ring group having 3 to 12 carbon atoms, and
$X^4$ is an oxygen atom, NH, or a sulfur atom)

(hereinafter sometimes to be abbreviated as "group (iia-1)"),

(iia-2) a monovalent group having 10 to 50 carbon atoms and represented by the formula (17):

$$*\text{-}R^{38}\text{-O-}R^{39} \qquad (17)$$

(in the formula (17), * is a bonding position, and

$R^{38}$ is an alkylene group having 2 to 10 carbon atoms, an alkenediyl group having 2 to 10 carbon atoms, or an alkynediyl group having 2 to 10 carbon atoms, at least one ethylene group or at least one trimethylene group in $R^{38}$ is optionally replaced by at least one bond selected from the group consisting of an ester bond, an amide bond, a carbamate bond, and a carbonate bond, and $R^{38}$ is optionally substituted by a substituent selected from the group consisting of a halogen atom, a hydroxy group, and a hydrocarbon ring group having 3 to 12 carbon atoms, and
$R^{39}$ is an alkyl group having 3 to 30 carbon atoms, an alkenyl group having 4 to 30 carbon atoms, or an alkenyl group having 4 to 30 carbon atoms, at least two methylene groups in $R^{39}$ are replaced by at least two carbonyl groups, and at least one methylene group of $R^{39}$ is optionally replaced by at least one ether bond)

(hereinafter sometimes to be abbreviated as "group (iia-2)"),

(iia-3) a monovalent group having 50 or less carbon atoms and represented by the formula (18):

[Chem. 35]

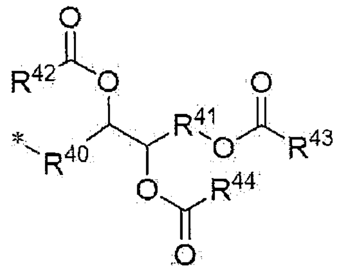

(18)

(in the formula (18), * is a bonding position,

$R^{40}$ and $R^{41}$ are each independently an alkylene group having 3 to 10 carbon atoms, and
$R^{42}$ to $R^{44}$ are each independently an alkyl group having 1 to 10 carbon atoms, an alkenyl group having 2 to 10 carbon atoms or an alkynyl group having 2 to 10 carbon atoms, at least one ethylene group or at least one trimethylene group in $R^{42}$ is optionally replaced by at least one bond selected from the group consisting of an ester bond, an amide bond, a carbamate bond, and a carbonate bond, at least one ethylene group or at least one trimethylene group in $R^{43}$ is optionally replaced by at least one bond selected from the group consisting of an ester bond, an amide bond, a carbamate bond, and a carbonate bond, at least one ethylene group or at least one trimethylene group in $R^{44}$ is optionally replaced by at least one bond selected from the group consisting of an ester bond, an amide bond, a carbamate bond, and a carbonate bond, and $R^{42}$ to $R^{44}$ are each independently optionally substituted by a substituent selected from the group consisting of a halogen atom, a hydroxy group, and a hydrocarbon ring group having 3 to 12 carbon atoms)

(hereinafter sometimes to be abbreviated as "group (iia-3)"), or

(iia-4) a monovalent group having 50 or less carbon atoms and represented by the formula (19):

[Chem. 36]

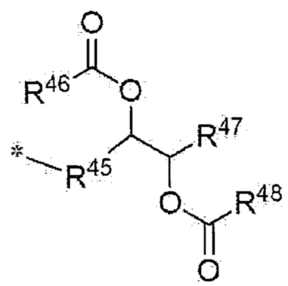

(19)

(in the formula (19), * is a bonding position,

$R^{45}$ is an alkylene group having 5 to 10 carbon atoms, and
$R^{46}$ to $R^{48}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{46}$ is optionally replaced by at least one bond selected from the group consisting of an ester bond, an amide bond, a carbamate bond, and a carbonate bond, at least one ethylene group or at least one trimethylene group in $R^{47}$ is optionally replaced by at least one bond selected from the group consisting of an ester bond, an amide bond, a carbamate bond, and a carbonate bond, at least one ethylene group or at least one trimethylene group in $R^{48}$ is optionally replaced by at least one bond selected from the group consisting of an ester bond, an amide bond, a carbamate bond, and a carbonate bond, and $R^{46}$ to $R^{48}$ are each independently optionally substituted by a substituent selected from the group consisting of a halogen atom, a hydroxy group, and a hydrocarbon ring group having 3 to 12 carbon atoms)

(hereinafter sometimes to be abbreviated as "group (iia-4)").

$R^{3b}$ is preferably a monovalent group recited below:

(ib-1) a monovalent group having 10 to 50 carbon atoms and represented by the aforementioned formula (14) (hereinafter sometimes to be abbreviated as "group (ib-1)"),
(ib-2) a monovalent group having 50 or less carbon atoms and represented by the aforementioned formula (15) (hereinafter sometimes to be abbreviated as "group (ib-2)"),
(iib-1) a monovalent group having 50 or less carbon atoms and represented by the aforementioned formula (16) (hereinafter sometimes to be abbreviated as "group (iib-1)"),
(iib-2) a monovalent group having 10 to 50 carbon atoms and represented by the aforementioned formula (17) (hereinafter sometimes to be abbreviated as "group (iib-2)"),
(iib-3) a monovalent group having 50 or less carbon atoms and represented by the aforementioned formula (18) (hereinafter sometimes to be abbreviated as "group (iib-3)"),
(iib-4) a monovalent group having 50 or less carbon atoms and represented by the aforementioned formula (19) (hereinafter sometimes to be abbreviated as "group (iib-4)"), or any of group (iiib) to group (xivb).

Group (ia) and the like are explained below in order.

Group (ia) is preferably group (ia-1) (i.e., group (14)) or group (ia-2) (i.e., group (15)). Group (ib) is preferably group (ib-1) (i.e., group (14)) or group (ib-2) (i.e., group (15)).

$R^{33}$ in the formula (14) is as mentioned above, and an alkylene group having 2 to 10 carbon atoms, an alkenediyl group having 2 to 10 carbon atoms, or an alkynediyl group having 2 to 10 carbon atoms, and $R^{33}$ is optionally substituted by a substituent selected from the group consisting of a halogen atom, a hydroxy group, and a hydrocarbon ring group having 3 to 12 carbon atoms.

In the present specification, "$R^{33}$ is optionally substituted by a substituent selected from the group consisting of a halogen atom, a hydroxy group, and a hydrocarbon ring group having 3 to 12 carbon atoms" means that the aforementioned alkylene group, the aforementioned alkenediyl group and the aforementioned alkynediyl group for $R^{33}$ are each independently optionally substituted by a substituent selected from the group consisting of a halogen atom, a hydroxy group, and a hydrocarbon ring group having 3 to 12 carbon atoms. Other expressions similar to "$R^{33}$ is a optionally substituted by a substituent selected from the group consisting of a halogen atom, a hydroxy group, and a hydrocarbon ring group having 3 to 12 carbon atoms" also have the same meaning as "$R^{33}$ is a optionally substituted by a substituent selected from the group consisting of a halogen atom, a hydroxy group, and a hydrocarbon ring group having 3 to 12 carbon atoms".

$R^{33}$ in the formula (14) is preferably an alkylene group having 2 to 10 carbon atoms, an alkenediyl group having 2 to 10 carbon atoms, or an alkynediyl group having 2 to 10 carbon atoms, more preferably an alkylene group having 2 to 8 carbon atoms or an alkenediyl group having 2 to 8 carbon atoms, further preferably an alkylene group having 2 to 8 carbon atoms. In the present specification, unless otherwise specified regarding the substituent, "alkylene group", "alkenediyl group", "alkynediyl group", "alkyl group", "alkenyl group", and "alkynyl group" show unsubstituted groups.

$R^{34}$ in the formula (14) is preferably an alkyl group having 1 to 40 carbon atoms, an alkenyl group having 2 to 40 carbon atoms, or an alkynyl group having 2 to 40 carbon atoms, more preferably an alkyl group having 1 to 20 carbon atoms, an alkenyl group having 2 to 20 carbon atoms, or an alkynyl group having 2 to 20 carbon atoms, further preferably an alkenyl group having 2 to 20 carbon atoms or an alkynyl group having 2 to 20 carbon atoms.

At least one of $R^{33}$ and $R^{34}$ in the formula (14) has, as mentioned above, at least one unsaturated bond selected from the group consisting of an olefinic carbon-carbon double bond and a carbon-carbon triple bond.

$X^3$ in the formula (14) is preferably an oxygen atom.

Group (14) is preferably a monovalent group derived from intermediate 19, intermediate 20, intermediate 21, or intermediate 22, produced in the below-mentioned Examples. In the present specification, the "monovalent group derived from intermediate X" (X: integer of one or more) means a monovalent group having a structure obtained by removing a carboxy group from intermediate X.

$R^{35}$ in the formula (15) is preferably an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and $R^{35}$ is optionally substituted by a substituent selected from the group consisting of a 3- to 14-membered heterocyclic group and a hydrocarbon ring group having 3 to 12 carbon atoms.

$R^{35}$ in the formula (15) is more preferably an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, and the aforementioned alkyl group is optionally substituted by a hydrocarbon ring group having 3 to 12 carbon atoms.

In one embodiment of the present invention, $R^{35}$ in the formula (15) is further preferably an alkyl group having 1 to 20 carbon atoms, an alkenyl group having 2 to 20 carbon atoms, an alkynyl group having 2 to 20 carbon atoms, or a cyclohexyl group, and the aforementioned alkyl group is optionally substituted by an adamantyl group.

In one embodiment of the present invention, $R^{35}$ in the formula (15) is further preferably an alkyl group having 1 to 20 carbon atoms, an alkenyl group having 2 to 20 carbon atoms, or an alkynyl group having 2 to 20 carbon atoms, and the aforementioned alkyl group is optionally substituted by a cyclohexyl group.

$R^{35}$ in the formula (15) is particularly preferably an alkyl group having 1 to 20 carbon atoms, an alkenyl group having 2 to 20 carbon atoms, or an alkynyl group having 2 to 20 carbon atoms.

Group (15) is preferably a monovalent group derived from intermediate 2, intermediate 3, intermediate 4, intermediate 5, intermediate 6, intermediate 7, intermediate 8, intermediate 9, intermediate 10, intermediate 55, intermediate 61, intermediate 62, intermediate 63, intermediate 64, intermediate 65, or intermediate 66, produced in the below-mentioned Examples, more preferably a monovalent group derived from intermediate 2, intermediate 3, intermediate 4, intermediate 5, intermediate 6, intermediate 7, intermediate 8, intermediate 9, intermediate 10, or intermediate 55.

Group (iia) is preferably group (iia-1) (i.e., group (16)), group (iia-2) (i.e., group (17)), group (iia-3) (i.e., group (18)), or group (iia-4) (i.e., group (19)). Group (iib) is preferably group (iib-1) (i.e., group (16)), group (iib-2) (i.e., group (17)), group (iib-3) (i.e., group (18)), or group (iib-4) (i.e., group (19)).

$X^4$ in the formula (16) is preferably an oxygen atom or NH, more preferably an oxygen atom.

In one embodiment of the present invention, $R^{36}$ in the formula (16) is preferably an alkylene group having 2 to 9 carbon atoms, an alkenediyl group having 2 to 9 carbon atoms, or an alkynediyl group having 2 to 9 carbon atoms, more preferably an alkylene group having 2 to 9 carbon atoms or an alkenediyl group having 2 to 9 carbon atoms, further preferably an alkylene group having 2 or 3 carbon atoms or an alkenediyl group having 2 or 3 carbon atoms.

In one embodiment of the present invention, $R^{36}$ in the formula (16) is preferably an alkylene group having 2 to 9 carbon atoms, an alkenediyl group having 2 to 9 carbon atoms, or an alkynediyl group having 2 to 9 carbon atoms, more preferably an alkylene group having 2 to 9 carbon atoms, further preferably an alkylene group having 2 or 3 carbon atoms.

$R^{37}$ in the formula (16) is, as mentioned above, an alkyl group having 7 to 45 carbon atoms, an alkenyl group having 7 to 45 carbon atoms, or an alkynyl group having 7 to 45 carbon atoms, at least one ethylene group or at least one trimethylene group in $R^{37}$ is replaced by at least one bond selected from the group consisting of an ester bond, an amide bond, a carbamate bond, and a carbonate bond, and $R^{37}$ is optionally substituted by a substituent selected from the group consisting of a halogen atom, a hydroxy group, and a hydrocarbon ring group having 3 to 12 carbon atoms.

In the present specification, "at least one ethylene group or at least one trimethylene group in $R^{37}$ is replaced by at least one bond selected from the group consisting of an ester bond, an amide bond, a carbamate bond, and a carbonate bond" means that at least one ethylene group or at least one trimethylene group in the aforementioned alkyl group for $R^{37}$ is replaced by at least one bond selected from the group consisting of an ester bond, an amide bond, a carbamate bond, and a carbonate bond, or at least one ethylene group or at least one trimethylene group in the aforementioned alkenyl for $R^{37}$ is replaced by at least one bond selected from the group consisting of an ester bond, an amide bond, a carbamate bond, and a carbonate bond, or at least one ethylene group or at least one trimethylene group in the aforementioned alkynyl group for $R^{37}$ is replaced by at least one bond selected from the group consisting of an ester bond, an amide bond, a carbamate bond, and a carbonate bond. Other expressions similar to "at least one ethylene group or at least one trimethylene group in $R^{37}$ is replaced by at least one bond selected from the group consisting of an ester bond, an amide bond, a carbamate bond, and a carbonate bond" also have the same meaning as "at least one ethylene group or at least one trimethylene group in $R^{37}$ is replaced by at least one bond selected from the group consisting of an ester bond, an amide bond, a carbamate bond, and a carbonate bond".

$R^{37}$ in the formula (16) is preferably

(iia-1-1) a monovalent group represented by the formula (20):

[Chem. 37]

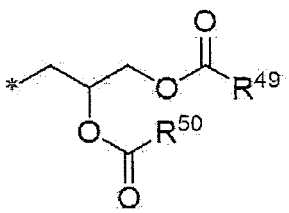

(20)

(in the formula (20), * is a bonding position, and
$R^{49}$ and $R^{50}$ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in $R^{49}$ is optionally replaced by at least one bond selected from the group consisting of an ester bond, an amide bond, a carbamate bond, and a carbonate bond, at least one ethylene group or at least one trimethylene group in $R^{50}$ is optionally replaced by at least one bond selected from the group consisting of an ester bond, an amide bond, a carbamate bond, and a carbonate bond, and $R^{49}$ and $R^{30}$ are each independently optionally substituted by a substituent selected from the group consisting of a halogen atom, a hydroxy group, and a hydrocarbon ring group having 3 to 12 carbon atoms),

(iia-1-2) a monovalent group represented by the formula (21):

[Chem. 38]

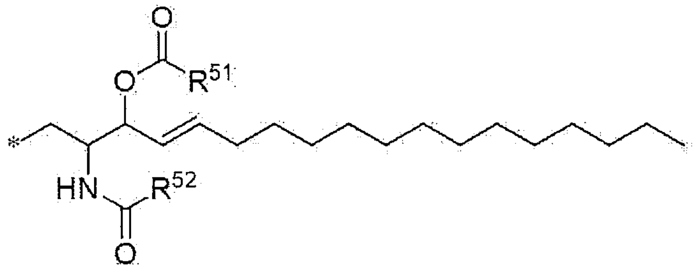

(21)

(in the formula (21), * is a bonding position, and

$R^{51}$ and $R^{52}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{51}$ is optionally replaced by at least one bond selected from the group consisting of an ester bond, an amide bond, a carbamate bond, and a carbonate bond, at least one ethylene group or at least one trimethylene group in $R^{52}$ is optionally replaced by at least one bond selected from the group consisting of an ester bond, an amide bond, a carbamate bond, and a carbonate bond, and $R^{51}$ and $R^{52}$ are each independently optionally substituted by a substituent selected from the group consisting of a halogen atom, a hydroxy group, and a hydrocarbon ring group having 3 to 12 carbon atoms), or

(iia-1-3) a monovalent group represented by the formula (22):

[Chem. 39]

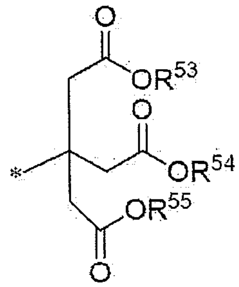

(22)

(in the formula (22), * is a bonding position, and

$R^{53}$ to $R^{55}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{53}$ is optionally replaced by at least one bond selected from the group consisting of an ester bond, an amide bond, a carbamate bond, and a carbonate bond, at least one ethylene group or at least one trimethylene group in $R^{54}$ is optionally replaced by at least one bond selected from the group consisting of an ester bond, an amide bond, a carbamate bond, and a carbonate bond, at least one ethylene group or at least one trimethylene group in $R^{55}$ is optionally replaced by at least one bond selected from the group consisting of an ester bond, an amide bond, a carbamate bond, and a carbonate bond, and $R^{53}$ to $R^{55}$ are each independently optionally substituted by a substituent selected from the group consisting of a halogen atom, a hydroxy group, and a hydrocarbon ring group having 3 to 12 carbon atoms).

In other words, group (16) is preferably a monovalent group having 50 or less carbon atoms and represented by the following formula (16-20), a monovalent group having 50 or less carbon atoms and represented by the following formula (16-21), or a monovalent group having 50 or less carbon atoms and represented by the following formula (16-22) (the definitions of the symbols in the following formulas are as mentioned above).

[Chem. 40]

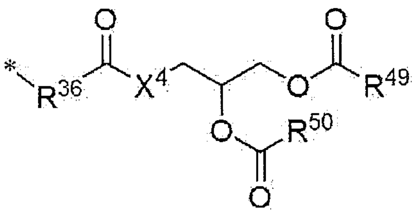

(16-20)

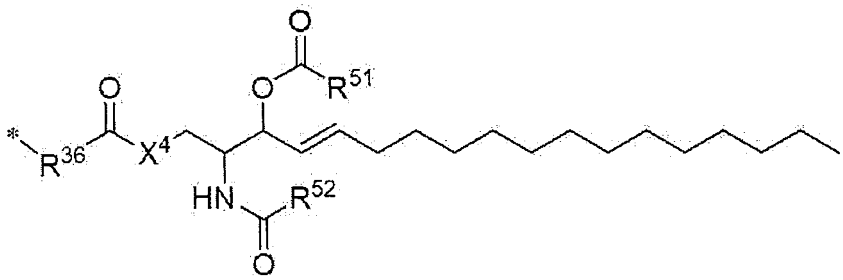

(16-21)

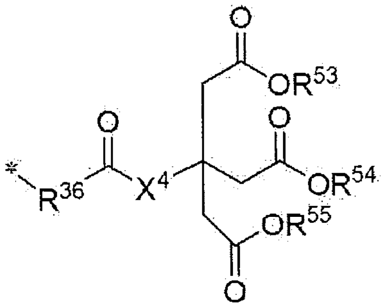

(16-22)

The explanation of $R^{36}$ and $X^4$ in the formula (16-20) to the formula (16-22) is as mentioned above.

$R^{49}$ and $R^{50}$ in the formula (20) and the formula (16-20) are preferably each independently an alkyl group having 1 to 17 carbon atoms, an alkenyl group having 2 to 17 carbon atoms, or an alkynyl group having 2 to 17 carbon atoms, more preferably each independently an alkyl group having 1 to 17 carbon atoms or an alkynyl group having 2 to 17 carbon atoms, further preferably each independently an alkyl group having 1 to 17 carbon atoms.

$R^{51}$ and $R^{52}$ in the formula (21) and the formula (16-21) are preferably each independently an alkyl group having 1 to 10 carbon atoms, an alkenyl group having 2 to 10 carbon atoms, or an alkynyl group having 2 to 10 carbon atoms, more preferably each independently an alkyl group having 1 to 10 carbon atoms.

$R^{53}$ to $R^{55}$ in the formula (22) and the formula (16-22) are preferably each independently an alkyl group having 1 to 10 carbon atoms, an alkenyl group having 2 to 10 carbon atoms, or an alkynyl group having 2 to 10 carbon atoms, more preferably an alkyl group having 1 to 10 carbon atoms.

Group (16-20) is preferably a monovalent group derived from intermediate 11, intermediate 12, intermediate 13, intermediate 14, intermediate 15, intermediate 16, intermediate 26, intermediate 27, intermediate 28, intermediate 29, or intermediate 59, produced in the below-mentioned Examples.

Group (16-21) is preferably a monovalent group derived from intermediate 23 produced in the below-mentioned Example.

Group (16-22) is preferably a monovalent group derived from intermediate 24 or intermediate 25 produced in the below-mentioned Examples.

$R^{38}$ in the formula (17) is preferably an alkylene group having 2 to 10 carbon atoms, an alkenediyl group having 2 to 10 carbon atoms, or an alkynediyl group having 2 to 10 carbon atoms, more preferably an alkylene group having 2 to 10 carbon atoms, further preferably an alkylene group having 2 or 3 carbon atoms.

$R^{39}$ in the formula (17) is preferably

(iia-2-1) a monovalent group represented by the formula (23):

[Chem. 41]

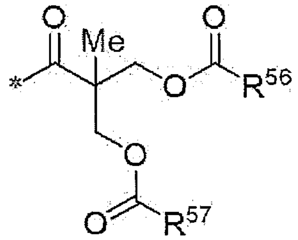

(23)

(in the formula (23), * is a bonding position,

Me is a methyl group, and
$R^{56}$ and $R^{57}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{56}$ is optionally replaced by at least one bond selected from the group consisting of an ester bond, an amide bond, a carbamate bond, and a carbonate bond, at least one ethylene group or at least one trimethylene group in $R^{57}$ is optionally replaced by at least one bond selected from the group consisting of an ester bond, an amide bond, a carbamate bond, and a carbonate bond, and $R^{56}$ and $R^{57}$ are each independently optionally substituted by a substituent selected from the group consisting of a halogen atom, a hydroxy group, and a hydrocarbon ring group having 3 to 12 carbon atoms), or

(iia-2-2) a monovalent group represented by the formula (24):

[Chem. 42]

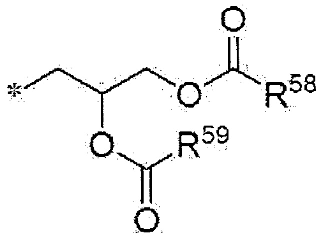

(24)

(in the formula (24), * is a bonding position, and
$R^{58}$ and $R^{59}$ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in $R^{58}$ is optionally replaced by at least one bond selected from the group consisting of an ester bond, an amide bond, a carbamate bond, and a carbonate bond, at least one ethylene group or at least one trimethylene group in $R^{59}$ is optionally replaced by at least one bond selected from the group consisting of an ester bond, an amide bond, a carbamate bond, and a carbonate bond, and $R^{58}$ and $R^{59}$ are each independently optionally substituted by a substituent selected from the group consisting of a halogen atom, a hydroxy group, and a hydrocarbon ring group having 3 to 12 carbon atoms).

In other words, group (17) is preferably a monovalent group having 50 or less carbon atoms and represented by the following formula (17-23), or a monovalent group having 1 to 50 carbon atoms and represented by the following formula (17-24) (the definitions of the symbols in the following formulas are as mentioned above).

[Chem. 43]

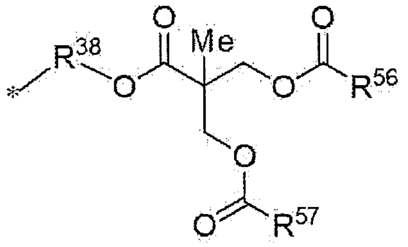

(17-23)

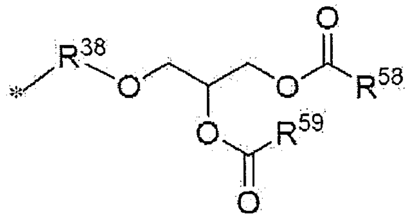

(17-24)

The explanation of $R^{38}$ in the formula (17-23) and the formula (17-24) is as mentioned above.

Me in the formula (17-23) is, as mentioned above, a methyl group.

$R^{56}$ and $R^{57}$ in the formula (23) and the formula (17-23) are preferably each independently an alkyl group having 1 to 10 carbon atoms, an alkenyl group having 2 to 10 carbon atoms, or an alkynyl group having 2 to 10 carbon atoms, more preferably each independently an alkyl group having 1 to 10 carbon atoms.

$R^{58}$ and $R^{59}$ in the formula (24) and the formula (17-24) are preferably each independently an alkyl group having 1 to 17 carbon atoms, an alkenyl group having 2 to 17 carbon atoms, or an alkynyl group having 2 to 17 carbon atoms, more preferably each independently an alkyl group having 1 to 17 carbon atoms, further preferably each independently an alkyl group having 1 to 10 carbon atoms.

Group (17-23) is preferably a monovalent group derived from intermediate 32 or intermediate 33 produced in the below-mentioned Examples.

Group (17-24) is preferably a monovalent group derived from intermediate 34 or intermediate 35 produced in the below-mentioned Examples.

$R^{40}$ and $R^{41}$ in the formula (18) are, as mentioned above, each independently an alkylene group having 3 to 10 carbon atoms.

$R^{42}$ to $R^{44}$ in the formula (18) are preferably each independently an alkyl group having 1 to 10 carbon atoms, an alkenyl group having 2 to 10 carbon atoms, or an alkynyl group having 2 to 10 carbon atoms, more preferably an alkyl group having 1 to 10 carbon atoms.

Group (18) is preferably a monovalent group derived from intermediate 36 produced in the below-mentioned Example.

$R^{45}$ in the formula (19) is, as mentioned above, an alkylene group having 5 to 10 carbon atoms.

$R^{46}$ to $R^{48}$ in the formula (19) are preferably each independently an alkyl group having 1 to 10 carbon atoms, an alkenyl group having 2 to 10 carbon atoms, or an alkynyl group having 2 to 10 carbon atoms, more preferably each independently an alkyl group having 1 to 10 carbon atoms.

Group (19) is preferably a monovalent group derived from intermediate 17 or intermediate 18 produced in the below-mentioned Examples.

$R^4$ in the formula (2) is, as mentioned above, an alkylene group having 1 to 10 carbon atoms.

$X^1$ in the formula (2) is preferably *-NH-CO-O-** (wherein * is a bonding position with $R^4$ in the formula (2), and ** is a bonding position with $R^5$ in the formula (2)), or *-O-CO-O-* (wherein * is a bonding position), more preferably *-NH-CO-O-** (wherein * is a bonding position with $R^4$ in the formula (2), and ** is a bonding position with $R^5$ in the formula (2)).

$R^5$ in the formula (2) is preferably an alkyl group having 1 to 25 carbon atoms.

Group (2) is preferably a monovalent group derived from intermediate 40 or intermediate 41 produced in the below-mentioned Examples.

$R^6$ in the formula (3) is, as mentioned above, an alkylene group having 1 to 10 carbon atoms.

$R^7$ in the formula (3) is preferably an alkyl group having 1 to 25 carbon atoms substituted by at least one fluorine atom.

Group (3) is preferably a monovalent group derived from intermediate 39 produced in the below-mentioned Examples.

$R^8$ and $R^9$ in the formula (4) are preferably each independently an alkylene group having 1 to 10 carbon atoms.

$R^{10}$ to $R^{12}$ in the formula (4) are preferably each independently a hydrogen atom, a benzyl group, or a *-Si($R^{13}$)

$(R^{14})(R^{15})$ group (wherein * is a bonding position, $R^{13}$ is a tert-butyl group, and $R^{14}$ and $R^{15}$ are each a methyl group or a phenyl group), more preferably each independently a hydrogen atom or a *-Si $(R^{13})(R^{14})(R^{15})$ group (wherein * is a bonding position, $R^{13}$ is a tert-butyl group, and $R^{14}$ and $R^{15}$ are each a methyl group or a phenyl group), further preferably each independently a hydrogen atom or a *-Si $(R^{13})(R^{14})(R^{15})$ group (wherein * is a bonding position, $R^{13}$ is a tert-butyl group, and $R^{14}$ and $R^{15}$ are each a methyl group) .

Group (4) is preferably a monovalent group derived from intermediate 42 produced in the below-mentioned Example, or a monovalent group derived from deprotected intermediate 42 (i.e., compound in which the tert-butyldimethylsilyloxy group of intermediate 42 is replaced by a hydroxy group).

$X^2$ in the formula (5) is, as mentioned above, a nitrogen atom or group (6), preferably group (6).

When $X^2$ in the formula (5) is a nitrogen atom, $R^{16}$ in the formula (5) is preferably an alkylene group having 1 to 10 carbon atoms, an alkenediyl group having 2 to 10 carbon atoms, or an alkynediyl group having 2 to 10 carbon atoms, more preferably an alkylene group having 1 to 10 carbon atoms.

When $X^2$ in the formula (5) is group (6), $R^{16}$ in the formula (5) is preferably an alkylene group having 1 to 10 carbon atoms.

When $X^2$ in the formula (5) is a nitrogen atom or group (6), $R^{17}$ and $R^{18}$ in the formula (5) are preferably each independently an alkyl group having 1 to 10 carbon atoms, and $R^{17}$ and $R^{18}$ are each independently optionally substituted by a hydroxy group. $R^{17}$ and $R^{18}$ are more preferably each independently an alkyl group having 1 to 10 carbon atoms.

Group (5) is preferably a monovalent group derived from intermediate 37, intermediate 38, intermediate 43, intermediate 44, or intermediate 47, produced in the below-mentioned Examples.

$R^{19}$ in the formula (7) is

preferably a hydrogen atom, a benzyl group, or a *-Si$(R^{13})(R^{14})(R^{15})$ group (wherein * is a bonding position, $R^{13}$ is a tert-butyl group, and $R^{14}$ and $R^{15}$ are each a methyl group or a phenyl group), or a *-CO-$R^{20}$ group (wherein * is a bonding position, and $R^{20}$ is an alkyl group having 1 to 9 carbon atoms),
more preferably a hydrogen atom, a *-Si $(R^{13})(R^{14})(R^{15})$ group (wherein * is a bonding position, $R^{13}$ is a tert-butyl group, and $R^{14}$ and $R^{15}$ are each a methyl group or a phenyl group), or a *-COR$^{20}$ group (wherein * is a bonding position, and $R^{20}$ is an alkyl group having 1 to 9 carbon atoms),
further preferably a hydrogen atom, a *-Si $(R^{13})(R^{14})(R^{15})$ group (wherein * is a bonding position, $R^{13}$ is a tert-butyl group, and $R^{14}$ and $R^{15}$ are each a methyl group), or a *-CO-$R^{20}$ group (wherein * is a bonding position, and $R^{20}$ is an alkyl group having 1 to 9 carbon atoms),
more preferably a *-CO-$R^{20}$ group (wherein * is a bonding position, and $R^{20}$ is an alkyl group having 1 to 9 carbon atoms).

Group (7) is preferably a monovalent group derived from intermediate 50, deprotected intermediate 50 (i.e., compound in which the tert-butyldimethylsilyloxy group of intermediate 50 is replaced by a hydroxy group), intermediate 51, or intermediate 52, produced in the below-mentioned Examples.

$R^{21}$ and $R^{22}$ in the formula (8) are preferably each independently a hydrogen atom, a benzyl group, or a *-Si$(R^{13})(R^{14})(R^{15})$ group (wherein * is a bonding position, $R^{13}$ is a tert-butyl group, and $R^{14}$ and $R^{15}$ are each a methyl group or a phenyl group), more preferably each independently a hydrogen atom or a *-Si $(R^{13})(R^{14})(R^{15})$ group (wherein * is a bonding position, $R^{13}$ is a tert-butyl group, and $R^{14}$ and $R^{15}$ are each a methyl group or a phenyl group), further preferably each independently a hydrogen atom or a *-Si $(R^{13})(R^{14})(R^{15})$ group (wherein * is a bonding position, $R^{13}$ is a tert-butyl group, and $R^{14}$ and $R^{15}$ are each a methyl group).

Group (8) is preferably a monovalent group derived from intermediate 48 or deprotected intermediate 48 (i.e., a compound in which the tert-butyldimethylsilyloxy group of intermediate 48 is replaced by a hydroxy group), produced in the below-mentioned Examples.

$R^{23}$ in the formula (9) is

preferably a hydrogen atom, a benzyl group, or a *-Si$(R^{13})(R^{14})(R^{15})$ group (wherein * is a bonding position, $R^{13}$ is a tert-butyl group, and $R^{14}$ and $R^{15}$ are each a methyl group or a phenyl group),
more preferably a hydrogen atom or *-Si $(R^{13})(R^{14})(R^{15})$ group (wherein * is a bonding position, $R^{13}$ is a tert-butyl group, and $R^{14}$ and $R^{15}$ are each a methyl group or a phenyl group),
further preferably a hydrogen atom or a *-Si$(R^{13})(R^{14})(R^{15})$ group (wherein * is a bonding position, $R^{13}$ is a tert-butyl group, and $R^{14}$ and $R^{15}$ are each a methyl group).

Group (9) is preferably a monovalent group derived from intermediate 45 or deprotected intermediate 45 (i.e., a compound in which the tert-butyldimethylsilyloxy group of intermediate 45 is replaced by a hydroxy group), produced in the below-mentioned Example.

$R^{24}$ in the formula (10) is preferably an alkylene group having 1 to 10 carbon atoms.

$R^{25}$ in the formula (10) is preferably an alkyl group having 1 to 30 carbon atoms.

Group (10) is preferably a monovalent group derived from intermediate 49 produced in the below-mentioned Example.

$R^{26}$ in the formula (11) is preferably an alkylene group having 1 to 10 carbon atoms.

$R^{27}$ and $R^{28}$ in the formula (11) are preferably each independently an alkyl group having 2 to 10 carbon atoms or an alkenyl group having 2 to 10 carbon atoms, more preferably each independently an alkenyl group having 2 to 10 carbon atoms.

Group (11) is preferably a monovalent group derived from intermediate 54 produced in the below-mentioned Examples.

$R^{29}$ in the formula (12) is, as mentioned above, an alkylene group having 1 to 10 carbon atoms.

$R^{30}$ and $R^{31}$ in the formula (12) are each independently an alkyl group having 1 to 10 carbon atoms.

Group (12) is preferably a monovalent group derived from intermediate 30 or intermediate 31 produced in the below-mentioned Examples.

Group (xiiib), which is one of the options for $R^{3b}$ in formula (1), is preferably an alkyl group having 1 to 30 carbon atoms or an alkenyl group having 2 to 20 carbon atoms, in which one ethylene group in the aforementioned alkyl group is optionally replaced by one ester bond. In other words, group (xiiib) is preferably an alkyl group having 1 to 30 carbon atoms in which one ethylene group is optionally replaced by one ester bond, or an alkenyl group having 2 to 20 carbon atoms.

In one embodiment of the present invention, group (xiiib) is more preferably a monovalent group derived from intermediate 53 produced in the below-mentioned Example, or oleic acid, further preferably a monovalent group derived from oleic acid. In the present specification, the "monovalent group derived from oleic acid" means a monovalent group having a structure obtained by removing a carboxy group from oleic acid (i.e., (Z)-8-heptadecenyl group).

In one embodiment of the present invention, group (xiiib) is more preferably an alkyl group having 1 to 30 carbon atoms or an alkenyl group having 2 to 20 carbon atoms, further preferably an alkenyl group having 2 to 20 carbon atoms (particularly, a monovalent group derived from oleic acid).

Group (xivb), which is one of the options for $R^{3b}$ in formula (1), is as mentioned above, $R^{3c}$-Co-$(CH_2)_p$- group (wherein $R^{3c}$ is a residue of a liposoluble vitamin having a hydroxy group or a residue of a sterol derivative having a hydroxy group, and p is an integer of 1 to 8). The p is preferably 2 or 3. The liposoluble vitamin having a hydroxy group is preferably a tocopherol. The sterol derivative having a hydroxy group is preferably a cholesterol or a cholestanol, more preferably cholesterol. $R^{3c}$ is preferably a residue of a liposoluble vitamin having a hydroxy group, more preferably a residue of tocopherol.

Only one kind of cationic lipid (1) may be used, or two or more kinds thereof may be used in combination.

In one embodiment of the present invention, preferred cationic lipid (1) includes compound 1 to compound 194, more preferably compound 1 to compound 158. In the present specification, "compound 1 to compound 194" and "compound 1 to compound 158" are cationic lipids described in the below-mentioned Examples, and "compound 1 to compound 194" and "compound 1 to compound 158" also include compound 118a, compound 119a, compound 120a, compound 127a, compound 128a, compound 129a, compound 133a, compound 134a, compound 135a, compound 139a, compound 140a, and compound 141a.

In one embodiment of the present invention, cationic lipid (1) is

preferably compound 1, compound 2, compound 3, compound 5, compound 7, compound 9, compound 10, compound 11, compound 12, compound 13, compound 14, compound 16, compound 17, compound 18, compound 19, compound 21, compound 22, compound 23, compound 24, compound 25, compound 26, compound 27, compound 28, compound 29, compound 30, compound 31, compound 32, compound 33, compound 34, compound 35, compound 36, compound 38, compound 40, or compound 45,

more preferably compound 5, compound 7, compound 9, compound 10, compound 11, compound 12, compound 13, compound 14, compound 16, compound 17, compound 18, compound 19, compound 21, compound 22, compound 23, compound 24, compound 25, compound 26, compound 27, compound 28, compound 29, compound 30, compound 31, compound 32, compound 33, compound 34, compound 35, compound 36, compound 38, or compound 45.

In one embodiment of the present invention, cationic lipid (1) is

preferably compound 1, compound 2, compound 3, compound 5, compound 6, compound 7, compound 9, compound 10, compound 11, compound 12, compound 14, compound 17, compound 18, compound 19, compound 21, compound 22, compound 23, compound 25, compound 26, compound 27, compound 28, compound 29, compound 30, compound 31, compound 32, compound 33, compound 34, compound 35, compound 36, compound 41, compound 42, compound 44, compound 94, compound 112, compound 124, compound 127a, compound 127, compound 145, compound 151, compound 152, compound 155, compound 160, compound 161, compound 162,

compound 163, compound 164, compound 168, compound 170, or compound 194,

more preferably compound 1, compound 2, compound 5, compound 7, compound 9, compound 10, compound 12, compound 14, compound 17, compound 18, compound 21, compound 22, compound 27, compound 28, compound 30, compound 31, compound 32, compound 33, compound 35, compound 94, compound 112, compound 124, compound 127a, compound 127, compound 145, compound 155, compound 160, compound 161, compound 162, compound 163, compound 164, compound 168, compound 170, or compound 194.

In one embodiment of the present invention, cationic lipid (1) is

preferably compound 1, compound 2, compound 3, compound 5, compound 6, compound 7, compound 9, compound 10, compound 11, compound 12, compound 14, compound 17, compound 18, compound 19, compound 21, compound 22, compound 23, compound 25, compound 26, compound 27, compound 28, compound 29, compound 30, compound 31, compound 32, compound 33, compound 34, compound 35, compound 36, compound 41, compound 42, compound 44, compound 112, compound 124, compound 151, compound 152, or compound 155,

more preferably compound 1, compound 2, compound 5, compound 7, compound 9, compound 10, compound 12, compound 14, compound 17, compound 18, compound 21, compound 22, compound 27, compound 28, compound 30, compound 31, compound 32, compound 33, compound 35, compound 112, compound 124, or compound 155.

In one embodiment of the present invention, cationic lipid (1) is preferably compound 58, compound 166, compound 173, compound 175, compound 181, compound 182, or compound 188.

In one embodiment of the present invention, cationic lipid (1) is preferably a cationic lipid in which $R^{3a}$ is group (14) (hereinafter sometimes to be abbreviated as to be abbreviated as "cationic lipid (1-14)"). Preferred examples of cationic lipid (1-14) include the following cationic lipids.

[Cationic lipid (1-14a)]

Cationic lipid (1-14), wherein

$R^{1a}$ and $R^{1b}$ are each independently an alkylene group having 1 to 3 carbon atoms,

$X^a$ and $X^b$ are each independently a cyclic alkylene tertiary amino group having 2 to 5 carbon atoms and 1 or 2 tertiary amino groups,

$R^{2a}$ and $R^{2b}$ are each independently an alkylene group having 1 to 8 carbon atoms,

$Y^a$ and $Y^b$ are each independently an ester bond or an amide bond,

$Z^a$ and $Z^b$ are each independently a divalent group represented by the formula (13):

[Chem. 44]

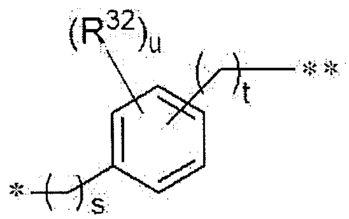

(13)

(in the formula (13), * is a bonding position with O in the formula (1),

** is a bonding position with $Y^a$ or $Y^b$ in the formula (1),

s is an integer of 0 to 3,

t is an integer of 0 to 3,

u is an integer of 0 to 4,

$R^{32}$ in the number of u are each independently a halogen atom, an alkyl group having 1 to 4 carbon atoms, or an alkoxy group having 1 to 4 carbon atoms),

$R^{3a}$ is a monovalent group having 10 to 50 carbon atoms and represented by the formula (14):

$$*\text{-}R^{33}\text{-CO-}X^3\text{-}R^{34} \qquad (14)$$

(in the formula (14), * is a bonding position,

$R^{33}$ is an alkylene group having 2 to 10 carbon atoms, an alkenediyl group having 2 to 10 carbon atoms, or an alkynediyl group having 2 to 10 carbon atoms,
$R^{34}$ is an alkyl group having 1 to 40 carbon atoms, an alkenyl group having 2 to 40 carbon atoms, or an alkynyl group having 2 to 40 carbon atoms, and
$X^3$ is an oxygen atom), and
$R^{3b}$ is

(A) a monovalent group having 10 to 50 carbon atoms and represented by the aforementioned formula (14) (the definitions of the symbols in the formula (14) are as mentioned above),
(B) an alkyl group having 1 to 30 carbon atoms, or
(C) an alkenyl group having 2 to 20 carbon atoms.

[Cationic lipid (1-14b)]

Cationic lipid (1-14), wherein

$R^{1a}$ and $R^{1b}$ are each an alkylene group having 1 to 3 carbon atoms,
$X^a$ and $X^b$ are each independently group (26) or group (27),
$R^{2a}$ and $R^{2b}$ are each independently an alkylene group having 1 to 4 carbon atoms,
$Y^a$ and $Y^b$ are each an ester bond,
$Z^a$ and $Z^b$ are each independently a divalent group represented by the formula (13):

[Chem. 45]

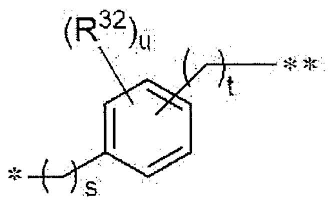

(13)

(in the formula (13), * is a bonding position with O in the formula (1),
** is a bonding position with $Y^a$ or $Y^b$ in the formula (1),
s is 0 or 1,
t is an integer of 0 to 2,
u is an integer of 0 to 2, and
$R^{32}$ in the number of u are each independently a halogen atom or an alkoxy group having 1 to 4 carbon atoms),
$R^{3a}$ is a monovalent group having 10 to 50 carbon atoms and represented by the formula (14):

$$*\text{-}R^{33}\text{-CO-}X^3\text{-}R^{34} \qquad (14)$$

(in the formula (14), * is a bonding position,
$R^{33}$ is an alkylene group having 2 to 8 carbon atoms or an alkenediyl group having 2 to 8 carbon atoms,
$R^{34}$ is an alkyl group having 1 to 20 carbon atoms, an alkenyl group having 2 to 20 carbon atoms, or an alkynyl group having 2 to 20 carbon atoms, and
$X^3$ is an oxygen atom), and
$R^{3b}$ is

(A) a monovalent group having 10 to 50 carbon atoms and represented by the aforementioned formula (14) (the definitions of the symbols in the formula (14) are as mentioned above), or
(B) an alkenyl group having 2 to 20 carbon atoms.

[Cationic lipid (1-14c)]

Cationic lipid (1-14), wherein

$R^{1a}$ and $R^{1b}$ are each an ethylene group,
$X^a$ and $X^b$ are each independently group (26) (preferably a piperidinediyl group),

$R^{2a}$ and $R^{2b}$ are each an ethylene group,
$Y^a$ and $Y^b$ are each *-CO-O-** (wherein * is a bonding position with $Z^a$ or $Z^b$ in the formula (1), and ** is a bonding position with $R^{2a}$ or $R^{2b}$ in the formula (1)),
$Z^a$ and $Z^b$ are each independently a divalent group represented by the formula (13):

[Chem. 46]

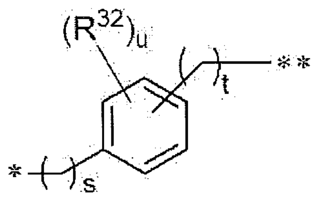

(13)

(in the formula (13), * is a bonding position with O in the formula (1),
** is a bonding position with $Y^a$ or $Y^b$ in the formula (1),
s is 0,
t is 1, and
u is 0),
$R^{3a}$ is a monovalent group having 10 to 50 carbon atoms and represented by the formula (14):

$$*\text{-}R^{33}\text{-CO-}X^{3}\text{-}R^{34} \qquad (14)$$

(in the formula (14), * is a bonding position,
$R^{33}$ is an alkylene group having 2 to 8 carbon atoms,
$R^{34}$ is preferably an alkenyl group having 2 to 20 carbon atoms or an alkynyl group having 2 to 20 carbon atoms, and
$X^3$ is an oxygen atom), and
$R^{3b}$ is

(A) a monovalent group having 10 to 50 carbon atoms and represented by the aforementioned formula (14) (the definitions of the symbols in the formula (14) are as mentioned above), or
(B) an alkenyl group having 2 to 20 carbon atoms (preferably a monovalent group derived from oleic acid).

Cationic lipid (1-14) is preferably compound 41, compound 45, or compound 160, more preferably compound 45 or compound 160.

In one embodiment of the present invention, cationic lipid (1) is preferably a cationic lipid in which $R^{3a}$ is group (15) (hereinafter abbreviated as "cationic lipid (1-15)"). Preferred examples of cationic lipid (1-15) include the following cationic lipids.

[Cationic lipid (1-15a)]

Cationic lipid (1-15), wherein

$R^{1a}$ and $R^{1b}$ are each independently an alkylene group having 1 to 3 carbon atoms,
$X^a$ and $X^b$ are each independently a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms,
$Y^a$ and $Y^b$ are each independently an ester bond or an amide bond,
$Z^a$ and $Z^b$ are each independently a divalent group represented by the formula (13):

[Chem. 47]

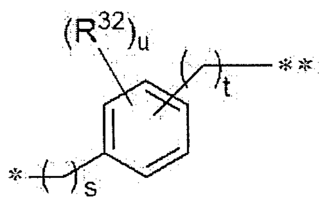

(13)

(in the formula (13), * is a bonding position with O in the formula (1),
** is a bonding position with $Y^a$ or $Y^b$ in the formula (1),
s is an integer of 0 to 3,
t is an integer of 0 to 3,
u is an integer of 0 to 4, and
$R^{32}$ in the number of u are each independently a halogen atom, an alkyl group having 1 to 4 carbon atoms, or an alkoxy group having 1 to 4 carbon atoms),
$R^{3a}$ is a monovalent group having 50 or less carbon atoms and represented by the formula (15):

[Chem. 48]

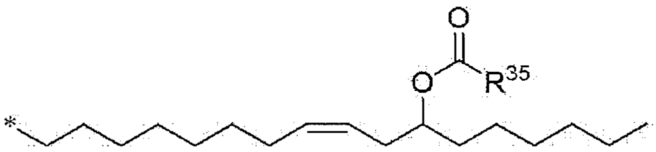

(15)

(in the formula (15), * is a bonding position, and
$R^{35}$ is an alkyl group having 1 to 20 carbon atoms, an alkenyl group having 2 to 20 carbon atoms, an alkynyl group having 2 to 20 carbon atoms, or a hydrocarbon ring group having 3 to 12 carbon atoms, the aforementioned alkyl group is optionally substituted by a hydrocarbon ring group having 3 to 12 carbon atoms), and
$R^{3b}$ is

(A) a monovalent having 10 to 50 carbon atoms and represented by the formula (14):

$$*-R^{33}-CO-X^3-R^{34} \qquad (14)$$

(in the formula (14), * is a bonding position,

$R^{33}$ is an alkylene group having 2 to 10 carbon atoms, an alkenediyl group having 2 to 10 carbon atoms, or an alkynediyl group having 2 to 10 carbon atoms,
$R^{34}$ is an alkyl group having 1 to 40 carbon atoms, an alkenyl group having 2 to 40 carbon atoms, or an alkynyl group having 2 to 40 carbon atoms, and
$X^3$ is an oxygen atom),

(B) a monovalent group having 50 or less carbon atoms and represented by the aforementioned formula (15) (the definitions of the symbols in the formula (15) are as mentioned above),
(C) a monovalent group having 50 or less carbon atoms and represented by the formula (16-20):

[Chem. 49]

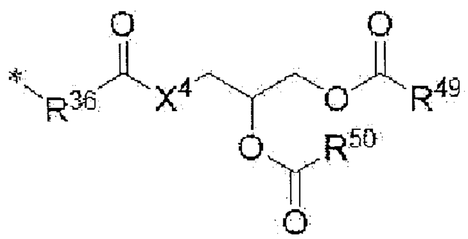

(16-20)

(in the formula (16), * is a bonding position,

$R^{36}$ is an alkylene group having 2 to 9 carbon atoms, an alkenediyl group having 2 to 9 carbon atoms, or an alkynediyl group having 2 to 9 carbon atoms,
$X^4$ is an oxygen atom or NH (preferably an oxygen atom), and
$R^{49}$ and $R^{50}$ are each independently an alkyl group having 1 to 17 carbon atoms, an alkenyl group having 2 to 17 carbon atoms, or an alkynyl group having 2 to 17 carbon atoms),

(D) an alkyl group having 1 to 30 carbon atoms in which one ethylene group is optionally replaced by one ester

bond, or
(E) an alkenyl group having 2 to 20 carbon atoms.

[Cationic lipid (1-15b)]

Cationic lipid (1-15), wherein

$R^{1a}$ and $R^{1b}$ are each an alkylene group having 1 to 3 carbon atoms,
$X^a$ and $X^b$ are each independently group (26) or group (27),
$R^{2a}$ and $R^{2b}$ are each independently an alkylene group having 1 to 4 carbon atoms,
$Y^a$ and $Y^b$ are each an ester bond,
$Z^a$ and $Z^b$ are each independently a divalent group represented by the formula (13):

[Chem. 50]

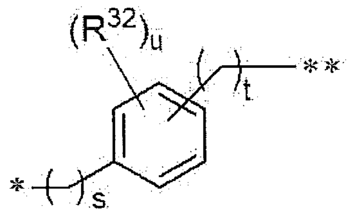

(13)

(in the formula (13), * is a bonding position with O in the formula (1),
** is a bonding position with $Y^a$ or $Y^b$ in the formula (1),
s is 0 or 1,
t is an integer of 0 to 2,
u is an integer of 0 to 2, and
$R^{32}$ in the number of u are each independently a halogen atom or an alkoxy group having 1 to 4 carbon atoms),
$R^{3a}$ is a monovalent group having 50 or less carbon atoms and represented by the formula (15):

[Chem. 51]

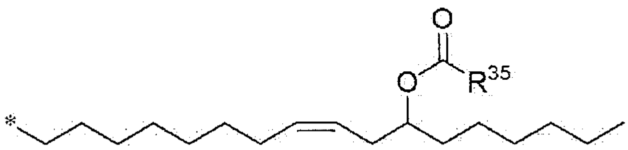

(15)

(in the formula (15), * is a bonding position, and
$R^{35}$ is an alkyl group having 1 to 20 carbon atoms, an alkenyl group having 2 to 20 carbon atoms, or an alkynyl group having 2 to 20 carbon atoms, and the aforementioned alkyl group is optionally substituted by a cyclohexyl group), and
$R^{3b}$ is

(A) a monovalent group having 50 or less carbon atoms and represented by the aforementioned formula (15) (the definitions of the symbols in the formula (15) are as mentioned above),
(B) a monovalent group having 50 or less carbon atoms and represented by the formula (16-20):

[Chem. 52]

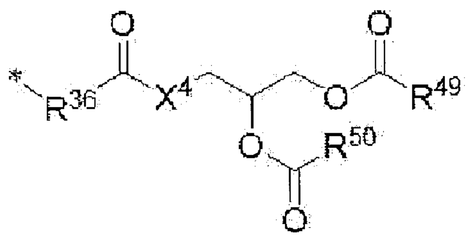

(16-20)

(in the formula (16), * is a bonding position,

$R^{36}$ is an alkylene group having 2 to 9 carbon atoms,
$X^4$ is an oxygen atom, and,
$R^{49}$ and $R^{50}$ are each independently an alkyl group having 1 to 17 carbon atoms or an alkynyl group having 2 to 17 carbon atoms), or

(C) an alkenyl group having 2 to 20 carbon atoms.

[Cationic lipid (1-15c)]

Cationic lipid (1-15), wherein

$R^{1a}$ and $R^{1b}$ are each an ethylene group,
$X^a$ and $X^b$ are each independently group (26) (preferably a piperidinediyl group),
$R^{2a}$ and $R^{2b}$ are each an ethylene group,
$Y^a$ and $Y^b$ are each *-CO-O-** (wherein * is a bonding position with $Z^a$ or $Z^b$ in the formula (1), and ** is a bonding position with $R^{2a}$ or $R^{2b}$ in the formula (1)),
$Z^a$ and $Z^b$ are each independently a divalent group represented by the formula (13):

[Chem. 53]

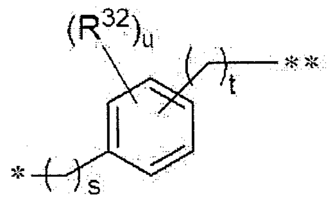

(13)

(in the formula (13), * is a bonding position with O in the formula (1),
** is a bonding position with $Y^a$ or $Y^b$ in the formula (1),
s is 0,
t is 1, and
u is 0),
$R^{3a}$ is a monovalent group having 50 or less carbon atoms and represented by the formula (15):

[Chem. 54]

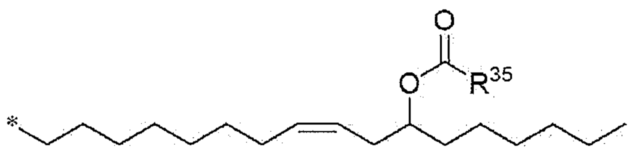

(15)

(in the formula (15), * is a bonding position, and
$R^{35}$ is an alkyl group having 1 to 20 carbon atoms, an alkenyl group having 2 to 20 carbon atoms, or an alkynyl group having 2 to 20 carbon atoms), and
$R^{3b}$ is

(A) a monovalent group having 50 or less carbon atoms and represented by the aforementioned formula (15) (the definitions of the symbols in the formula (15) are as mentioned above),
(B) a monovalent group having 50 or less carbon atoms and represented by the formula (16-20):

[Chem. 55]

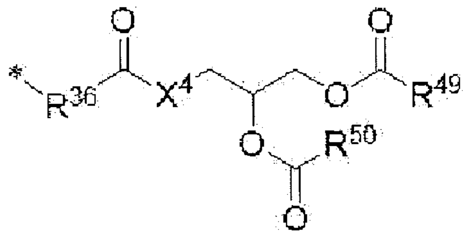

(16-20)

(in the formula (16), * is a bonding position,

$R^{36}$ is an alkylene group having 2 or 3 carbon atoms,
$X^4$ is an oxygen atom, and
$R^{49}$ and $R^{50}$ are each independently an alkyl group having 1 to 17 carbon atoms), or

(C) an alkenyl group having 2 to 20 carbon atoms (preferably a monovalent group derived from oleic acid).

Cationic lipid (1-15) is

preferably compound 1, compound 2, compound 3, compound 9, compound 10, compound 11, compound 12, compound 16, compound 17, compound 18, compound 19, compound 21, compound 22, compound 23, compound 24, compound 26, compound 27, compound 30, compound 31, compound 33, compound 34, compound 38, compound 42, compound 44, compound 151, compound 152, compound 161, compound 162, compound 163, compound 164, compound 166, compound 168, compound 170, compound 173, compound 175, compound 181, or compound 182,
more preferably compound 1, compound 2, compound 9, compound 10, compound 11, compound 12, compound 16, compound 17, compound 18, compound 19, compound 21, compound 22, compound 23, compound 24, compound 26, compound 27, compound 30, compound 31, compound 33, compound 34, compound 38, compound 161, compound 162, compound 163, compound 164, compound 166, compound 168, compound 170, compound 173, compound 175, compound 181, or compound 182,
further preferably compound 1, compound 2, compound 9, compound 10, compound 11, compound 12, compound 16, compound 17, compound 18, compound 19, compound 21, compound 22, compound 23, compound 24, compound 26, compound 27, compound 30, compound 31, compound 33, compound 34, or compound 38.

In one embodiment of the present invention, cationic lipid (1) is preferably a cationic lipid in which $R^{3a}$ is group (16-20) (hereinafter to be abbreviated as "cationic lipid (1-16-20)"). Preferred examples of cationic lipid (1-16-20) include the following cationic lipids.

[Cationic lipid (1-16-20a)]

Cationic lipid (1-16-20), wherein

$R^{1a}$ and $R^{1b}$ are each independently an alkylene group having 1 to 3 carbon atoms,
$X^a$ and $X^b$ are each independently a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms,
$Y^a$ and $Y^b$ are each independently an ester bond or an amide bond,
$Z^a$ and $Z^b$ are each independently a divalent group represented by the formula (13):

[Chem. 56]

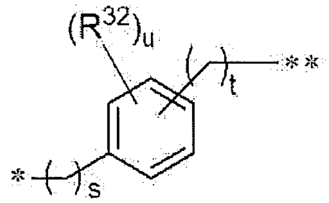

(13)

(in the formula (13), * is a bonding position with O in the formula (1),
** is a bonding position with $Y^a$ or $Y^b$ in the formula (1),
s is an integer of 0 to 3,
t is an integer of 0 to 3,
u is an integer of 0 to 4, and
$R^{32}$ in the number of u are each independently a halogen atom, an alkyl group having 1 to 4 carbon atoms, or an alkoxy group having 1 to 4 carbon atoms),
$R^{3a}$ is a monovalent group having 50 or less carbon atoms and represented by the formula (16-20):

[Chem. 57]

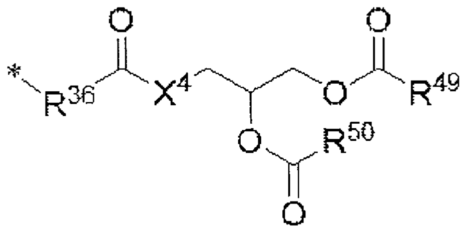

(16-20)

(in the formula (16), * is a bonding position,
$R^{36}$ is an alkylene group having 2 to 9 carbon atoms, an alkenediyl group having 2 to 9 carbon atoms, or an alkynediyl group having 2 to 9 carbon atoms,
$X^4$ is an oxygen atom or NH (preferably an oxygen atom), and
$R^{49}$ and $R^{50}$ are each independently an alkyl group having 1 to 17 carbon atoms, an alkenyl group having 2 to 17 carbon atoms, or an alkynyl group having 2 to 17 carbon atoms), and
$R^{3b}$ is

(A) a monovalent group having 50 or less carbon atoms and represented by the aforementioned formula (16-20) (the definitions of the symbols in the formula (16-20) are as mentioned above),
(B) an alkyl group having 1 to 30 carbon atoms in which one ethylene group is optionally replaced by one ester bond,
(C) an alkenyl group having 2 to 20 carbon atoms, or
(D) an $R^{3c}$-CO-$(CH_2)_p$- group (wherein $R^{3c}$ is a residue of a liposoluble vitamin having a hydroxy group or a residue of a sterol derivative having a hydroxy group, and p is an integer of 1 to 8).

[Cationic lipid (1-16-20b)]

Cationic lipid (1-16-20), wherein

$R^{1a}$ and $R^{1b}$ are each an alkylene group having 1 to 3 carbon atoms,
$X^a$ and $X^b$ are each independently group (26) or group (27),
$R^{2a}$ and $R^{2b}$ are each independently an alkylene group having 1 to 4 carbon atoms,
$Y^a$ and $Y^b$ are each an ester bond,
$Z^a$ and $Z^b$ are each independently a divalent group represented by the formula (13):

[Chem. 58]

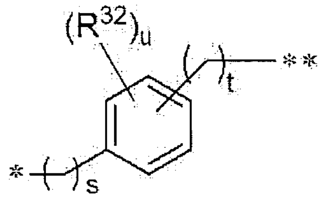

(13)

(in the formula (13), * is a bonding position with O in the formula (1), ** is a bonding position with $Y^a$ or $Y^b$ in the formula (1), s is 0 or 1, t is an integer of 0 to 2, u is an integer of 0 to 2, and $R^{32}$ in the number of u are each independently a halogen atom or an alkoxy group having 1 to 4 carbon atoms), $R^{3a}$ is a monovalent group having 50 or less carbon atoms and represented by the formula (16-20):

[Chem. 59]

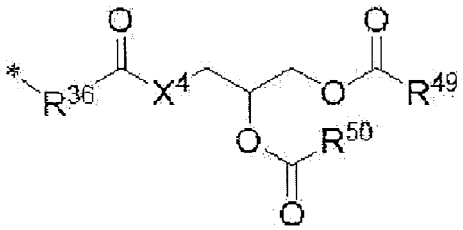

(16-20)

(in the formula (16), * is a bonding position, $R^{36}$ is an alkylene group having 2 to 9 carbon atoms, $X^4$ is an oxygen atom, and, $R^{49}$ and $R^{50}$ are each independently an alkyl group having 1 to 17 carbon atoms or an alkynyl group having 2 to 17 carbon atoms), and $R^{3b}$ is

(A) a monovalent group having 50 or less carbon atoms and represented by the aforementioned formula (16-20) (the definitions of the symbols in the formula (16-20) are as mentioned above),
(B) an alkyl group having 1 to 30 carbon atoms in which one ethylene group is optionally replaced by one ester bond (preferably a monovalent group derived from intermediate 53),
(C) an alkenyl group having 2 to 20 carbon atoms, or
(D) an $R^{3c}$-Co-$(CH_2)_p$- group (wherein $R^{3c}$ is a residue of a liposoluble vitamin having a hydroxy group, and p is an integer of 2 or 3).

[Cationic lipid (1-16-20c)]

Cationic lipid (1-16-20), wherein

$R^{1a}$ and $R^{1b}$ are each an ethylene group,
$X^a$ and $X^b$ are each independently group (26) (preferably a piperidinediyl group),
$R^{2a}$ and $R^{2b}$ are each an ethylene group,
$Y^a$ and $Y^b$ are each *-CO-O-** (wherein * is a bonding position with $Z^a$ or $Z^b$ in the formula (1), and ** is a bonding position with $R^{2a}$ or $R^{2b}$ in the formula (1)),
$Z^a$ and $Z^b$ are each independently a divalent group represented by the formula (13):

[Chem. 60]

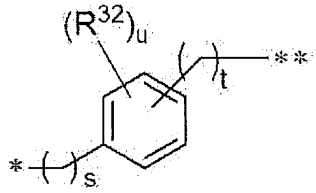

(13)

(in the formula (13), * is a bonding position with O in the formula (1),
** is a bonding position with $Y^a$ or $Y^b$ in the formula (1),
s is 0,
t is 1, and
u is 0),
$R^{3a}$ is a monovalent group having 50 or less carbon atoms and represented by the formula (16-20):

[Chem. 61]

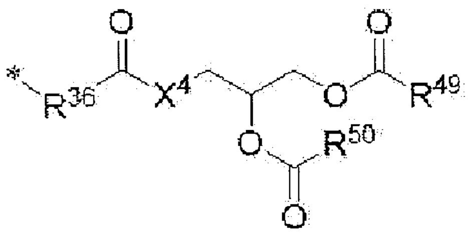

(16-20)

(in the formula (16), * is a bonding position,
$R^{36}$ is an alkylene group having 2 or 3 carbon atoms,
$X^4$ is an oxygen atom, and
$R^{49}$ and $R^{50}$ are each independently an alkyl group having 1 to 17 carbon atoms or an alkynyl group having 2 to 17 carbon atoms (preferably an alkyl group having 1 to 17 carbon atoms)), and
$R^{3b}$ is

(A) a monovalent group having 50 or less carbon atoms and represented by the aforementioned formula (16-20) (the definitions of the symbols in the formula (16-20) are as mentioned above),
(B) a monovalent group derived from intermediate 53,
(C) an alkenyl group having 2 to 20 carbon atoms (preferably a monovalent group derived from oleic acid), or
(D) an $p^{3c}$-Co-$(CH_2)_p$- group (wherein $R^{3c}$ is a residue of tocopherol, and p is 2 or 3).

Cationic lipid (1-16-20) is

preferably compound 5, compound 6, compound 7, compound 13, compound 14, compound 25, compound 28, compound 29, compound 32, compound 35, compound 36, compound 40, compound 58, or compound 155,
more preferably compound 5, compound 7, compound 13, compound 14, compound 25, compound 28, compound 29, compound 32, compound 35, compound 36, compound 40, compound 58, or compound 155.

In one embodiment of the present invention, cationic lipid (1) is preferably a cationic lipid in which $R^{3a}$ is group (19) (hereinafter abbreviated as "cationic lipid (1-19)"). Preferred examples of cationic lipid (1-19) include the following cationic lipids.

[Cationic lipid (1-19a)]

Cationic lipid (1-19), wherein

$R^{1a}$ and $R^{1b}$ are each independently an alkylene group having 1 to 3 carbon atoms,
$X^a$ and $X^b$ are each independently a cyclic alkylene tertiary amino group having 2 to 5 carbon atoms and 1 or 2 tertiary amino groups,

$R^{2a}$ and $R^{2b}$ are each independently an alkylene group having 1 to 8 carbon atoms,
$Y^a$ and $Y^b$ are each independently an ester bond or an amide bond,
$Z^a$ and $Z^b$ are each independently a divalent group represented by the formula (13):

[Chem. 62]

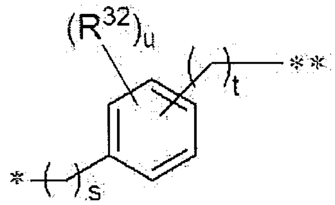

(13)

(in the formula (13), * is a bonding position with O in the formula (1),
** is a bonding position with $Y^a$ or $Y^b$ in the formula (1),
s is an integer of 0 to 3,
t is an integer of 0 to 3,
u is an integer of 0 to 4, and
$R^{32}$ in the number of u are each independently a halogen atom, an alkyl group having 1 to 4 carbon atoms, or an alkoxy group having 1 to 4 carbon atoms),
$R^{3a}$ is a monovalent group having 50 or less carbon atoms and represented by the formula (19):

[Chem. 63]

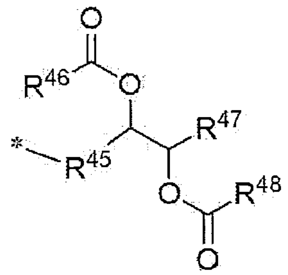

(19)

(in the formula (19), * is a bonding position,
$R^{45}$ is an alkylene group having 5 to 10 carbon atoms, and
$R^{46}$ to $R^{48}$ are each independently an alkyl group having 1 to 10 carbon atoms, an alkenyl group having 2 to 10 carbon atoms, or an alkynyl group having 2 to 10 carbon atoms), and
$R^{3b}$ is

(A) a monovalent group having 50 or less carbon atoms and represented by the aforementioned formula (19) (the definitions of the symbols in the formula (19) are as mentioned above),
(B) an alkyl group having 1 to 30 carbon atoms, or
(C) an alkenyl group having 2 to 20 carbon atoms.

[Cationic lipid (1-19b)]

Cationic lipid (1-19), wherein

$R^{1a}$ and $R^{1b}$ are each an alkylene group having 1 to 3 carbon atoms,
$X^a$ and $X^b$ are each independently group (26) or group (27),
$R^{2a}$ and $R^{2b}$ are each independently an alkylene group having 1 to 4 carbon atoms,
$Y^a$ and $Y^b$ are each an ester bond,
$Z^a$ and $Z^b$ are each independently a divalent group represented by the formula (13):

[Chem. 64]

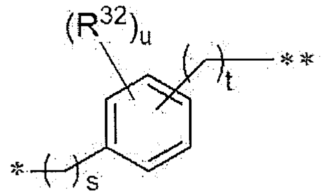

(13)

(in the formula (13), * is a bonding position with O in the formula (1),
** is a bonding position with $Y^a$ or $Y^b$ in the formula (1),
s is 0 or 1,
t is an integer of 0 to 2,
u is an integer of 0 to 2, and
$R^{32}$ in the number of u are each independently a halogen atom or an alkoxy group having 1 to 4 carbon atoms),
$R^{3a}$ is a monovalent group having 50 or less carbon atoms and represented by the formula (19):

[Chem. 65]

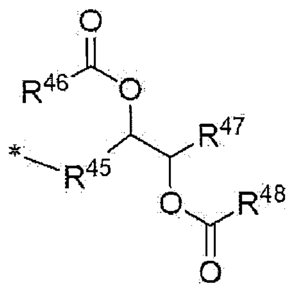

(19)

(in the formula (19), * is a bonding position,
$R^{45}$ is an alkylene group having 5 to 10 carbon atoms, and
$R^{46}$ to $R^{48}$ are each independently an alkyl group having 1 to 10 carbon atoms), and
$R^{3b}$ is

(A) a monovalent group having 50 or less carbon atoms and represented by the aforementioned formula (19) (the definitions of the symbols in the formula (19) are as mentioned above), or
(B) an alkenyl group having 2 to 20 carbon atoms.

[Cationic lipid (1-19c)]

Cationic lipid (1-19), wherein

$R^{1a}$ and $R^{1b}$ are each an ethylene group,
$X^a$ and $X^b$ are each independently group (26) (preferably a piperidinediyl group),
$R^{2a}$ and $R^{2b}$ are each an ethylene group,
$Y^a$ and $Y^b$ are each *-CO-O-** (wherein * is a bonding position with $Z^a$ or $Z^b$ in the formula (1), and ** is a bonding position with $R^{2a}$ or $R^{2b}$ in the formula (1)),
$Z^a$ and $Z^b$ are each independently a divalent group represented by the formula (13):

[Chem. 66]

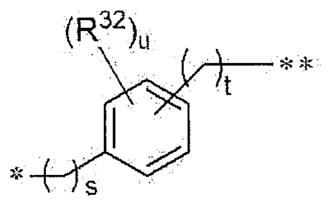

(13)

(in the formula (13), * is a bonding position with O in the formula (1),
** is a bonding position with $Y^a$ or $Y^b$ in the formula (1),
s is 0,
t is 1, and
u is 0),
$R^{3a}$ is a monovalent group having 50 or less carbon atoms and represented by the formula (19):

[Chem. 67]

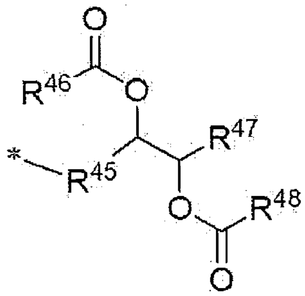

(19)

(in the formula (19), * is a bonding position,
$R^{45}$ is an alkylene group having 5 to 10 carbon atoms, and
$R^{46}$ to $R^{48}$ are each independently an alkyl group having 1 to 10 carbon atoms), and
$R^{3b}$ is

(A) a monovalent group having 50 or less carbon atoms and represented by the aforementioned formula (19) (the definitions of the symbols in the formula (19) are as mentioned above), or
(B) an alkenyl group having 2 to 20 carbon atoms (preferably a monovalent group derived from oleic acid).

Cationic lipid (1-19) is preferably compound 94 or compound 188, more preferably compound 94.
In one embodiment of the present invention, cationic lipid (1) is preferably a cationic lipid in which $R^{3a}$ is group (2) (hereinafter abbreviated as "cationic lipid (1-2)"). Preferred examples of cationic lipid (1-2) include the following cationic lipids.

[Cationic lipid (1-2a)]

Cationic lipid (1-2), wherein

$R^{1a}$ and $R^{1b}$ are each independently an alkylene group having 1 to 3 carbon atoms,
$X^a$ and $X^b$ are each independently a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms,
$Y^a$ and $Y^b$ are each independently an ester bond or an amide bond,
$Z^a$ and $Z^b$ are each independently a divalent group represented by the formula (13):

[Chem. 68]

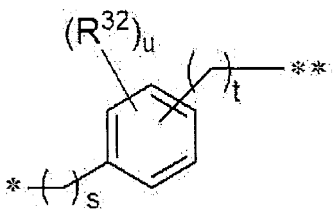

(13)

(in the formula (13), * is a bonding position with O in the formula (1),
** is a bonding position with $Y^a$ or $Y^b$ in the formula (1),
s is an integer of 0 to 3,
t is an integer of 0 to 3,
u is an integer of 0 to 4, and

$R^{32}$ in the number of u are each independently a halogen atom, an alkyl group having 1 to 4 carbon atoms, or an alkoxy group having 1 to 4 carbon atoms),
$R^{3a}$ is a monovalent group represented by the formula (2):

$$*-R^4-X^1-R^5 \qquad (2)$$

(in the formula (2), * is a bonding position,
$R^4$ is an alkylene group having 1 to 10 carbon atoms,
$X^1$ is *-NH-CO-O-** (wherein * is a bonding position with $R^4$ in the formula (2), and ** is a bonding position with $R^5$ in the formula (2)), or *-O-CO-O-* (wherein * is a bonding position), and
$R^5$ is an alkyl group having 1 to 25 carbon atoms), and
$R^{3b}$,

(A) a monovalent group represented by the aforementioned formula (2) (the definitions of the symbols in the formula (2) are as mentioned above),
(B) an alkyl group having 1 to 30 carbon atoms, or
(C) an alkenyl group having 2 to 20 carbon atoms.

[Cationic lipid (1-2b)]

Cationic lipid (1-2), wherein

$R^{1a}$ and $R^{1b}$ are each an alkylene group having 1 to 3 carbon atoms,
$X^a$ and $X^b$ are each independently group (26) or group (27),
$R^{2a}$ and $R^{2b}$ are each independently an alkylene group having 1 to 4 carbon atoms,
$Y^a$ and $Y^b$ are each an ester bond,
$Z^a$ and $Z^b$ are each independently a divalent group represented by the formula (13):

[Chem. 69]

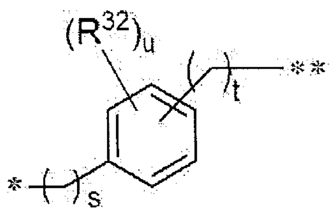

(13)

(in the formula (13), * is a bonding position with O in the formula (1),
** is a bonding position with $Y^a$ or $Y^b$ in the formula (1),
s is 0 or 1,
t is an integer of 0 to 2,
u is an integer of 0 to 2, and
$R^{32}$ in the number of u are each independently a halogen atom or an alkoxy group having 1 to 4 carbon atoms),
$R^{3a}$ is a monovalent group represented by the formula (2):

$$*-R^4-X^1-R^5 \qquad (2)$$

(in the formula (2), * is a bonding position,
$R^4$ is an alkylene group having 1 to 10 carbon atoms,
$X^1$ is *-NH-CO-O-** (wherein * is a bonding position with $R^4$ in the formula (2), and ** is a bonding position with $R^5$ in the formula (2)), and
$R^5$ is an alkyl group having 1 to 25 carbon atoms), and
$R^{3b}$ is

(A) a monovalent group represented by the aforementioned formula (2) (the definitions of the symbols in the formula (2) are as mentioned above), or
(B) an alkenyl group having 2 to 20 carbon atoms.

[Cationic lipid (1-2c)]

Cationic lipid (1-2), wherein

$R^{1a}$ and $R^{1b}$ are each an ethylene group,
$X^a$ and $X^b$ are each independently group (26) (particularly a piperidinediyl group),
$R^{2a}$ and $R^{2b}$ are each an ethylene group,
$Y^a$ and $Y^b$ are each an *-CO-O-** (wherein * is a bonding position with $Z^a$ or $Z^b$ in the formula (1), and ** is a bonding position with $R^{2a}$ or $R^{2b}$ in the formula (1)),
$Z^a$ and $Z^b$ are each independently a divalent group represented by the formula (13):

[Chem. 70]

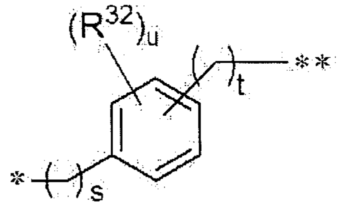

(13)

(in the formula (13), * is a bonding position with O in the formula (1),
** is a bonding position with $Y^a$ or $Y^b$ in the formula (1),
s is 0,
t is 1, and
u is 0),
$R^{3a}$ is a monovalent group represented by the formula (2):

$$*\text{-}R^4\text{-}X^1\text{-}R^5 \qquad (2)$$

(in the formula (2), * is a bonding position,
$R^4$ is an alkylene group having 1 to 10 carbon atoms,
$X^1$ is *-NH-CO-O-** (wherein * is a bonding position with $R^4$ in the formula (2), and ** is a bonding position with $R^5$ in the formula (2)), and
$R^5$ is an alkyl group having 1 to 25 carbon atoms), and
$R^{3b}$ is an alkenyl group having 2 to 20 carbon atoms (preferably a monovalent group derived from oleic acid).

Cationic lipid (1-2) is preferably compound 112.

In one embodiment of the present invention, cationic lipid (1) is preferably a cationic lipid in which $R^{3a}$ is group (5) (hereinafter abbreviated as "cationic lipid (1-5)"). Preferred examples of cationic lipid (1-5) include the following cationic lipids.

[Cationic lipid (1-5a)]

Cationic lipid (1-5), wherein

$R^{1a}$ and $R^{1b}$ are each independently an alkylene group having 1 to 3 carbon atoms,
$X^a$ and $X^b$ are each independently a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms,
$Y^a$ and $Y^b$ are each independently an ester bond or an amide bond,
$Z^a$ and $Z^b$ are each independently a divalent group represented by the formula (13):

[Chem. 71]

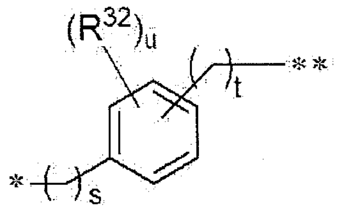

(13)

(in the formula (13), * is a bonding position with O in the formula (1),
** is a bonding position with $Y^a$ or $Y^b$ in the formula (1),
s is an integer of 0 to 3,
t is an integer of 0 to 3,
u is an integer of 0 to 4, and
$R^{32}$ in the number of u are each independently a halogen atom, an alkyl group having 1 to 4 carbon atoms, or an alkoxy group having 1 to 4 carbon atoms),
$R^{3a}$ is a monovalent group represented by the formula (5):

[Chem. 72]

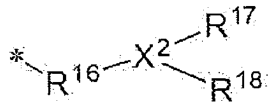

(5)

(in the formula (5), * is a bonding position,
$X^2$ is a trivalent group represented by the formula (6):

[Chem. 73]

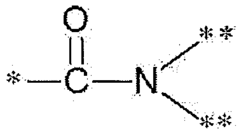

(6)

(in the formula (6), * is a bonding position with $R^{16}$, and
** is a bonding position with $R^{17}$ or $R^{18}$),
$R^{16}$ is an alkylene group having 1 to 10 carbon atoms, and
$R^{17}$ and $R^{18}$ are each independently an alkyl group having 1 to 10 carbon atoms), and
$R^{3b}$ is

(A) a monovalent group represented by the aforementioned formula (5) (the definitions of the symbols in the formula (5) are as mentioned above),
(B) an alkyl group having 1 to 30 carbon atoms, or
(C) an alkenyl group having 2 to 20 carbon atoms.

[Cationic lipid (1-5b)]

Cationic lipid (1-5), wherein

$R^{1a}$ and $R^{1b}$ are each an alkylene group having 1 to 3 carbon atoms,
$X^a$ and $X^b$ are each independently group (26) or group (27),
$R^{2a}$ and $R^{2b}$ are each independently an alkylene group having 1 to 4 carbon atoms,
$Y^a$ and $Y^b$ are each an ester bond,
$Z^a$ and $Z^b$ are each independently a divalent group represented by the formula (13):

[Chem. 74]

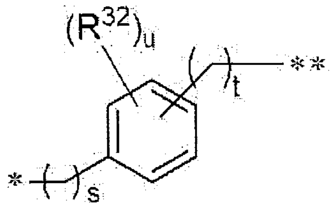

(13)

(in the formula (13), * is a bonding position with O in the formula (1),
** is a bonding position with $Y^a$ or $Y^b$ in the formula (1),
s is 0 or 1,
t is an integer of 0 to 2,
u is an integer of 0 to 2, and
$R^{32}$ in the number of u are each independently a halogen atom or an alkoxy group having 1 to 4 carbon atoms),
$R^{3a}$ is a monovalent group represented by the formula (5):

[Chem. 75]

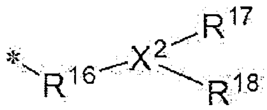

(5)

(in the formula (5), * is a bonding position,
$X^2$ is a trivalent group represented by the formula (6):

[Chem. 76]

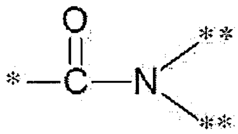

(6)

(in the formula (6), * is a bonding position with $R^{16}$, and
** is a bonding position with $R^{17}$ or $R^{18}$),
$R^{16}$ is an alkylene group having 1 to 10 carbon atoms, and
$R^{17}$ and $R^{18}$ are each independently an alkyl group having 1 to 10 carbon atoms), and
$R^{3b}$ is

(A) a monovalent group represented by the aforementioned formula (5) (the definitions of the symbols in the formula (5) are as mentioned above), or
(B) an alkenyl group having 2 to 20 carbon atoms.

[Cationic lipid (1-5c)]

Cationic lipid (1-5), wherein

$R^{1a}$ and $R^{1b}$ are each an ethylene group,
$X^a$ and $X^b$ are each independently group (26) (particularly a piperidinediyl group),
$R^{2a}$ and $R^{2b}$ are each an ethylene group,
$Y^a$ and $Y^b$ are each *-CO-O-** (wherein * is a bonding position with $Z^a$ or $Z^b$ in the formula (1), and ** is a bonding position with $R^{2a}$ or $R^{2b}$ in the formula (1)),
$Z^a$ and $Z^b$ are each independently a divalent group represented by the formula (13):

[Chem. 77]

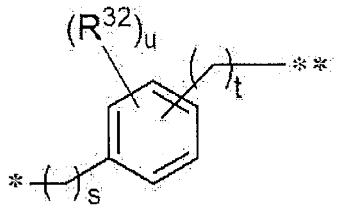

(13)

(in the formula (13), * is a bonding position with O in the formula (1),
** is a bonding position with $Y^a$ or $Y^b$ in the formula (1),
s is 0,
t is 1, and
u is 0),
$R^{3a}$ is a monovalent group represented by the formula (5):

[Chem. 78]

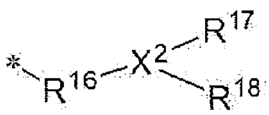

(5)

(in the formula (5), * is a bonding position,
$X^2$ is a trivalent group represented by the formula (6):

[Chem. 79]

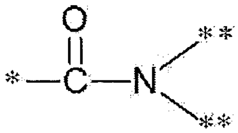

(6)

(in the formula (6), * is a bonding position with $R^{16}$, and
** is a bonding position with $R^{17}$ or $R^{18}$),
$R^{16}$ is an alkylene group having 1 to 10 carbon atoms, and
$R^{17}$ and $R^{18}$ are each independently an alkyl group having 1 to 10 carbon atoms), and
$R^{3b}$ is an alkenyl group having 2 to 20 carbon atoms (preferably a monovalent group derived from oleic acid).

Cationic lipid (1-5) is preferably compound 124.

In one embodiment of the present invention, cationic lipid (1) is preferably a cationic lipid in which $R^{3a}$ is group (7) (hereinafter abbreviated as "cationic lipid (1-7)"). Preferred examples of cationic lipid (1-7) include the following cationic lipids.

[Cationic lipid (1-7a)]

Cationic lipid (1-7), wherein

$R^{1a}$ and $R^{1b}$ are each independently an alkylene group having 1 to 3 carbon atoms,
$X^a$ and $X^b$ are each independently a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms,
$Y^a$ and $Y^b$ are each independently an ester bond or an amide bond,
$Z^a$ and $Z^b$ are each independently a divalent group represented by the formula (13):

[Chem. 80]

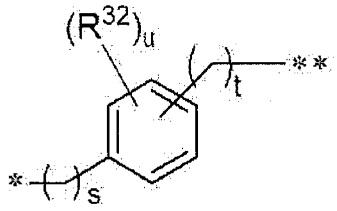

(13)

(in the formula (13), * is a bonding position with O in the formula (1),
** is a bonding position with $Y^a$ or $Y^b$ in the formula (1),
s is an integer of 0 to 3,
t is an integer of 0 to 3,
u is an integer of 0 to 4, and
$R^{32}$ in the number of u are each independently a halogen atom, an alkyl group having 1 to 4 carbon atoms, or an alkoxy group having 1 to 4 carbon atoms),
$R^{3a}$ is a monovalent group represented by the formula (7):

[Chem. 81]

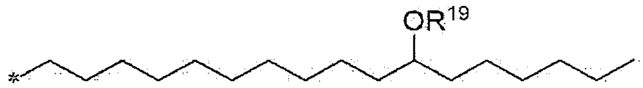

(7)

(in the formula (7), * is a bonding position, and
$R^{19}$ is a *-CO-$R^{20}$ group (wherein * is a bonding position, and $R^{20}$ is an alkyl group having 1 to 9 carbon atoms)), and
$R^{3b}$ is

(A) a monovalent group represented by the aforementioned formula (7) (the definitions of the symbols in the formula (7) are as mentioned above),
(B) an alkyl group having 1 to 30 carbon atoms, or
(C) an alkenyl group having 2 to 20 carbon atoms.

[Cationic lipid (1-7b)]

Cationic lipid (1-7), wherein

$R^{1a}$ and $R^{1b}$ are each an alkylene group having 1 to 3 carbon atoms,
$X^a$ and $X^b$ are each independently group (26) or group (27),
$R^{2a}$ and $R^{2b}$ are each independently an alkylene group having 1 to 4 carbon atoms,
$Y^a$ and $Y^b$ are each an ester bond,
$Z^a$ and $Z^b$ are each independently a divalent group represented by the formula (13):

[Chem. 82]

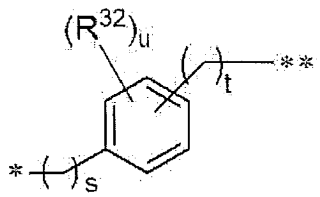

(13)

(in the formula (13), * is a bonding position with O in the formula (1),
** is a bonding position with $Y^a$ or $Y^b$ in the formula (1),
s is 0 or 1,
t is an integer of 0 to 2,
u is an integer of 0 to 2, and
$R^{32}$ in the number of u are each independently a halogen atom or an alkoxy group having 1 to 4 carbon atoms),
$R^{3a}$ is a monovalent group represented by the formula (7):

[Chem. 83]

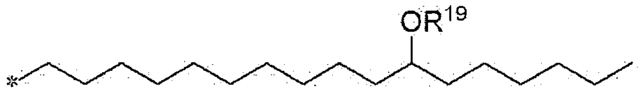

(7)

(in the formula (7), * is a bonding position, and $R^{19}$ is a *-CO-$R^{20}$ group (wherein * is a bonding position, and $R^{20}$ is an alkyl group having 1 to 9 carbon atoms)), and $R^{3b}$ is

(A) a monovalent group represented by the aforementioned formula (7) (the definitions of the symbols in the formula (7) are as mentioned above), or
(B) an alkenyl group having 2 to 20 carbon atoms.

[Cationic lipid (1-7c)]

Cationic lipid (1-7), wherein

$R^{1a}$ and $R^{1b}$ are each an ethylene group,
$X^a$ and $X^b$ are each independently group (26) (particularly a piperidinediyl group),
$R^{2a}$ and $R^{2b}$ are each an ethylene group,
$Y^a$ and $Y^b$ are each *-CO-O-** (wherein * is a bonding position with $Z^a$ or $Z^b$ in the formula (1), and ** is a bonding position with $R^{2a}$ or $R^{2b}$ in the formula (1)),
$Z^a$ and $Z^b$ are each independently a divalent group represented by the formula (13):

[Chem. 84]

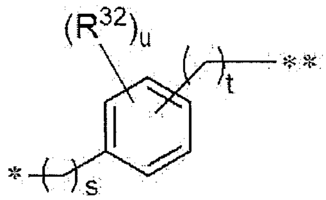

(13)

(in the formula (13), * is a bonding position with O in the formula (1),
** is a bonding position with $Y^a$ or $Y^b$ in the formula (1),
s is 0,
t is 1, and
u is 0),
$R^{3a}$ is a monovalent group represented by the formula (7):

[Chem. 85]

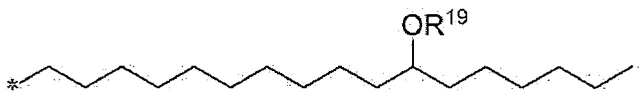

(7)

(in the formula (7), * is a bonding position, and $R^{19}$ is a *-CO-$R^{20}$ group (wherein * is a bonding position, and $R^{20}$ is an alkyl group having 1 to 9 carbon atoms)), and $R^{3b}$ is an alkenyl group having 2 to 20 carbon atoms (preferably a monovalent group derived from oleic acid).

Cationic lipid (1-7) is preferably compound 145.

In one embodiment of the present invention, cationic lipid (1) is preferably a cationic lipid in which $R^{3a}$ is group (9) (hereinafter abbreviated as "cationic lipid (1-9)"). Preferred examples of cationic lipid (1-9) include the following cationic lipids.

[Cationic lipid (1-9a)]

Cationic lipid (1-9), wherein

$R^{1a}$ and $R^{1b}$ are each independently an alkylene group having 1 to 3 carbon atoms,
$X^a$ and $X^b$ are each independently a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms,
$Y^a$ and $Y^b$ are each independently an ester bond or an amide bond,
$Z^a$ and $Z^b$ are each independently a divalent group represented by the formula (13):

[Chem. 86]

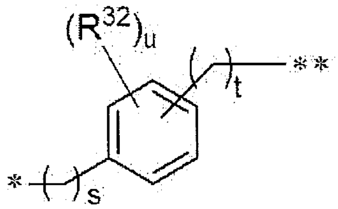

(13)

(in the formula (13), * is a bonding position with O in the formula (1),
** is a bonding position with $Y^a$ or $Y^b$ in the formula (1),
s is an integer of 0 to 3,
t is an integer of 0 to 3,
u is an integer of 0 to 4, and
$R^{32}$ in the number of u are each independently a halogen atom, an alkyl group having 1 to 4 carbon atoms, or an alkoxy group having 1 to 4 carbon atoms),
$R^{3a}$ is a monovalent group represented by the formula (9):

[Chem. 87]

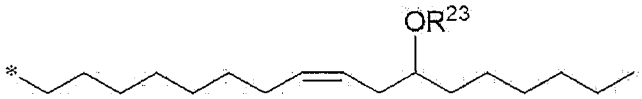

(9)

(in the formula (9), * is a bonding position, and
$R^{23}$ is a hydrogen atom or a *-Si ($R^{13}$)($R^{14}$)($R^{15}$) group (wherein * is a bonding position, $R^{13}$ is a tert-butyl group, and $R^{14}$ and $R^{15}$ are each a methyl group or a phenyl group)), and
$R^{3b}$ is

(A) a monovalent group represented by the aforementioned formula (9) (the definitions of the symbols in the formula (9) are as mentioned above),
(B) an alkyl group having 1 to 30 carbon atoms, or
(C) an alkenyl group having 2 to 20 carbon atoms.

[Cationic lipid (1-9b)]

Cationic lipid (1-9), wherein

$R^{1a}$ and $R^{1b}$ are each an alkylene group having 1 to 3 carbon atoms,
$X^a$ and $X^b$ are each independently group (26) or group (27),
$R^{2a}$ and $R^{2b}$ are each independently an alkylene group having 1 to 4 carbon atoms,
$Y^a$ and $Y^b$ are each an ester bond,
$Z^a$ and $Z^b$ are each independently a divalent group represented by the formula (13):

[Chem. 88]

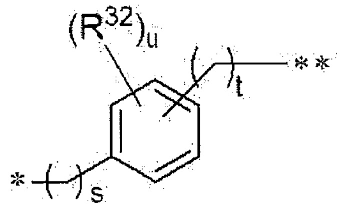

(13)

(in the formula (13), * is a bonding position with O in the formula (1),
** is a bonding position with $Y^a$ or $Y^b$ in the formula (1),
s is 0 or 1,
t is an integer of 0 to 2,
u is an integer of 0 to 2, and
$R^{32}$ in the number of u are each independently a halogen atom or an alkoxy group having 1 to 4 carbon atoms),
$R^{3a}$ is a monovalent group represented by the formula (9):

[Chem. 89]

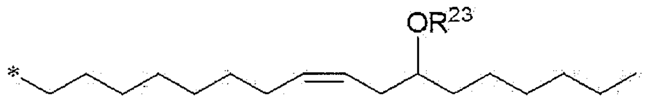

(9)

(in the formula (9), * is a bonding position, and
$R^{23}$ is a hydrogen atom or a *-Si ($R^{13}$)($R^{14}$)($R^{15}$) group (wherein * is a bonding position, $R^{13}$ is a tert-butyl group, and $R^{14}$ and $R^{15}$ are each a methyl group)), and
$R^{3b}$ is

(A) a monovalent group represented by the aforementioned formula (9) (the definitions of the symbols in the formula (9) are as mentioned above), or
(B) an alkenyl group having 2 to 20 carbon atoms.

[Cationic lipid (1-9c)]

Cationic lipid (1-9), wherein

$R^{1a}$ and $R^{1b}$ are each an ethylene group,
$X^a$ and $X^b$ are each independently group (26) (particularly piperidinediyl group),
$R^{2a}$ and $R^{2b}$ are each an ethylene group,
$Y^a$ and $Y^b$ are each *-CO-O-** (wherein * is a bonding position with $Z^a$ or $Z^b$ in the formula (1), and ** is a bonding position with $R^{2a}$ or $R^{2b}$ in the formula (1)),
$Z^a$ and $Z^b$ are each independently a divalent group represented by the formula (13):

[Chem. 90]

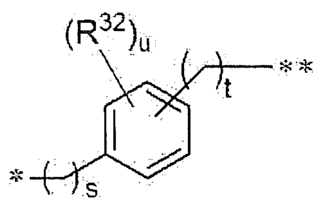

(13)

(in the formula (13), * is a bonding position with O in the formula (1),
** is a bonding position with $Y^a$ or $Y^b$ in the formula (1),
s is 0,
t is 1, and
u is 0),
$R^{3a}$ is a monovalent group represented by the formula (9):

[Chem. 91]

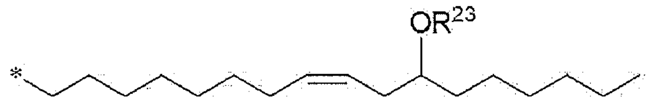

(9)

(in the formula (9), * is a bonding position, and

$R^{23}$ is a hydrogen atom or a *-Si ($R^{13}$)($R^{14}$)($R^{15}$) group (wherein * is a bonding position, $R^{13}$ is a tert-butyl group, and $R^{14}$ and $R^{15}$ are each a methyl group)), and

$R^{3b}$ is

(A) a monovalent group represented by the aforementioned formula (9) (the definitions of the symbols in the formula (9) are as mentioned above), or

(B) an alkenyl group having 2 to 20 carbon atoms (preferably a monovalent group derived from oleic acid).

Cationic lipid (1-9) is preferably compound 127a, compound 127, or compound 194, more preferably compound 127a or compound 127.

The production method of cationic lipid (1) is now explained.

Cationic lipid (1) has an -S-S- (disulfide) bond. Therefore, the production method of cationic lipid (1) includes

($\alpha$) a method for obtaining cationic lipid (1) containing an -S-S- bond by producing $R^{3a}$-CO-O-$Z^a$-$Y^a$-$R^{2a}$-$X^a$-$R^{1a}$-SH (i.e., thiol compound), and $R^{3b}$-CO-O-$Z^b$-$Y^b$-$R^{2b}$-$X^b$-$R^{1b}$-SH (i.e., thiol compound) and then oxidizing (coupling) same, or

($\beta$) a method in which necessary parts are sequentially synthesized from a compound containing an -S-S- bond to finally obtain cationic lipid (1). It is preferably method ($\beta$). Specific examples of method ($\beta$) are shown below but the production method of cationic lipid (1) is not limited.

As a starting compound, a both-terminal carboxylic acid containing an -S-S- bond, a both-terminal amine, a both-terminal isocyanate, a both-terminal alcohol, a both-terminal alcohol with a leaving group such as methanesulfonyl group, and a both-terminal carbonate with a leaving group such as p-nitrophenylcarbonate group can be mentioned.

For example, when cationic lipid (1') in which $R^{1a}$ and $R^{1b}$ are the same $R^1$, $X^a$ and $X^b$ are the same X, $R^{2a}$ and $R^{2b}$ are the same $R^2$, $Y^a$ and $Y^b$ are the same Y, $Z^a$ and $Z^b$ are the same Z, and $R^{3a}$ and $R^{3b}$ are the same $R^3$ is produced, the cationic lipid (1') of interest can be obtained by the following synthesis pathway.

[Chem. 92]

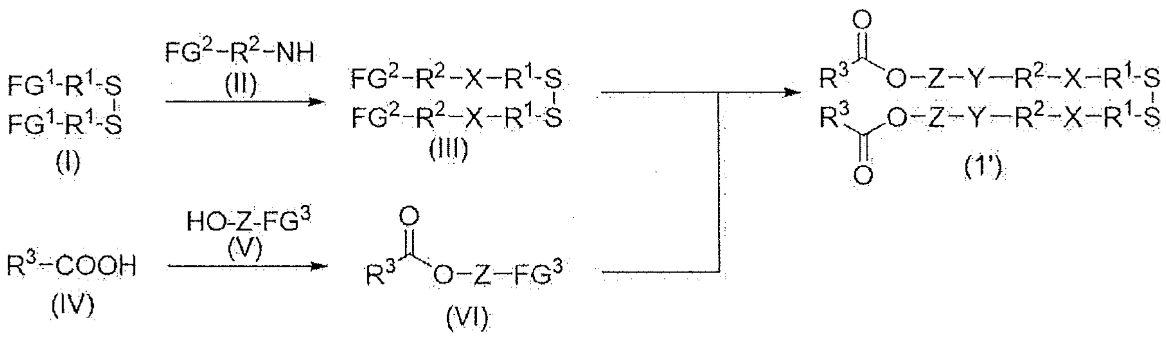

Compound (I) having a reactive functional group ($FG^1$) at both ends and containing an -S-S- bond, and compound (II) having a secondary amino group and one reactive functional group ($FG^2$) at the end are reacted to synthesize compound (III). Compound (IV) (carboxylic acid) having $R^3$ and compound (V) having a hydroxyl group and a reactive functional group ($FG^3$) are reacted to synthesize compound (VI), and finally, compound (VI) and compound (III) are reacted to synthesize cationic lipid (1') containing -S-S- bond, $R^1$, X, $R^2$, Y, Z and $R^3$.

Among the aforementioned production methods, compound (III) can be produced by the method described in WO 2016/121942 or WO 2019/188867.

For the reaction between compound (IV) and compound (V), a base catalyst such as potassium carbonate, sodium carbonate, potassium hydroxide, triethylamine, 4-dimethylaminopyridine (hereinafter sometimes to be abbreviated as "DMAP"), or the like may be used, and an acid catalyst such as p-toluenesulfonic acid, methanesulfonic acid, or the like may also be used. The aforementioned reaction may be performed without a catalyst.

In addition, compound (IV) and compound (V) may be directly reacted using a condensing agent such as dicyclohexylcarbodiimide (hereinafter "DCC"), diisopropylcarbodiimide (hereinafter sometimes to be abbreviated as "DIC"), 1-ethyl-3-(3-dimethylaminopropyl)carbodiimide hydrochloride (hereinafter sometimes to be abbreviated as "EDC hydrochloride"), or the like. Alternatively, compound (IV) may be converted into an anhydride or the like using a condensing agent, and then reacted with compound (V).

The amount of compound (IV) charged is generally 1 to 50 molar equivalents, preferably 1 to 10 molar equivalents, relative to compound (V).

The catalyst to be used in the reaction between compound (IV) and compound (V) can be appropriately selected according to the type of the compound to be reacted.

The amount of the catalyst is generally 0.05 to 100 molar equivalents, preferably 0.1 to 20 molar equivalents, more preferably 0.1 to 5 molar equivalents, relative to compound (V).

The solvent to be used for the reaction between compound (IV) and and compound (V) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably 10 to 50°C. The time of the above-mentioned reaction is generally 1 to for 48 hr, preferably 1 to for 24 hr.

The reaction product (VI) obtained by the above-mentioned reaction can be appropriately purified by a general purification method such as extraction purification, recrystallization, adsorption purification, reprecipitation, column chromatography, ion exchange chromatography, or the like.

When compound (III) and compound (VI) are reacted, a base catalyst such as potassium carbonate, sodium carbonate, potassium hydroxide, triethylamine, 4-dimethylaminopyridine, or the like may be used, and an acid catalyst such as p-toluenesulfonic acid, methanesulfonic acid, or the like may also be used. The aforementioned reaction may be performed without a catalyst.

In addition, compound (III) and compound (VI) may be directly reacted using a condensing agent such as DCC, DIC, EDC hydrochloride, or the like. Alternatively, compound (VI) may be converted into an anhydride or the like using a condensing agent, and then reacted with compound (III).

The amount of compound (VI) charged is generally 0.1 to 1.0 molar equivalents, preferably 0.2 to 0.8 molar equivalents, relative to compound (III).

The catalyst to be used in the reaction between compound (III) and compound (VI) may be appropriately selected according to the type of the compound to be reacted.

The amount of the catalyst is generally 0.05 to 100 molar equivalents, preferably 0.1 to 20 molar equivalents, more preferably 0.1 to 5 molar equivalents, relative to compound (III).

The solvent to be used for the reaction between compound (III) and compound (IV) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably 10 to 50°C. The time of the above-mentioned reaction is generally 1 to 48 hr, preferably 1 to 24 hr.

The cationic lipid (1') obtained by the above-mentioned reaction can be appropriately purified by a general purification method such as extraction purification, recrystallization, adsorption purification, reprecipitation, column chromatography, ion exchange chromatography, or the like.

The cationic lipid represented by the formula (1') can also be synthesized by the following synthesis pathway.

[Chem. 93]

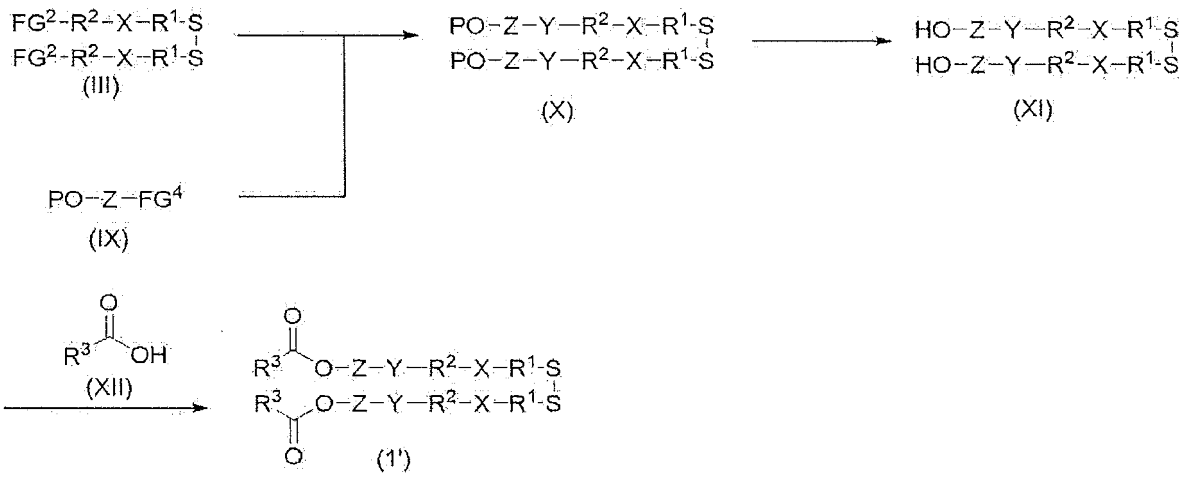

Compound (III) having reactive functional groups ($FG^2$s) and compound (IX) having protected hydroxy groups (POs) and a reactive functional group ($FG^4$) are reacted to synthesize compound (X). Then, the protecting groups (Ps) of compound (X) are removed to synthesize compound (XI) having hydroxy groups. Successively, compound (XI) and compound (XII) having a carboxy group are reacted to synthesize cationic lipid (1') containing - S-S- bond, $R^1$, X, $R^2$, Y, Z and $R^3$.

When compound (III) and compound (IX) are reacted, a base catalyst such as potassium carbonate, sodium carbonate, potassium hydroxide, triethylamine, 4-dimethylaminopyridine, or the like may be used, and an acid catalyst such as p-toluenesulfonic acid, methanesulfonic acid, or the like may also be used. The aforementioned reaction may be performed without a catalyst.

In addition, compound (III) and compound (IX) may be directly reacted using a condensing agent such as DCC, DIC, EDC hydrochloride, or the like. Alternatively, compound (IX) may be converted into an anhydride or the like using a condensing agent, and then reacted with compound (III).

The amount of compound (IX) charged is generally 1.0 to 10 molar equivalents, preferably 2.0 to 8.0 molar equivalents, relative to compound (III).

The catalyst to be used in the reaction between compound (III) and compound (IX) can be appropriately selected according to the type of the compound to be reacted.

The amount of the catalyst is generally 0.05 to 100 molar equivalents, preferably 0.1 to 20 molar equivalents, more preferably 0.1 to 5 molar equivalents, relative to compound (III).

The solvent to be used for the reaction between compound (III) and compound (IX) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably 10 to 50°C. The time of the above-mentioned reaction is generally 1 to 48 hr, preferably 1 to 24 hr.

When removing the protecting group (P) of compound (X), they are appropriately selected depending on the kind of protecting group.

The solvent to be used for removing the protecting group (P) of compound (X) is not particularly limited as long as it does not inhibit the reaction. Examples thereof include water, ethyl acetate, dichloromethane, chloroform, acetonitrile, toluene, THF, and the like. Among these, chloroform, toluene, and THF are preferred.

The temperature of deprotection reaction is generally 0 to 150°C, preferably 0 to 80°C, more preferably 10 to 50°C. The time of the deprotection reaction is generally 1 to 48 hr, preferably 1 to 24 hr.

When compound (XI) and compound (XII) are reacted, a base catalyst such as potassium carbonate, sodium carbonate, potassium hydroxide, triethylamine, 4-dimethylaminopyridine, or the like may be used, and an acid catalyst such as p-toluenesulfonic acid, methanesulfonic acid, or the like may also be used. The aforementioned reaction may be performed without a catalyst.

In addition, compound (XI) and compound (XII) may be directly reacted using a condensing agent such as DCC, DIC, EDC hydrochloride, or the like. Alternatively, compound (XII) may be converted into an anhydride or the like using a condensing agent, and then reacted with compound (XI).

The amount of compound (XII) charged is generally 0.1 to 50 molar equivalents, preferably 0.5 to 10 molar equivalents, relative to compound (XI).

The catalyst to be used in the reaction between compound (XI) and compound (XII) can be appropriately selected

according to the type of the compound to be reacted.

The amount of the catalyst is generally 0.05 to 100 molar equivalents, preferably 0.1 to 20 molar equivalents, more preferably 0.1 to 5 molar equivalents, relative to compound (XII).

The solvent to be used for the reaction between compound (XI) and compound (XII) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably 10 to 50°C. The time of the above-mentioned reaction is generally 1 to 48 hr, preferably 1 to 24 hr.

The cationic lipid (1') obtained by the above-mentioned reaction can be appropriately purified by a general purification method such as extraction purification, recrystallization, adsorption purification, reprecipitation, column chromatography, ion exchange chromatography, or the like.

Furthermore, for example, when producing cationic lipid (1) in which $R^{1a}$ and $R^{1b}$ are the same $R^1$, $X^a$ and $X^b$ are the same X, $R^{2a}$ and $R^{2b}$ are the same $R^2$, $Y^a$ and $Y^b$ are the same Y, $Z^a$ and $Z^b$ are the same Z, $R^{3a}$ is $R^3$, and $R^{3b}$ is $R^{3'}$ ($R^3 \neq R^{3'}$), the desired cationic lipid (1") can be obtained by the synthetic route shown below.

[Chem. 94]

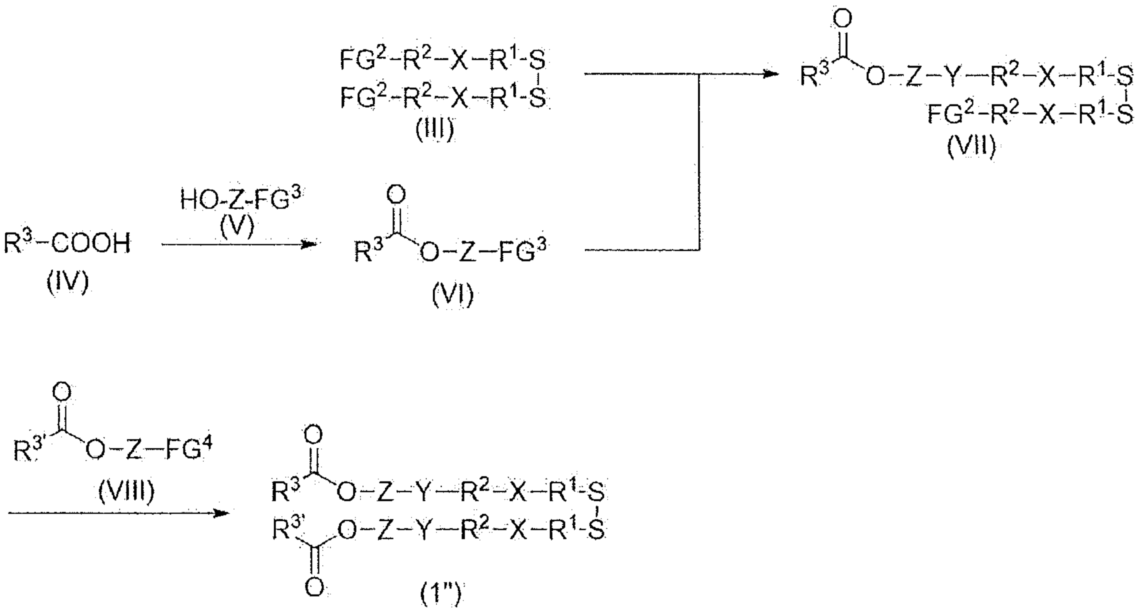

Compound (IV) (carboxylic acid) having $R^3$ and compound (V) having a hydroxy group and a reactive functional group ($FG^3$) are reacted to synthesize compound (VI). Then, compound (VI) and compound (III) having reactive functional groups ($FG^2$s) are reacted to synthesize compound (VII). Compound (VII) and compound (VIII) having a reactive functional group ($FG^4$) are reacted to obtain cationic lipid (1") containing -S-S- bond, $R^1$, X, $R^2$, Y, Z and $R^3$.

In the reaction between compound (IV) and compound (V), a base catalyst such as potassium carbonate, sodium carbonate, potassium hydroxide, triethylamine, DMAP, or the like may be used, and an acid catalyst such as p-toluenesulfonic acid, methanesulfonic acid, or the like may also be used. The aforementioned reaction may be performed without a catalyst.

In addition, compound (IV) and compound (V) may be directly reacted using a condensing agent such as DCC, DIC, EDC hydrochloride, or the like. Alternatively, compound (IV) may be converted into an anhydride or the like using a condensing agent, and then reacted with compound (V).

The amount of compound (IV) charged is generally 1 to 50 molar equivalents, preferably 1 to 10 molar equivalents, relative to compound (V).

The catalyst to be used in the reaction between compound (IV) and compound (V) can be appropriately selected according to the type of the compound to be reacted.

The amount of the catalyst is generally 0.05 to 100 molar equivalents, preferably 0.1 to 20 molar equivalents, more preferably 0.1 to 5 molar equivalents, relative to compound (V).

The solvent to be used for the reaction between compound (IV) and compound (V) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably

10 to 50°C. The time of the above-mentioned reaction is generally 1 to 48 hr, preferably 1 to 24 hr.

The reaction product (VI) obtained by the above-mentioned reaction can be appropriately purified by a general purification method such as extraction purification, recrystallization, adsorption purification, reprecipitation, column chromatography, ion exchange chromatography, or the like.

When compound (III) and compound (VI) are reacted, a base catalyst such as potassium carbonate, sodium carbonate, potassium hydroxide, triethylamine, 4-dimethylaminopyridine, or the like may be used, and an acid catalyst such as p-toluenesulfonic acid, methanesulfonic acid, or the like may also be used. The aforementioned reaction may be performed without a catalyst.

In addition, compound (III) and compound (VI) may be directly reacted using a condensing agent such as DCC, DIC, EDC hydrochloride, or the like. Alternatively, compound (VI) may be converted into an anhydride or the like using a condensing agent, and then reacted with compound (III).

The amount of compound (VI) charged is generally 0.1 to 1.0 molar equivalents, preferably 0.2 to 0.8 molar equivalents, relative to compound (III).

The catalyst to be used in the reaction between compound (III) and compound (VI) can be appropriately selected according to the type of the compound to be reacted.

The amount of the catalyst is generally 0.05 to 100 molar equivalents, preferably 0.1 to 20 molar equivalents, more preferably 0.1 to 5 molar equivalents, relative to compound (III).

The solvent to be used for the reaction between compound (III) and compound (VI) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably 10 to 50°C. The time of the above-mentioned reaction is generally 1 to 48 hr, preferably 1 to 24 hr.

When compound (VII) and compound (VIII) are reacted, a base catalyst such as potassium carbonate, sodium carbonate, potassium hydroxide, triethylamine, 4-dimethylaminopyridine, or the like may be used, and an acid catalyst such as p-toluenesulfonic acid, methanesulfonic acid, or the like may also be used. The aforementioned reaction may be performed without a catalyst.

In addition, compound (VII) and compound (VIII) may be directly reacted using a condensing agent such as DCC, DIC, EDC hydrochloride, or the like. Alternatively, compound (VIII) may be converted into an anhydride or the like using a condensing agent, and then reacted with compound (VII).

The amount of compound (VII) charged is generally 1 to 50 molar equivalents, preferably 1 to 10 molar equivalents, relative to compound (VIII).

The catalyst to be used in the reaction between compound (VII) and compound (VIII) can be appropriately selected according to the type of the compound to be reacted.

The amount of the catalyst is generally 0.05 to 100 molar equivalents, preferably 0.1 to 20 molar equivalents, more preferably 0.1 to 5 molar equivalents, relative to compound (VII).

The solvent to be used for the reaction between compound (VII) and compound (VIII) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably 10 to 50°C. The time of the above-mentioned reaction is generally 1 to 48 hr, preferably 1 to 24 hr.

Cationic lipid (1") obtained by the above-mentioned reaction, can be appropriately purified by a general purification method such as extraction purification, recrystallization, adsorption purification, reprecipitation, column chromatography, ion exchange chromatography, or the like.

The cationic lipid (1") can also be synthesized by the following synthesis pathway.

[Chem. 95]

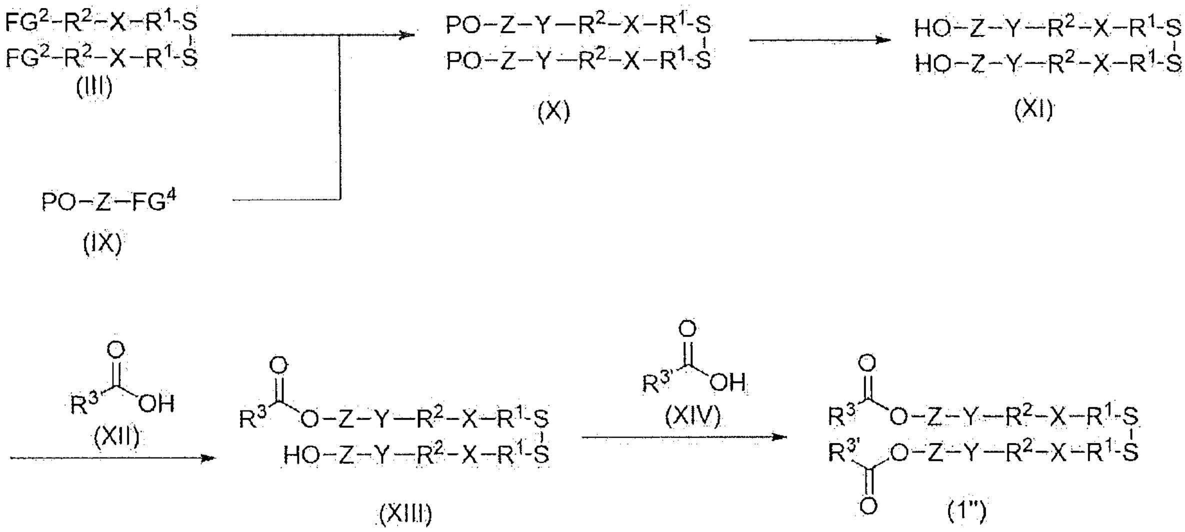

(III) (IX) (X) (XI) (XII) (XIII) (XIV) (1")

Compound (III) having reactive functional groups ($FG^2$s) and compound (IX) having a protected hydroxy group (PO) and a reactive functional group ($FG^4$) are reacted to synthesize compound (X). Then, the protecting groups (Ps) of compound (X) are removed to synthesize compound (XI) having hydroxy groups. Successively, compound (XI) and compound (XII) (carboxylic acid) are reacted to synthesize compound (XIII). Compound (XIII) and compound (XIV) are reacted to obtain cationic lipid (1") containing -S-S- bond, $R^1$, X, $R^2$, Y, Z and $R^3$.

When compound (III) and compound (IX) are reacted, a base catalyst such as potassium carbonate, sodium carbonate, potassium hydroxide, triethylamine, 4-dimethylaminopyridine, or the like may be used, and an acid catalyst such as p-toluenesulfonic acid, methanesulfonic acid, or the like may also be used. The aforementioned reaction may be performed without a catalyst.

In addition, compound (III) and compound (IX) may be directly reacted using a condensing agent such as DCC, DIC, EDC hydrochloride, or the like. Alternatively, compound (IX) may be converted into an anhydride or the like using a condensing agent, and then reacted with compound (III).

The amount of compound (IX) to be charged is generally 1.0 to 10 molar equivalents, preferably 2.0 to 8.0 molar equivalents, relative to compound (III).

The catalyst to be used in the reaction between compound (III) and compound (IX) can be appropriately selected according to the type of the compound to be reacted.

The amount of the catalyst is generally 0.05 to 100 molar equivalents, preferably 0.1 to 20 molar equivalents, more preferably 0.1 to 5 molar equivalents, relative to compound (III).

The solvent to be used for the reaction between compound (III) and compound (IX) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably 10 to 50°C. The time of the above-mentioned reaction is generally 1 to 48 hr, preferably 1 to 24 hr.

When removing the protecting group (P) of compound (X), they are appropriately selected depending on the kind of protecting group.

The solvent to be used for removing the protecting group (P) of compound (X) is not particularly limited as long as it does not inhibit the reaction. Examples thereof include water, ethyl acetate, dichloromethane, chloroform, acetonitrile, toluene, THF, and the like. Among these, chloroform, toluene, and THF are preferred.

The temperature of deprotection reaction is generally 0 to 150°C, preferably 0 to 80°C, more preferably 10 to 50°C. The time of the deprotection reaction is generally 1 to 48 hr, preferably 1 to 24 hr.

When compound (XI) and compound (XII) are reacted, a base catalyst such as potassium carbonate, sodium carbonate, potassium hydroxide, triethylamine, 4-dimethylaminopyridine, or the like may be used, and an acid catalyst such as p-toluenesulfonic acid, methanesulfonic acid, or the like may also be used. The aforementioned reaction may be performed without a catalyst.

In addition, compound (XI) and compound (XII) may be directly reacted using a condensing agent such as DCC, DIC, EDC hydrochloride, or the like. Alternatively, compound (XII) may be converted into an anhydride or the like using a condensing agent, and then reacted with compound (XI).

The amount of compound (XII) charged is generally 0.1 to 50 molar equivalents, preferably 0.5 to 10 molar equivalents, relative to compound (XI).

The catalyst to be used in the reaction between compound (XI) and compound (XII) can be appropriately selected according to the type of the compound to be reacted.

The amount of the catalyst is generally 0.05 to 100 molar equivalents, preferably 0.1 to 20 molar equivalents, more preferably 0.1 to 5 molar equivalents, relative to compound (XII).

The solvent to be used for the reaction between compound (XI) and compound (XII) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably 10 to 50°C. The time of the above-mentioned reaction is generally 1 to 48 hr, preferably 1 to 24 hr.

When compound (XIII) and compound (XIV)are reacted, a base catalyst such as potassium carbonate, sodium carbonate, potassium hydroxide, triethylamine, 4-dimethylaminopyridine, or the like may be used, and an acid catalyst such as p-toluenesulfonic acid, methanesulfonic acid, or the like may also be used. The aforementioned reaction may be performed without a catalyst.

In addition, compound (XIII) and compound (XIV) may be directly reacted using a condensing agent such as DCC, DIC, EDC hydrochloride, or the like. Alternatively, compound (XIV) may be converted into an anhydride or the like using a condensing agent, and then reacted with compound (XIII).

The amount of compound (XIV) charged is generally 1 to 50 molar equivalents, preferably 1 to 10 molar equivalents, relative to compound (XIII).

The catalyst to be used in the reaction between compound (XIII) and compound (XIV) can be appropriately selected according to the type of the compound to be reacted.

The amount of the catalyst is generally 0.05 to 100 molar equivalents, preferably 0.1 to 20 molar equivalents, more preferably 0.1 to 5 molar equivalents, relative to compound (XIV).

The solvent to be used for the reaction between compound (XIII) and compound (XIV) is not particularly limited as long as it does not inhibit the reaction. Examples thereof include water, ethyl acetate, dichloromethane, chloroform, acetonitrile, toluene, and the like. Among these, chloroform and toluene are preferred.

The temperature of the above-mentioned reaction is generally 0 to 150°C, preferably 0 to 80°C, more preferably 10 to 50°C. The time of the above-mentioned reaction is generally 1 to 48 hr, preferably 1 to 24 hr.

Cationic lipid (1") obtained by the above-mentioned reaction, can be appropriately purified by a general purification method such as extraction purification, recrystallization, adsorption purification, reprecipitation, column chromatography, ion exchange chromatography, or the like.

Specific examples are described below. Those of ordinary skill in the art can produce a desired cationic lipid (1) by appropriately selecting the starting material and performing the reaction according to the methods described in Examples in the present specification.

The present invention also provides a lipid membrane structure containing cationic lipid (1) as a constituent lipid of the membrane. The lipid membrane structure of the present invention may further contain a nucleic acid. From the aspects of nucleic acid encapsulation efficiency and toxicity, the L/R ratio (=total lipid amount (nmol)/nucleic acid amount (μg)) of the lipid membrane structure containing nucleic acid is preferably 1 to 300 nmol/μg, more preferably 10 to 250 nmol/μg, further preferably 30 to 200 nmol/μg.

In the present specification, the "lipid membrane structure" means a particle having a membrane structure in which the hydrophilic groups of amphipathic lipid are arranged in the interface, facing the aqueous phase side. The "amphiphilic lipid" means a lipid having both a hydrophilic group showing hydrophilicity and a hydrophobic group showing hydrophobicity. Examples of the amphiphilic lipid include cationic lipid, phospholipid, and the like.

While the form of the lipid membrane structure of the present invention is not particularly limited, for example, liposome (e.g., monolayer liposome, multilayer liposome, and the like), O/W emulsion, W/O emulsion, spherical micelle, worm-like micelle, lipid nano particle (sometimes to be abbreviated as "LNP" in the present specification), disordered layer structure, and the like can be mentioned as a form of cationic lipid (1) dispersed in an aqueous solvent. The lipid membrane structure of the present invention is preferably a liposome. In another embodiment of the present invention, the lipid membrane structure of the present invention is preferably LNP.

The lipid membrane structure of the present invention may further contain other constituent components in addition to cationic lipid (1). Examples of said other constituent component include lipid (phospholipid (phosphatidylinositol, phosphatidylethanolamine, phosphatidylserine, phosphatidic acid, phosphatidylglycerol, phosphatidylcholine, etc.), glycolipid, peptide lipid, cholesterol, cationic lipid other than cationic lipid (1), PEG lipid, etc.), surfactant (e.g., 3-[(3-cholamidopropyl)dimethylammonio]propanesulfonate, sodium cholate salt, octylglycoside, N-D-gluco-N-methylalkane amides, etc.), polyethylene glycol, protein, and the like. The content of said other constituent component in the lipid membrane structure of the present invention is generally 5 to 95 mol%, preferably 10 to 90 mol%, more preferably 30 to 80 mol%.

While the content of cationic lipid (1) to be contained in the lipid membrane structure of the present invention is not particularly limited, when the lipid membrane structure is used as the below-mentioned nucleic acid-introducing agent,

cationic lipid (1) is contained in an amount sufficient for introducing a nucleic acid. The aforementioned content is, for example, 5 to 100 mol%, preferably 10 to 90 mol%, more preferably 20 to 70 mol%, of the total lipids.

The lipid membrane structure of the present invention can be prepared by dispersing cationic lipid (1) and other constituent components (lipid etc.) in a suitable solvent or dispersion medium, for example, aqueous solvent and alcoholic solvent, and performing an operation to induce organization as necessary.

Examples of the "operation to induce organization" include, but are not limited to, methods known per se such as ethanol dilution method using a micro flow path or vortex, simple hydration method, sonication, heating, vortex, ether injecting method, French press method, cholic acid method, $Ca^{2+}$ fusion method, freeze-thaw method, reversed-phase evaporation method, and the like.

A nucleic acid can be introduced into a cell in vivo and/or in vitro by bringing a lipid membrane structure containing the nucleic acid into contact with the cell. Therefore, the present invention provides a nucleic acid-introducing agent containing cationic lipid (1). The nucleic acid-introducing agent of the present invention is preferably the lipid structure of the present invention. The nucleic acid-introducing agent of the present invention preferably contains a nucleic acid.

The nucleic acid-introducing agent of the present invention can introduce any nucleic acid into a cell. The type of nucleic acid includes, but is not limited to, DNA, RNA, RNA chimeric nucleic acid, DNA/RNA hybrid, and the like. While any of single-stranded to triple-stranded nucleic acids can be used, single-stranded or double-strand one is preferred. The nucleic acid may also be other type of nucleotide that is N-glycoside of a purine or pyrimidine base, other oligomer having a non-nucleotide backbone (e.g., commercially available peptide nucleic acid (PNA) and the like), other oligomer containing a special bond (said oligomer containing a nucleotide having a a configuration permitting base pairing or base attachment, which are found in DNA and RNA), or the like. Furthermore, the nucleic acid may also be, for example, a nucleic acid added with known modification, a nucleic acid with a label known in the pertinent field, a nucleic acid with a cap, a methylated nucleic acid, a nucleic acid in which one or more natural nucleotides substituted by an analog, a nucleic acid with an intramolecularly modified nucleotide, a nucleic acid with a non-charge bond (e.g., methylphosphonate, phosphotriester, phosphoramidate, carbamate, etc.), a nucleic acid with a charged bond or sulfur-containing bond (e.g., phosphorothioate, phosphorodithioate, etc.), a nucleic acid with a side chain group such as protein (nuclease, nuclease inhibitor, toxin, antibody, signal peptide, poly-L-lysine, etc.), sugar (e.g., monosaccharide and the like), or the like, a nucleic acid containing an intercalating compound (e.g., acridine, psoralen, etc.), a nucleic acid containing a chelate compound (e.g., metal, radioactive metal, boron, oxidative metal, etc.), a nucleic acid containing an alkylating agent, a nucleic acid with a modified bond (e.g., $\alpha$ anomer-type nucleic acid and the like), or the like.

The type of the DNA that can be used in the present invention is not particularly limited, and can be selected as appropriate according to the purpose of use. Examples thereof include plasmid DNA, cDNA, antisense DNA, chromosomal DNA, PAC, BAC, CpG oligo, and the like. Plasmid DNA, cDNA, and antisense DNA are preferred, and plasmid DNA is more preferred. Circular DNA such as plasmid DNA and the like can be digested as appropriate with restriction enzymes and the like, and also used as a linear DNA.

The type of the RNA that can be used in the present invention is not particularly limited, and can be selected as appropriate according to the purpose of use. Examples thereof include siRNA, miRNA, shRNA, antisense RNA, messenger RNA (mRNA), single strand RNA genome, double strand RNA genome, RNA replicon, transfer RNA, ribosomal RNA, and the like. SiRNA, miRNA, shRNA, mRNA, antisense RNA, and RNA replicon are preferred.

The nucleic acid used in the present invention is preferably purified by a method generally used by those of ordinary skill in the art.

The nucleic acid-introducing agent of the present invention containing a nucleic acid can be administered in vivo for the purpose of, for example, prevention and/or treatment of diseases. Therefore, the nucleic acid to be used in the present invention is preferably one having a preventive and/or therapeutic activity against a given disease (prophylactic/therapeutic nucleic acid). Examples of such nucleic acid include nucleic acids used for so-called gene therapy, and the like.

In order to introduce a nucleic acid into a cell by using the nucleic acid-introducing agent of the present invention, the lipid membrane structure of the present invention containing the nucleic acid (i.e., the nucleic acid-introducing agent of the present invention containing the nucleic acid) is formed by achieving the co-presence of the nucleic acid of interest when forming the lipid membrane structure of the present invention. For example, when a liposome is formed as the lipid membrane structure by an ethanol dilution method, an aqueous nucleic acid solution and an ethanol solution of the constituent components (lipid and the like) of the lipid membrane structure of the present invention are vigorously mixed in a vortex, micro flow path, or the like, and the mixture is diluted with an appropriate buffer. When a liposome is formed as the lipid membrane structure by a simple hydration method, the constituent components (lipid and the like) of the lipid membrane structure of the present invention are dissolved in an appropriate organic solvent, and the solution is placed in a glass container and dried under reduced pressure to evaporate the solvent, whereby a lipid thin film is obtained. An aqueous solution of nucleic acid is added thereto, and after hydration, the mixture is subjected to ultrasonic treatment using a sonicator.

One form of the lipid membrane structure of the present invention containing a nucleic acid is LNP encapsulating

the nucleic acid by forming an electrostatic complex between the nucleic acid and a cationic lipid. This LNP can be used as a drug delivery system for selectively delivering a nucleic acid and the like into a particular cell, and is useful for, for example, DNA vaccines by introduction of antigen gene into dendritic cells, gene therapy drugs for tumor, nucleic acid pharmaceutical products that suppress expression of target genes by utilizing RNA interference, and the like.

The particle size of the lipid membrane structure of the present invention containing a nucleic acid is not particularly limited, and is preferably 10 nm to 500 nm, more preferably 30 nm to 300 nm. The particle size can be measured by using a particle size distribution measuring device such as Zetasizer Nano (Malvern) and the like. The particle size of the lipid membrane structure can be appropriately adjusted by the method for preparing the lipid membrane structure.

The surface potential (zeta potential) of the lipid membrane structure of the present invention containing a nucleic acid is not particularly limited and preferably -15 to +15 mV, more preferably -10 to +10 mV. In conventional gene transfer, particles electrically charged to have a plus surface potential have been mainly used. This is useful as a method for promoting electrostatic interactions with negatively-charged heparin sulfate on the cell surface to enhance uptake into cells. However, the positive surface potential may suppress release of nucleic acid from the carrier in the cell due to the interaction with a nucleic acid to be delivered, or protein synthesis due to the interaction between mRNA and a nucleic acid to be delivered. This problem can be solved by adjusting the surface potential to fall within the above-mentioned range. The surface potential can be measured using a zeta potential measuring device such as Zetasizer Nano and the like. The surface potential of the lipid membrane structure can be adjusted by the composition of the constituent component of the lipid membrane structure containing cationic lipid (1).

The lipid membrane surface pKa (hereinafter sometimes to be abbreviated as "Liposomal pKa") of the lipid membrane structure of the present invention is not particularly limited, and the Liposomal pKa is preferably 5.5 to 7.2, further preferably 6.0 to 6.8. Liposomal pKa is used as an index indicating the susceptibility of protonation of a lipid membrane structure which is incorporated by endocytosis in an endosome in a weakly acidic environment inside the endosome. As described in Angewante Chemie International Edition 51: 8529-8533, 2012 or Molecular Therapy 24(4): 786-795, 2016, to escape from an endosome and deliver a nucleic acid into a cytoplasm, it is important to set the Liposomal pKa to a value preferable for the escape from the endosome. By adjusting the Liposomal pKa to fall within the above ranges, a nucleic acid can be efficiently delivered into a cytoplasm. The Liposomal pKa can be appropriately adjusted by the composition of the constituent component of a lipid membrane structure containing cationic lipid (1).

Furthermore, the N/P ratio is one of the indices for evaluating the nucleic acid delivery efficiency of the lipid membrane complex of the present invention. The N/P ratio is the molar ratio of the amino group of the cationic lipid to the phosphate group of the nucleic acid (amino group of cationic lipid/phosphate group of nucleic acid). When the N/P ratio is too low, the lipid membrane complex cannot be formed, resulting in low nucleic acid delivery efficiency, and when the N/P ratio is too high, it can lead to lipid-derived toxicity. Therefore, in order to efficiently deliver nucleic acid into the cytoplasm, it is important to maintain the N/P ratio at a value favorable for nucleic acid delivery. For the lipid membrane complex of the present invention, the N/P ratio is preferably in the range of 7 or more to 130 or less.

One of the indices for evaluating the nucleic acid delivery efficiency of the lipid membrane complex of the present invention is the membrane fusion ability with an endosomal membrane. There are no particular limitations on the method for evaluating membrane fusion ability, and examples thereof include hemolysis. As described in Proceedings of the National Academy of Science 110(32): 12881-12886, 2013, hemolysis is one of the means by which lipid membrane structure taken up by endocytosis escapes from endosomes and is an index for evaluating membrane fusion ability. Higher hemolytic activity enables more efficient delivery of nucleic acids into the cytoplasm. However, when hemolytic activity is present at physiological pH, nucleic acids may be delivered to unintended cells during blood retention, also leading to reduced targeting property and toxicity.

The hemolytic activity of the lipid membrane structures of the present invention is not particularly limited, but the hemolytic activity is preferably less than 5% at physiological pH (pH 7.4) and 5% or more in the weakly acidic environment (pH 5.5) within the endosome. The hemolysis activity can be adjusted by the composition of the constituent component of the lipid membrane structure containing cationic lipid (1).

By contacting a cell with the nucleic acid-introducing agent of the present invention containing a nucleic acid (preferably the lipid membrane structure of the present invention containing nucleic acid), the nucleic acid contained in the aforementioned nucleic acid-introducing agent can be introduced into the aforementioned cell. Therefore, the present invention also provides a method for introducing the nucleic acid contained in the nucleic acid-introducing agent into the cell, which includes contacting the cell with the nucleic acid-introducing agent of the present invention containing the nucleic acid.

The kind of the aforementioned cell is not particularly limited, and cells of prokaryote and eukaryote can be used. Preferred is eucaryote. The kind of the eucaryote is also not particularly limited, and examples thereof include vertebrates such as mammals including human (e.g., human, monkey, mouse, rat, hamster, bovine, etc.), birds (e.g., chicken, ostrich, etc.), amphibia (e.g., frog and the like), fishes (e.g., zebrafish, rice-fish, etc.) and the like, invertebrates such as insects (silk moth, moth, Drosophila, etc.), and the like, plants, microorganisms (e.g., yeasts), and the like. The cell to be the target in the present invention is more preferably an animal or plant cell, further preferably a mammalian cell. The cell may be a culture

cell line including a cancer cell, or a cell isolated from an individual or tissue, or a cell of a tissue or tissue piece. The cell may be an adherent cell or a non-adherent cell.

The step of contacting the lipid membrane structure of the present invention encapsulating a nucleic acid with a cell in vitro is specifically described below.

Cells are suspended in a suitable medium several days before contact with the lipid membrane structure, and cultured under appropriate conditions. At the time of contact with the lipid membrane structure, the cells may or may not be in a proliferative phase.

The culture medium at the time of the contact may be a serum-containing medium or a serum-free medium. The serum concentration of the medium is preferably not more than 30 wt%, more preferably not more than 20 wt%. When the medium contains excess proteins such as serum and the like, the contact between the lipid membrane structure and the cell may be inhibited.

The cell density at the time of the contact is not particularly limited, and can be appropriately determined in consideration of the kind of the cell and the like. It is generally within the range of $1\times10^4$ to $1\times10^7$ cells/mL.

For example, a suspension of the lipid membrane structure of the present invention containing a nucleic acid (i.e., the nucleic acid-introducing agent of the present invention containing a nucleic acid) is added to cells. The amount of the suspension to be added is not particularly limited, and can be appropriately determined in consideration of the cell number and the like. The concentration of the lipid membrane structure when contacting cells is not particularly limited as long as the desired introduction of the nucleic acid into the cells can be achieved. The lipid concentration is generally 1 to 100 nmol/ml, preferably 10 to 50 nmol/ml, and the nucleic acid concentration is generally 0.01 to 100 μg/ml, preferably 0.1 to 10 μg/ml.

After the aforementioned suspension is added to cells, the cells are cultured. The temperature, humidity, $CO_2$ concentration, and the like during culturing are appropriately determined in consideration of the kind of the cell. When the cell is derived from a mammal, generally, the temperature is about 37°C, the humidity is about 95%, and the $CO_2$ concentration is about 5 vol%. While the culture time can also be appropriately determined in consideration of the conditions such as the kind of the cell and the like, it is generally a range of 0.1 to 76 hr, preferably a range of 0.2 to 24 hr, more preferably a range of 0.5 to 12 hr. When the above-mentioned culture time is too short, the nucleic acid is not sufficiently introduced into the cells, and when the culture time is too long, the cells may become weak.

By the above-mentioned culture, a nucleic acid is introduced into cells. The culture is further continued preferably by exchanging the medium with a fresh medium, or adding a fresh medium to the medium. When the cell is a mammal-derived cell, the fresh medium preferably contains a serum or nutrition factor.

As mentioned above, a nucleic acid can be introduced into cells not only in vitro but also in vivo by using the nucleic acid-introducing agent of the present invention containing a nucleic acid. That is, by administration of the nucleic acid-introducing agent containing a nucleic acid to a living organism, the nucleic acid contained in the nucleic acid-introducing agent can be introduced into the target cells in the aforementioned living organism. Therefore, the present invention also provides a method for introducing a nucleic acid contained in a nucleic acid-introducing agent into a target cell in a living organism, comprising administering the nucleic acid-introducing agent of the present invention containing the nucleic acid to the living organism.

The living organisms to which the nucleic acid-introducing agent of the present invention containing a nucleic acid can be administered are not particularly limited, and examples thereof include vertebrates such as mammals (e.g., human, monkey, mouse, rat, hamster, bovine, etc.), birds (e.g., chicken, ostrich, etc.), amphibia (e.g., frog and the like), fishes (e.g., zebrafish, rice-fish, etc.), and the like, invertebrates such as insects (e.g., silk moth, moth, Drosophila, etc.) and the like, plants, and the like. The living organisms to which the nucleic acid-introducing agent of the present invention containing a nucleic acid can be administered are preferably humans or other mammals.

The kind of the target cell is not particularly limited, and a nucleic acid can be introduced into cells in various tissues (e.g., liver, kidney, pancreas, lung, spleen, heart, blood, muscle, bone, brain, stomach, small intestine, large intestine, skin, adipose tissue, lymph node, tumor, etc.) by using the nucleic acid-introducing agent of the present invention containing a nucleic acid.

The method of administering the nucleic acid-introducing agent of the present invention containing a nucleic acid to a living organism is not particularly limited, and an administration method known per se (e.g., oral administration, parenteral administration (e.g., intravenous administration, intramuscular administration, topical administration, transdermal administration, subcutaneous administration, intraperitoneal administration, spray, etc.), etc.) can be appropriately selected. The dose of the nucleic acid-introducing agent of the present invention containing a nucleic acid is not particularly limited, and can be appropriately selected taking into account the kind of living organism to be the subject of administration, the administration method, the type and site of target cells, and the like.

When cationic lipid (1) or lipid membrane structure is used as the nucleic acid-introducing agent, it can be formulated according to a conventional method.

When the nucleic acid-introducing agent of the present invention, which is the lipid membrane structure of the present invention, is provided as a reagent for studies, it can be provided as is, or as a sterile solution or suspension of the

lipid membrane structure of the present invention in, for example, water or another physiologically acceptable liquid (e.g., water-soluble solvent (e.g., malic acid buffer solution, etc.), an organic solvent (e.g., ethanol, methanol, DMSO, tert-butanol, etc.), or a mixture of a water-soluble solvent and an organic solvent, etc.). The nucleic acid-introducing agent of the present invention may appropriately contain physiologically acceptable additives (e.g., excipient, vehicle, preservative, stabilizer, binder, etc.), which are known per se.

Furthermore, when the nucleic acid-introducing agent of the present invention, which is the lipid membrane structure of the present invention, is provided as a medicament, the lipid membrane structure of the present invention can be used as is, or the lipid membrane structure of the present invention can be used together with known pharmaceutically acceptable additives (e.g., carrier, flavor, excipient, vehicle, preservative, stabilizer, binder, etc.) and mixed in a unit dosage form required for generally accepted pharmaceutical practice, to produce the nucleic acid-introducing agent of the present invention as an oral agent (e.g., tablet, capsule, etc.) or parenteral agent (e.g., injectable preparation, spray, etc.), preferably parenteral agent (more preferably, injectable preparation).

The nucleic acid-introducing agent of the present invention can also be provided in the form of a kit. The kit can contain, in addition to cationic lipid (1) or the lipid membrane structure of the present invention, a reagent used for the introduction of a nucleic acid. In one embodiment, the nucleic acid-introducing agent (or kit) of the present invention further contains a polycation (e.g., protamine). Using the nucleic acid-introducing agent (or kit) of the present invention in this embodiment, an electrostatic complex between a nucleic acid and a polycation (e.g., protamine) can be encapsulated easily in the lipid membrane structure of the present invention, whereby the nucleic acid can be introduced into cells.

The present invention also provides a pharmaceutical composition containing cationic lipid (1). The pharmaceutical composition of the present invention may further contain a nucleic acid. The pharmaceutical composition of the present invention may also contain known pharmaceutically acceptable additives (e.g., carrier, flavor, excipient, vehicle, preservative, stabilizer, binder, etc.).

The pharmaceutical composition of the present invention may be a powder composition obtained by removing the solvent by lyophilization or the like, or a liquid composition. A powder composition may be produced by removing the solvent from a liquid composition by filtration, centrifugation, or the like, or by lyophilizing a liquid composition.

The pharmaceutical composition of the present invention can be formulated as an oral preparation (e.g., tablet, capsule, etc.) or a parenteral preparation (e.g., 「injectable preparation, spray, etc.), preferably a parenteral preparation (more preferably an 「injectable preparation). The pharmaceutical composition of the present invention can be formulated not only for adults but also for children.

The present invention also provides a method for producing a cellular medicine containing cells expressing a gene in a nucleic acid, the method including contacting cells with the nucleic acid-introducing agent of the present invention containing the nucleic acid and introducing the nucleic acid contained in the aforementioned nucleic acid-introducing agent into the cell.

The aforementioned cell expressing the gene in the nucleic acid refers to a cell in which a gene of interest has been expressed by introducing the nucleic acid into the cell.

The nucleic acid-introducing agent of the present invention containing a nucleic acid in vitro is brought into contact with cells to introduce the aforementioned nucleic acid into the cells.

Examples of the cell to be used in the production of cellular medicines include T cell, B cell, NK cell, dendritic cell, macrophage, monocyte, and the like. T cells to be used in the production of cellular medicines may be T cells induced to differentiate from lymphocyte precursor cells, including pluripotent cells. Examples of the lymphocyte precursor cell, including pluripotent cell, include embryonic stem cell (ES cell), induced pluripotent stem cell (iPS cell), and the like. Undifferentiated cells, such as pluripotent cell, can be differentiated into T cells by a known method.

Examples of nucleic acid to be used in the production of cellular medicine include nucleic acids encoding chimeric antigen receptors (CARs) and T cell receptors (TCRs).

The nucleic acid encoding the CAR to be used in the production of a cellular medicine contains an antigen-binding domain of an antibody capable of specifically recognizing the surface antigen to be recognized by the target immune cell, an extracellular hinge domain, a transmembrane domain, and an intracellular T cell signaling domain.

The TCR-encoding nucleic acid to be used in the production of cellular medicines encodes the $\alpha$ chain and $\beta$ chain of TCR capable of specifically recognizing the surface antigen to be recognized by the target T cell.

The nucleic acid encoding CAR or TCR is not particularly limited, and examples thereof include DNA, RNA, chimera nucleic acid of RNA, DNA/RNA hybrid, and the like.

Cellular medicine contains a cell expressing a specific gene and may further contain pharmaceutically acceptable additives (e.g., carrier, excipient, vehicle, preservative, stabilizer, etc.). Cellular medicine is preferably a parenteral preparation, more preferably an injectable preparation.

The cellular medicine can be used for the treatment or prophylaxis of diseases such as cancer and the like. The cancer to be the application target of the cellular medicine is not particularly limited, and examples thereof include lung cancer, breast cancer, gastric cancer, colon cancer, uterine cancer, ovarian cancer, osteosarcoma, chondrosarcoma, rhabdomyosarcoma, leiomyosarcoma, fibrosarcoma, liposarcoma, angiosarcoma, leukemia, malignant lymphoma,

myeloma, and the like.

The subject to which the cellular medicine can be administered is not particularly limited, and examples thereof include mammals (e.g., human, monkey, mouse, rat, hamster, bovine, etc.) and the like. The subject of administration of the cellular medicine is preferably human or other mammals.

The method of administering a cellular medicine is not particularly limited as long as it allows the cells to express the target gene, and parenteral administration (e.g., intravenous administration, intramuscular administration, topical administration, transdermal administration, subcutaneous administration, intraperitoneal administration, spray, and the like), and the like can be appropriately selected in consideration of the kind of the cell, target disease, and the like. The dose of the cellular medicine is not particularly limited as long as the cell can express the target gene, and can be appropriately selected in consideration of the kind of the subject of administration, the administration method, the kind of the cell, target disease, and the like.

[Example]

The present invention is explained in more detail in the following by referring to Examples and Experimental Examples, but the present invention is not limited by these.

The abbreviations used in the description of Examples each mean the following.

Chol: cholesterol
DMAP: 4-dimethylaminopyridine
DMF: N,N-dimethylformamide
DMG-PEG2k: 1,2-dimyristoyl-rac-glycero-3-methoxypolyethylene glycol (number average molecular weight of PEG chain: 2000)
DMSO: dimethyl sulfoxide
DOPC: 1,2-dioleoyl-sn-glycero-3-phosphocholine
DOPE: 1,2-dioleoyl-sn-glycero-3-phosphoethanolamine
DSC: N,N'-disuccinimidyl carbonate
DSG-PEG5k: 1,2-distearoyl-rac-glycero-3-methoxypolyethylene glycol (number average molecular weight of PEG chain: 5000)
EDC hydrochloride: 1-ethyl-3-(3-dimethylaminopropyl)carbodiimide hydrochloride
LNP: lipid nano particle
Me: methyl
MES: 2-morpholinoethanesulfonic acid
mRNA: messenger RNA
MsOH: mesylic acid
PBS: phosphate buffered saline
siRNA: small interfering RNA
TBAF: tetra-n-butylammonium fluoride
TBDPS: tert-butyldiphenylsilyl
TBDPS-Cl: tert-butyldiphenylsilyl chloride
TBS: tert-butyldimethylsilyl
TBS-Cl: tert-butyldimethylsilyl chloride
tBu: tert-butyl
THF: tetrahydrofuran
THP: 3,4-dihydro-2H-pyran
TNS: sodium 6-(p-toluidino)-2-naphthalenesulfonate

Table 1-1 to Table 1-31 show the names and structures of the cationic lipids produced in the following Examples. In addition, Table 2 shows the structures of the cationic lipids of Comparative Examples. The cationic lipids of Comparative Examples were produced according to the production methods of WO 2016/121942. Table 3-1 to Table 3-9 show the names and structures of the intermediates used in the production of the cationic lipids of Examples.

[Table 1-1]

| name | structure | $R^{3a}$ | $R^{3b}$ |
|---|---|---|---|
| compound 1 | 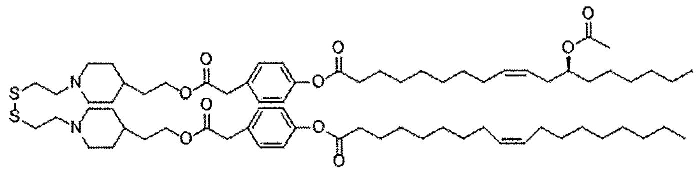 | $R^{3a}$: formula (15)<br>$R^{35}$: methyl | $R^{3b}$: (Z)-8-heptadecenyl |
| compound 2 | 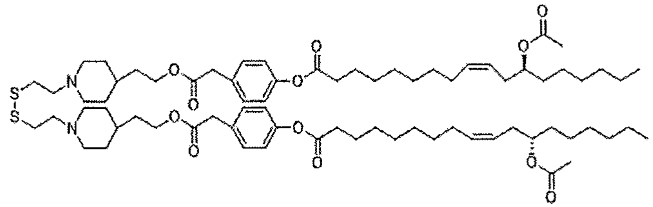 | $R^{3a}$: formula (15)<br>$R^{35}$: methyl | $R^{3b}$: formula (15)<br>$R^{35}$: methyl |
| compound 3 | 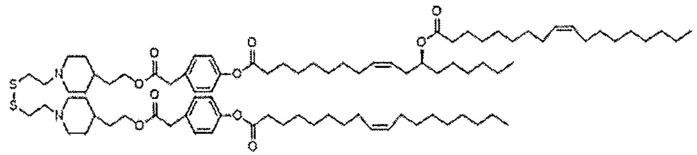 | $R^{3a}$: formula (15)<br>$R^{35}$: (Z)-8-heptadecenyl | $R^{3b}$: (Z)-8-heptadecenyl |
| compound 4 | 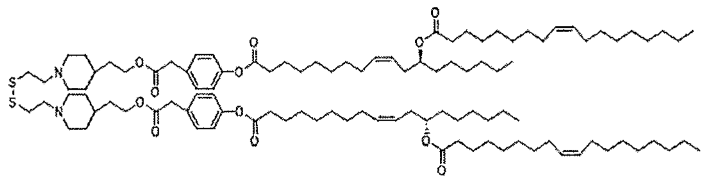 | $R^{3a}$: formula (15)<br>$R^{35}$: (Z)-8-heptadecenyl | $R^{3b}$: formula (15)<br>$R^{35}$: (Z)-8-heptadecenyl |
| compound 5 | 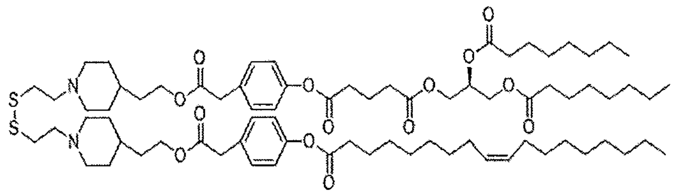 | $R^{3a}$: formula (16-20)<br>$X^4$: O<br>$R^{36}$: trimethylene<br>$R^{49}$: heptyl<br>$R^{50}$: heptyl | $R^{3b}$: (Z)-8-heptadecenyl |

(continued)

| name | structure | $R^{3a}$ | $R^{3b}$ |
|---|---|---|---|
| compound 6 | 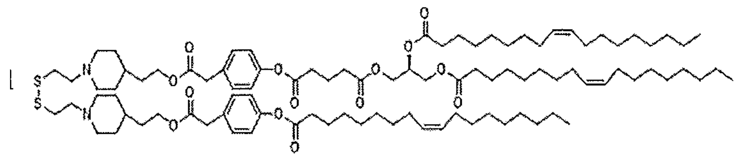 | $R^{3a}$: formula (16-20)<br>$X^4$: O<br>$R^{36}$: trimethylene<br>$R^{49}$: (Z)-8-heptadecenyl<br>$R^{50}$: (Z)-8-heptadecenyl | $R^{3b}$: (Z)-8-heptadecenyl |
| com-pounc 7 | 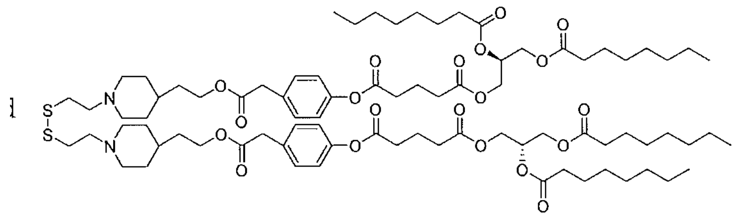 | $R^{3a}$: formula (16-20)<br>$X^4$: O<br>$R^{36}$: trimethylene<br>$R^{49}$: heptyl<br>$R^{50}$: heptyl | $R^{3b}$: formula (16-20)<br>$X^4$: O<br>$R^{36}$: trimethylene<br>$R^{49}$: heptyl<br>$R^{50}$ : heptyl |

[Table 1-2]

| name | structure | $R^{3a}$ | $R^{3b}$ |
|---|---|---|---|
| compound 8 | 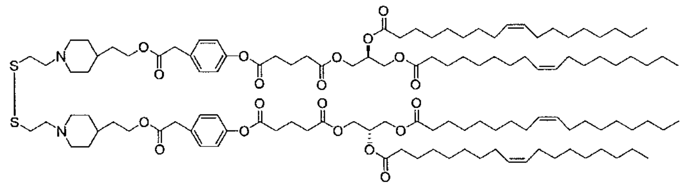 | $R^{3a}$: formula (16-20)<br>$X^4$: O<br>$R^{36}$: trimethylene<br>$R^{49}$: (Z)-8-heptadecenyl<br>$R^{50}$: (Z)-8-heptadecenyl | $R^{3b}$ : formula (16-20)<br>$X^4$: O<br>$R^{36}$: trimethylene<br>$R^{49}$: (Z)-8-heptadecenyl<br>$R^{50}$: (Z)-8-heptadecenyl |
| compound 9 | 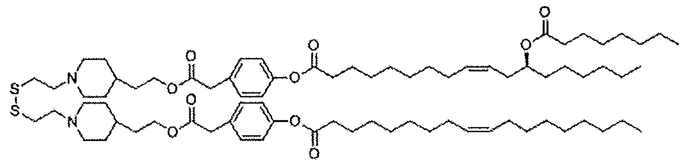 | $R^{3a}$: formula (15)<br>$R^{35}$: heptyl | $R^{3b}$: (Z) -8-heptadecenyl |
| compound 10 | 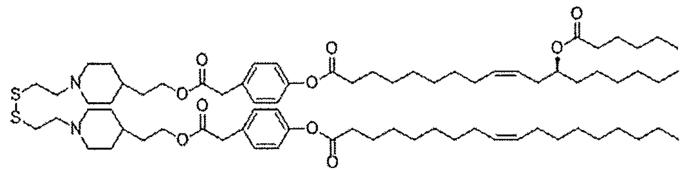 | $R^{3a}$: formula (15)<br>$R^{35}$: pentyl | $R^{3b}$: (Z)-8-heptadecenyl |
| compound 11 | 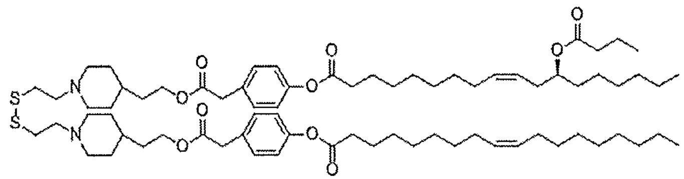 | $R^{3a}$: formula (15)<br>$R^{35}$: propyl | $R^{3b}$: (Z)-8-heptadecenyl |
| compound 12 | 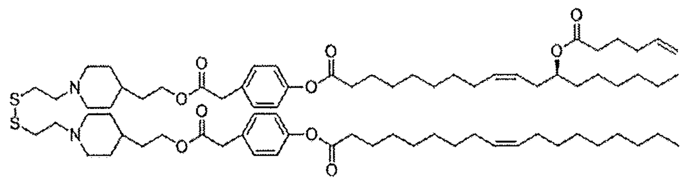 | $R^{3a}$: formula (15)<br>$R^{35}$: 4-pentenyl | $R^{3b}$: (Z)-8-heptadecenyl |
| compound 13 | 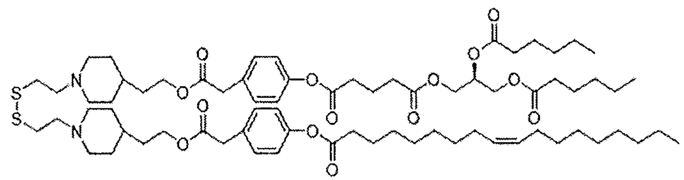 | $R^{3a}$: formula (16-20)<br>$X^4$: O<br>$R^{36}$: trimethylene<br>$R^{49}$: pentyl<br>$R^{59}$: pentyl | $R^{3b}$: (Z)-8-heptadecenyl |

(continued)

| name | structure | $R^{3a}$ | $R^{3b}$ |
|---|---|---|---|
| compound 14 | 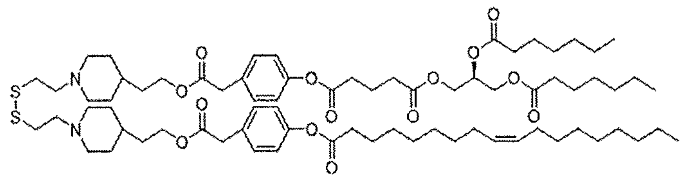 | $R^{3a}$: formula (16-20)<br>$X^4$: O<br>$R^{36}$: trimethylene<br>$R^{49}$: hexyl<br>$R^{50}$: hexyl | $R^{3b}$: (Z) -8-heptadecenyl |

[Table 1-3]

| name | structure | $R^{3a}$ | $R^{3b}$ |
| --- | --- | --- | --- |
| com-pound 15 | 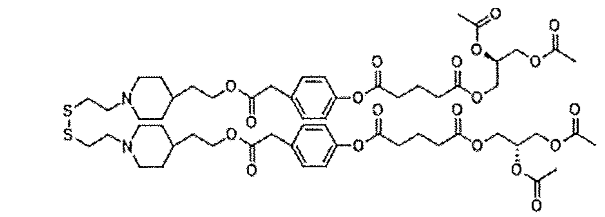 | $R^{3a}$: formula (16-20)<br>$X^4$: O<br>$R^{36}$: trimethy-lene<br>$R^{49}$: methyl<br>$R^{50}$: methyl | $R^{3b}$: formula (16-20)<br>$X^4$: O<br>$R^{36}$: trimeth-ylene<br>$R^{49}$: methyl<br>$R^{50}$: methyl |
| com-pound 16 | 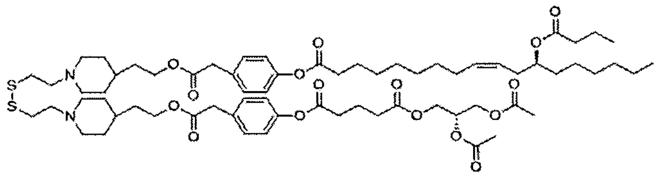 | $R^{3a}$: formula (15)<br>$R^{35}$: propyl | $R^{3b}$: formula (16-20)<br>$X^4$: O<br>$R^{36}$: tri-methylene<br>$R^{49}$: methyl<br>$R^{50}$: methyl |
| com-pound 17 | 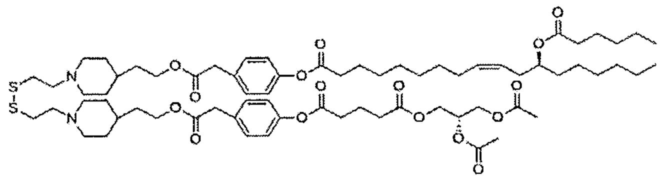 | $R^{3a}$: formula (15)<br>$R^{35}$: pentyl | $R^{3b}$: formula (16-20)<br>$X^4$: O<br>$R^{36}$: trimethylene<br>$R^{49}$: methyl<br>$R^{50}$: methyl |
| com-pound 18 | 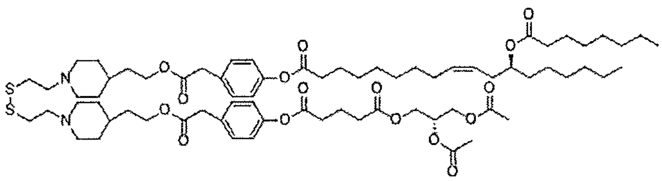 | $R^{3a}$: formula (15)<br>$R^{35}$: heptyl | $R^{3b}$: formula (16-20)<br>$X^4$: O<br>$R^{36}$: trimethylene<br>$R^{49}$: methyl<br>$R^{50}$: methyl |
| com-pound 19 | 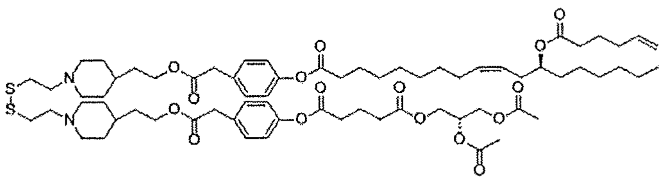 | $R^{3a}$: formula (15)<br>$R^{35}$: 4-pentenyl | $R^{3b}$: formula (16-20)<br>$X^4$: O<br>$R^{36}$: trimethylene<br>$R^{49}$: methyl<br>$R^{50}$: methyl |
| com-pound 20 | 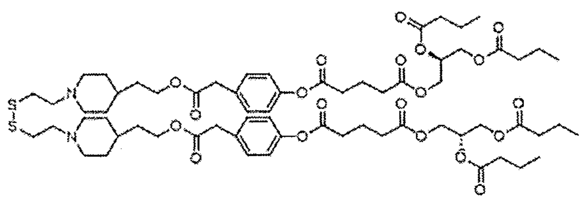 | $R^{3a}$: formula (16-20)<br>$X^4$: O<br>$R^{36}$: trimethy-lene<br>$R^{49}$: propyl<br>$R^{50}$: propyl | $R^{3b}$: formula (16-20)<br>$X^4$: O<br>$R^{36}$: trimethylene<br>$R^{49}$: propyl<br>$R^{50}$: propyl |
| com-pound 21 | 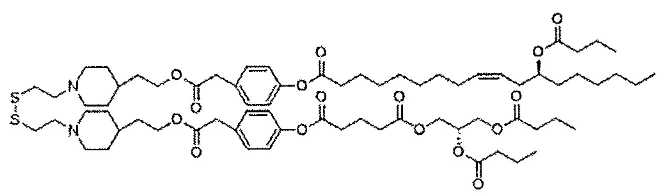 | $R^{3a}$: formula (15)<br>$R^{35}$: propyl | $R^{3b}$: formula (16-20)<br>$X^4$: O<br>$R^{36}$: trimethylene<br>$R^{49}$: propyl<br>$R^{50}$: propyl |

[Table 1-4]

| name | structure | $R^{3a}$ | $R^{3b}$ |
|---|---|---|---|
| com-pound 22 | 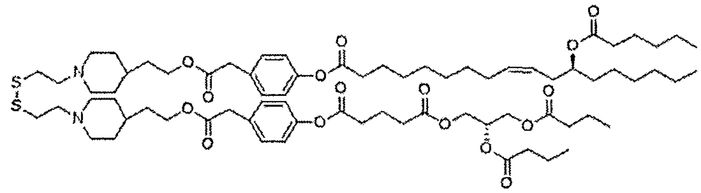 | $R^{3a}$ : formula (15)<br>$R^{35}$: pentyl | $R^{3b}$: formula (16-20)<br>$X^4$: O<br>$R^{36}$: trimethylene<br>$R^{49}$: propyl<br>$R^{50}$: propyl |
| com-pound 23 | 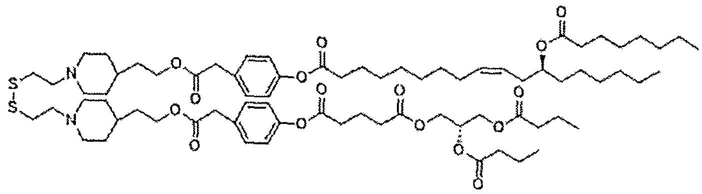 | $R^{3a}$: formula (15)<br>$R^{35}$: heptyl | $R^{3b}$: formula (16-20)<br>$X^4$: O<br>$R^{36}$: trimethylene<br>$R^{49}$: propyl<br>$R^{50}$: propyl |
| com-pound 24 | 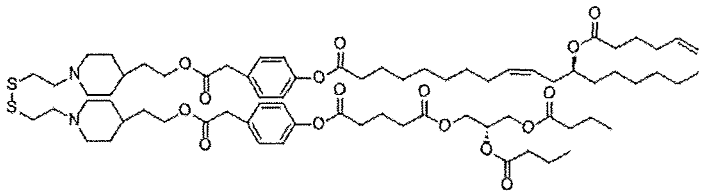 | $R^{3a}$: formula (15)<br>$R^{35}$: 4-pente-nyl | $R^{3b}$: formula (16-20)<br>$X^4$: O<br>$R^{36}$: trimethylene<br>$R^{49}$: propyl<br>$R^{50}$: propyl |
| com-pound 25 | 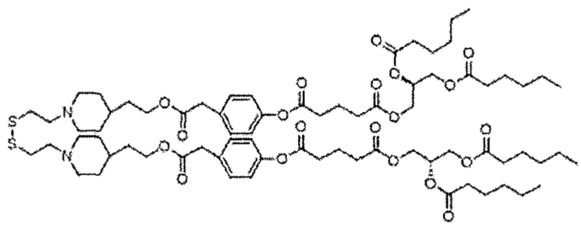 | $R^{3a}$: formula (16-20)<br>$X^4$: O<br>$R^{36}$: trimethy-lene<br>$R^{49}$: pentyl<br>$R^{50}$: pentyl | $R^{3b}$: formula<br>(16-20) $X^4$: O<br>$R^{36}$: trimethylene<br>$R^{49}$: pentyl<br>$R^{50}$: pentyl |
| com-pound 26 | 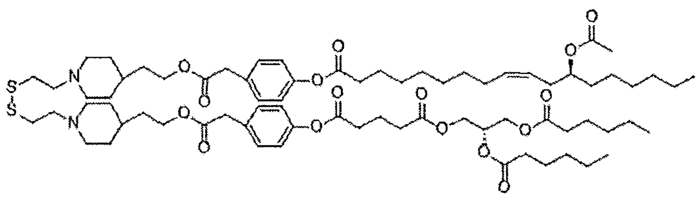 | $R^{3a}$: formula (15)<br>$R^{35}$: methyl | $R^{3b}$: formula (16-20)<br>$X^4$: O<br>$R^{36}$: trimethylene<br>$R^{49}$: pentyl<br>$R^{50}$: pentyl |
| com-pound 27 | 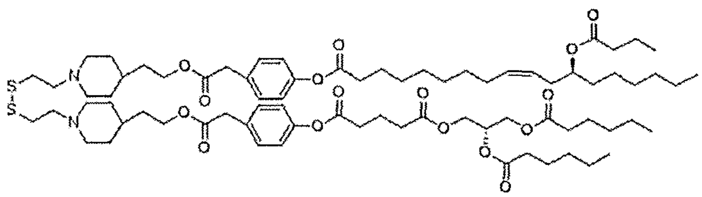 | $R^{3a}$: formula (15)<br>$R^{35}$: propyl | $R^{3b}$ : formula (16-20)<br>$X^4$: O<br>$R^{36}$: trimethylene<br>$R^{49}$: pentyl<br>$R^{50}$: pentyl |
| com-pound 28 | 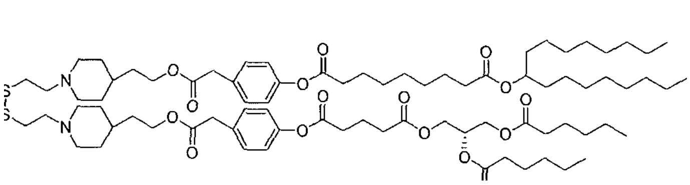 | $R^{3a}$: formula (16-20)<br>$X^4$: O<br>$R^{36}$: tri-methylene<br>$R^{49}$: pentyl<br>$R^{50}$: pentyl | $R^{3b}$ : derived from intermedi-ate 53 |

[Table 1-5]

| name | structure | $R^{3a}$ | $R^{3b}$ |
|---|---|---|---|
| com-pound 29 | 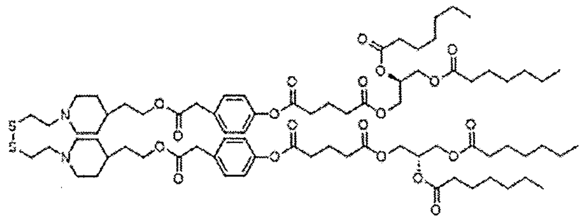 | $R^{3a}$: formula (16-20) O $R^{36}$: tri-methylene $R^{49}$: hexyl $R^{50}$: hexyl | $R^{3b}$: formula (16-20) $X^4$: O $R^{36}$: trimethylene $R^{49}$: hexyl $R^{50}$: hexyl |
| com-pound 30 | 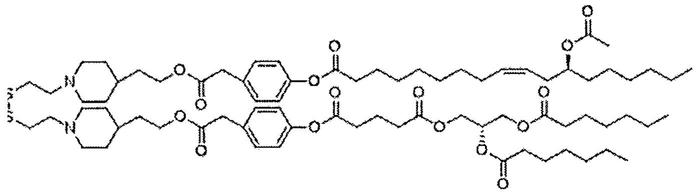 | $R^{3a}$: formula (15) $R^{35}$: methyl | $R^{3b}$: formula (16-20) $X^4$: O $R^{36}$: trimeth-ylene $R^{49}$: hexyl $R^{50}$: hexyl |
| com-pound 31 | 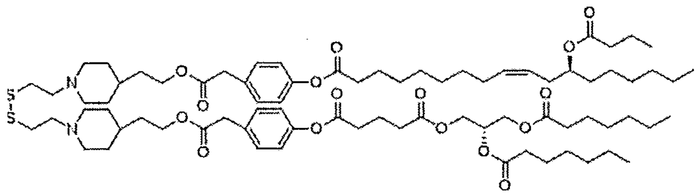 | $R^{3a}$: formula (15) $R^{35}$: propyl | $R^{3b}$: formula (16-20) $X^4$: O $R^{36}$: trimeth-ylene $R^{49}$: hexyl $R^{50}$: hexyl |
| com-pound 32 | 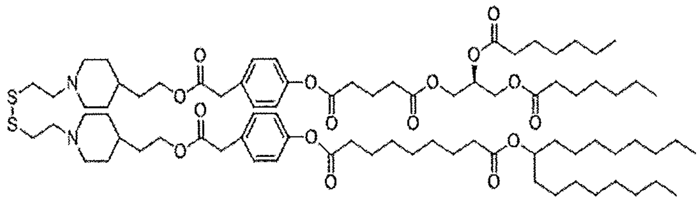 | $R^{3a}$: formula (16-20) $X^4$: O $R^{36}$: trimethy-lene $R^{49}$: hexyl $R^{50}$: hexyl | $R^{3b}$: derived from intermediate 53 |
| com-pound 33 | 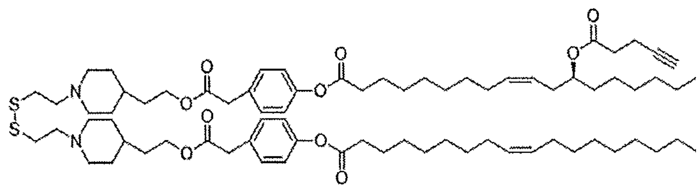 | $R^{3a}$: formula (15) $R^{35}$: 3-butynyl | $R^{3b}$: (Z)-8-heptade-cenyl |
| com-pound 34 | 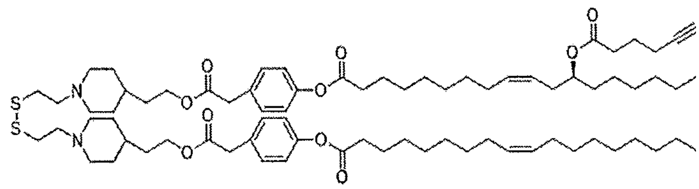 | $R^{3a}$: formula (15) $R^{35}$: 4-penty-nyl | $R^{3b}$: (Z)-8-heptade-cenyl |
| com-pound 35 | 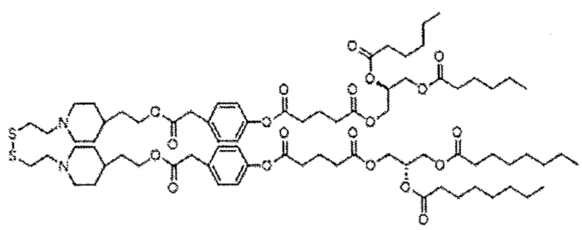 | $R^{3a}$: formula (16-20) $X^4$: O $R^{36}$: trimethy-lene $R^{49}$: pentyl $R^{50}$: pentyl | $R^{3b}$: formula (16-20) $X^4$: O $R^{36}$: trimethylene $R^{49}$: heptyl $R^{50}$: heptyl |

[Table 1-6]

| name | structure | $R^{3a}$ | $R^{3b}$ |
|---|---|---|---|
| compound 36 | 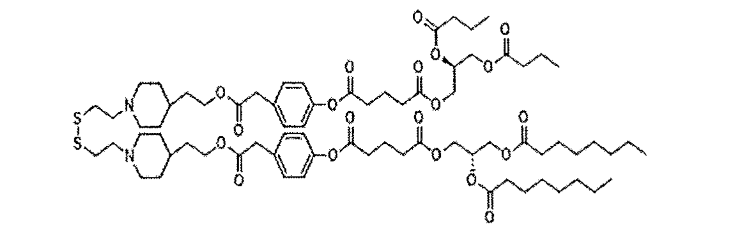 | $R^{3a}$: formula (16-20)<br>$X^4$: O<br>$R^{36}$: trinethylene<br>$R^{49}$: propyl<br>$R^{50}$: propyl | $R^{3b}$: formula (16-20)<br>$X^4$: O<br>$R^{36}$: trimethylene<br>$R^{49}$: heptyl<br>$R^{50}$: heptyl |
| compound 37 | 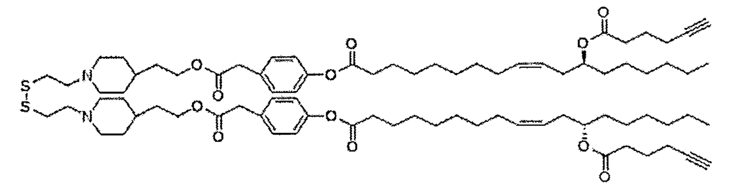 | $R^{3a}$: formula (15)<br>$R^{35}$: 4-pentynyl | $R^{3b}$: formula (15)<br>$R^{35}$: 4-pentynyl |
| compound 38 | 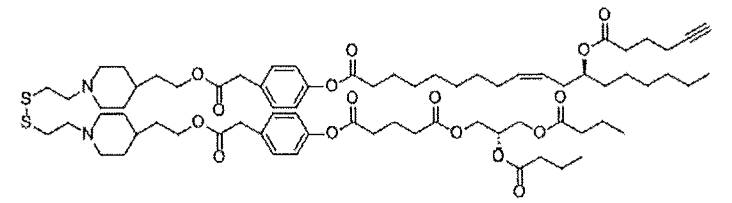 | $R^{3a}$: formula (15)<br>$R^{35}$: 4-pentynyl | $R^{3b}$: formula (16-20)<br>$X^4$: O<br>$R^{36}$: trimethylene<br>$R^{49}$: propyl<br>$R^{50}$: propyl |
| compound 39 | 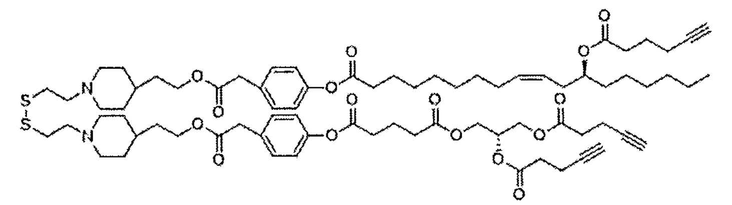 | $R^{3a}$: formula (15)<br>$R^{35}$: 4-pentynyl | $R^{3b}$: formula (16-20)<br>$X^4$: O<br>$R^{36}$: trimethylene<br>$R^{49}$: 3-butynyl<br>$R^{50}$: 3-butynyl |
| compound 40 | 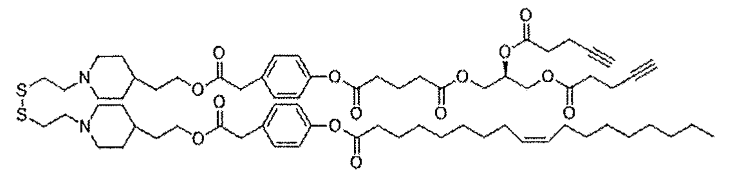 | $R^{3a}$: formula (16-20)<br>$X^4$: O<br>$R^{36}$: trimethylene<br>$R^{49}$: 3-butynyl<br>$R^{50}$: 3-butynyl | $R^{3b}$: (Z)-8-heptadecenyl |
| compound 41 | 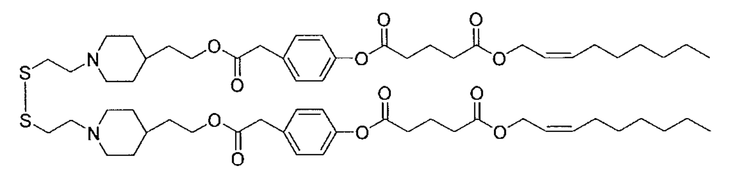 | $R^{3a}$: formula (14)<br>$X^3$: O<br>$R^{33}$: trimethylene<br>$R^{34}$: (Z)-2-nonenyl | $R^{3b}$: formula (14)<br>$X^3$: O<br>$R^{36}$: tri-methylene<br>$R^{34}$: (Z)-2-nonenyl |
| compound 42 | 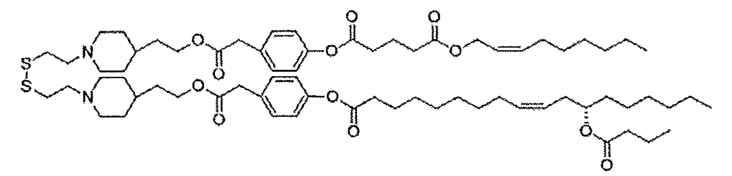 | $R^{3a}$ : formula (15)<br>$R^{35}$: propyl | $R^{3b}$: formula (14)<br>$X^3$: O<br>$R^{33}$: tri-methylene<br>$R^{34}$: (Z)-2-nonenyl |

[Table 1-7]

| name | structure | $R^{3a}$ | $R^{3b}$ |
|---|---|---|---|
| com- pound 43 | 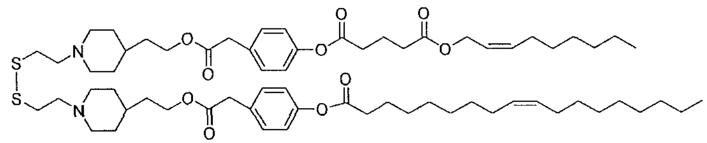 | $R^{3a}$: formula 14)<br>$X^3$: O<br>$R^{33}$: trimethylene<br>$R^{34}$: (Z)-2-ionenyl | $R^{3b}$: (Z)-8-heptadecenyl |
| com- pound 44 | 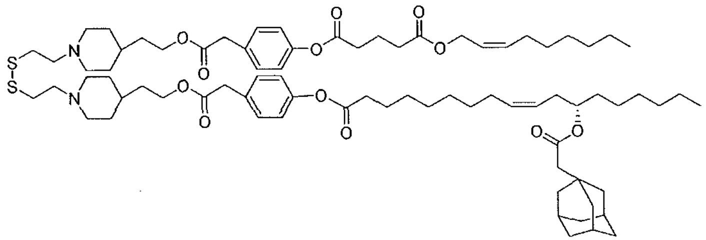 | $R^{3a}$: formula 15)<br>$R^{35}$: 1-adamantylmethyl | $R^{3b}$ : formula (14)<br>$X^3$: O<br>$R^{33}$: trimethylene<br>$R^{34}$: (Z)-2-nonenyl |
| com- pound 45 | 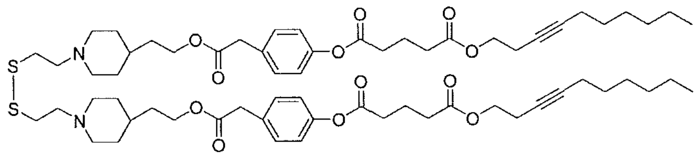 | $R^{3a}$: formula (14)<br>$X^3$: O<br>$R^{33}$: trimethylene<br>$R^{34}$: 3-decynyl | $R^{3b}$ : formula (14)<br>$X^3$: O<br>$R^{33}$: tri-methylene<br>$R^{34}$: 3-decynyl |
| com- pound 46 | 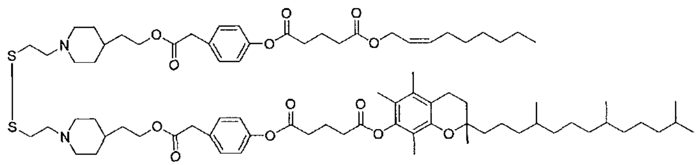 | $R^{3a}$: formula (14)<br>$X^3$: O<br>$R^{33}$: trimethylene<br>$R^{34}$: (Z)-2-nonenyl | $R^{3b}$: $^{R3c}$-CO- $(CH_2)_p$-<br>p:3<br>$R^{3c}$:tocopherol residue |
| com- pound 47 | 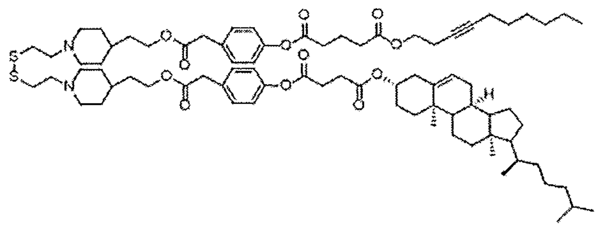 | $R^{3a}$: formula (14)<br>$X^3$: O<br>$R^{33}$: trimethylene<br>$R^{34}$: 3-decynyl | $R^{3b}$: $R^{3c}$-CO- $(CH_2)_p$-<br>p: 2<br>$R^{3c}$: chole-sterolresidue |

(continued)

| name | structure | $R^{3a}$ | $R^{3b}$ |
|---|---|---|---|
| com- pound 48 | 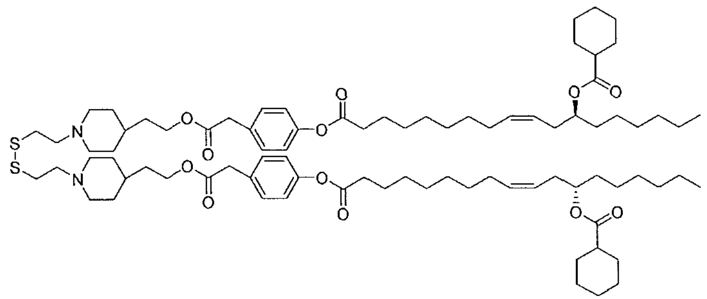 | $R^{3a}$: formula (15)<br>$R^{35}$: cyclohexyl | $R^{3b}$ : formula(15)<br>$R^{35}$: cyclohexyl |
| com- pound 49 | 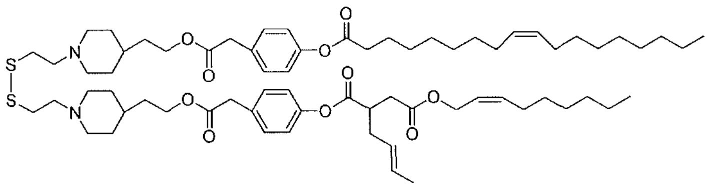 | $R^{3a}$: formula (14)<br>$R^{33}$: 1-((E)- 2-butenyl)-ethylene<br>$R^{34}$: (Z)-2-nonenyl | $R^{3b}$: (Z)-8-heptadecenyl |

[Table 1-8]

| name | structure | $R^{3a}$ | $R^{3b}$ |
|---|---|---|---|
| com- pound 50 | 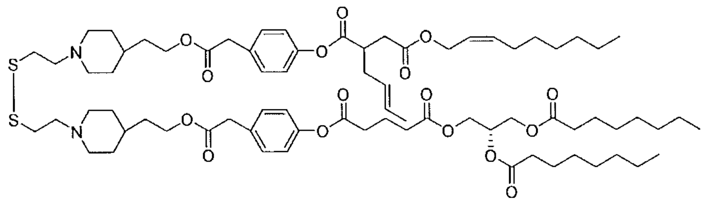 | $R^{3a}$: formula (14)<br>$R^{33}$: 1-((E)-2-butenyl) - ethylene<br>$R^{34}$: (Z)-2-nonenyl | $R^{3b}$ : formula(16-20)<br>$X^4$: O<br>$R^{36}$: tri-methylene<br>$R^{49}$: heptyl<br>$R^{50}$: heptyl |
| com- pound 51 | 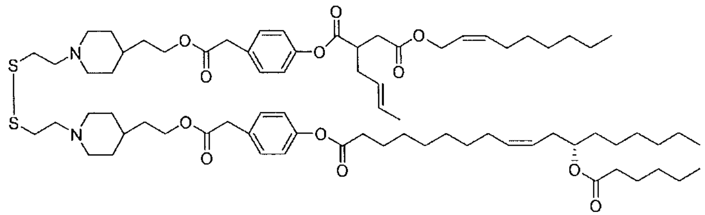 | $R^{3a}$: formula (14)<br>$R^{33}$: 1-((E)-2-butenyl)-ethylene<br>$R^{34}$: (Z)-2-nonenyl | $R^{3b}$:<br>formula (15)<br>$R^{35}$: pentyl |
| com- pound 52 | 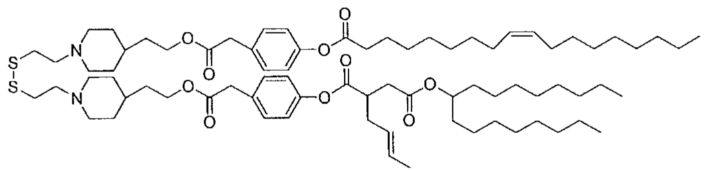 | $R^{3a}$: formula (14)<br>$R^{33}$: 1- ((E)-2-butenyl)-ethylene<br>$R^{34}$: (1-octyl)nonyl | $R^{3b}$: (Z)-8-heptadecenyl |
| com- pound 53 | 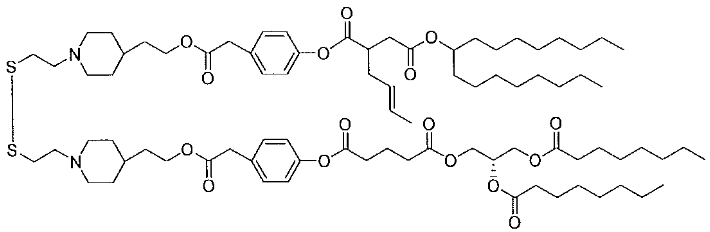 | $R^{3a}$: formula (14)<br>- $R^{33}$: 1- ((E) 2-butenyl) - ethylene<br>$R^{34}$: (1-octyl)nonyl | $R^{3b}$: formula<br>(16-20) $X^4$: O<br>$R^{36}$: tri-<br>methylene $R^{49}$: heptyl<br>$R^{50}$: heptyl |
| com- pound 54 | 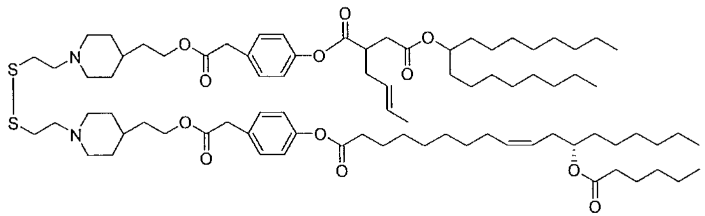 | $R^{3a}$: formula (14)<br>$R^{33}$: 1-((E)-2-butenyl)-ethylene<br>$R^{34}$: (1-octyl)nonyl | $R^{3b}$: formula (15)<br>$R^{35}$: pentyl |

(continued)

| name | structure | $R^{3a}$ | $R^{3b}$ |
|---|---|---|---|
| com- pound 55 | 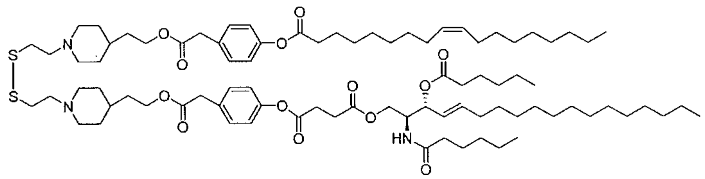 | $R^{3a}$: formula (16-21)<br>$X^4$: O<br>$R^{36}$: ethylene<br>$R^{51}$: pentyl<br>$R^{52}$: pentyl | $R^{3b}$: (Z)-8-heptadecenyl |
| com- pound 56 | 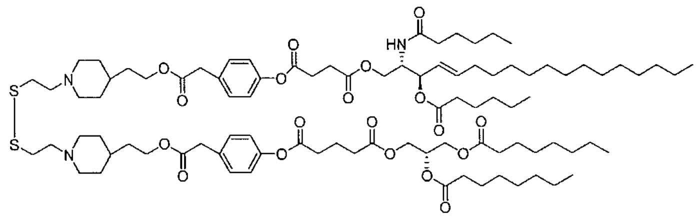 | $R^{3a}$: formula (16-20)<br>$X^4$: O<br>$R^{36}$: trimethylene<br>$R^{49}$: heptyl<br>$R^{50}$: heptyl | $R^{3b}$: formula (16-21)<br>$X^4$: O<br>$R^{36}$: ethylene<br>$R^{51}$: pentyl<br>$R^{52}$: pentyl |

[Table 1-9]

| name | structure | $R^{3a}$ | $R^{3b}$ |
|---|---|---|---|
| compound 57 | 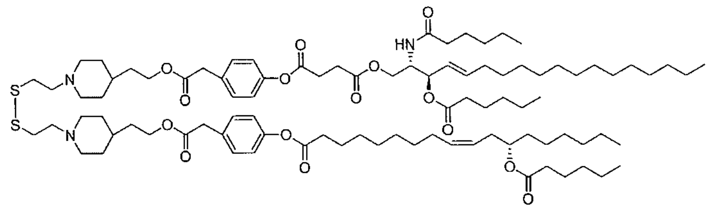 | $R^{3a}$: formula (15)<br>$R^{35}$: pentyl | $R^{3b}$: formula (16-21)<br>$X^4$: O<br>$R^{36}$: ethylene<br>$R^{51}$: pentyl<br>$R^{52}$: pentyl |
| compound 58 | 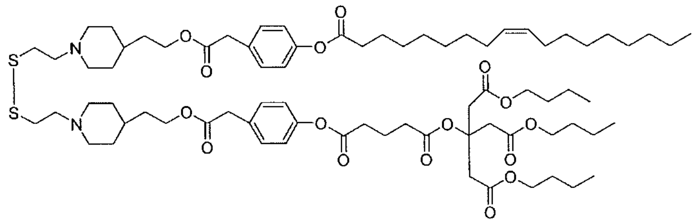 | $R^{3a}$: formula (16-22)<br>$X^4$: O<br>$R^{36}$: trimethylene<br>$R^{53}$-$R^{55}$ : butyl | $R^{3b}$: (Z)-8-heptadecenyl |
| compound 59 | 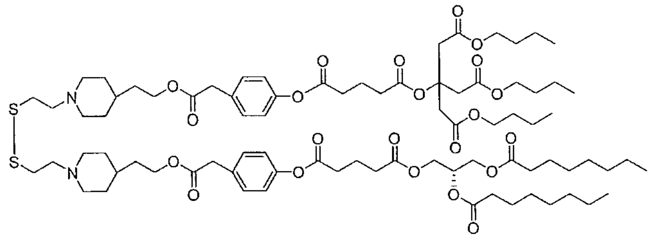 | $R^{3a}$: formula (16-20)<br>$X^4$: O<br>$R^{36}$: trimethylene<br>$R^{49}$: heptyl<br>$R^{50}$: heptyl | $R^{3b}$: formula (16-22)<br>$X^4$: O<br>$R^{36}$: trimethylene<br>$R^{53}$-$R^{55}$ butyl |
| compound 60 | 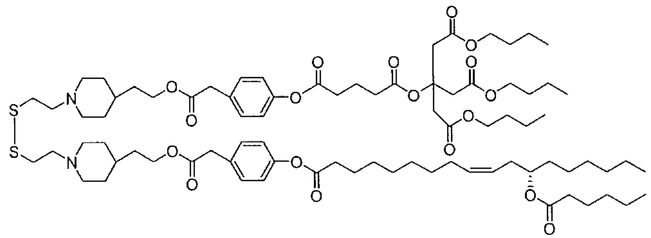 | $R^{3a}$: formula (15)<br>$R^{35}$: pentyl | $R^{3b}$: formula (16-22)<br>$X^4$: O<br>$R^{36}$: trimethylene<br>$R^{53}$-$R^{55}$ : butyl |

(continued)

| name | structure | $R^{3a}$ | $R^{3b}$ |
|---|---|---|---|
| compound 61 | 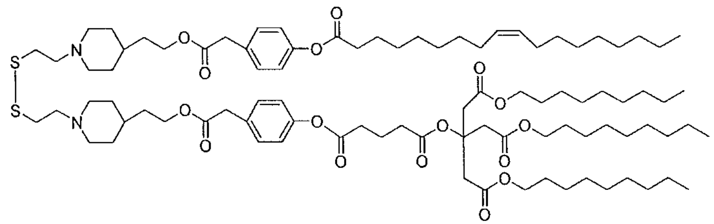 | $R^{3a}$: formula (16-22)<br>$X^4$: O<br>$R^{36}$: trimethylene<br>$R^{53}$-$R^{55}$: nonyl | $R^{3b}$: (Z)-8-heptadecenyl |
| compound 62 | 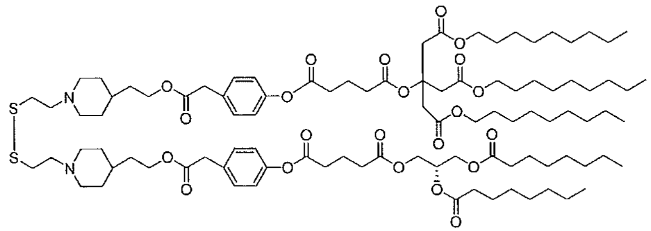 | $R^{3a}$: formula (16-20)<br>$X^4$: O<br>$R^{36}$: trimethylene<br>$R^{49}$: heptyl<br>$R^{50}$: heptyl | $R^{3b}$: formula (16-22)<br>$X^4$: O<br>$R^{36}$: trimethylene<br>$R^{53}$-$R^{55}$: nonyl |
| compound 63 | 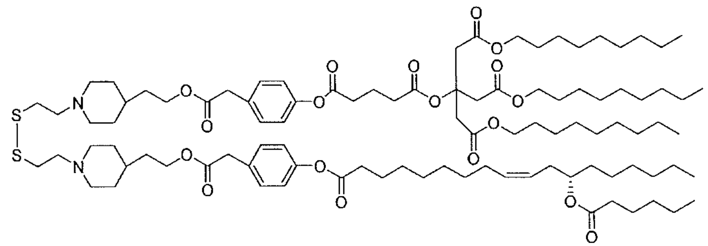 | $R^{3a}$: formula (15)<br>$R^{35}$: pentyl | $R^{3b}$: formula (16-22)<br>$X^4$: O<br>$R^{36}$: trimethylene<br>$R^{53}$-$R^{55}$ : nonyl |

[Table 1-10]

| name | structure | $R^{3a}$ | $R^{3b}$ |
|---|---|---|---|
| compound 64 | 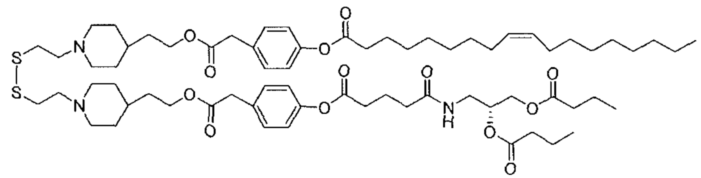 | $R^{3a}$: formula (16-20)<br>$X^4$: NH<br>$R^{36}$: trimethylene<br>$R^{49}$: propyl<br>$R^{50}$: propyl | $R^{3b}$: (Z)-8-heptadecenyl |
| compound 65 | 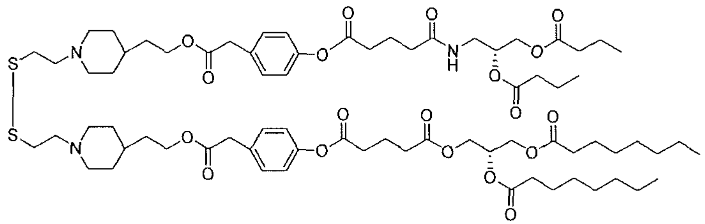 | $R^{3a}$: formula (16-20)<br>$X^4$: NH<br>$R^{36}$: trimethylene<br>$R^{49}$: propyl<br>$R^{50}$: propyl | $R^{3b}$: formula (16-20)<br>$X^4$: O<br>$R^{36}$: trimethylene<br>$R^{49}$: heptyl<br>$R^{50}$: heptyl |
| compound 66 | 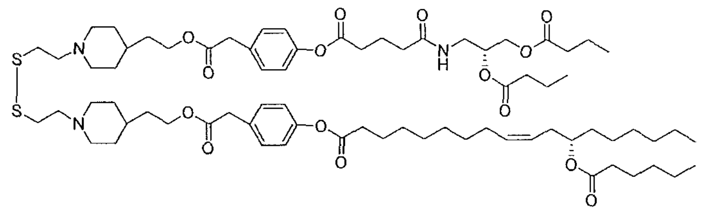 | $R^{3a}$: formula (15)<br>$R^{35}$: pentyl | $R^{3b}$: formula (16-20)<br>$X^4$: NH<br>$R^{36}$: trimethylene<br>$R^{49}$: propyl<br>$R^{50}$: propyl |
| compound 67 | 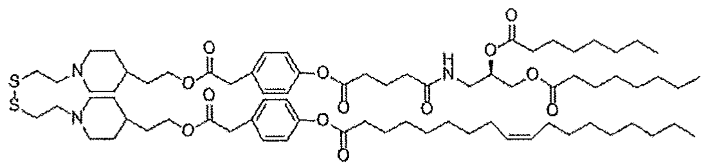 | $R^{3a}$: formula (16-20)<br>$X^4$: NH<br>$R^{36}$: trimethylene<br>$R^{49}$: heptyl<br>$R^{50}$: heptyl | $R^{3b}$: (Z)-8-heptadecenyl |
| compound 68 | 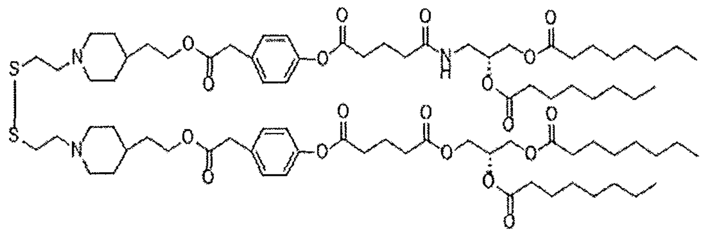 | $R^{3a}$: formula (16-20)<br>$X^4$: NH<br>$R^{36}$: trimethylene<br>$R^{49}$: heptyl<br>$R^{50}$: heptyl | $R^{3b}$: formula (16-20)<br>$X^4$: O<br>$R^{36}$: trimethylene<br>$R^{49}$: heptyl<br>$R^{50}$: heptyl |

(continued)

| name | structure | $R^{3a}$ | $R^{3b}$ |
|---|---|---|---|
| compound 69 | 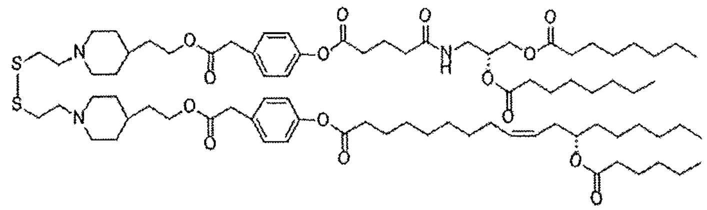 | $R^{3a}$: formula (15)<br>$R^{35}$: pentyl | $R^{3b}$: formula (16-20)<br>$X^4$: NH<br>$R^{36}$: trimethylene<br>$R^{49}$: heptyl<br>$R^{50}$: heptyl |
| compound 70 | 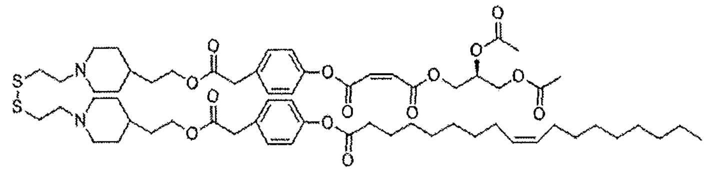 | $R^{3a}$: formula (16-20)<br>$X^4$: O<br>$R^{36}$: ethenediyl<br>$R^{49}$: methyl<br>$R^{50}$: methyl | $R^{3b}$: (Z)-8-heptadecenyl |

[Table 1-11]

| name | structure | $R^{3a}$ | $R^{3b}$ |
|---|---|---|---|
| compound 71 | 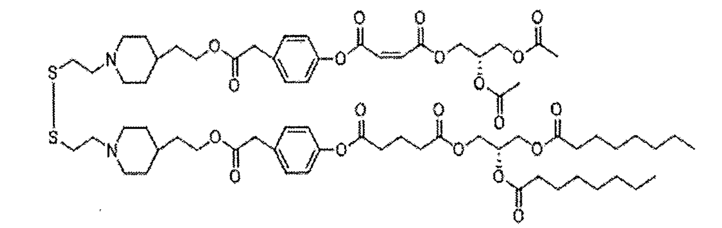 | $R^{3a}$: formula (16-20)<br>$X^4$: O<br>$R^{36}$: ethenediyl<br>$R^{49}$: methyl<br>$R^{50}$: methyl | $R^{3b}$: formula (16-20)<br>$X^4$: O<br>$R^{36}$: trimethylene<br>$R^{49}$: heptyl<br>$R^{50}$: heptyl |
| compound 72 | 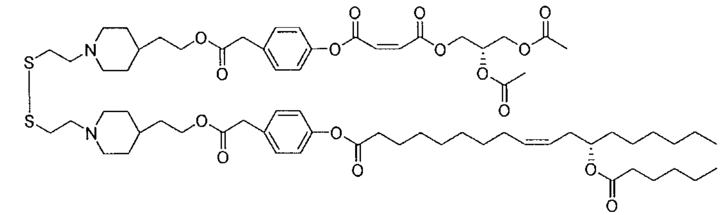 | $R^{3a}$: formula (15)<br>$R^{35}$: pentyl | $R^{3b}$: formula (16-20)<br>$X^4$: O<br>$R^{36}$: ethenediyl<br>$R^{49}$: methyl<br>$R^{50}$: methyl |
| compound 73 | 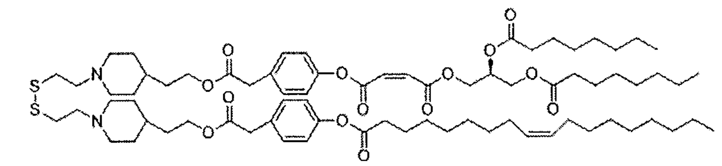 | $R^{3a}$: formula (16-20)<br>$X^4$: O<br>$R^{36}$: ethenediyl<br>$R^{49}$: heptyl<br>$R^{50}$: heptyl | $R^{3b}$: (Z)-8-heptadecenyl |
| compound 74 | 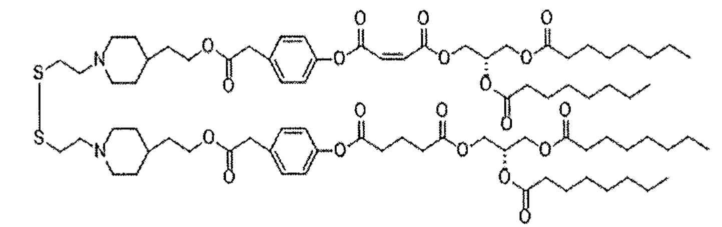 | $R^{3a}$: formula (16-20)<br>$X^4$: O<br>$R^{36}$: ethenediyl<br>$R^{49}$: heptyl<br>$R^{30}$: heptyl | $R^{3b}$: formula (16-20)<br>$X^4$: O<br>$R^{36}$: trimethylene<br>$R^{49}$: heptyl<br>$R^{50}$: heptyl |
| compound 75 | 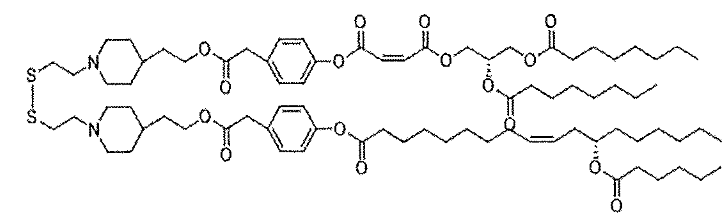 | $R^{3a}$: formula (15)<br>$R^{35}$: pentyl | $R^{3b}$: formula (16-20)<br>$X^4$: O<br>$R^{36}$: ethenediyl<br>$R^{49}$: heptyl<br>$R^{50}$: heptyl |

(continued)

| name | structure | $R^{3a}$ | $R^{3b}$ |
|---|---|---|---|
| compound 76 | 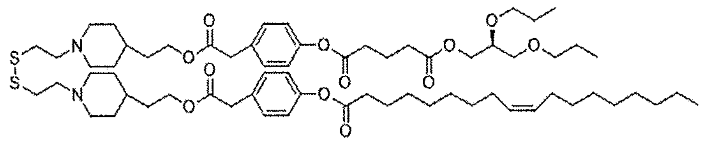 | $R^{3a}$: formula (12)<br>$R^{29}$ : trimethylene<br>$R^{30}$: propyl<br>$R^{31}$: propyl | $R^{3b}$: (Z)-8-heptadecenyl |
| compound 77 | 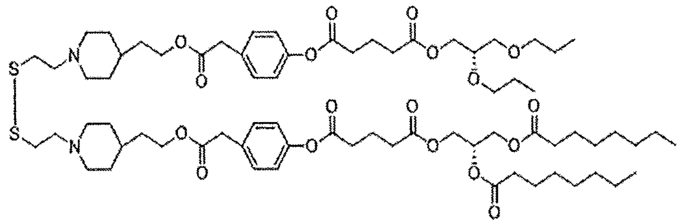 | $R^{3a}$: formula (16-20)<br>$X^4$: O<br>$R^{36}$: trimethylene<br>$R^{49}$: heptyl<br>$R^{50}$: heptyl | $R^{3b}$: formula (12)<br>$R^{29}$: trimethylene<br>$R^{30}$: propyl<br>$R^{31}$: propyl |

[Table 1-12]

| name | structure | $R^{3a}$ | $R^{3b}$ |
|---|---|---|---|
| compound 78 | 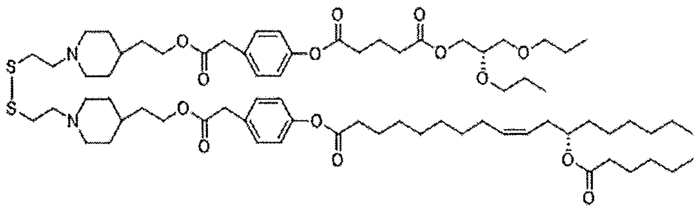 | $R^{3a}$: formula (15)<br>$R^{35}$ : pentyl | $R^{3b}$: formula (12)<br>$R^{29}$: trimethylene<br>$R^{30}$: propyl<br>$R^{31}$: propyl |
| compound 79 | 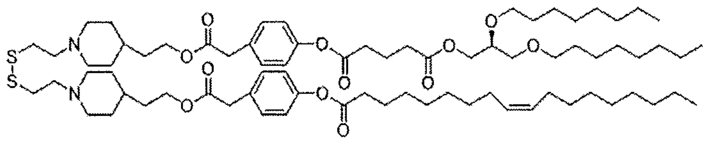 | $R^{3a}$: formula (12)<br>$R^{29}$: trimethylene<br>$R^{30}$: octyl<br>$R^{31}$: octyl | $R^{3b}$: (Z)-8-heptadecenyl |
| compound 80 | 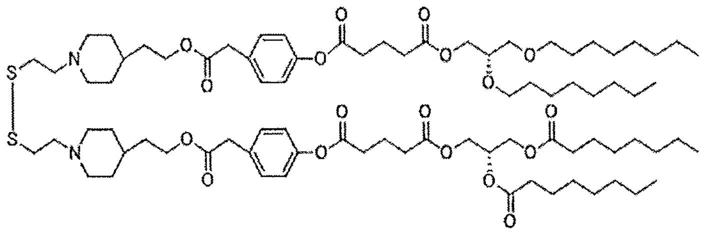 | $R^{3a}$: formula (16-20)<br>$X^4$: O<br>$R^{36}$: trimethylene<br>$R^{49}$: heptyl<br>$R^{50}$: heptyl | $R^{3b}$: formula (12)<br>$R^{29}$: trimethylene<br>$R^{30}$: octyl<br>$R^{31}$: octyl |
| compound 81 | 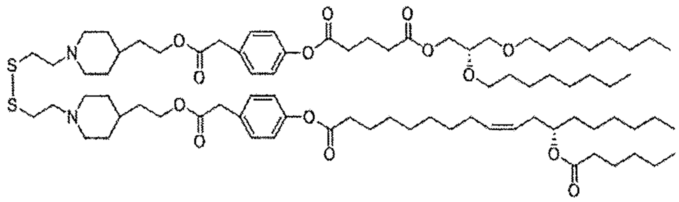 | $R^{3a}$: formula (15)<br>$R^{35}$: pentyl | $R^{3b}$: formula (12)<br>$R^{29}$: trimeth-ylene<br>$R^{30}$: octyl<br>$R^{31}$: octyl |
| compound 82 | 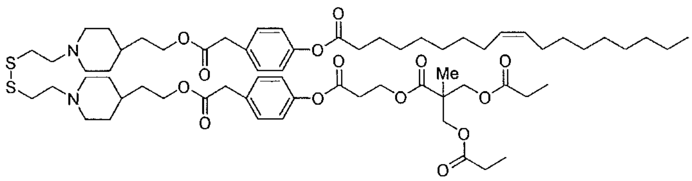 | $R^{3a}$: formula (17-23)<br>$R^{38}$ : ethylene<br>$R^{56}$: ethyl<br>$R^{57}$: ethyl | $R^{3b}$: (Z)-8-heptadecenyl |
| compound 83 | 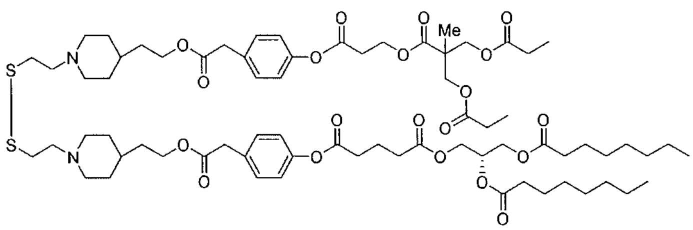 | $R^{3a}$: formula (16-20)<br>$X^4$: O<br>$R^{36}$: trimethylene<br>$R^{49}$: heptyl<br>$R^{50}$: heptyl | $R^{3b}$: formula (17-23)<br>$R^{38}$: ethylene<br>$R^{51}$: ethyl<br>$R^{57}$: ethyl |
| compound 84 | 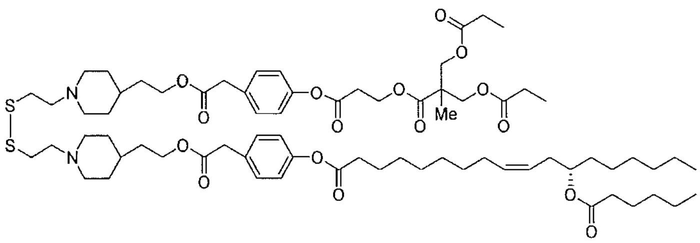 | $R^{3a}$: formula (15)<br>$R^{35}$: pentyl | $R^{3b}$: formula (17-23)<br>$R^{38}$: ethylene<br>$R^{51}$: ethyl<br>$R^{57}$: ethyl |

[Table 1-13]

| name | structure | $R^{3a}$ | $R^{3b}$ |
|---|---|---|---|
| com- pound 85 | 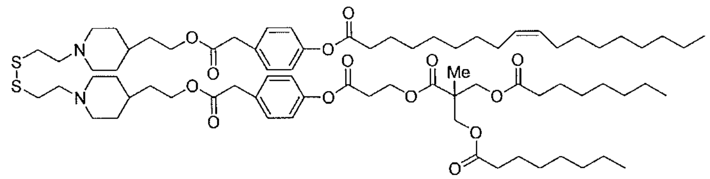 | $R^{3a}$: formula (17-23)<br>$R^{38}$: ethylene<br>$R^{56}$: heptyl<br>$R^{57}$: heptyl | $R^{3b}$: (Z)-8-heptadecenyl |
| compound 86 | 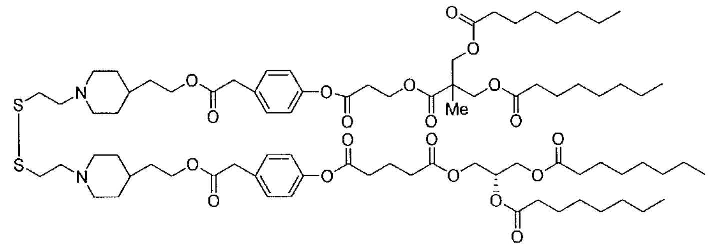 | $R^{3a}$: formula (16-20)<br>$X^4$: O<br>$R^{36}$: trimethylene<br>$R^{49}$: heptyl<br>$R^{50}$: heptyl | $R^{3b}$: formula (17-23)<br>$R^{38}$: ethylene<br>$R^{56}$: heptyl<br>$R^{57}$: heptyl |
| compound 87 | 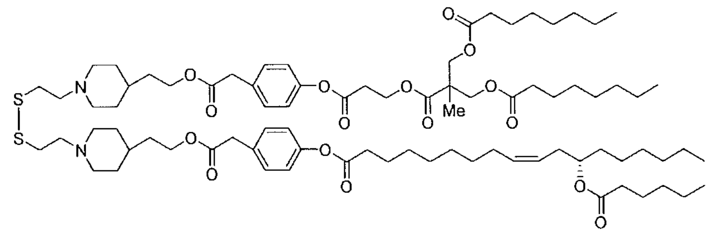 | $R^{3a}$: formula (15)<br>$R^{35}$: pentyl | $R^{3b}$: formula (17-23)<br>$R^{38}$: ethylene<br>$R^{56}$: heptyl<br>$R^{57}$: heptyl |
| compound 88 | 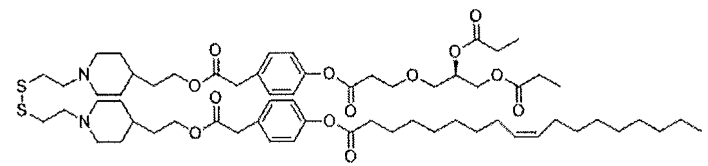 | $R^{3a}$: formula (17-24)<br>$R^{38}$: ethylene<br>$R^{58}$: ethyl<br>$R^{59}$: ethyl | $R^{3b}$: (Z)-8-heptadecenyl |
| compound 89 | 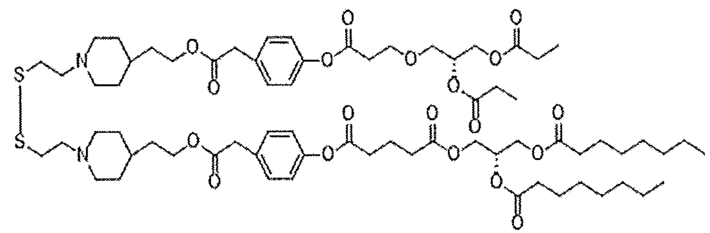 | $R^{3a}$: formula (16-20)<br>$X^4$: O<br>$R^{36}$: trimethylene<br>$R^{49}$: heptyl<br>$R^{50}$: heptyl | $R^{3b}$: formula (17-24)<br>$R^{38}$: ethylene<br>$R^{58}$: ethyl<br>$R^{59}$: ethyl |

(continued)

| name | structure | $R^{3a}$ | $R^{3b}$ |
|---|---|---|---|
| compound 90 | 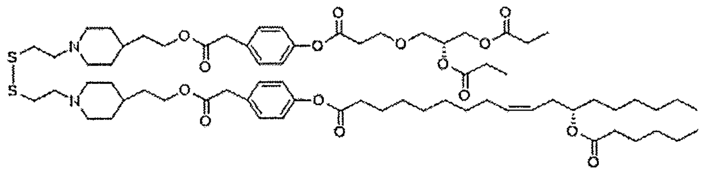 | $R^{3a}$: formula (15)<br>$R^{35}$: pentyl | $R^{3b}$: formula (17-24)<br>$R^{38}$: ethylene<br>$R^{58}$: ethyl<br>$R^{59}$: ethyl |
| compound 91 | 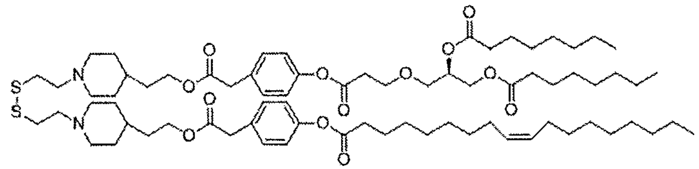 | $R^{3a}$: formula (17-24)<br>$R^{38}$: ethylene<br>$R^{58}$: heptyl<br>$R^{59}$: heptyl | $R^{3b}$: (Z)-8-heptadecenyl |

[Table 1-14]

| name | structure | $R^{3a}$ | $R^{3b}$ |
|---|---|---|---|
| compound 92 | 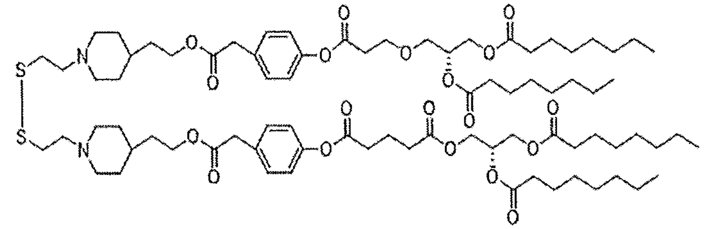 | $R^{3a}$: formula (16-20)<br>$X^4$: O<br>$R^{36}$: trimethylene<br>$R^{49}$: heptyl<br>$R^{50}$: heptyl | $R^{3b}$: formula (17-24)<br>$R^{38}$: ethylene<br>$R^{58}$: heptyl<br>$R^{59}$: heptyl |
| compound 93 | 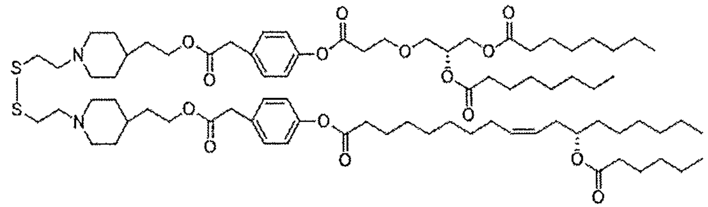 | $R^{3a}$: formula (15)<br>$R^{35}$: pentyl | $R^{3b}$: formula (17-24)<br>$R^{38}$: ethylene<br>$R^{58}$: heptyl<br>$R^{59}$: heptyl |
| compound 94 | 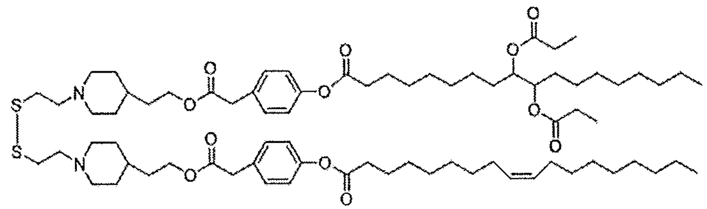 | $R^{3a}$: formula (19)<br>$R^{45}$: heptamethylene<br>$R^{46}$: ethyl<br>$R^{47}$: octyl<br>$R^{48}$: ethyl | $R^{3b}$: (Z)-8-heptadecenyl |
| compound 95 | 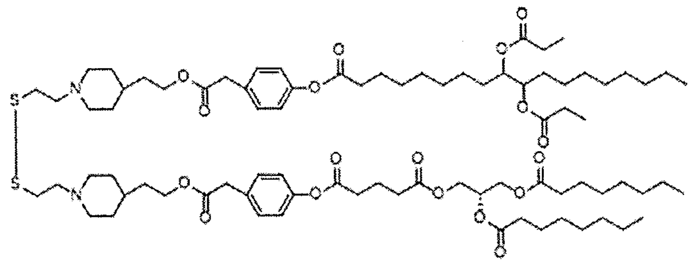 | $R^{3a}$: formula(16-20)<br>$X^4$: O<br>$R^{36}$: trimethylene<br>$R^{49}$: heptyl<br>$R^{50}$: heptyl | $R^{3b}$: formula (19)<br>$R^{45}$: heptamethylene<br>$R^{46}$: ethyl<br>$R^{47}$: octyl<br>$R^{48}$: ethyl |

(continued)

| name | structure | $R^{3a}$ | $R^{3b}$ |
|---|---|---|---|
| compound 96 | 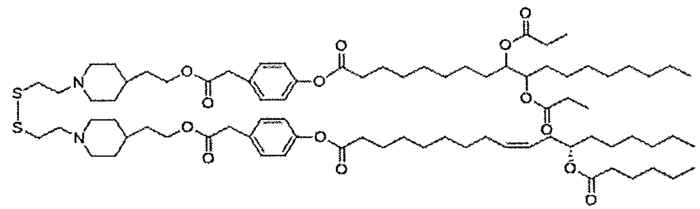 | $R^{3a}$: formula (15)<br>$R^{35}$: pentyl | $R^{3b}$: formula (19)<br>$R^{45}$: heptamethylene<br>$R^{46}$: ethyl<br>$R^{47}$: octyl<br>$R^{48}$: ethyl |
| compound 97 | 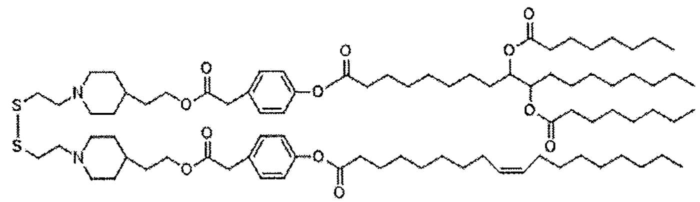 | $R^{3a}$: formula (19)<br>$R^{45}$: heptamethylene<br>$R^{46}$: heptyl<br>$R^{47}$: octyl<br>$R^{48}$: heptyl | $R^{3b}$: (Z)-8-heptadecenyl |

[Table 1-15]

| name | structure | $R^{3a}$ | $R^{3b}$ |
|---|---|---|---|
| compound 98 | 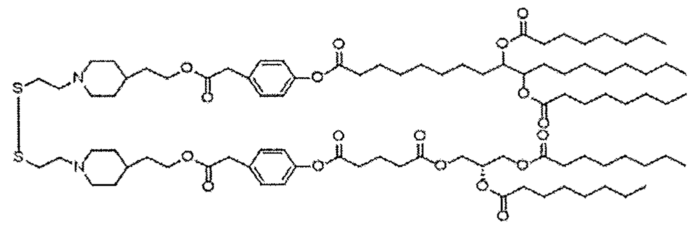 | $R^{3a}$: formula (16-20)<br>$X^4$: O<br>$R^{36}$: trimethylene<br>$R^{49}$: heptyl<br>$R^{50}$: heptyl | $R^{3b}$: formula (19)<br>$R^{45}$: heptamethylene<br>$R^{46}$: heptyl<br>$R^{47}$: octyl<br>$R^{48}$: heptyl |
| compound 99 | 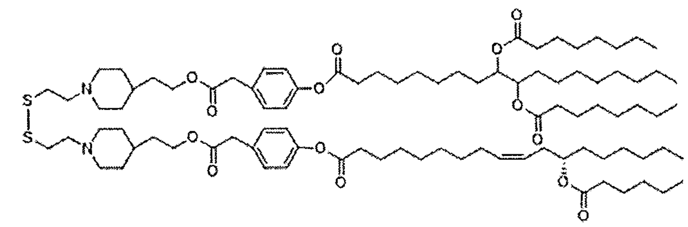 | $R^{3a}$: formula (15)<br>$R^{35}$: pentyl | $R^{3b}$: formula (19)<br>$R^{45}$: heptamethylene<br>$R^{46}$: heptyl<br>$R^{47}$: octyl<br>$R^{48}$: heptyl |
| compound 100 | 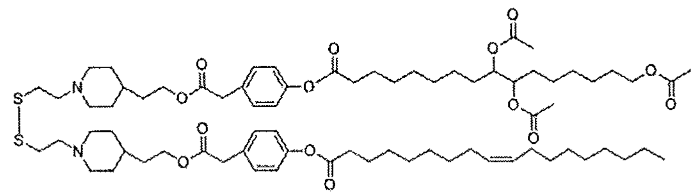 | $R^{3a}$: formula (18)<br>$R^{40}$: heptamethylene<br>$R^{41}$: hexamethylene<br>$R^{42}$-$R^{44}$: methyl | $R^{3b}$: (Z)-8-heptadecenyl |
| compound 101 | 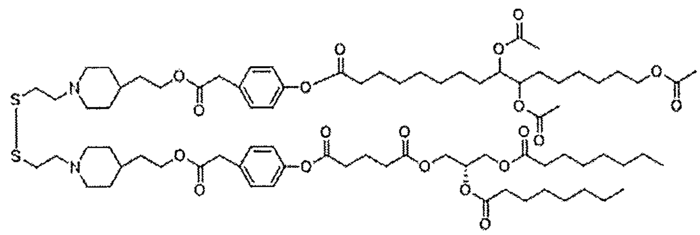 | $R^{3a}$: formula (16-20)<br>$X^4$: O<br>$R^{36}$: trimethylene<br>$R^{49}$: heptyl<br>$R^{50}$: heptyl | $R^{3b}$: formula (18)<br>$R^{40}$: hepta-methylene<br>$R^{41}$: hexamethylene<br>$R^{42}$-$R^{44}$: methyl |
| compound 102 | 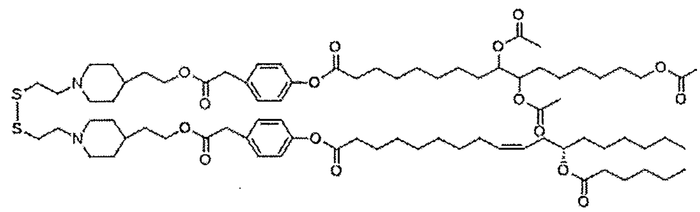 | $R^{3a}$: formula (15)<br>$R^{35}$: pentyl | $R^{3b}$: formula (18)<br>$R^{40}$: heptamethylene<br>$R^{41}$: hexamethylene<br>$R^{42}$-$R^{44}$: methyl |
| compound 103 | 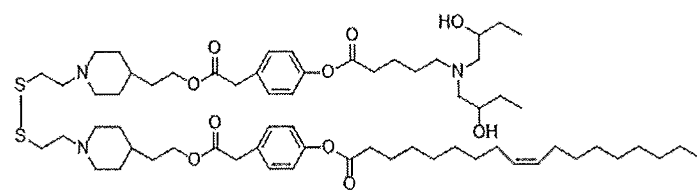 | $R^{3a}$: formula (5)<br>$X^2$: N<br>$R^{16}$: tetramethylene<br>$R^{17}$,$R^{18}$: (2-hydroxy)-butyl | $R^{3b}$: (Z)-8-heptadecenyl |

[Table 1-16]

| name | structure | $R^{3a}$ | $R^{3b}$ |
|---|---|---|---|
| compound 104 | 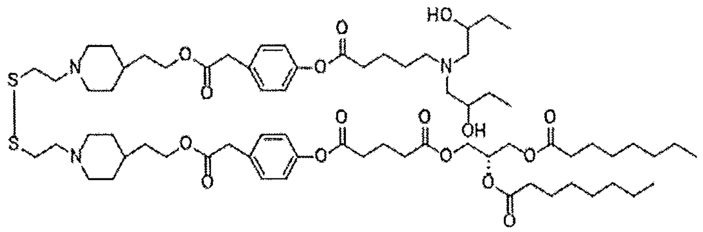 | $R^{3a}$: formula (16-20)<br>$X^4$: O<br>$R^{36}$: trimethylene<br>$R^{49}$: heptyl<br>$R^{50}$: heptyl | $R^{3b}$: formula (5)<br>$X^2$: N<br>$R^{16}$: tetramethylene<br>$R^{17}$,$R^{18}$: (2-hydroxy) - butyl |
| compound 105 | 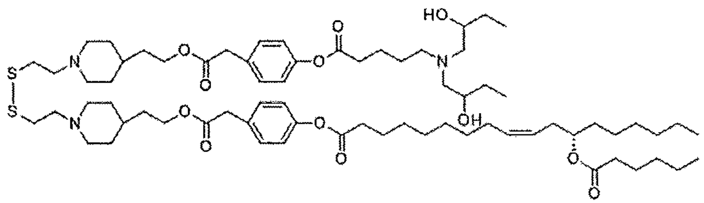 | $R^{3a}$: formula (15)<br>$R^{35}$: pentyl | $R^{3b}$: formula (5)<br>$X^2$: N<br>$R^{16}$: tetramethylene<br>$R^{17}$,$R^{18}$: (2-hydroxy)-butyl |
| compound 106 | 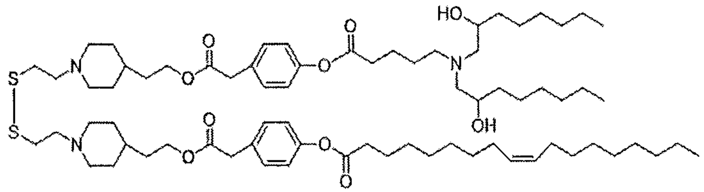 | $R^{3a}$: formula (5)<br>$X^2$: N<br>$R^{16}$: tetramethylene<br>$R^{17}$,$R^{18}$: (2-hydroxy) - octyl | $R^{3b}$: (Z)-8-heptadecenyl |
| compound 107 | 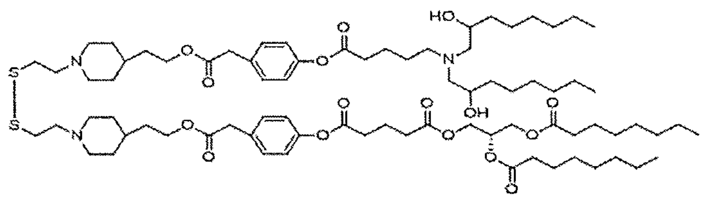 | $R^{3a}$: formula (16-20)<br>$X^4$: O<br>$R^{36}$: trimethylene<br>$R^{49}$: heptyl<br>$R^{50}$: heptyl | $R^{3b}$: formula (5)<br>$X^2$: N<br>$R^{16}$: tetramethylene<br>$R^{17}$,$R^{18}$: (2-hydroxy)-octyl |
| compound 108 | 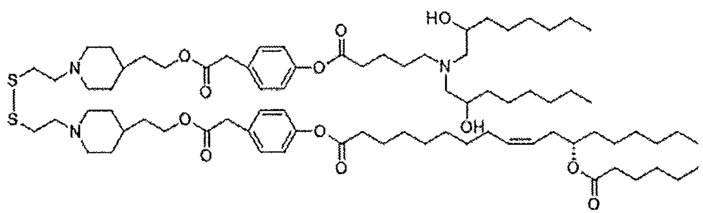 | $R^{3a}$:<br>formula (15)<br>$R^{35}$: pentyl | $R^{3b}$: formula (5)<br>$X^2$: N<br>$R^{16}$: tetramethylene<br>$R^{17}$,$R^{18}$: (2-hydroxy)-octyl |

(continued)

| name | structure | $R^{3a}$ | $R^{3b}$ |
|---|---|---|---|
| compound 109 | 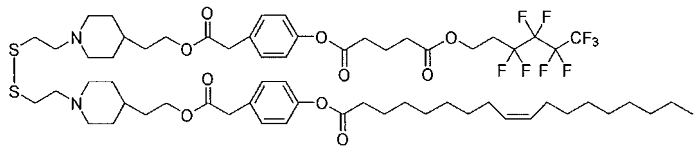 | $R^{3a}$: formula (3)<br>$R^6$: trimethylene<br>$R^7$: 3,3,4,4, 5,5,6,6,6-nonafluorohexyl | $R^{3b}$: (Z)-8-heptadecenyl |
| compound 110 | 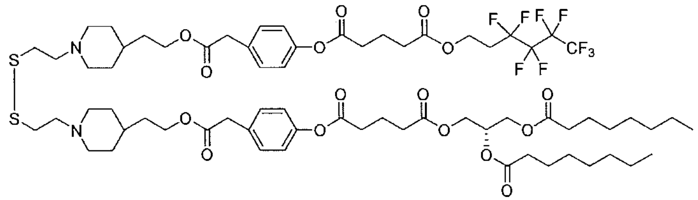 | $R^{3a}$: formula (16-20)<br>$X^4$: O<br>$R^{36}$: trimethylene<br>$R^{49}$: heptyl<br>$R^{50}$: heptyl | $R^{3b}$: formula (3)<br>$R^6$: trimethylene<br>$R^7$:3,3,4,4, 5,5,6,6,6-nonafluorohexyl |

[Table 1-17]

| name | structure | $R^{3a}$ | $R^{3b}$ |
|---|---|---|---|
| compound 111 | 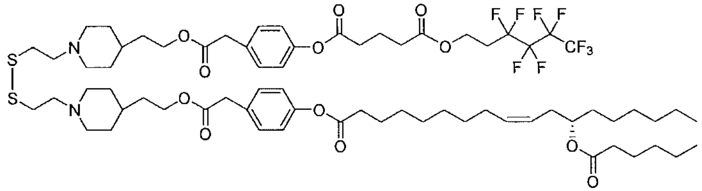 | $R^{3a}$: formula (15)<br>$R^{35}$: pentyl | $R^{3b}$: formula (3)<br>$R^6$: trimethylene<br>$R^7$: 3,3,4,4, 5,5,6,6,6-nonafluorohexyl |
| compound 112 | 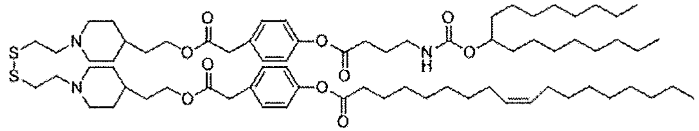 | $R^{3a}$: formula (2)<br>$X^1$: carbamate bond<br>$R^4$: trimethylene<br>$R^5$: (1-octyl)nonyl | $R^{3b}$: (Z)-8-heptadecenyl |
| compound 113 | 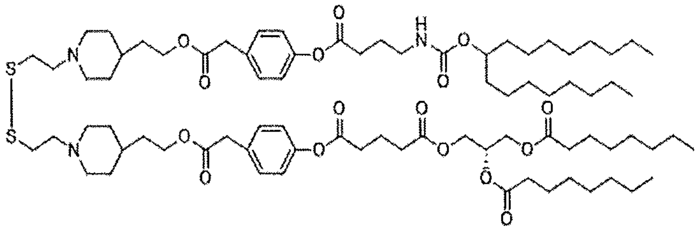 | $R^{3a}$: formula (16-20)<br>$X^4$: O<br>$R^{36}$: trimethylene<br>$R^{49}$: heptyl<br>$R^{50}$: heptyl | $R^{3b}$: formula (2)<br>$X^1$: carbamate bond<br>$R^4$: trimethylene<br>$R^5$: (1-octyl)nonyl |
| compound 114 | 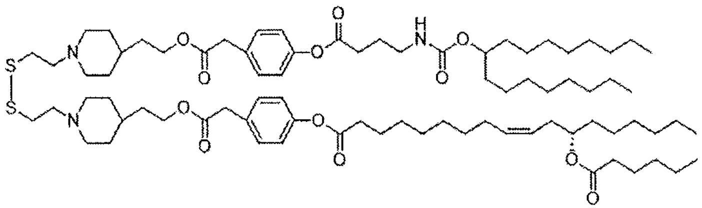 | $R^{3a}$: formula (15)<br>$R^{35}$: pentyl | $R^{3b}$: formula (2)<br>$X^1$: carbamate bond<br>$R^4$: trimethylene<br>$R^5$: (1-octyl)nonyl |
| compound 115 | 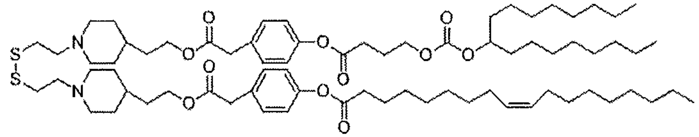 | $R^{3a}$: formula (2)<br>$X^1$: carbonate bond<br>$R^4$: trimethylene<br>$R^5$: (1-octyl)nonyl | $R^{3b}$: (Z)-8-heptadecenyl |

(continued)

| name | structure | $R^{3a}$ | $R^{3b}$ |
|---|---|---|---|
| compound 116 | 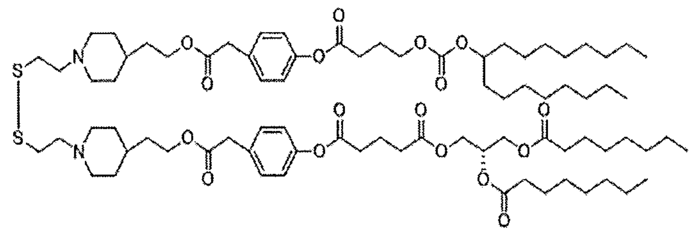 | $R^{3a}$: formula (16-20)<br>$X^4$: O<br>$R^{36}$: trimethylene<br>$R^{49}$: heptyl<br>$R^{50}$: heptyl | $R^{3b}$: formula (2)<br>$X^1$: carbonate bond<br>$R^4$: trimethylene<br>$R^5$: (1-octyl)nonyl |
| compound 117 | 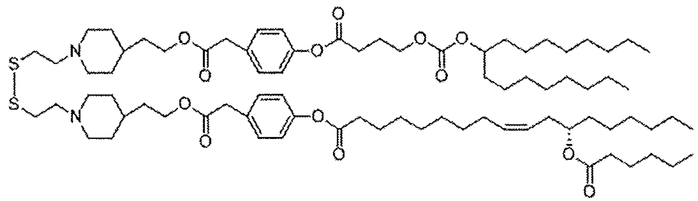 | $R^{3a}$: formula (15)<br>$R^{35}$: pentyl | $R^{3b}$: formula (2)<br>$X^1$: carbonate bond<br>$R^4$: trimethylene<br>$R^5$: (1-octyl)nonyl |

[Table 1-18]

| name | structure | $R^{3a}$ | $R^{3b}$ |
|---|---|---|---|
| compound 118a | 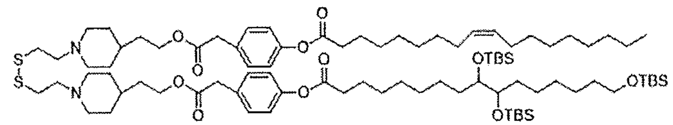 | $R^{3a}$: formula (4)<br>$R^8$: heptamethylene<br>$R^9$: hexamethylene<br>$R^{10}$-$R^{12}$: TBS | $R^{3b}$: (Z)-8-heptadecenyl |
| compound 118 | 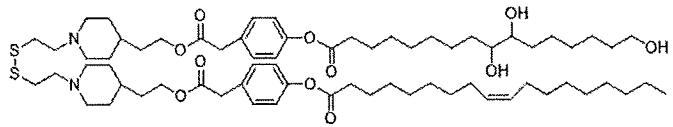 | $R^{3a}$: formula (4)<br>$R^8$: heptamethylene<br>$R^9$: hexa-methylene<br>$R^{10}$-$R^{12}$: H | $R^{3b}$: (Z)-8-heptadecenyl |
| compound 119a | 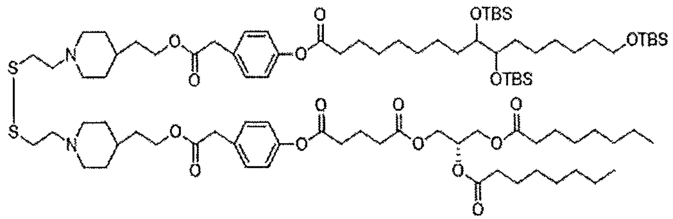 | $R^{3a}$: formula (16-20)<br>$X^4$: O<br>$R^{36}$: trimethylene<br>$R^{49}$: heptyl<br>$R^{50}$: heptyl | $R^{3b}$: formula (4)<br>$R^8$: heptamethylene<br>$R^9$: hexamethylene<br>$R^{10}$-$R^{12}$: TBS |
| compound 119 | 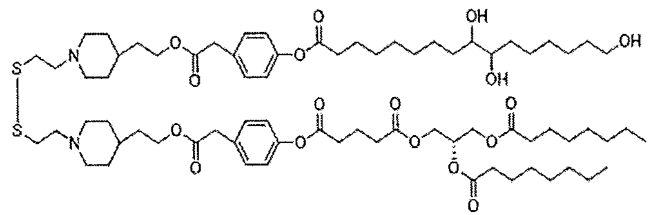 | $R^{3a}$: formula (16-20)<br>$X^4$: O<br>$R^{36}$: tri-methylene<br>$R^{49}$: heptyl<br>$R^{50}$: heptyl | $R^{3b}$: formula (4)<br>$R^8$: heptamethylene<br>$R^9$: hexamethylene<br>$R^{10}$-$R^{12}$: H |
| compound 120a | 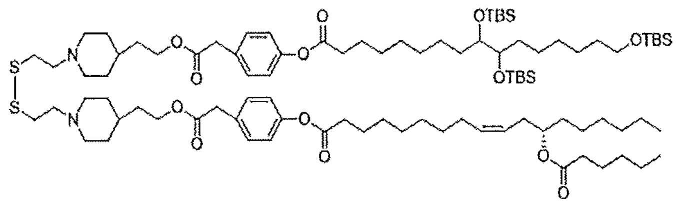 | $R^{3a}$: formula (15)<br>$R^{35}$: pentyl | $R^{3b}$: formula (4)<br>$R^8$: heptamethylene<br>$R^9$: hexamethylene<br>$R^{10}$-$R^{12}$: TBS |

(continued)

| name | structure | $R^{3a}$ | $R^{3b}$ |
|---|---|---|---|
| compound 120 | 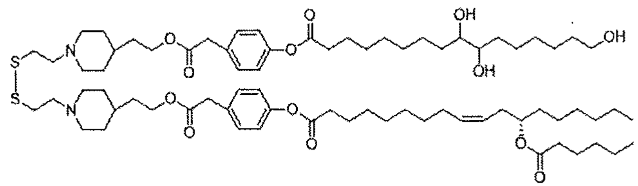 | $R^{3a}$: formula (15)<br>$R^{35}$: pentyl | $R^{3b}$: formula (4)<br>$R^8$: heptamethylene<br>$R^9$: hexamethylene<br>$R^{10}$-$R^{12}$: H |
| compound 121 | 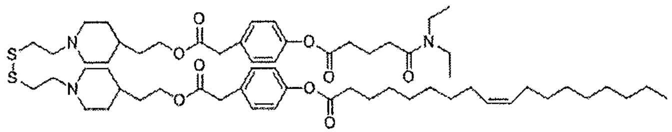 | $R^{3a}$: formula (5)<br>$X^2$: formula (6)<br>$R^{16}$: trimethylene<br>$R^{17}$: ethyl<br>$R^{18}$: ethyl | $R^{3b}$: (Z)-8-heptadecenyl |

[Table 1-19]

| name | structure | $R^{3a}$ | $R^{3b}$ |
|---|---|---|---|
| compound 122 | 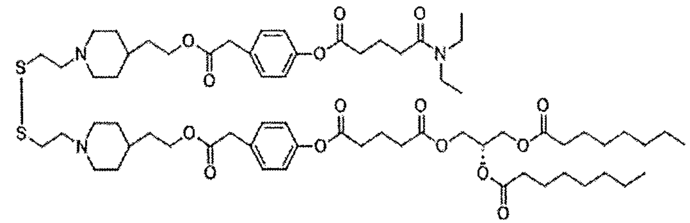 | $R^{3a}$: formula (16-20)<br>$X^4$: O<br>$R^{36}$: tri-methylene<br>$R^{49}$: heptyl<br>$R^{50}$: heptyl | $R^{3b}$: formula (5)<br>$X^2$: formula (6)<br>$R^{16}$: trimethylene<br>$R^{17}$: ethyl<br>$R^{18}$: ethyl |
| compound 123 | 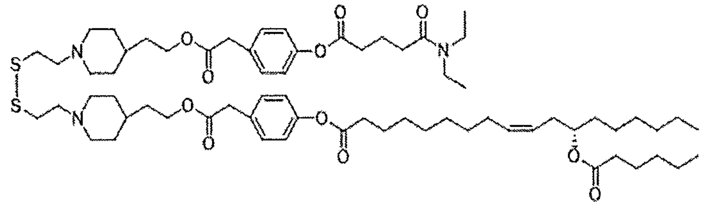 | $R^{3a}$: formula (15)<br>$R^{35}$: pentyl | $R^{3b}$: formula (5)<br>$X^2$: formula (6)<br>$R^{16}$: trimethylene<br>$R^{17}$: ethyl<br>$R^{18}$: ethyl |
| compound 124 | 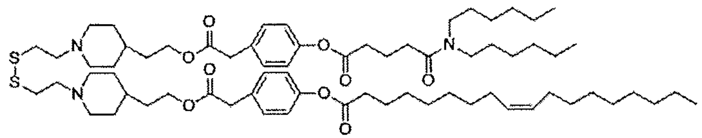 | $R^{3a}$: formula (5)<br>$X^2$: formula (6)<br>$R^{16}$: trimethylene<br>$R^{17}$: hexyl<br>$R^{18}$: hexyl | $R^{3b}$: (Z)-8-heptadecenyl |
| compound 125 | 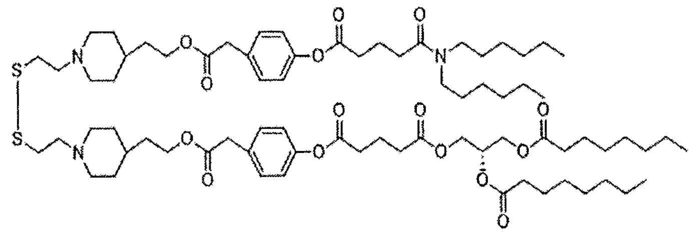 | $R^{3a}$: formula (16-20)<br>$X^4$: O<br>$R^{36}$: trimethylene<br>$R^{49}$: heptyl<br>$R^{50}$: heptyl | $R^{3b}$: formula (5)<br>$X^2$: formula (6)<br>$R^{16}$: trimethylene<br>$R^{17}$: hexyl<br>$R^{18}$: hexyl |

(continued)

| name | structure | $R^{3a}$ | $R^{3b}$ |
|---|---|---|---|
| compound 126 | 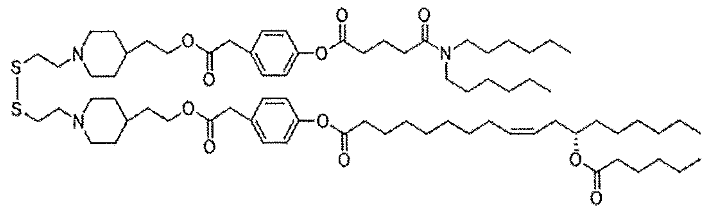 | $R^{3a}$: formula (15)<br>$R^{35}$: pentyl | $R^{3b}$: formula (5)<br>$X^2$: formula (6)<br>$R^{16}$: trimethylene<br>$R^{17}$: hexyl<br>$R^{18}$: hexyl |
| compound 127a | 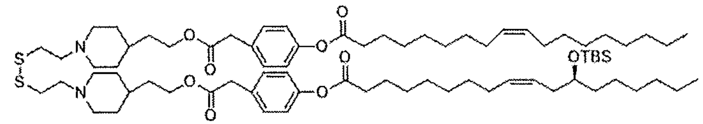 | $R^{3a}$: formula (9)<br>$R^{23}$: TBS | $R^{3b}$: (Z)-8-heptadecenyl |
| compound 127 | 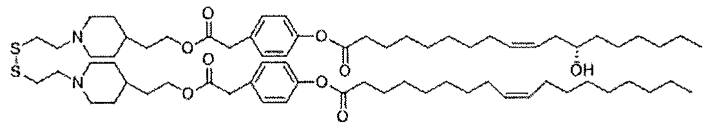 | $R^{3a}$: formula (9)<br>$R^{23}$: H | $R^{3b}$: (Z)-8-heptadecenyl |

[Table 1-20]

| name | structure | $R^{3a}$ | R3b |
|---|---|---|---|
| compound 128a | 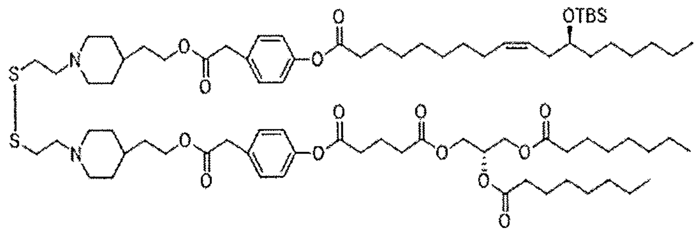 | $R^{3a}$: formula (16-20)<br>$X^4$: O<br>$R^{36}$: trimethylene<br>$R^{49}$: heptyl<br>$R^{50}$: heptyl | $R^{3b}$: formula (9)<br>$R^{23}$: TBS |
| compound 128 | 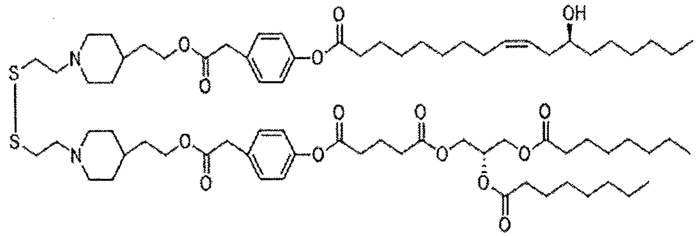 | $R^{3a}$: formula (16-20)<br>$X^4$: O<br>$R^{36}$: trimethylene<br>$R^{49}$: heptyl<br>$R^{50}$: heptyl | $R^{3b}$: formula (9)<br>$R^{23}$: H |
| compound 129a | 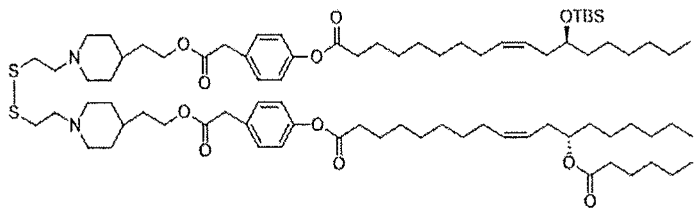 | $R^{3a}$: formula (15)<br>$R^{35}$: pentyl | $R^{3b}$: formula (9)<br>$R^{23}$: TBS |
| compound 129 | 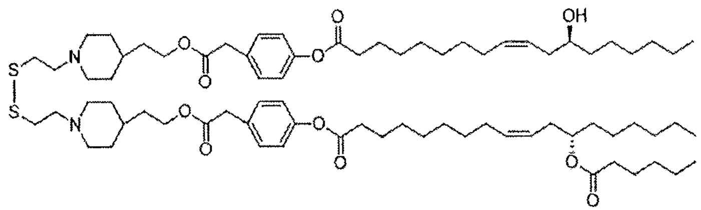 | $R^{3a}$: formula (15)<br>$R^{35}$: pentyl | $R^{3b}$: formula (9)<br>$R^{23}$: H |
| compound 130 | 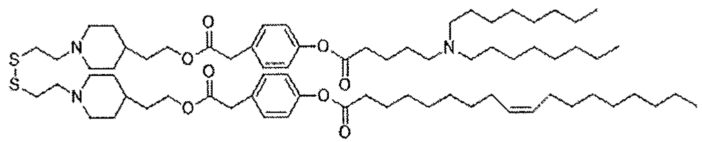 | $R^{3a}$: formula (5)<br>$X^2$: N<br>$R^{16}$: tetramethylene<br>$R^{17}$: octyl<br>$R^{18}$: octyl | $R^{3b}$: (Z)-8-heptadecenyl |

(continued)

| name | structure | $R^{3a}$ | R3b |
|---|---|---|---|
| compound 131 | 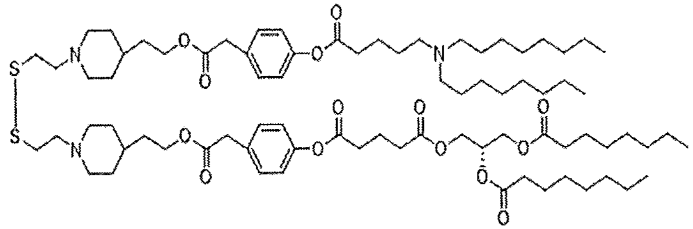 | $R^{3a}$: formula(16-20)<br>$X^4$: O<br>$R^{36}$: trimethylene<br>$R^{49}$: heptyl<br><br>$R^{50}$: heptyl | $R^{3b}$: formula (5)<br>$X^2$: N<br>$R^{16}$: tetramethylene<br>$R^{17}$: octyl<br><br>$R^{18}$: octyl |

[Table 1-21]

| name | structure | $R^{3a}$ | $R^{3b}$ |
|---|---|---|---|
| compound 132 | 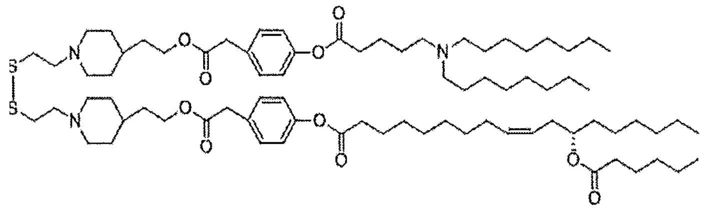 | $R^{3a}$: formula (15)<br>$R^{35}$: pentyl | $R^{3b}$: formula (5)<br>$X^2$: N<br>$R^{16}$: tetramethylene<br>$R^{17}$: octyl<br>$R^{18}$: octyl |
| compound 133a | 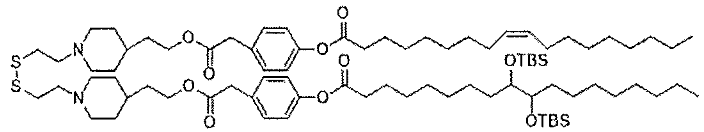 | $R^{3a}$: formula (8)<br>$R^{21}$,$R^{22}$: TBS | $R^{3b}$: (Z)-8-heptadecenyl |
| compound 133 | 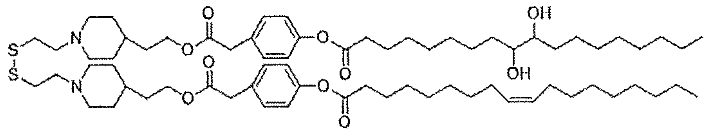 | $R^{3a}$: formula (8)<br>$R^{21}$,$R^{22}$: H | $R^{3b}$: (Z)-8-heptadecenyl |
| compound 134a | 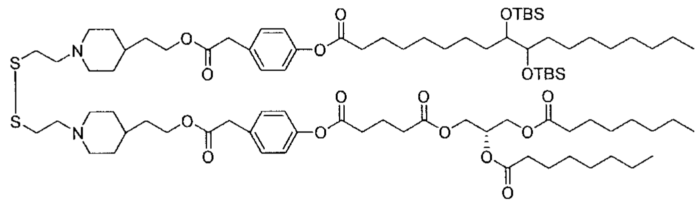 | $R^{3a}$: formula (16-20)<br>$X^4$: O<br>$R^{36}$: trimethylene<br>$R^{49}$: heptyl<br>$R^{50}$: heptyl | $R^{3b}$: formula (8)<br>$R^{21}$,$R^{22}$: TBS |
| compound 134 | 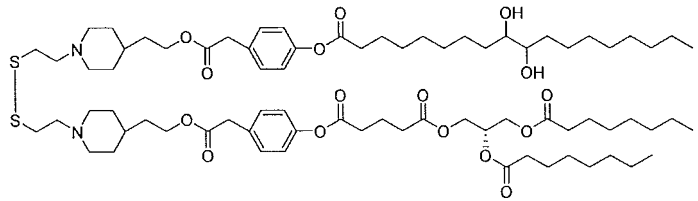 | $R^{3a}$: formula (16-20)<br>$X^4$: O<br>$R^{36}$: trimethylene<br>$R^{49}$: heptyl<br>$R^{50}$: heptyl | $R^{3b}$: formula (8)<br>$R^{21}$,$R^{22}$: H |

(continued)

| name | structure | $R^{3a}$ | $R^{3b}$ |
|---|---|---|---|
| compound 135a | 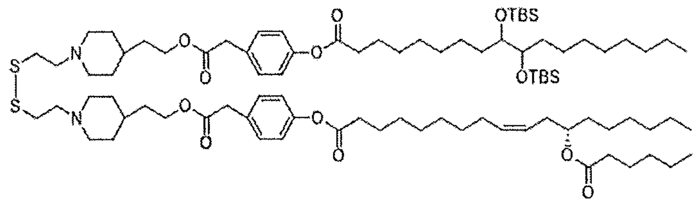 | $R^{3a}$: formula (15)<br>$R^{35}$: pentyl | $R^{3b}$: formula (8)<br>$R^{21}$,$R^{22}$: TBS |
| compound 135 | 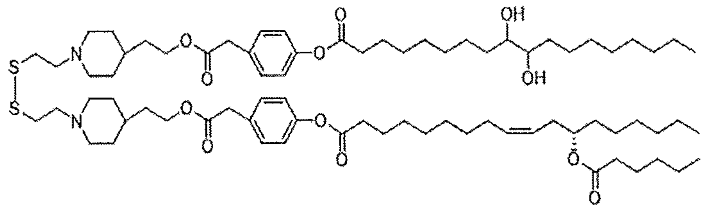 | $R^{3a}$: formula (15)<br>$R^{35}$: pentyl | $R^{3b}$: formula (8)<br>$R^{21}$,$R^{22}$: H |

[Table 1-22]

| name | structure | $R^{3a}$ | $R^{3b}$ |
|---|---|---|---|
| compound 136 | 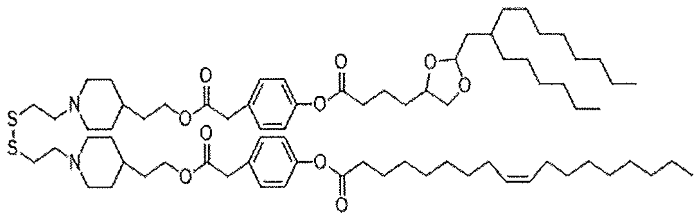 | $R^{3a}$: formula (10)<br>$R^{24}$: trimethylene<br>$R^{25}$: (2-hexyl)-decyl | $R^{3b}$: (Z)-8-heptadecenyl |
| compound 137 | 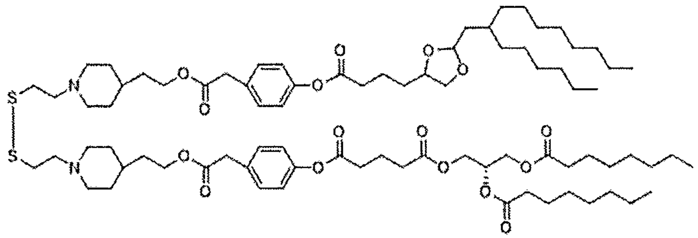 | $R^{3a}$: formula (16-20)<br>$X^4$: O<br>$R^{36}$: trimethylene<br>$R^{49}$: heptyl<br>$R^{50}$: heptyl | $R^{3b}$: formula (10)<br>$R^{24}$: trimethylene<br>$R^{25}$: (2-hexyl)-decyl |
| compound 138 | 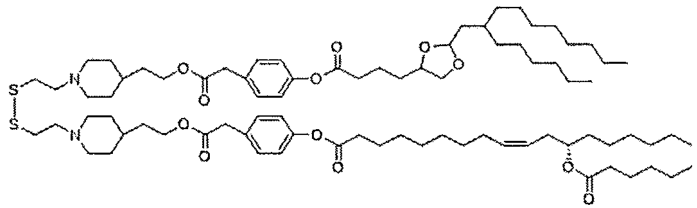 | $R^{3a}$: formula (15)<br>$R^{35}$: pentyl | $R^{3b}$: formula (10)<br>$R^{24}$: trimethylene<br>$R^{25}$: (2-hexyl)-decyl |
| compound 139a | 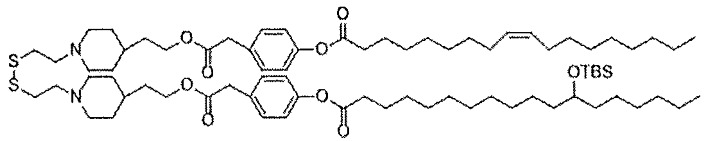 | $R^{3a}$: formula (7)<br>$R^{19}$: TBS | $R^{3b}$: (Z)-8-heptadecenyl |
| compound 139 | 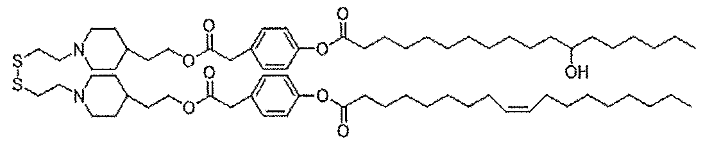 | $R^{3a}$: formula (7)<br>$R^{19}$: H | $R^{3b}$: (Z)-8-heptadecenyl |

(continued)

| name | structure | $R^{3a}$ | $R^{3b}$ |
|---|---|---|---|
| compound 140a | 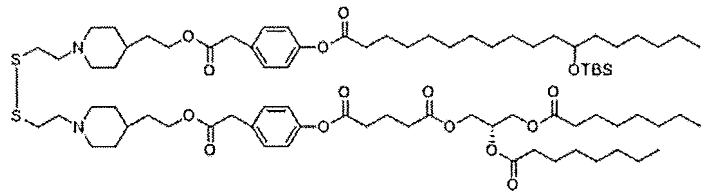 | $R^{3a}$: formula (16-20)<br>$X^4$: O<br>$R^{36}$: trimethylene<br>$R^{49}$: heptyl<br>$R^{50}$: heptyl | $R^{3b}$: formula (7)<br>$R^{19}$: TBS |
| compound 140 | 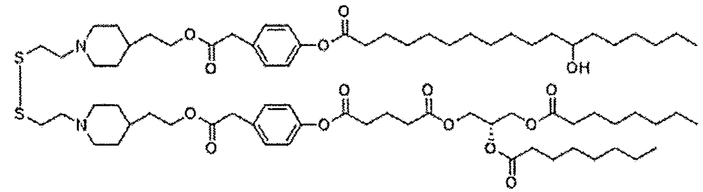 | $R^{3a}$: formula (16-20)<br>$X^4$: O<br>$R^{36}$: trimethylene<br>$R^{49}$: heptyl<br>$R^{50}$: heptyl | $R^{3b}$: formula (7)<br>$R^{19}$: H |

[Table 1-23]

| name | structure | $R^{3a}$ | $R^{3b}$ |
|---|---|---|---|
| compound 141a | 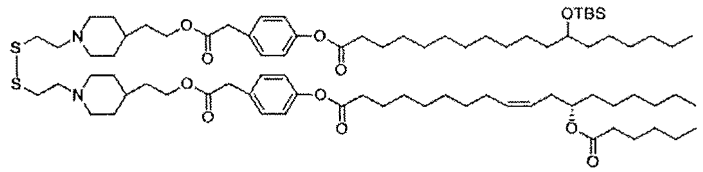 | $R^{3a}$: formula (15)<br>$R^{35}$: pentyl | $R^{3b}$: formula (7)<br>$R^{19}$: TBS |
| compound 141 | 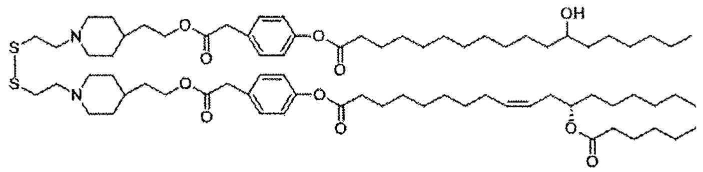 | $R^{3a}$: formula (15)<br>$R^{35}$: pentyl | $R^{3b}$: formula (7)<br>$R^{19}$: H |
| compound 142 | 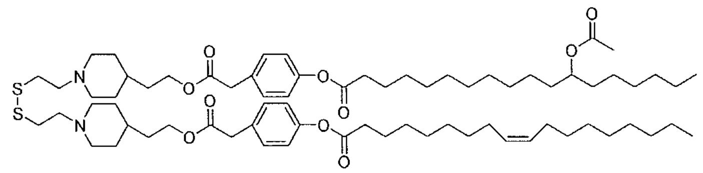 | $R^{3a}$: formula (7)<br>$R^{19}$: $-COR^{20}$<br>$R^{20}$: methyl | $R^{3b}$: (Z)-8-heptadecenyl |
| compound 143 | 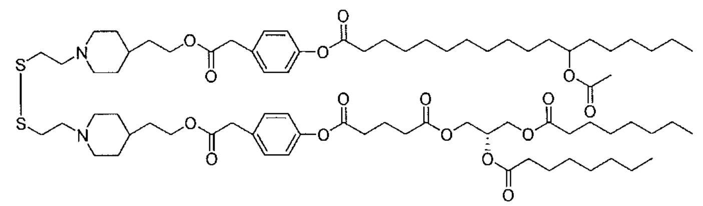 | $R^{3a}$: formula (16-20)<br>$X^4$: O<br>$R^{36}$: trimethylene<br>$R^{49}$: heptyl<br>$R^{50}$: heptyl | $R^{3b}$: formula (7)<br>$R^{19}$: $-COR^{20}$<br>$R^{20}$: methyl |
| compound 144 | 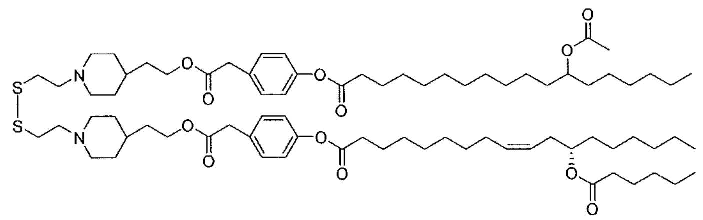 | $R^{3a}$: formula (15)<br>$R^{35}$: pentyl | $R^{3b}$: formula (7)<br>$R^{19}$: $-COR^{20}$<br>$R^{20}$: methyl |
| compound 145 | 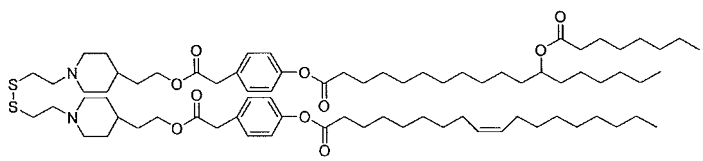 | $R^{3a}$: formula (7)<br>$R^{19}$: $-COR^{20}$<br>$R^{20}$: heptyl | $R^{3b}$: (Z)-8-heptadecenyl |
| compound 146 | 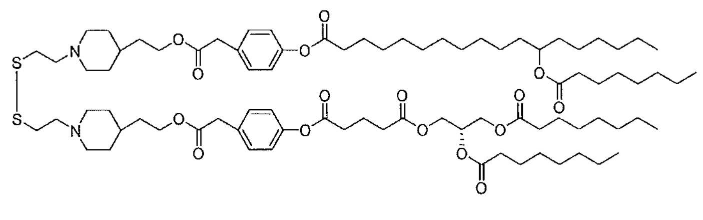 | $R^{3a}$: formula (16-20)<br>$X^4$: O<br>$R^{36}$: trimethylene<br>$R^{49}$: heptyl<br>$R^{50}$: heptyl | $R^{3b}$: formula (7)<br>$R^{19}$: $-CO-R^{20}$<br>$R^{20}$: heptyl |

[Table 1-24]

| name | structure | $R^{3a}$ | $R^{3b}$ |
|---|---|---|---|
| compound 147 | 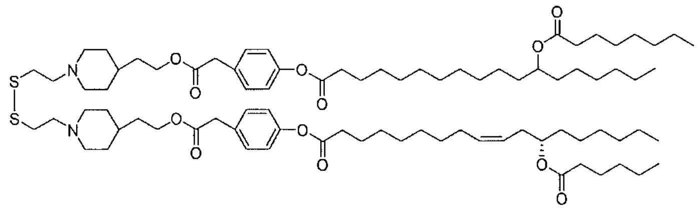 | $R^{3a}$: formula (15)<br>$R^{35}$: pentyl | $R^{3b}$: formula (7)<br>$R^{19}$: $-COR^{20}$<br>$R^{20}$: heptyl |
| compound 148 | 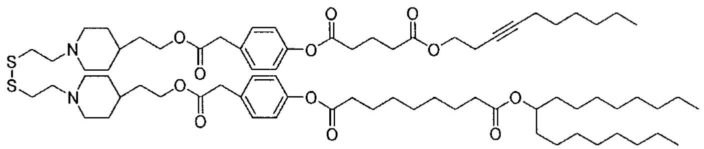 | $R^{3a}$: formula (14)<br>$X^3$: O<br>$R^{33}$: trimethylene<br>$R^{34}$: 3-decynyl | $R^{3b}$: derived from intermediate 53 |
| compound 149 | 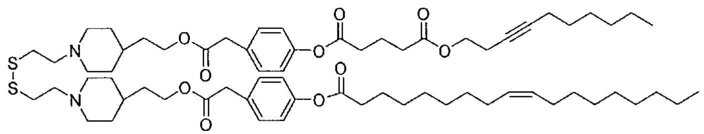 | $R^{3a}$: formula (14)<br>$X^3$: O<br>$R^{33}$: trimethylene<br>$R^{34}$: 3-decynyl | $R^{3b}$: (Z)-8-heptadecenyl |
| compound 150 | 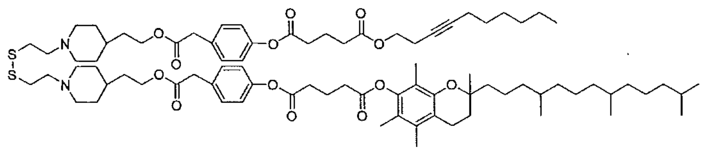 | $R^{3a}$: formula (14)<br>$X^3$: O<br>$R^{33}$: trimethylene<br>$R^{34}$: 3-decynyl | $R^{3b}$: $R^{3c}$-CO-$(CH_2)_p$-<br>p: 3<br>$R^{3c}$: tocopherol residue |
| compound 151 | 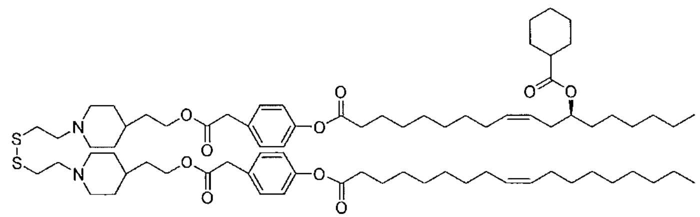 | $R^{3a}$: formula (15)<br>$R^{35}$: cyclohexyl | $R^{3b}$: (Z)-8-heptadecenyl |
| compound 152 | 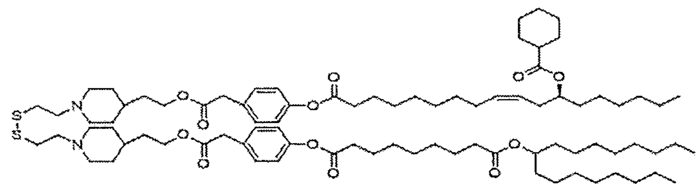 | $R^{3a}$: formula (15)<br>$R^{35}$: cyclohexyl | $R^{3b}$: derived from intermediate 53 |

(continued)

| name | structure | $R^{3a}$ | $R^{3b}$ |
|---|---|---|---|
| compound 153 | 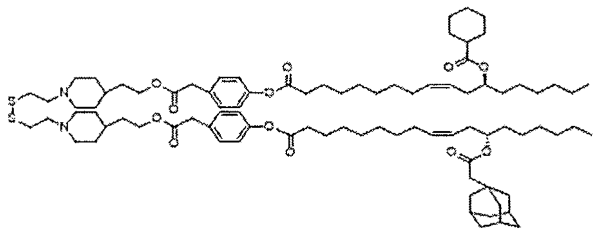 | $R^{3a}$: formula (15)<br>$R^{35}$: cyclohexyl | $R^{3b}$: formula (15)<br>$R^{35}$: 1-adamantylmethyl |

[Table 1-25]

| name | structure | $R^{3a}$ | $R^{3b}$ |
|---|---|---|---|
| compound 154 | 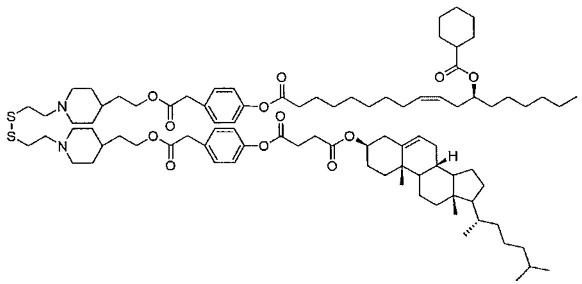 | $R^{3a}$: formula (15)<br>$R^{35}$: cyclohexyl | $R^{3b}$: $R^{3c}$-CO-$(CH_2)_p$-<br>p: 2<br>$R^{3c}$: cholesterol residue |
| compound 155 | 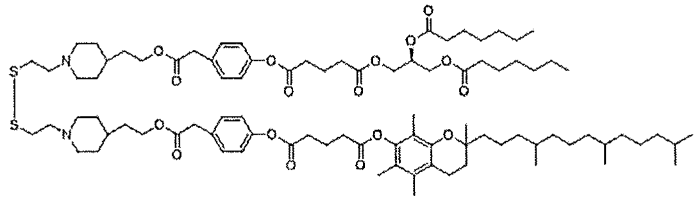 | $R^{3a}$: formula (16-20)<br>$X^4$: O<br>$R^{36}$: trimethylene<br>$R^{49}$: hexyl<br>$R^{50}$: hexyl | $R^{3b}$: $R^{3c}$-CO-$(CH_2)_p$-<br>p: 3<br>$R^{3c}$: tocopherol residue |
| compound 156 | 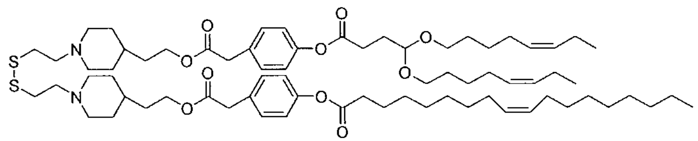 | $R^{3a}$: formula (11)<br>$R^{26}$:<br>ethylene $R^{27}$,$R^{28}$: (Z)-5-octenyl | $R^{3b}$: (Z)-8-heptadecenyl |
| compound 157 | 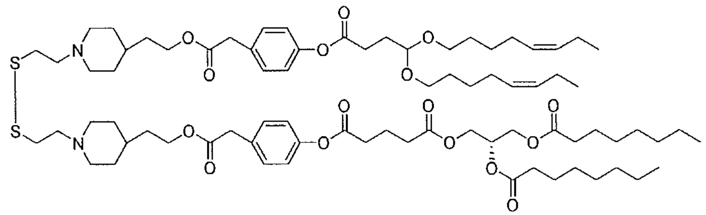 | $R^{3a}$: formula (16-20)<br>$X^4$: O<br>$R^{36}$: trimethylene<br>$R^{49}$: heptyl<br>$R^{50}$: heptyl | $R^{3b}$: formula (11)<br>$R^{26}$: ethylene<br>(Z)-5-octenyl |
| compound 158 | 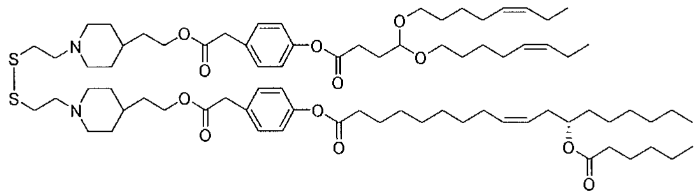 | $R^{3a}$: formula (15)<br>$R^{35}$: pentyl | $R^{3b}$: formula (11)<br>$R^{26}$: ethylene<br>$R^{27}$,$R^{28}$: (Z)-5-octenyl |

[Table 1-26]

| name | structure | $R^{3a}$ | $R^{3b}$ |
|---|---|---|---|
| compound 159 | 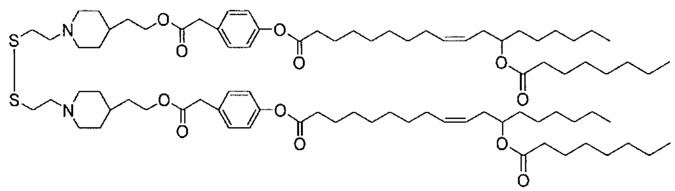 | $R^{3a}$: formula (15)<br>$R^{35}$: heptyl | $R^{3b}$: formula (15)<br>$R^{35}$: heptyl |
| compound 160 | 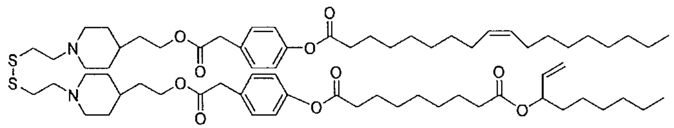 | $R^{3a}$: formula (14)<br>$X^3$: O<br>$R^{33}$: hepta-methylene<br>$R^{34}$: 1-ethenyl-hexyl | $R^{3b}$: (Z)-8-heptadecenyl |
| compound 161 | 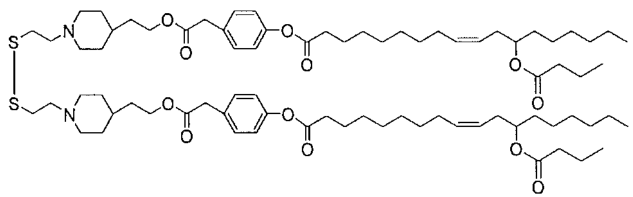 | $R^{3a}$: formula (15)<br>$R^{35}$: propyl | $R^{3b}$: formula (15)<br>$R^{35}$: propyl |
| compound 162 | 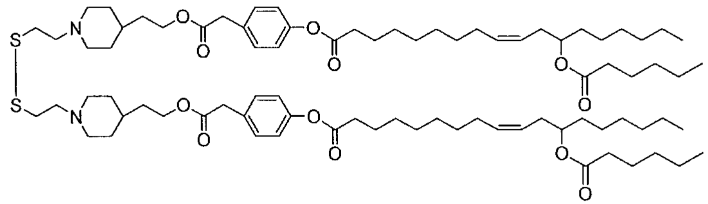 | $R^{3a}$: formula (15)<br>$R^{35}$: pentyl | $R^{3b}$: formula (15)<br>$R^{35}$: heptyl |
| compound 163 | 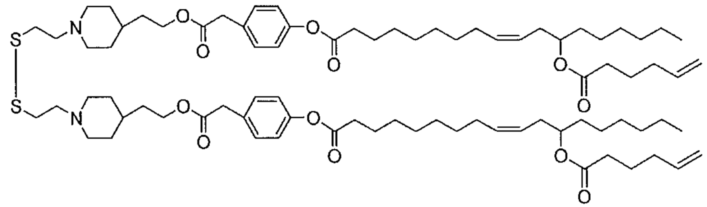 | $R^{3a}$: formula (15)<br>$R^{35}$: 4-pentenyl | R3b: formula (15)<br>$R^{35}$: 4-pentenyl |

(continued)

| name | structure | $R^{3a}$ | $R^{3b}$ |
|---|---|---|---|
| compound 164 | 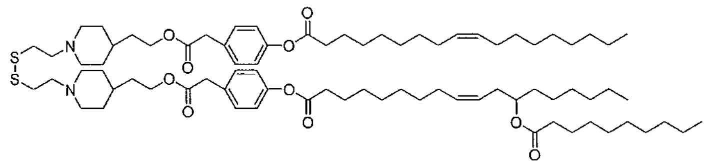 | $R^{3a}$: formula (15)<br>$R^{35}$: nonyl | $R^{3b}$: (Z)-8-heptadecenyl |
| compound 165 | 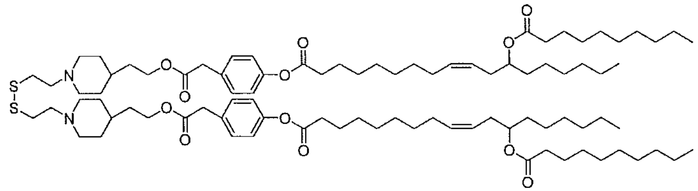 | $R^{3a}$: formula (15)<br>$R^{35}$: nonyl | $R^{3b}$: formula (15)<br>$R^{35}$: nonyl |

[Table 1-27]

| name | structure | $R^{3a}$ | $R^{3b}$ |
|---|---|---|---|
| compound 166 | 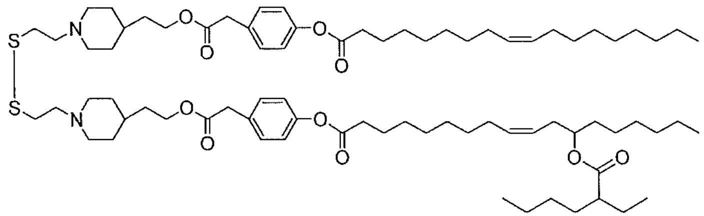 | $R^{3a}$: formula (15)<br>$R^{35}$: 1-ethyl-pentyl | $R^{3b}$: (Z)-8-heptadecenyl |
| compound 167 | 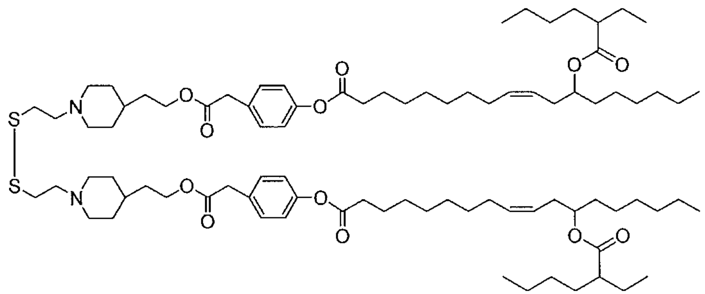 | $R^{3a}$: formula (15)<br>$R^{35}$: 1-ethyl-pentyl | $R^{3b}$: formula (15)<br>$R^{35}$: 1-ethyl-pentyl |
| compound 168 | 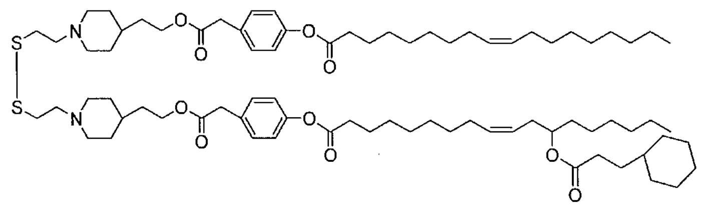 | $R^{3a}$: formula (15)<br>$R^{35}$: 2-cyclohexyl-ethyl | $R^{3b}$: (Z)-8-heptadecenyl |
| compound 169 | 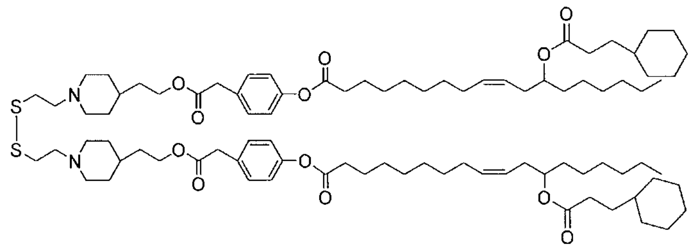 | $R^{3a}$: formula (15)<br>$R^{35}$: 2-cyclohexyl-ethyl | $R^{3b}$: formula (15)<br>$R^{35}$: 2-cyclohexyl-ethyl |

(continued)

| name | structure | $R^{3a}$ | $R^{3b}$ |
|---|---|---|---|
| compound 170 | 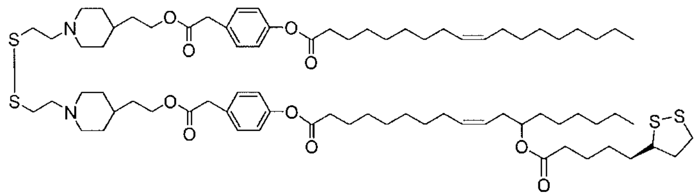 | $R^{3a}$: formula (15)<br>$R^{35}$: 4-(1,2-dithi-olan-3-yl)butyl | $R^{3b}$: (Z)-8-heptadecenyl |
| compound 171 | 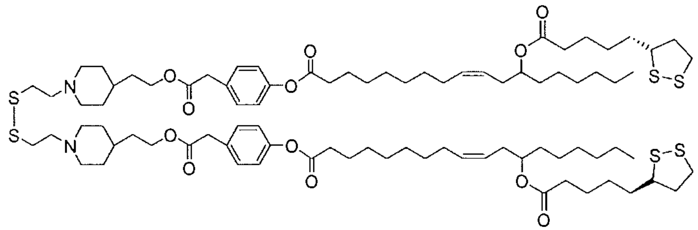 | $R^{3a}$: formula (15)<br>$R^{35}$: 4-(1,2-dithi-olan-3-yl)butyl | $R^{3b}$: formula (15)<br>$R^{35}$: 4-(1,2-dithi-olan-3-yl)butyl |

[Table 1-28]

| name | structure | $R^{3a}$ | $R^{3b}$ |
|---|---|---|---|
| compound 172 | 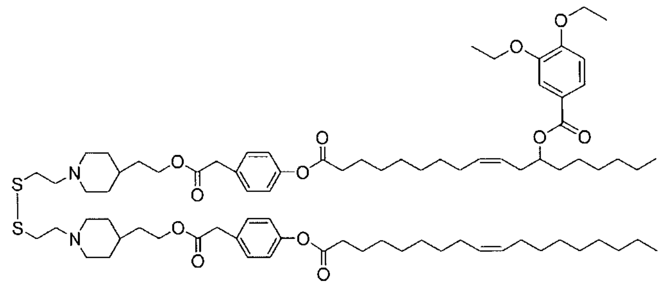 | $R^{3a}$: formula (15)<br>$R^{35}$: 3,4-diethoxyphenyl | $R^{3b}$: (Z)-8-heptadecenyl |
| compound 173 | 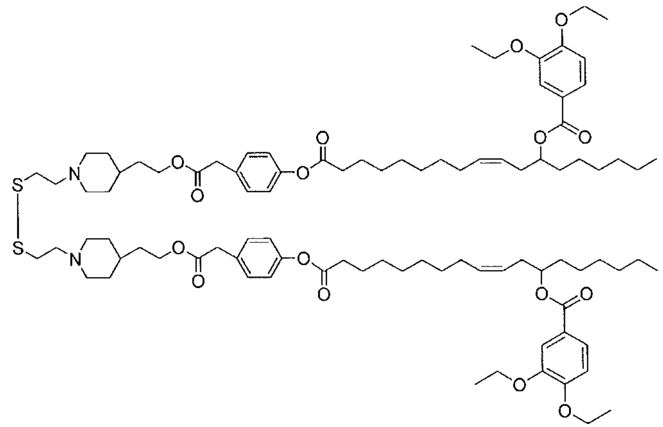 | $R^{3a}$: formula (15)<br>$R^{35}$: 3,4-diethoxyphenyl | $R^{3b}$: formula (15)<br>$R^{35}$: 3,4-diethoxyphenyl |
| compound 174 | 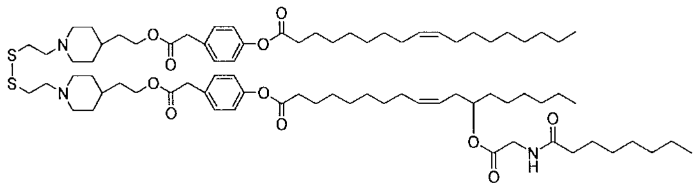 | $R^{3a}$: formula (15)<br>$R^{35}$: (octanoyl-amino) - methyl | $R^{3b}$: (Z)-8-heptadecenyl |

(continued)

| name | structure | $R^{3a}$ | $R^{3b}$ |
|---|---|---|---|
| compound 175 | 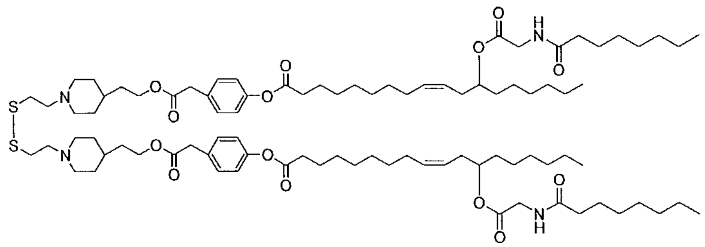 | $R^{3a}$: formula (15)<br><br>$R^{35}$: (octanoyl-amino)-methyl | $R^{3b}$: formula (15)<br><br>$R^{35}$: (octano-ylamino) - methyl |
| compound 176 | 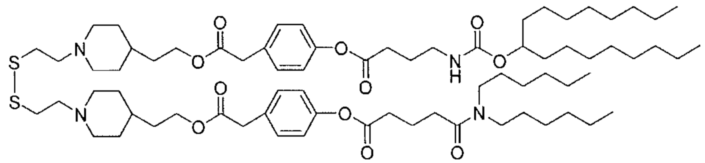 | $R^{3a}$: formula (2)<br>$X^1$: carbamate bond<br>$R^4$: trimethylene<br>$R^5$: (1-octyl)nonyl | $R^{3b}$: formula (5)<br>$X^2$: formula (6)<br>$R^{16}$: trimethylene<br>$R^{17}$: hexyl<br>$R^{18}$: hexyl |

[Table 1-29]

| name | structure | $R^{3a}$ | $R^{3b}$ |
|---|---|---|---|
| compound 177 | 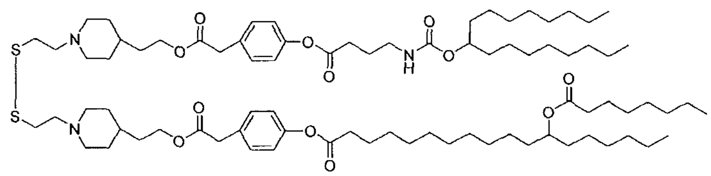 | $R^{3a}$: formula (2)<br>$X^1$: carbamate bond<br>$R^4$: trimethylene<br>$R^5$: (1-octyl) nonyl | $R^{3b}$: formula (7)<br>$R^{19}$: $-COR^{20}$<br>$R^{20}$: heptyl |
| compound 178 | 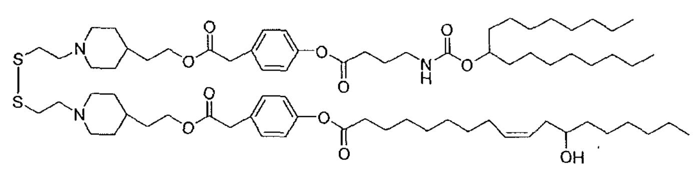 | $R^{3a}$: formula (2)<br>$X^1$: carbamate bond<br>$R^4$: trimethylene<br>$R^5$: (1-octyl) nonyl | $R^{3b}$: formula (7)<br>$R^{19}$: H |
| compound 179 | 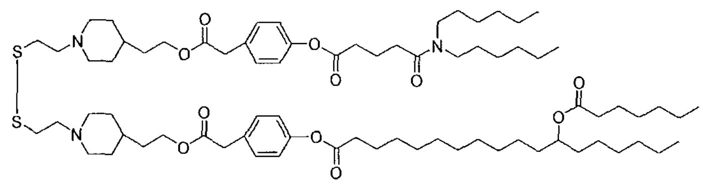 | $R^{3a}$: formula (5)<br>$X^2$: formula (6)<br>$R^{16}$: tri-methylene<br>$R^{17}$: hexyl<br>$R^{18}$: hexyl | $R^{3b}$: formula (7)<br>$R^{19}$: $-COR^{20}$<br>$R^{20}$: heptyl |
| compound 180 | 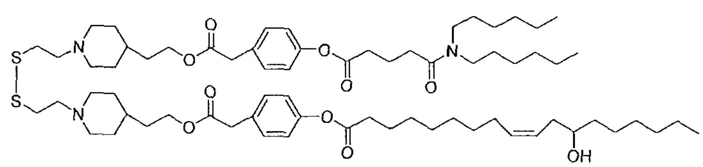 | $R^{3a}$: formula (5)<br>$X^2$: formula (6)<br>$R^{16}$: trimethylene<br>$R^{17}$: hexyl<br>$R^{18}$: hexyl | $R^{3b}$: formula (7)<br>$R^{19}$: H |
| compound 181 | 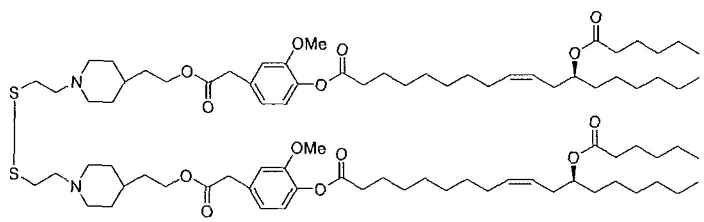 | $R^{3a}$: formula (15)<br>$R^{35}$: pentyl | $R^{3b}$: formula (15)<br>$R^{35}$: pentyl |
| compound 182 | 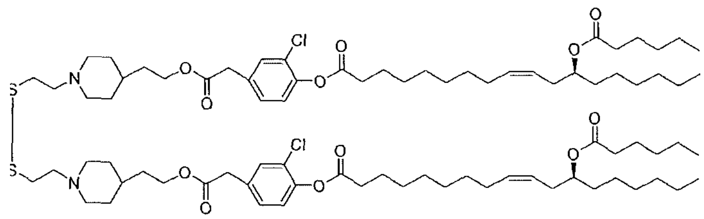 | $R^{3a}$: formula (15)<br>$R^{35}$: pentyl | $R^{3b}$: formula (15)<br>$R^{35}$: pentyl |
| compound 183 | 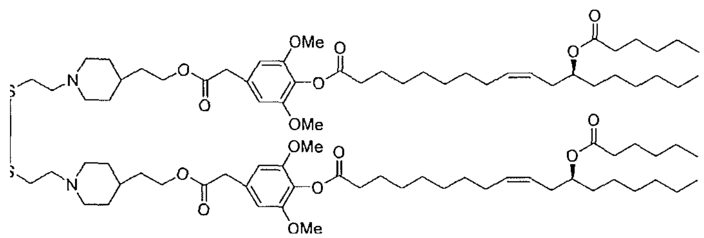 | $R^{3a}$: formula (15)<br>$R^{35}$: pentyl | $R^{3b}$: formula (15)<br>$R^{35}$: pentyl |

[Table 1-30]

| name | structure | $R^{3a}$ | $R^{3b}$ |
|---|---|---|---|
| compound 184 | 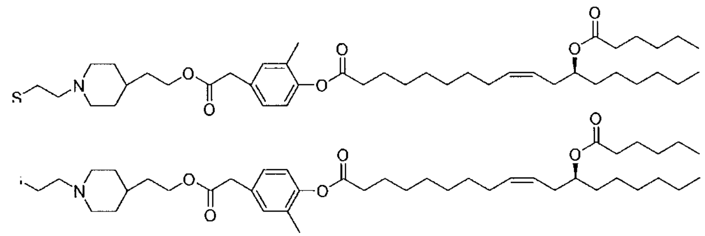 | $R^{3a}$: formula (15)<br>$R^{35}$: pentyl | $R^{3b}$: formula (15)<br>$R^{35}$: pentyl |
| compound 185 | 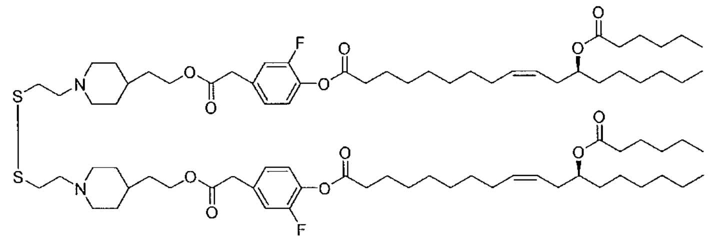 | $R^{3a}$: formula (15)<br>$R^{35}$: pentyl | $R^{3b}$ : formula (15)<br>$R^{35}$: pentyl |
| compound 186 | 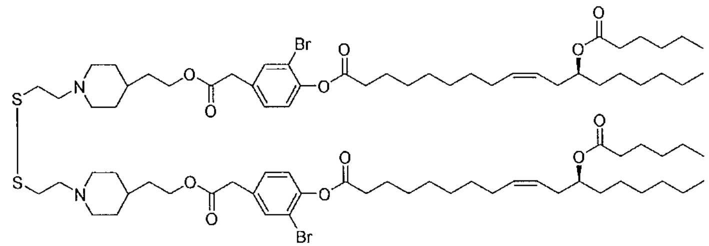 | $R^{3a}$: formula (15)<br>$R^{35}$: pentyl | $R^{3b}$ : formula (15)<br>$R^{35}$: pentyl |
| compound 187 | 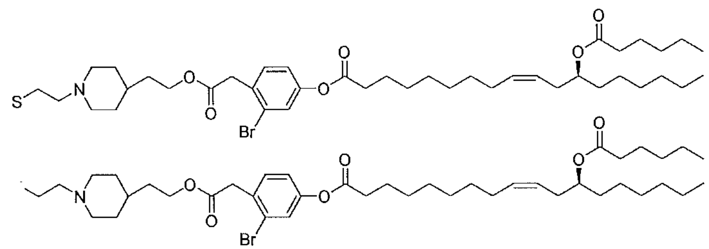 | $R^{3a}$: formula (15)<br>$R^{35}$: pentyl | $R^{3b}$: formula (15)<br>$R^{35}$: pentyl |

(continued)

| name | structure | $R^{3a}$ | $R^{3b}$ |
|---|---|---|---|
| compound 188 | 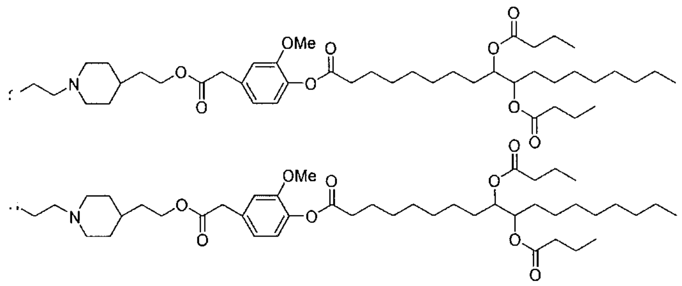 | $R^{3a}$: formula (19)<br>$R^{45}$ : heptamethylene<br>$R^{46}$: ethyl<br>$R^{47}$: octyl<br>$R^{48}$: ethyl | $R^{3a}$: formula<br>(19) $R^{45}$: heptamethylene<br>$R^{46}$: ethyl<br>$R^{47}$: octyl<br>$R^{48}$: ethyl |
| compound 189 | 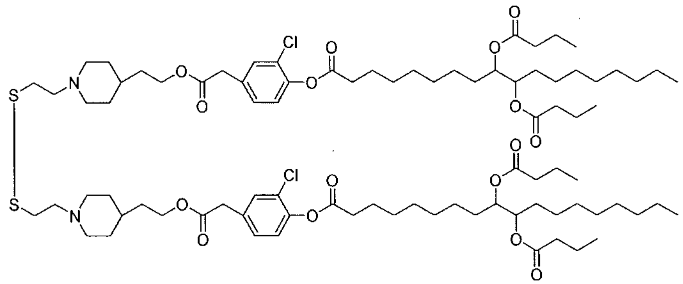 | $R^{3a}$: formula (19)<br>$R^{45}$ : heptamethylene<br>$R^{46}$: ethyl<br>$R^{47}$: octyl<br>$R^{48}$: ethyl | $R^{3a}$: formula(19)<br>$R^{45}$: heptamethylene<br>$R^{46}$: ethyl<br>$R^{47}$: octyl<br>$R^{48}$: ethyl |

[Table 1-31]

| name | structure | $R^{3a}$ | $R^{3b}$ |
|---|---|---|---|
| compound 190 | 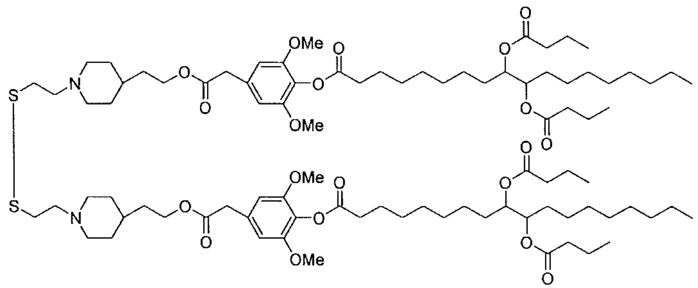 | $R^{3a}$; formula (19)<br>$R^{45}$ : hepta-nethylene<br>$R^{46}$: ethyl<br>$R^{47}$: octyl<br>$R^{48}$: ethyl | $R^{3a}$: formula (19)<br>$R^{45}$: heptamethylene<br>$R^{46}$: ethyl<br>$R^{47}$: octyl<br>$R^{48}$: ethyl |
| compound 191 | 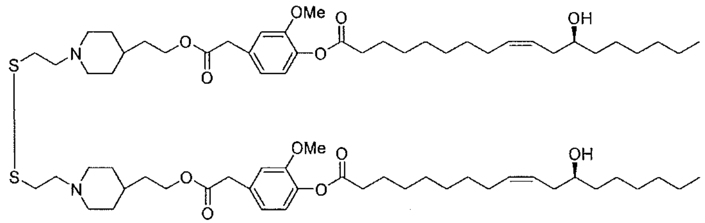 | $R^{3a}$: formula (9)<br>$R^{23}$: H | $R^{3b}$: formula (9)<br>$R^{23}$: H |
| compound 192 | 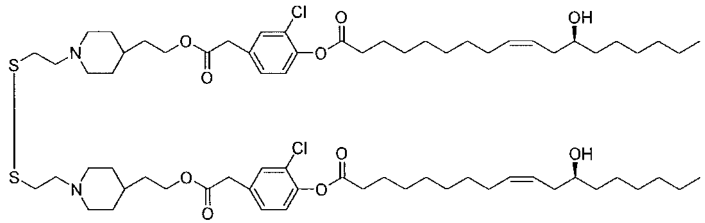 | $R^{3a}$: formula (9)<br>$R^{23}$: H | $R^{3b}$: formula (9)<br>$R^{23}$: H |
| compound 193 | 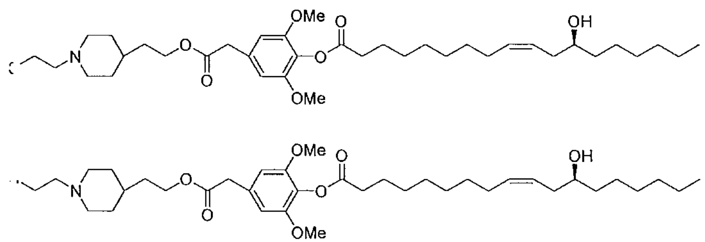 | $R^{3a}$ : formula (9)<br>$R^{23}$: H | $R^{3b}$: formula (9)<br>$R^{23}$: H |

(continued)

| name | structure | $R^{3a}$ | $R^{3b}$ |
|---|---|---|---|
| compound 194 | 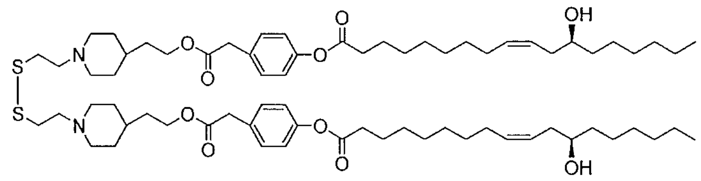 | $R^{3a}$: formula (9)<br>$R^{19}$: H | $R^{3b}$ : formula (9)<br>$R^{19}$: H |
| compound 195 | 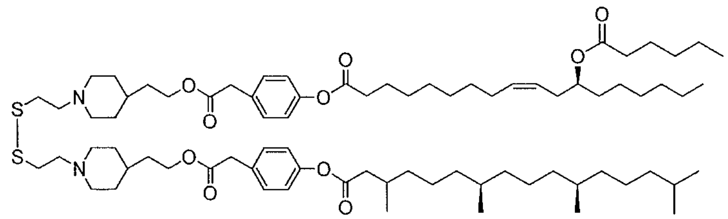 | $R^{3a}$: $R^{3a}$: formula (15)<br>$R^{35}$ : pentyl | $R^{3b}$: 2,6,10,14-tetramethyl-pentadecyl |

[Table 2]

| | structure |
|---|---|
| Comparative Example | 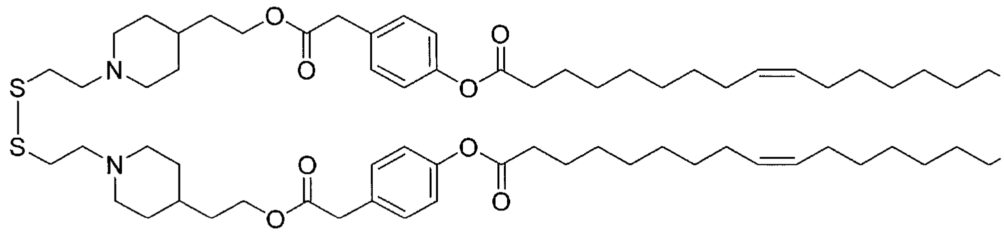 |

[Table 3-1]

| name | structure |
|---|---|
| intermediate 1 | 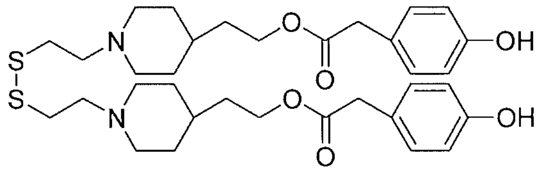 |
| intermediate 2 | 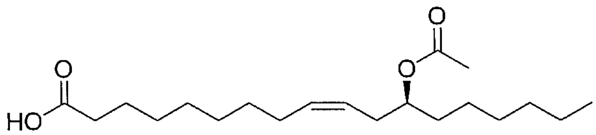 |
| intermediate 3 | 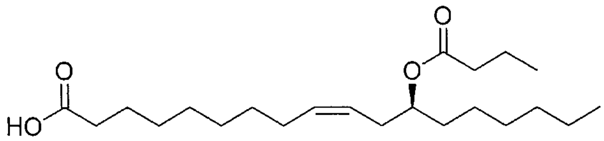 |
| intermediate 4 | 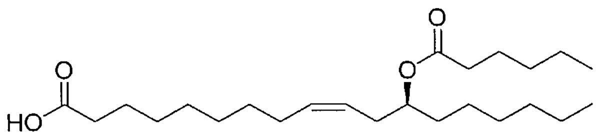 |
| intermediate 5 | 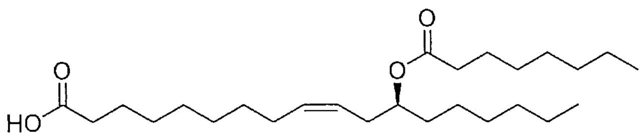 |
| intermediate 6 | 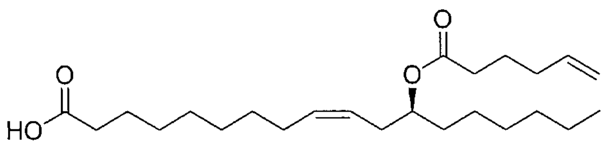 |
| intermediate 7 | 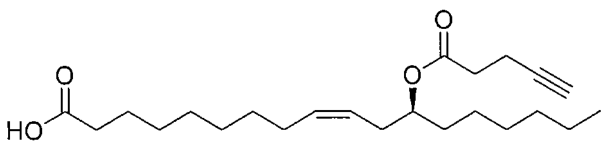 |
| intermediate 8 | 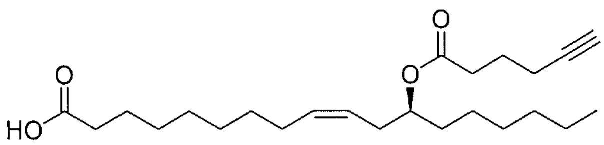 |

(continued)

| name | structure |
|---|---|
| intermediate 9 | 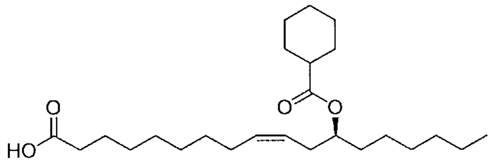 |
| intermediate 10 | 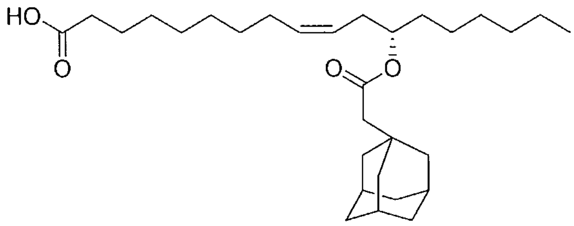 |

[Table 3-2]

| name | structure |
|---|---|
| intermediate 11 | 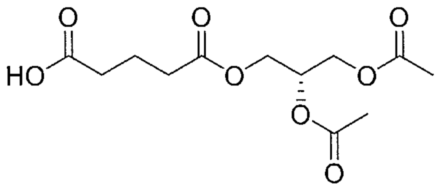 |
| intermediate 12 | 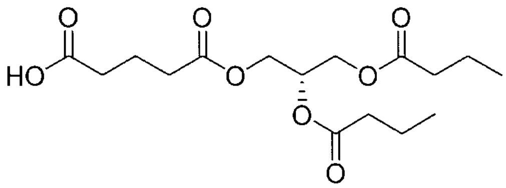 |
| intermediate 13 | 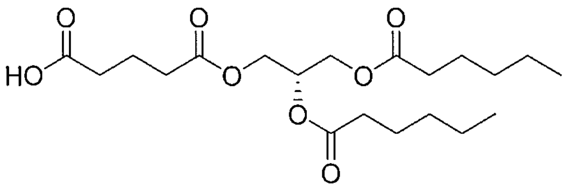 |
| intermediate 14 | 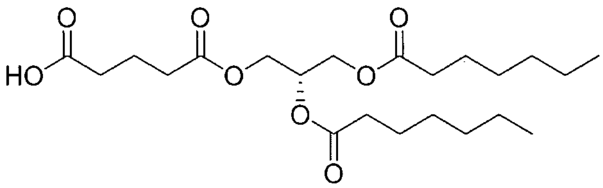 |
| intermediate 15 | 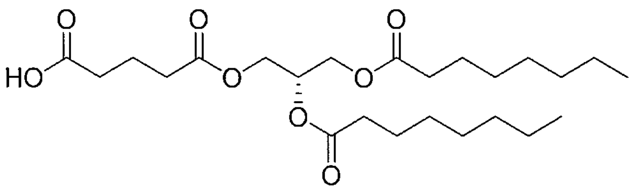 |

(continued)

| name | structure |
|---|---|
| intermediate 16 | 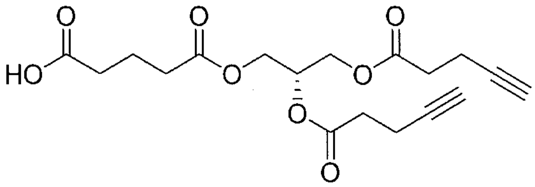 |
| intermediate 17 | 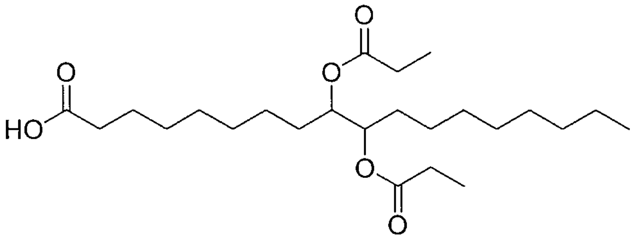 |

[Table 3-3]

| name | structure |
|---|---|
| intermediate 18 | 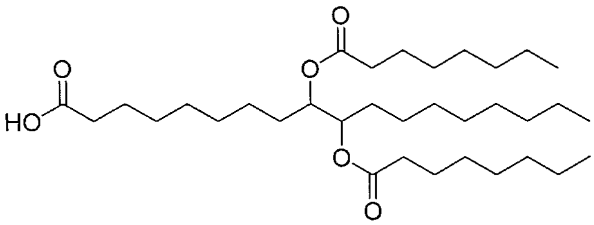 |
| intermediate 19 | 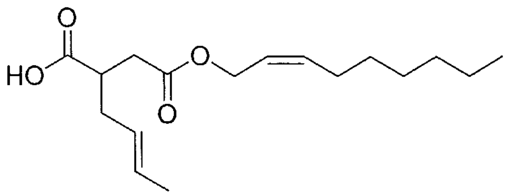 |
| intermediate 20 | 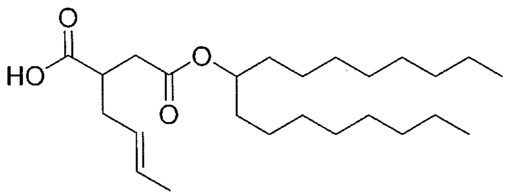 |
| intermediate 21 | 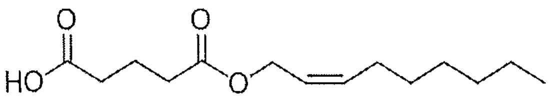 |
| intermediate 22 | 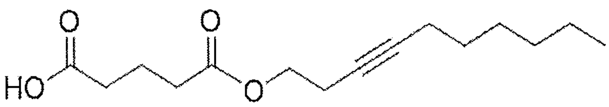 |

(continued)

| name | structure |
|---|---|
| intermediate 23 | 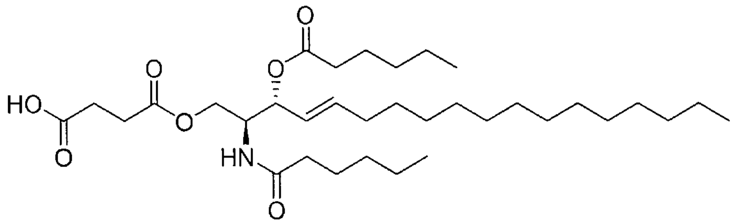 |
| intermediate 24 | 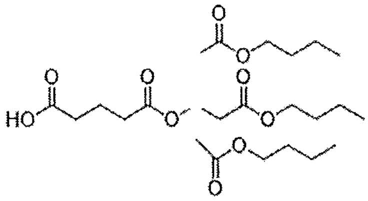 |
| intermediate 25 | 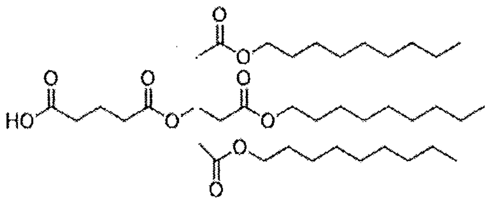 |

[Table 3-4]

| name | structure |
|---|---|
| intermediate 26 | 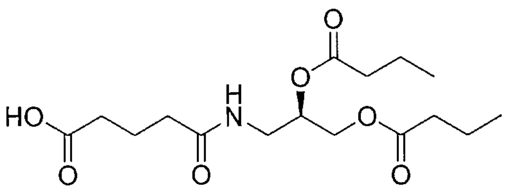 |
| intermediate 27 | 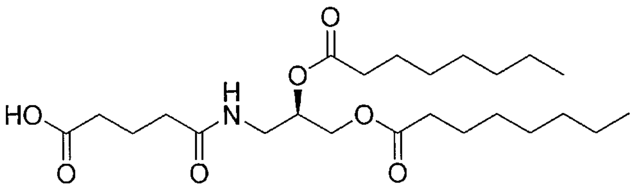 |
| intermediate 28 | 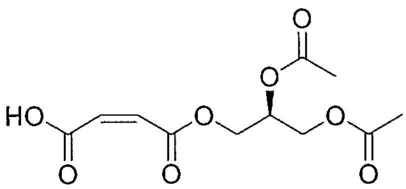 |
| intermediate 29 | 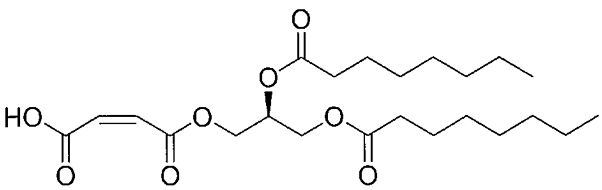 |

(continued)

| name | structure |
|---|---|
| intermediate 30 | 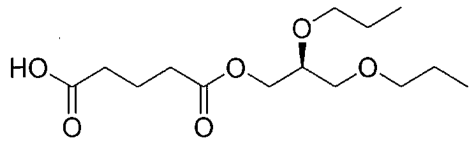 |
| intermediate 31 | 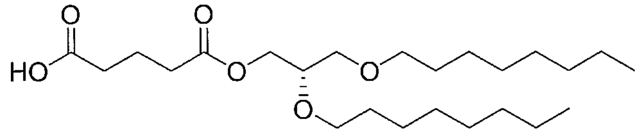 |
| intermediate 32 | 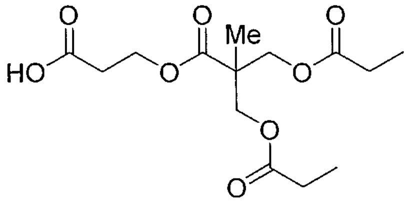 |
| intermediate 33 | 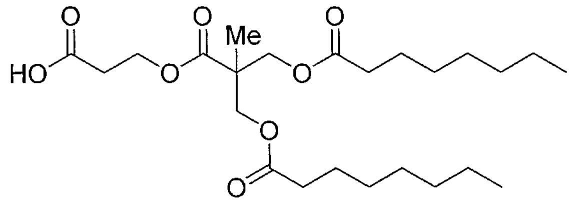 |

[Table 3-5]

| name | structure |
|---|---|
| intermediate 34 | 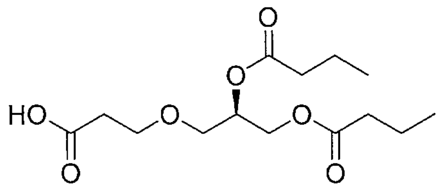 |
| intermediate 35 | 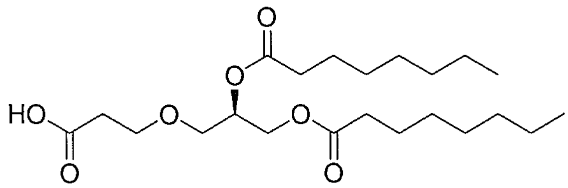 |
| intermediate 36 | 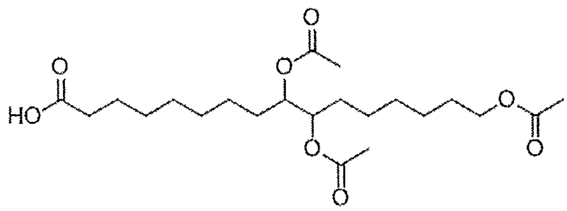 |

(continued)

| name | structure |
|---|---|
| intermediate 37 | 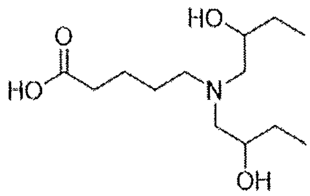 |
| intermediate 38 | 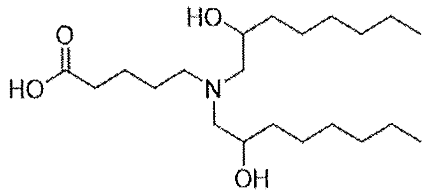 |
| intermediate 39 | 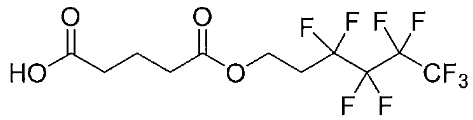 |
| intermediate 40 | 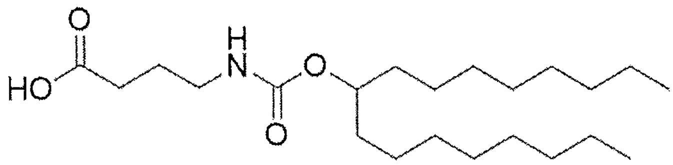 |
| intermediate 41 | 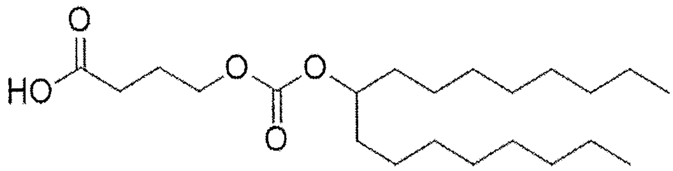 |

[Table 3-6]

| name | structure |
|---|---|
| intermediate 42 | 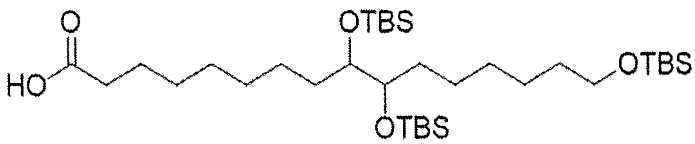 |
| intermediate 43 | 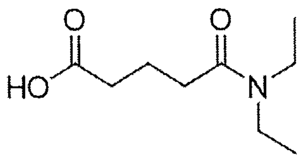 |
| intermediate 44 | 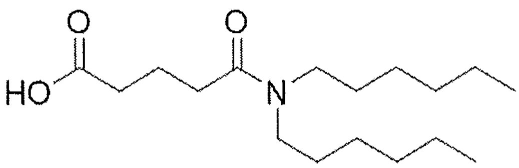 |
| intermediate 45 | 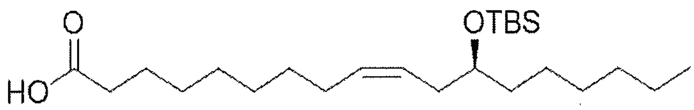 |

(continued)

| name | structure |
|---|---|
| intermediate 47 | 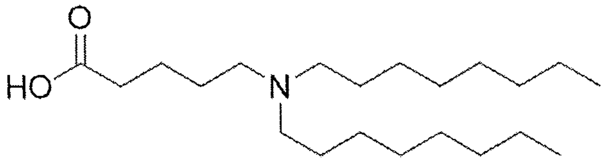 |
| intermediate 48 | 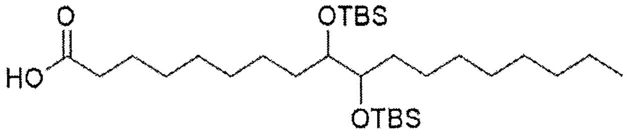 |
| intermediate 49 | 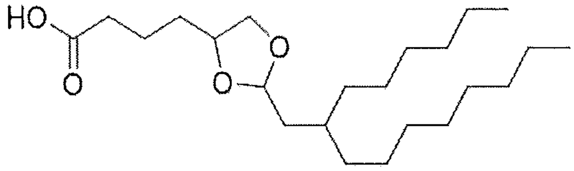 |
| intermediate 50 | 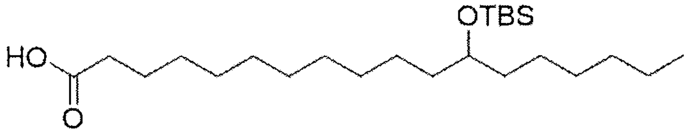 |
| intermediate 51 | 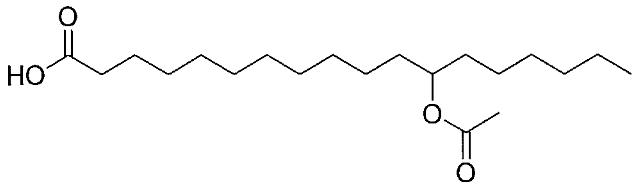 |

[Table 3-7]

| name | structure |
|---|---|
| intermediate 52 | 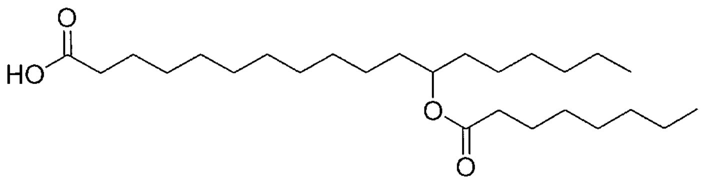 |
| intermediate 53 | 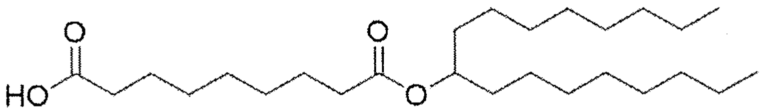 |
| intermediate 54 | 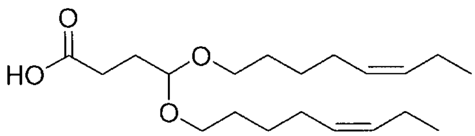 |
| intermediate 55 | 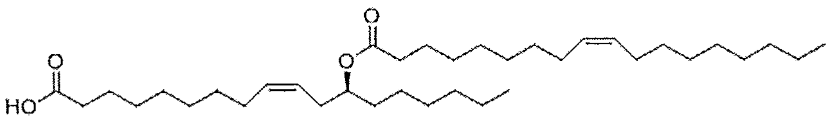 |

(continued)

| name | structure |
|---|---|
| intermediate 57 | 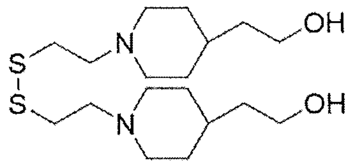 |
| intermediate 58 | 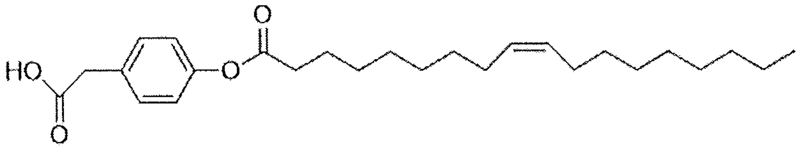 |
| intermediate 59 | 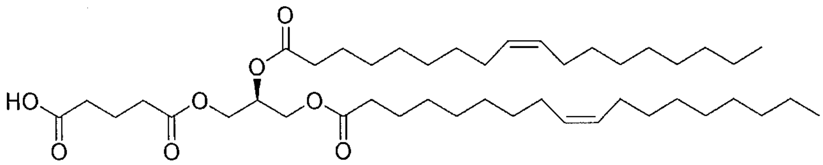 |

[Table 3-8]

| name | structure |
|---|---|
| intermediate 60 | 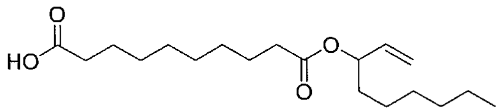 |
| intermediate 61 | 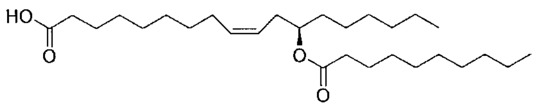 |
| intermediate 62 | 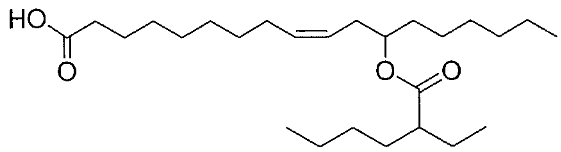 |
| intermediate 63 | 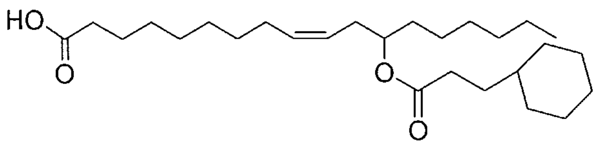 |
| intermediate 64 | 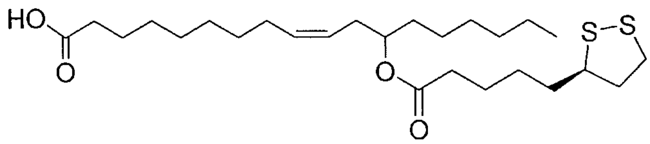 |
| intermediate 65 | 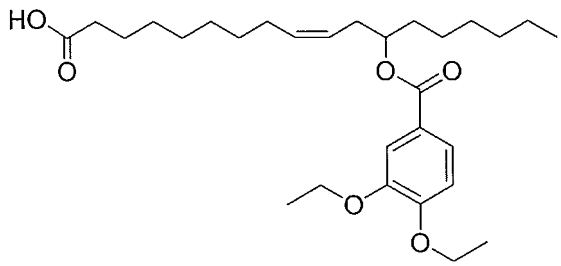 |

(continued)

| name | structure |
|---|---|
| intermediate 66 | 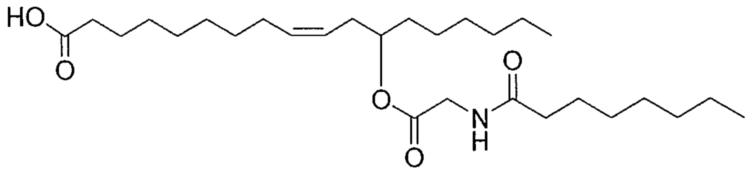 |
| intermediate 67 | 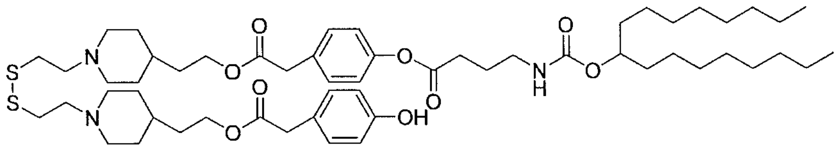 |
| intermediate 68 | 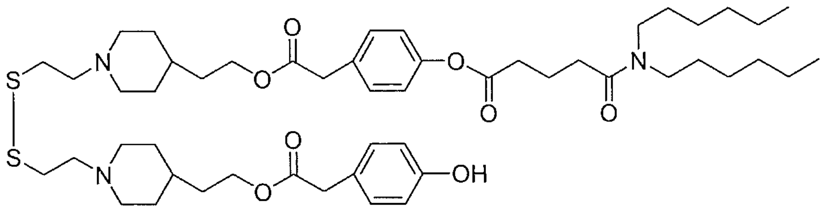 |

[Table 3-9]

| name | structure |
|---|---|
| intermediate 69 | 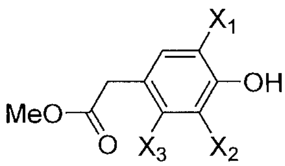 |
| intermediate 70 | 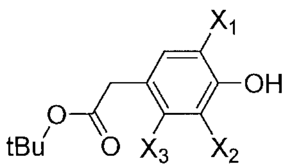 |
| intermediate 71 | 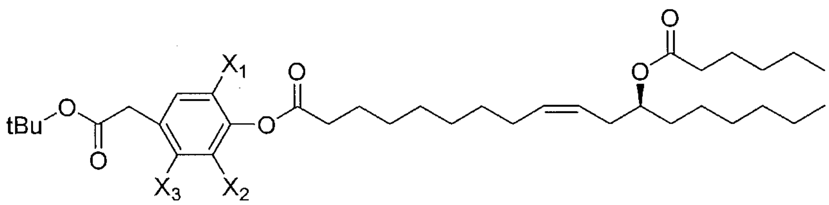 |
| intermediate 72 | 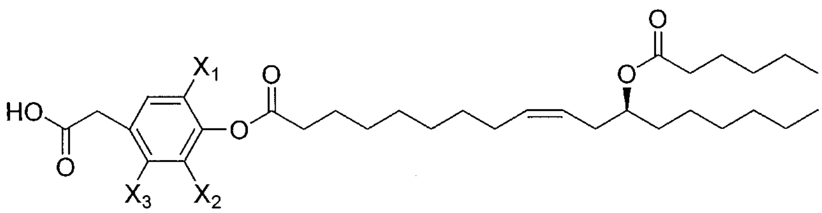 |

(continued)

| name | structure |
|---|---|
| intermediate 73 | 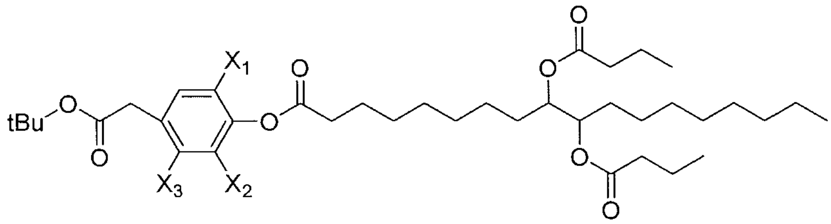 |
| intermediate 74 | 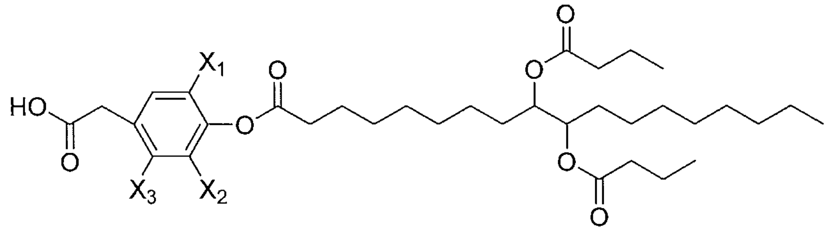 |
| intermediate 75 | 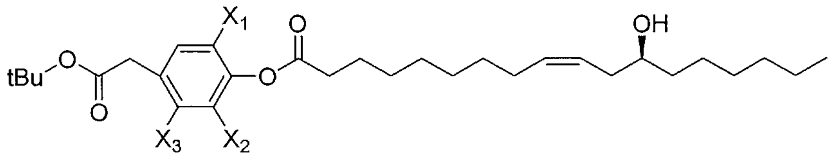 |
| intermediate 76 | 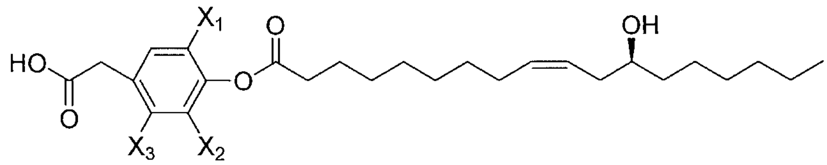 |

[Example 1-1] Synthesis of compound 1

While compound 1 was synthesized by the following synthesis pathway, the present invention is not limited to such synthesis pathway.

[Chem. 96]

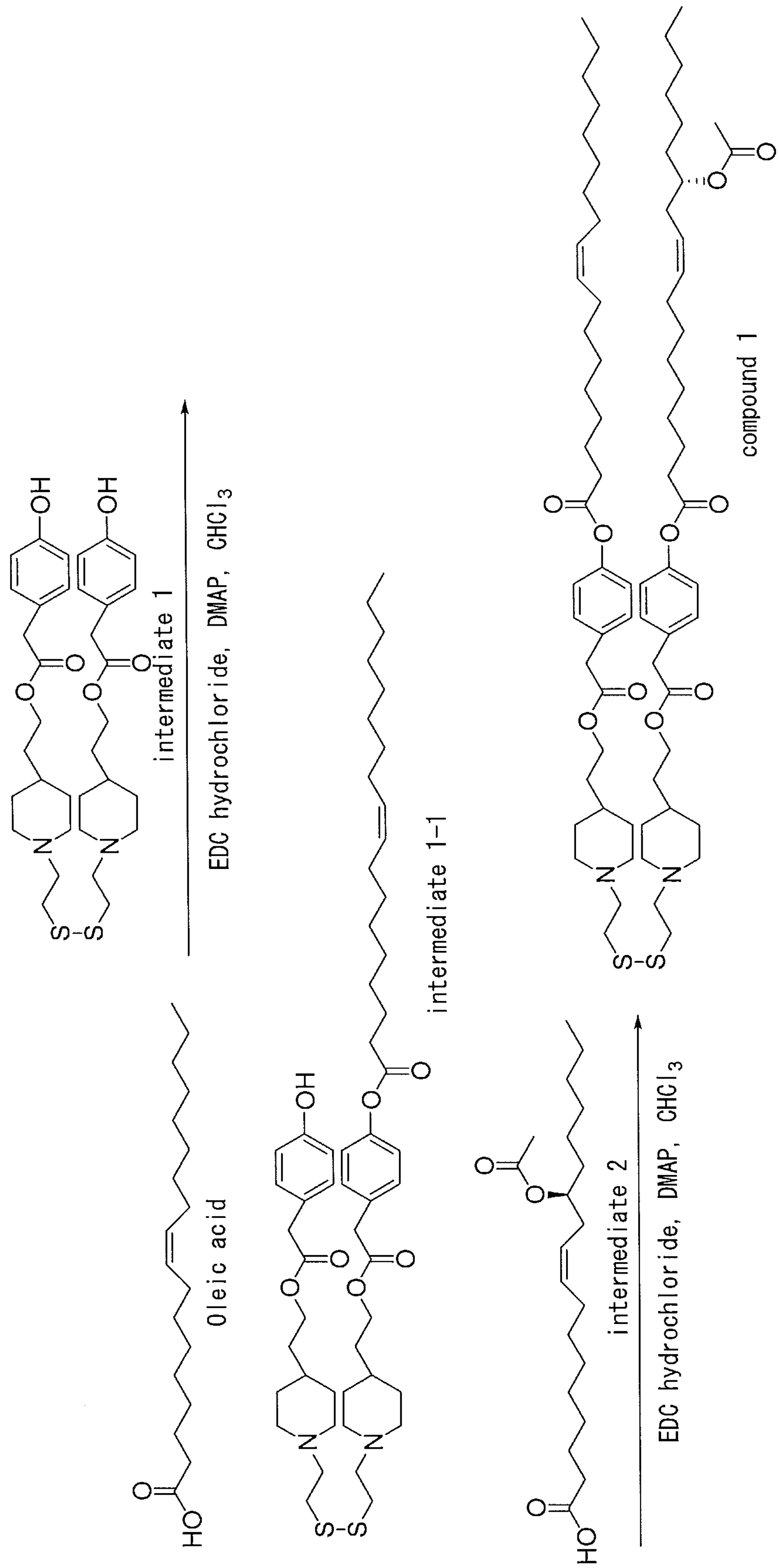


Intermediate 1 was synthesized by the following synthesis pathway.

[Chem. 97]

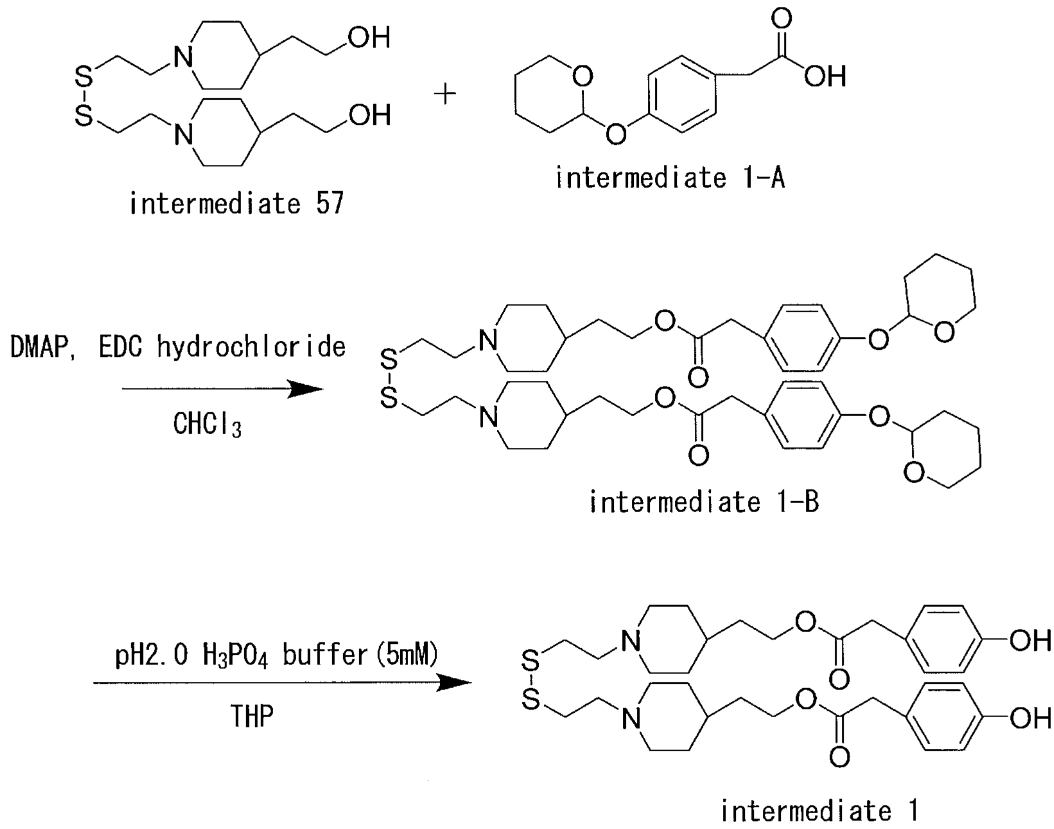


<Synthesis of intermediate 1-A>

4-Hydroxyphenylacetic acid (30.0 g, 197 mmol), and pyridinium p-toluenesulfonate (5.00 g, 19.9 mmol) were dissolved in dichloromethane (120 mL) at room temperature. To the obtained mixture was added dropwise a mixed solution of 3,4-dihydro-2H-pyran (83.0 g, 987 mmol) and dichloromethane (31.1 mL) at 20°C or below and the mixture was reacted at room temperature for 2 hr. Thereafter, the reaction mixture was neutralized with DMAP (12.0 g, 98.2 mmol). To the obtained mixture were added 2-propanol (301 mL), 100 g/L NaOH aqueous solution (160 g), and the mixture was reacted at room temperature for 1 hr. The mixed solution was concentrated by an evaporator, the concentrate was washed with chloroform and neutralized with 6 M hydrochloric acid. The obtained mixture was extracted with chloroform, and the organic layer was dehydrated by adding sodium sulfate. Sodium sulfate was removed by filtration, and the filtrate was concentrated by an evaporator to give 47.0 g of intermediate 1-A.

<Synthesis of intermediate 1-B>

Intermediate 57 (36.0 g, 95.6 mmol) synthesized according to the method described in WO 2016/121942, intermediate 1-A (49.7 g, 210 mmol), and DMAP (4.68 mg, 38.3 mmol) were dissolved in chloroform (240 mL) at room temperature. To the obtained mixture was added EDC hydrochloride (55.1 g, 287 mmol), and the mixture was reacted at room temperature for 2 hr. Thereafter, the reaction solution was washed with 5 wt% sodium dihydrogen phosphate aqueous solution, 9 wt% sodium hydrogen carbonate aqueous solution, and 20 wt% saline, and dehydrated by adding sodium sulfate. Sodium sulfate was removed by filtration, and the filtrate was concentrated by an evaporator to give 77.9 g of intermediate 1-B.

<Synthesis of intermediate 1>

Intermediate 1-B (77.7 g, 95.6 mmol) was dissolved in THF (325 mL) at room temperature. To the obtained mixture were added 2-propanol (319 mL), p-toluenesulfonic acid monohydrate (38.2 g, 201 mmol) and the mixture was reacted at 25°C or below for 1 hr. Thereafter, to the reaction mixture was neutralized with DMAP (25.0 g, 205 mmol). DMAP was removed by filtration, and the filtrate was concentrated by an evaporator. The obtained residue was dissolved in chloroform (627 mL), washed with 0.5 M phosphate buffer (pH=6.5), 0.5 M glycine buffer (pH=9.5) and dehydrated by

adding sodium sulfate. Sodium sulfate was removed by filtration, and toluene (1.56 L) was added to allow for crystallization. The obtained crude product was washed with hexane and vacuum dried to give 40.3 g of intermediate 1.

<$^1$H-NMR (600 MHz, DMSO-$d_6$) of intermediate 1>

δ: 1.07-1.11 (m, 4H), 1.16-1.25 (m, 2H), 1.42-1.59 (m, 8H), 1.79-1.88 (m, 4H), 2.25-2.59 (m, 4H), 2.76-2.89 (m, 8H), 3.49 (s, 4H), 4.02-4.09 (m, 4H), 6.67-6.72 (m, 4H), 7.02-7.07 (m, 4H)

<Synthesis of intermediate 1-1>

Oleic acid (5.11 g, 18.1 mmol), intermediate 1 (16.6 g, 25.8 mmol), and DMAP (630 mg, 5.16 mmol) were dissolved in chloroform (166 mL) at room temperature. To the obtained mixture was added EDC hydrochloride (5.94 g, 31.0 mmol) and the mixture was reacted at room temperature for 2 hr. Thereafter, the reaction solution was washed with 5 wt% sodium hydrogen phosphate aqueous solution, 0.5 M phosphate buffer (pH=2.0), and 20 wt% saline, and dehydrated by adding sodium sulfate. Sodium sulfate was removed by filtration, and the filtrate was concentrated by an evaporator. The obtained residue was purified by silica gel column chromatography (chloroform/ethanol=88/12) to give 8.21 g of intermediate 1-1.

<Synthesis of compound 1 using intermediate 1-1>

Intermediate 1-1 (492 mg, 0.541 mmol) was dissolved in chloroform (2.91 g), intermediate 2 (184 mg, 0.541 mmol) synthesized as described in Example 2-1, DMAP (13.2 mg, 0.108 mmol), and EDC hydrochloride (207 mg, 1.08 mmol) were added, and the mixture was reacted at room temperature for 2 hr. The solution after the reaction was washed with 5 wt% saline, and concentrated by an evaporator, and the concentrate was subjected to silica gel column purification using ethanol/chloroform to give compound 1 (498 mg).

<$^1$H-NMR (600 MHz, $CDCl_3$) of compound 1>

δ: 0.86-0.90 (t, 6H), 1.22-1.42 (m, 42H), 1.54-1.76 (m, 14H), 1.91-1.98 (m, 4H), 2.00-2.06 (m, 9H), 2.23-2.34 (m, 2H), 2.52-2.55 (m, 4H), 2.62-2.66 (m, 4H), 2.79-2.92 (m, 8H), 3.59 (s, 4H), 4.11-4.13 (t, 4H), 4.84-4.90 (m, 1H), 5.30-5.38 (m, 3H), 5.45-5.50 (m, 1H), 7.02-7.04 (m, 4H), 7.26-7.30 (m, 4H)

[Example 1-2] Synthesis of Compound 1 by synthesis pathway different from Example 1-1

Compound 1 was also synthesized by the following synthesis pathway.

[Chem. 98]

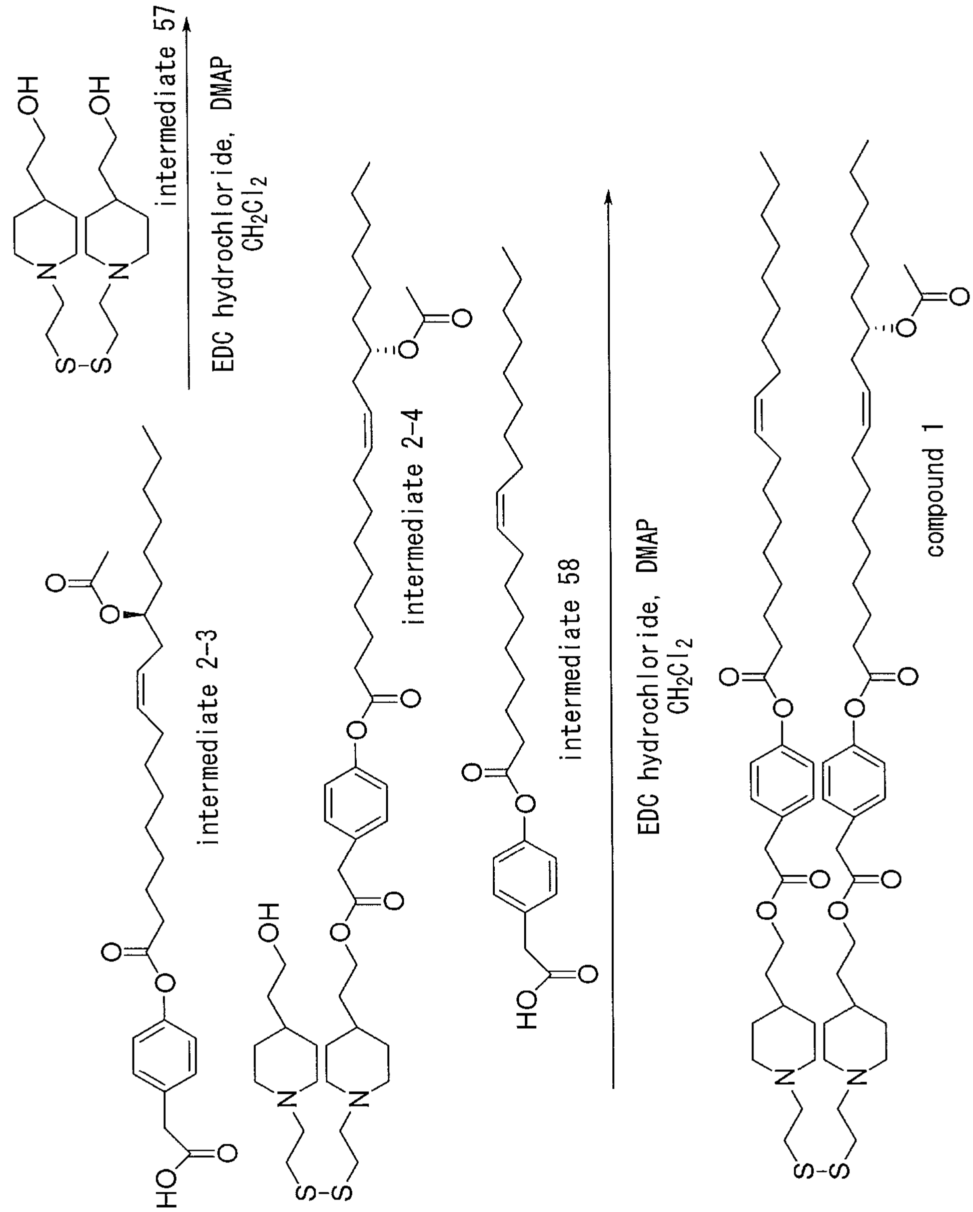


<Synthesis of intermediate 2-4>

Intermediate 2-3 (122 mg, 0.258 mmol) obtained by the synthesis described in Example 2-2 was dissolved in dichloromethane (2.91 g), intermediate 57 (100 mg, 0.258 mmol) synthesized according to the method described in WO 2016/121942, DMAP (6.30 mg, 0.0516 mmol) and EDC hydrochloride (9.20 mg, 0.310 mmol) were added, and the mixture was reacted at room temperature for 2 hr. The solution after the reaction was washed with 5 wt% saline, and concentrated by an evaporator, and the concentrate was subjected to silica gel column purification using ethanol/chloroform to give 75.2 mg of intermediate 2-4.

<Synthesis of compound 1 using intermediate 2-4>

Intermediate 2-4 (217 mg, 0.260 mmol) was dissolved in dichloromethane (3.26 g), intermediate 58 (119 mg, 0.286 mmol) obtained as mentioned below, DMAP (6.35 mg, 0.052 mmol) and EDC hydrochloride (99.7 mg, 0.520 mmol) were added and the mixture was reacted at room temperature for 2 hr. The solution after the reaction was washed with 5 wt% saline, concentrated by an evaporator, and the residue was purified by silica gel column chromatography (chloroform/ethanol=92/8 ((volume ratio))) to give 224 mg of compound 1.

<Synthesis of intermediate 58-1>

Oleic acid (0.438 g, 1.55 mmol) was dissolved in chloroform (4.38 g), 2-(4-hydroxyphenyl)tert-butyl acetate (0.354 g, 1.70 mmol), DMAP (37.9 mg, 0.310 mmol) and EDC hydrochloride (386 mg, 2.02 mmol) were added and the mixture was reacted at room temperature for 2 hr. The solution after the reaction was washed with 0.1 M hydrochloric acid, 5 wt% sodium hydrogen carbonate aqueous solution, and ion-exchanged water, and concentrated by an evaporator. The concentrate was dissolved in hexane, subjected to adsorption purification using silica gel PSQ100B, the filtrate after filtration was concentrated by an evaporator, and the concentrate was vacuum dried to give 0.433 g of intermediate 58-1.

[Chem. 99]

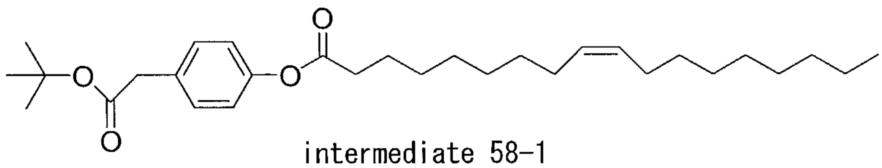

intermediate 58-1

<Synthesis of intermediate 58>

Intermediate 58-1 (401 mg, 0.850 mmol) was dissolved in acetonitrile (1.60 g), a diluted solution of methanesulfonic acid (86.6 mg, 0.901 mmol) in acetonitrile (0.401 g) was added and the mixture was reacted at room temperature for 2 hr. To the solution after the reaction was added chloroform, and the mixture was washed with ion-exchanged water. After washing, the mixture was concentrated by an evaporator, and the residue was purified by silica gel column purification using chloroform/ethanol to give 142 mg of intermediate 58.

[Example 2-1] Synthesis of compound 2

Compound 2 was synthesized by the following synthesis pathway, but the present invention is not limited to such synthesis pathway.

[Chem. 100]

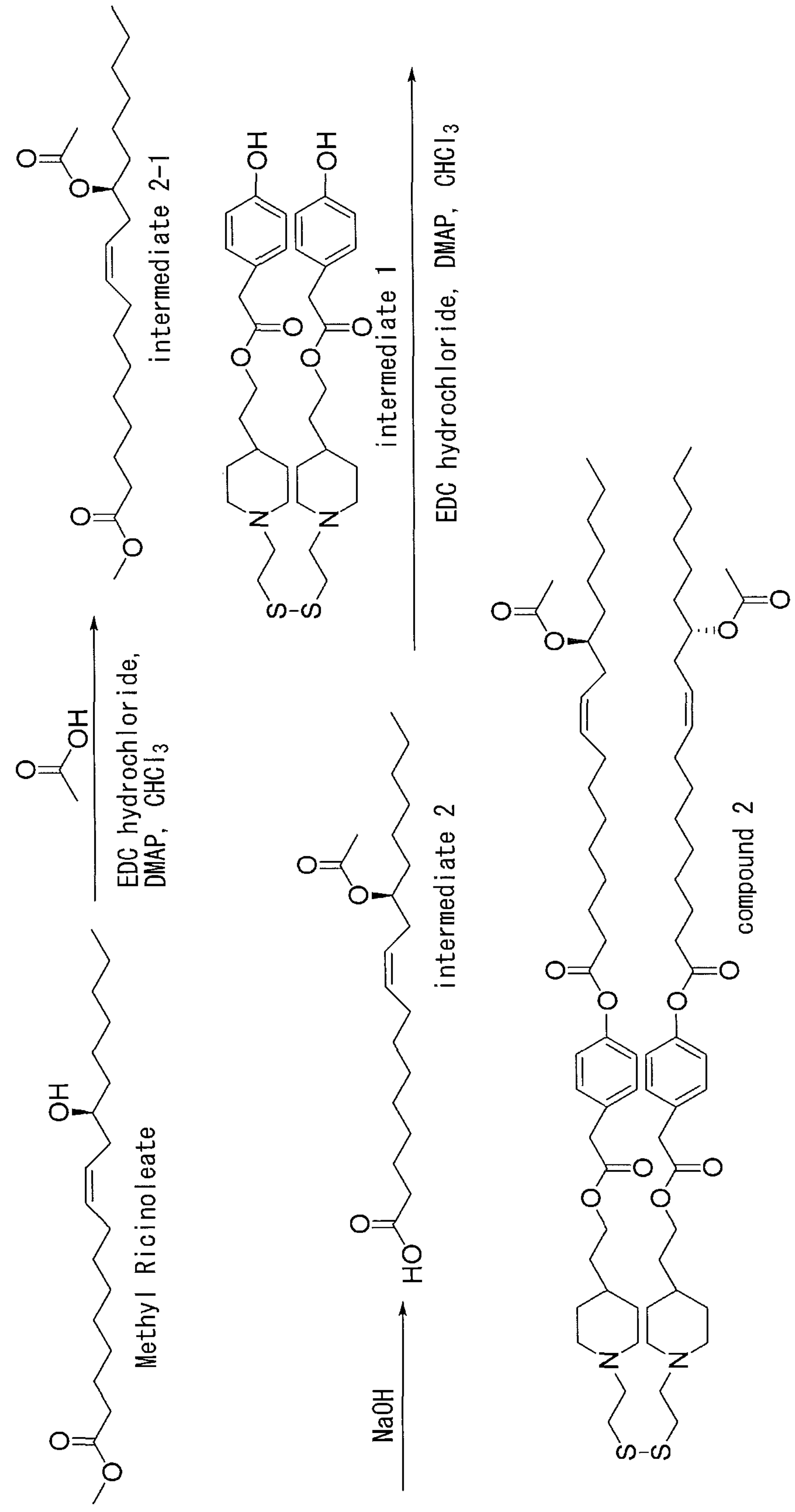
Methyl Ricinoleate
EDC hydrochloride,
DMAP, $CHCl_3$
intermediate 2-1
NaOH
intermediate 2
intermediate 1
EDC hydrochloride, DMAP, $CHCl_3$
compound 2

<Synthesis of intermediate 2-1>

Methyl ricinoleate (4.96 g, 15.9 mmol), acetic acid (1.05 g, 17.5 mmol), and DMAP (194 mg, 1.59 mmol) were dissolved in chloroform (36.0 g) at room temperature. To the obtained mixture was added EDC hydrochloride (3.35 g, 17.5 mmol) and the mixture was reacted at room temperature for 1 hr. Thereafter, the reaction solution was washed with 0.5 M phosphate buffer (pH 4.0), 7 wt% sodium bicarbonate water, and 20 wt% saline, and dehydrated by adding sodium sulfate. Sodium sulfate was removed by filtration, and the filtrate was concentrated by an evaporator to give 2.20 g of intermediate 2-1.

<Synthesis of intermediate 2>

Intermediate 2-1 (5.61 g, 15.8 mmol) was dissolve in tert-butanol to 0.3 M at room temperature. To the obtained mixture was added 2 M NaOH aqueous solution (8.92 g) and the mixture was reacted at room temperature for 8 hr. Thereafter, 1 M hydrochloric acid was added, and the obtained mixture was extracted with hexane. The organic layer was washed with 20 wt% saline, and dehydrated by adding sodium sulfate. Sodium sulfate was removed by filtration, and the filtrate was concentrated by an evaporator. The obtained residue was purified by silica gel column chromatography (chloroform/ethanol=94/6 ((volume ratio))) to give 2.59 g of intermediate 2.

<Synthesis of compound 2>

Intermediate 2 (368 mg, 1.08 mmol), intermediate 1 (350 mg, 0.542 mmol), and DMAP (26.5 mg, 0.217 mmol) were dissolved in chloroform (5.30 g) at room temperature. To the obtained mixture was added EDC hydrochloride (312 mg, 1.63 mmol) and the mixture was reacted at room temperature for 2 hr. Thereafter, the reaction solution was washed with 5 wt% sodium hydrogen phosphate aqueous solution, and 20 wt% saline, and dehydrated by adding sodium sulfate. Sodium sulfate was removed by filtration, and the filtrate was concentrated by an evaporator. The obtained residue was purified by silica gel column chromatography (chloroform/ethanol=92/8 ((volume ratio))) to give 524 mg of compound 2.

<$^1$H-NMR (600 MHz, $CDCl_3$) of compound 2>

$\delta$: 0.87-0.89 (t, 6H), 1.22-1.42 (m, 38H), 1.54-1.76 (m, 16H), 1.91-1.98 (m, 4H), 2.00-2.06 (m, 10H), 2.24-2.34 (m, 4H), 2.52-2.55 (m, 4H), 2.62-2.66 (m, 4H), 2.79-2.92 (m, 8H), 3.59 (s, 4H), 4.11-4.14 (t, 4H), 4.86-4.88 (m, 2H), 5.31-5.37 (m, 2H), 5.45-5.50 (m, 2H), 7.02-7.04 (m, 4H), 7.26-7.29 (m, 4H)

[Example 2-2] Synthesis of Compound 2 by synthesis pathway different from Example 2-1

Compound 2 was also synthesized by the following synthesis pathway.

[Chem. 101]

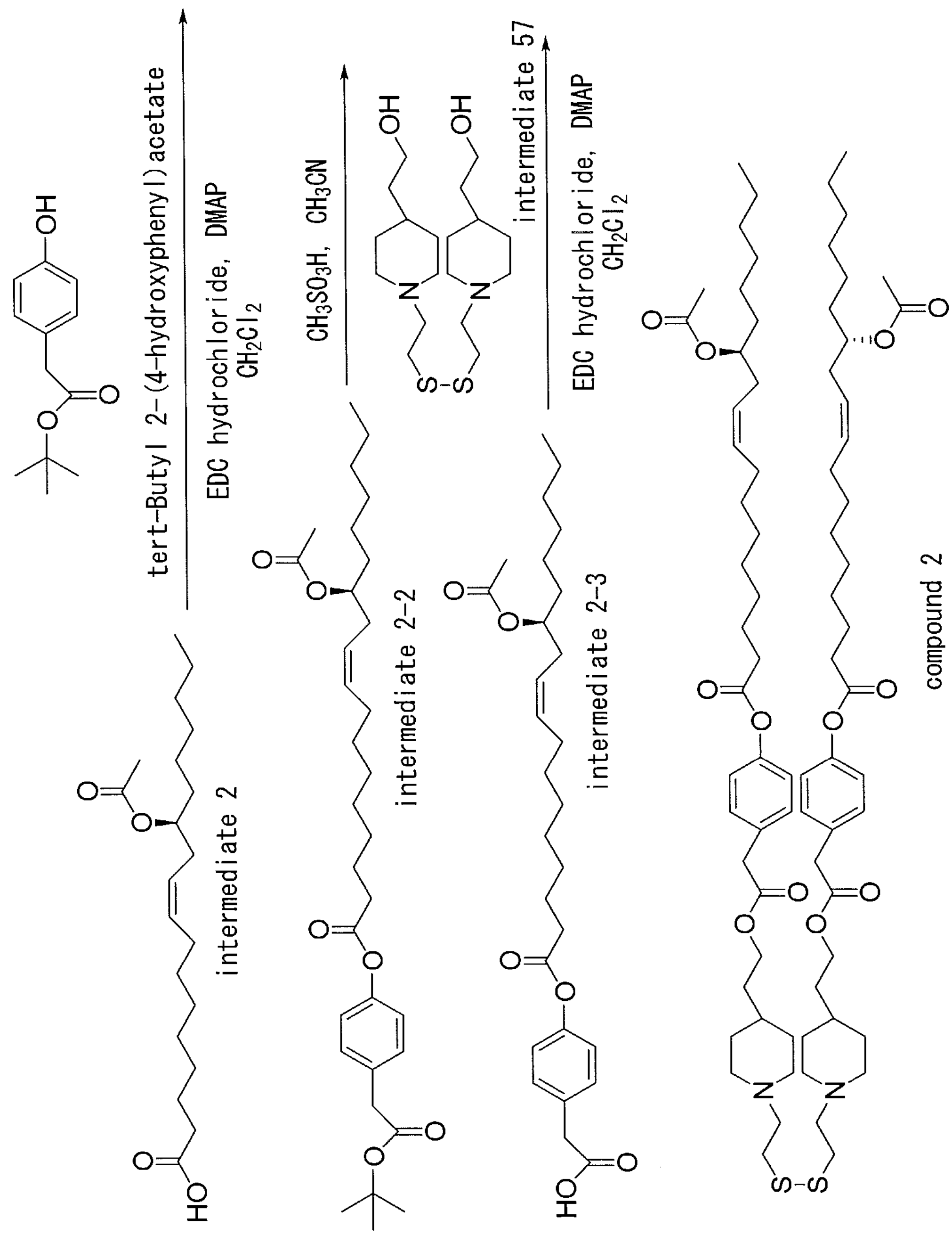


<Synthesis of intermediate 2-2>

Intermediate 2 (0.30 g, 1.44 mmol) was dissolved in chloroform (3.00 g), 2-(4-hydroxyphenyl)tert-butyl acetate (0.329 g, 1.58 mmol), DMAP (35.2 mg, 0.288 mmol) and EDC hydrochloride (359 mg, 1.87 mmol) were added and the mixture was reacted at room temperature for 2 hr. The solution after the reaction was washed with 0.1 M hydrochloric acid, 5 wt% sodium hydrogen carbonate aqueous solution, and ion-exchanged water, and concentrated by an evaporator. The concentrate was dissolved in hexane, the obtained solution was purified by adsorption using silica gel PSQ100B, and the filtrate after the adsorption purification was concentrated by an evaporator. The concentrate was then dried in vacuum to give 0.405 g of intermediate 2-2.

<Synthesis of intermediate 2-3>

Intermediate 2-2 (300 mg, 0.57 mmol) was dissolved in acetonitrile (1.20 g), a diluted solution of methanesulfonic

acid (86.5 mg, 0.900 mmol) of methanesulfonic acid in acetonitrile (0.300 g) was added and the mixture was reacted at room temperature for 2 hr. Chloroform was added to the solution after the reaction, and then the solution was washed with ion-exchanged water. After washing, the solution was concentrated using an evaporator and then purified on a silica gel column using ethanol/chloroform to obtain 153 mg of intermediate 2-3.

<Synthesis of compound 2 using intermediate 2-3>

Intermediate 2-3 (122 mg, 0.26 mmol) was dissolved in dichloromethane (2.91 g), intermediate 57 (100 mg, 0.13 mmol) synthesized according to the method described in WO 2016/121942, DMAP (6.3 mg, 0.05 mmol) and EDC hydrochloride (59.2 mg, 0.33 mmol) were added and the mixture was reacted at room temperature for 2 hr. The solution after the reaction was washed with 5 wt% saline, and concentrated by an evaporator, and the concentrate was subjected to silica gel column purification using ethanol/chloroform to give 50.4 mg of compound 2.

[Example 3] Synthesis of compound 3

<Synthesis of intermediate 55-1>

Methyl ricinoleate (5.00 g, 16.0 mmol), oleic acid (4.97 g, 17.6 mmol), and DMAP (195 mg, 1.60 mmol) were dissolved in chloroform (50.0 g) at room temperature. To the obtained mixture was added EDC hydrochloride (3.37 g, 17.6 mmol) and the mixture was reacted at room temperature for 1 hr. The subsequent steps were performed in the same manner as in the synthesis of intermediate 2-1 described in Example 2-1 to obtain 7.38 g of intermediate 55-1.

[Chem. 102]

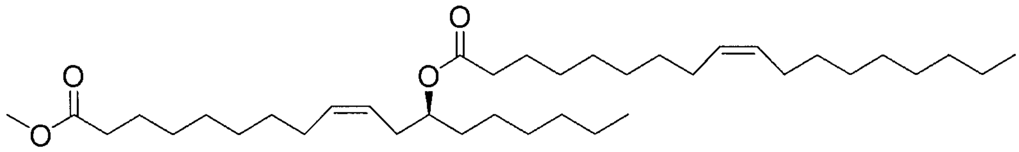

intermediate 55-1

<Synthesis of intermediate 55>

Intermediate 55-1 (7.00 g, 12.1 mmol) was dissolved in tert-butanol to 0.3 M at room temperature. To the obtained mixture was added 2 M NaOH aqueous solution (26.6 g) and the mixture was reacted at room temperature for 8 hr. The subsequent steps were performed in the same manner as in the synthesis of intermediate 2 described in Example 2-1 to obtain 5.59 g of intermediate 55.

[Chem. 103]

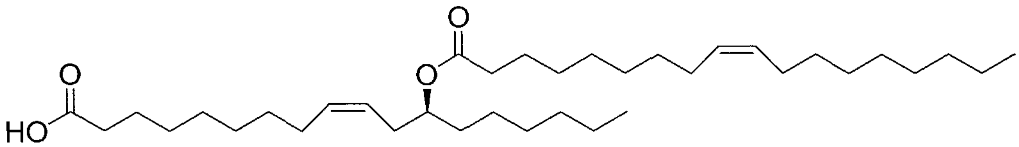

intermediate 55

<Synthesis of compound 3>

Using intermediate 55 and intermediate 1-1 and in the same manner as in the synthesis of compound 1 described in Example 1-1, compound 3 was synthesized.

[Chem. 104]

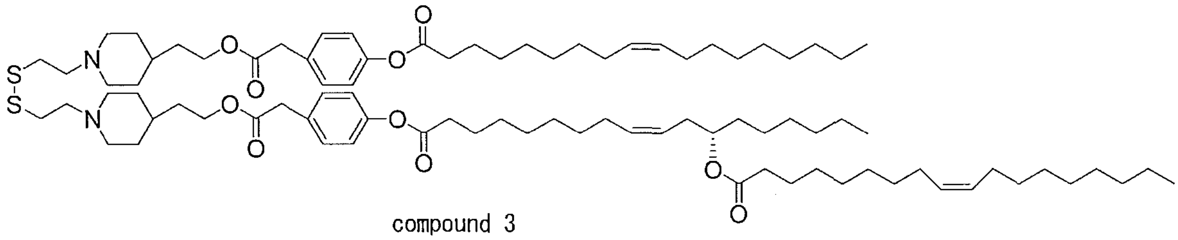

compound 3

<$^{1}$H-NMR (600 MHz, $CDCl_3$) of compound 3>

δ: 0.86-0.89 (m, 9H), 1.22-1.42 (m, 62H), 1.54-1.76 (m, 16H), 1.91-1.98 (m, 4H), 2.00-2.06 (m, 10H), 2.23-2.33 (m, 4H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 1H), 5.31-5.39 (m, 5H), 5.44-5.49 (m, 1H), 7.02-7.04 (m, 4H), 7.26-7.30 (m, 4H)

[Example 4] Synthesis of compound 4

Using intermediate 55 and intermediate 1 and in the same manner as in the synthesis of compound 2 described in Example 2-1, compound 4 was synthesized.

[Chem. 105]

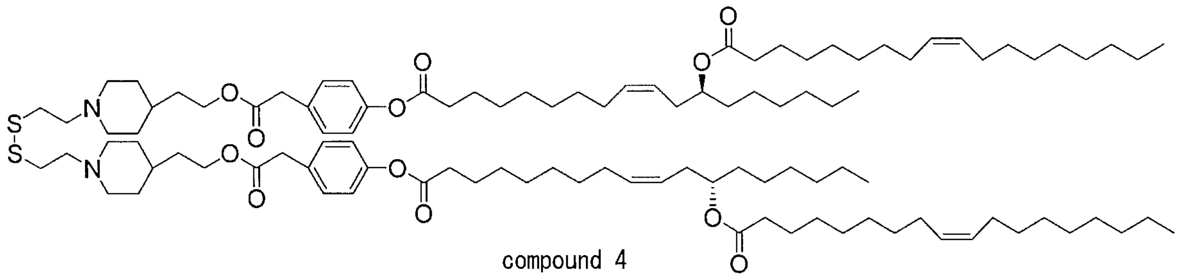

compound 4

<$^{1}$H-NMR (600 MHz, $CDCl_3$) of compound 4>

δ: 0.86-0.89 (m, 12H), 1.22-1.42 (m, 78H), 1.54-1.76 (m, 20H), 1.91-1.98 (m, 4H), 2.00-2.06 (m, 12H), 2.23-2.34 (m, 8H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 2H), 5.31-5.39 (m, 6H), 5.44-5.49 (m, 2H), 7.02-7.04 (m, 4H), 7.26-7.30 (m, 4H)

[Example 5] Synthesis of compound 5

<Synthesis of intermediate 15-1>

Intermediate 11-2 (6.90 g, 20.9 mmol) obtained by the synthesis described in Example 15, octanoic acid (6.64 mg, 46.0 mmol), and DMAP (533 mg, 4.36 mmol) were dissolved in chloroform (68.9 g) at room temperature. To the obtained mixture was added EDC hydrochloride (12.0 g, 62.7 mmol) and the mixture was reacted at room temperature for 1 hr. The subsequent steps were performed in the same manner as in the synthesis of intermediate 11-3 described in Example 15 to obtain 11.0 g of intermediate 15-1.

[Chem. 106]

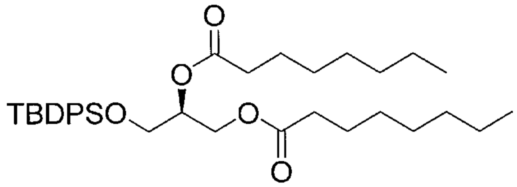

intermediate 15-1

<Synthesis of intermediate 15-2>

Intermediate 15-1 (11.0 g, 19.0 mmol), and acetic acid (4.56 g, 76.0 mmol) were dissolved in THF (33.0 g) at room temperature. To the obtained mixture was added 1 M TBAF solution (19.9 g, 76.0 mmol) in THF and the mixture was reacted at room temperature for 18 hr. Thereafter, to the reaction solution was added ethyl acetate (110 g). The subsequent steps were performed in the same manner as in the synthesis of intermediate 11-4 described in Example 15 to obtain 5.21 g of intermediate 15-2.

[Chem. 107]

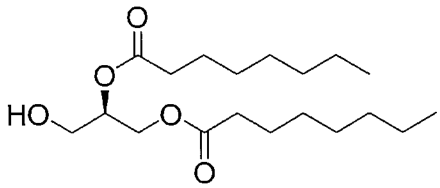

intermediate 15-2

<Synthesis of intermediate 15>

Intermediate 15-2 (5.00 g, 14.5 mmol), glutaric anhydride (2.00 g, 17.5 mmol), triethylamine (5.91 g, 58.4 mmol), and DMAP (0.178 g, 1.46 mmol) were dissolved in chloroform (50.0 g) at room temperature, and the mixture was reacted at room temperature for 1 hr. The subsequent steps were performed in the same manner as in the synthesis of intermediate 11 described in Example 15 to obtain 5.36 g of intermediate 15.

[Chem. 108]

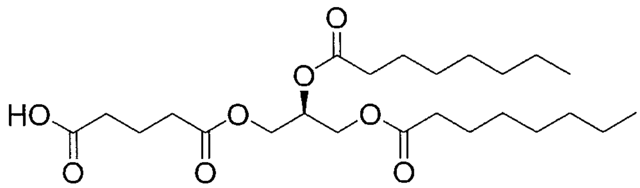

intermediate 15

<Synthesis of compound 5>

Using intermediate 15 and intermediate 1-1 and in the same manner as in the synthesis of compound 1 described in Example 1-1, compound 5 was synthesized.

[Chem. 109]

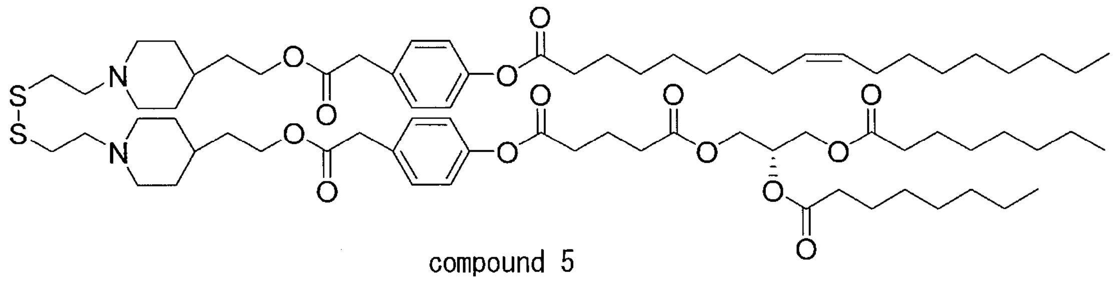

compound 5

<¹H-NMR (600 MHz, $CDCl_3$) of compound 5>

δ: 0.86-0.89 (t, 9H), 1.22-1.42 (m, 42H), 1.54-1.76 (m, 14H), 1.91-1.98 (m, 4H), 1.99-2.10 (m, 6H), 2.27-2.34 (m, 4H), 2.45-2.50 (t, 2H), 2.52-2.56 (t, 2H), 2.61-2.67 (m, 6H), 2.78-2.92 (m, 8H), 3.59 (s, 4H), 4.10-4.19 (m, 6H), 4.27-4.35 (m, 2H), 5.25-5.30 (m, 1H), 5.30-5.38 (m, 2H), 7.02-7.04 (m, 4H), 7.26-7.29 (m, 4H)

[Example 6] Synthesis of compound 6

<Synthesis of intermediate 59-1>

Intermediate 11-2 (6.91 g, 20.9 mmol), oleic acid (12.4 g, 43.9 mmol), and DMAP (1.53 g, 4.18 mmol) were dissolved in chloroform (6.91 g) at room temperature. To the obtained mixture was added EDC hydrochloride (12.0 g, 62.7 mmol) and the mixture was reacted at room temperature for 1 hr. The subsequent steps were performed in the same manner as in the synthesis of intermediate 11-3 described in Example 15 to obtain 17.1 g of intermediate 59-1.

[Chem. 110]

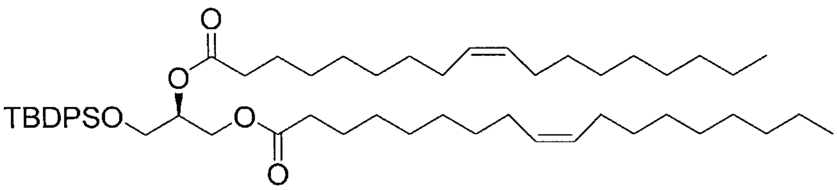

intermediate 59-1

<Synthesis of intermediate 59-2>

Intermediate 59-1 (17.1 g, 19.9 mmol), and acetic acid (4.73 g, 78.8 mmol) were dissolved in THF (51.3 g) at room temperature. To the obtained mixture was added 1 M TBAF solution (72.5 g, 78.8 mmol) in THF and the mixture was reacted at room temperature for 18 hr. Thereafter, to the reaction solution was added ethyl acetate (171 g). The subsequent steps were performed in the same manner as in the synthesis of intermediate 11-4 described in Example 15 to obtain 7.91 g of intermediate 59-2.

[Chem. 111]

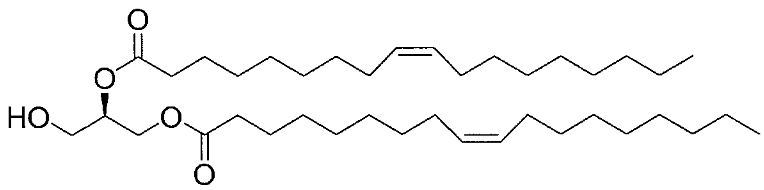

intermediate 59-2

<Synthesis of intermediate 59>

Intermediate 59-2 (7.89 g, 12.7 mmol), glutaric anhydride (2.90 g, 25.4 mmol), triethylamine (3.86 g, 38.1 mmol), and DMAP (310 mg, 2.54 mmol) were dissolved in chloroform (78.9 g) at room temperature, and the mixture was reacted at

room temperature for 1 hr. The subsequent steps were performed in the same manner as in the synthesis of intermediate 11 described in Example 15 to obtain 7.02 g of intermediate 59.

[Chem. 112]

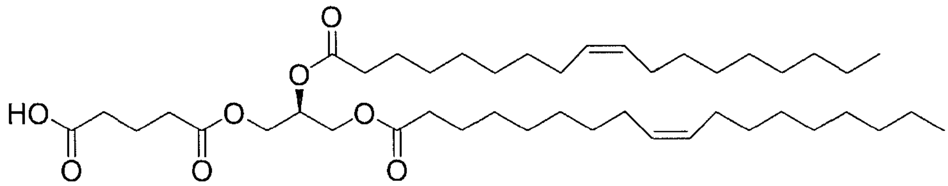

intermediate 59

<Synthesis of compound 6>

Using intermediate 59 and intermediate 1-1 and in the same manner as in the synthesis of compound 1 described in Example 1-1, compound 6 was synthesized.

[Chem. 113]

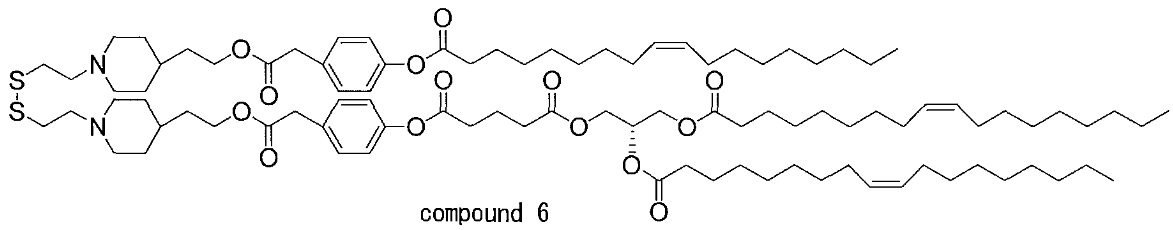

compound 6

<$^1$H-NMR (600 MHz, $CDCl_3$) of compound 6>

δ: 0.86-0.89 (t, 9H), 1.22-1.42 (m, 66H), 1.54-1.76 (m, 14H), 1.91-1.98 (m, 4H), 1.99-2.10 (m, 14H), 2.27-2.34 (m, 4H), 2.45-2.50 (t, 2H), 2.52-2.56 (t, 2H), 2.61-2.67 (m, 6H), 2.78-2.92 (m, 8H), 3.59 (s, 4H), 4.10-4.19 (m, 6H), 4.27-4.35 (m, 2H), 5.25-5.30 (m, 1H), 5.30-5.38 (m, 6H), 7.02-7.04 (m, 4H), 7.26-7.29 (m, 4H)

[Example 7] Synthesis of compound 7

Using intermediate 15 and intermediate 1 and in the same manner as in the synthesis of compound 2 described in Example 2-1, compound 7 was synthesized.

[Chem. 114]

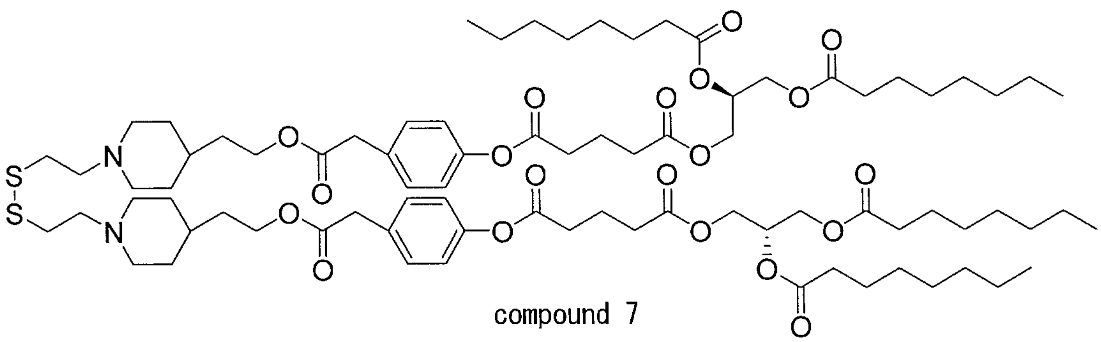

compound 7

<$^1$H-NMR (600 MHz, $CDCl_3$) of compound 7>

δ: 0.86-0.89 (m, 12H), 1.22-1.42 (m, 38H), 1.54-1.76 (m, 16H), 1.91-1.98 (m, 4H), 1.99-2.10 (m, 4H), 2.27-2.35 (m, 8H), 2.46-2.51 (t, 4H), 2.61-2.67 (m, 8H), 2.78-2.95 (m, 8H), 3.59 (s, 4H), 4.09-4.21 (m, 8H), 4.27-4.35 (m, 4H), 5.25-5.30 (m, 2H), 7.02-7.04 (m, 4H), 7.26-7.29 (m, 4H)

[Example 8] Synthesis of compound 8

Using intermediate 59 and intermediate 1 and in the same manner as in the synthesis of compound 2 described in Example 2-1, compound 8 was synthesized.

[Chem. 115]

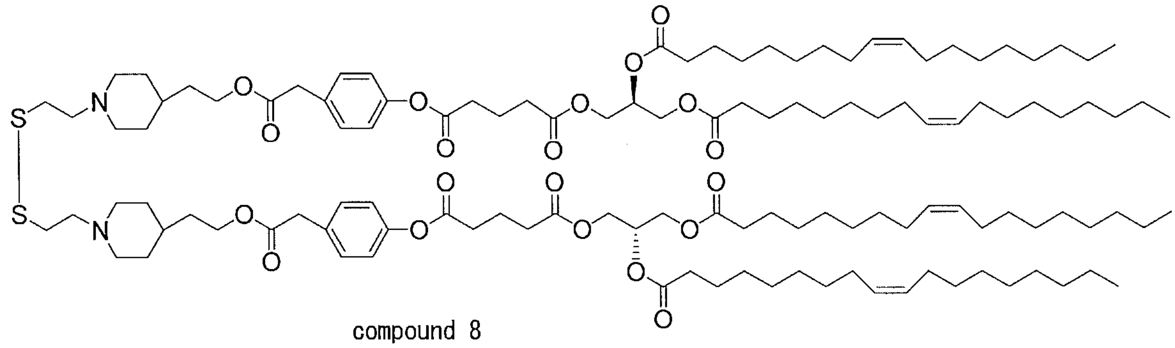

compound 8

<$^1$H-NMR (600 MHz, $CDCl_3$) of compound 8>

δ: 0.86-0.89 (t, 12H), 1.22-1.42 (m, 86H), 1.54-1.76 (m, 16H), 1.91-1.98 (m, 4H), 1.99-2.10 (m, 18H), 2.27-2.34 (m, 10H), 2.44-2.56 (m, 4H), 2.61-2.67 (m, 8H), 2.78-2.92 (m, 8H), 3.59 (s, 4H), 4.10-4.19 (m, 8H), 4.27-4.35 (m, 4H), 5.25-5.30 (m, 2H), 5.30-5.38 (m, 8H), 7.02-7.04 (m, 4H), 7.26-7.29 (m, 4H)

[Example 9] Synthesis of compound 9

<Synthesis of intermediate 5-1>

Methyl ricinoleate (5.00 g, 16.0 mmol), octanoic acid (2.54 g, 17.6 mmol), and DMAP (391 mg, 3.20 mmol) were dissolved in chloroform (50.0 g) at room temperature. To the obtained mixture was added EDC hydrochloride (4.60 g, 24.0 mmol) and the mixture was reacted at room temperature for 1 hr. The subsequent steps were performed in the same manner as in the synthesis of intermediate 2-1 described in Example 2-1 to obtain 6.78 g of intermediate 5-1.

[Chem. 116]

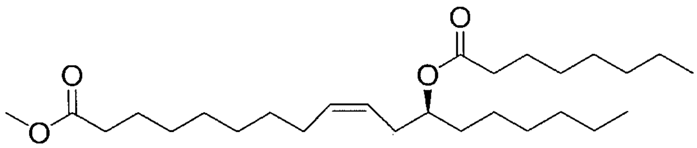

intermediate 5-1

<Synthesis of intermediate 5>

Intermediate 5-1 (6.40 g, 14.6 mmol) was dissolved in tert-butanol to 0.3 M at room temperature. To the obtained mixture was added 2 M NaOH aqueous solution (8.17 g) and the mixture was reacted at room temperature for 8 hr. The subsequent steps were performed in the same manner as in the synthesis of intermediate 2 described in Example 2-1 to obtain 5.93 g of intermediate 5.

[Chem. 117]

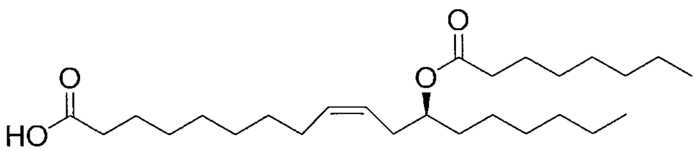

intermediate 5

<Synthesis of compound 9>

Using intermediate 5 and intermediate 1-1 and in the same manner as in the synthesis of compound 1 described in Example 1-1, compound 9 was synthesized.

[Chem. 118]

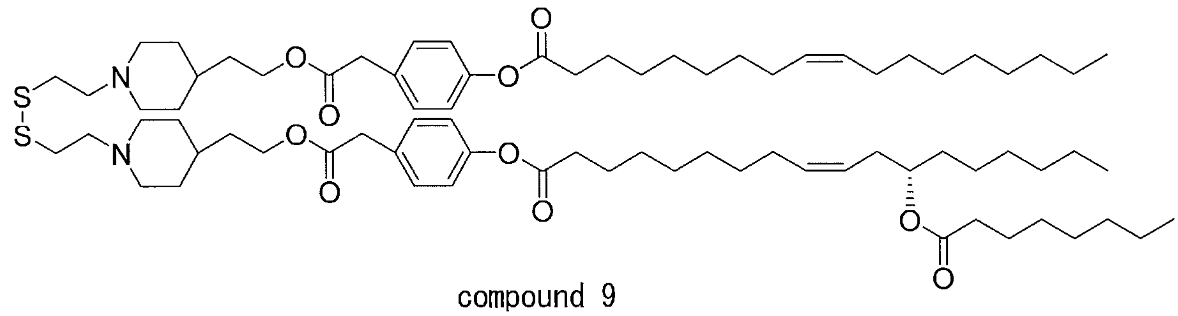

compound 9

<$^1$H-NMR (600 MHz, $CDCl_3$) of compound 9>

δ: 0.86-0.90 (m, 9H), 1.22-1.42 (m, 50H), 1.54-1.76 (m, 16H), 1.91-1.98 (m, 4H), 2.00-2.06 (m, 6H), 2.23-2.33 (m, 4H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 1H), 5.31-5.40 (m, 3H), 5.44-5.49 (m, 1H), 7.02-7.04 (m, 4H), 7.26-7.30 (m, 4H)

[Example 10] Synthesis of compound 10

<Synthesis of intermediate 4-1>

Methyl ricinoleate (5.00 g, 16.0 mmol), hexanoic acid (2.05 g, 17.6 mmol), and DMAP (391 mg, 3.20 mmol) were dissolved in chloroform (50.2 g) at room temperature. To the obtained mixture was added EDC hydrochloride (4.60 g, 24.0 mmol) and the mixture was reacted at room temperature for 1 hr. The subsequent steps were performed in the same manner as in the synthesis of intermediate 2-1 described in Example 2-1 to obtain 6.04 g of intermediate 4-1.

[Chem. 119]

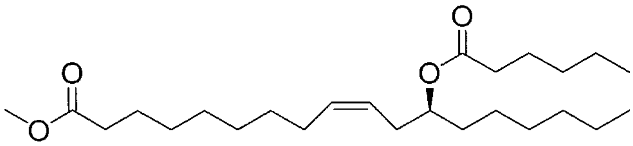

intermediate 4-1

<Synthesis of intermediate 4>

Intermediate 4-1 (5.95 g, 14.5 mmol) was dissolved in tert-butanol to 0.3 M at room temperature. To the obtained mixture was added 2 M NaOH aqueous solution (8.14 g) and the mixture was reacted at room temperature for 8 hr. The subsequent steps were performed in the same manner as in the synthesis of intermediate 2 described in Example 2-1 to obtain 5.20 g of intermediate 4.

[Chem. 120]

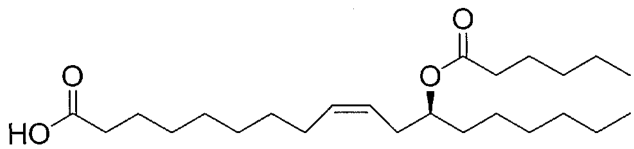

intermediate 4

<Synthesis of compound 10>

Using intermediate 4 and intermediate 1-1 and in the same manner as in the synthesis of compound 1 described in Example 1-1, compound 10 was synthesized.

[Chem. 121]

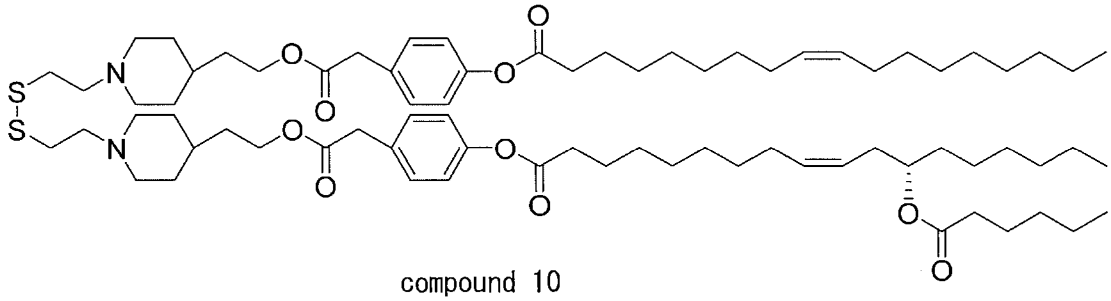

compound 10

<$^1$H-NMR (600 MHz, $CDCl_3$) of compound 10>

δ: 0.86-0.90 (m, 9H), 1.22-1.42 (m, 46H), 1.54-1.76 (m, 16H), 1.91-1.98 (m, 4H), 2.00-2.06 (m, 6H), 2.23-2.33 (m, 4H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 1H), 5.31-5.40 (m, 3H), 5.44-5.49 (m, 1H), 7.02-7.04 (m, 4H), 7.26-7.30 (m, 4H)

[Example 11] Synthesis of compound 11

<Synthesis of intermediate 3-1>

Methyl ricinoleate (5.00 g, 16.0 mmol), butanoic acid (1.55 g, 17.6 mmol), and DMAP (391 mg, 3.2 mmol) were dissolved in chloroform (50.1 g) at room temperature. To the obtained mixture was added EDC hydrochloride (4.60 g, 24.0 mmol) and the mixture was reacted at room temperature for 1 hr. The subsequent steps were performed in the same manner as in the synthesis of intermediate 2-1 described in Example 2-1 to obtain 5.53 g of intermediate 3-1.

[Chem. 122]

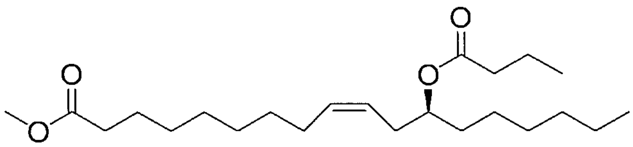

intermediate 3-1

<Synthesis of intermediate 3>

Intermediate 3-1 (5.53 g, 14.4 mmol) was dissolved in tert-butanol to 0.3 M at room temperature. To the obtained mixture was added 2 M NaOH aqueous solution (8.06 g) and the mixture was reacted at room temperature for 8 hr. The subsequent steps were performed in the same manner as in the synthesis of intermediate 2 described in Example 2-1 to obtain 2.76 g of intermediate 3.

[Chem. 123]

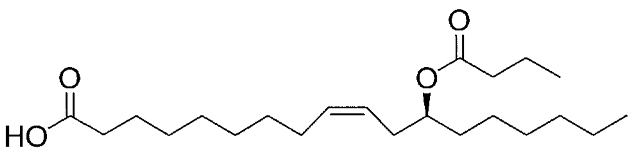

intermediate 3

<Synthesis of compound 11>

Using intermediate 3 and intermediate 1-1 and in the same manner as in the synthesis of compound 1 described in Example 1-1, compound 11 was synthesized.

[Chem. 124]

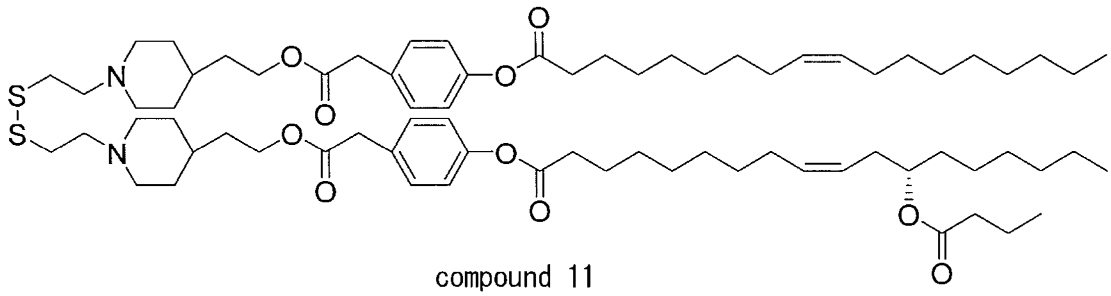

compound 11

<$^1$H-NMR (600 MHz, $CDCl_3$) of compound 11>

δ: 0.86-0.89 (m, 6H), 0.93-0.96 (m, 3H), 1.22-1.42 (m, 42H), 1.54-1.76 (m, 16H), 1.91-1.98 (m, 4H), 2.00-2.06 (m, 6H), 2.23-2.33 (m, 4H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 1H), 5.31-5.40 (m, 3H), 5.44-5.49 (m, 1H), 7.02-7.04 (m, 4H), 7.26-7.30 (m, 4H)

[Example 12] Synthesis of compound 12

<Synthesis of intermediate 6-1>

Methyl ricinoleate (5.00 g, 16.0 mmol), 5-hexenoic acid (2.01 g, 17.6 mmol), and DMAP (393 mg, 3.22 mmol) were dissolved in chloroform (50.0 g) at room temperature. To the obtained mixture was added EDC hydrochloride (4.60 g, 24.0 mmol) and the mixture was reacted at room temperature for 1 hr. The subsequent steps were performed in the same manner as in the synthesis of intermediate 2-1 described in Example 2-1 to obtain 6.29 g of intermediate 6-1.

[Chem. 125]

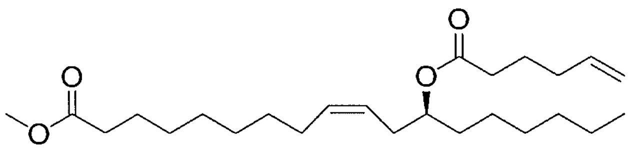

intermediate 6-1

<Synthesis of intermediate 6>

Intermediate 6-1 (6.28 g, 15.4 mmol) was dissolved in tert-butanol to 0.3 M at room temperature. To the obtained mixture was added 2 M NaOH aqueous solution (8.62 g) and the mixture was reacted at room temperature for 8 hr. The subsequent steps were performed in the same manner as in the synthesis of intermediate 2 described in Example 2-1 to give 3.21 g of intermediate 6.

[Chem. 126]

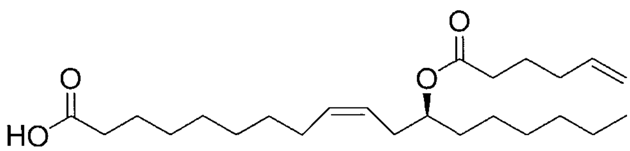

intermediate 6

<Synthesis of compound 12>

Using intermediate 6 and intermediate 1-1 and in the same manner as in the synthesis of compound 1 described

in Example 1-1, compound 12 was synthesized.

[Chem. 127]

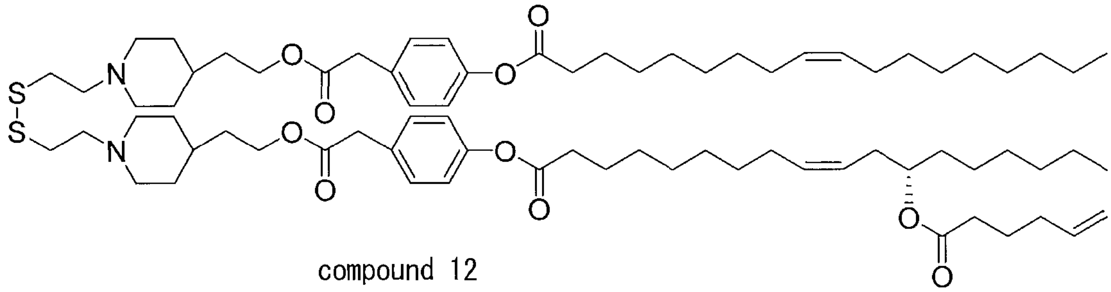

compound 12

<$^1$H-NMR (600 MHz, $CDCl_3$) of compound 12>

δ: 0.86-0.90 (m, 6H), 1.22-1.42 (m, 42H), 1.54-1.76 (m, 16H), 1.91-1.98 (m, 4H), 2.00-2.06 (m, 6H), 2.06-2.12 (m, 2H), 2.23-2.33 (m, 4H), 2.52-2.56 (m, 4H), 2.61-2.67 (m, 4H), 2.79-2.92 (m, 8H), 3.59 (s, 4H), 4.11-4.13 (t, 4H), 4.87-4.92 (m, 1H), 4.97-5.05 (m, 2H), 5.30-5.38 (m, 3H), 5.44-5.49 (m, 1H), 5.74-5.82 (m, 1H), 7.02-7.04 (m, 4H), 7.26-7.30 (m, 4H)

[Example 13] Synthesis of compound 13

<Synthesis of intermediate 13-1>

Intermediate 11-2 (6.90 g, 20.9 mmol) obtained by the synthesis described in Example 15, hexanoic acid (5.38 g, 46.0 mmol), and DMAP (0.511 g, 4.18 mmol) were dissolved in chloroform (69.5 g) at room temperature. To the obtained mixture was added EDC hydrochloride (12.0 g, 62.7 mmol) and the mixture was reacted at room temperature for 1 hr. The subsequent steps were performed in the same manner as in the synthesis of intermediate 11-3 described in Example 15 to obtain 9.405 g of intermediate 13-1.

[Chem. 128]

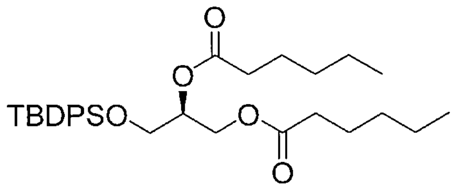

intermediate 13-1

<Synthesis of intermediate 13-2>

Intermediate 13-1 (9.41 g, 17.9 mmol), and acetic acid (4.29 g, 71.6 mmol) were dissolved in THF (28.2 g) at room temperature. To the obtained mixture was added 1 M TBAF solution (65.9 g, 71.6 mmol) in THF and the mixture was reacted at room temperature for 18 hr. Thereafter, to the reaction solution was added ethyl acetate (94.3 g). The subsequent steps were performed in the same manner as in the synthesis of intermediate 11-4 described in Example 15 to obtain 3.10 g of intermediate 13-2.

[Chem. 129]

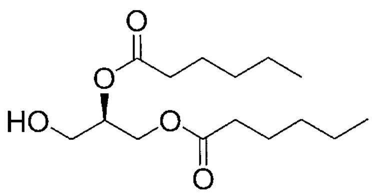

intermediate 13-2

<Synthesis of intermediate 13>

Intermediate 13-2 (3.10 g, 10.7 mmol), glutaric anhydride (2.45 g, 21.5 mmol), triethylamine (3.25 g, 32.1 mmol), and DMAP (0.261 g, 2.14 mmol) were dissolved in chloroform (51.6 g) at room temperature, and the mixture was reacted at room temperature for 1 hr. The subsequent steps were performed in the same manner as in the synthesis of intermediate 11 described in Example 15 to obtain 3.21 g of intermediate 13.

[Chem. 130]

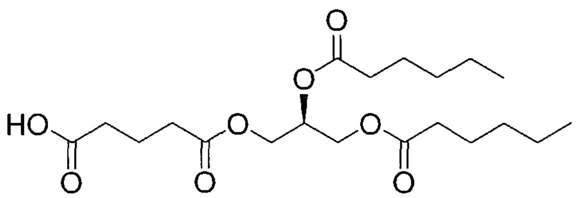

intermediate 13

<Synthesis of compound 13>

Using intermediate 13 and intermediate 1-1 and in the same manner as in the synthesis of compound 1 described in Example 1-1, compound 13 was synthesized.

[Chem. 131]

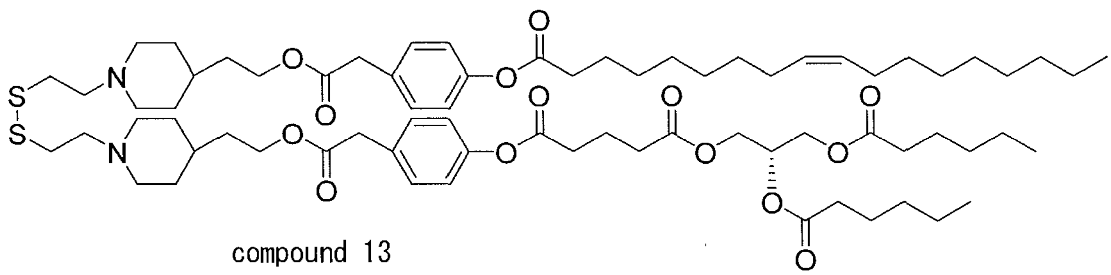

compound 13

<$^1$H-NMR (600 MHz, $CDCl_3$) of compound 13>

δ: 0.86-0.89 (t, 9H), 1.22-1.42 (m, 34H), 1.54-1.76 (m, 14H), 1.91-1.98 (m, 4H), 1.99-2.10 (m, 6H), 2.27-2.34 (m, 4H), 2.45-2.50 (t, 2H), 2.52-2.56 (t, 2H), 2.61-2.67 (m, 6H), 2.78-2.92 (m, 8H), 3.59 (s, 4H), 4.10-4.19 (m, 6H), 4.27-4.35 (m, 2H), 5.25-5.30 (m, 1H), 5.30-5.38 (m, 2H), 7.02-7.04 (m, 4H), 7.26-7.29 (m, 4H)

[Example 14] Synthesis of compound 14

<Synthesis of intermediate 14-1>

Intermediate 11-2 (6.90 g, 20.9 mmol) obtained by the synthesis described in Example 15, heptanoic acid (6.84 g, 46.0 mmol), and DMAP (0.511 g, 4.18 mmol) were dissolved in chloroform (69.5 g) at room temperature. To the obtained mixture was added EDC hydrochloride (12.0 g, 62.7 mmol) and the mixture was reacted at room temperature for 1 hr. The subsequent steps were performed in the same manner as in the synthesis of intermediate 11-3 described in Example 15 to obtain 9.03 g of intermediate 14-1.

[Chem. 132]

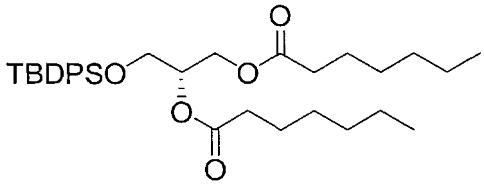
intermediate 14-1

<Synthesis of intermediate 14-2>

Intermediate 14-1 (9.41 g, 17.9 mmol), and acetic acid (3.92 g, 65.3 mmol) were dissolved in THF (27.2 g) at room temperature. To the obtained mixture was added 1 M TBAF solution (59.9 g, 65.2 mmol) in THF and the mixture was reacted at room temperature for 18 hr. Thereafter, to the reaction solution was added ethyl acetate (94.3 g). The subsequent steps were performed in the same manner as in the synthesis of intermediate 11-4 described in Example 15 to obtain 4.90 g of intermediate 14-2.

[Chem. 133]

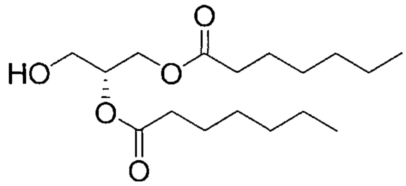
intermediate 14-2

<Synthesis of intermediate 14>

Intermediate 14-2 (4.90 g, 15.4 mmol), glutaric anhydride (3.73 g, 32.7 mmol), triethylamine (4.95 g, 48.9 mmol), and DMAP (0.398 g, 3.26 mmol) were dissolved in chloroform (51.6 g) at room temperature, and the mixture was reacted at room temperature for 1 hr. The subsequent steps were performed in the same manner as in the synthesis of intermediate 11 described in Example 15 to obtain 3.16 g of intermediate 14.

[Chem. 134]

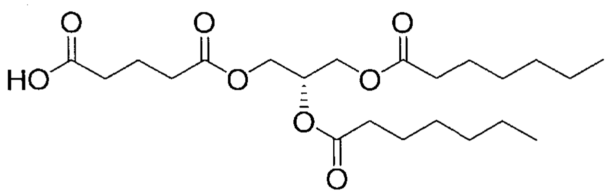
intermediate 14

<Synthesis of compound 14>

Using intermediate 14 and intermediate 1-1 and in the same manner as in the synthesis of compound 1 described in Example 1-1, compound 14 was synthesized.

[Chem. 135]

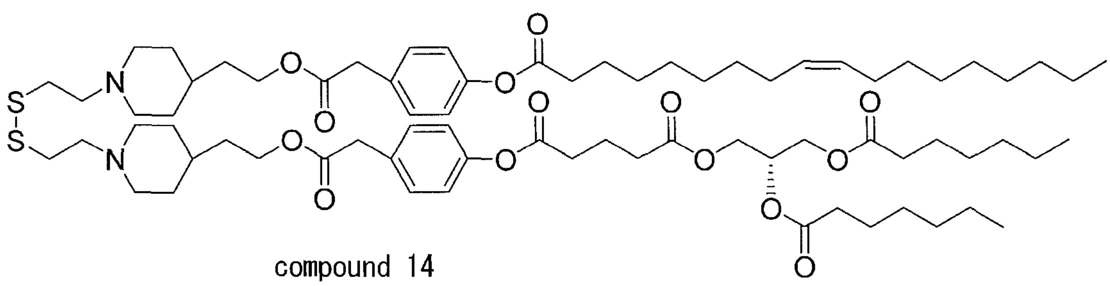
compound 14

<$^1$H-NMR (600 MHz, $CDCl_3$) of compound 14>

δ: 0.86-0.89 (t, 9H), 1.22-1.42 (m, 38H), 1.54-1.76 (m, 14H), 1.91-1.98 (m, 4H), 1.99-2.10 (m, 6H), 2.27-2.34 (m, 4H), 2.45-2.50 (t, 2H), 2.52-2.56 (t, 2H), 2.61-2.67 (m, 6H), 2.78-2.92 (m, 8H), 3.59 (s, 4H), 4.10-4.19 (m, 6H), 4.27-4.35 (m, 2H), 5.25-5.30 (m, 1H), 5.30-5.38 (m, 2H), 7.02-7.04 (m, 4H), 7.26-7.29 (m, 4H)

[Example 15] Synthesis of compound 15

<Synthesis of intermediate 11>

Intermediate 11 was synthesized by the following synthesis pathway.

[Chem. 136]

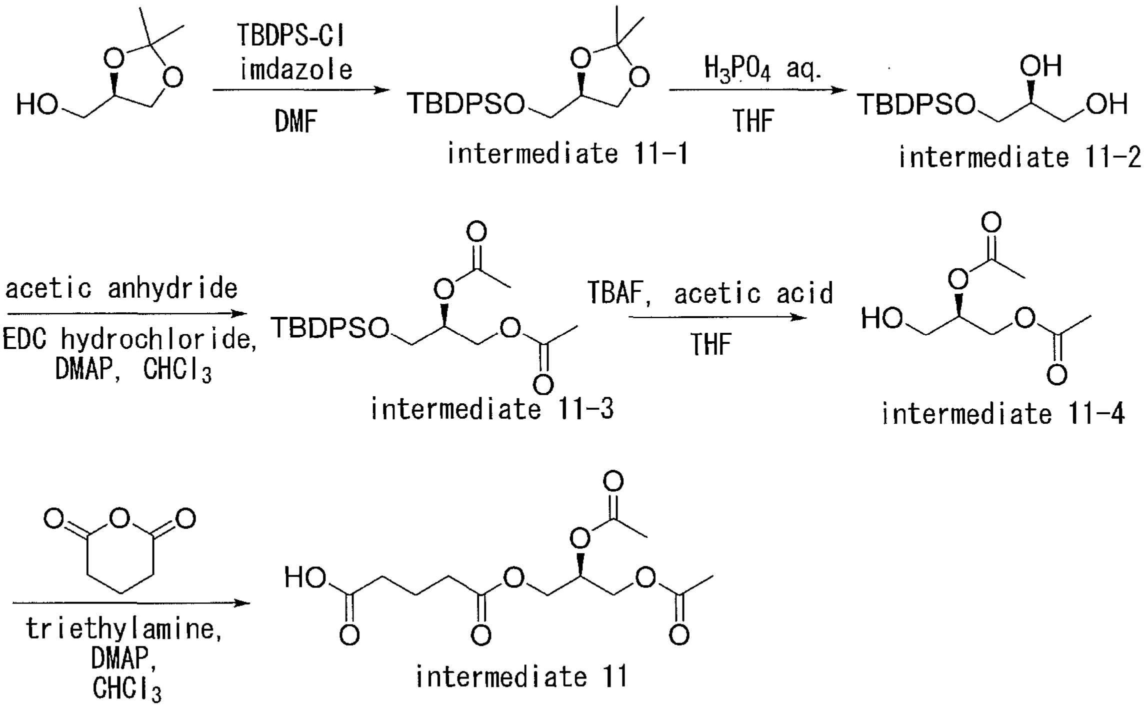


<Synthesis of intermediate 11-1>

(R)-(-)-2,2-dimethyl-1,3-dioxolane-4-methanol (25.0 g, 189 mmol), and imidazole (19.3 g, 284 mmol) were dissolve in dimethylformamide (251 g) at room temperature. To the obtained mixture was added TBDPS-Cl (57.3 g, 208 mmol) and the mixture was reacted at room temperature for 1 hr. Thereafter, to the reaction solution was added chloroform, and the mixture was washed with 5 wt% sodium hydrogen phosphate aqueous solution, and ion-exchanged water, and dehydrated by adding sodium sulfate. Sodium sulfate was removed by filtration, and the filtrate was concentrated by an evaporator to give 56.0 g of intermediate 11-1.

<Synthesis of intermediate 11-2>

Intermediate 11-1 (55.9 g, 151 mmol), 0.5 M phosphate buffer (pH 1.0) (503 g) were dissolved in THF (530 g) at room temperature, and the mixture was reacted at 50°C for 12 hr. 1.0 M NaOH aqueous solution was added to pH 7.0. To the obtained mixture was added chloroform, and the mixture was washed with 20 wt% saline, and dehydrated by adding sodium sulfate. Sodium sulfate was removed by filtration, and the filtrate was concentrated by an evaporator to give 44.9 g of intermediate 11-2.

<Synthesis of intermediate 11-3>

Intermediate 11-2 (6.89 g, 20.9 mmol), acetic anhydride (4.70 g, 46.0 mmol), and triethylamine (6.37 g, 62.7 mmol) were dissolved in chloroform (69.0 g) at room temperature. To the obtained mixture was added DMAP (0.511 g, 4.18

mmol) and the mixture was reacted at room temperature for 1 hr. Thereafter, the reaction solution was washed with 0.5 M phosphate buffer (pH 4.0), 7 wt% sodium bicarbonate water, and 20 wt% saline, and dehydrated by adding sodium sulfate. Sodium sulfate was removed by filtration, and the filtrate was concentrated by an evaporator to give 7.71 g of intermediate 11-3.

<Synthesis of intermediate 11-4>

Intermediate 11-3 (7.70 g, 18.6 mmol), and acetic acid (4.84 g, 74.4 mmol) were dissolved in THF (23.1 g) at room temperature. To the obtained mixture was added 1 M TBAF solution (68.4 g, 74.4 mmol) in THF and the mixture was reacted at room temperature for 18 hr. Thereafter, to the reaction solution was added ethyl acetate (77.0 g). Thereafter, the reaction solution was washed with 0.5 M phosphate buffer (pH 4.0), and 20 wt% saline, and dehydrated by adding sodium sulfate. Sodium sulfate was removed by filtration, and the filtrate was concentrated by an evaporator to give 2.95 g of intermediate 11-4.

<Synthesis of intermediate 11>

Intermediate 11-4 (2.94 g, 16.7 mmol), glutaric anhydride (3.81 g, 33.4 mmol), triethylamine (5.07 g, 50.1 mmol), and DMAP (0.408 g, 3.34 mmol) were dissolved in chloroform (29.4 g) at room temperature, and the mixture was reacted at room temperature for 1 hr. Thereafter, the reaction solution was washed with 0.5 M phosphate buffer (pH 4.0), 7 wt% sodium bicarbonate water, and 20 wt% saline, and dehydrated by adding sodium sulfate. Sodium sulfate was removed by filtration, and the filtrate was concentrated by an evaporator to give 1.81 g of intermediate 11.

<Synthesis of compound 15>

Using intermediate 11 and intermediate 1 and in the same manner as in the synthesis of compound 2 described in Example 2-1, compound 15 was synthesized.

[Chem. 137]

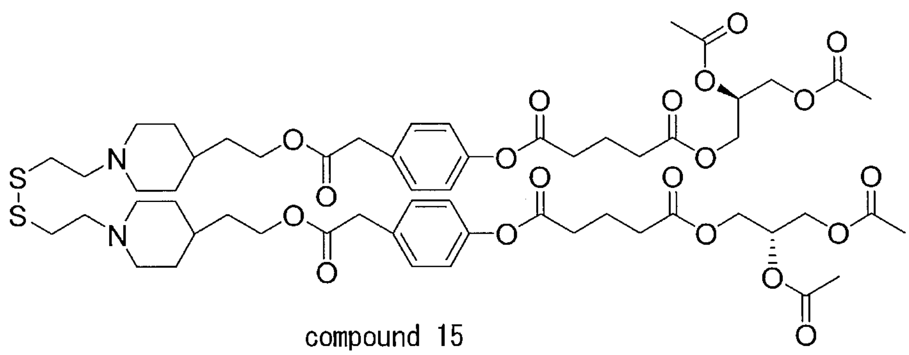

compound 15

<$^1$H-NMR (600 MHz, $CDCl_3$) of compound 15>

δ: 1.22-1.42 (m, 6H), 1.54-1.76 (m, 8H), 1.91-2.00 (m, 4H), 2.06-2.11 (m, 16H), 2.46-2.51 (m, 4H), 2.61-2.67 (m, 8H), 2.78-2.95 (m, 8H), 3.60 (s, 4H), 4.09-4.21 (m, 8H), 4.27-4.35 (m, 4H), 5.25-5.30 (m, 2H), 7.02-7.04 (m, 4H), 7.26-7.30 (m, 4H)

[Example 16] Synthesis of compound 16

Compound 16 was synthesized by the following synthesis pathway.

[Chem. 138]

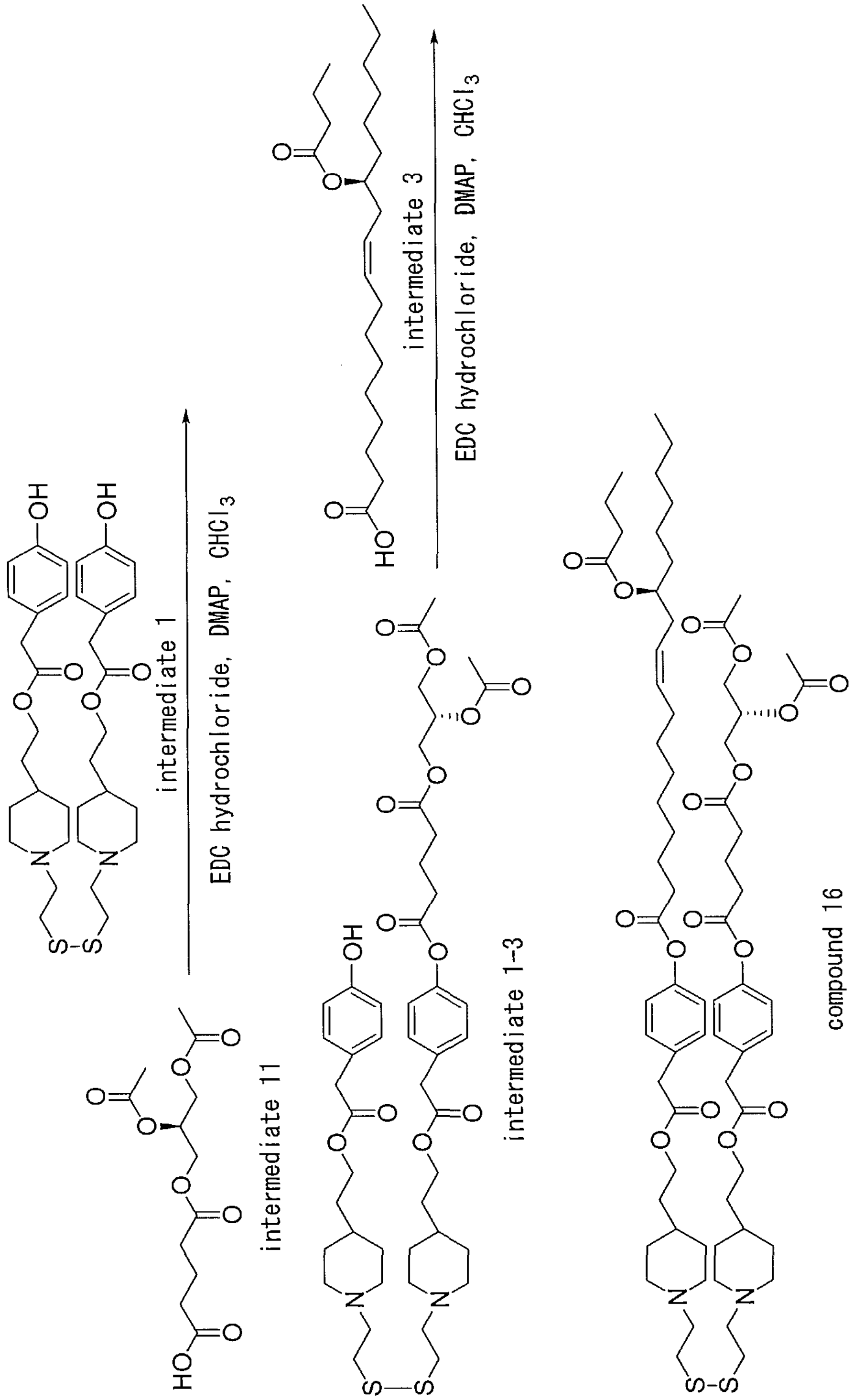


<Synthesis of intermediate 1-3>

Using intermediate 11 and intermediate 1 and in the same manner as in the synthesis of intermediate 1-1 described in Example 1-1, intermediate 1-3 was synthesized.

<Synthesis of compound 16>

Using intermediate 1-3 and intermediate 3 and in the same manner as in the synthesis of compound 1 described

in Example 1-1, compound 16 was synthesized.

<$^1$H-NMR (600 MHz, $CDCl_3$) of compound 16>

δ: 0.86-0.89 (t, 3H), 0.93-0.96 (t, 3H), 1.22-1.42 (m, 22H), 1.54-1.76 (m, 14H), 1.91-1.98 (m, 4H), 2.00-2.06 (m, 10H), 2.23-2.33 (m, 4H), 2.46-2.56 (m, 4H), 2.61-2.67 (m, 6H), 2.79-2.92 (m, 8H), 3.59 (s, 4H), 4.09-4.20 (m, 6H), 4.27-4.36 (m, 2H), 4.86-4.92 (m, 1H), 5.25-5.36 (m, 2H), 5.44-5.49 (m, 1H), 7.02-7.04 (m, 4H), 7.26-7.30 (m, 4H)

[Example 17] Synthesis of compound 17

Using intermediate 1-3 and intermediate 4 and in the same manner as in the synthesis of compound 1 described in Example 1-1, compound 17 was synthesized.

[Chem. 139]

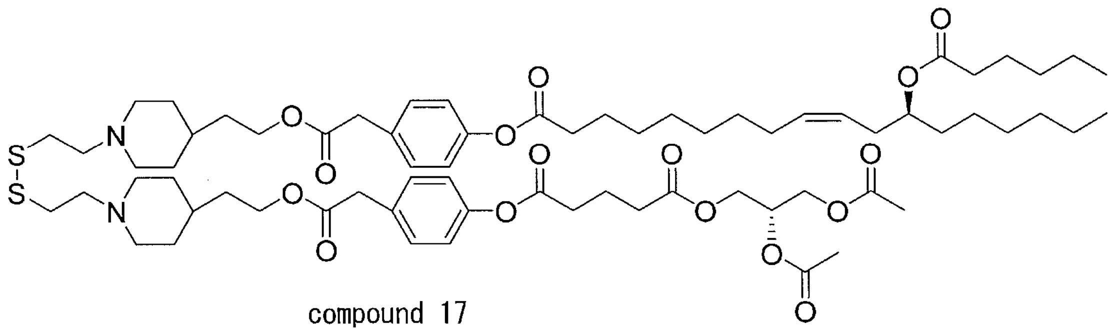

compound 17

<$^1$H-NMR (600 MHz, $CDCl_3$) of compound 17>

δ: 0.86-0.91 (m, 6H), 1.22-1.42 (m, 26H), 1.54-1.76 (m, 14H), 1.91-1.98 (m, 4H), 2.00-2.06 (m, 10H), 2.23-2.33 (m, 4H), 2.46-2.56 (m, 4H), 2.61-2.67 (m, 6H), 2.79-2.92 (m, 8H), 3.59 (s, 4H), 4.09-4.20 (m, 6H), 4.27-4.36 (m, 2H), 4.86-4.92 (m, 1H), 5.25-5.36 (m, 2H), 5.44-5.49 (m, 1H), 7.02-7.04 (m, 4H), 7.26-7.30 (m, 4H)

[Example 18] Synthesis of compound 18

Using intermediate 1-3 and intermediate 5 and in the same manner as in the synthesis of compound 1 described in Example 1-1, compound 18 was synthesized.

[Chem. 140]

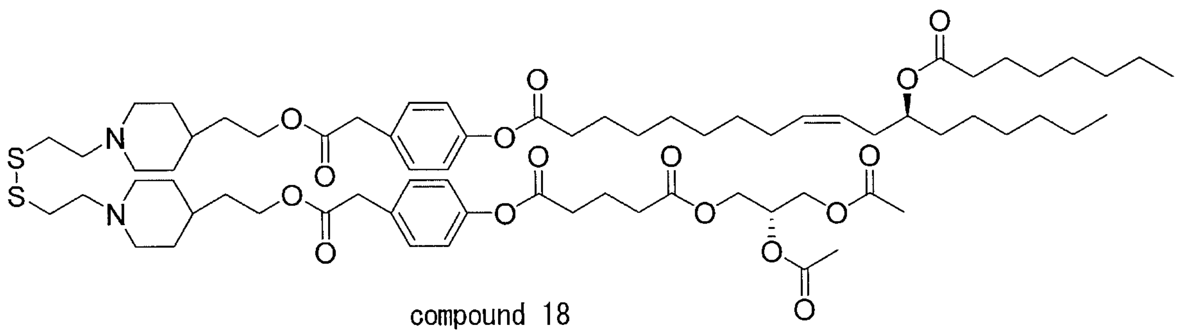

compound 18

<$^1$H-NMR (600 MHz, $CDCl_3$) of compound 18>

δ: 0.86-0.91 (m, 6H), 1.22-1.42 (m, 30H), 1.54-1.76 (m, 14H), 1.91-1.98 (m, 4H), 2.00-2.06 (m, 10H), 2.23-2.33 (m, 4H), 2.46-2.56 (m, 4H), 2.61-2.67 (m, 6H), 2.79-2.92 (m, 8H), 3.59 (s, 4H), 4.09-4.20 (m, 6H), 4.27-4.36 (m, 2H), 4.86-4.92 (m, 1H), 5.25-5.36 (m, 2H), 5.44-5.49 (m, 1H), 7.02-7.04 (m, 4H), 7.26-7.30 (m, 4H)

[Example 19] Synthesis of compound 19

Using intermediate 1-3 and intermediate 6 and in the same manner as in the synthesis of compound 1 described in Example 1-1, compound 19 was synthesized.

[Chem. 141]

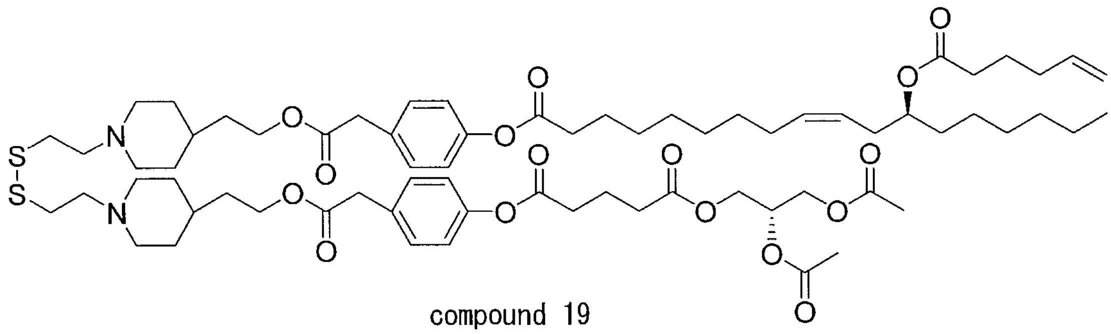

compound 19

<$^1$H-NMR (600 MHz, $CDCl_3$) of compound 19>

δ: 0.86-0.89 (t, 3H), 1.22-1.42 (m, 22H), 1.54-1.76 (m, 14H), 1.91-1.98 (m, 4H), 2.00-2.06 (m, 12H), 2.23-2.33 (m, 4H), 2.46-2.56 (m, 4H), 2.61-2.67 (m, 6H), 2.79-2.92 (m, 8H), 3.59 (s, 4H), 4.09-4.20 (m, 6H), 4.27-4.36 (m, 2H), 4.86-4.92 (m, 1H), 4.97-5.05 (m, 2H), 5.25-5.36 (m, 2H), 5.44-5.49 (m, 1H), 5.74-5.82 (m, 1H), 7.02-7.04 (m, 4H), 7.26-7.30 (m, 4H)

[Example 20] Synthesis of compound 20

<Synthesis of intermediate 12-1>

Intermediate 11-2 (6.91 g, 20.9 mmol), butanoic acid (4.05 g, 46.0 mmol), and DMAP (0.510 g, 4.18 mmol) were dissolved in chloroform (69.4 g) at room temperature. To the obtained mixture was added EDC hydrochloride (12.0 g, 62.7 mmol) and the mixture was reacted at room temperature for 1 hr. The subsequent steps were performed in the same manner as in the synthesis of intermediate 11-3 described in Example 15 to obtain 8.42 g of intermediate 12-1.

[Chem. 142]

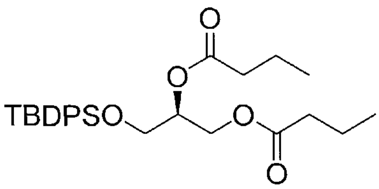

intermediate 12-1

<Synthesis of intermediate 12-2>

Intermediate 12-1 (8.42 g, 17.9 mmol), and acetic acid (4.30 g, 71.6 mmol) were dissolved in THF (25.3 g) at room temperature. To the obtained mixture was added 1 M TBAF solution (65.8 g, 71.6 mmol) in THF and the mixture was reacted at room temperature for 18 hr. Thereafter, to the reaction solution was added ethyl acetate (84.2 g). The subsequent steps were performed in the same manner as in the synthesis of intermediate 11-4 described in Example 15 to obtain 3.53 g of intermediate 12-2.

[Chem. 143]

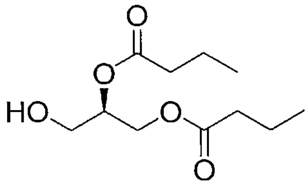

intermediate 12-2

<Synthesis of intermediate 12>

Intermediate 12-2 (3.51 g, 15.1 mmol), glutaric anhydride (3.45 g, 30.2 mmol), triethylamine (4.58 g, 45.3 mmol), and DMAP (0.369 g, 3.02 mmol) were dissolved in chloroform (35.0 g) at room temperature, and the mixture was reacted at room temperature for 1 hr. The subsequent steps were performed in the same manner as in the synthesis of intermediate 11 described in Example 15 to obtain 3.47 g of intermediate 12.

[Chem. 144]

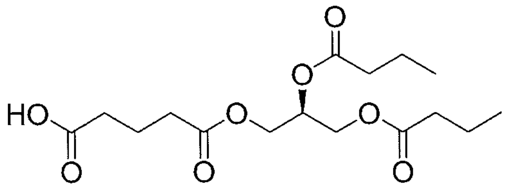

intermediate 12

<Synthesis of compound 20>

Using intermediate 12 and intermediate 1 and in the same manner as in the synthesis of compound 2 described in Example 2-1, compound 20 was synthesized.

[Chem. 145]

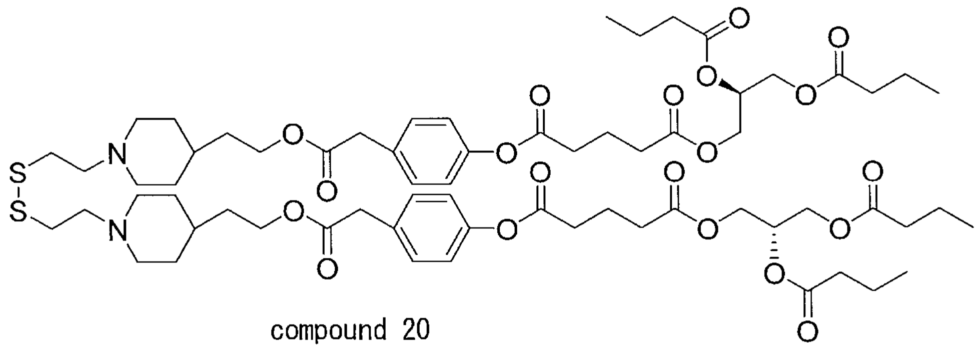

compound 20

<$^1$H-NMR (600 MHz, $CDCl_3$) of compound 20>

δ: 0.86-0.89 (m, 12H), 1.22-1.42 (m, 6H), 1.54-1.76 (m, 16H), 1.91-1.98 (m, 4H), 2.03-2.09 (m, 4H), 2.27-2.35 (m, 8H), 2.46-2.51 (t, 4H), 2.61-2.67 (m, 8H), 2.78-2.95 (m, 8H), 3.60 (s, 4H), 4.10-4.19 (m, 8H), 4.27-4.35 (m, 4H), 5.27-5.32 (m, 2H), 7.02-7.05 (m, 4H), 7.26-7.29 (m, 4H)

[Example 21] Synthesis of compound 21

Compound 21 was synthesized by the following synthesis pathway.

[Chem. 146]

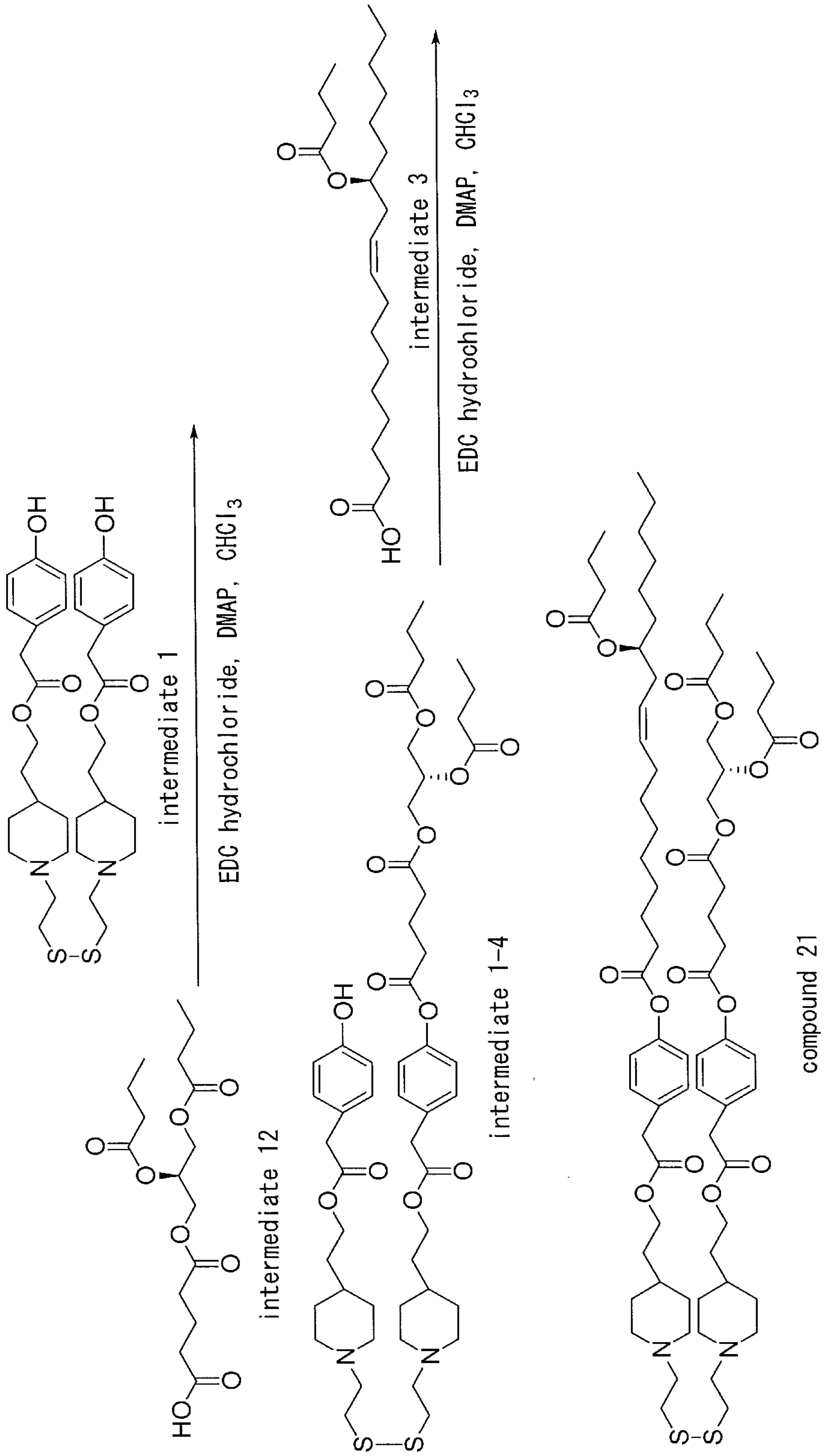


<Synthesis of intermediate 1-4>

Using intermediate 12 and intermediate 1 and in the same manner as in the synthesis of intermediate 1-1, intermediate 1-4 was synthesized.

<Synthesis of compound 21>

Using intermediate 1-4 and intermediate 3 and in the same manner as in the synthesis of compound 1 described in Example 1-1, compound 21 was synthesized.

<$^1$H-NMR (600 MHz, $CDCl_3$) of compound 21>

δ: 0.87 (t, 3H), 0.94 (tt, 9H), 1.21-1.41 (m, 24H), 1.52-1.76 (m, 16H), 1.93-2.08 (m, 8H), 2.24-2.32 (m, 8H), 2.46-2.65 (m, 10H), 2.80-2.89 (m, 8H), 3.59 (s, 4H), 4.10-4.18 (m, 6H), 4.29-4.34 (m, 2H), 4.88 (q, 1H), 5.27-5.35 (m, 2H), 5.44-5.48 (m, 1H), 7.01-7.04 (m, 4H), 7.28 (dd, 4H)

[Example 22] Synthesis of compound 22

Using intermediate 1-4 and intermediate 4 and in the same manner as in the synthesis of compound 1 described in Example 1-1, compound 22 was synthesized.

[Chem. 147]

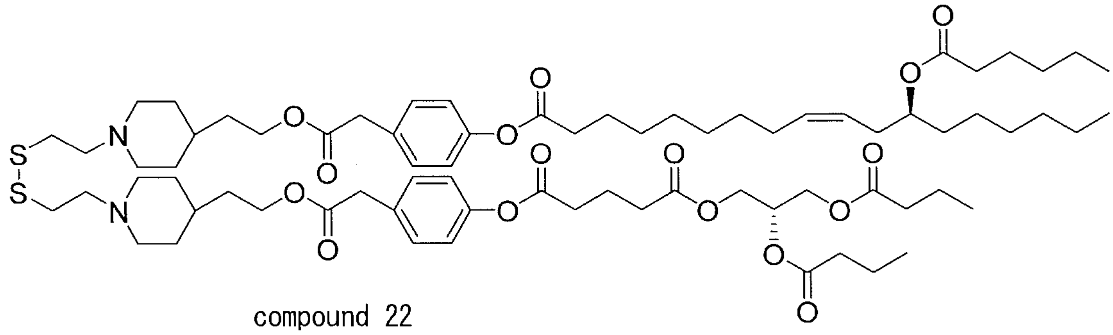

compound 22

<$^1$H-NMR (600 MHz, $CDCl_3$) of compound 22>

δ: 0.86-0.95 (m, 12H), 1.23-1.41 (m, 27H), 1.52-1.76 (m, 17H), 1.93-2.08 (m, 8H), 2.25-2.32 (m, 8H), 2.46-2.65 (m, 10H), 2.80-2.89 (m, 8H), 3.59 (s, 4H), 4.11-4.18 (m, 6H), 4.29-4.35 (m, 2H), 4.88 (t, 1H), 5.27-5.34 (m, 2H), 5.44-5.48 (m, 1H), 7.01-7.04 (m, 4H), 7.28 (dd, 4H)

[Example 23] Synthesis of compound 23

Using intermediate 1-4 and intermediate 5 and in the same manner as in the synthesis of compound 1 described in Example 1-1, compound 23 was synthesized.

[Chem. 148]

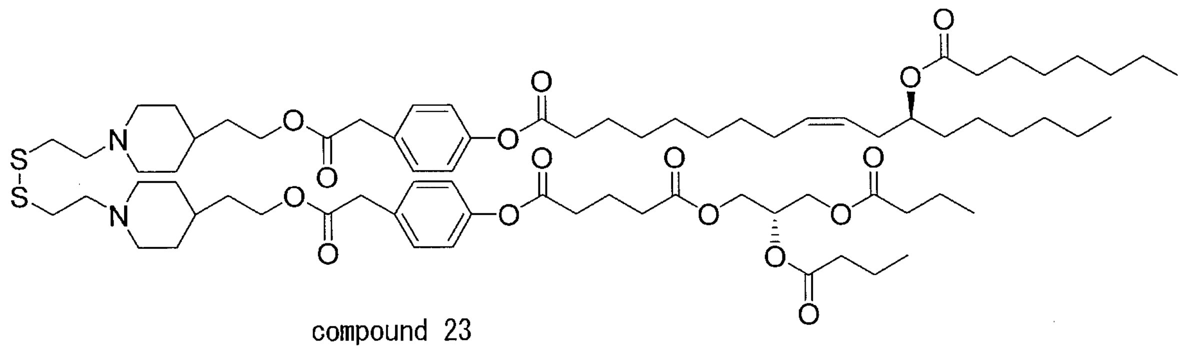

compound 23

<$^1$H-NMR (600 MHz, $CDCl_3$) of compound 23>

δ: 0.86-0.95 (m, 12H), 1.23-1.41 (m, 32H), 1.52-1.76 (m, 16H), 1.93-2.08 (m, 8H), 2.25-2.32 (m, 8H), 2.46-2.55

(m, 4H), 2.63 (td, 6H), 2.80-2.89 (m, 8H), 3.59 (s, 4H), 4.11-4.18 (m, 6H), 4.29-4.35 (m, 2H), 4.88 (t, 1H), 5.27-5.34 (m, 2H), 5.44-5.48 (m, 1H), 7.01-7.04 (m, 4H), 7.28 (dd, 4H)

[Example 24] Synthesis of compound 24

Using intermediate 1-4 and intermediate 6 and in the same manner as in the synthesis of compound 1 described in Example 1-1, compound 24 was synthesized.

[Chem. 149]

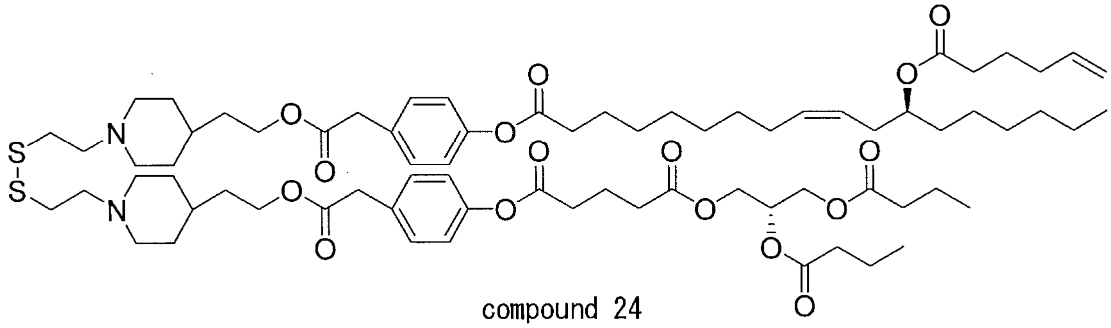

compound 24

<$^1$H-NMR (600 MHz, $CDCl_3$) of compound 24>

δ: 0.86-0.96 (m, 9H), 1.21-1.41 (m, 24H), 1.53-1.77 (m, 16H), 1.93-2.11 (m, 10H), 2.26-2.33 (m, 8H), 2.47-2.65 (m, 10H), 2.81-2.89 (m, 8H), 3.59 (s, 4H), 4.11-4.19 (m, 6H), 4.29-4.35 (m, 2H), 4.88-5.04 (m, 3H), 5.28-5.35 (m, 2H), 5.45-5.48 (m, 1H), 5.78 (dq, 1H), 7.02-7.04 (m, 4H), 7.28 (dd, 4H)

[Example 25] Synthesis of compound 25

Using intermediate 13 and intermediate 1 and in the same manner as in the synthesis of compound 2 described in Example 2-1, compound 25 was synthesized.

[Chem. 150]

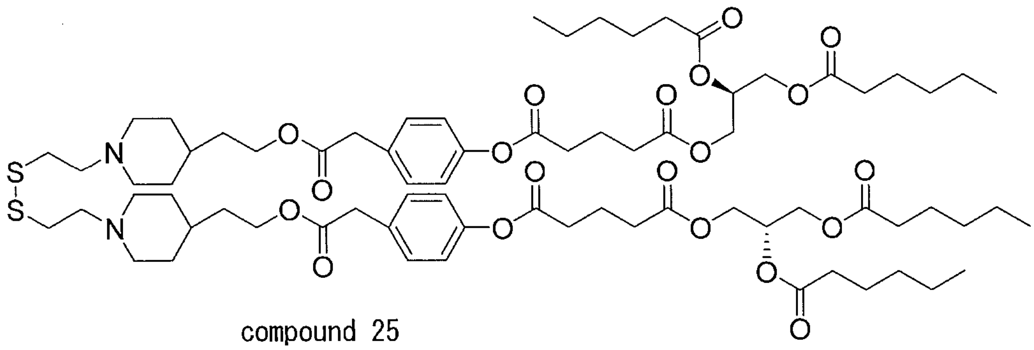

compound 25

<$^1$H-NMR (600 MHz, $CDCl_3$) of compound 25>

δ: 0.82-0.85 (m, 12H), 1.04-1.12 (m, 4H), 1.21-1.28 (m, 18H), 1.45-1.56 (m, 16H), 1.82-1.89 (m, 8H), 2.26-2.30 (m, 8H), 2.41-2.44 (m, 4H), 2.52 (d, 4H), 2.62 (td, 4H), 2.78-2.84 (m, 8H), 3.65 (s, 4H), 4.05-4.29 (m, 12H), 5.19-5.22 (m, 2H), 7.04-7.06 (m, 4H), 7.28 (d, 4H)

[Example 26] Synthesis of compound 26

Compound 26 was synthesized by the following synthesis pathway.

[Chem. 151]

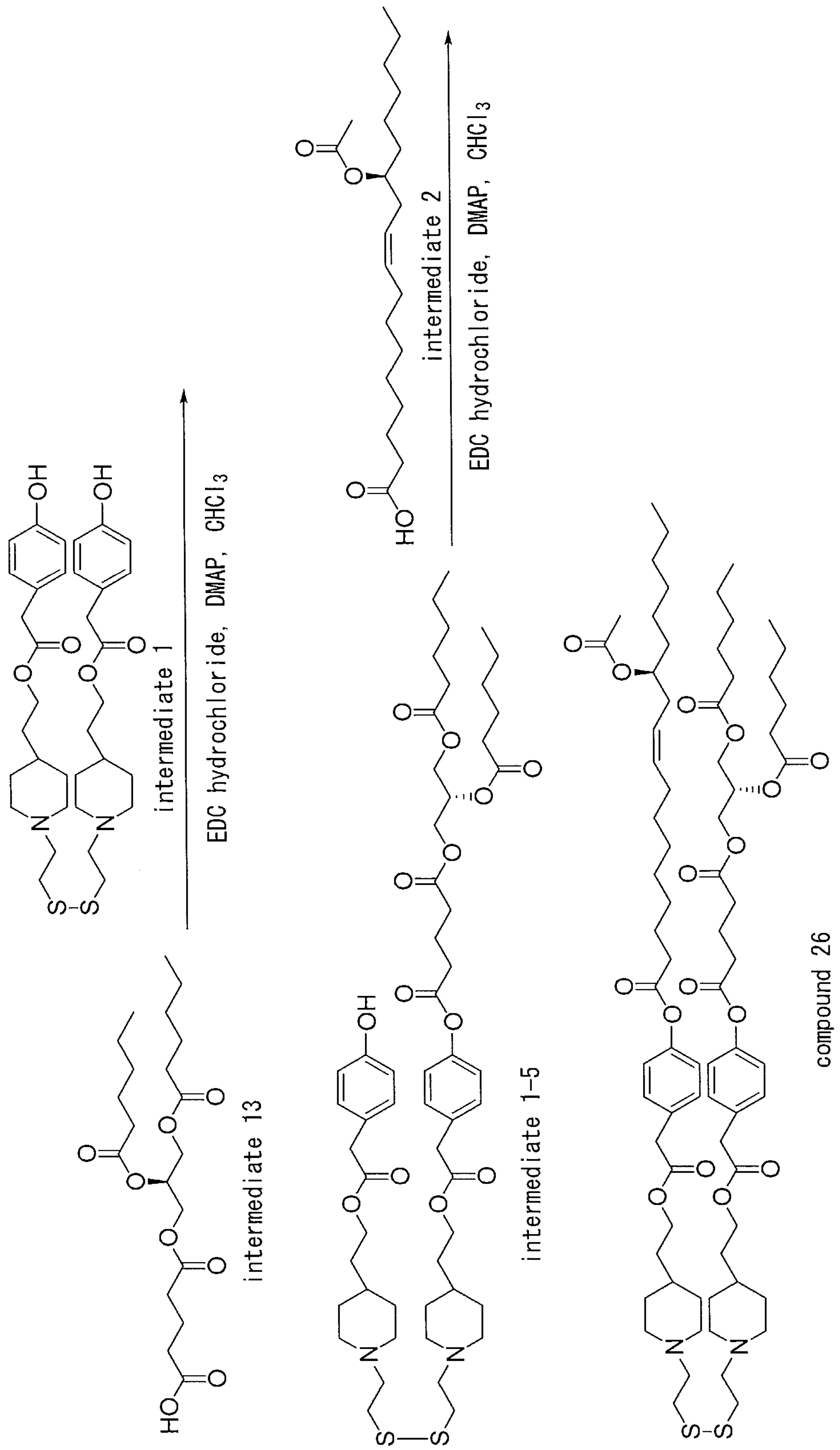


<Synthesis of intermediate 1-5>

Using intermediate 13 and intermediate 1 and in the same manner as in the synthesis of intermediate 1-1 described in Example 1-1, intermediate 1-5 was synthesized.

<Synthesis of compound 26>

Using intermediate 1-5 and intermediate 2 and in the same manner as in the synthesis of compound 1 described in Example 1-1, compound 26 was synthesized.

<$^1$H-NMR (600 MHz, $CDCl_3$) of compound 26>

δ: 0.87-0.91 (m, 9H), 1.26-1.42 (m, 32H), 1.53-1.77 (m, 15H), 1.92-2.09 (m, 11H), 2.26-2.34 (m, 6H), 2.48 (td, 2H), 2.54 (t, 2H), 2.64 (td, 6H), 2.81-2.89 (m, 7H), 3.59 (s, 4H), 4.11-4.19 (m, 6H), 4.29-4.35 (m, 2H), 4.85-4.89 (m, 1H), 5.27-5.35 (m, 2H), 5.46-5.50 (m, 1H), 7.02-7.05 (m, 4H), 7.29 (dd, 4H)

[Example 27] Synthesis of compound 27

Using intermediate 1-5 and intermediate 3 and in the same manner as in the synthesis of compound 1 described in Example 1-1, compound 27 was synthesized.

[Chem. 152]

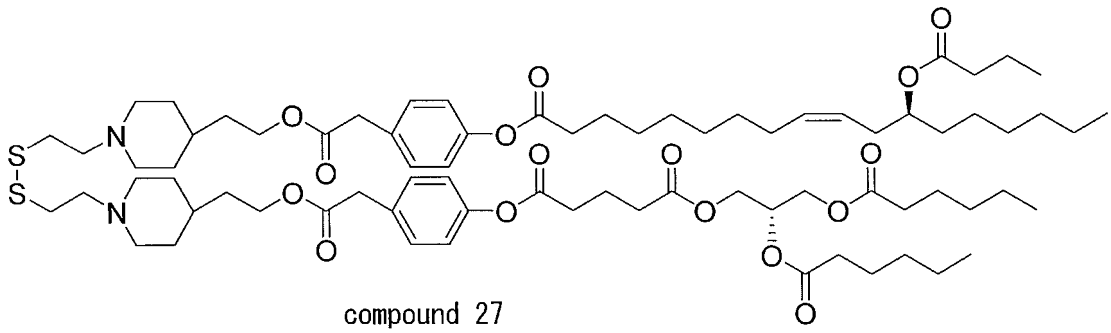

compound 27

<$^1$H-NMR (600 MHz, $CDCl_3$) of compound 27>

δ: 0.82-0.88 (m, 12H), 1.04-1.10 (m, 4H), 1.22-1.36 (m, 28H), 1.46-1.64 (m, 18H), 1.81-1.89 (m, 6H), 1.99 (t, 2H), 2.21-2.30 (m, 9H), 2.41-2.45 (m, 2H), 2.55 (t, 3H), 2.62 (td, 2H), 2.78-2.84 (m, 8H), 3.66 (s, 4H), 4.06-4.30 (m, 8H), 4.79 (t, 1H), 5.20-5.31 (m, 2H), 5.45 (d, 1H), 7.03-7.07 (m, 4H), 7.28 (d, 4H)

[Example 28] Synthesis of compound 28

<Synthesis of intermediate 53>

9-Heptadecanol (3.00 g, 11.7 mmol) was dissolved in chloroform (45.0 g), azelaic acid (4.40 g, 23.4 mmol), DMAP (572 mg, 4.68 mmol), and EDC hydrochloride (6.73 g, 35.1 mmol) were added, and the mixture was reacted at room temperature for 4 hr. The solution after the reaction was washed with 5 wt% saline, and concentrated by an evaporator, and the concentrate was subjected to silica gel column purification using ethanol/chloroform to give 4.23 g of intermediate 53.

[Chem. 153]

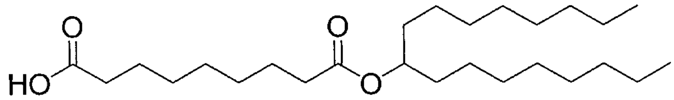

intermediate 53

<Synthesis of compound 28>

Using intermediate 1-5 and intermediate 53 and in the same manner as in the synthesis of compound 1 described in Example 1-1, compound 28 was synthesized.

[Chem. 154]

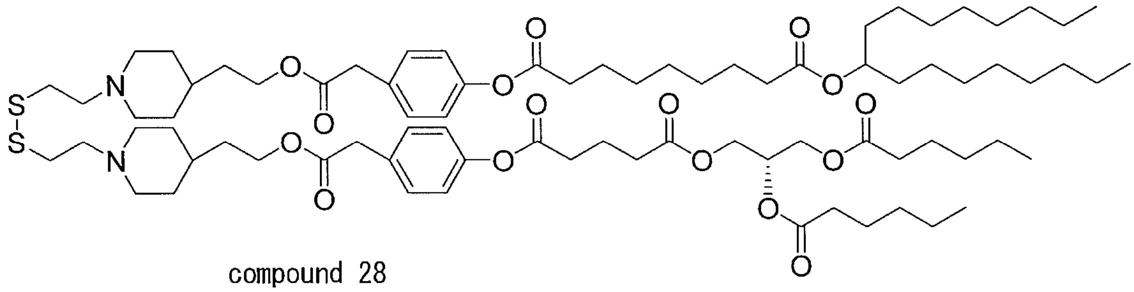

compound 28

<$^1$H-NMR (600 MHz, $CDCl_3$) of compound 28>

δ: 0.83-0.86 (m, 12H), 1.05-1.10 (m, 3H), 1.23-1.34 (m, 42H), 1.47-1.64 (m, 20H), 1.83-1.90 (m, 5H), 2.26-2.31 (m, 6H), 2.39-2.45 (m, 2H), 2.54-2.64 (m, 8H), 2.79-2.83 (m, 8H), 3.66 (s, 4H), 4.06-4.18 (m, 6H), 4.26-4.30 (m, 2H), 4.77-4.79 (m, 1H), 5.22 (td, 1H), 7.03-7.07 (m, 4H), 7.29 (d, 4H)

[Example 29] Synthesis of compound 29

Using intermediate 14 and intermediate 1 and in the same manner as in the synthesis of compound 2 described in Example 2-1, compound 29 was synthesized.

[Chem. 155]

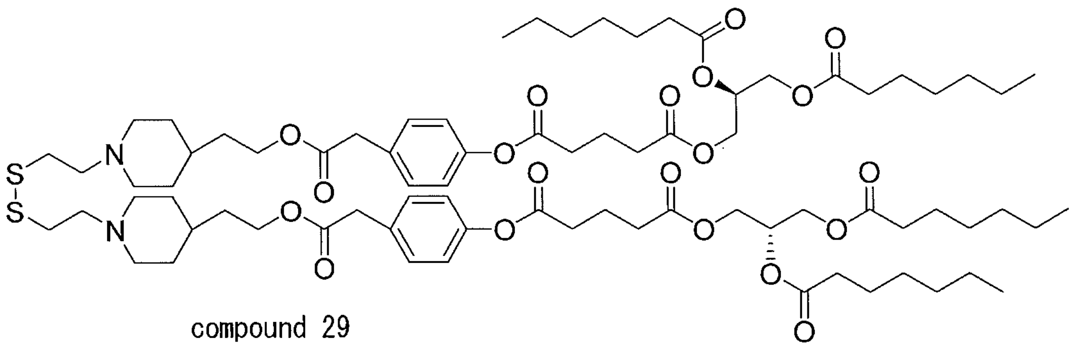

compound 29

<$^1$H-NMR (600 MHz, $CDCl_3$) of compound 29>

δ: 0.87 (qd, 12H), 1.23-1.33 (m, 32H), 1.54-1.65 (m, 12H), 1.95 (s, 4H), 2.04-2.08 (m, 4H), 2.30-2.33 (m, 8H), 2.47 (td, 4H), 2.63 (td, 8H), 2.81-2.89 (m, 8H), 3.59 (s, 4H), 4.11-4.18 (m, 8H), 4.28-4.34 (m, 4H), 5.28 (t, 4H), 7.02-7.04 (m, 4H), 7.28 (d, 4H)

[Example 30] Synthesis of compound 30

Compound 30 was synthesized by the following synthesis pathway.

[Chem. 156]

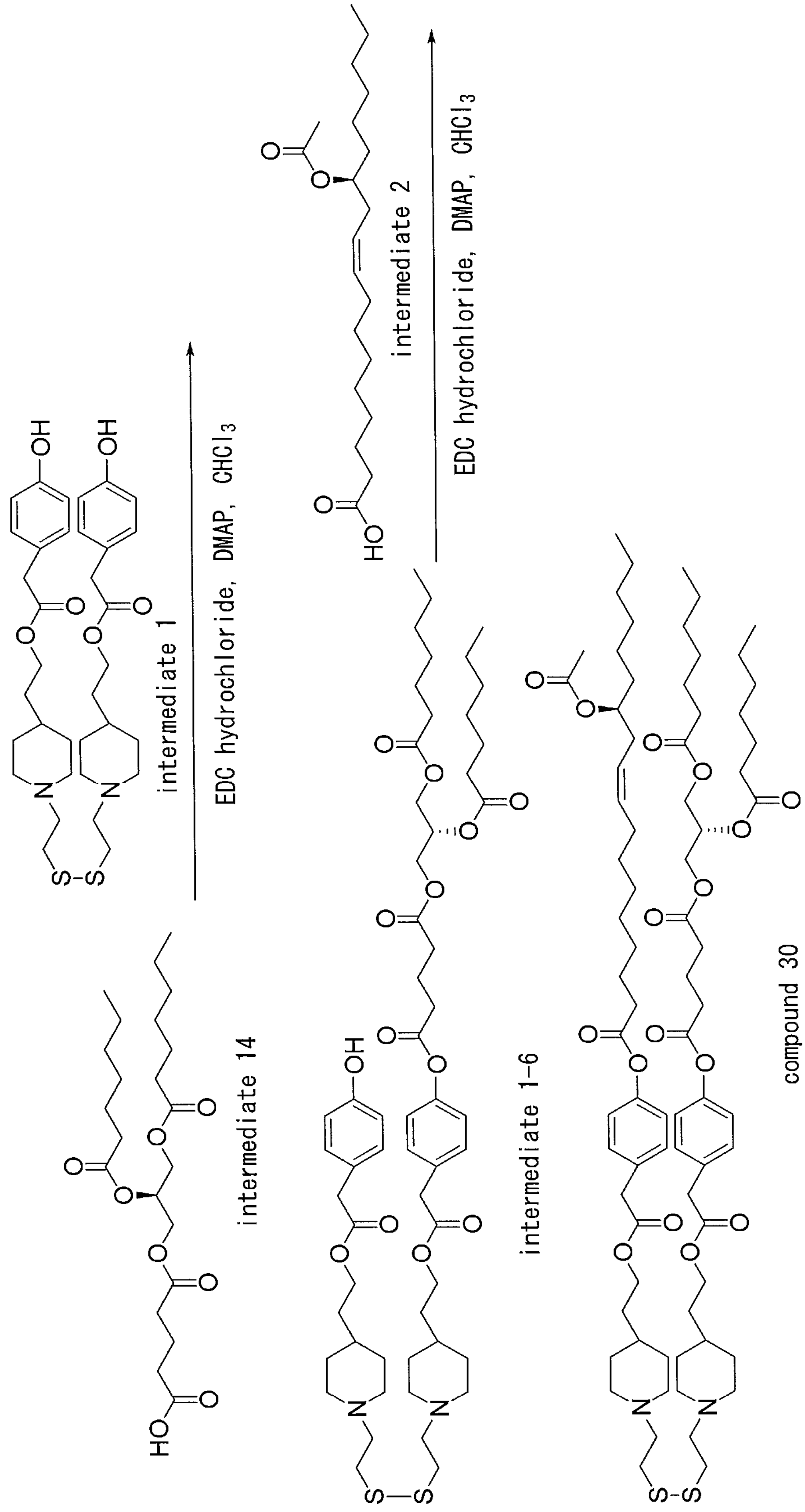
intermediate 1
EDC hydrochloride, DMAP, $CHCl_3$
intermediate 2
EDC hydrochloride, DMAP, $CHCl_3$
intermediate 14
intermediate 1-6
compound 30

<Synthesis of intermediate 1-6>

Using intermediate 14 and intermediate 1 and in the same manner as in the synthesis of intermediate 1-1 described in Example 1-1, intermediate 1-6 was synthesized.

<Synthesis of compound 30>

Using intermediate 1-6 and intermediate 2 and in the same manner as in the synthesis of compound 1 described in Example 1-1, compound 30 was synthesized.

<$^1$H-NMR (600 MHz, $CDCl_3$) of compound 30>

δ: 0.82-0.85 (m, 9H), 1.04-1.09 (m, 5H), 1.23-1.33 (m, 31H), 1.46-1.65 (m, 16H), 1.82-2.01 (m, 11H), 2.23-2.30 (m, 6H), 2.38-2.44 (m, 2H), 2.54-2.63 (m, 6H), 2.78-2.84 (m, 8H), 3.66 (s, 4H), 4.05-4.17 (m, 6H), 4.26-4.35 (m, 2H), 4.75-4.77 (m, 1H), 5.20-5.33 (m, 2H), 5.43-5.46 (m, 1H), 7.03-7.06 (m, 4H), 7.28 (d, 4H)

[Example 31] Synthesis of compound 31

Using intermediate 1-6 and intermediate 3 and in the same manner as in the synthesis of compound 1 described in Example 1-1, compound 31 was synthesized.

[Chem. 157]

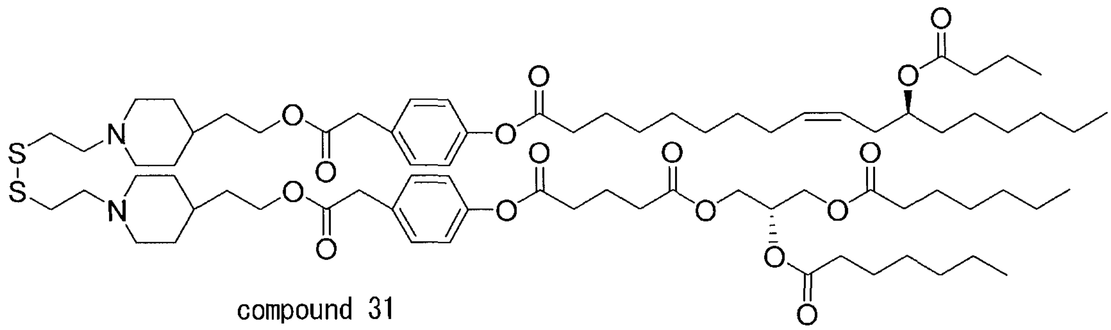

compound 31

<$^1$H-NMR (600 MHz, $CDCl_3$) of compound 31>

δ: 0.83-0.88 (m, 12H), 1.05-1.10 (m, 4H), 1.23-1.34 (m, 31H), 1.47-1.64 (m, 19H), 1.82-1.89 (m, 6H), 2.01 (d, 2H), 2.21-2.45 (m, 10H), 2.54-2.64 (m, 6H), 2.79-2.85 (m, 8H), 3.72-3.61 (m, 4H), 4.06-4.17 (m, 6H), 4.26-4.30 (m, 2H), 4.78-4.80 (m, 1H), 5.20-5.32 (m, 2H), 5.45 (d, 1H), 7.05 (td, 4H), 7.29 (d, 4H)

[Example 32] Synthesis of compound 32

Using intermediate 1-6 and intermediate 53 and in the same manner as in the synthesis of compound 1 described in Example 1-1, compound 32 was synthesized.

[Chem. 158]

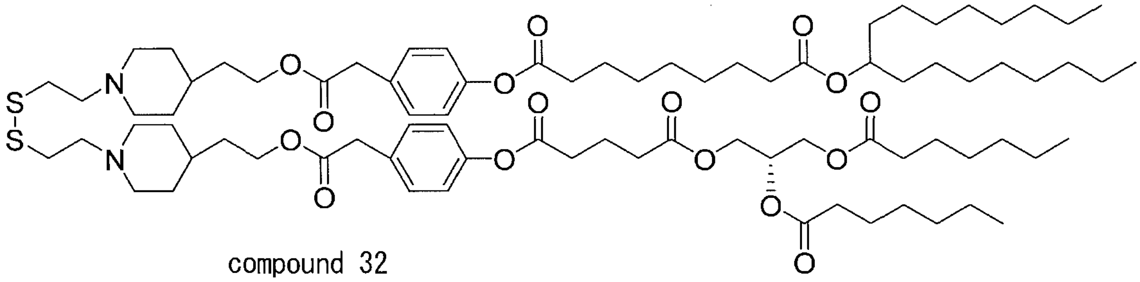

compound 32

<$^1$H-NMR (600 MHz, $CDCl_3$) of compound 32>

δ: 0.86-0.90 (m, 12H), 1.26-1.41 (m, 50H), 1.50-1.65 (m, 16H), 1.72-1.77 (m, 2H), 1.94-2.08 (m, 6H), 2.27-2.34 (m, 6H), 2.48 (td, 2H), 2.54 (t, 2H), 2.64 (td, 6H), 2.81-2.89 (m, 8H), 3.59 (s, 4H), 4.11-4.18 (m, 6H), 4.29-4.35 (m, 2H), 4.85-4.89 (m, 1H), 5.26-5.30 (m, 1H), 7.02-7.09 (m, 4H), 7.28 (td, 4H)

[Example 33] Synthesis of compound 33

<Synthesis of intermediate 7-1>

Methyl ricinoleate (5.07 g, 16.0 mmol), 4-pentynoic acid (1.74 g, 17.6 mmol), and DMAP (393 mg, 3.20 mmol) were dissolved in chloroform (50.0 g) at room temperature. To the obtained mixture was added EDC hydrochloride (4.60 g, 24.0 mmol) and the mixture was reacted at room temperature for 1 hr. The subsequent steps were performed in the same manner as in the synthesis of intermediate 2-1 described in Example 2-1 to obtain 6.13 g of intermediate 7-1.

[Chem. 159]

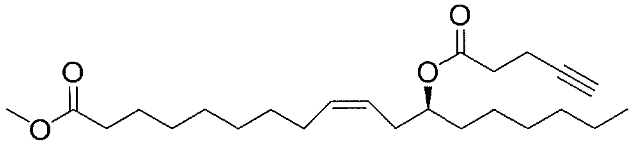

intermediate 7-1

<Synthesis of intermediate 7>

Intermediate 7-1 (6.13 g, 15.6 mmol) was dissolved in tert-butanol to 0.3 M at room temperature. To the obtained mixture was added 2 M NaOH aqueous solution (8.74 g) and the mixture was reacted at room temperature for 8 hr. The subsequent steps were performed in the same manner as in the synthesis of intermediate 2 described in Example 2-1 to obtain 1.80 g of intermediate 7.

[Chem. 160]

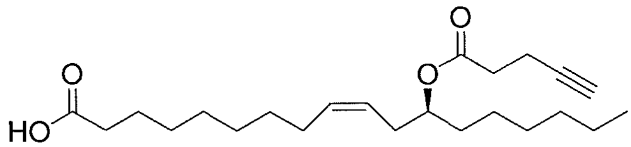

intermediate 7

<Synthesis of compound 33>

Using intermediate 7 and intermediate 1-1 and in the same manner as in the synthesis of compound 1 described in Example 1-1, compound 33 was synthesized.

[Chem. 161]

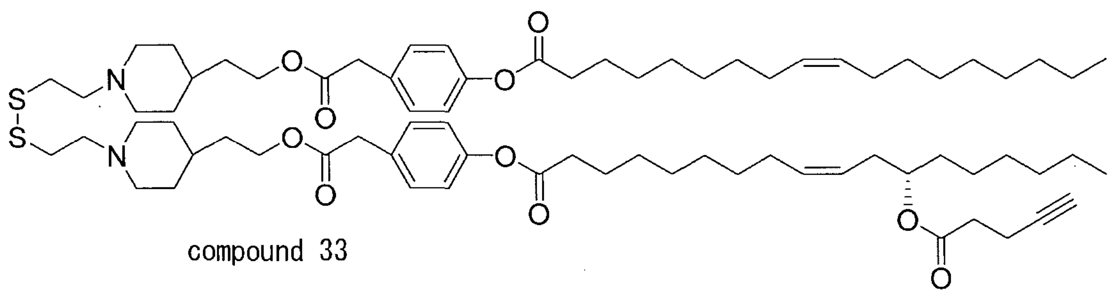

compound 33

<$^1$H-NMR (600 MHz, $CDCl_3$) of compound 33>

δ: 0.84 (td, 6H), 1.07 (q, 4H), 1.22-1.33 (m, 38H), 1.45-1.65 (m, 14H), 1.83 (t, 4H), 1.97-2.01 (m, 6H), 2.26 (t, 2H),

2.37-2.47 (m, 4H), 2.52-2.61 (m, 9H), 2.77-2.84 (m, 8H), 3.65 (s, 4H), 4.06 (t, 4H), 4.78-4.82 (m, 1H), 5.30-5.47 (m, 4H), 7.04 (d, 4H), 7.28 (d, 4H)

[Example 34] Synthesis of compound 34

<Synthesis of intermediate 8-1>

Methyl ricinoleate (5.00 g, 16.0 mmol), 5-hexynoic acid (1.97 g, 17.6 mmol), and DMAP (391 mg, 3.20 mmol) were dissolved in chloroform (50.0 g) at room temperature. To the obtained mixture was added EDC hydrochloride (4.6 g, 24.0 mmol) and the mixture was reacted at room temperature for 1 hr. The subsequent steps were performed in the same manner as in the synthesis of intermediate 2-1 described in Example 2-1 to obtain 6.77 g of intermediate 8-1.

[Chem. 162]

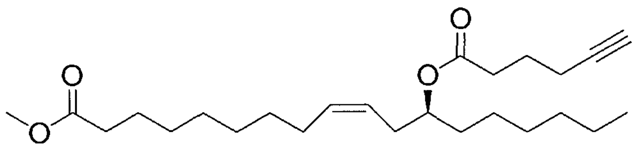

intermediate 8-1

<Synthesis of intermediate 8>

Intermediate 8-1 (6.77 g, 16.6 mmol) was dissolved in tert-butanol to 0.3 M at room temperature. To the obtained mixture was added 2 M NaOH aqueous solution (10.0 g) and the mixture was reacted at room temperature for 8 hr. The subsequent steps were performed in the same manner as in the synthesis of intermediate 2 described in Example 2-1 to obtain 3.43 g of intermediate 8.

[Chem. 163]

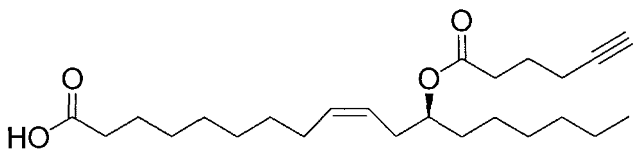

intermediate 8

<Synthesis of compound 34>

Using intermediate 8 and intermediate 1-1 and in the same manner as in the synthesis of compound 1 described in Example 1-1, compound 34 was synthesized.

[Chem. 164]

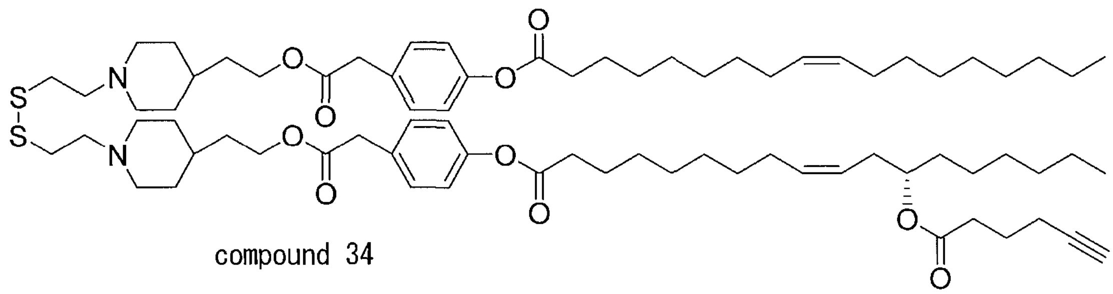

compound 34

<$^1$H-NMR (600 MHz, $CDCl_3$) of compound 34>

δ: 0.87 (td, 6H), 1.23-1.41 (m, 43H), 1.53-1.64 (m, 9H), 1.71-1.76 (m, 4H), 1.81-1.86 (m, 2H), 1.92-2.02 (m, 11H), 2.24-2.32 (m, 4H), 2.42 (t, 2H), 2.58 (dt, 8H), 2.80-2.88 (m, 8H), 3.59 (s, 4H), 4.12 (t, 4H), 4.87-4.91 (m, 1H), 5.30-5.36 (m, 3H), 5.45-5.49 (m, 1H), 7.01-7.08 (m, 4H), 7.33-7.26 (m, 4H)

[Example 35] Synthesis of compound 35

Using intermediate 1-5 and intermediate 15 and in the same manner as in the synthesis of compound 1 described in Example 1-1, compound 35 was synthesized.

[Chem. 165]

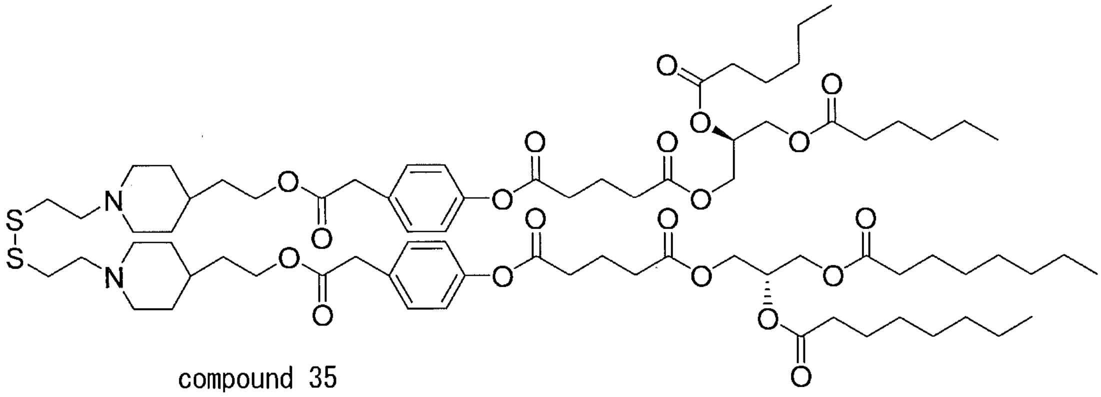

compound 35

<$^1$H-NMR (600 MHz, $CDCl_3$) of compound 35>

δ: 0.83-0.86 (m, 12H), 1.05-1.12 (m, 4H), 1.25 (d, 24H), 1.46-1.57 (m, 16H), 1.83-1.90 (m, 8H), 2.25-2.31 (m, 8H), 2.42-2.45 (m, 4H), 2.52-2.64 (m, 10H), 2.79-2.85 (m, 8H), 3.66 (s, 4H), 4.06-4.18 (m, 8H), 4.26-4.35 (m, 4H), 5.21 (q, 2H), 7.05-7.07 (m, 4H), 7.29 (d, 4H)

[Example 36] Synthesis of compound 36

Using intermediate 1-4 and intermediate 15 and under conditions similar to those in the synthesis of compound 1 described in Example 1-1, the reaction was performed to synthesize 36.

[Chem. 166]

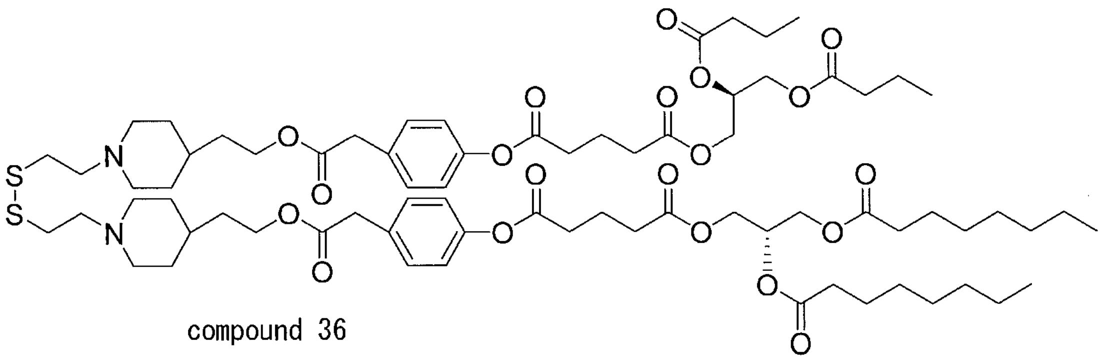

compound 36

<$^1$H-NMR (600 MHz, $CDCl_3$) of compound 36>

δ: 0.87-0.89 (t, 6H), 0.94-0.96 (t, 6H), 1.27-1.31 (m, 22H), 1.56-1.67 (m, 20H), 1.94-2.03 (m, 4H), 2.05-2.08 (m, 4H), 2.29-2.33 (m, 8H), 2.47-2.49 (t, 4H), 2.62-2.65 (t, 4H), 2.82-2.90 (m, 8H), 3.60 (s, 4H), 4.11-4.18 (m, 8H), 4.30-4.37 (m, 4H), 5.27-5.32 (m, 2H), 7.03-7.04 (m, 4H), 7.26-7.29 (m, 4H)

[Example 37] Synthesis of compound 37

Using intermediate 8 and intermediate 1 and in the same manner as in the synthesis of compound 2 described in Example 2-1, compound 37 was synthesized.

[Chem. 167]

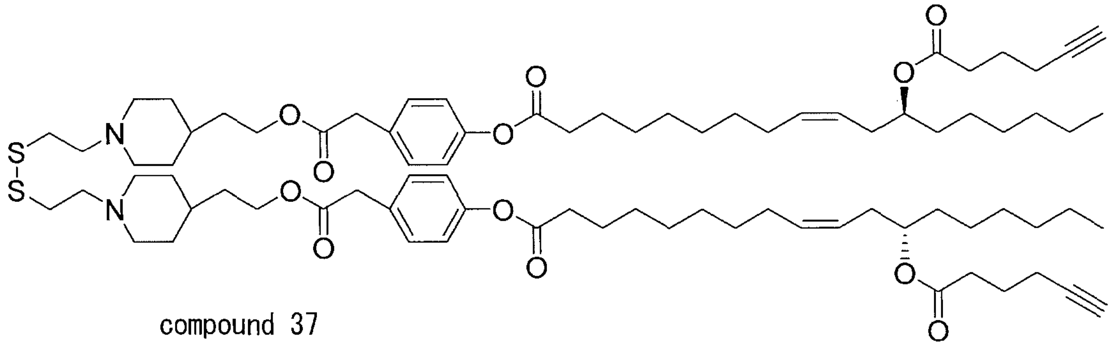

compound 37

<$^{1}$H-NMR (600 MHz, $CDCl_3$) of compound 37>

δ: 0.87-0.89 (t, 6H), 1.26-1.41 (m, 32H), 1.55-1.65 (m, 20H), 1.72-1.76 (m, 4H), 1.82-1.87 (m, 4H), 1.96-2.04 (m, 8H), 2.24-2.31 (m, 8H), 2.41-2.43 (t, 4H), 2.53-2.56 (t, 4H), 2.64 (m, 4H), 2.85-2.87 (m, 8H), 3.59 (s, 4H), 4.11-4.14 (t, 4H), 4.88-4.91 (m, 2H), 5.32-5.35 (m, 2H), 5.47-5.48 (m, 2H), 7.02-7.04 (m, 4H), 7.26-7.29 (m, 4H)

[Example 38] Synthesis of compound 38

Using intermediate 1-4 and intermediate 8 and in the same manner as in the synthesis of compound 1 described in Example 1-1, compound 38 was synthesized.

[Chem. 168]

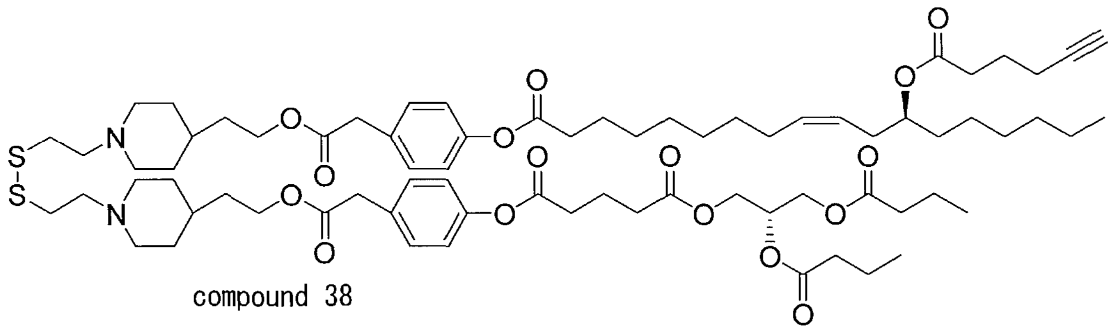

compound 38

<$^{1}$H-NMR (600 MHz, $CDCl_3$) of compound 38>

δ: 0.87-0.89 (t, 3H), 0.94-0.96 (m, 6H), 1.21-1.43 (m, 24H), 1.51-1.69 (m, 10H), 1.72-1.76 (m, 2H), 1.82-1.87 (m, 2H), 1.92-1.99 (m, 5H), 2.00-2.09 (m, 4H), 2.24-2.38 (m, 8H), 2.41-2.43 (t, 4H), 2.46-2.50 (t, 4H), 2.52-2.56 (t, 4H), 2.60-2.67 (m, 4H), 2.79-2.84 (m, 6H), 3.59 (s, 4H), 4.11-4.19 (m, 6H), 4.29-4.36 (m, 2H), 4.88-4.93 (m, 1H), 5.26-5.35 (m, 2H), 5.44-5.51 (m, 1H), 7.02-7.04 (m, 4H), 7.26-7.29 (m, 4H)

[Example 39] Synthesis of compound 39

Compound 39 was synthesized by the following synthesis pathway.

[Chem. 169]

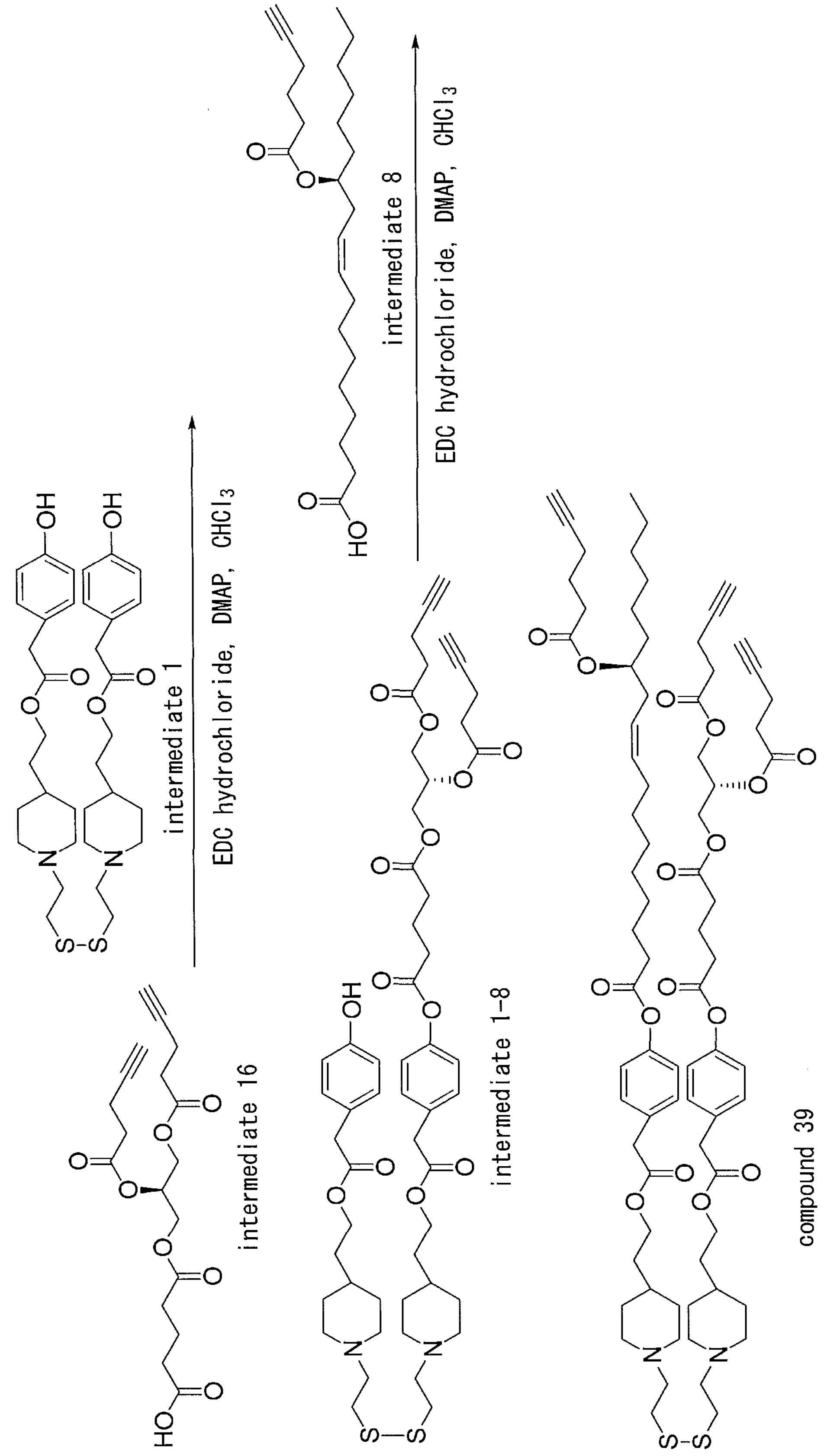


intermediate 8

intermediate 1

intermediate 16

intermediate 1-8

compound 39

<Synthesis of intermediate 16-1>

Intermediate 11-2 (6.91 g, 20.9 mmol) obtained by the synthesis described in Example 15, 4-pentynoic acid (4.52 g, 46.1 mmol), and DMAP (510 mg, 4.17 mmol) were dissolved in chloroform (70.0 g) at room temperature. To the obtained mixture was added EDC hydrochloride (12.0 g, 62.6 mmol) and the mixture was reacted at room temperature for 1 hr. The subsequent steps were performed in the same manner as in the synthesis of intermediate 11-3 described in Example 15 to obtain 9.03 g of intermediate 16-1.

[Chem. 170]

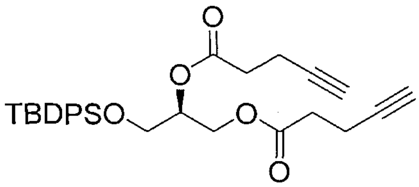

intermediate 16-1

<Synthesis of intermediate 16-2>

Intermediate 16-1 (9.03 g, 18.4 mmol), and acetic acid (4.42 g, 73.6 mmol) were dissolved in THF (27.1 g) at room temperature. To the obtained mixture was added 1 M TBAF solution (67.7 g, 73.6 mmol) in THF and the mixture was reacted at room temperature for 18 hr. Thereafter, to the reaction solution was added ethyl acetate (90.6 g). The subsequent steps were performed in the same manner as in the synthesis of intermediate 11-4 described in Example 15 to obtain 3.94 g of intermediate 16-2.

[Chem. 171]

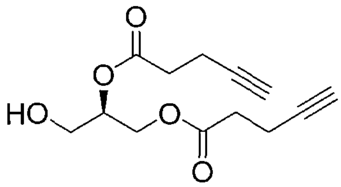

intermediate 16-2

<Synthesis of intermediate 16>

Intermediate 16-2 (3.94 g, 15.6 mmol), glutaric anhydride (3.16 g, 27.7 mmol), triethylamine (5.58 g, 55.1 mmol), and DMAP (449 mg, 3.67 mmol) were dissolved in chloroform (46.6 g) at room temperature, and the mixture was reacted at room temperature for 1 hr. The subsequent steps were performed in the same manner as in the synthesis of intermediate 11 described in Example 15 to obtain 1.83 g of intermediate 16.

<Synthesis of intermediate 1-8>

Using intermediate 16 and intermediate 1 and in the same manner as in the synthesis of intermediate 1-1 described in Example 1-1, intermediate 1-8 was synthesized.

<Synthesis of compound 39>

Using intermediate 1-8 and intermediate 8 and under conditions similar to those in the synthesis of compound 1 described in Example 1-1, the reaction was performed to synthesize compound 39.

<$^1$H-NMR (600 MHz, $CDCl_3$) of compound 39>

δ: 0.87-0.89 (t, 3H), 1.20-1.43 (m, 24H), 1.51-1.67 (m, 12H), 1.70-1.77 (m, 2H), 1.80-1.86 (m, 2H), 1.92-2.00 (m, 6H), 2.01-2.09 (m, 4H), 2.23-2.33 (m, 4H), 2.40-2.43 (t, 2H), 2.47-2.60 (m, 11H), 2.61-2.6 (m, 5H), 2.79-2.92 (m, 7H), 3.59 (s, 4H), 4.11-4.14 (t, 4H), 4.19-4.24 (m, 2H), 4.36-4.40 (m, 2H), 4.86-4.92 (m, 1H), 5.27-5.35 (m, 2H), 5.44-5.50 (m, 1H), 7.02-7.04 (m, 4H), 7.26-7.29 (m, 4H)

[Example 40] Synthesis of compound 40

Using intermediate 16 and intermediate 1-1 and in the same manner as in the synthesis of compound 1 described in Example 1-1, compound 40 was synthesized.

[Chem. 172]

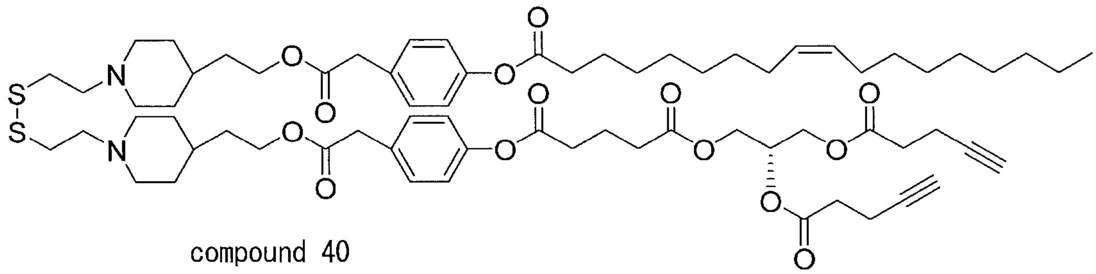

compound 40

<$^1$H-NMR (600 MHz, $CDCl_3$) of compound 40>

δ: 0.89-0.93 (t, 3H), 1.23-1.47 (m, 26H), 1.55-1.72 (m, 16H), 1.73-1.81 (m, 2H), 1.94-2.14 (m, 10H), 2.49-2.64 (m, 10H), 2.61-2.67 (m, 4H), 2.82-2.97 (m, 6H), 3.62 (s, 4H), 4.11-4.18 (t, 4H), 4.19-4.27 (m, 2H), 4.36-4.44 (m, 2H), 5.30-5.42 (m, 3H), 7.02-7.04 (m, 4H), 7.26-7.29 (m, 4H)

[Example 41] Synthesis of compound 41

<Synthesis of intermediate 21>

Intermediate 21 was synthesized by the following reaction.

cis-2-Nonen-1-ol (985 mg, 7.68 mmol), glutaric anhydride (963 mg, 8.44 mmol), and DMAP (188 mg, 1.54 mmol) were dissolved in chloroform (14.8 g) at room temperature, and the mixture was reacted at room temperature for 1 hr. The subsequent steps were performed in the same manner as in the synthesis of intermediate 11 described in Example 5 to obtain 1.90 g of intermediate 21.

[Chem. 173]

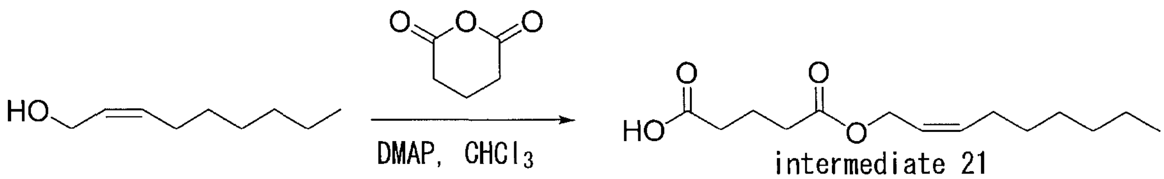


intermediate 21

<Synthesis of compound 41>

Using intermediate 21 and intermediate 1 and in the same manner as in the synthesis of compound 2 described in Example 2-1, compound 41 was synthesized.

[Chem. 174]

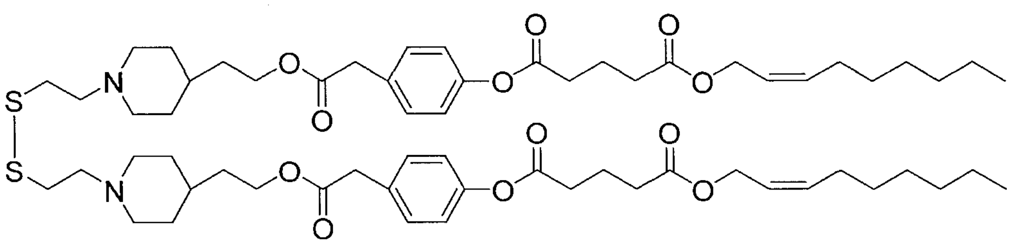

compound 41

<$^1$H-NMR (600 MHz, $CDCl_3$) of compound 41>

δ: 0.86-0.90 (t, 6H), 1.20-1.40 (m, 18H), 1.52-1.66 (m, 12H), 1.91-1.99 (m, 4H), 2.04-2.14 (m, 8H), 2.44-2.49 (m, 4H), 2.61-2.67 (m, 8H), 2.80-2.92 (m, 8H), 3.59 (s, 4H), 4.10-4.14 (t, 4H), 4.64-4.66 (m, 4H), 5.50-5.54 (m, 2H), 5.13-5.18 (m, 2H), 7.02-7.04 (m, 4H), 7.26-7.29 (m, 4H)

[Example 42] Synthesis of compound 42

Compound 42 was synthesized by the following synthesis pathway.

[Chem. 175]

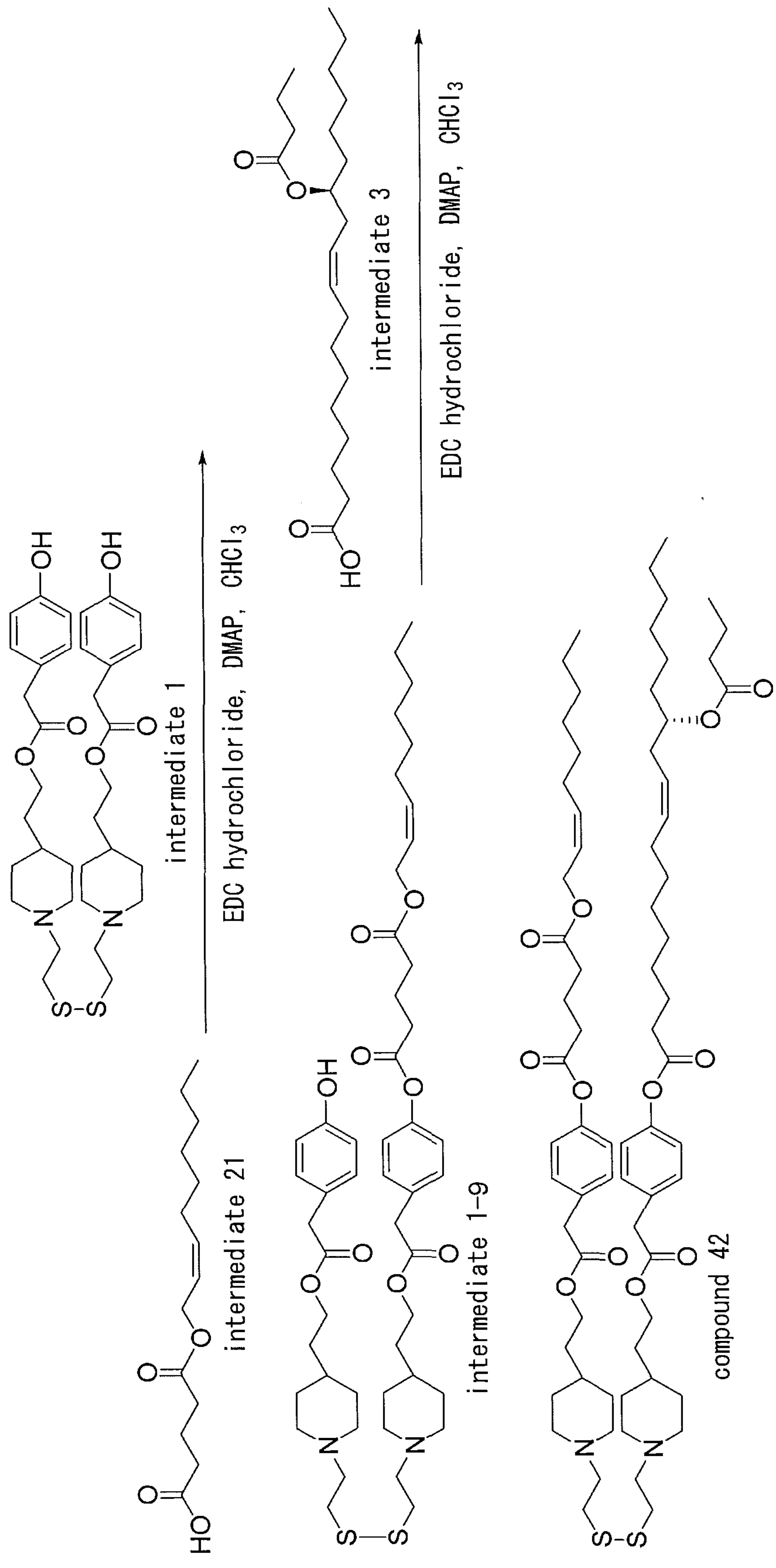
intermediate 3
EDC hydrochloride, DMAP, CHCl3
intermediate 1
EDC hydrochloride, DMAP, CHCl3
intermediate 21
intermediate 1-9
compound 42

<Synthesis of intermediate 1-9>

Using intermediate 21 and intermediate 1 and in the same manner as in the synthesis of intermediate 1-1, intermediate 1-9 was synthesized.

<Synthesis of compound 42>

Using intermediate 1-9 and intermediate 3 and in the same manner as in the synthesis of compound 1 described in Example 1-1, compound 42 was synthesized.

<$^1$H-NMR (600 MHz, $CDCl_3$) of compound 42>

δ: 0.86-0.89 (m, 6H), 0.99 (t, 3H), 1.22-1.42 (m, 30H), 1.46-1.72 (m, 14H), 1.91-1.98 (m, 2H), 1.99-2.10 (m, 8H), 2.52-2.66 (m, 14H), 2.78-2.92 (m, 8H), 3.59 (s, 4H), 4.11-4.13 (t, 4H), 4.58-4.71 (m, 3H), 5.30-5.38 (m, 4H), 7.02-7.04 (m, 4H), 7.26-7.29 (m, 4H)

[Example 43] Synthesis of compound 43

Using intermediate 21 and intermediate 1-1 and in the same manner as in the synthesis of compound 1 described in Example 1-1, compound 43 was synthesized.

[Chem. 176]

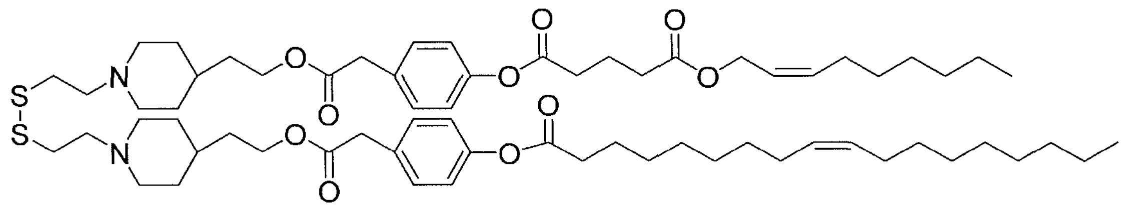

compound 43

<$^1$H-NMR (600 MHz, $CDCl_3$) of compound 43>

δ: 0.86-0.89 (m, 6H), 1.22-1.42 (m, 34H), 1.54-1.76 (m, 10H), 1.91-1.98 (m, 4H), 1.99-2.38 (m, 12H), 2.52-2.56 (t, 2H), 2.62-2.66 (m, 4H), 2.78-2.92 (m, 8H), 3.59 (s, 4H), 4.11-4.13 (t, 4H), 4.67-4.71 (m, 2H), 5.30-5.38 (m, 2H), 5.58-5.64 (m, 2H), 7.02-7.04 (m, 4H), 7.26-7.29 (m, 4H)

[Example 44] Synthesis of compound 44

<Synthesis of intermediate 10-1>

Methyl ricinoleate (5.09 g, 16.0 mmol), 1-adamantaneacetic acid (3.45 g, 17.8 mmol), and DMAP (391 mg, 3.20 mmol) were dissolved in chloroform (50.5 g) at room temperature. To the obtained mixture was added EDC hydrochloride (4.60 g, 24.0 mmol) and the mixture was reacted at room temperature for 1 hr. The subsequent steps were performed in the same manner as in the synthesis of intermediate 2-1 described in Example 2-1 to obtain 7.32 g of intermediate 10-1.

[Chem. 177]

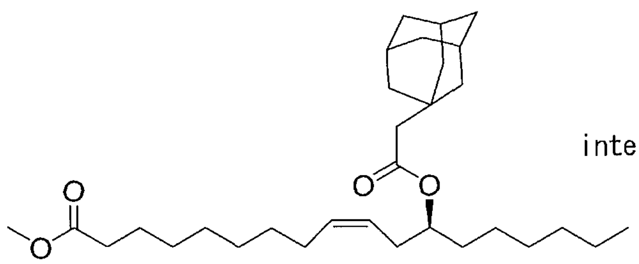

intermediate 10-1

<Synthesis of intermediate 10>

Intermediate 10-1 (7.32 g, 15.0 mmol) was dissolved in tert-butanol to 0.3 M at room temperature. To the obtained mixture was added 2 M NaOH aqueous solution (8.46 g) and the mixture was reacted at room temperature for 8 hr. The subsequent steps were performed in the same manner as in the synthesis of intermediate 2 described in Example 2-1 to obtain 5.00 g of intermediate 10.

[Chem. 178]

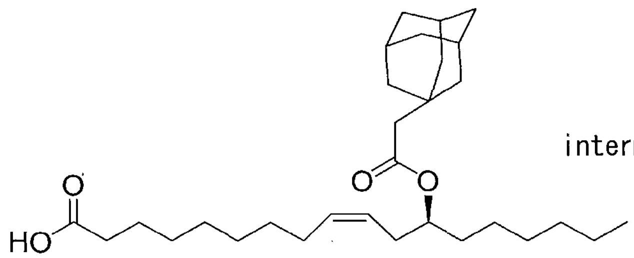

intermediate 10

<Synthesis of compound 44>

Using intermediate 1-9 and intermediate 10 and in the same manner as in the synthesis of compound 1 described in Example 1-1, compound 44 was synthesized.

[Chem. 179]

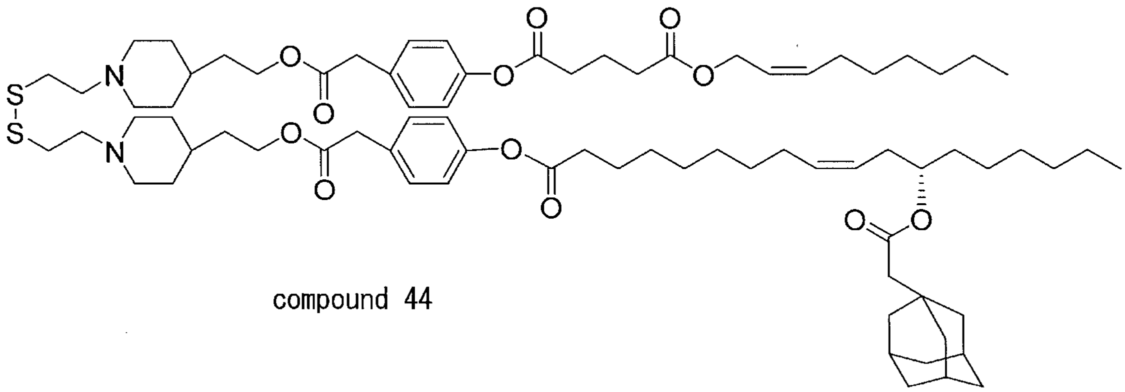

compound 44

<$^1$H-NMR (600 MHz, $CDCl_3$) of compound 44>

δ: 0.86-0.89 (m, 6H), 1.21-1.43 (m, 30H), 1.52-1.77 (m, 25H), 1.92-1.99 (m, 6H), 2.01-2.14 (m, 8H), 2.24-2.65 (m, 2H), 2.44-2.48 (t, 2H), 2.52-2.56 (t, 2H), 2.62-2.67 (m, 6H), 2.79-2.91 (m, 8H), 3.59 (s, 4H), 4.09-4.14 (t, 4H), 4.63-4.67 (m, 2H), 4.85-4.89 (m, 1H), 5.31-5.58 (m, 1H), 5.44-5.50 (m, 1H), 5.51-5.56 (m, 1H), 5.64-5.69 (m, 1H), 7.02-7.04 (m, 4H), 7.26-7.29 (m, 4H)

[Example 45] Synthesis of compound 45

<Synthesis of intermediate 22>

3-Decyn-1-ol (1.18 g, 7.68 mmol), glutaric anhydride (963 mg, 8.44 mmol), and DMAP (188 mg, 1.54 mmol) were dissolved in chloroform (18.0 g) at room temperature, and the mixture was reacted at room temperature for 1 hr. The subsequent steps were performed in the same manner as in the synthesis of intermediate 21 described in Example 41 to obtain 1.99 g of intermediate 22.

[Chem. 180]

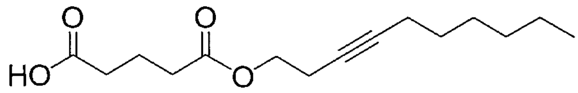

intermediate 22

[0811]

<Synthesis of compound 45>

Using intermediate 22 and intermediate 1 and in the same manner as in the synthesis of compound 1 described in Example 1-1, compound 45 was synthesized.

[Chem. 181]

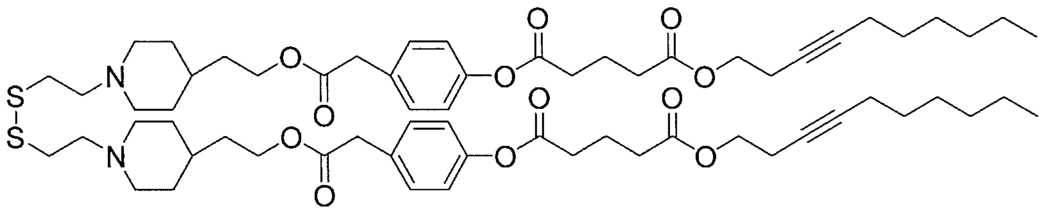

compound 45

<$^1$H-NMR (600 MHz, $CDCl_3$) of compound 45>

δ: 0.86-0.90 (m, 6H), 1.20-1.39 (m, 18H), 1.43-1.49 (m, 4H), 1.52-1.67 (m, 10H), 1.92-1.99 (m, 2H), 2.04-2.10 (m, 4H), 2.20-2.24 (m, 4H), 2.44-2.51 (m, 8H), 2.61-2.67 (m, 8H), 2.79-2.91 (m, 8H), 3.59 (s, 4H), 4.09-4.14 (t, 4H), 4.15-4.18 (t, 4H), 7.02-7.04 (m, 4H), 7.26-7.29 (m, 4H)

[Example 46] Synthesis of compound 46

<Synthesis of ester of tocopherol and glutaric acid>

Tocopherol (5.00 g, 11.6 mmol), glutaric anhydride (2.65 g, 23.2 mmol), DMAP (425 mg, 3.48 mmol), and triethylamine (3.52 g, 34.8 mmol) were dissolved in chloroform (50.0 g) at room temperature. The mixture was reacted at room temperature for 12 hr. The subsequent steps were performed in the same manner as in the synthesis of intermediate 11 described in Example 15 to obtain 6.19 g of an ester of tocopherol and glutaric acid.

[Chem. 182]

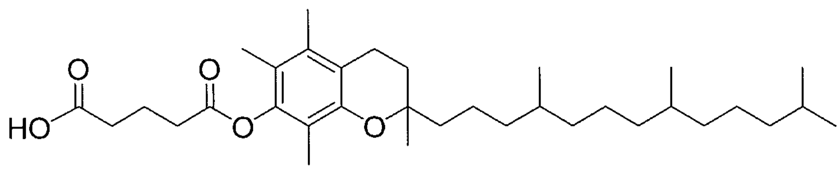

ester of tocopherol and glutaric acid

<Synthesis of compound 46>

Using intermediate 1-9 and an ester of tocopherol and glutaric acid and in the same manner as in the synthesis of compound 1 described in Example 1-1, compound 46 was synthesized.

[Chem. 183]

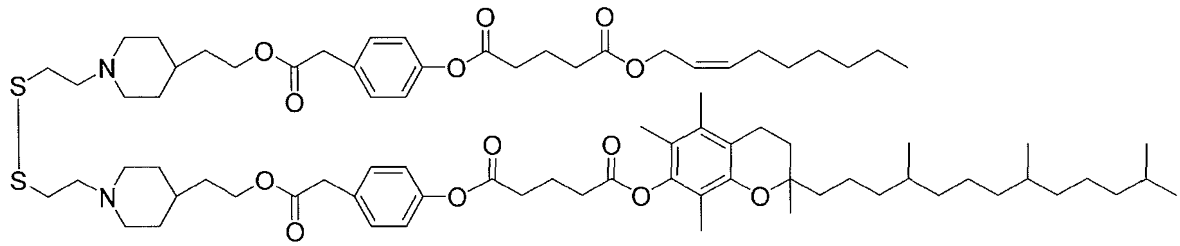

compound 46

<$^1$H-NMR (600 MHz, DMSO-$d_6$) of compound 46>

δ: 0.87-0.92 (m, 15H), 1.17-1.60 (m, 46H), 1.79 (ddd, 1H), 2.04 (ddd, 1H), 2.08 (s, 3H), 2.16-2.87 (m, 38H), 3.71 (s, 4H), 4.13 (t, 4H), 4.69 (d, 2H), 5.59-5.62 (m, 2H), 7.02-7.04 (m, 4H), 7.26-7.29 (m, 4H)

[Example 47] Synthesis of compound 47

Compound 47 was synthesized by the following synthesis pathway.

[Chem. 184]

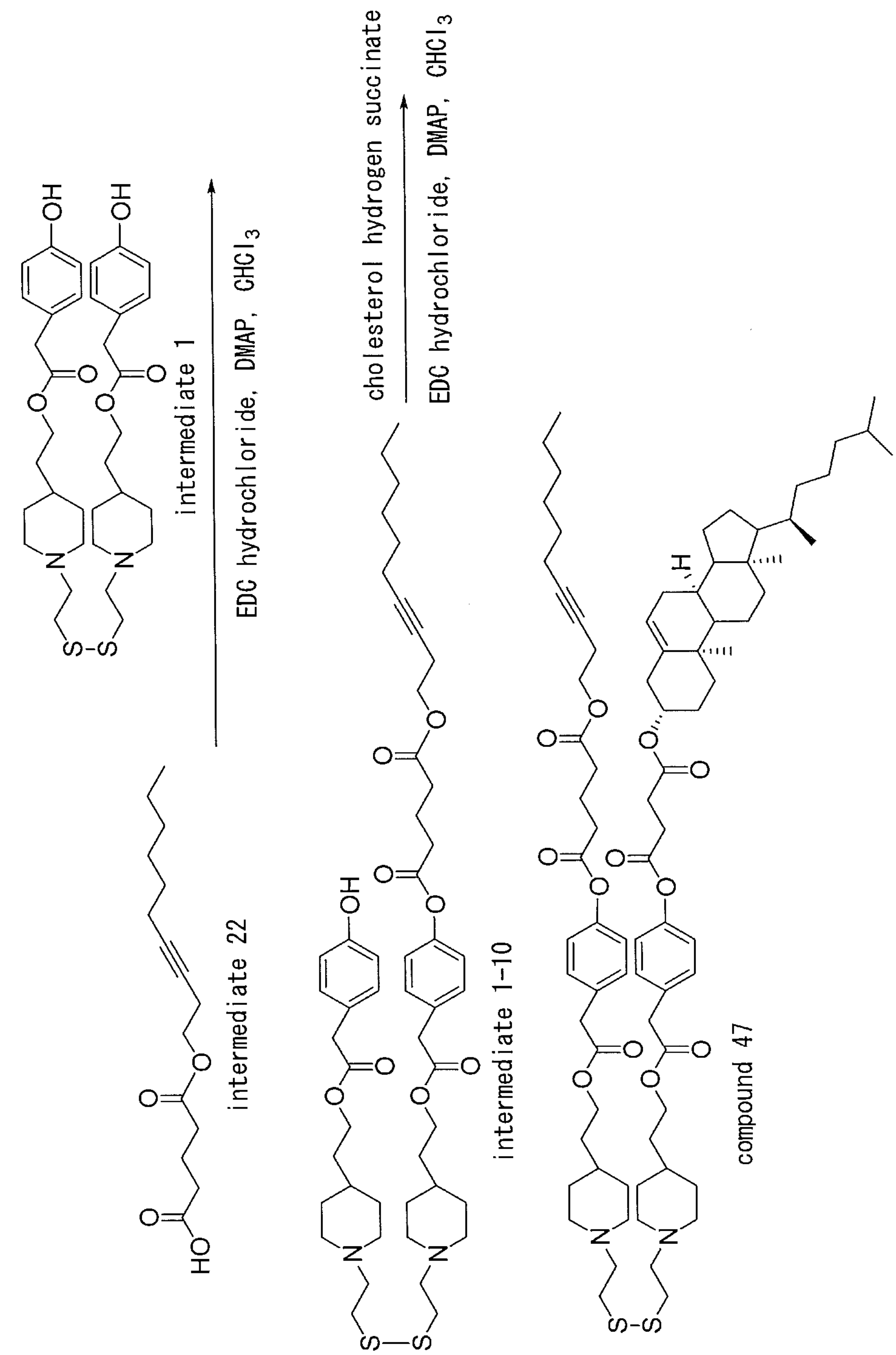


intermediate 1

intermediate 22

intermediate 1-10

compound 47

<Synthesis of intermediate 1-10>

Using intermediate 22 and intermediate 1 and in the same manner as in the synthesis of intermediate 1-1 described in Example 1-1, intermediate 1-10 was synthesized.

<Synthesis of compound 47>

Using intermediate 1-10 and cholesterol hydrogen succinate and in the same manner as in the synthesis of compound 1 described in Example 1-1, compound 47 was synthesized.

<$^1$H-NMR (600 MHz, $CDCl_3$) of compound 47>

5: 0.66-0.70 (m, 3H), 0.80-0.91 (m, 26H), 1.20-1.39 (m, 16H), 1.41-1.62 (m, 18H), 1.78-1.88 (m, 3H), 1.94-2.02 (m, 5H), 2.03-2.10 (m, 2H), 2.11-2.16 (m, 2H), 2.27-2.35 (m, 2H), 2.45-2.52 (m, 4H), 2.56-2.59 (m, 1H), 2.61-2.73 (m, 7H), 2.78-2.96 (m, 9H), 3.59 (s, 4H), 4.09-4.18 (m, 6H), 4.59-4.67 (m, 1H), 5.34-5.39 (m, 1H), 7.02-7.04 (m, 4H), 7.26-7.29 (m, 4H)

[Example 48] Synthesis of compound 48

<Synthesis of intermediate 9-1>

Methyl ricinoleate (5.03 g, 16.0 mmol), cyclohexanecarboxylic acid (2.26 g, 17.6 mmol), and DMAP (389 mg, 3.20 mmol) were dissolved in chloroform (50.3 g) at room temperature. To the obtained mixture was added EDC hydrochloride (4.60 g, 24.0 mmol) and the mixture was reacted at room temperature for 1 hr. The subsequent steps were performed in the same manner as in the synthesis of intermediate 2-1 described in Example 2-1 to obtain 6.31 g of intermediate 9-1.

[Chem. 185]

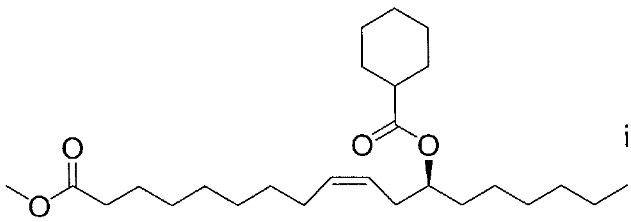

intermediate 9-1

<Synthesis of intermediate 9>

Intermediate 9-1 (6.31 g, 14.9 mmol) was dissolved in tert-butanol to 0.3 M at room temperature. To the obtained mixture was added 2 M NaOH aqueous solution (8.35 g) and the mixture was reacted at room temperature for 8 hr. The subsequent steps were performed in the same manner as in the synthesis of intermediate 2 described in Example 2-1 to obtain 4.95 g of intermediate 9.

[Chem. 186]

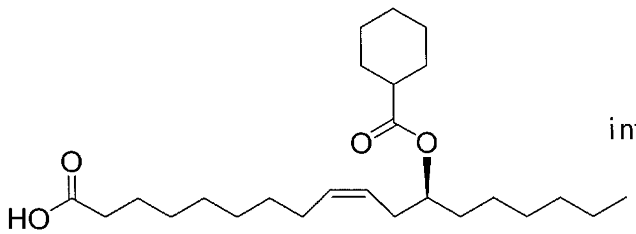

intermediate 9

<Synthesis of compound 48>

Using intermediate 9 and intermediate 1 and in the same manner as in the synthesis of compound 2 described in Example 2-1, compound 48 was synthesized.

[Chem. 187]

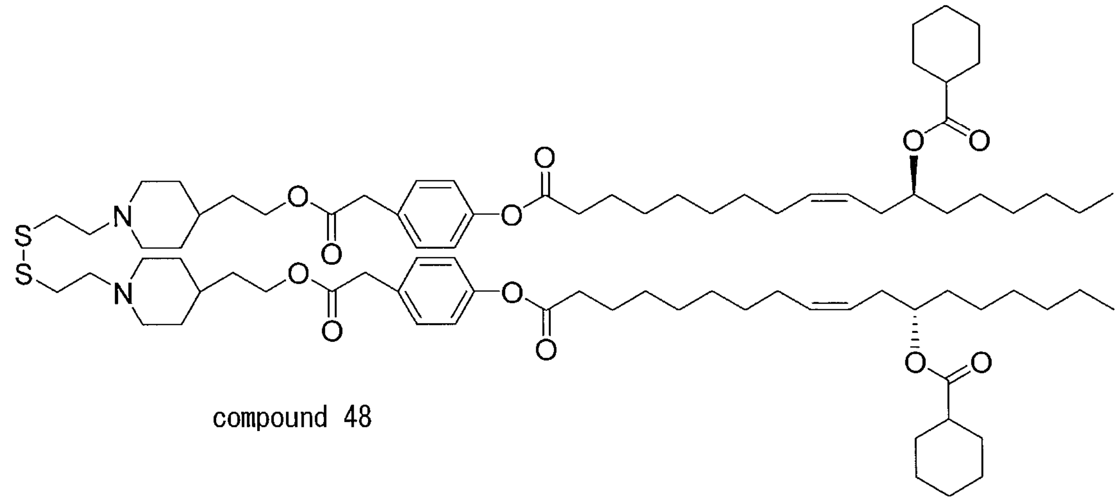

compound 48

<$^1$H-NMR (600 MHz, $CDCl_3$) of compound 48>

δ: 0.84-0.89 (t, 6H), 1.17-1.47 (m, 42H), 1.47-1.60 (m, 18H), 1.61-1.67 (m, 6H), 1.70-1.78 (m, 8H), 1.79-1.92 (m, 4H), 1.93-1.99 (m, 2H), 2.00-2.06 (m, 4H), 2.22-2.33 (m, 4H), 2.52-2.57 (m, 4H), 2.61-2.66 (m, 4H), 2.79-2.84 (m, 4H), 2.85-2.90 (m, 4H), 3.59 (s, 4H), 4.11-4.14 (t, 4H), 4.86-4.88 (m, 2H), 5.29-5.36 (m, 2H), 5.43-5.48 (m, 2H), 7.02-7.04 (m, 4H), 7.26-7.29 (m, 4H)

[Example 49] Synthesis of compound 49

<Synthesis of intermediate 19>

Intermediate 19 was synthesized by the following reaction.

cis-2-Nonen-1-ol (1.09 g, 7.68 mmol), 2-buten-1-ylsuccinic anhydride (1.30 g, 8.44 mmol), and DMAP (188 mg, 1.54 mmol) were dissolved in chloroform (14.8 g) at room temperature, and the mixture was reacted at room temperature for 1 hr. Thereafter, the reaction solution was washed with 5 wt% sodium dihydrogen phosphate water, and 20 wt% saline, and dehydrated by adding sodium sulfate. Sodium sulfate was removed by filtration, and the filtrate was concentrated by an evaporator to give 2.09 g of intermediate 19.

[Chem. 188]

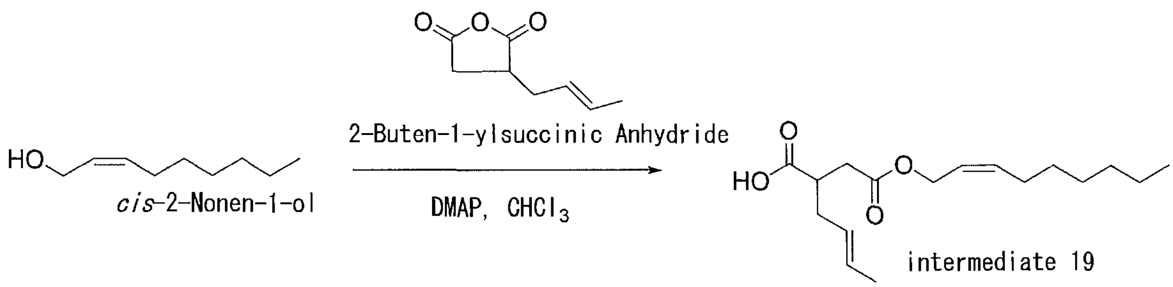


*cis*-2-Nonen-1-ol

intermediate 19

<Synthesis of compound 49>

Using intermediate 19 and intermediate 1-1 and in the same manner as in the synthesis of compound 1 described in Example 1-1, compound 49 was synthesized.

[Chem. 189]

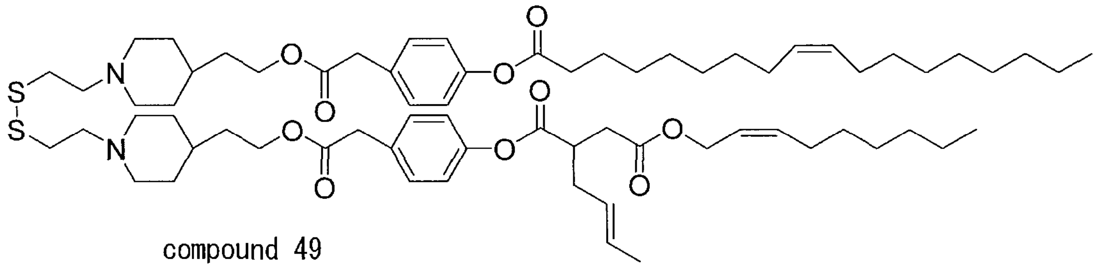

compound 49

<$^1$H-NMR (600 MHz, DMSO-$d_6$) of compound 49>

δ: 0.86-0.89 (m, 6H), 1.22-1.42 (m, 34H), 1.54-1.76 (m, 13H), 1.91-1.98 (m, 4H), 1.99-2.32 (m, 8H), 2.52-2.57 (t, 3H), 2.62-2.66 (m, 4H), 2.78-2.92 (m, 9H), 3.00-3.05 (m, 1H), 3.59 (s, 4H), 4.12 (t, 4H), 4.69 (d, 2H), 5.30-5.38 (m, 2H), 5.41-5.60 (m, 4H), 7.02-7.04 (m, 4H), 7.26-7.29 (m, 4H)

[Example 50] Synthesis of compound 50

<Synthesis of intermediate 1-7>

Using intermediate 15 and intermediate 1 and in the same manner as in the synthesis of intermediate 1-1, intermediate 1-7 was synthesized.

[Chem. 190]

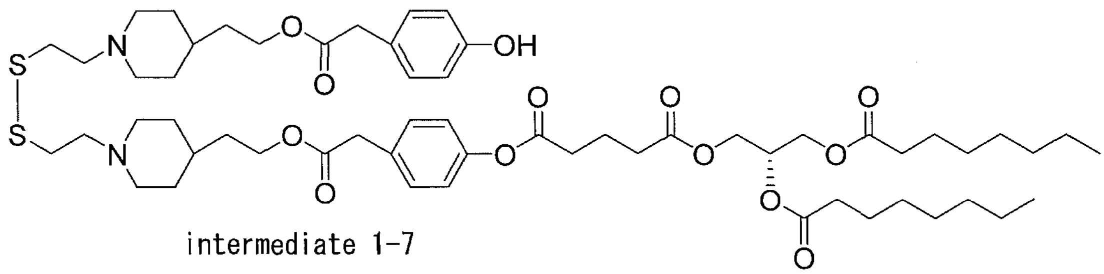

intermediate 1-7

<Synthesis of compound 50>

Using intermediate 19 and intermediate 1-7 and in the same manner as in the synthesis of compound 1 described in Example 1-1, compound 50 was synthesized.

[Chem. 191]

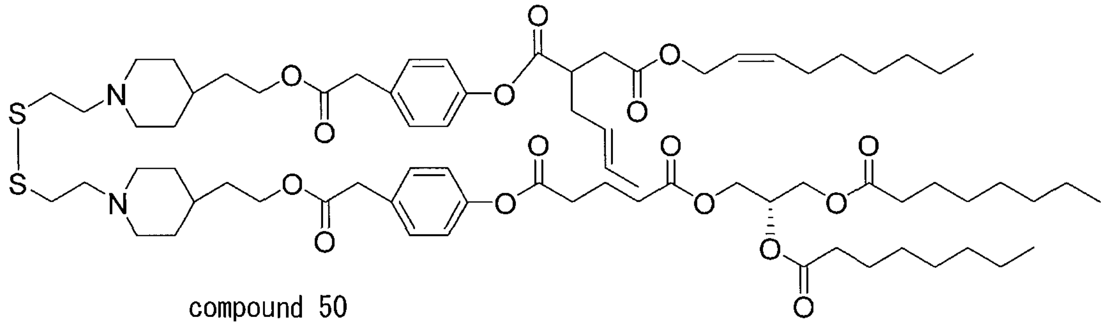

compound 50

<$^1$H-NMR (600 MHz, DMSO-$d_6$) of compound 50>

δ: 0.86-0.89 (m, 9H), 1.22-1.42 (m, 30H), 1.54-1.76 (m, 15H), 1.91-1.98 (m, 4H), 1.99-2.35 (m, 10H), 2.46-2.51 (t, 2H), 2.56 (dd, 1H), 2.61-2.67 (m, 6H), 2.78-2.95 (m, 9H), 3.00-3.05 (m, 1H), 3.59 (s, 4H), 4.09-4.21 (m, 6H), 4.27-4.35 (m, 2H), 4.69 (d, 2H), 5.25-5.30 (m, 1H), 5.41-5.60 (m, 4H), 7.02-7.04 (m, 4H), 7.26-7.29 (m, 4H)

[Example 51] Synthesis of compound 51

<Synthesis of intermediate 1-2>

Using intermediate 4 and intermediate 1 and in the same manner as in the synthesis of intermediate 1-1 described in Example 1-1, intermediate 1-2 was synthesized.

[Chem. 192]

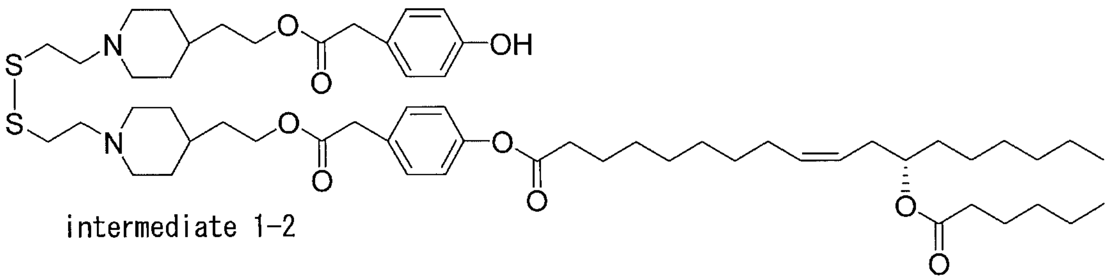

intermediate 1-2

<Synthesis of compound 51>

Using intermediate 19 and intermediate 1-2 and in the same manner as in the synthesis of compound 1 described in Example 1-1, compound 51 was synthesized.

[Chem. 193]

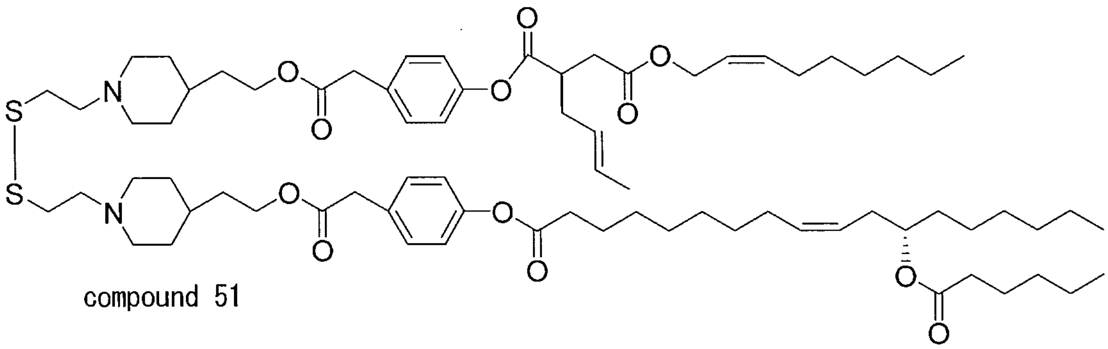

compound 51

<$^1$H-NMR (600 MHz, DMSO-$d_6$) of compound 51>

δ: 0.86-0.90 (m, 9H), 1.22-1.42 (m, 34H), 1.54-1.76 (m, 17H), 1.91-1.98 (m, 4H), 2.00-2.33 (m, 10H), 2.52-2.57 (m, 3H), 2.61-2.67 (m, 4H), 2.79-2.92 (m, 9H), 3.00-3.05 (m, 1H), 3.59 (s, 4H), 4.12 (t, 4H), 4.69 (d, 2H), 4.85-4.90 (m, 1H), 5.31-5.40 (m, 1H), 5.41-5.60 (m, 5H), 7.02-7.04 (m, 4H), 7.26-7.30 (m, 4H)

[Example 52] Synthesis of compound 52

<Synthesis of intermediate 20>

9-Heptadecanol (1.97 g, 7.68 mmol), 2-buten-1-ylsuccinic anhydride (1.30 g, 8.44 mmol), and DMAP (188 mg, 1.54 mmol) were dissolved in chloroform (33.1 g) at room temperature, and the mixture was reacted at room temperature for 1 hr. The subsequent steps were performed in the same manner as in the synthesis of intermediate 19 described in

Example 49 to obtain 2.84 g of intermediate 20.

[Chem. 194]

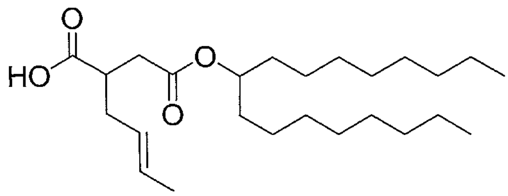

intermediate 20

<Synthesis of compound 52>

Using intermediate 20 and intermediate 1-1 and in the same manner as in the synthesis of compound 1 described in Example 1-1, compound 52 was synthesized.

[Chem. 195]

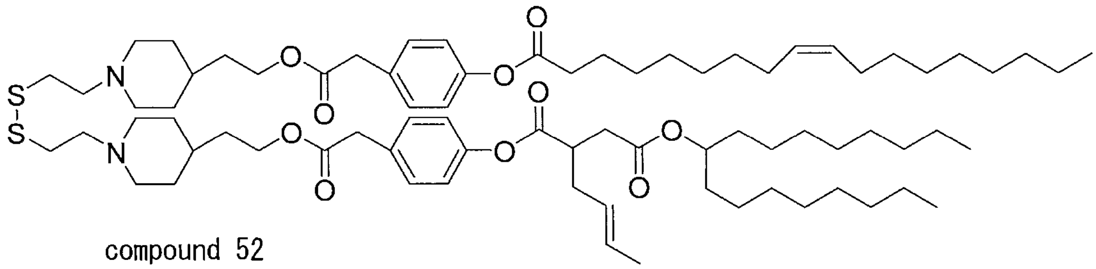

compound 52

<$^1$H-NMR (600 MHz, DMSO-$d_6$) of compound 52>

δ: 0.86-0.89 (m, 9H), 1.22-1.48 (m, 54H), 1.54-1.76 (m, 13H), 1.91-1.98 (m, 4H), 1.99-2.10 (m, 5H), 2.28-2.30 (m, 1H), 2.52-2.56 (m, 3H), 2.62-2.66 (m, 4H), 2.78-2.92 (m, 9H), 3.01-3.05 (m, 1H), 3.59 (s, 4H), 4.11-4.13 (t, 4H), 4.45-4.49 (m, 1H), 5.30-5.38 (m, 2H), 5.41-5.44 (m, 2H), 7.02-7.04 (m, 4H), 7.26-7.29 (m, 4H)

[Example 53] Synthesis of compound 53

Using intermediate 20 and intermediate 1-7 and in the same manner as in the synthesis of compound 1 described in Example 1-1, compound 53 was synthesized.

[Chem. 196]

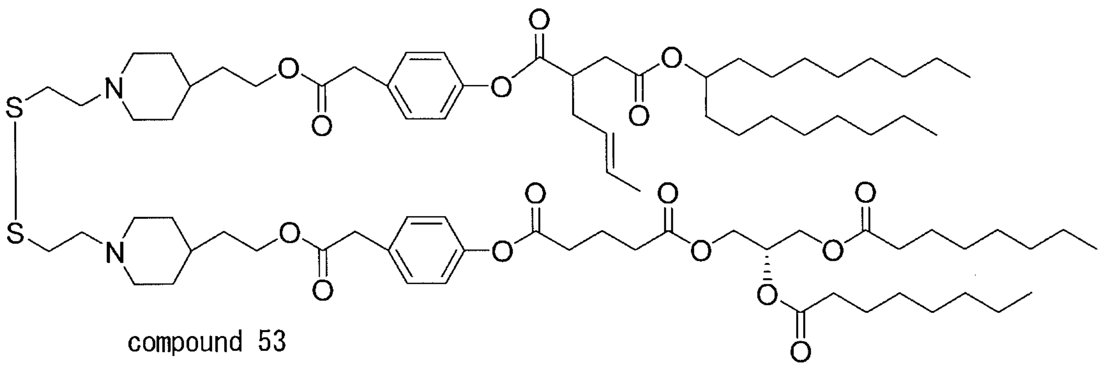

compound 53

<$^1$H-NMR (600 MHz, DMSO-$d_6$) of compound 53>

δ: 0.86-0.89 (m, 12H), 1.22-1.48 (m, 50H), 1.54-1.76 (m, 15H), 1.91-1.98 (m, 4H), 1.99-2.10 (m, 3H), 2.27-2.35

(m, 5H), 2.46-2.51 (t, 2H), 2.56 (dd, 1H), 2.61-2.67 (m, 6H), 2.78-2.95 (m, 9H), 3.01-3.05 (m, 1H), 3.59 (s, 4H), 4.09-4.21 (m, 6H), 4.27-4.35 (m, 2H), 4.45-4.49 (m, 1H), 5.25-5.30 (m, 1H), 5.41-5.44 (m, 2H), 7.02-7.04 (m, 4H), 7.26-7.29 (m, 4H)

[Example 54] Synthesis of compound 54

Using intermediate 20 and intermediate 1-2 and in the same manner as in the synthesis of compound 1 described in Example 1-1, compound 54 was synthesized.

[Chem. 197]

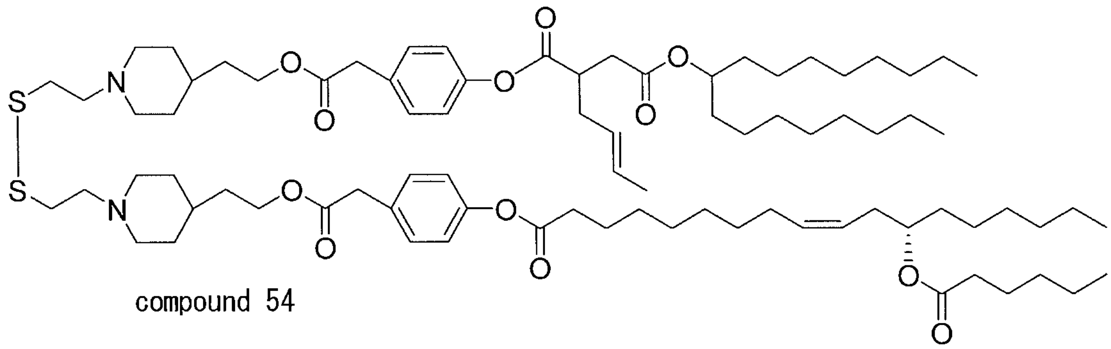

compound 54

<$^1$H-NMR (600 MHz, DMSO-$d_6$) of compound 54>

δ: 0.86-0.90 (m, 12H), 1.22-1.48 (m, 54H), 1.54-1.76 (m, 17H), 1.91-1.98 (m, 4H), 2.00-2.06 (m, 3H), 2.23-2.33 (m, 5H), 2.52-2.56 (m, 3H), 2.61-2.67 (m, 4H), 2.79-2.92 (m, 9H), 3.01-3.05 (m, 1H), 3.59 (s, 4H), 4.11-4.14 (t, 4H), 4.45-4.49 (m, 1H), 4.85-4.90 (m, 1H), 5.31-5.40 (m, 1H), 5.41-5.44 (m, 2H), 5.44-5.49 (m, 1H), 7.02-7.04 (m, 4H), 7.26-7.30 (m, 4H)

[Example 55] Synthesis of compound 55

<Synthesis of intermediate 23>

Intermediate 23 was synthesized by the following synthesis pathway.

[Chem. 198]

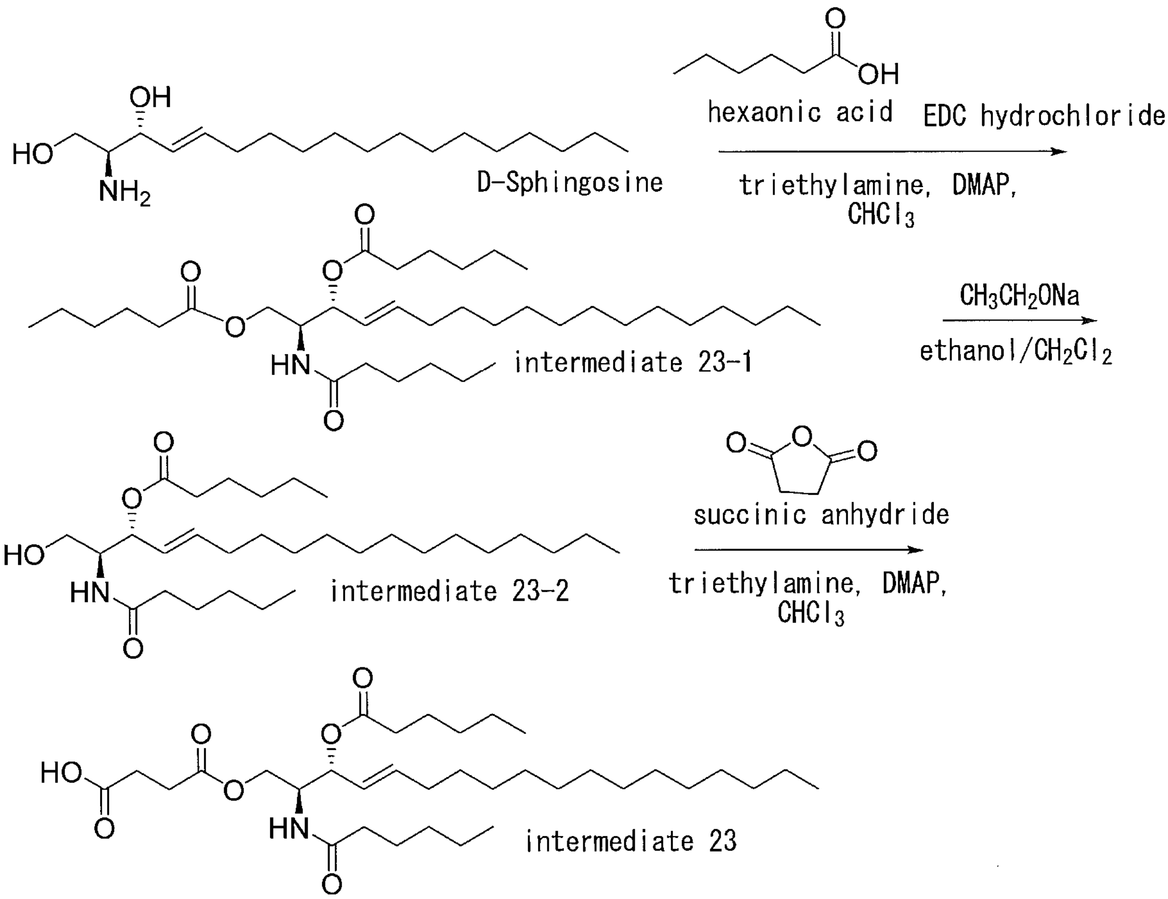


<Synthesis of intermediate 23-1>

D-sphingosine (500 mg, 1.67 mmol) was dissolved in chloroform (5.00 g), triethylamine (507 mg, 5.01 mmol), hexanoic acid (970 mg, 8.35 mmol), DMAP (204 mg, 1.67 mmol), and EDC hydrochloride (1.60 g, 8.35 mmol) were added, and the mixture was reacted at room temperature for 4 hr. The solution after the reaction was washed with 5 wt% saline, concentrated by an evaporator, and subjected to silica gel column purification using ethanol/chloroform after concentration to give 935 mg of intermediate 23-1.

<Synthesis of intermediate 23-2>

To intermediate 23-1 (933 mg, 1.50 mmol) were added ethanol (5.00 g), and dichloromethane (5.00 g), to the obtained solution was added sodium ethoxide (154 mg, 2.25 mmol), and the mixture was reacted at room temperature for 2 hr. The reaction solution was neutralized with 0.1 M hydrochloric acid, and the mixture obtained after neutralization was washed with 10 wt% sodium hydrogen carbonate aqueous solution and saline. Thereafter, it was concentrated by an evaporator, and the concentrate was subjected to silica gel column purification using ethanol/chloroform to give 314 mg of intermediate 23-2.

<Synthesis of intermediate 23>

Intermediate 23-2 (300 mg, 0.605 mmol), succinic anhydride (80.0 mg, 0.800 mmol), triethylamine (184 mg, 1.82 mmol), and DMAP (13.4 mg, 0.110 mmol) were dissolved in chloroform at room temperature, and the mixture was reacted at room temperature for 1 hr. After the reaction, the mixture was washed with 10 wt% sodium hydrogen carbonate aqueous solution, and 0.5 M hydrochloric acid, and concentrated by an evaporator. The concentrate was subjected to silica gel column purification using ethanol/chloroform to give 297 mg of intermediate 23.

<Synthesis of compound 55>

Using intermediate 23 and intermediate 1-1 and in the same manner as in the synthesis of compound 1 described

in Example 1-1, compound 55 was synthesized.

[Chem. 199]

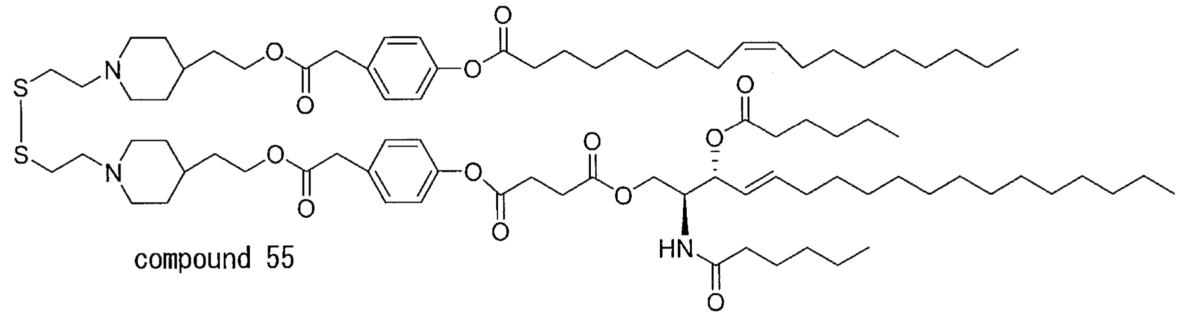

compound 55

<$^1$H-NMR (600 MHz, DMSO-$d_6$) of compound 55>

δ: 0.86-0.89 (m, 12H), 1.22-1.42 (m, 56H), 1.54-1.76 (m, 14H), 1.91-1.98 (m, 6H), 1.99-2.10 (m, 6H), 2.35 (t, 2H), 2.52-2.64 (t, 6H), 2.62-2.66 (m, 4H), 2.78-2.92 (m, 8H), 3.59 (s, 4H), 4.11-4.17 (m, 5H), 4.41 (dd, 1H), 4.81 (quint, 2H), 5.30-5.38 (m, 2H), 5.59-5.62 (m, 2H), 5.85-5.89 (m, 1H), 7.02-7.04 (m, 4H), 7.26-7.29 (m, 4H)

[Example 56] Synthesis of compound 56

Using intermediate 23 and intermediate 1-7 and in the same manner as in the synthesis of compound 1 described in Example 1-1, compound 56 was synthesized.

[Chem. 200]

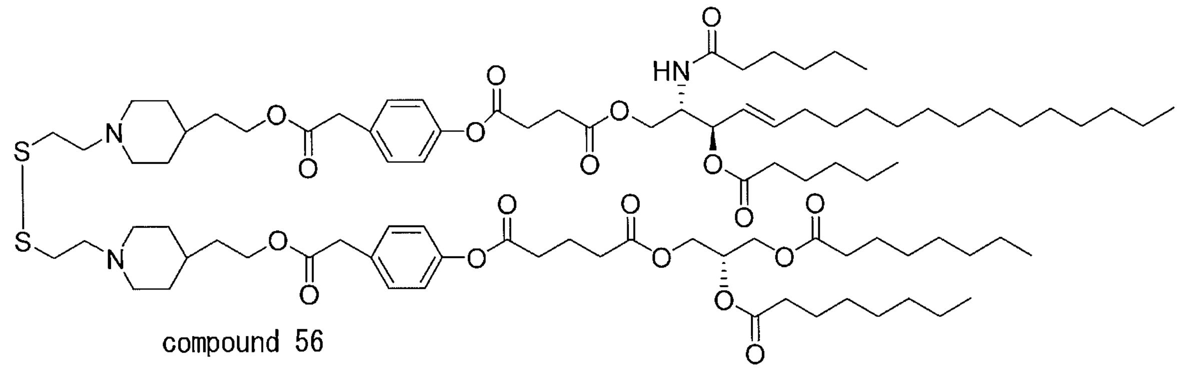

compound 56

<$^1$H-NMR (600 MHz, DMSO-$d_6$) of compound 56>

δ: 0.86-0.89 (m, 15H), 1.22-1.42 (m, 52H), 1.54-1.76 (m, 16H), 1.91-1.98 (m, 6H), 1.99-2.10 (m, 4H), 2.27-2.40 (m, 6H), 2.46-2.51 (t, 2H), 2.58-2.67 (m, 10H), 2.78-2.95 (m, 8H), 3.59 (s, 4H), 4.09-4.21 (m, 7H), 4.27-4.42 (m, 3H), 4.81 (quint, 2H), 5.25-5.30 (m, 1H), 5.59-5.62 (m, 2H), 5.85-5.89 (m, 1H), 7.02-7.04 (m, 4H), 7.26-7.29 (m, 4H)

[Example 57] Synthesis of compound 57

Using intermediate 23 and intermediate 1-2 and in the same manner as in the synthesis of compound 1 described in Example 1-1, compound 57 was synthesized.

[Chem. 201]

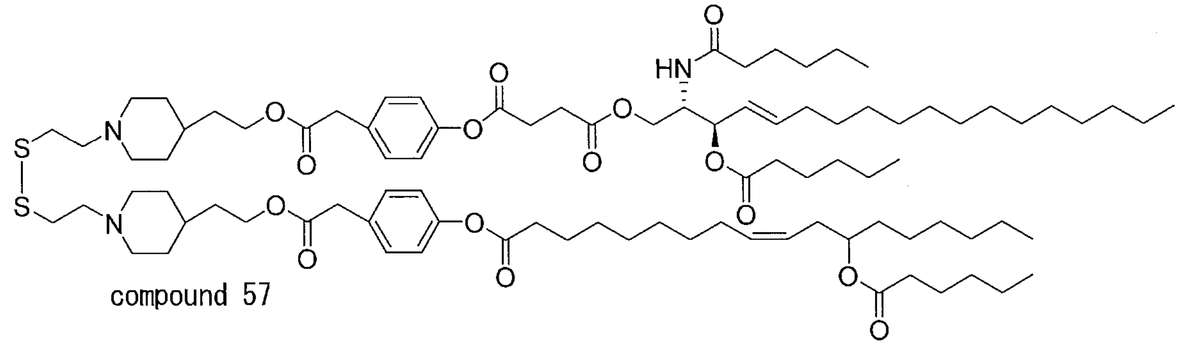

compound 57

<$^1$H-NMR (600 MHz, DMSO-$d_6$) of compound 57>

δ: 0.86-0.90 (m, 15H), 1.22-1.42 (m, 56H), 1.51-1.76 (m, 18H), 1.91-1.98 (m, 6H), 2.00-2.06 (m, 4H), 2.23-2.37 (m, 6H), 2.52-2.67 (m, 10H), 2.79-2.92 (m, 8H), 3.59 (s, 4H), 4.11-4.18 (m, 5H), 4.41 (dd, 1H), 4.79-4.90 (m, 2H), 5.31-5.40 (m, 1H), 5.44-5.49 (m, 1H), 5.59-5.62 (m, 2H), 5.85-5.89 (m, 1H), 7.02-7.04 (m, 4H), 7.26-7.30 (m, 4H)

[Example 58] Synthesis of compound 58

<Synthesis of compound 24>

Intermediate 24 was synthesized by the following synthesis pathway.

[Chem. 202]

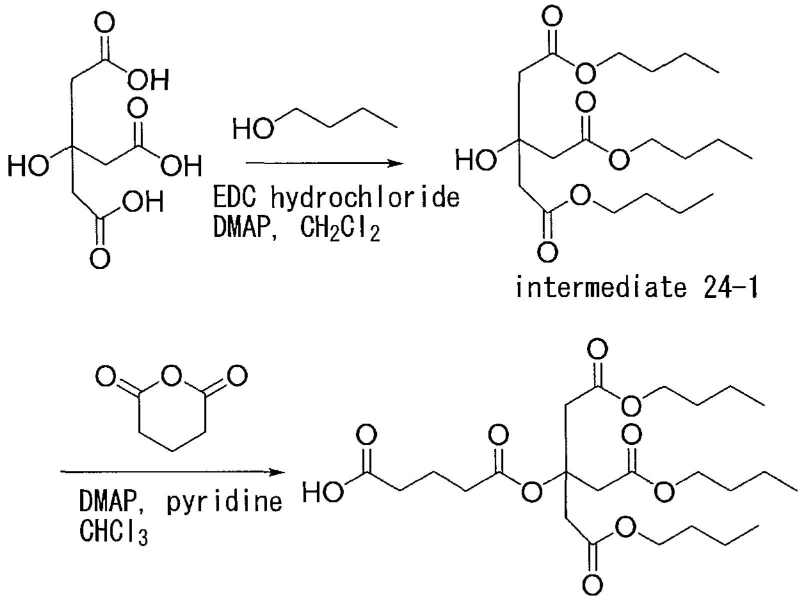


intermediate 24-1

intermediate 24

<Synthesis of compound 24-1>

Citric acid (1.48 g, 7.68 mmol), 1-butanol (3.76 g, 50.7 mmol), and DMAP (938 mg, 7.68 mmol) were dissolved in dichloromethane (23.7 g) at room temperature. To the obtained mixture was added EDC hydrochloride (9.72 g, 50.7 mmol) and the mixture was reacted at room temperature for 1 hr. The subsequent steps were performed in the same manner as in the synthesis of intermediate 1-1 described in Example 1-1 to give 2.78 g of intermediate 24-1.

<Synthesis of compound 24>

Intermediate 24-1 (2.78 g, 7.42 mmol), glutaric anhydride (1.69 g, 14.8 mmol), DMAP (906 mg, 7.42 mmol), and pyridine (2.35 g, 29.7 mmol) were dissolved in chloroform (41.7 g) at room temperature, and the mixture was reacted at room temperature for 9 hr. The subsequent steps were performed in the same manner as in the synthesis of intermediate 11 described in Example 15 to obtain 1.54 g of intermediate 24.

<Synthesis of compound 58>

Using intermediate 24 and intermediate 1-1 and in the same manner as in the synthesis of compound 1 described in Example 1-1, compound 58 was synthesized.

[Chem. 203]

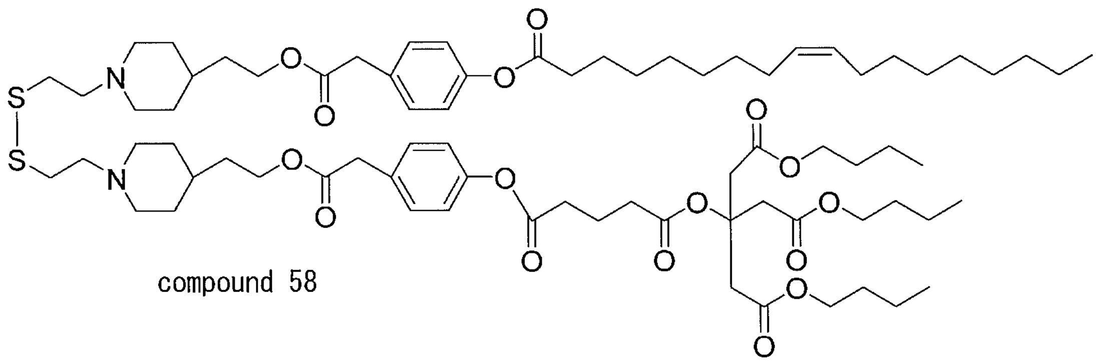

compound 58

<$^1$H-NMR (600 MHz, DMSO-$d_6$) of compound 58>

δ: 0.86-0.89 (m, 12H), 1.22-1.76 (m, 48H), 1.91-1.98 (m, 4H), 1.99-2.16 (m, 6H), 2.28-2.38 (m, 4H), 2.41 (s, 6H), 2.52-2.56 (t, 2H), 2.62-2.66 (m, 4H), 2.78-2.92 (m, 8H), 3.59 (s, 4H), 4.11-4.15 (t, 10H), 5.30-5.38 (m, 2H), 7.02-7.04 (m, 4H), 7.26-7.29 (m, 4H)

[Example 59] Synthesis of compound 59

Using intermediate 24 and intermediate 1-7 and in the same manner as in the synthesis of compound 1 described in Example 1-1, compound 59 was synthesized.

[Chem. 204]

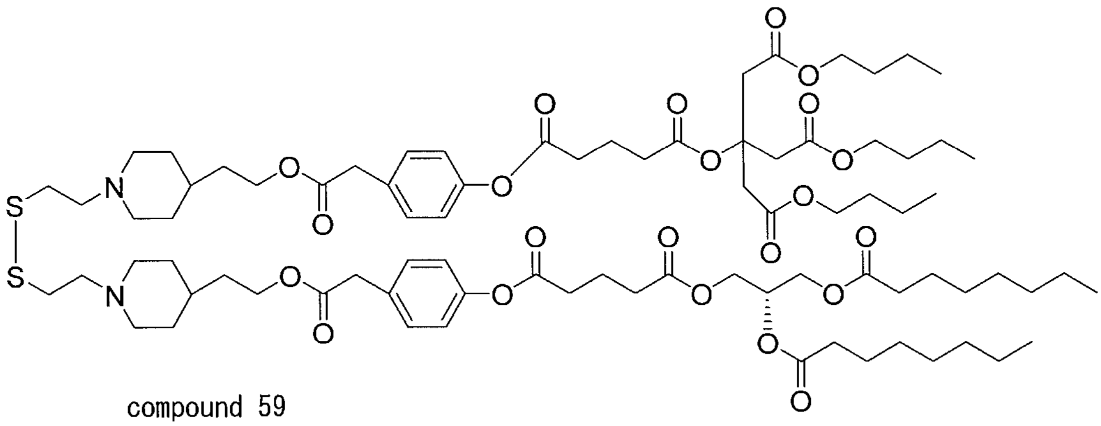

compound 59

<$^1$H-NMR (600 MHz, DMSO-$d_6$) of compound 59>

δ: 0.86-0.92 (m, 15H), 1.22-1.76 (m, 46H), 1.91-1.98 (m, 4H), 1.99-2.16 (m, 4H), 2.27-2.38 (m, 8H), 2.41 (s, 6H), 2.46-2.51 (t, 2H), 2.61-2.67 (m, 6H), 2.78-2.95 (m, 8H), 3.59 (s, 4H), 4.09-4.21 (m, 12H), 4.27-4.35 (m, 2H), 5.25-5.30 (m,

1H), 7.02-7.04 (m, 4H), 7.26-7.29 (m, 4H)

[Example 60] Synthesis of compound 60

Using intermediate 24 and intermediate 1-2 and in the same manner as in the synthesis of compound 1 described in Example 1-1, compound 60 was synthesized.

[Chem. 205]

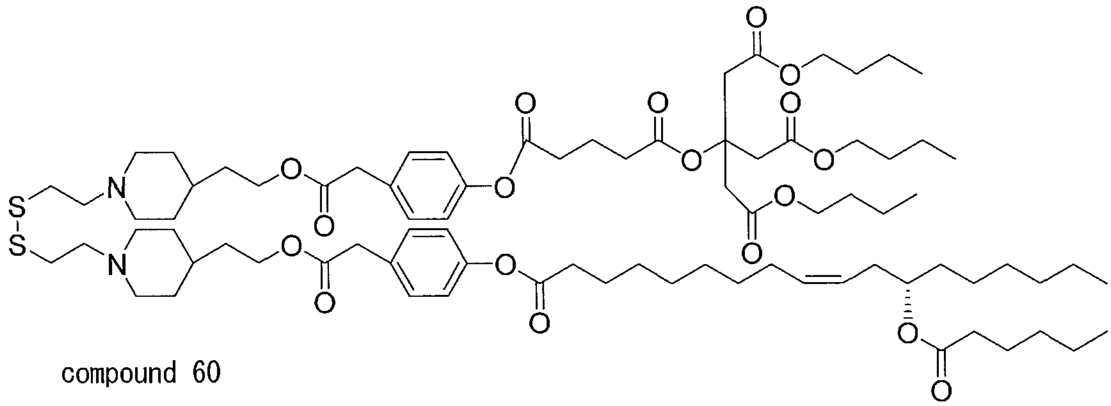

compound 60

<$^1$H-NMR (600 MHz, DMSO-$d_6$) of compound 60>

δ: 0.86-0.92 (m, 15H), 1.22-1.76 (m, 52H), 1.91-1.98 (m, 4H), 2.00-2.16 (m, 4H), 2.23-2.38 (m, 8H), 2.41 (s, 6H), 2.52-2.56 (m, 2H), 2.61-2.67 (m, 4H), 2.79-2.92 (m, 8H), 3.59 (s, 4H), 4.11-4.14 (t, 10H), 4.85-4.90 (m, 1H), 5.31-5.40 (m, 1H), 5.44-5.49 (m, 1H), 7.02-7.04 (m, 4H), 7.26-7.30 (m, 4H)

[Example 61] Synthesis of compound 61

<Synthesis of compound 25-1>

Citric acid (1.48 g, 7.68 mmol), 1-nonanol (7.31 g, 50.7 mmol), and DMAP (938 mg, 7.68 mmol) were dissolved in dichloromethane (23.7 g) at room temperature. To the obtained mixture was added EDC hydrochloride (9.72 g, 50.7 mmol) and the mixture was reacted at room temperature for 1 hr. The subsequent steps were performed in the same manner as in the synthesis of intermediate 1-1 described in Example 1-1 to obtain 4.23 g of intermediate 25-1.

[Chem. 206]

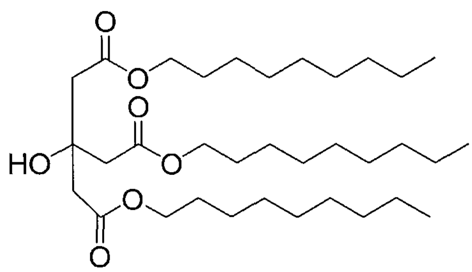

intermediate 25-1

<Synthesis of compound 25>

Intermediate 25-1 (4.23 g, 7.42 mmol), glutaric anhydride (1.69 g, 14.8 mmol), DMAP (906 mg, 7.42 mmol), and pyridine (2.35 g, 29.7 mmol) were dissolved in chloroform (63.5 g) at room temperature, and the mixture was reacted at room temperature for 9 hr. The subsequent steps were performed in the same manner as in the synthesis of intermediate 11 described in Example 15 to obtain 2.20 g of intermediate 25.

[Chem. 207]

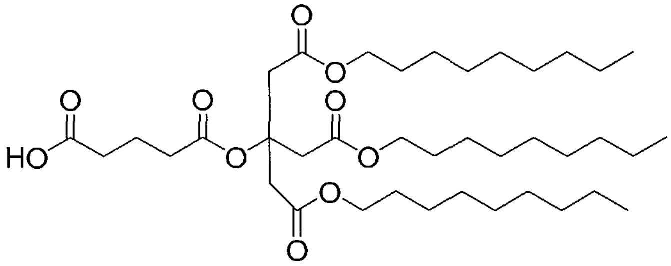

intermediate 25

<Synthesis of compound 61>

Using intermediate 25 and intermediate 1-1 and in the same manner as in the synthesis of compound 1 described in Example 1-1, compound 61 was synthesized.

[Chem. 208]

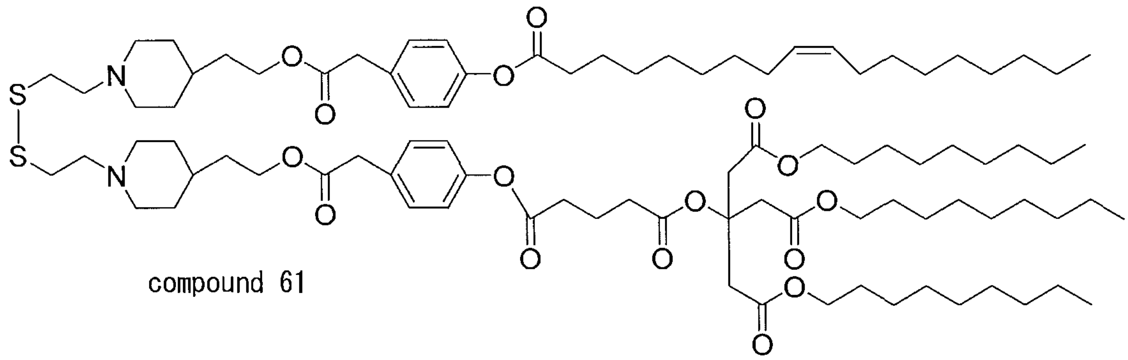

compound 61

<$^1$H-NMR (600 MHz, DMSO-$d_6$) of compound 61>

δ: 0.86-0.92 (m, 12H), 1.22-1.76 (m, 78H), 1.91-1.98 (m, 4H), 1.99-2.16 (m, 6H), 2.28-2.35 (m, 4H), 2.41 (s, 6H), 2.52-2.56 (t, 2H), 2.62-2.66 (m, 4H), 2.78-2.92 (m, 8H), 3.59 (s, 4H), 4.11-4.16 (m, 10H), 5.30-5.38 (m, 2H), 7.02-7.04 (m, 4H), 7.26-7.29 (m, 4H)

[Example 62] Synthesis of compound 62

Using intermediate 25 and intermediate 1-7 and in the same manner as in the synthesis of compound 1 described in Example 1-1, compound 62 was synthesized.

[Chem. 209]

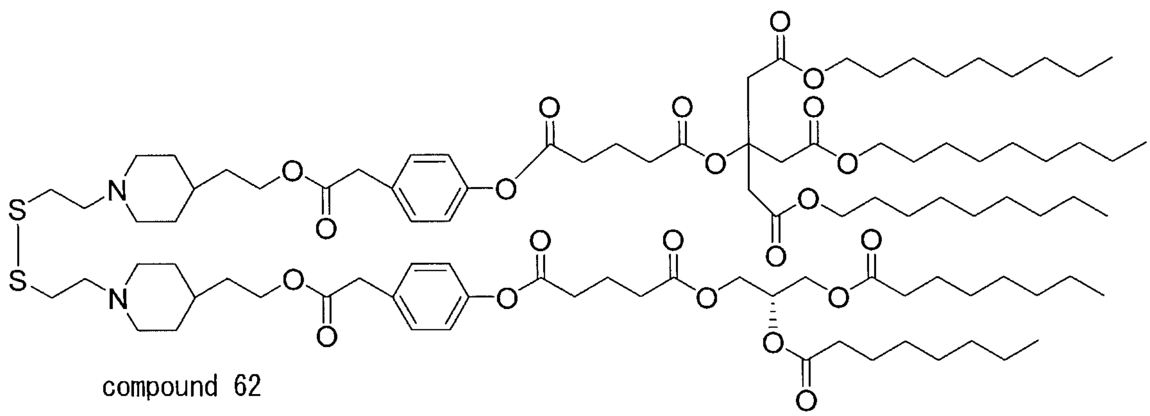

compound 62

<$^1$H-NMR (600 MHz, DMSO-$d_6$) of compound 62>

δ: 0.86-0.89 (m, 15H), 1.22-1.76 (m, 76H), 1.91-1.98 (m, 4H), 1.99-2.16 (m, 4H), 2.27-2.38 (m, 8H), 2.41 (s, 6H), 2.46-2.51 (t, 2H), 2.61-2.67 (m, 6H), 2.78-2.95 (m, 8H), 3.59 (s, 4H), 4.09-4.21 (m, 12H), 4.27-4.35 (m, 2H), 5.25-5.30 (m, 1H), 7.02-7.04 (m, 4H), 7.26-7.29 (m, 4H)

[Example 63] Synthesis of compound 63

Using intermediate 25 and intermediate 1-2 and in the same manner as in the synthesis of compound 1 described in Example 1-1, compound 63 was synthesized.

<$^1$H-NMR (600 MHz, DMSO-$d_6$) of compound 63>

δ: 0.86-0.90 (m, 15H), 1.22-1.76 (m, 82H), 1.91-1.98 (m, 4H), 2.00-2.16 (m, 4H), 2.23-2.38 (m, 8H), 2.41 (s, 6H), 2.52-2.56 (m, 2H), 2.61-2.67 (m, 4H), 2.79-2.92 (m, 8H), 3.59 (s, 4H), 4.11-4.14 (m, 10H), 4.85-4.90 (m, 1H), 5.31-5.40 (m, 1H), 5.44-5.49 (m, 1H), 7.02-7.04 (m, 4H), 7.26-7.30 (m, 4H)

[Chem. 210]

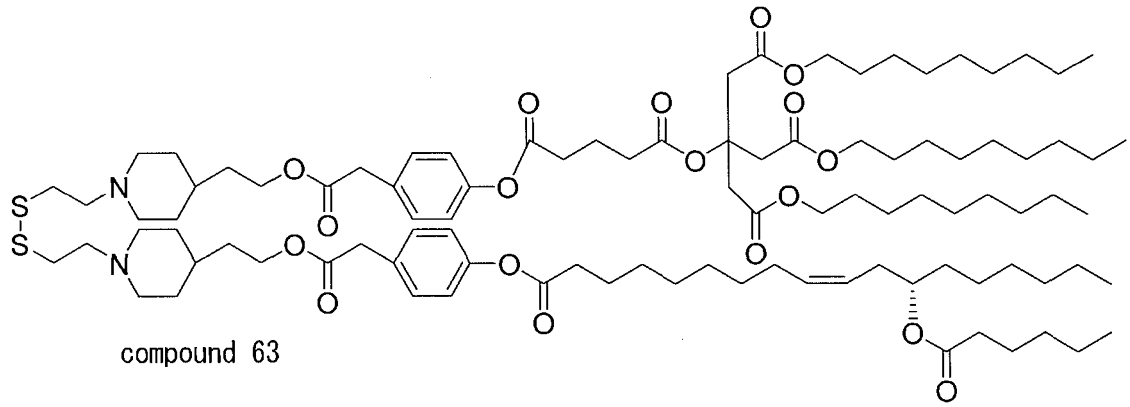

compound 63

[Example 64] Synthesis of compound 64

<Synthesis of intermediate 26>

Intermediate 26 was synthesized by the following synthesis pathway.

[Chem. 211]

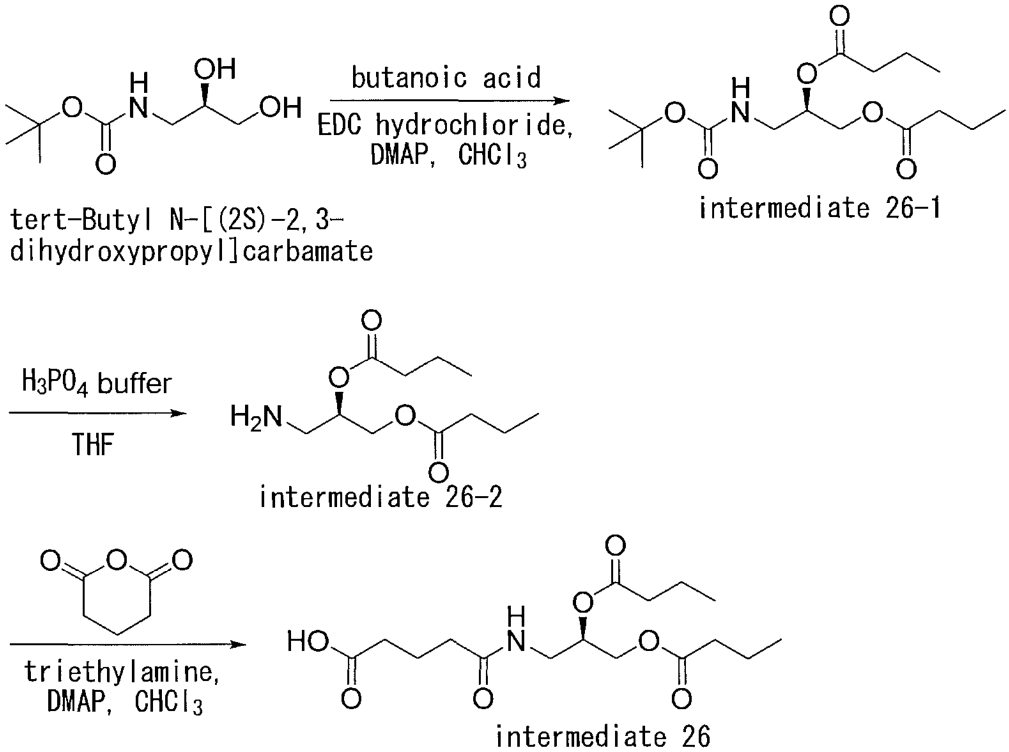


<Synthesis of intermediate 26-1>

tert-Butyl N-[(2S)-2,3-dihydroxypropyl]carbamate (2.00 g, 10.5 mmol), butanoic acid (1.89 g, 21.4 mmol), and DMAP (256 mg, 2.10 mmol) were dissolved in chloroform (20.0 g) at room temperature. To the obtained mixture was added EDC hydrochloride (6.02 g, 31.4 mmol) and the mixture was reacted at room temperature for 1 hr. Thereafter, the reaction solution was washed with 0.5 M phosphate buffer (pH 4.0), 7 wt% sodium bicarbonate water, and 20 wt% saline, and dehydrated by adding sodium sulfate. Sodium sulfate was removed by filtration, and the filtrate was concentrated by an evaporator to give 3.29 g of intermediate 26-1.

<Synthesis of intermediate 26-2>

Intermediate 26-1 (3.29 g, 9.93 mmol), and 0.5 M phosphate buffer (pH 1.0) (4.00 g) were dissolved in THF (74.1 g) at room temperature, and the mixture was reacted at 50°C for 12 hr. 1.0 M NaOH aqueous solution was added to pH 7.0. Chloroform was added and the mixture was washed with 20 wt% saline and dehydrated by adding sodium sulfate. Sodium sulfate was removed by filtration, and the filtrate was concentrated by an evaporator to give 1.38 g of intermediate 26-2.

<Synthesis of intermediate 26>

Intermediate 26-2 (1.38 g, 5.96 mmol), glutaric anhydride (816 mg, 7.15 mmol), triethylamine (905 mg, 8.94 mmol), and DMAP (146 mg, 1.19 mmol) were dissolved in chloroform (13.8 g) at room temperature, and the mixture was reacted at room temperature for 1 hr. The reaction solution was washed with 0.5 M phosphate buffer (pH 4.0), 7 wt% sodium bicarbonate water, and 20 wt% saline, and dehydrated by adding sodium sulfate. Sodium sulfate was removed by filtration, and the filtrate was concentrated by an evaporator to give 1.64 g of intermediate 26-3.

<Synthesis of compound 64>

Using intermediate 26 and intermediate 1-1 and in the same manner as in the synthesis of compound 1 described in Example 1-1, compound 64 was synthesized.

[Chem. 212]

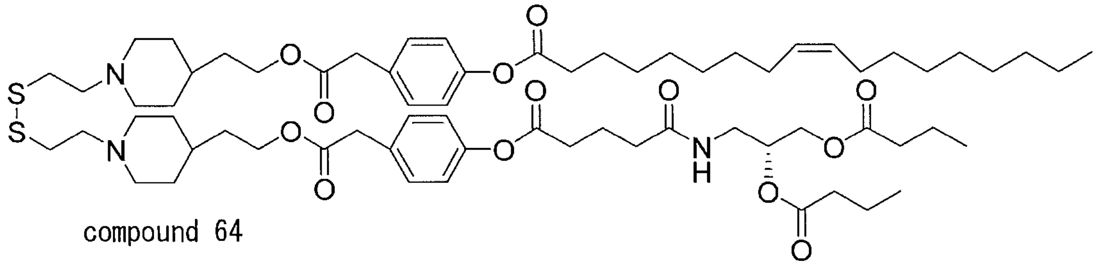

compound 64

<$^1$H-NMR (600 MHz, DMSO-$d_6$) of compound 64>

δ: 0.86-0.89 (m, 3H), 0.99 (t, 6H), 1.22-1.42 (m, 26H), 1.54-1.76 (m, 14H), 1.91-1.98 (m, 4H), 1.99-2.10 (m, 6H), 2.31-2.40 (m, 8H), 2.54 (t, 2H), 2.62-2.66 (m, 4H), 2.78-2.92 (m, 8H), 3.37 (dd, 1H), 3.59 (s, 4H), 3.62 (dd, 1H), 4.11-4.13 (t, 4H), 4.17 (dd, 1H), 4.42 (dd, 1H), 5.30-5.38 (m, 2H), 5.84-5.90 (m, 1H), 7.02-7.04 (m, 4H), 7.26-7.29 (m, 4H), 7.99-8.04 (m, 1H)

[Example 65] Synthesis of compound 65

Using intermediate 26 and intermediate 1-7 and in the same manner as in the synthesis of compound 1 described in Example 1-1, compound 65 was synthesized.

[Chem. 213]

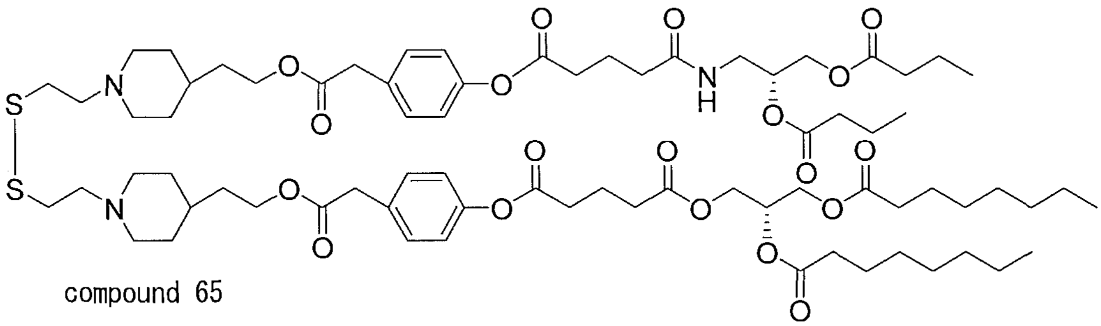

compound 65

<$^1$H-NMR (600 MHz, DMSO-$d_6$) of compound 65>

δ: 0.86-0.89 (m, 6H), 0.99 (t, 6H), 1.22-1.42 (m, 22H), 1.54-1.76 (m, 16H), 1.91-1.98 (m, 4H), 1.99-2.10 (m, 4H), 2.27-2.40 (m, 12H), 2.46-2.51 (t, 2H), 2.61-2.67 (m, 6H), 2.78-2.95 (m, 8H), 3.37 (dd, 1H), 3.59 (s, 4H), 3.62 (dd, 1H), 4.09-4.21 (m, 7H), 4.27-4.35 (m, 2H), 4.42 (dd, 1H), 5.25-5.30 (m, 1H), 5.84-5.90 (m, 1H), 7.02-7.04 (m, 4H), 7.26-7.29 (m, 4H), 7.99-8.04 (m, 1H)

[Example 66] Synthesis of compound 66

Using intermediate 26 and intermediate 1-2 and in the same manner as in the synthesis of compound 1 described in Example 1-1, compound 66 was synthesized.

[Chem. 214]

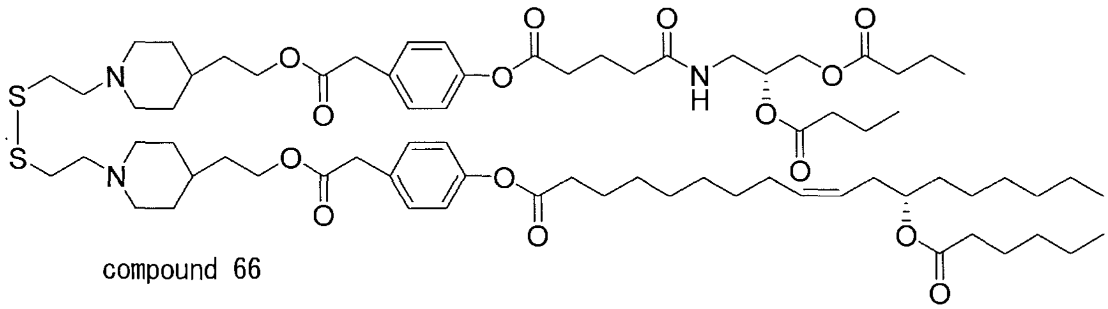

compound 66

<$^1$H-NMR (600 MHz, DMSO-$d_6$) of compound 66>

δ: 0.86-0.90 (m, 6H), 0.99 (t, 6H), 1.22-1.42 (m, 26H), 1.54-1.76 (m, 18H), 1.91-1.98 (m, 4H), 2.00-2.06 (m, 4H), 2.23-2.40 (m, 12H), 2.52-2.56 (m, 2H), 2.61-2.67 (m, 4H), 2.79-2.92 (m, 8H), 3.37 (dd, 1H), 3.59 (s, 4H)), 3.62 (dd, 1H), 4.11-4.19 (m, 5H), 4.42 (dd, 1H), 4.85-4.90 (m, 1H), 5.31-5.40 (m, 1H), 5.44-5.49 (m, 1H), 5.84-5.90 (m, 1H), 7.02-7.04 (m, 4H), 7.26-7.30 (m, 4H), 7.99-8.04 (m, 1H)

[Example 67] Synthesis of compound 67

<Synthesis of intermediate 27-1>

tert-Butyl N-[(2S)-2,3-dihydroxypropyl]carbamate (5.00 g, 26.1 mmol), octanoic acid (8.28 g, 57.4 mmol), and DMAP (638 mg, 5.22 mmol) were dissolved in chloroform (50.0 g) at room temperature. To the obtained mixture was added EDC hydrochloride (7.51 g, 39.2 mmol) and the mixture was reacted at room temperature for 1 hr. The subsequent steps were performed in the same manner as in the synthesis of intermediate 26-1 described in Example 64 to obtain 9.26 g of intermediate 27-1.

[Chem. 215]

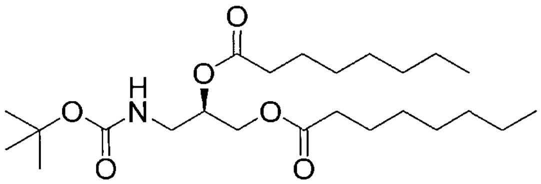

intermediate 27-1

<Synthesis of intermediate 27-2>

Intermediate 27-1 (9.26 g, 20.9 mmol), and 0.5 M phosphate buffer (pH 1.0) (176 g) were dissolved in THF (92.6 g) at room temperature, and the mixture was reacted at 50°C for 12 hr. 1.0 M NaOH aqueous solution was added to pH 7.0. The subsequent steps were performed in the same manner as in the synthesis of intermediate 26-2 described in Example 64 to obtain 4.31 g of intermediate 27-2.

[Chem. 216]

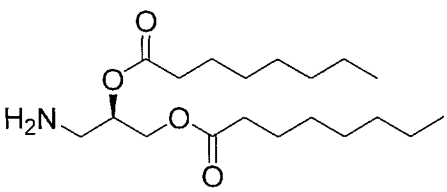

intermediate 27-2

<Synthesis of intermediate 27>

Intermediate 27 (4.31 g, 12.5 mmol), glutaric anhydride (2.85 g, 25.0 mmol), triethylamine (3.79 g, 37.5 mmol), and DMAP (305 mg, 2.50 mmol) were dissolved in chloroform (43.1 g) at room temperature, and the mixture was reacted at

room temperature for 1 hr. The subsequent steps were performed in the same manner as in the synthesis of intermediate 26 described in Example 64 to obtain 4.58 g of intermediate 27.

[Chem. 217]

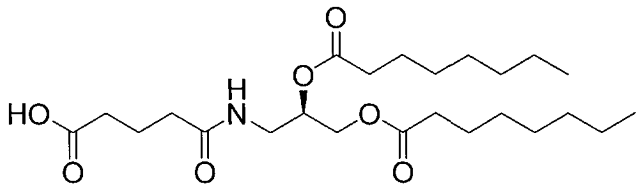

intermediate 27

<Synthesis of compound 67>

Using intermediate 27 and intermediate 1-1 and in the same manner as in the synthesis of compound 1 described in Example 1-1, compound 67 was synthesized.

[Chem. 218]

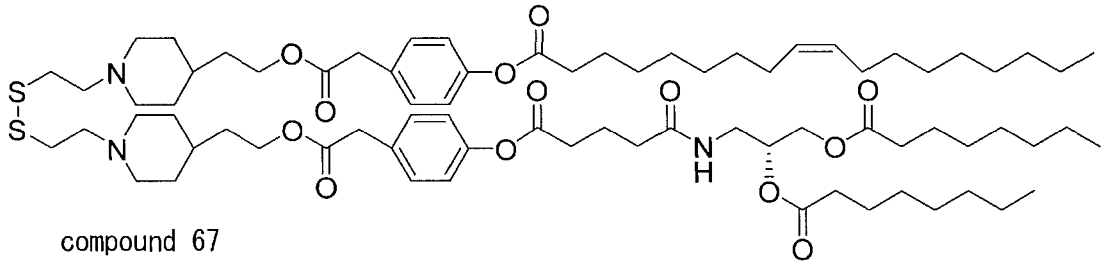

compound 67

<$^1$H-NMR (600 MHz, DMSO-$d_6$) of compound 67>

δ: 0.84-0.92 (m, 9H), 1.22-1.42 (m, 42H), 1.54-1.76 (m, 14H), 1.91-2.10 (m, 10H), 2.31-2.40 (m, 8H), 2.52-2.56 (t, 2H), 2.62-2.66 (m, 4H), 2.78-2.92 (m, 8H), 3.37 (dd, 1H), 3.59 (s, 4H), 3.62 (dd, 1H), 4.12 (t, 4H), 4.17 (dd, 1H), 4.42 (dd, 1H), 5.30-5.38 (m, 2H), 5.84-5.90 (m, 1H), 7.02-7.04 (m, 4H), 7.26-7.29 (m, 4H), 7.99-8.04 (m, 1H)

[Example 68] Synthesis of compound 68

Using intermediate 27 and intermediate 1-7 and in the same manner as in the synthesis of compound 1 described in Example 1-1, compound 68 was synthesized.

[Chem. 219]

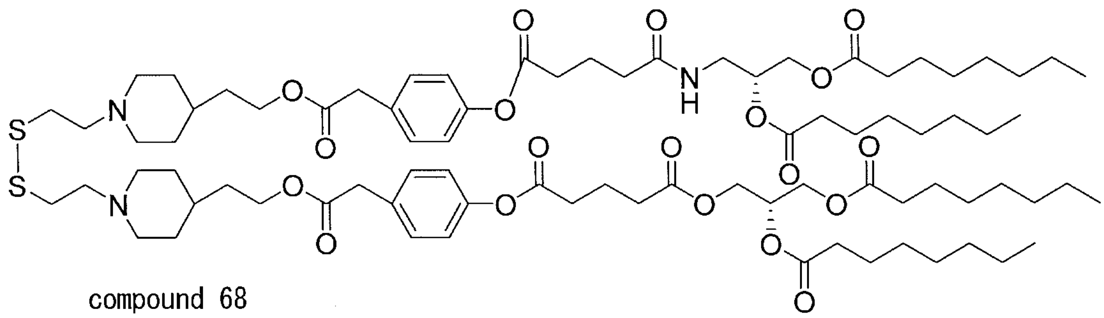

compound 68

<$^1$H-NMR (600 MHz, DMSO-$d_6$) of compound 68>

δ: 0.86-0.92 (m, 12H), 1.22-1.42 (m, 38H), 1.54-1.76 (m, 16H), 1.91-2.10 (m, 8H), 2.27-2.40 (m, 12H), 2.46-2.51 (t, 2H), 2.61-2.67 (m, 6H), 2.78-2.95 (m, 8H), 3.37 (dd, 1H), 3.59 (s, 4H), 3.62 (dd, 1H), 4.09-4.21 (m, 7H), 4.27-4.44 (m, 3H), 5.25-5.30 (m, 1H), 5.84-5.92 (m, 1H), 7.02-7.04 (m, 4H), 7.26-7.29 (m, 4H), 7.98-8.02 (m, 1H)

[Example 69] Synthesis of compound 69

Using intermediate 27 and intermediate 1-2 and in the same manner as in the synthesis of compound 1 described in Example 1-1, compound 69 was synthesized.

[Chem. 220]

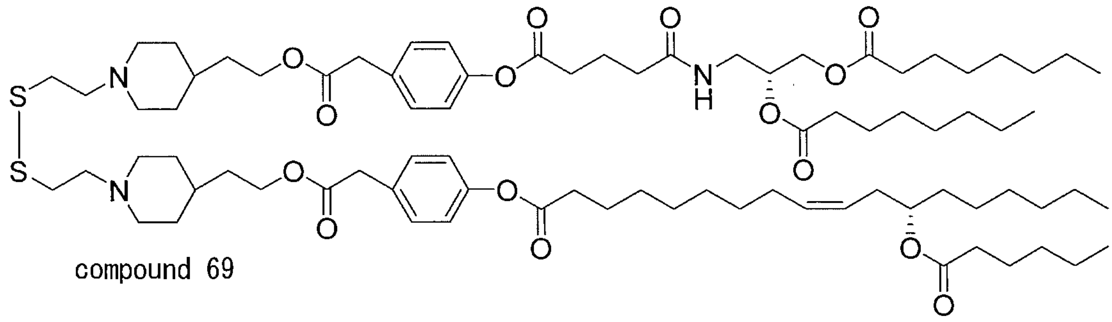

compound 69

<$^1$H-NMR (600 MHz, DMSO-$d_6$) of compound 69>

δ: 0.86-0.92 (m, 12H), 1.22-1.42 (m, 42H), 1.54-1.76 (m, 18H), 1.91-2.06 (m, 8H), 2.23-2.40 (m, 12H), 2.52-2.56 (m, 2H), 2.61-2.67 (m, 4H), 2.79-2.92 (m, 8H), 3.37 (dd, 1H), 3.59 (s, 4H), 3.62 (dd, 1H), 4.11-4.19 (m, 5H), 4.42 (dd, 1H), 4.85-4.90 (m, 1H), 5.31-5.40 (m, 1H), 5.44-5.49 (m, 1H), 5.84-5.92 (m, 1H), 7.02-7.04 (m, 4H), 7.26-7.30 (m, 4H), 7.95-8.04 (m, 1H)

[Example 70] Synthesis of compound 70

<Synthesis of intermediate 28>

Intermediate 11-4 (3.52 g, 20.0 mmol) obtained by the synthesis described in Example 15, maleic acid (4.64 g, 40.0 mmol), triethylamine (6.07 g, 60.0 mmol), and DMAP (0.489 g, 4.00 mmol) were dissolved in chloroform (35.2 g) at room temperature, and the mixture was reacted at room temperature for 1 hr. The reaction solution was washed with 0.5 M phosphate buffer (pH 4.0), 7 wt% sodium bicarbonate water, and 20 wt% saline, and dehydrated by adding sodium sulfate. Sodium sulfate was removed by filtration, and the filtrate was concentrated by an evaporator to give 1.92 g of intermediate 28.

[Chem. 221]

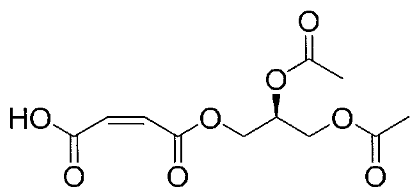

intermediate 28

<Synthesis of compound 70>

Using intermediate 28 and intermediate 1-1 and in the same manner as in the synthesis of compound 1 described in Example 1-1, compound 70 was synthesized.

[Chem. 222]

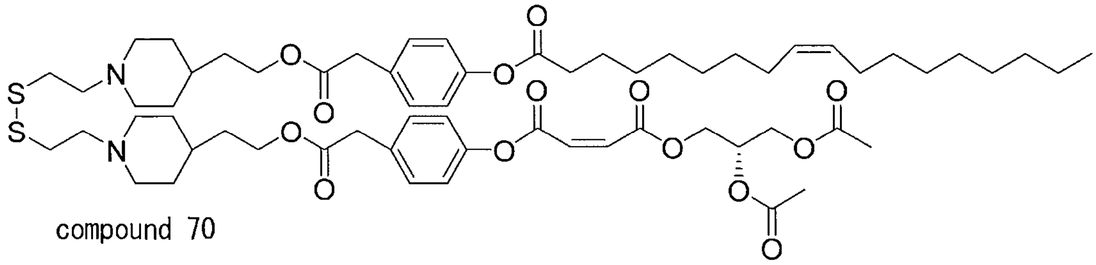

compound 70

<$^1$H-NMR (600 MHz, DMSO-$d_6$) of compound 70>

δ: 0.86-0.89 (m, 3H), 1.22-1.42 (m, 26H), 1.54-1.76 (m, 10H), 1.91-1.98 (m, 4H), 1.99-2.10 (m, 10H), 2.52-2.56 (t, 2H), 2.62-2.66 (m, 4H), 2.78-2.92 (m, 8H), 3.59 (s, 4H), 4.12 (t, 4H), 4.15-4.25 (m, 2H), 4.48-4.52 (m, 2H), 5.30-5.38 (m, 2H), 5.83-5.87 (m, 1H), 6.35 (d, 1H), 6.40 (d, 1H), 7.02-7.04 (m, 4H), 7.26-7.29 (m, 4H)

[Example 71] Synthesis of compound 71

Using intermediate 28 and intermediate 1-7 and in the same manner as in the synthesis of compound 1 described in Example 1-1, compound 71 was synthesized.

[Chem. 223]

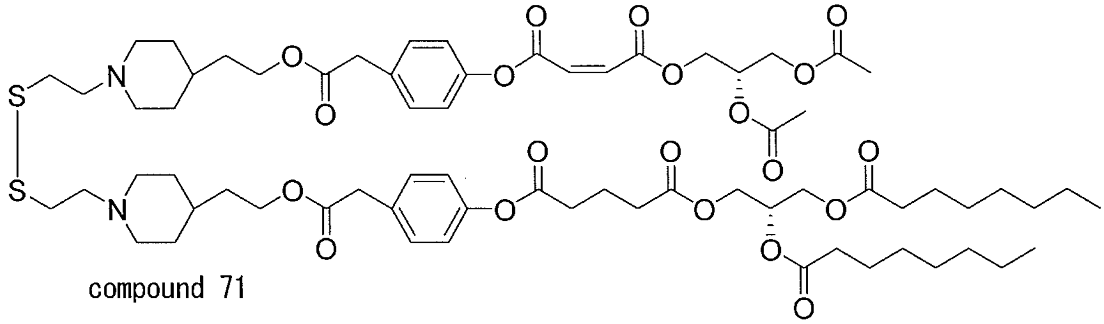

compound 71

<$^1$H-NMR (600 MHz, DMSO-$d_6$) of compound 71>

δ: 0.86-0.89 (m, 6H), 1.22-1.42 (m, 22H), 1.54-1.76 (m, 12H), 1.91-1.98 (m, 4H), 1.99-2.10 (m, 8H), 2.27-2.35 (m, 4H), 2.46-2.51 (t, 2H), 2.61-2.67 (m, 6H), 2.78-2.95 (m, 8H), 3.59 (s, 4H), 4.09-4.25 (m, 8H), 4.27-4.35 (m, 2H), 4.48-4.52 (m, 2H), 5.25-5.30 (m, 1H), 5.83-5.87 (m, 1H), 6.35 (d, 1H), 6.40 (d, 1H), 7.02-7.04 (m, 4H), 7.26-7.29 (m, 4H)

[Example 72] Synthesis of compound 72

Using intermediate 28 and intermediate 1-2 and in the same manner as in the synthesis of compound 1 described in Example 1-1, compound 72 was synthesized.

[Chem. 224]

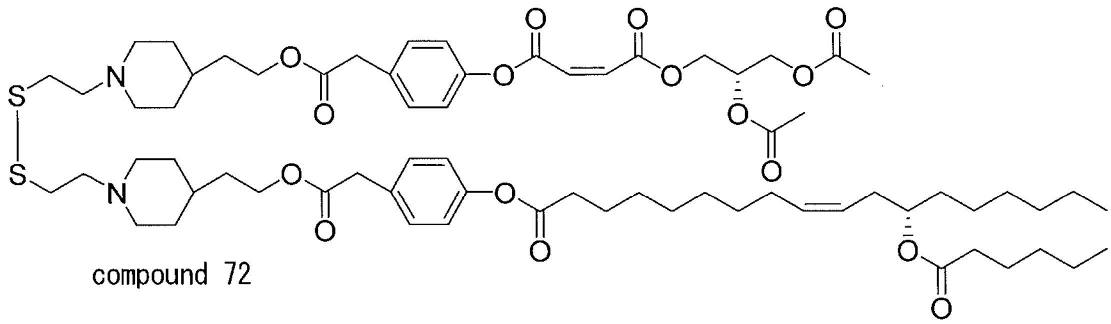

compound 72

<$^1$H-NMR (600 MHz, DMSO-$d_6$) of compound 72>

δ: 0.86-0.90 (m, 6H), 1.22-1.42 (m, 26H), 1.54-1.76 (m, 14H), 1.91-1.98 (m, 4H), 2.00-2.06 (m, 8H), 2.23-2.33 (m, 4H), 2.52-2.56 (m, 2H), 2.61-2.67 (m, 4H), 2.79-2.92 (m, 8H), 3.59 (s, 4H), 4.11-4.25 (m, 6H), 4.48-4.52 (m, 2H), 4.85-4.90 (m, 1H), 5.31-5.40 (m, 1H), 5.44-5.49 (m, 1H), 5.83-5.87 (m, 1H), 6.35 (d, 1H), 6.40 (d, 1H), 7.02-7.04 (m, 4H), 7.26-7.30 (m, 4H)

[Example 73] Synthesis of compound 73

<Synthesis of intermediate 29>

Intermediate 15-2 (1.72 g, 5.00 mmol) obtained by the synthesis described in Example 5, maleic acid (0.696 g, 6.00 mmol), triethylamine (2.02 g, 20.0 mmol), and DMAP (0.305 g, 2.50 mmol) were dissolved in chloroform (17.2 g) at room temperature, and the mixture was reacted at room temperature for 1 hr. The subsequent steps were performed in the same manner as in the synthesis of intermediate 28 described in Example 70 to obtain 1.55 g of intermediate 29.

[Chem. 225]

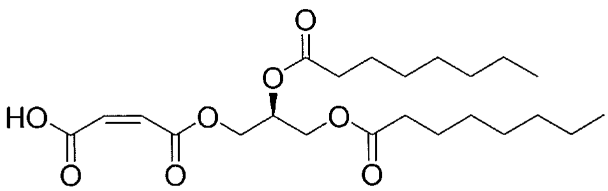

intermediate 29

<Synthesis of compound 73>

Using intermediate 29 and intermediate 1-1 and in the same manner as in the synthesis of compound 1 described in Example 1-1, compound 73 was synthesized.

[Chem. 226]

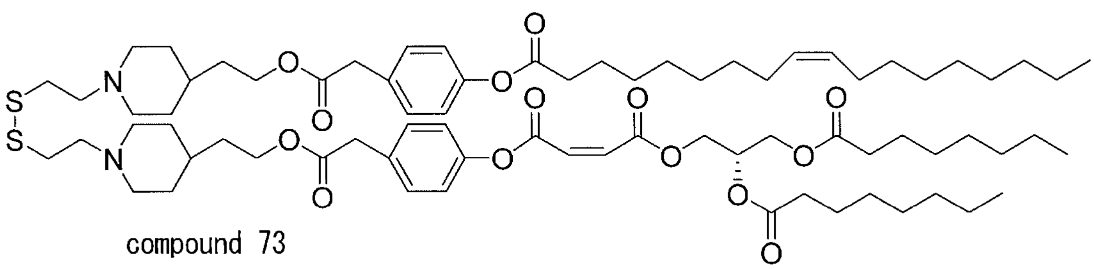

compound 73

<$^1$H-NMR (600 MHz, DMSO-$d_6$) of compound 73>

δ: 0.86-0.89 (m, 9H), 1.22-1.42 (m, 42H), 1.54-1.76 (m, 14H), 1.91-1.98 (m, 4H), 1.99-2.10 (m, 4H), 2.31-2.42 (m, 4H), 2.54 (t, 2H), 2.62-2.66 (m, 4H), 2.78-2.92 (m, 8H), 3.59 (s, 4H), 4.12 (t, 4H), 4.15-4.25 (m, 2H), 4.48-4.52 (m, 2H),

5.30-5.38 (m, 2H), 5.83-5.87 (m, 1H), 6.35 (d, 1H), 6.40 (d, 1H), 7.02-7.04 (m, 4H), 7.26-7.29 (m, 4H)

[Example 74] Synthesis of compound 74

Using intermediate 29 and intermediate 1-7 and in the same manner as in the synthesis of compound 1 described in Example 1-1, compound 74 was synthesized.

[Chem. 227]

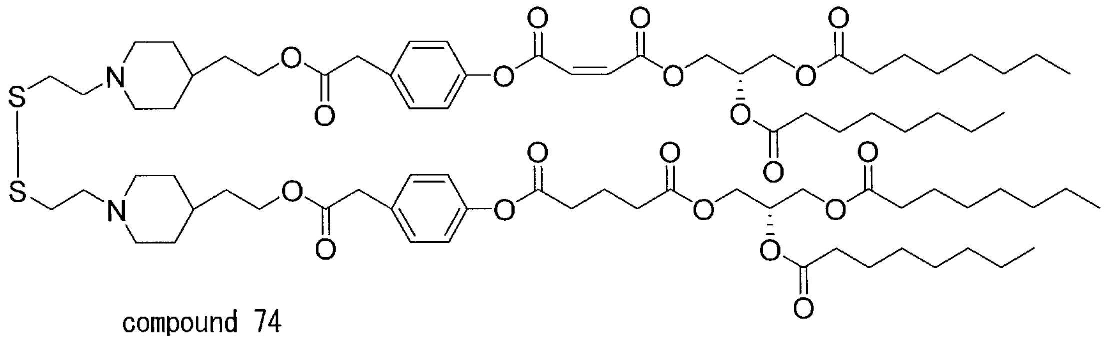

compound 74

<$^1$H-NMR (600 MHz, DMSO-$d_6$) of compound 74>

δ: 0.86-0.89 (m, 12H), 1.22-1.42 (m, 38H), 1.54-1.76 (m, 16H), 1.91-1.98 (m, 4H), 1.99-2.10 (m, 2H), 2.27-2.42 (m, 8H), 2.46-2.51 (t, 2H), 2.61-2.67 (m, 6H), 2.78-2.95 (m, 8H), 3.59 (s, 4H), 4.09-4.21 (m, 8H), 4.27-4.35 (m, 2H), 4.48-4.52 (m, 2H), 5.25-5.30 (m, 1H), 5.83-5.87 (m, 1H), 6.35 (d, 1H), 6.40 (d, 1H), 7.02-7.04 (m, 4H), 7.26-7.29 (m, 4H)

[Example 75] Synthesis of compound 75

Using intermediate 29 and intermediate 1-2 and in the same manner as in the synthesis of compound 1 described in Example 1-1, compound 75 was synthesized.

[Chem. 228]

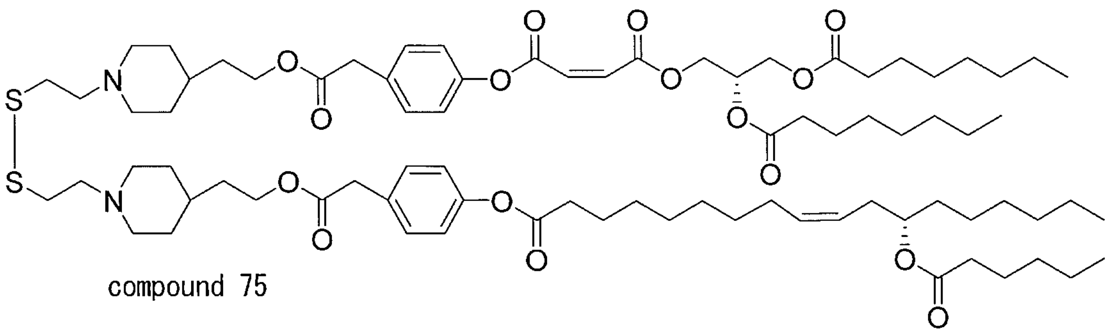

compound 75

<$^1$H-NMR (600 MHz, DMSO-$d_6$) of compound 75>

δ: 0.86-0.90 (m, 12H), 1.22-1.42 (m, 42H), 1.54-1.76 (m, 18H), 1.91-1.98 (m, 4H), 2.00-2.06 (m, 2H), 2.23-2.42 (m, 8H), 2.52-2.56 (m, 2H), 2.61-2.67 (m, 4H), 2.79-2.92 (m, 8H), 3.59 (s, 4H), 4.11-4.25 (m, 6H), 4.48-4.52 (m, 2H), 4.85-4.90 (m, 1H), 5.31-5.40 (m, 1H), 5.44-5.49 (m, 1H), 5.83-5.87 (m, 1H), 6.35 (d, 1H), 6.40 (d, 1H), 7.02-7.04 (m, 4H), 7.26-7.30 (m, 4H)

[Example 76] Synthesis of compound 76

<Synthesis of intermediate 30>

Intermediate 30 was synthesized by the following synthesis pathway.

[Chem. 229]

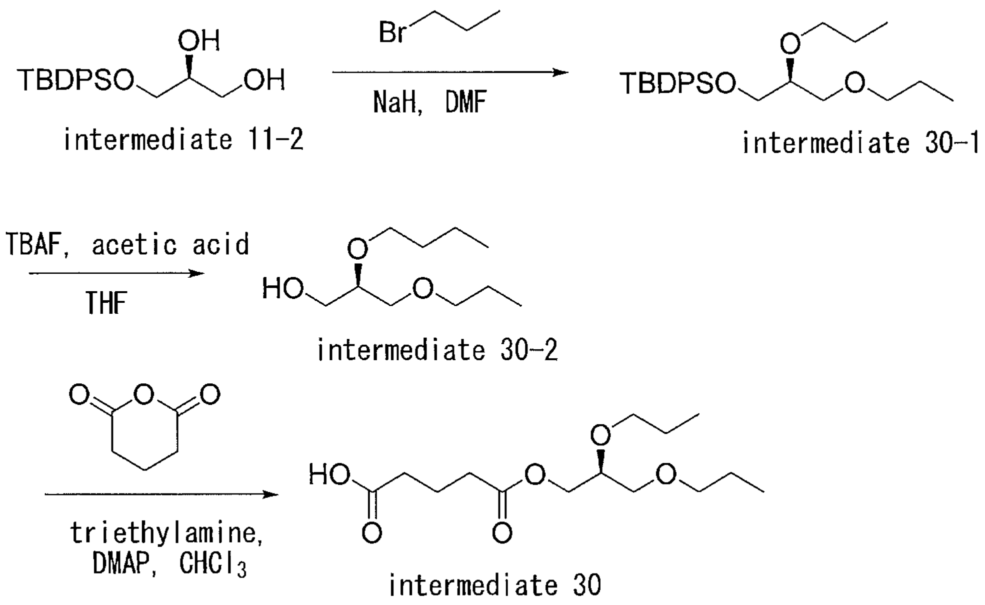


intermediate 11-2

intermediate 30-1

intermediate 30-2

intermediate 30

<Synthesis of intermediate 30-1>

Intermediate 11-2 (2.44 g, 7.37 mmol) obtained by the synthesis described in Example 15, 1-bromopropane (907 mg, 7.37 mmol), sodium hydride (265 mg, 11.1 mmol), and potassium iodide (122 mg, 0.737 mmol) were dissolved in DMF (24.4 g) at room temperature, and the mixture was reacted at 70°C for 16 hr. Thereafter, chloroform was added. The subsequent steps were performed in the same manner as in the synthesis of intermediate 11-1 described in Example 15 to obtain 2.14 g of intermediate 30-1.

<Synthesis of intermediate 30-2>

Intermediate 30-1 (2.14 g, 5.16 mmol), and acetic acid (1.24 g, 20.6 mmol) were dissolved in THF (21.4 g) at room temperature. To the obtained mixture was added 1 M TBAF solution (18.9 g, 20.6 mmol) in THF and the mixture was reacted at room temperature for 18 hr. Thereafter, to the reaction solution was added ethyl acetate (21.4 g). The subsequent steps were performed in the same manner as in the synthesis of intermediate 11-4 described in Example 15 to obtain 951 mg of intermediate 30-2.

<Synthesis of intermediate 30>

Intermediate 30-2 (950 mg, 4.99 mmol), glutaric anhydride (626 mg, 5.49 mmol), triethylamine (1.52 g, 15.0 mmol), and DMAP (122 mg, 0.998 mmol) were dissolved in chloroform (14.3 g) at room temperature, and the mixture was reacted at room temperature for 1 hr. The subsequent steps were performed in the same manner as in the synthesis of intermediate 11 described in Example 15 to obtain 1.10 g of intermediate 30.

<Synthesis of compound 76>

Using intermediate 30 and intermediate 1-1 and in the same manner as in the synthesis of compound 1 described in Example 1-1, compound 76 was synthesized.

[Chem. 230]

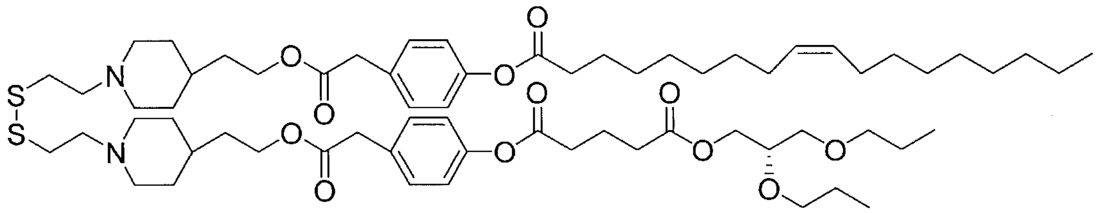

compound 76

<$^{1}$H-NMR (600 MHz, DMSO-$d_6$) of compound 76>

δ: 0.86-0.89 (m, 3H), 0.99 (t, 6H), 1.22-1.42 (m, 26H), 1.54-1.76 (m, 14H), 1.91-1.98 (m, 4H), 1.99-2.10 (m, 4H), 2.21-2.43 (m, 6H), 2.52-2.56 (t, 2H), 2.62-2.66 (m, 4H), 2.78-2.92 (m, 8H), 3.35 (m, 5H), 3.46 (dd, 1H), 3.71 (s, 4H), 4.12 (t, 4H), 4.17 (dd, 1H), 4.42 (dd, 2H), 5.30-5.38 (m, 2H), 7.02-7.04 (m, 4H), 7.26-7.29 (m, 4H)

[Example 77] Synthesis of compound 77

Using intermediate 30 and intermediate 1-7 and under conditions similar to those in Example 1-1, the reaction was performed to synthesize compound 77.

[Chem. 231]

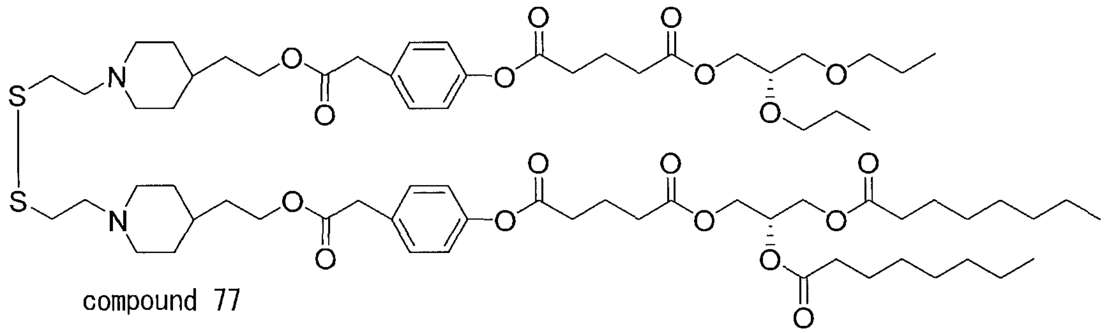

compound 77

<$^{1}$H-NMR (600 MHz, DMSO-$d_6$) of compound 77>

δ: 0.86-0.89 (m, 6H), 0.99 (t, 6H), 1.22-1.42 (m, 22H), 1.54-1.76 (m, 16H), 1.91-1.98 (m, 4H), 1.99-2.10 (m, 2H), 2.21-2.43 (m, 10H), 2.46-2.51 (t, 2H), 2.61-2.67 (m, 6H), 2.78-2.95 (m, 8H), 3.46 (dd, 1H), 3.55-3.74 (m, 9H), 4.09-4.21 (m, 7H), 4.27-4.44 (m, 3H), 5.25-5.30 (m, 1H), 5.40-5.52 (m, 1H), 7.02-7.04 (m, 4H), 7.26-7.29 (m, 4H)

[Example 78] Synthesis of compound 78

Using intermediate 30 and intermediate 1-2 and in the same manner as in the synthesis of compound 1 described in Example 1-1, compound 78 was synthesized.

[Chem. 232]

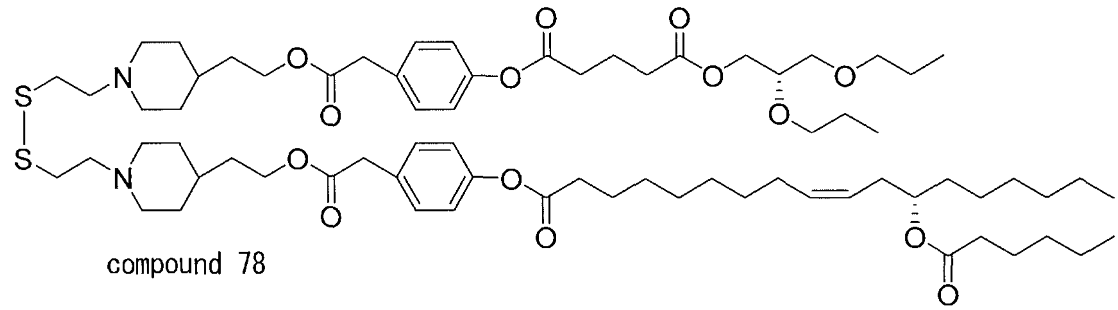

compound 78

<$^1$H-NMR (600 MHz, DMSO-$d_6$) of compound 78>

δ: 0.86-0.90 (m, 6H), 0.99 (t, 6H), 1.22-1.42 (m, 26H), 1.54-1.76 (m, 18H), 1.91-1.98 (m, 4H), 2.00-2.06 (m, 2H), 2.21-2.43 (m, 10H), 2.52-2.56 (m, 2H), 2.61-2.67 (m, 4H), 2.79-2.92 (m, 8H), 3.46 (dd, 1H), 3.55-3.74 (m, 9H), 4.12 (t, 4H), 4.17 (dd, 1H), 4.42 (dd, 1H), 4.85-4.90 (m, 1H), 5.31-5.40 (m, 1H), 5.40-5.52 (m, 2H), 7.02-7.04 (m, 4H), 7.26-7.30 (m, 4H)

[Example 79] Synthesis of compound 79

<Synthesis of intermediate 31-1>

Intermediate 11-2 (2.44 g, 7.37 mmol) obtained by the synthesis described in Example 15, 1-bromopropane (1.41 g, 7.37 mmol), sodium hydride (265 mg, 11.1 mmol), and potassium iodide (122 mg, 0.737 mmol) were dissolved in DMF (24.4 g) at room temperature, and the mixture was reacted at 70°C for 16 hr. Thereafter, chloroform was added. The subsequent steps were performed in the same manner as in the synthesis of intermediate 11-1 described in Example 15 to obtain 2.14 g of intermediate 31-1.

[Chem. 233]

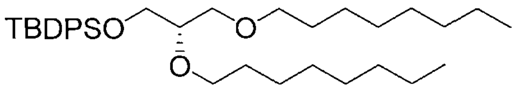

intermediate 31-1

<Synthesis of intermediate 31-2>

Intermediate 31-1 (2.14 g, 3.86 mmol), and acetic acid (0.925 g, 15.4 mmol) were dissolved in THF (21.4 g) at room temperature. To the obtained mixture was added 1 M TBAF solution (14.2 g, 15.4 mmol) in THF and the mixture was reacted at room temperature for 18 hr. Thereafter, to the reaction solution was added ethyl acetate (21.4 g). The subsequent steps were performed in the same manner as in the synthesis of intermediate 11-4 described in Example 15 to obtain 1.04 g of intermediate 31-2.

[Chem. 234]

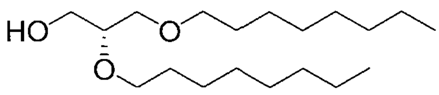

intermediate 31-2

<Synthesis of intermediate 31>

Intermediate 31-2 (1.04 g, 3.29 mmol), glutaric anhydride (413 mg, 3.62 mmol), triethylamine (999 mg, 9.87 mmol), and DMAP (80.4 mg, 0.658 mmol) were dissolved in chloroform (10.4 g) at room temperature, and the mixture was reacted at room temperature for 1 hr. The subsequent steps were performed in the same manner as in the synthesis of intermediate 11 described in Example 15 to obtain 992 mg of intermediate 31.

[Chem. 235]

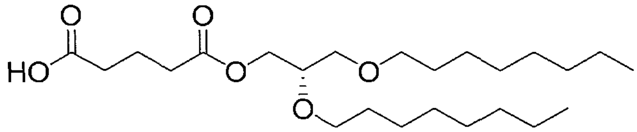

intermediate 31

<Synthesis of compound 79>

Using intermediate 31 and intermediate 1-1 and in the same manner as in the synthesis of compound 1 described in Example 1-1, compound 79 was synthesized.

[Chem. 236]

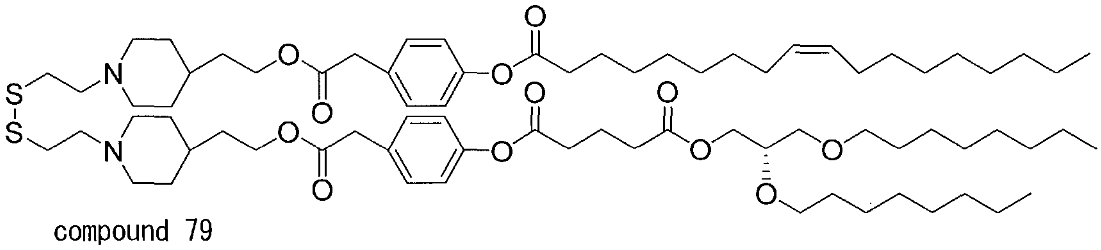

compound 79

<$^1$H-NMR (600 MHz, DMSO-$d_6$) of compound 79>

δ: 0.82-0.94 (m, 9H), 1.18-1.68 (m, 60H), 2.11-2.18 (m, 4H), 2.18-2.68 (m, 24H), 3.28-3.40 (m, 5H), 3.54-3.62 (m, 1H), 3.71 (s, 4H), 4.02-4.38 (m, 7H), 5.23-5.38 (m, 2H), 7.13-7.22 (m, 8H)

[Example 80] Synthesis of compound 80

Using intermediate 31 and intermediate 1-7 and in the same manner as in the synthesis of compound 1 described in Example 1-1, compound 80 was synthesized.

[Chem. 237]

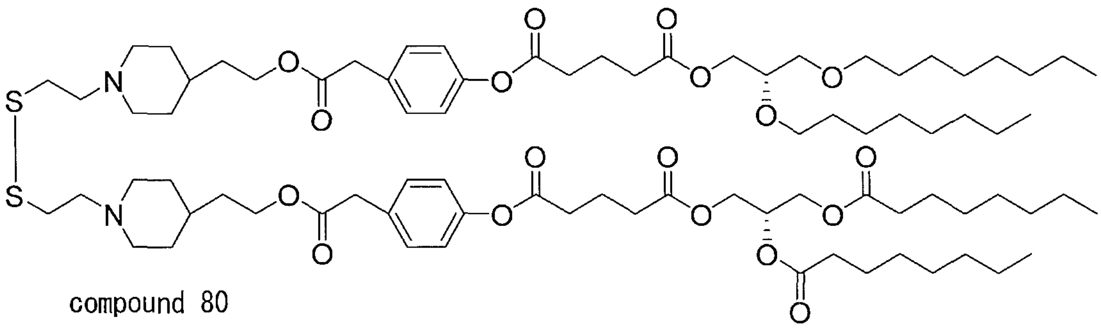

compound 80

<$^1$H-NMR (600 MHz, DMSO-$d_6$) of compound 80>

δ: 0.82-0.94 (m, 12H), 1.13-1.71 (m, 58H), 2.14-2.70 (m, 32H), 3.28-3.40 (m, 5H), 3.54-3.62 (m, 1H), 3.71 (s, 4H), 4.02-4.48 (m, 11H), 5.76-5.91 (m, 1H), 7.13-7.22 (m, 8H)

[Example 81] Synthesis of compound 81

Using intermediate 31 and intermediate 1-2 and in the same manner as in the synthesis of compound 1 described in Example 1-1, compound 81 was synthesized.

[Chem. 238]

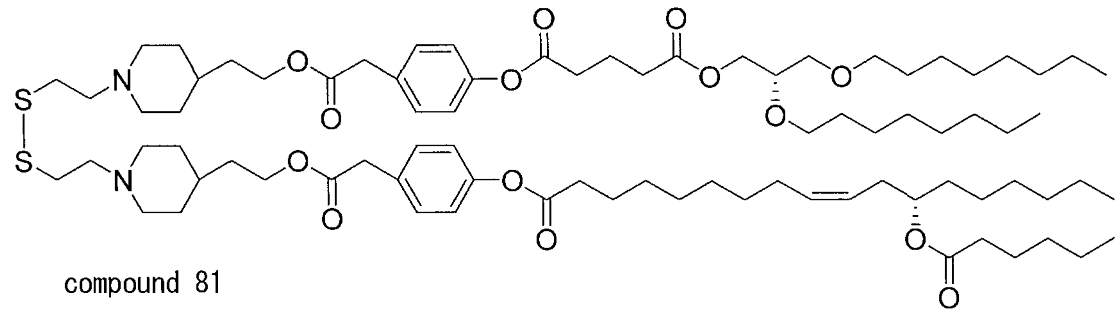

compound 81

<$^1$H-NMR (600 MHz, DMSO-$d_6$) of compound 81>

δ: 0.82-0.94 (m, 12H), 1.18-1.72 (m, 64H), 2.02-2.10 (m, 1H), 2.11-2.19 (m, 2H), 2.21-2.70 (m, 27H), 3.27-3.40 (m, 5H), 3.56-3.64 (m, 1H), 3.71 (s, 4H), 4.02-4.37 (m, 7H), 4.53-4.68 (m, 1H), 5.23-5.38 (m, 2H), 7.13-7.22 (m, 8H)

[Example 82] Synthesis of compound 82

<Synthesis of intermediate 32>

Intermediate 32 was synthesized by the following synthesis pathway.

[Chem. 239]

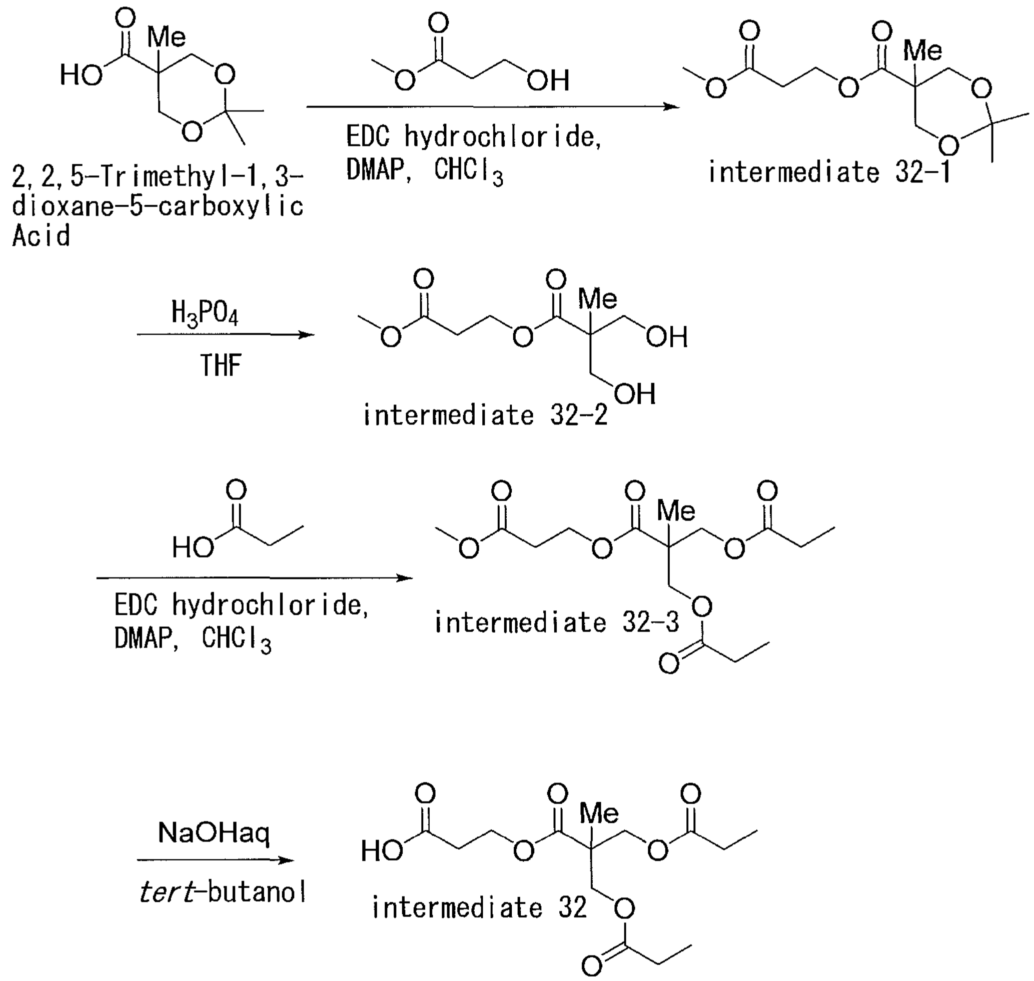

<Synthesis of intermediate 32-1>

2,2,5-Trimethyl-1,3-dioxane-5-carboxylic acid (1.00 g, 5.74 mmol) was dissolved in chloroform (5.00 g), methyl 4-hydroxybutyrate (745 mg, 6.31 mmol), DMAP (140 mg, 1.15 mmol) and EDC hydrochloride (1.21 g, 6.31 mmol) were added, and the mixture was reacted at at room temperature for 2 hr. The solution after the reaction was washed with 5 wt% saline, and concentrated by an evaporator, and the concentrate was subjected to silica gel column purification using ethanol/chloroform to give 1.41 g of intermediate 32-1.

<Synthesis of intermediate 32-2>

Intermediate 32-1 (1.40 g, 5.38 mmol) was dissolved in tetrahydrofuran (12.6 g) and isopropanol (12.6 g), phosphoric acid (2.80 g, 24.3 mmol) was added and the mixture was reacted at room temperature for 4 hr. The solution after the reaction was neutralized with 1 M NaOH aqueous solution, washed with 5 wt% saline, and concentrated by an evaporator. The concentrate was subjected to silica gel column purification using ethanol/chloroform to give 1.08 g of intermediate 32-2.

<Synthesis of intermediate 32-3>

Intermediate 32-2 (1.00 g, 4.54 mmol) was dissolved in chloroform (5.00 g), propionic acid (741 mg, 10.0 mmol), DMAP (111 mg, 0.908 mmol), and EDC hydrochloride (1.92 g, 10.0 mmol) were added and the mixture was reacted at room temperature for 2 hr. The solution after the reaction was washed with 5 wt% saline, and concentrated by an evaporator, and the concentrate was subjected to silica gel column purification using ethanol/chloroform to give 1.41 g of intermediate 32-3.

<Synthesis of intermediate 32>

Intermediate 32-3 (1.00 g, 3.00 mmol) was dissolved in tert-butanol (10.0 mL), 400 g/L NaOH aqueous solution (0.32 mL), and ion-exchanged water (1.28 mL) were added, and the mixture was reacted at room temperature for 4 hr. The mixture after the reaction was neutralized with 1 M hydrochloric acid, and the solution after neutralization was extracted with ethyl acetate. The organic layer was washed with 5 wt% saline, and concentrated by an evaporator, and the concentrate was subjected to silica gel column purification using ethanol/chloroform to give 0.35 g of intermediate 32.

<Synthesis of compound 82>

Using intermediate 32 and intermediate 1-1 and in the same manner as in the synthesis of compound 1 described in Example 1-1, compound 82 was synthesized.

[Chem. 240]

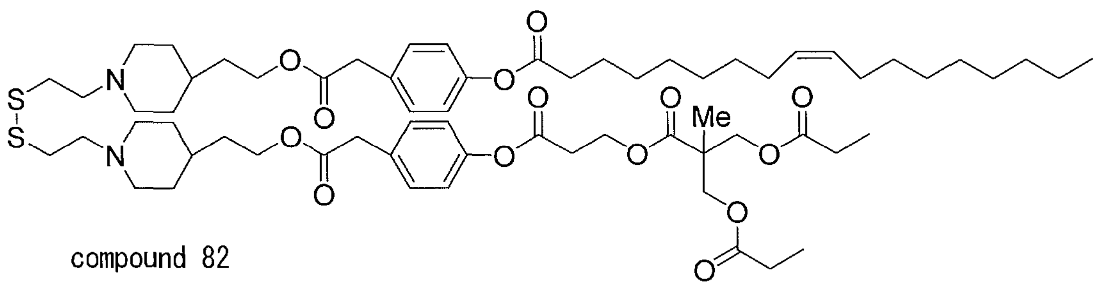

compound 82

<$^1$H-NMR (600 MHz, DMSO-$d_6$) of compound 82>

δ: 0.82-0.94 (m, 3H), 1.13-1.72 (m, 45H), 1.99-2.11 (m, 4H), 2.32-2.76 (m, 24H), 3.71 (s, 4H), 4.13 (t, 4H), 4.35 (s, 4H), 4.47 (t, 2H), 5.26-5.39 (m, 2H), 7.13-7.22 (m, 8H)

[Example 83] Synthesis of compound 83

Using intermediate 32 and intermediate 1-7 and in the same manner as in the synthesis of compound 1 described in Example 1-1, compound 83 was synthesized.

[Chem. 241]

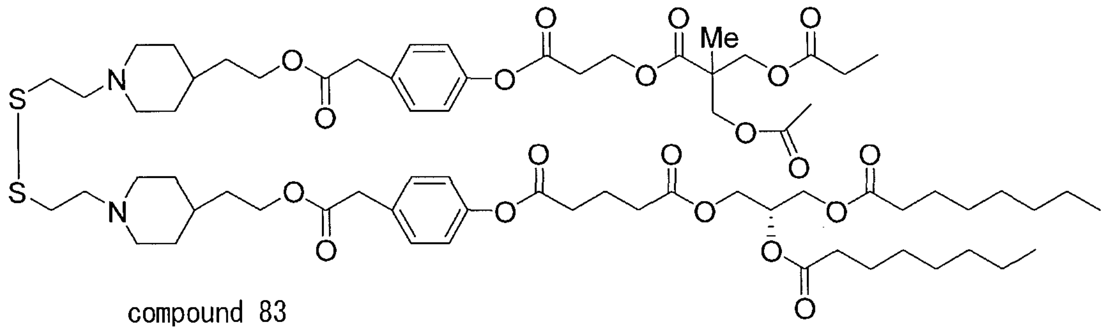

compound 83

<$^1$H-NMR (600 MHz, DMSO-$d_6$) of compound 83>

δ: 0.82-0.94 (m, 6H), 1.13-1.70 (m, 40H), 2.04 (s, 3H), 2.16-2.78 (m, 30H), 3.71 (s, 4H), 4.06-4.53 (m, 14H), 5.73-5.91 (m, 1H), 7.13-7.22 (m, 8H)

[Example 84] Synthesis of compound 84

Using intermediate 32 and intermediate 1-2 and in the same manner as in the synthesis of compound 1 described in Example 1-1, compound 84 was synthesized.

[Chem. 242]

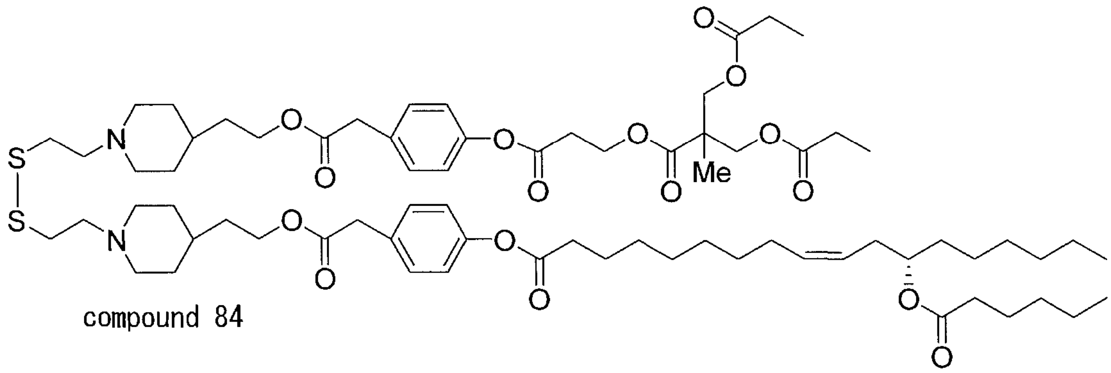

compound 84

<$^1$H-NMR (600 MHz, DMSO-$d_6$) of compound 84>

δ: 0.82-0.94 (m, 6H), 1.13-1.71 (m, 49H), 2.01-2.11 (m, 1H), 2.12-2.20 (m, 2H), 2.15-2.78 (m, 27H), 3.71 (s, 4H), 4.13 (t, 4H), 4.35 (s, 4H), 4.47 (t, 2H), 4.53-4.66 (m, 1H), 5.24-5.41 (m, 2H), 7.13-7.22 (m, 8H)

[Example 85] Synthesis of compound 85

<Synthesis of intermediate 33-1>

Intermediate 32-2 (1.00 g, 4.54 mmol) was dissolved in chloroform (5.00 g), octanoic acid (1.44 g, 10.0 mmol), DMAP (110 mg, 0.908 mmol), and EDC hydrochloride (1.92 g, 10.0 mmol) were added, and the mixture was reacted at room temperature for 2 hr. The solution after the reaction was washed with 5 wt% saline, and concentrated by an evaporator, and the concentrate was subjected to silica gel column purification using ethanol/chloroform to give 1.95 g of intermediate 33-1.

[Chem. 243]

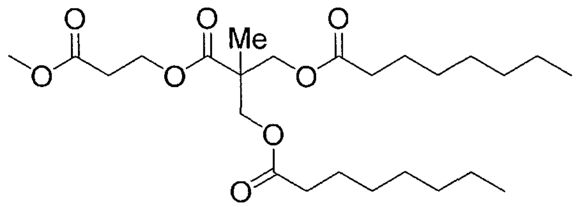

intermediate 33-1

<Synthesis of intermediate 33>

Intermediate 33-1 (1.80 g, 3.80 mmol) was dissolved in tert-butanol (12.6 mL), 400 g/L NaOH aqueous solution (0.40 mL), and ion-exchanged water (1.60 mL) were added, and the mixture was reacted at room temperature for 4 hr. The mixture after the reaction was neutralized with 1 M hydrochloric acid, and extracted with ethyl acetate. The organic layer was washed with 5 wt% saline, and concentrated by an evaporator, and the concentrate was subjected to silica gel column purification using ethanol/chloroform to give 0.958 g of intermediate 33.

[Chem. 244]

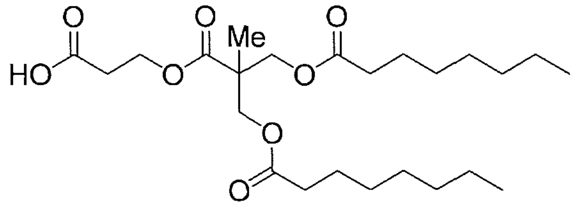

intermediate 33

<Synthesis of compound 85>

Using intermediate 33 and intermediate 1-1 and in the same manner as in the synthesis of compound 1 described in Example 1-1, compound 85 was synthesized.

[Chem. 245]

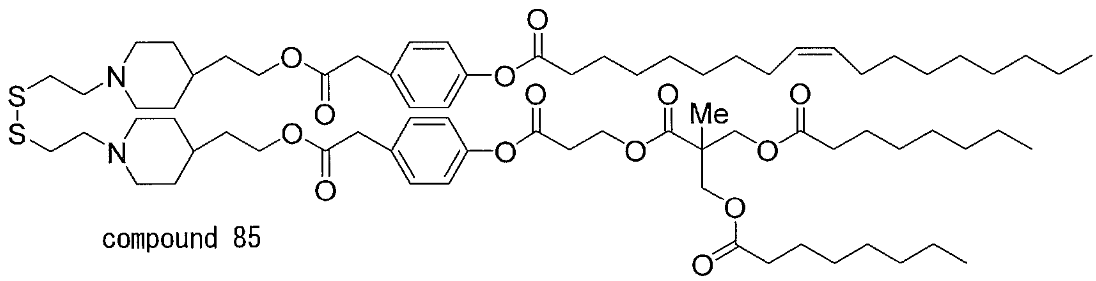

compound 85

<$^1$H-NMR (600 MHz, DMSO-$d_6$) of compound 85>

δ: 0.82-0.94 (m, 9H), 1.14-1.72 (m, 59H), 1.99-2.11 (m, 4H), 2.26-2.78 (m, 24H), 3.71 (s, 4H), 4.13 (t, 4H), 4.35 (s, 4H), 4.47 (t, 2H), 5.24-5.40 (m, 2H), 7.13-7.22 (m, 8H)

[Example 86] Synthesis of compound 86

Using intermediate 33 and intermediate 1-7 and in the same manner as in the synthesis of compound 1 described in Example 1-1, compound 86 was synthesized.

[Chem. 246]

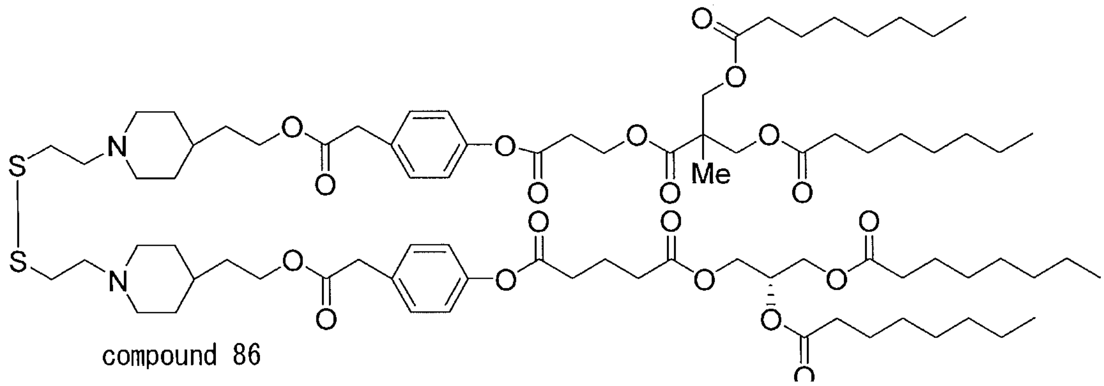

compound 86

<$^1$H-NMR (600 MHz, DMSO-$d_6$) of compound 86>

δ: 0.82-0.94 (m, 12H), 1.13-1.73 (m, 57H), 2.14-2.77 (m, 32H), 3.71 (s, 4H), 4.07-4.20 (m, 6H), 4.35 (s, 4H), 4.37-4.52 (m, 4H), 5.73-5.91 (m, 1H), 7.13-7.22 (m, 8H)

[Example 87] Synthesis of compound 87

Using intermediate 33 and intermediate 1-2 and in the same manner as in the synthesis of compound 1 described in Example 1-1, compound 87 was synthesized.

[Chem. 247]

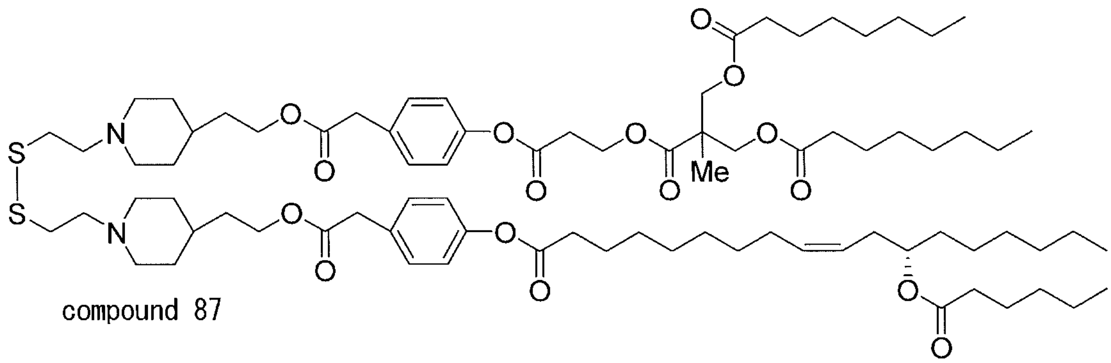

compound 87

<$^1$H-NMR (600 MHz, DMSO-$d_6$) of compound 87>

δ: 0.82-0.94 (m, 12H), 1.17-1.72 (m, 63H), 2.00-2.09 (m, 1H), 2.10-2.19 (m, 2H), 2.25-2.72 (m, 27H), 3.71 (s, 4H), 4.13 (t, 4H), 4.35 (s, 4H), 4.47 (t, 2H), 4.52-4.66 (m, 1H), 5.27-5.38 (m, 2H), 7.13-7.22 (m, 8H)

[Example 88] Synthesis of compound 88

<Synthesis of intermediate 34>

Intermediate 34 was synthesized by the following reaction.

Intermediate 12-2 (4.30 g, 18.5 mmol) obtained by the synthesis described in Example 20, 3-bromopropionic acid (8.50 g, 55.5 mmol), and sodium hydride (1.02 g, 42.6 mmol), sodium hydride (1.02 g, 42.6 mmol) were dissolved in DMF (50.0 g) at room temperature, and the mixture was reacted at 70°C for 16 hr. Thereafter, to the reaction solution was added chloroform. The reaction solution was washed with 0.5 M phosphate buffer (pH 4.0), 7 wt% sodium bicarbonate water, and 20 wt% saline, and dehydrated by adding sodium sulfate. Sodium sulfate was removed by filtration, and the filtrate was concentrated by an evaporator to give 2.03 g of intermediate 34.

[Chem. 248]

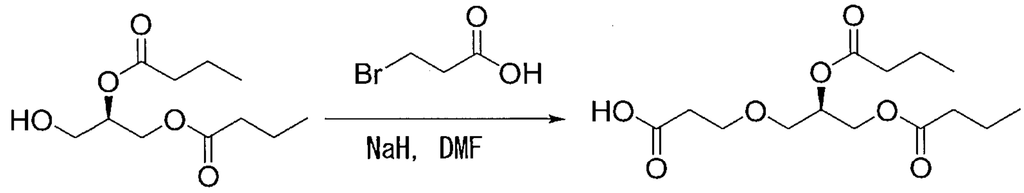


intermediate 12-2 intermediate 34

<Synthesis of compound 88>

Using intermediate 34 and intermediate 1-1 and in the same manner as in the synthesis of compound 1 described in Example 1-1, compound 88 was synthesized.

[Chem. 249]

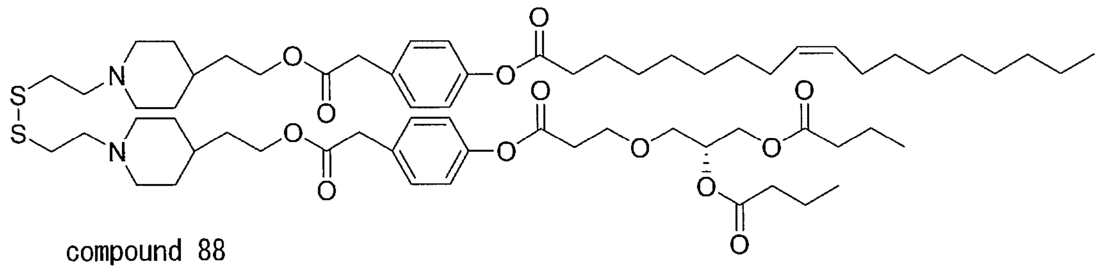

compound 88

<$^1$H-NMR (600 MHz, DMSO-$d_6$) of compound 88>

δ: 0.82-0.94 (m, 3H), 0.99 (t, 6H), 1.15-1.76 (m, 40H), 2.10-2.21 (m, 4H), 2.26-2.69 (m, 24H), 3.39-3.78 (m, 8H), 4.08-4.47 (m, 6H), 5.28-5.38 (m, 2H), 5.40-5.52 (m, 1H), 7.13-7.22 (m, 8H)

[Example 89] Synthesis of compound 89

Using intermediate 34 and intermediate 1-7 and in the same manner as in the synthesis of compound 1 described in Example 1-1, compound 89 was synthesized.

[Chem. 250]

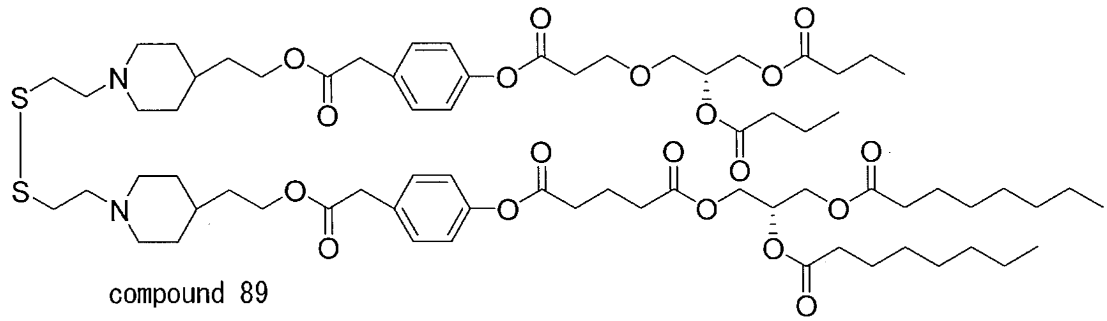

compound 89

<$^1$H-NMR (600 MHz, DMSO-$d_6$) of compound 89>

δ: 0.82-0.94 (m, 6H), 0.99 (t, 6H), 1.16-1.73 (m, 38H), 2.18-2.70 (m, 32H), 3.38-3.78 (m, 8H), 4.08-4.47 (m, 10H), 5.38-5.52 (m, 1H), 5.75-5.89 (m, 1H), 7.13-7.22 (m, 8H)

[Example 90] Synthesis of compound 90

Using intermediate 34 and intermediate 1-2 and in the same manner as in the synthesis of compound 1 described in Example 1-1, compound 90 was synthesized.

[Chem. 251]

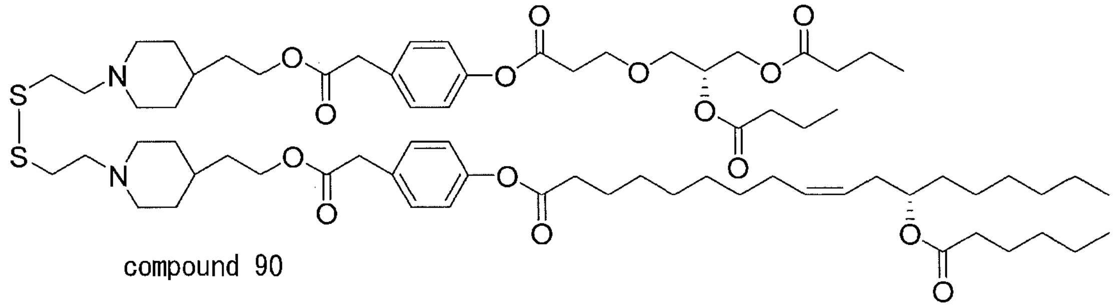

compound 90

<$^1$H-NMR (600 MHz, DMSO-$d_6$) of compound 90>

δ: 0.82-0.94 (m, 6H), 0.99 (t, 6H), 1.18-1.75 (m, 44H), 2.01-2.10 (m, 1H), 2.11-2.19 (m, 2H), 2.23-2.69 (m, 27H), 3.39-3.78 (m, 8H), 4.08-4.47 (m, 6H), 4.52-4.68 (m, 1H), 5.24-5.38 (m, 2H), 5.39-5.52 (m, 1H), 7.13-7.22 (m, 8H)

[Example 91] Synthesis of compound 91

<Synthesis of intermediate 35>

Intermediate 15-2 (4.30 g, 12.5 mmol), 3-bromopropionic acid (5.73 g, 37.4 mmol), and sodium hydride (690 mg, 28.8 mmol) were dissolved in DMF (50.0 g) at room temperature, and the mixture was reacted at 70°C for 16 hr. Thereafter, chloroform was added. The subsequent steps were performed in the same manner as in the synthesis of intermediate 34 described in Example 88 to obtain 1.67 g of intermediate 35.

[Chem. 252]

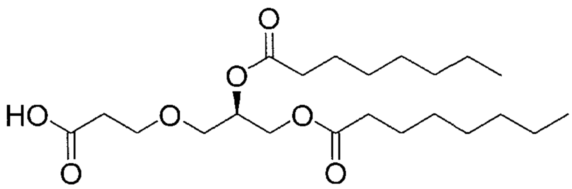

intermediate 35

<Synthesis of compound 91>

Using intermediate 35 and intermediate 1-1 and in the same manner as in the synthesis of compound 1 described in Example 1-1, compound 91 was synthesized.

[Chem. 253]

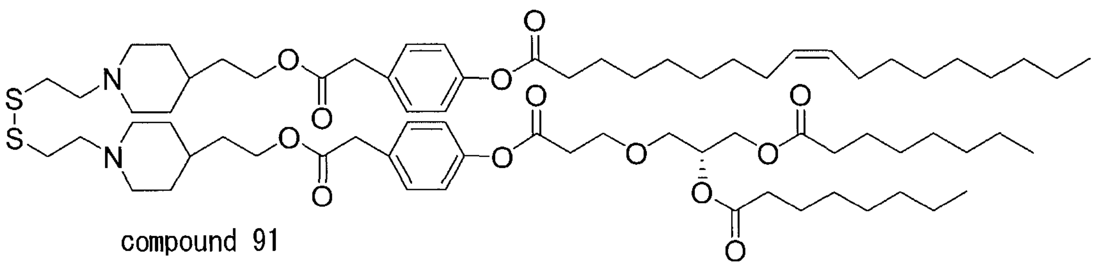

compound 91

<$^1$H-NMR (600 MHz, DMSO-$d_6$) of compound 91>

δ: 0.82-0.94 (m, 9H), 1.18-1.75 (m, 56H), 2.11-2.21 (m, 4H), 2.28-2.68 (m, 24H), 3.40-3.78 (m, 8H), 4.08-4.47 (m, 6H), 5.25-5.39 (m, 2H), 5.40-5.52 (m, 1H), 7.13-7.22 (m, 8H)

[Example 92] Synthesis of compound 92

Using intermediate 35 and intermediate 1-7 and in the same manner as in the synthesis of compound 1 described in Example 1-1, compound 92 was synthesized.

[Chem. 254]

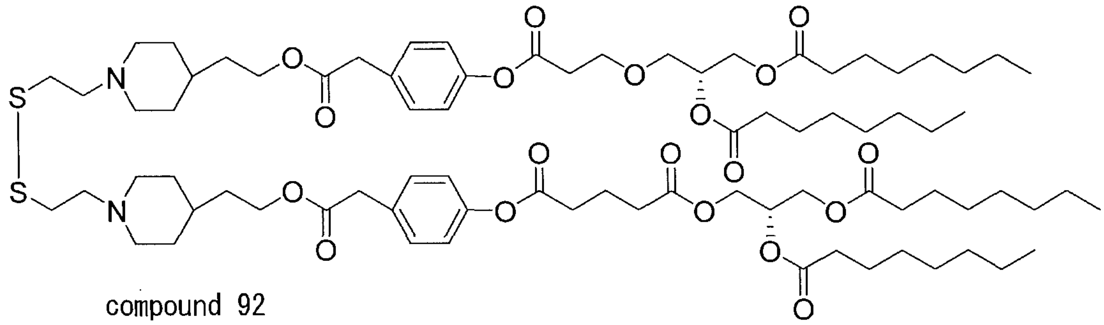

compound 92

<$^1$H-NMR (600 MHz, DMSO-$d_6$) of compound 92>

δ: 0.84-0.90 (m, 12H), 1.22-1.58 (m, 46H), 1.62-1.68 (m, 8H), 2.30-2.68 (m, 32H), 3.40-3.50 (m, 1H), 3.68-3.78 (m, 7H), 4.10-4.31 (m, 7H), 4.41-4.50 (m, 3H), 5.42-5.53 (m, 1H), 5.79-5.87 (m, 1H), 7.13-7.22 (m, 8H)

[Example 93] Synthesis of compound 93

Using intermediate 35 and intermediate 1-2 and in the same manner as in the synthesis of compound 1 described in Example 1-1, compound 93 was synthesized.

[Chem. 255]

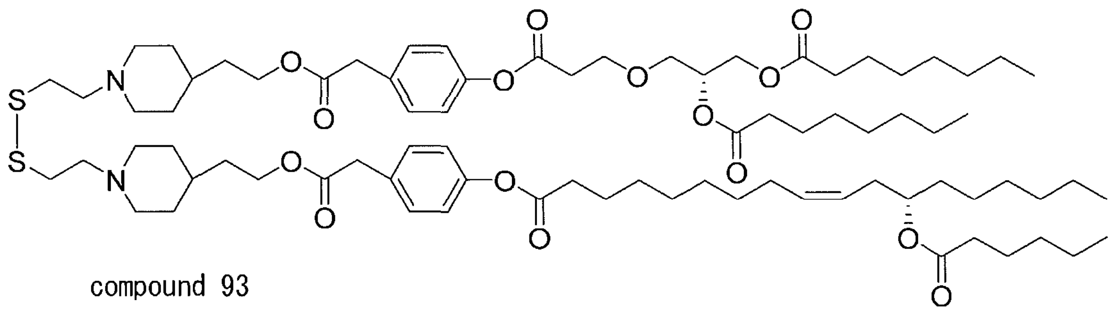

compound 93

<$^1$H-NMR (600 MHz, DMSO-$d_6$) of compound 93>

δ: 0.84-0.90 (m, 12H), 1.18-1.75 (m, 60H), 2.00-2.12 (m, 1H), 2.12-2.22 (m, 2H), 2.27-2.68 (m, 27H), 3.41-3.52 (m, 1H), 3.65-3.80 (m, 7H), 4.08-4.20 (m, 5H), 4.35-4.48 (m, 1H), 4.53-4.71 (m, 1H), 5.25-5.38 (m, 2H), 5.38-5.54 (m, 1H), 7.13-7.22 (m, 8H)

[Example 94] Synthesis of compound 94

<Synthesis of intermediate 17>

Intermediate 17 was synthesized by the following synthesis pathway.

[Chem. 256]

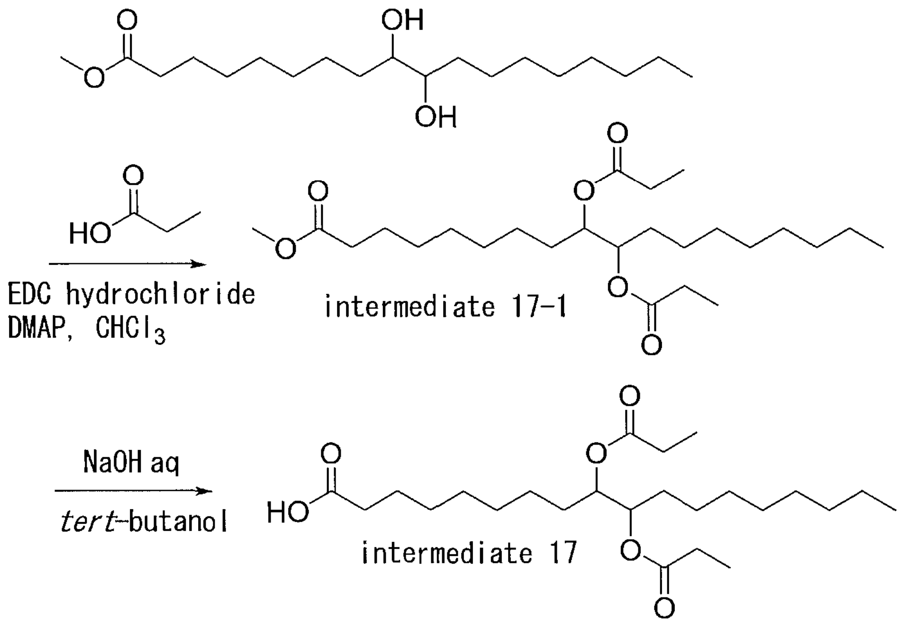


<Synthesis of intermediate 17-1>

Methyl 9,10-dihydroxystearate (4.99 g, 15.1 mmol), propionic acid (2.35 g, 31.8 mmol), and DMAP (745 mg, 6.10 mmol) were dissolved in chloroform (56.6 g) at room temperature. To the obtained mixture was added EDC hydrochloride (8.70 g, 45.4 mmol) and the mixture was reacted at room temperature for 1 hr. Thereafter, the reaction solution was washed with 5 wt% sodium hydrogen phosphate aqueous solution, and 20 wt% saline, and dehydrated by adding sodium sulfate. Sodium sulfate was removed by filtration, and the filtrate was concentrated by an evaporator to give 6.36 g of intermediate 17-1.

<Synthesis of intermediate 17>

Intermediate 17-1 (5.00 g, 11.3 mmol) was dissolved in tert-butanol (29.6 g) at room temperature. To the obtained mixture was added 2 M NaOH aqueous solution (6.21 g) and the mixture was reacted at room temperature for 8 hr. Thereafter, 1 M hydrochloric acid was added, and the obtained mixture was extracted with hexane. The organic layer was washed with 20 wt% saline, and dehydrated by adding sodium sulfate. Sodium sulfate was removed by filtration, and the filtrate was concentrated by an evaporator to give 3.87 g of intermediate 17.

<Synthesis of compound 94>

Using intermediate 17 and intermediate 1-1 and in the same manner as in the synthesis of compound 1 described in Example 1-1, compound 94 was synthesized.

[Chem. 257]

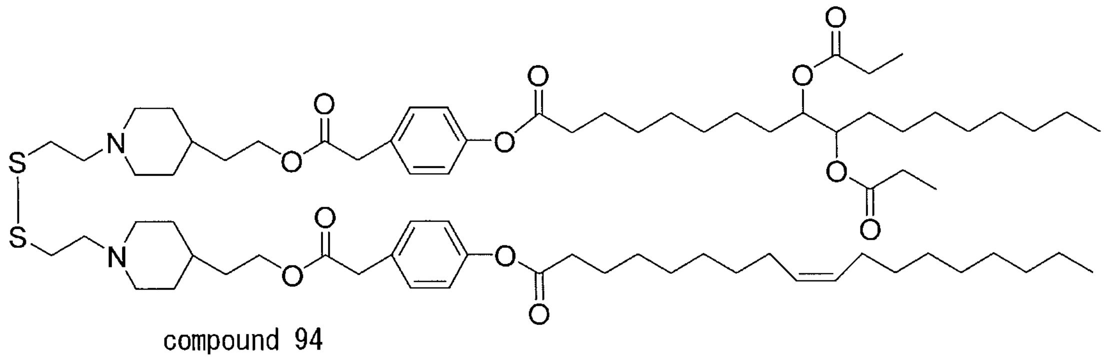

compound 94

<$^{1}$H-NMR (600 MHz, DMSO-$d_6$) of compound 94>

δ: 0.84-0.90 (m, 6H), 1.16-1.76 (m, 68H), 2.08-2.20 (m, 4H), 2.13-2.73 (m, 24H), 3.71 (s, 4H), 4.13 (t, 4H), 5.01-5.12 (m, 2H), 5.27-5.43 (m, 2H), 7.13-7.22 (m, 8H)

[Example 95] Synthesis of compound 95

Using intermediate 17 and intermediate 1-7 and in the same manner as in the synthesis of compound 1 described in Example 1-1, compound 95 was synthesized.

[Chem. 258]

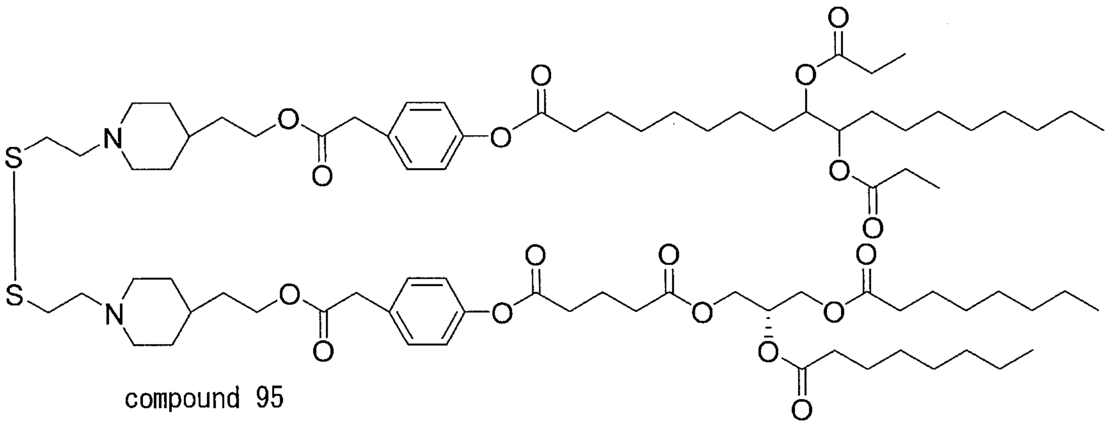

compound 95

<$^{1}$H-NMR (600 MHz, DMSO-$d_6$) of compound 95>

δ: 0.84-0.90 (m, 9H), 1.16-1.72 (m, 66H), 2.18-2.72 (m, 32H), 3.71 (s, 4H), 4.13 (t, 4H), 4.11-4.47 (m, 4H), 5.02-5.13 (m, 2H), 5.76-5.92 (m, 1H), 7.13-7.22 (m, 8H)

[Example 96] Synthesis of compound 96

Using intermediate 17 and intermediate 1-2 and in the same manner as in the synthesis of compound 1 described in Example 1-1, compound 96 was synthesized.

[Chem. 259]

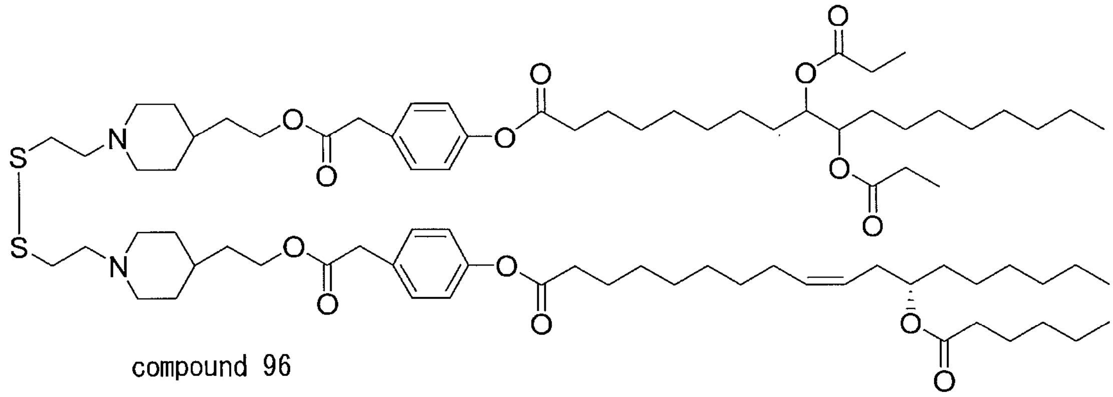

compound 96

<$^1$H-NMR (600 MHz, DMSO-$d_6$) of compound 96>

δ: 0.84-0.90 (m, 9H), 1.15-1.73 (m, 72H), 2.01-2.11 (m, 1H), 2.12-2.21 (m, 2H), 2.23-2.70 (m, 27H), 3.71 (s, 4H), 4.13 (t, 4H), 4.52-4.67 (m, 1H), 5.02-5.12 (m, 2H), 5.28-5.40 (m, 2H), 7.13-7.22 (m, 8H)

[Example 97] Synthesis of compound 97

<Synthesis of intermediate 18-1>

Methyl 9,10-dihydroxystearate (4.99 g, 15.1 mmol), octanoic acid (4.58 g, 31.8 mmol), and DMAP (745 mg, 6.10 mmol) were dissolved in chloroform (53.6 g) at room temperature. To the obtained mixture was added EDC hydrochloride (8.70 g, 45.4 mmol) and the mixture was reacted at room temperature for 1 hr. Purification was performed in the same manner as in the synthesis of intermediate 17-1 described in Example 94 to give 8.38 g of intermediate 18-1.

[Chem. 260]

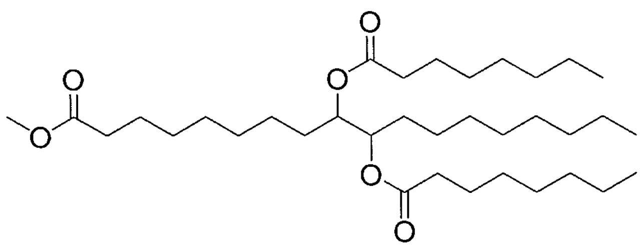

intermediate 18-1

<Synthesis of intermediate 18>

Intermediate 18-1 (5.00 g, 8.58 mmol) was dissolved in tert-butanol (22.6 g) at room temperature. To the obtained mixture was added 2 M NaOH aqueous solution (4.72 g) and the mixture was reacted at room temperature for 8 hr. Purification was performed in the same manner as in the synthesis of intermediate 17 described in Example 94 to give 3.90 g of intermediate 18.

[Chem. 261]

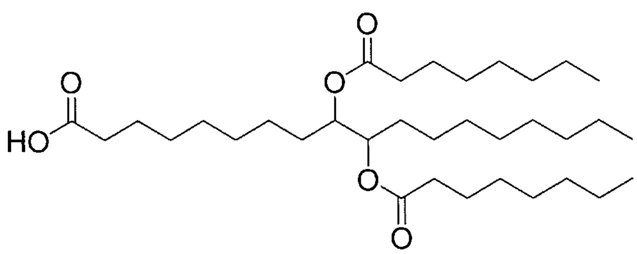

intermediate 18

<Synthesis of compound 97>

Using intermediate 18 and intermediate 1-1 and in the same manner as in the synthesis of compound 1 described in Example 1-1, compound 97 was synthesized.

[Chem. 262]

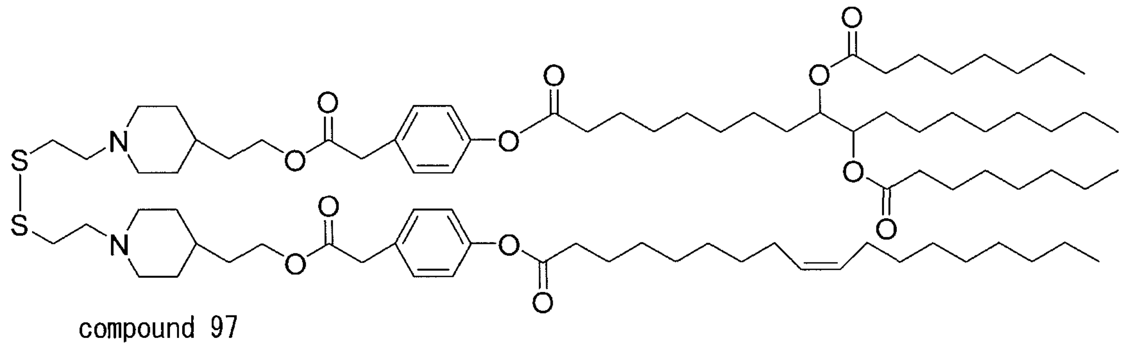

compound 97

<$^1$H-NMR (600 MHz, DMSO-$d_6$) of compound 97>

δ: 0.84-0.90 (m, 12H), 1.17-1.70 (m, 82H), 2.10-2.23 (m, 4H), 2.28-2.68 (m, 24H), 3.71 (s, 4H), 4.13 (t, 4H), 5.02-5.12 (m, 2H), 5.28-5.40 (m, 2H), 7.13-7.22 (m, 8H)

[Example 98] Synthesis of compound 98

Using intermediate 18 and intermediate 1-7 and in the same manner as in the synthesis of compound 1 described in Example 1-1, compound 98 was synthesized.

[Chem. 263]

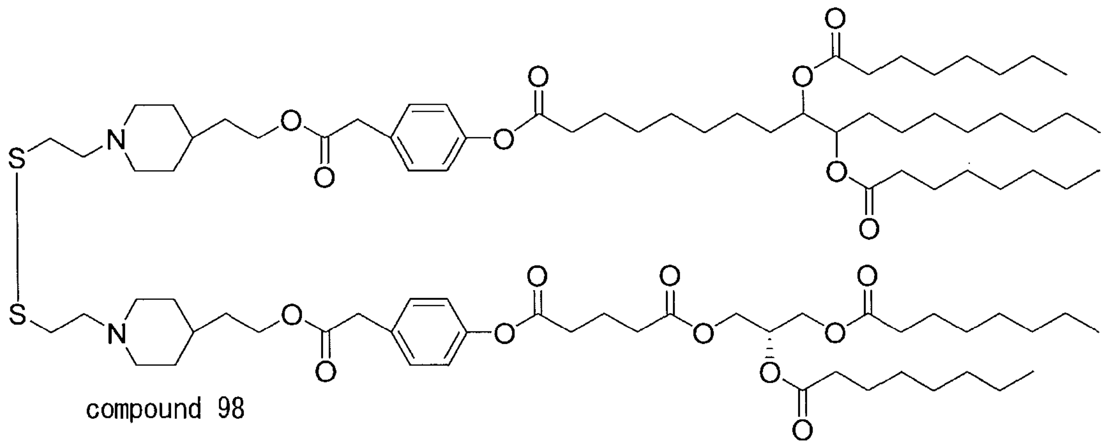

compound 98

<$^1$H-NMR (600 MHz, DMSO-$d_6$) of compound 98>

δ: 0.84-0.90 (m, 15H), 1.14-1.73 (m, 80H), 2.13-2.72 (m, 32H), 3.71 (s, 4H), 4.07-4.48 (m, 8H), 5.02-5.12 (m, 2H), 5.76-5.88 (m, 1H), 7.13-7.22 (m, 8H)

[Example 99] Synthesis of compound 99

Using intermediate 18 and intermediate 1-2 and in the same manner as in the synthesis of compound 1 described in Example 1-1, compound 99 was synthesized.

[Chem. 264]

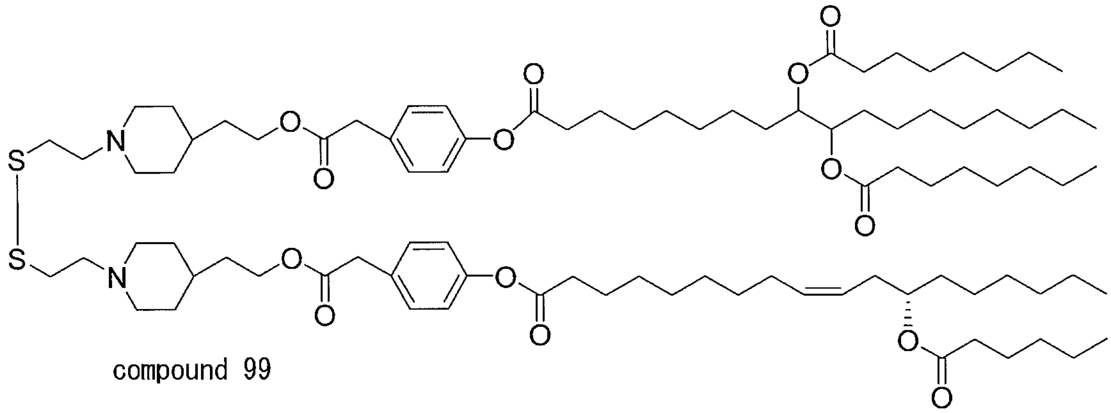

compound 99

<$^1$H-NMR (600 MHz, DMSO-$d_6$) of compound 99>

δ: 0.84-0.90 (m, 15H), 1.18-1.72 (m, 86H), 2.01-2.11 (m, 1H), 2.12-2.21 (m, 2H), 2.23-2.72 (m, 27H), 3.71 (s, 4H), 4.13 (t, 4H), 4.52-4.67 (m, 1H), 5.02-5.12 (m, 2H), 5.28-5.40 (m, 2H), 7.13-7.22 (m, 8H)

[Example 100] Synthesis of compound 100

<Synthesis of intermediate 36>

Intermediate 36 was synthesized by the following synthesis pathway.

[Chem. 265]

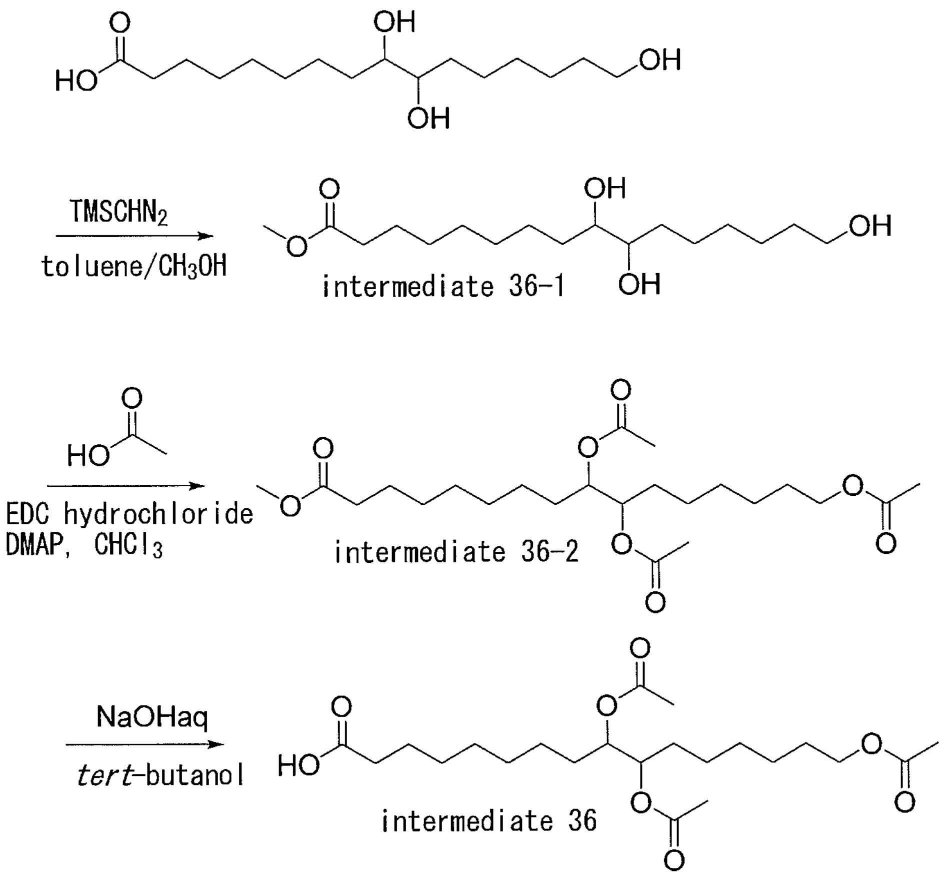

<Synthesis of intermediate 36-1>

9,10,16-Trihydroxypalmitic acid (5.00 g, 16.4 mmol) was dissolved in toluene (85.2 g) and methanol (51.5 g) at room temperature. To the obtained mixture was added about 10 wt% trimethylsilyldiazomethane solution (20.6 g) in hexane, and the mixture was reacted at room temperature for 1 hr. Thereafter, the mixture was concentrated by an evaporator to give 5.07 g of intermediate 36-1.

<Synthesis of intermediate 36-2>

Intermediate 36-1 (5.00 g, 15.7 mmol), acetic acid (2.86 g, 47.7 mmol), and DMAP (745 mg, 6.10 mmol) were dissolved in chloroform (56.6 g) at room temperature. To the obtained mixture was added EDC hydrochloride (11.6 g, 60.5 mmol) and the mixture was reacted at room temperature for 1 hr. Thereafter, the reaction solution was washed with 5 wt% sodium hydrogen phosphate aqueous solution, and 20 wt% saline, and dehydrated by adding sodium sulfate. Sodium sulfate was removed by filtration, and the filtrate was concentrated by an evaporator to give 6.63 g of intermediate 36-2.

<Synthesis of intermediate 36>

Intermediate 36-2 (5.00 g, 11.3 mmol) was dissolved in tert-butanol (28.9 g) at room temperature. To the obtained mixture was added 2 M NaOH aqueous solution (6.19 g) and the mixture was reacted at room temperature for 8 hr. Thereafter, 1 M hydrochloric acid was added and the obtained mixture was extracted with hexane. The organic layer was washed with 20 wt% saline, and dehydrated by adding sodium sulfate. Sodium sulfate was removed by filtration, and the filtrate was concentrated by an evaporator to give 4.23 g of intermediate 36.

<Synthesis of compound 100>

Using intermediate 36 and intermediate 1-1 and in the same manner as in the synthesis of compound 1 described in Example 1-1, compound 100 was synthesized.

[Chem. 266]

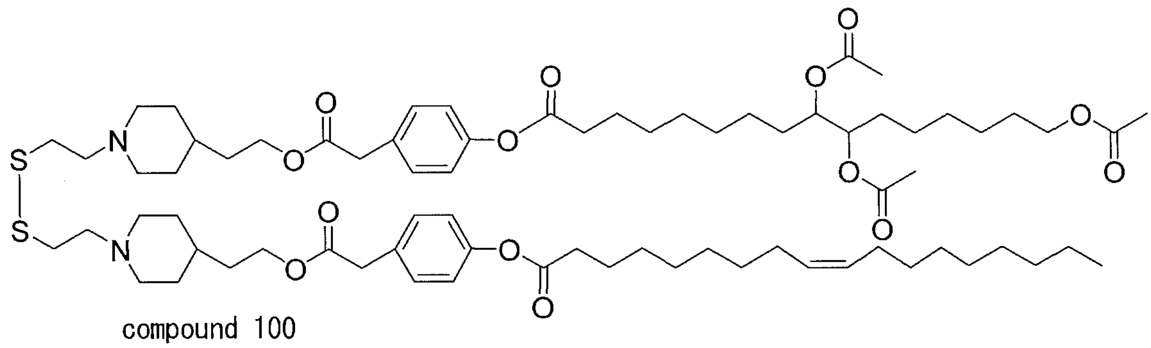

compound 100

<$^1$H-NMR (600 MHz, DMSO-$d_6$) of compound 100>

δ: 0.84-0.90 (m, 3H), 1.18-1.73 (m, 58H), 2.02 (s, 6H), 2.04 (s, 3H), 2.10-2.21 (m, 4H), 2.32-2.70 (m, 20H), 3.71 (s, 4H), 4.13 (t, 6H), 5.02-5.12 (m, 2H), 5.28-5.40 (m, 2H), 7.13-7.22 (m, 8H)

[Example 101] Synthesis of compound 101

Using intermediate 36 and intermediate 1-7 and in the same manner as in the synthesis of compound 1 described in Example 1-1, compound 101 was synthesized.

[Chem. 267]

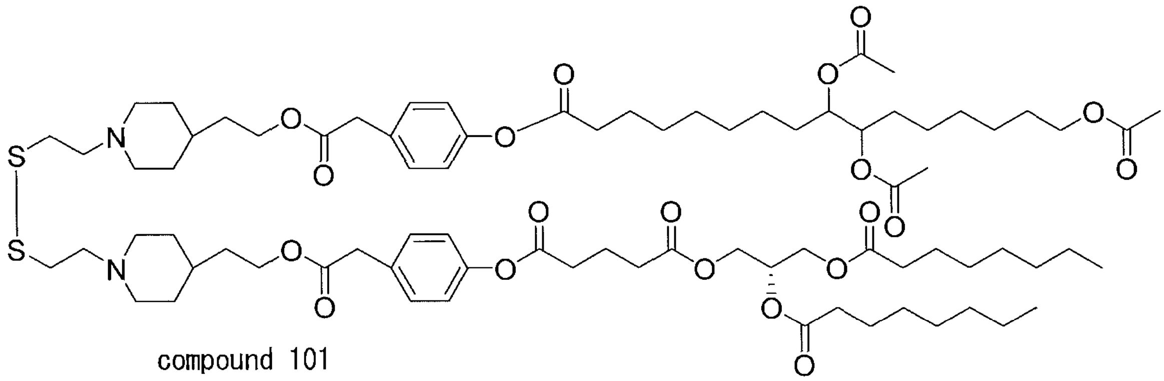

compound 101

<$^{1}$H-NMR (600 MHz, DMSO-$d_6$) of compound 101>

δ: 0.84-0.90 (m, 6H), 1.15-1.73 (m, 56H), 2.02 (s, 6H), 2.04 (s, 3H), 2.16-2.69 (m, 28H), 3.71 (s, 4H), 4.13 (t, 6H), 4.30 (d, 4H), 5.02-5.12 (m, 2H), 5.76-5.92 (m, 1H), 7.13-7.22 (m, 8H)

[Example 102] Synthesis of compound 102

Using intermediate 36 and intermediate 1-2 and in the same manner as in the synthesis of compound 1 described in Example 1-1, compound 102 was synthesized.

[Chem. 268]

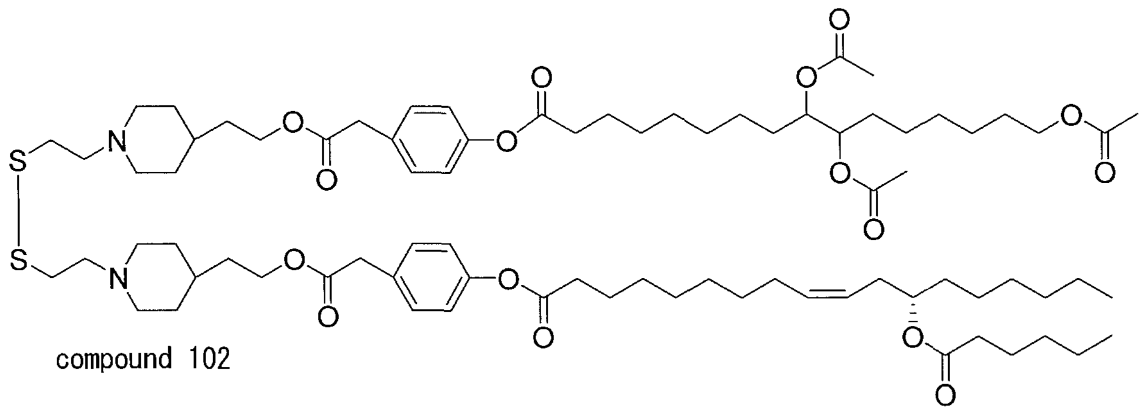

compound 102

<$^{1}$H-NMR (600 MHz, DMSO-$d_6$) of compound 102>

δ: 0.84-0.90 (m, 6H), 1.16-1.72 (m, 62H), 2.02 (s, 6H), 2.04 (s, 3H), 2.04-2.11 (m, 1H), 2.12-2.18 (m, 2H), 2.24-2.68 (m, 23H), 3.71 (s, 4H), 4.13 (t, 6H), 4.52-4.67 (m, 1H), 5.02-5.12 (m, 2H), 5.28-5.40 (m, 2H), 7.13-7.22 (m, 8H)

[Example 103] Synthesis of compound 103

<Synthesis of intermediate 37>

To 5-aminovaleric acid (1.00 g, 8.54 mmol) was added 1,2-butylene oxide (1.36 g, 18.8 mmol), and the mixture was reacted at 80°C for 10 hr. The solution after the reaction was washed with 5 wt% saline, and concentrated by an evaporator, and the concentrate was subjected to silica gel column purification using ethanol/chloroform to give 0.959 g of intermediate 37.

[Chem. 269]

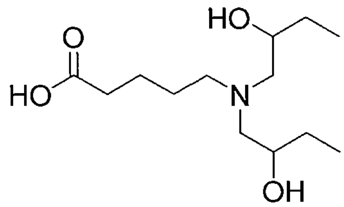

intermediate 37

<Synthesis of compound 103>

Using intermediate 37 and intermediate 1-1 and in the same manner as in the synthesis of compound 1 described in Example 1-1, compound 103 was synthesized.

[Chem. 270]

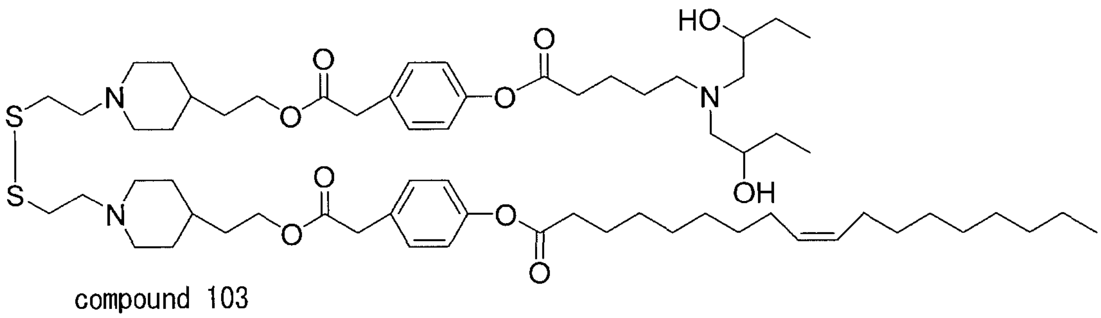

compound 103

<$^1$H-NMR (600 MHz, DMSO-$d_6$) of compound 103>

δ: 0.82-0.94 (m, 9H), 1.14-1.70 (m, 46H), 2.08-2.18 (m, 4H), 2.28-2.70 (m, 24H), 3.37-3.52 (m, 2H), 3.71 (s, 4H), 4.13 (t, 4H), 5.24-5.42 (m, 2H), 7.13-7.22 (m, 8H)

[Example 104] Synthesis of compound 104

Using intermediate 37 and intermediate 1-7 and in the same manner as in the synthesis of compound 1 described in Example 1-1, compound 104 was synthesized.

[Chem. 271]

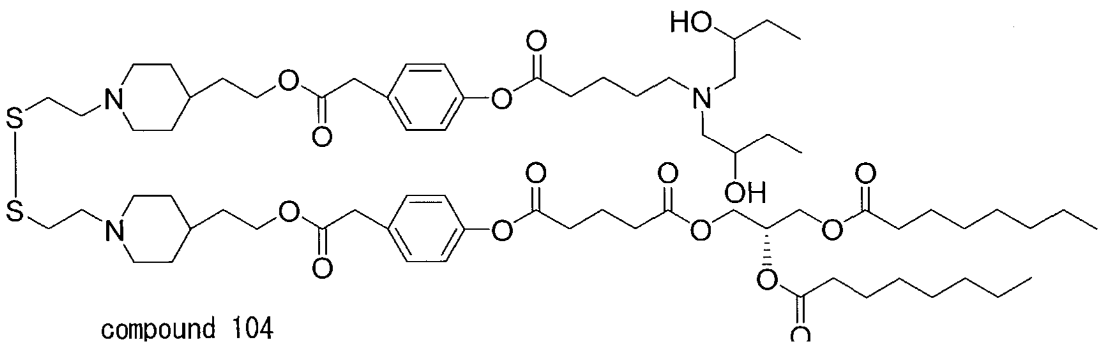

compound 104

<$^1$H-NMR (600 MHz, DMSO-$d_6$) of compound 104>

δ: 0.82-0.94 (m, 12H), 1.14-1.72 (m, 42H), 2.13-2.72 (m, 34H), 3.35-3.52 (m, 2H), 3.71 (s, 4H), 4.07-4.47 (m, 8H), 5.76-5.91 (m, 1H), 7.13-7.22 (m, 8H)

[Example 105] Synthesis of compound 105

Using intermediate 37 and intermediate 1-2 and in the same manner as in the synthesis of compound 1 described in Example 1-1, compound 105 was synthesized.

[Chem. 272]

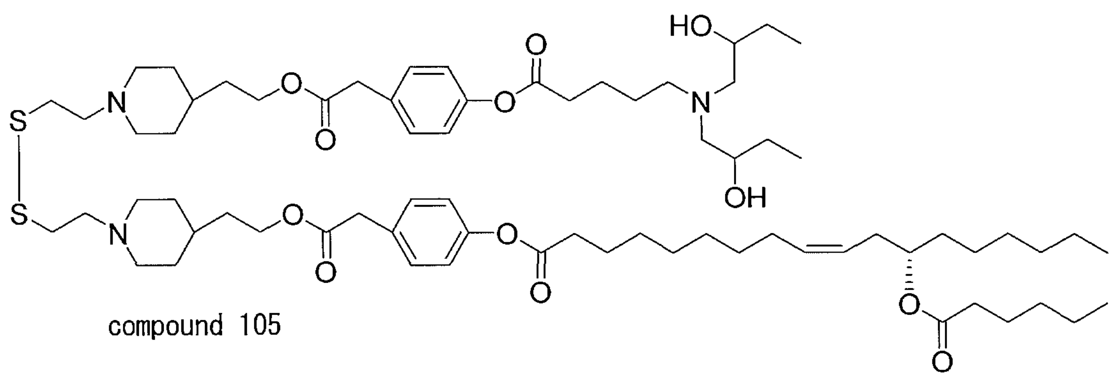

compound 105

<$^1$H-NMR (600 MHz, DMSO-$d_6$) of compound 105>

δ: 0.82-0.94 (m, 12H), 1.14-1.72 (m, 48H), 2.01-2.09 (m, 1H), 2.11-2.22 (m, 2H), 2.26-2.72 (m, 29H), 3.35-3.52 (m, 2H), 3.71 (s, 4H), 4.13 (t, 4H), 4.53-4.64 (m, 1H), 5.25-5.43 (m, 2H), 7.13-7.22 (m, 8H)

[Example 106] Synthesis of compound 106

<Synthesis of intermediate 38>

To 5-aminovaleric acid (1.00 g, 8.54 mmol) was added 1,2-epoxyoctane (2.41 g, 18.8 mmol), and the mixture was reacted at 80°C for 12 hr. The solution after the reaction was washed with 5 wt% saline, and concentrated by an evaporator, and the concentrate was subjected to silica gel column purification using ethanol/chloroform to give 1.41 g of intermediate 38.

[Chem. 273]

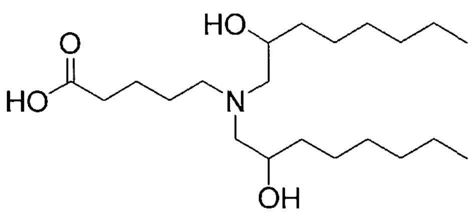

intermediate 38

<Synthesis of compound 106>

Using intermediate 38 and intermediate 1-1 and in the same manner as in the synthesis of compound 1 described in Example 1-1, compound 106 was synthesized.

[Chem. 274]

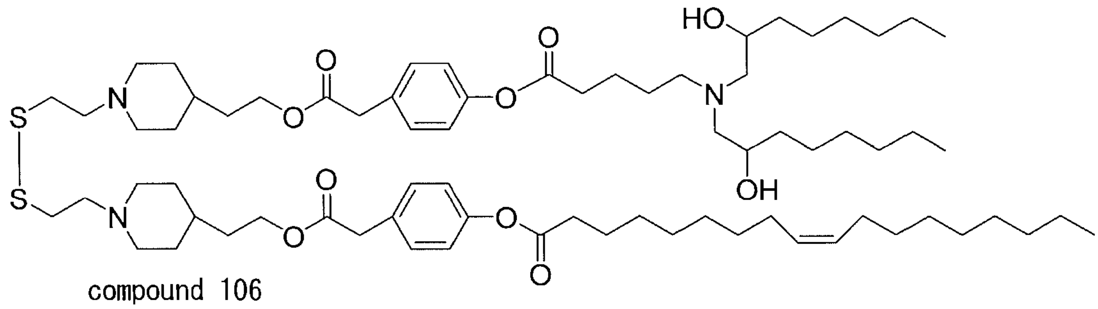

compound 106

<$^1$H-NMR (600 MHz, DMSO-$d_6$) of compound 106>

δ: 0.82-0.94 (m, 9H), 1.13-1.72 (m, 60H), 2.08-2.18 (m, 4H), 2.28-2.72 (m, 26H), 3.35-3.52 (m, 2H), 3.71 (s, 4H), 4.13 (t, 4H), 5.25-5.43 (m, 2H), 7.13-7.22 (m, 8H)

[Example 107] Synthesis of compound 107

Using intermediate 38 and intermediate 1-7 and in the same manner as in the synthesis of compound 1 described in Example 1-1, compound 107 was synthesized.

[Chem. 275]

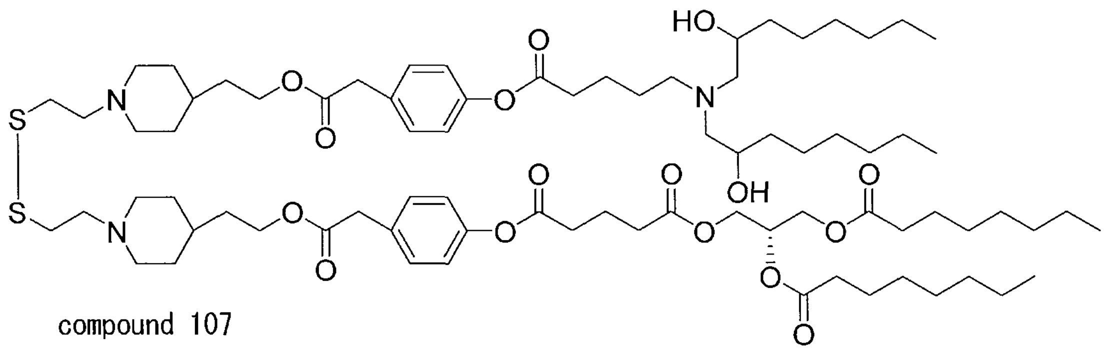

compound 107

<$^1$H-NMR (600 MHz, DMSO-$d_6$) of compound 107>

δ: 0.82-0.94 (m, 12H), 1.14-1.72 (m, 58H), 2.13-2.72 (m, 34H), 3.35-3.52 (m, 2H), 3.71 (s, 4H), 4.07-4.47 (m, 8H), 5.76-5.91 (m, 1H), 7.13-7.22 (m, 8H)

[Example 108] Synthesis of compound 108

Using intermediate 38 and intermediate 1-2 and in the same manner as in the synthesis of compound 1 described in Example 1-1, compound 108 was synthesized.

[Chem. 276]

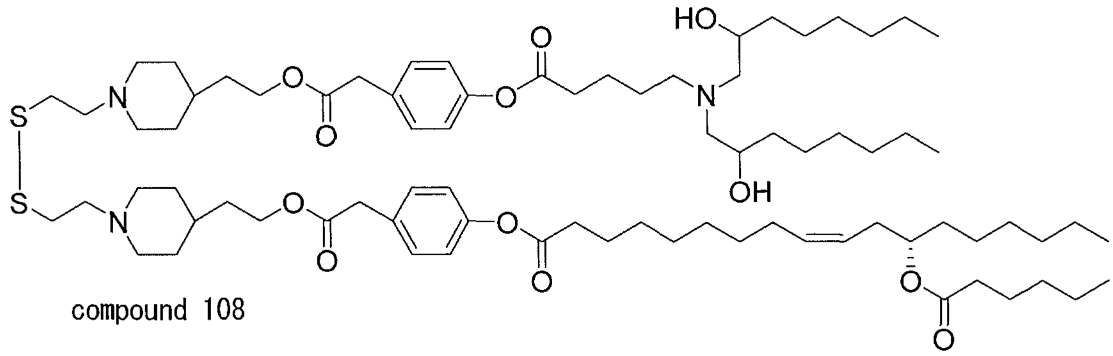

compound 108

<$^1$H-NMR (600 MHz, DMSO-$d_6$) of compound 108>

δ: 0.82-0.94 (m, 12H), 1.14-1.72 (m, 64H), 2.01-2.09 (m, 1H), 2.11-2.22 (m, 2H), 2.26-2.72 (m, 29H), 3.35-3.52 (m, 2H), 3.71 (s, 4H), 4.13 (t, 4H), 4.53-4.64 (m, 1H), 5.25-5.43 (m, 2H), 7.13-7.22 (m, 8H)

[Example 109] Synthesis of compound 109

<Synthesis of intermediate 39>

1H,1H,2H,2H-nonafluoro-1-hexanol (2.03 g, 7.68 mmol), glutaric anhydride (963 mg, 8.44 mmol), and DMAP (188 mg, 1.54 mmol) were dissolved in chloroform (23.7 g) at room temperature, and the mixture was reacted at room temperature for 1 hr. The subsequent steps were performed in the same manner as in the synthesis of intermediate 11 described in Example 15 to obtain 2.38 g of intermediate 39.

[Chem. 277]

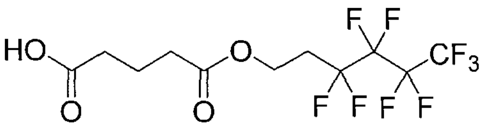

intermediate 39

<Synthesis of compound 109>

Using intermediate 39 and intermediate 1-1 and in the same manner as in the synthesis of compound 1 described in Example 1-1, compound 109 was synthesized.

[Chem. 278]

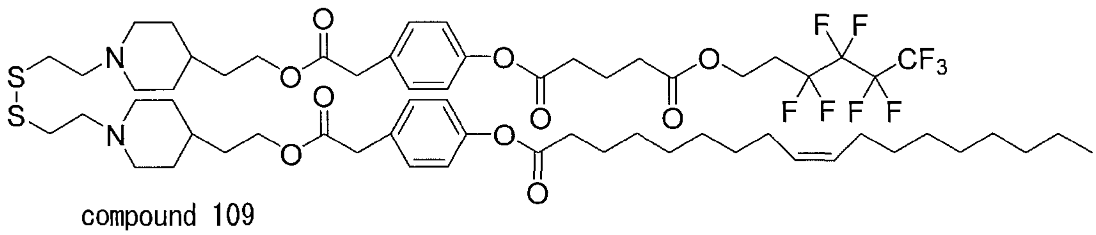

compound 109

<$^1$H-NMR (600 MHz, DMSO-$d_6$) of compound 109>

δ: 0.82-0.94 (m, 3H), 1.18-1.72 (m, 38H), 2.08-2.19 (m, 4H), 2.19-2.68 (m, 24H), 3.71 (s, 4H), 4.13 (t, 6H), 5.24-5.38 (m, 2H), 7.13-7.22 (m, 8H)

[Example 110] Synthesis of compound 110

Using intermediate 39 and intermediate 1-7 and in the same manner as in the synthesis of compound 1 described in Example 1-1, compound 110 was synthesized.

[Chem. 279]

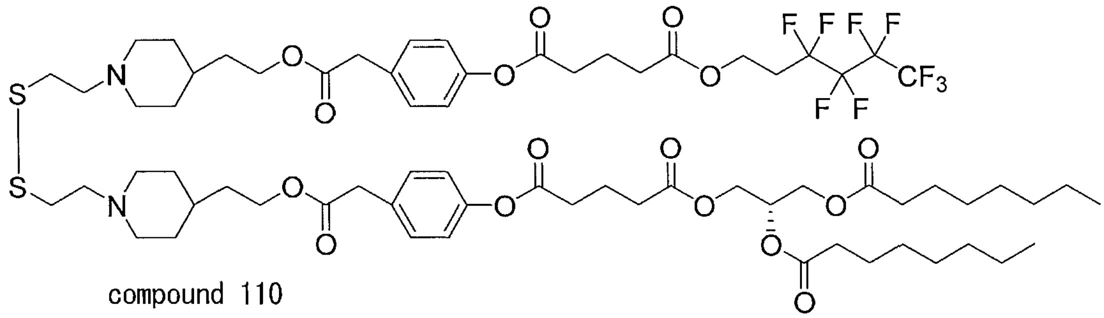

compound 110

<$^1$H-NMR (600 MHz, DMSO-$d_6$) of compound 110>

δ: 0.82-0.94 (m, 6H), 1.14-1.71 (m, 36H), 2.14-2.72 (m, 32H), 3.71 (s, 4H), 4.08-4.47 (m, 10H), 5.74-5.88 (m, 1H), 7.13-7.22 (m, 8H)

[Example 111] Synthesis of compound 111

Using intermediate 39 and intermediate 1-2 and in the same manner as in the synthesis of compound 1 described in Example 1-1, compound 111 was synthesized.

[Chem. 280]

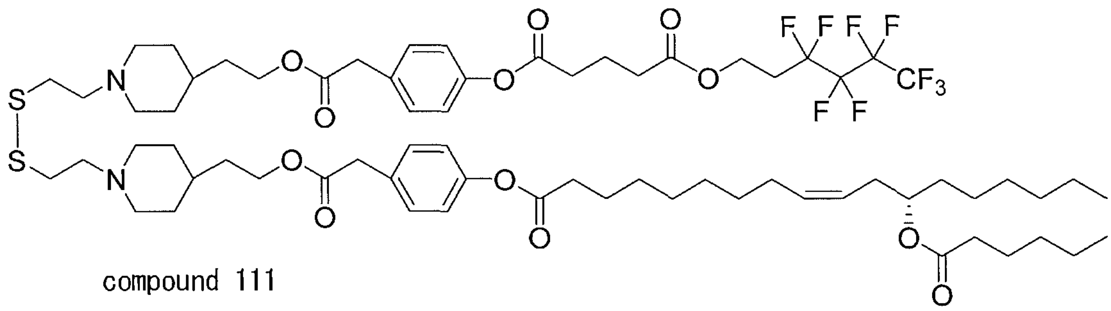

compound 111

<$^1$H-NMR (600 MHz, DMSO-$d_6$) of compound 111>

δ: 0.82-0.94 (m, 6H), 1.13-1.69 (m, 42H), 2.01-2.11 (m, 1H), 2.12-2.20 (m, 2H), 2.21-2.71 (m, 27H), 3.71 (s, 4H), 4.13 (t, 6H), 4.52-4.66 (m, 1H), 5.24-5.47 (m, 2H), 7.13-7.22 (m, 8H)

[Example 112] Synthesis of compound 112

<Synthesis of intermediate 40>

Intermediate 40 was synthesized by the following synthesis pathway.

[Chem. 281]

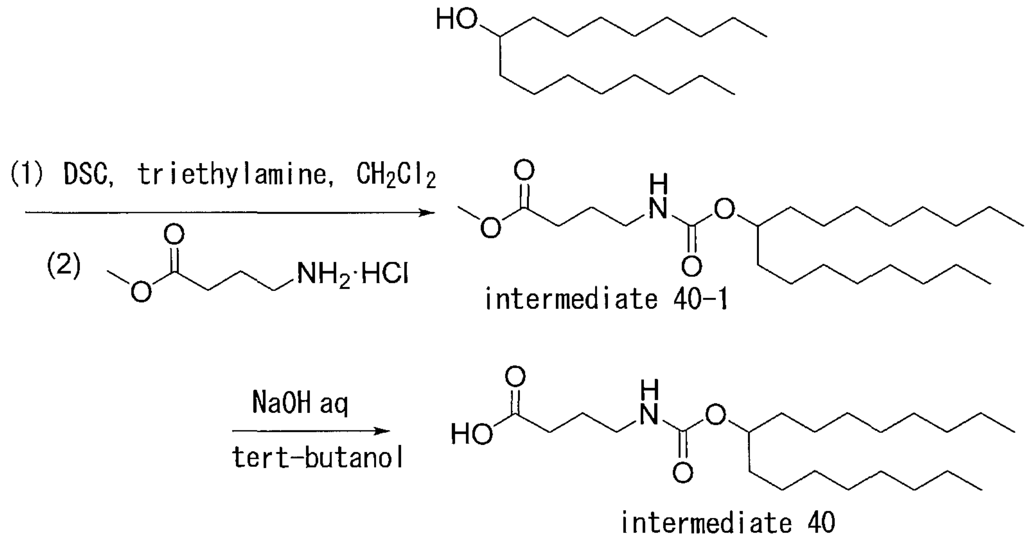


<Synthesis of intermediate 40-1>

9-Heptadecanol (1.00 g, 3.90 mmol), and triethylamine (3.35 g, 33.1 mmol) were dissolved in dichloromethane (7.50 mL) at room temperature. To the obtained mixture was added DSC (7.99 g, 31.1 mmol) and the mixture was reacted at room temperature for 30 hr. Thereafter, the reaction solution was filtered, methyl 4-aminobutyrate hydrochloride (1.20 g, 7.80 mmol) was added and the mixture was reacted at room temperature for 4 hr. Thereafter, the reaction solution was washed with 5 wt% sodium dihydrogen phosphate aqueous solution, 7 wt% sodium bicarbonate water, and 20 wt% saline, and dehydrated by adding sodium sulfate. Sodium sulfate was removed by filtration, and the filtrate was concentrated by an evaporator to give 977 mg of intermediate 40-1.

<Synthesis of intermediate 40>

Intermediate 40-1 (783 mg, 1.96 mmol) was dissolved in tert-butanol (6.52 mL) at room temperature. To the obtained mixture was added 2 M NaOH aqueous solution (1.35 g) and the mixture was reacted at room temperature for 2 hr. Thereafter, 1 M hydrochloric acid was added, and the obtained mixture was extracted with ethyl acetate. The organic layer was washed with 20 wt% saline, and dehydrated by adding sodium sulfate. Sodium sulfate was removed by filtration, and the filtrate was concentrated by an evaporator. The obtained residue was purified by silica gel column chromatography (chloroform/ethanol=90/10)to give 634 mg of intermediate 40.

<Synthesis of compound 112>

Using intermediate 40 and intermediate 1-1 and in the same manner as in the synthesis of compound 1 described in Example 1-1, compound 112 was synthesized.

[Chem. 282]

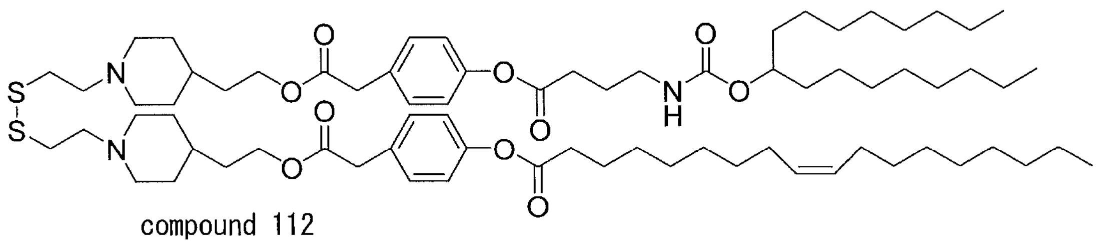

compound 112

<$^1$H-NMR (600 MHz, DMSO-$d_6$) of compound 112>

δ: 0.82-0.94 (m, 9H), 1.16-1.71 (m, 64H), 1.98-2.08 (m, 2H), 2.11-2.22 (m, 4H), 2.32-2.68 (m, 20H), 3.18 (t, 2H),

3.71 (s, 4H), 4.13 (t, 4H), 4.38-4.53 (m, 1H), 5.24-5.38 (m, 2H), 6.76 (brs, 1H), 7.13-7.22 (m, 8H)

[Example 113] Synthesis of compound 113

Using intermediate 40 and intermediate 1-7 and in the same manner as in the synthesis of compound 1 described in Example 1-1, compound 113 was synthesized.

[Chem. 283]

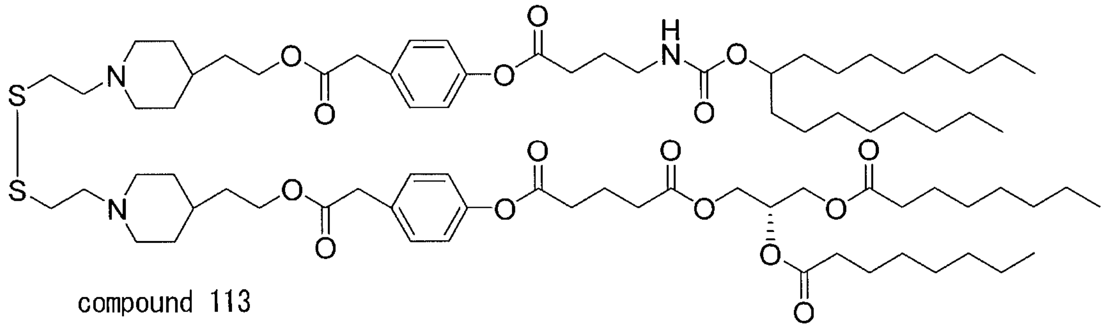

compound 113

<$^1$H-NMR (600 MHz, DMSO-$d_6$) of compound 113>

δ: 0.82-0.94 (m, 12H), 1.14-1.71 (m, 62H), 1.98-2.10 (m, 2H), 2.16-2.70 (m, 28H), 3.18 (t, 2H), 3.71 (s, 4H), 4.13 (t, 4H), 4.30 (d, 4H), 4.40-4.53 (m, 1H), 5.76-5.91 (m, 1H), 6.76 (brs, 1H), 7.13-7.22 (m, 8H)

[Example 114] Synthesis of compound 114

Using intermediate 40 and intermediate 1-2 and in the same manner as in the synthesis of compound 1 described in Example 1-1, compound 114 was synthesized.

[Chem. 284]

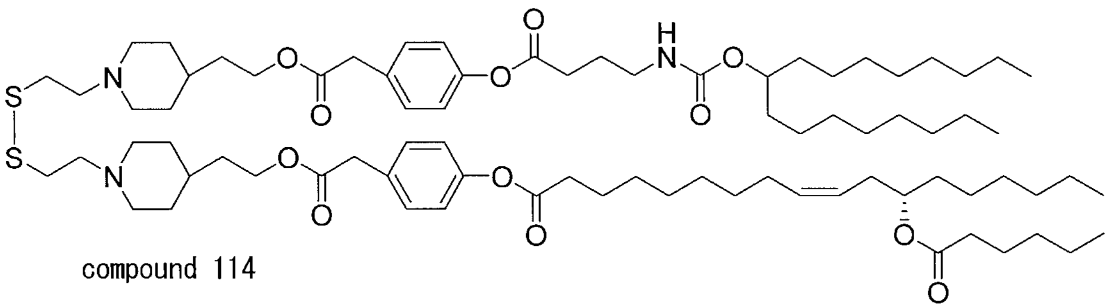

compound 114

<$^1$H-NMR (600 MHz, DMSO-$d_6$) of compound 114>

δ: 0.82-0.94 (m, 12H), 1.16-1.69 (m, 68H), 1.98-2.09 (m, 3H), 2.11-2.22 (m, 2H), 2.25-2.69 (m, 23H), 3.18 (t, 2H), 3.71 (s, 4H), 4.13 (t, 4H), 4.40-4.52 (m, 1H), 4.53-4.63 (m, 1H), 5.28-5.37 (m, 2H), 6.76 (brs, 1H), 7.13-7.22 (m, 8H)

[Example 115] Synthesis of compound 115

<Synthesis of intermediate 41>

Intermediate 41 was synthesized by the following synthesis pathway.

[Chem. 285]

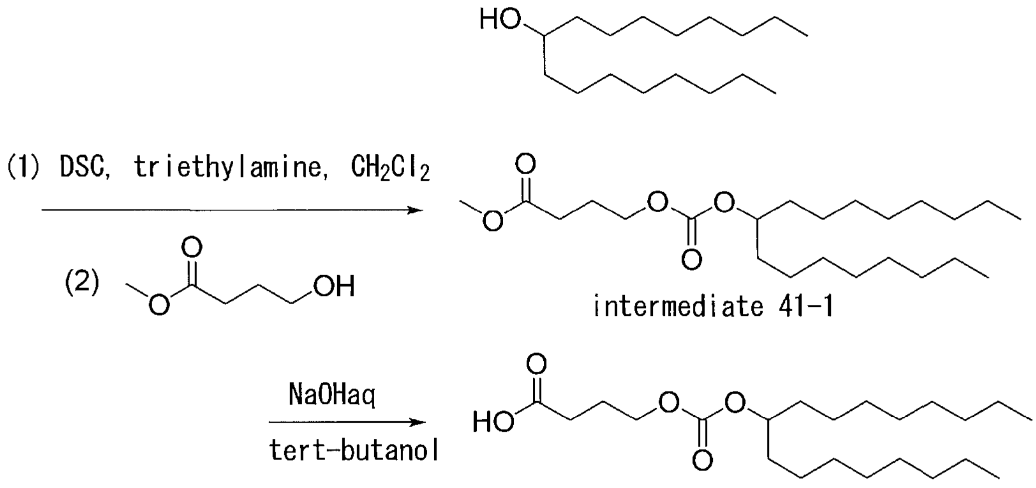


intermediate 41-1

intermediate 41

<Synthesis of intermediate 41-1>

9-Heptadecanol (1.00 g, 3.90 mmol), and triethylamine (3.35 g, 33.1 mmol) were dissolved in dichloromethane (7.50 mL) at room temperature. To the obtained mixture was added DSC (7.99 g, 31.1 mmol) and the mixture was reacted at room temperature for 30 hr. Thereafter, the reaction solution was filtered, methyl 4-hydroxybutyrate (0.921 g, 7.80 mmol) was added and the mixture was reacted at room temperature for 4 hr. Thereafter, the reaction solution was washed with 5 wt% sodium dihydrogen phosphate aqueous solution, 7 wt% sodium bicarbonate water, and 20 wt% saline, and dehydrated by adding sodium sulfate. Sodium sulfate was removed by filtration, and the filtrate was concentrated by an evaporator to give 1.01 g of intermediate 41-1.

<Synthesis of intermediate 41>

Intermediate 41-1 (950 mg, 2.37 mmol) was dissolved in tert-butanol (7.90 g) at room temperature. To the obtained mixture was added 2 M NaOH aqueous solution (1.54 g) and the mixture was reacted at room temperature for 3 hr. Thereafter, 1 M hydrochloric acid was added, and the obtained mixture was extracted with ethyl acetate. The organic layer was washed with 20 wt% saline and dehydrated by adding sodium sulfate. Sodium sulfate was removed by filtration, and the filtrate was concentrated by an evaporator to give 550 mg of intermediate 41.

<Synthesis of compound 115>

Using intermediate 41 and intermediate 1-1 and in the same manner as in the synthesis of compound 1 described in Example 1-1, compound 115 was synthesized.

[Chem. 286]

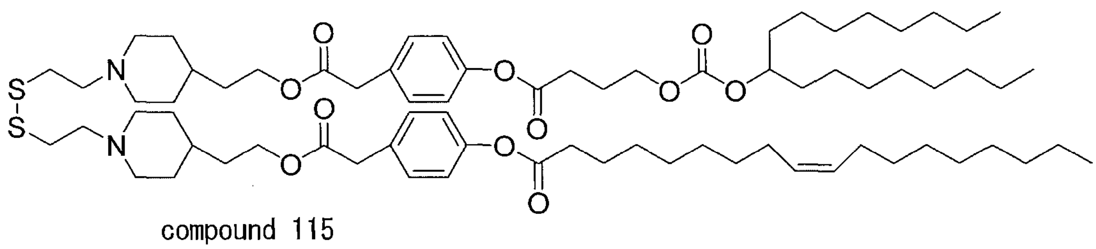

compound 115

<$^1$H-NMR (600 MHz, DMSO-$d_6$) of compound 115>

δ: 0.82-0.94 (m, 9H), 1.14-1.69 (m, 64H), 1.98-2.09 (m, 2H), 2.10-2.18 (m, 4H), 2.32-2.71 (m, 20H), 3.71 (s, 4H), 4.13 (t, 4H), 4.21 (t, 2H), 4.38-4.53 (m, 1H), 5.28-5.37 (m, 2H), 7.13-7.22 (m, 8H)

[Example 116] Synthesis of compound 116

Using intermediate 41 and intermediate 1-7 and in the same manner as in the synthesis of compound 1 described in Example 1-1, compound 116 was synthesized.

[Chem. 287]

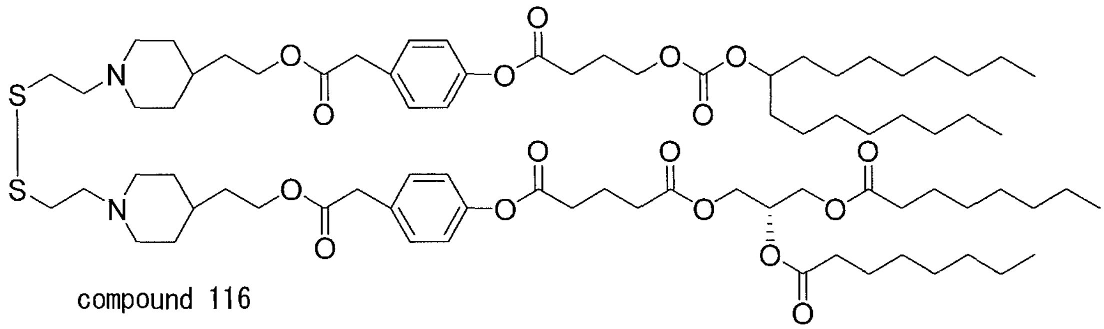

compound 116

<$^1$H-NMR (600 MHz, DMSO-$d_6$) of compound 116>

δ: 0.82-0.94 (m, 12H), 1.18-1.71 (m, 62H), 1.99-2.11 (m, 2H), 2.17-2.68 (m, 28H), 3.71 (s, 4H), 4.13 (t, 4H), 4.21 (t, 2H), 4.30 (d, 4H), 4.38-4.54 (m, 1H), 5.74-5.88 (m, 1H), 7.13-7.22 (m, 8H)

[Example 117] Synthesis of compound 117

Using intermediate 41 and intermediate 1-2 and in the same manner as in the synthesis of compound 1 described in Example 1-1, compound 117 was synthesized.

[Chem. 288]

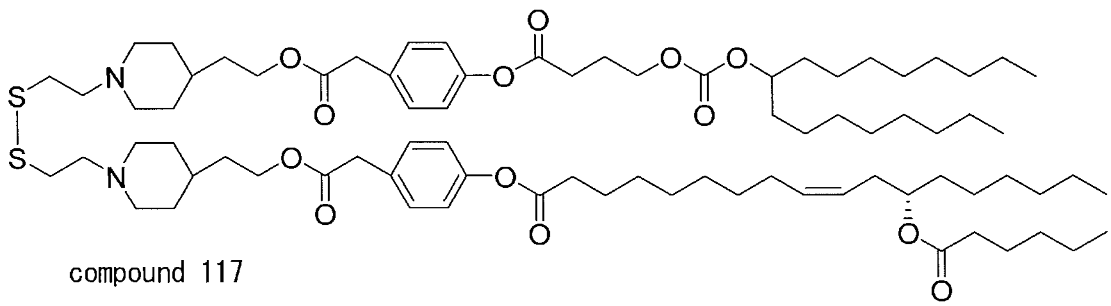

compound 117

<$^1$H-NMR (600 MHz, DMSO-$d_6$) of compound 117>

δ: 0.82-0.94 (m, 12H), 1.15-1.69 (m, 68H), 2.01-2.09 (m, 3H), 2.11-2.19 (m, 2H), 2.25-2.71 (m, 23H), 3.71 (s, 4H), 4.13 (t, 4H), 4.21 (t, 2H), 4.41-4.53 (m, 1H), 4.53-4.64 (m, 1H), 5.26-5.38 (m, 2H), 7.13-7.22 (m, 8H)

[Example 118] Synthesis of compound 118a and compound 118

<Synthesis of intermediate 42>

Intermediate 42 was synthesized by the following synthesis pathway.

[Chem. 289]

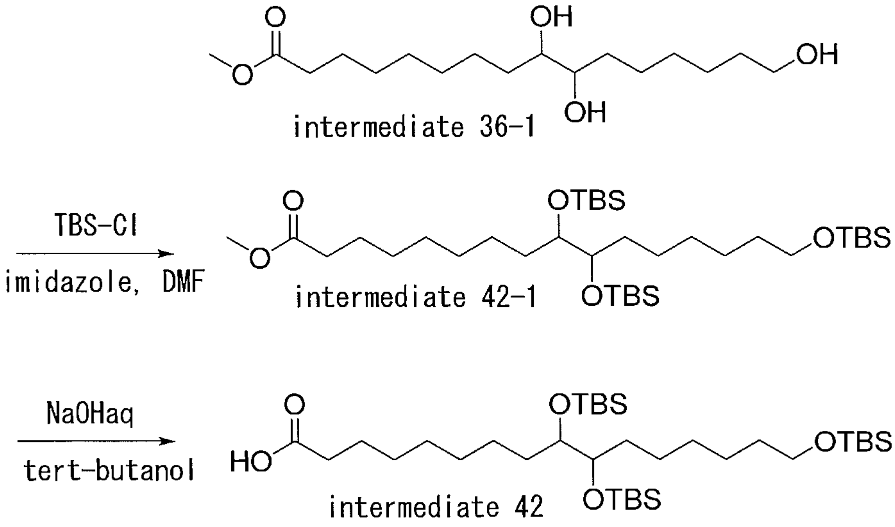


intermediate 36-1

intermediate 42-1

intermediate 42

<Synthesis of intermediate 42-1>

Intermediate 36-1 (1.00 g, 3.14 mmol) obtained by the synthesis described in Example 100, and imidazole (1.28 g, 18.8 mmol) were dissolved in dimethylformamide (10.5 mL) at room temperature. To the obtained mixture was added TBS-Cl (2.27 g, 15.1 mmol) and the mixture was reacted at room temperature for 1 hr. Thereafter, to the reaction solution was added chloroform, and the mixture was washed with 5 wt% sodium hydrogen phosphate aqueous solution, and ion-exchanged water, and dehydrated by adding sodium sulfate. Sodium sulfate was removed by filtration, and the filtrate was concentrated by an evaporator to give 1.66 g of intermediate 42-1.

<Synthesis of intermediate 42>

Intermediate 42-1 (2.80 g) was dissolved in tert-butanol (10.4 mL) at room temperature. To the obtained mixture was added 2 M NaOH aqueous solution (3.84 g) and the mixture was reacted at room temperature for 8 hr. Thereafter, ethyl acetate was added and the obtained mixture was washed with 7 wt% sodium hydrogen carbonate aqueous solution, and 20 wt% saline, and dehydrated by adding sodium sulfate. Sodium sulfate was removed by filtration, and the filtrate was concentrated by an evaporator to give 2.04 g of intermediate 42.

<Synthesis of compound 118a>

Using intermediate 42 and intermediate 1-1 and in the same manner as in the synthesis of compound 1 described in Example 1-1, compound 118a was synthesized.

[Chem. 290]

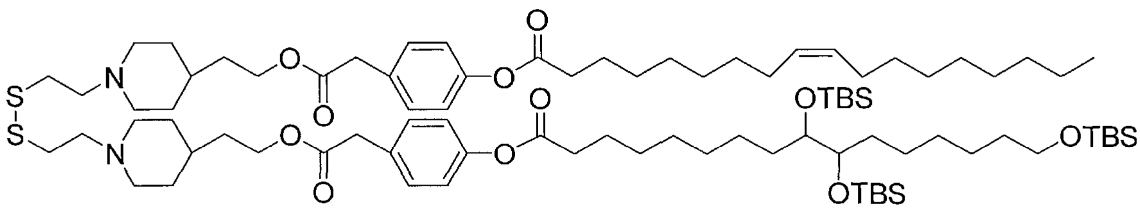

compound 118a

<$^1$H-NMR (600 MHz, DMSO-$d_6$) of compound 118a>

δ: 0.21 (s, 18H), 0.82-0.94 (m, 3H), 0.98 (s, 27H), 1.18-1.75 (m, 58H), 2.07-2.18 (m, 4H), 2.32-2.72 (m, 20H), 3.33-3.46 (m, 2H), 3.71 (s, 4H), 3.77 (t, 2H), 4.13 (t, 4H), 5.27-5.36 (m, 2H), 7.13-7.22 (m, 8H)

<Synthesis of compound 118>

Compound 118a (400 mg, 0.252 mmol) was dissolved in isopropanol (6.40 mL) at room temperature. To the obtained mixture was added p-toluenesulfonic acid monohydrate (173 mg, 0.910 mmol) and the mixture was reacted at room temperature for 7 hr. Thereafter, to the reaction solution was added chloroform (6.40 mL), and the mixture was washed with 5 wt% sodium hydrogen carbonate aqueous solution, and 20 wt% saline, and dehydrated by adding sodium sulfate. Sodium sulfate was removed by filtration, and the filtrate was concentrated by an evaporator. The obtained residue was purified by silica gel column chromatography to give 169 mg of compound 118.

[Chem. 291]

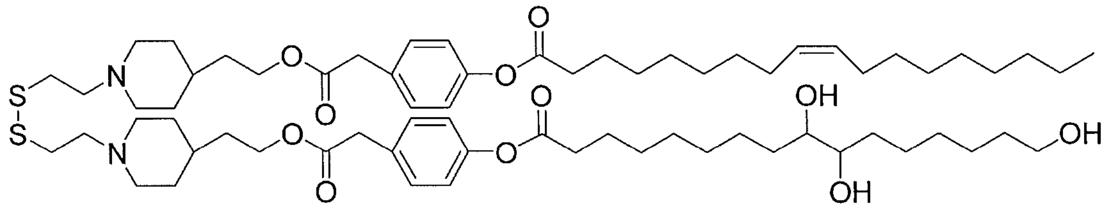

compound 118

<$^1$H-NMR (600 MHz, DMSO-$d_6$) of compound 118>

δ: 0.82-0.94 (m, 3H), 1.18-1.75 (m, 58H), 2.07-2.18 (m, 4H), 2.32-2.72 (m, 20H), 3.33-3.46 (m, 2H), 3.62 (t, 2H), 3.71 (s, 4H), 4.13 (t, 4H), 5.27-5.36 (m, 2H), 7.13-7.22 (m, 8H)

[Example 119] Synthesis of compound 119a and compound 119

<Synthesis of compound 119a>

Using intermediate 42 and intermediate 1-7 and in the same manner as in the synthesis of compound 1 described in Example 1-1, compound 119a was synthesized.

[Chem. 292]

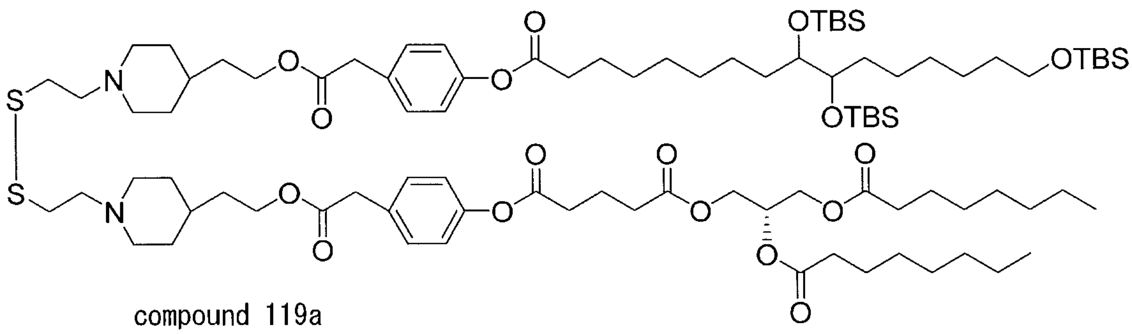

compound 119a

<$^1$H-NMR (600 MHz, DMSO-$d_6$) of compound 119a>

δ: 0.21 (s, 18H), 0.82-0.94 (m, 6H), 0.98 (s, 27H), 1.16-1.72 (m, 56H), 2.18-2.69 (m, 28H), 3.32-3.45 (m, 2H), 3.71 (s, 4H), 3.77 (t, 2H), 4.07-4.48 (m, 8H), 5.77-5.90 (m, 1H), 7.13-7.22 (m, 8H)

<Synthesis of compound 119>

Using compound 119a and in the same manner as in the synthesis of compound 118 described in Example 118, compound 119 was synthesized.

[Chem. 293]

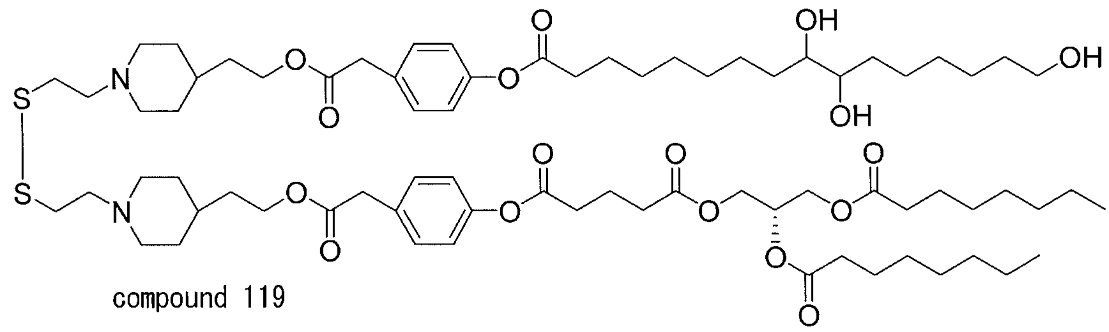

compound 119

<$^1$H-NMR (600 MHz, DMSO-$d_6$) of compound 119>

δ: 0.82-0.94 (m, 6H), 1.16-1.72 (m, 56H), 2.18-2.69 (m, 28H), 3.32-3.45 (m, 2H), 3.62 (t, 2H), 3.71 (s, 4H), 4.07-4.48 (m, 8H), 5.77-5.90 (m, 1H), 7.13-7.22 (m, 8H)

[Example 120] Synthesis of compound 120a and compound 120

<Synthesis of compound 120a>

Using intermediate 42 and intermediate 1-2 and in the same manner as in the synthesis of compound 1 described in Example 1-1, compound 120a was synthesized.

[Chem. 294]

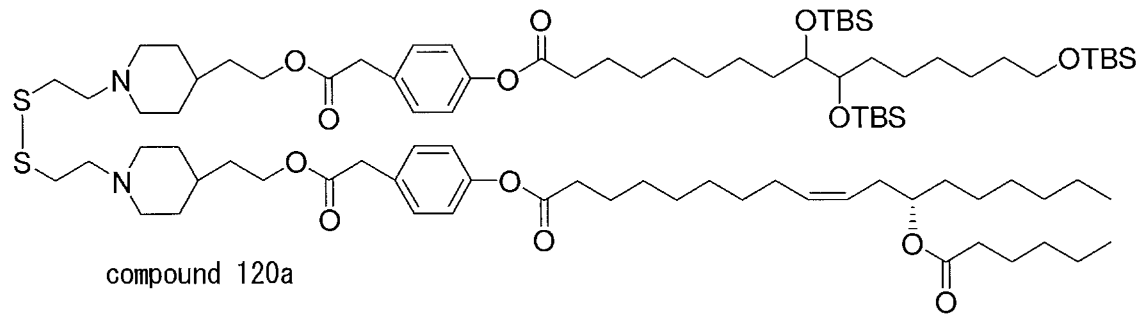

compound 120a

<$^1$H-NMR (600 MHz, DMSO-$d_6$) of compound 120a>

δ: 0.21 (s, 18H), 0.82-0.94 (m, 6H), 0.98 (s, 27H), 1.15-1.72 (m, 62H), 2.02-2.11 (m, 1H), 2.12-2.22 (m, 2H), 2.27-2.71 (m, 23H), 3.32-3.45 (m, 2H), 3.71 (s, 4H), 3.77 (t, 2H), 4.13 (t, 4H), 4.53-4.63 (m, 1H), 5.25-5.37 (m, 2H), 7.13-7.22 (m, 8H)

<Synthesis of compound 120>

Using compound 120a and in the same manner as in the synthesis of compound 118 described in Example 118, compound 120 was synthesized.

[Chem. 295]

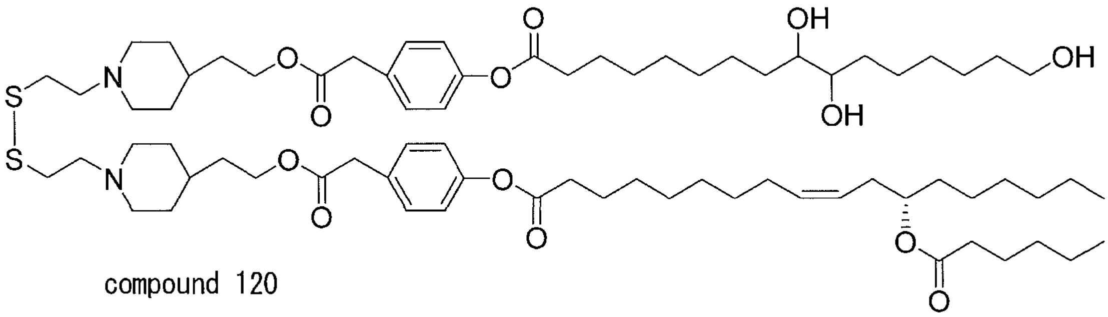

compound 120

<$^1$H-NMR (600 MHz, DMSO-$d_6$) of compound 120>

δ: 0.82-0.94 (m, 6H), 1.15-1.72 (m, 62H), 2.02-2.11 (m, 1H), 2.12-2.22 (m, 2H), 2.27-2.71 (m, 23H), 3.32-3.45 (m, 2H), 3.62 (t, 2H), 3.71 (s, 4H), 4.13 (t, 4H), 4.53-4.63 (m, 1H), 5.25-5.37 (m, 2H), 7.13-7.22 (m, 8H)

[Example 121] Synthesis of compound 121

<Synthesis of intermediate 43>

Diethylamine (562 mg, 7.68 mmol), glutaric anhydride (963 mg, 8.44 mmol), and DMAP (188 mg, 1.54 mmol) were dissolved in chloroform (8.43 g) at room temperature, and the mixture was reacted at room temperature for 1 hr. The subsequent steps were performed in the same manner as in the synthesis of intermediate 11 described in Example 15 to obtain 1.39 g of intermediate 43.

[Chem. 296]

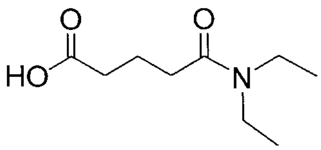

intermediate 43

<Synthesis of compound 121>

Using intermediate 43 and intermediate 1-1 and in the same manner as in the synthesis of compound 1 described in Example 1-1, compound 121 was synthesized.

[Chem. 297]

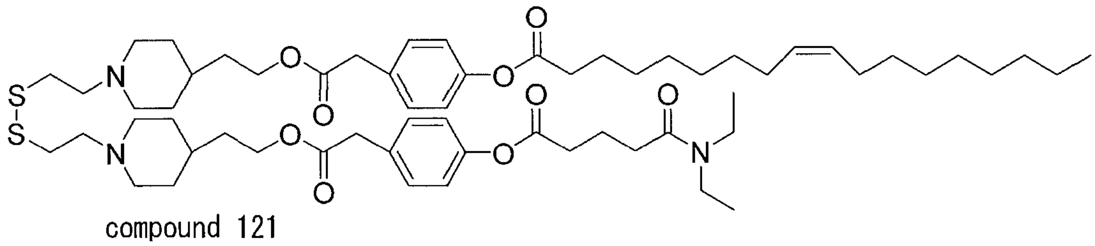

compound 121

<$^1$H-NMR (600 MHz, DMSO-$d_6$) of compound 121>

δ: 0.82-0.94 (m, 3H), 1.08 (t, 6H), 1.18-1.73 (m, 36H), 2.08-2.20 (m, 4H), 2.28-2.71 (m, 24H), 3.34 (q, 4H), 3.71 (s, 4H), 4.13 (t, 4H), 5.24-5.38 (m, 2H), 7.13-7.22 (m, 8H)

[Example 122] Synthesis of compound 122

Using intermediate 43 and intermediate 1-7 and in the same manner as in the synthesis of compound 1 described in Example 1-1, compound 122 was synthesized.

[Chem. 298]

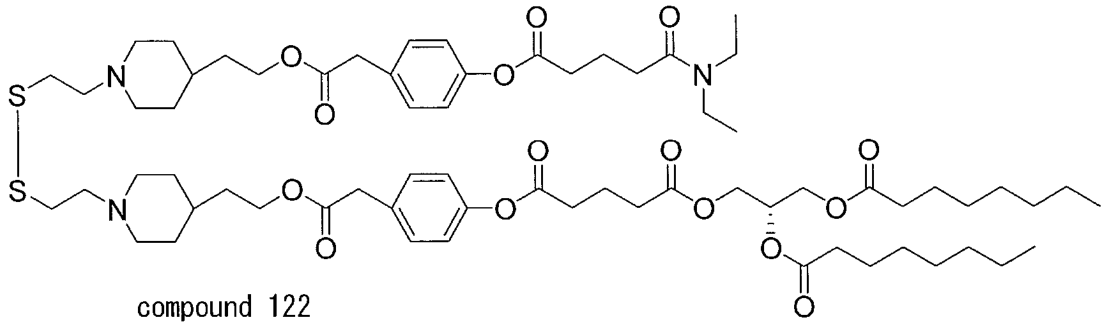

compound 122

<$^1$H-NMR (600 MHz, DMSO-$d_6$) of compound 122>

δ: 0.82-0.94 (m, 6H), 1.08 (t, 6H), 1.14-1.70 (m, 32H), 2.06-2.71 (m, 34H), 3.34 (q, 4H), 3.71 (s, 4H), 4.05-4.48 (m, 8H), 5.77-5.91 (m, 1H), 7.13-7.22 (m, 8H)

[Example 123] Synthesis of compound 123

Using intermediate 43 and intermediate 1-2 and in the same manner as in the synthesis of compound 1 described in Example 1-1, compound 123 was synthesized.

[Chem. 299]

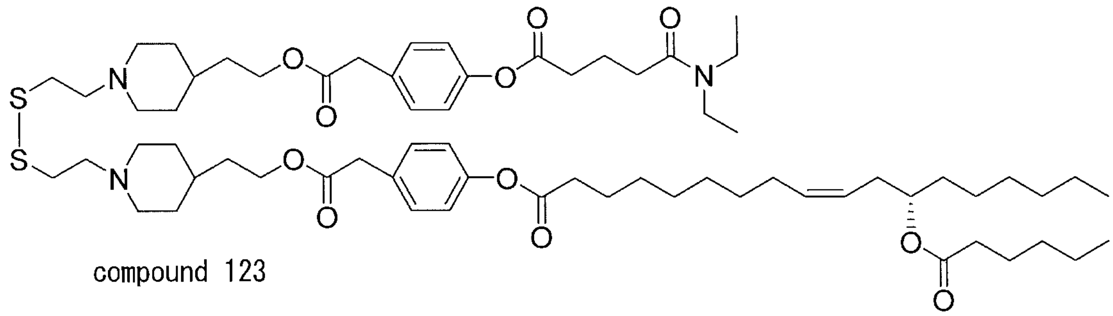

compound 123

<$^1$H-NMR (600 MHz, DMSO-$d_6$) of compound 123>

δ: 0.82-0.94 (m, 6H), 1.08 (t, 6H), 1.14-1.71 (m, 40H), 2.00-2.19 (m, 5H), 2.23-2.71 (m, 25H), 3.34 (q, 4H), 3.71 (s, 4H), 4.13 (t, 4H), 4.52-4.63 (m, 1H), 5.25-5.41 (m, 2H), 7.13-7.22 (m, 8H)

[Example 124] Synthesis of compound 124

<Synthesis of intermediate 44>

Dihexylamine (1.01 g, 5.40 mmol), glutaric anhydride (1.25 g, 10.8 mmol), and triethylamine (1.65 g, 16.2 mmol) were dissolved in chloroform (10.1 g) at room temperature, and the mixture was reacted at room temperature for 1 hr. The subsequent steps were performed in the same manner as in the synthesis of intermediate 11 described in Example 15 to obtain 1.45 g of intermediate 44.

[Chem. 300]

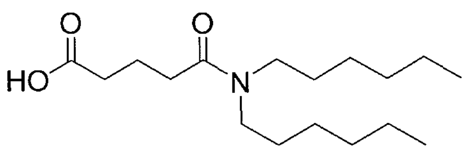

intermediate 44

<Synthesis of compound 124>

Using intermediate 44 and intermediate 1-1 and in the same manner as in the synthesis of compound 1 described in Example 1-1, compound 124 was synthesized.

[Chem. 301]

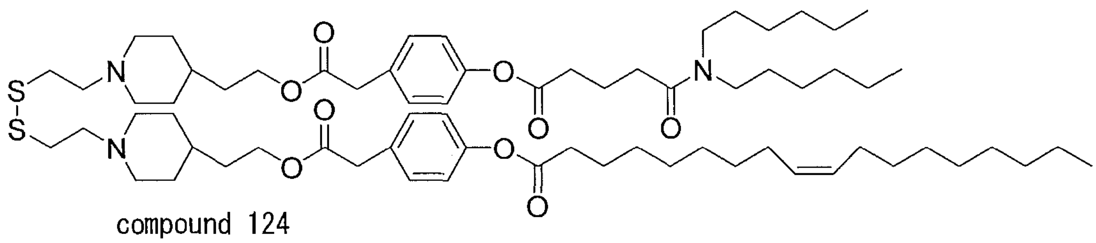

compound 124

<$^1$H-NMR (600 MHz, DMSO-$d_6$) of compound 124>

δ: 0.86-0.89 (m, 9H), 1.22-1.42 (m, 38H), 1.54-1.76 (m, 14H), 1.91-1.98 (m, 4H), 1.99-2.14 (m, 6H), 2.32-2.37 (m, 2H), 2.52-2.56 (m, 4H), 2.62-2.66 (m, 4H), 2.78-2.92 (m, 8H), 3.16-3.20 (t, 4H), 3.59 (s, 4H), 4.11-4.13 (t, 4H), 5.30-5.38 (m, 2H), 7.02-7.04 (m, 4H), 7.26-7.29 (m, 4H)

[Example 125] Synthesis of compound 125

Using intermediate 44 and intermediate 1-7 and in the same manner as in the synthesis of compound 1 described in Example 1-1, compound 125 was synthesized.

[Chem. 302]

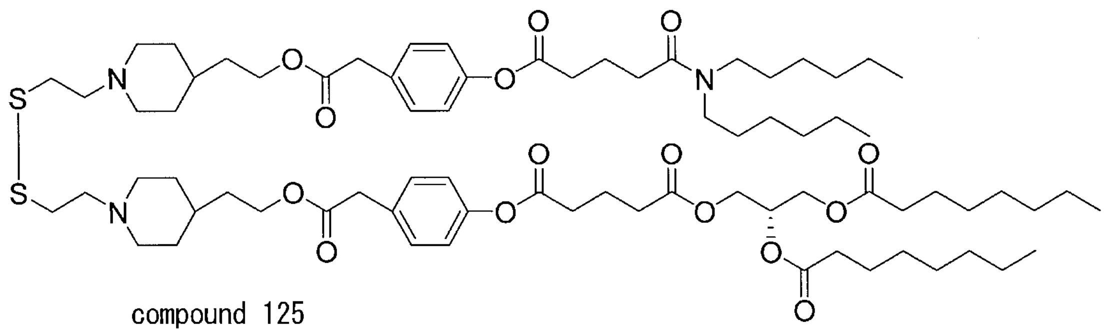

compound 125

<$^1$H-NMR (600 MHz, DMSO-$d_6$) of compound 125>

δ: 0.86-0.89 (m, 12H), 1.22-1.42 (m, 34H), 1.54-1.76 (m, 16H), 1.91-1.98 (m, 4H), 1.99-2.14 (m, 4H), 2.27-2.35 (m, 6H), 2.46-2.52 (m, 4H), 2.61-2.67 (m, 6H), 2.78-2.95 (m, 8H), 3.16-3.20 (t, 4H), 3.59 (s, 4H), 4.09-4.21 (m, 6H), 4.27-4.35 (m, 2H), 5.25-5.30 (m, 1H), 7.02-7.04 (m, 4H), 7.26-7.29 (m, 4H)

[Example 126] Synthesis of compound 126

Using intermediate 44 and intermediate 1-2 and in the same manner as in the synthesis of compound 1 described in Example 1-1, compound 126 was synthesized.

[Chem. 303]

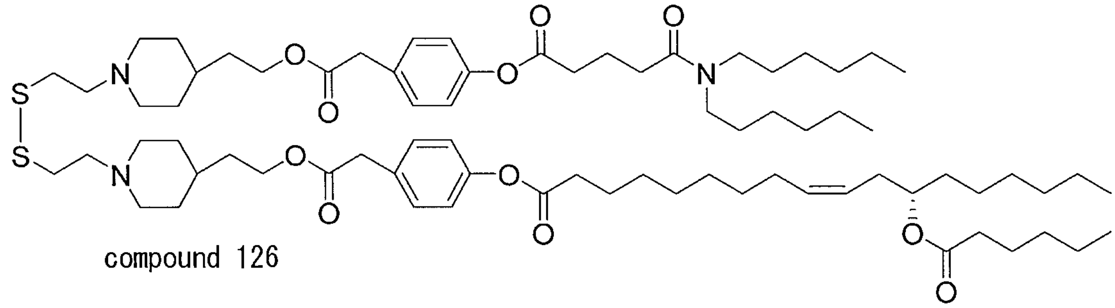

compound 126

<$^{1}$H-NMR (600 MHz, DMSO-$d_6$) of compound 126>

δ: 0.86-0.90 (m, 12H), 1.22-1.42 (m, 38H), 1.54-1.76 (m, 18H), 1.91-1.98 (m, 4H), 2.00-2.14 (m, 4H), 2.23-2.34 (m, 6H), 2.52-2.56 (m, 4H), 2.61-2.67 (m, 4H), 2.79-2.92 (m, 8H), 3.16-3.20 (t, 4H), 3.59 (s, 4H), 4.11-4.14 (t, 4H), 4.85-4.90 (m, 1H), 5.31-5.40 (m, 1H), 5.44-5.49 (m, 1H), 7.02-7.04 (m, 4H), 7.26-7.30 (m, 4H)

[Example 127] Synthesis of compound 127a and compound 127

<Synthesis of intermediate 45>

Intermediate 45 was synthesized by the following synthesis pathway.

[Chem. 304]

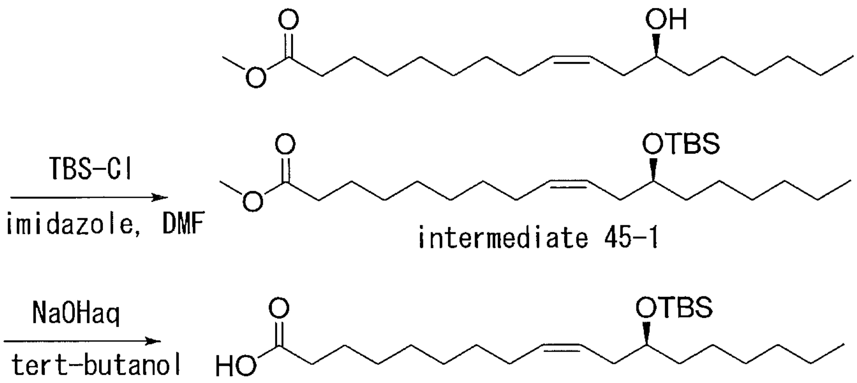


intermediate 45-1

intermediate 45

<Synthesis of intermediate 45-1>

Methyl ricinoleate (1.00 g, 3.20 mmol), and imidazole (436 mg, 6.40 mmol) were dissolved in dimethylformamide (10.5 mL) at room temperature. To the obtained mixture was added TBS-Cl (772 mg, 5.12 mmol) and the mixture was reacted at room temperature for 7 hr. Thereafter, to the reaction solution was added chloroform, and the mixture was washed with 5 wt% sodium hydrogen phosphate aqueous solution, and ion-exchanged water, and dehydrated by adding sodium sulfate. Sodium sulfate was removed by filtration, and the filtrate was concentrated by an evaporator to give 1.00 g of intermediate 45-1.

<Synthesis of intermediate 45>

Intermediate 45-1 (1.00 g, 2.34 mmol) was dissolved in tert-butanol (4.95 mL) at room temperature. To the obtained mixture was added 2 M NaOH aqueous solution (1.83 g) and the mixture was reacted at room temperature for 8 hr. Thereafter, to the reaction solution was added ethyl acetate, and the mixture was washed with 7 wt% sodium hydrogen carbonate aqueous solution, and 20 wt% saline, and dehydrated by adding sodium sulfate. Sodium sulfate was removed by filtration, and the filtrate was concentrated by an evaporator to give 774 mg of intermediate 45.

<Synthesis of compound 127a>

Using intermediate 45 and intermediate 1-1 and in the same manner as in the synthesis of compound 1 described in Example 1-1, compound 127a was synthesized.

[Chem. 305]

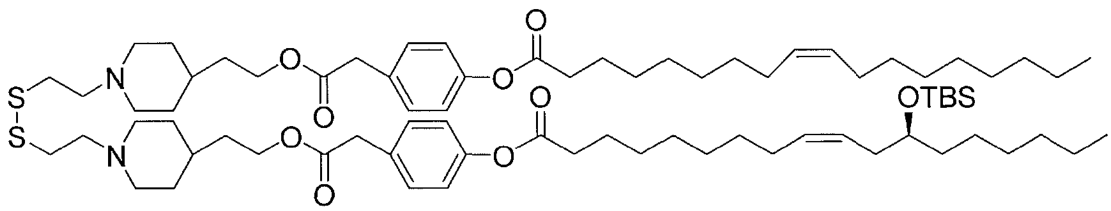

compound 127a

<$^1$H-NMR (600 MHz, DMSO-$d_6$) of compound 127a>

δ: 0.21 (s, 6H), 0.86-0.89 (m, 6H), 0.98 (s, 9H), 1.22-1.42 (m, 44H), 1.54-1.76 (m, 12H), 1.91-1.98 (m, 6H), 1.99-2.16 (m, 6H), 2.52-2.56 (m, 4H), 2.62-2.66 (m, 4H), 2.78-2.92 (m, 8H), 3.51-3.59 (m, 5H), 4.11-4.13 (t, 4H), 5.30-5.38 (m, 3H), 5.50-5.58 (m, 1H), 7.02-7.04 (m, 4H), 7.26-7.29 (m, 4H)

<Synthesis of compound 127>

Compound 127a (400 mg, 0.307 mmol) was dissolved in isopropanol (6.40 mL) at room temperature. To the obtained mixture was added p-toluenesulfonic acid monohydrate (87.5 mg, 0.460 mmol) and the mixture was reacted at room temperature for 7 hr. Thereafter, to the reaction solution was added chloroform (6.40 mL), and the mixture was washed with 5 wt% sodium hydrogen carbonate aqueous solution, and 20 wt% saline, and dehydrated by adding sodium sulfate. Sodium sulfate was removed by filtration, and the filtrate was concentrated by an evaporator. The obtained residue was purified by silica gel column chromatography to give 204 mg of compound 127.

[Chem. 306]

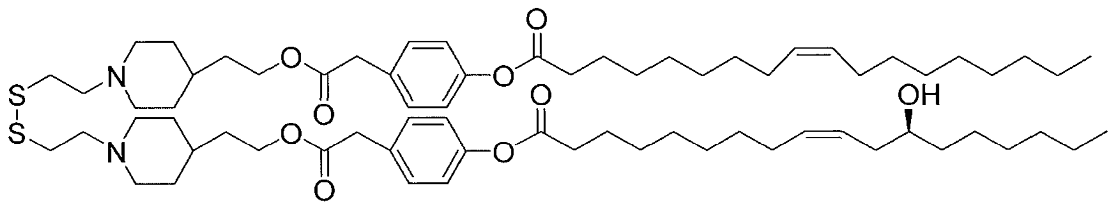

compound 127

<$^1$H-NMR (600 MHz, DMSO-$d_6$) of compound 127>

δ: 0.86-0.89 (m, 6H), 1.22-1.42 (m, 44H), 1.54-1.76 (m, 12H), 1.91-1.98 (m, 6H), 1.99-2.16 (m, 6H), 2.52-2.56 (m, 4H), 2.62-2.66 (m, 4H), 2.78-2.92 (m, 8H), 3.51-3.59 (m, 5H), 4.11-4.13 (t, 4H), 5.30-5.38 (m, 3H), 5.50-5.58 (m, 1H), 7.02-7.04 (m, 4H), 7.26-7.29 (m, 4H)

[Example 128] Synthesis of compound 128a and compound 128

<Synthesis of compound 128a>

Using intermediate 45 and intermediate 1-7 and in the same manner as in the synthesis of compound 1 described in Example 1-1, compound 128a was synthesized.

[Chem. 307]

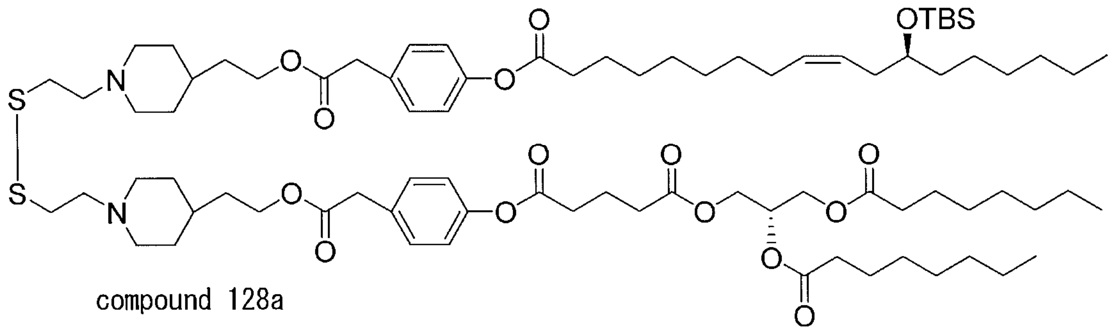

compound 128a

<¹H-NMR (600 MHz, DMSO-$d_6$) of compound 128a>

δ: 0.21 (s, 6H), 0.86-0.89 (m, 9H), 0.98 (s, 9H), 1.22-1.42 (m, 40H), 1.54-1.76 (m, 14H), 1.91-1.98 (m, 6H), 1.99-2.16 (m, 4H), 2.27-2.35 (m, 4H), 2.46-2.51 (t, 4H), 2.61-2.67 (m, 6H), 2.78-2.95 (m, 8H), 3.51-3.59 (m, 5H), 4.09-4.21 (m, 6H), 4.27-4.35 (m, 2H), 5.25-5.38 (m, 2H), 5.50-5.58 (m, 1H), 7.02-7.04 (m, 4H), 7.26-7.29 (m, 4H)

<Synthesis of compound 128>

Using compound 128a and in the same manner as in the synthesis of compound 127 described in Example 127, compound 128 was synthesized.

[Chem. 308]

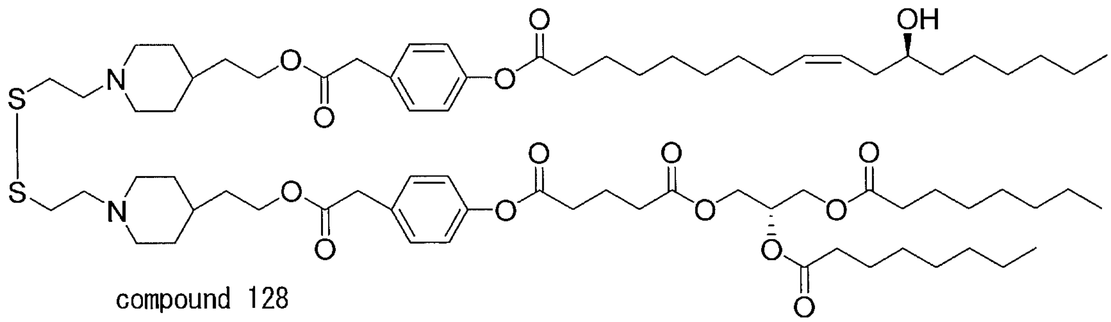

compound 128

<¹H-NMR (600 MHz, DMSO-$d_6$) of compound 128>

δ: 0.86-0.89 (m, 9H), 1.22-1.42 (m, 40H), 1.54-1.76 (m, 14H), 1.91-1.98 (m, 6H), 1.99-2.16 (m, 4H), 2.27-2.35 (m, 4H), 2.46-2.51 (t, 4H), 2.61-2.67 (m, 6H), 2.78-2.95 (m, 8H), 3.51-3.59 (m, 5H), 4.09-4.21 (m, 6H), 4.27-4.35 (m, 2H), 5.25-5.38 (m, 2H), 5.50-5.58 (m, 1H), 7.02-7.04 (m, 4H), 7.26-7.29 (m, 4H)

[Example 129] Synthesis of compound 129a and compound 129

<Synthesis of compound 129a>

Using intermediate 45 and intermediate 1-2 and in the same manner as in the synthesis of compound 1 described in Example 1-1, compound 129a was synthesized.

[Chem. 309]

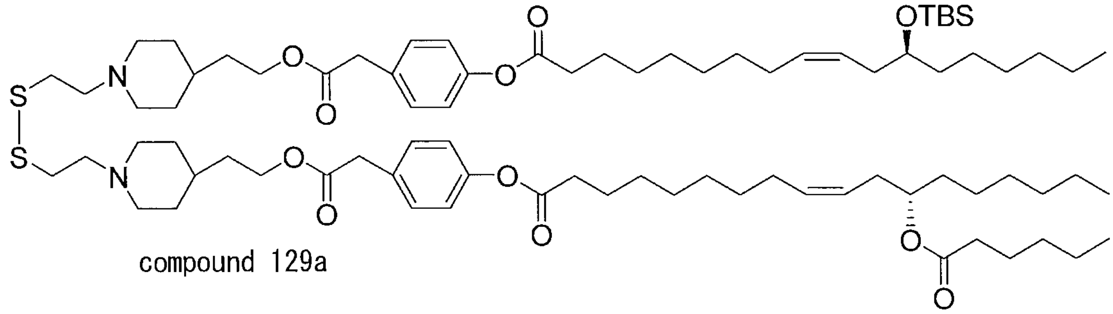

compound 129a

<$^1$H-NMR (600 MHz, DMSO-$d_6$) of compound 129a>

δ: 0.21 (s, 6H), 0.86-0.90 (m, 9H), 0.98 (s, 9H), 1.22-1.42 (m, 44H), 1.54-1.76 (m, 16H), 1.91-1.98 (m, 6H), 2.00-2.16 (m, 4H), 2.23-2.33 (m, 4H), 2.52-2.56 (m, 4H), 2.61-2.67 (m, 4H), 2.79-2.92 (m, 8H), 3.51-3.59 (m, 5H), 4.11-4.14 (t, 4H), 4.85-4.90 (m, 1H), 5.31-5.40 (m, 2H), 5.44-5.58 (m, 2H), 7.02-7.04 (m, 4H), 7.26-7.30 (m, 4H)

<Synthesis of compound 129>

Using compound 129a and in the same manner as in the synthesis of compound 127 described in Example 127, compound 129 was synthesized.

[Chem. 310]

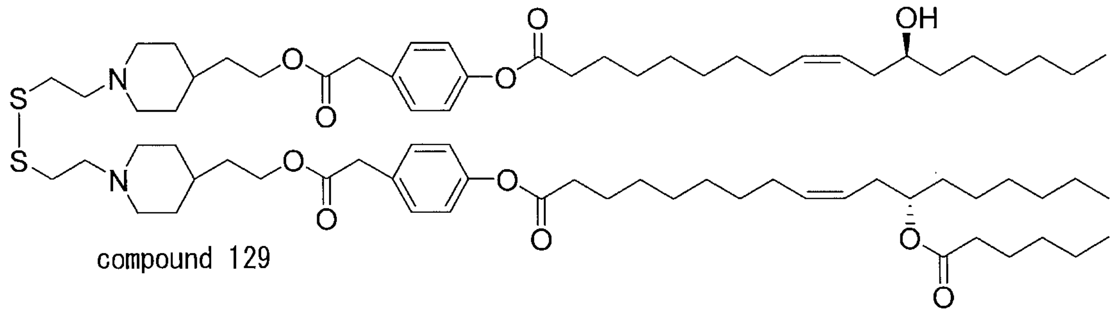

compound 129

<$^1$H-NMR (600 MHz, DMSO-$d_6$) of compound 129>

δ: 0.86-0.90 (m, 9H), 1.22-1.42 (m, 44H), 1.54-1.76 (m, 16H), 1.91-1.98 (m, 6H), 2.00-2.16 (m, 4H), 2.23-2.33 (m, 4H), 2.52-2.56 (m, 4H), 2.61-2.67 (m, 4H), 2.79-2.92 (m, 8H), 3.51-3.59 (m, 5H), 4.11-4.14 (t, 4H), 4.85-4.90 (m, 1H), 5.31-5.40 (m, 2H), 5.44-5.58 (m, 2H), 7.02-7.04 (m, 4H), 7.26-7.30 (m, 4H)

[Example 130] Synthesis of compound 130

<Synthesis of intermediate 47>

To 5-aminopentanoic acid (1.00 g, 8.54 mmol) were added chloroform (5.00 g), triethylamine (3.46 g, 34.2 mmol), and 1-iodooctane (6.15 g, 25.6 mmol), and the mixture was reacted at room temperature for 12 hr. After the reaction, 1 M potassium hydroxide aqueous solution (5.00 g) was added and the mixture was stirred and neutralized with 1 M hydrochloric acid. The solution was washed with 5 wt% saline, and concentrated by an evaporator, and the concentrate was subjected to silica gel column purification using ethanol/chloroform to give 2.33 g of intermediate 47.

[Chem. 311]

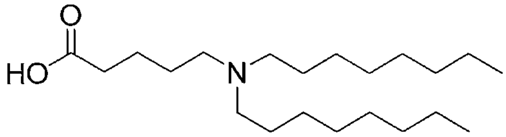

intermediate 47

<Synthesis of compound 130>

Using intermediate 47 and intermediate 1-1 and in the same manner as in the synthesis of compound 1 described in Example 1-1, compound 130 was synthesized.

[Chem. 312]

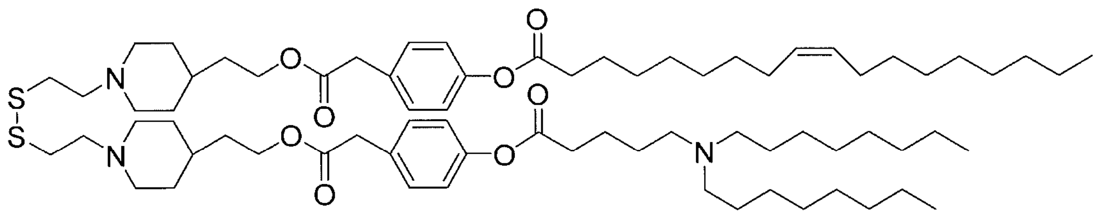

compound 130

<$^1$H-NMR (600 MHz, DMSO-$d_6$) of compound 130>

δ: 0.88 (m, 9H), 1.26-1.56 (m, 64H), 2.41-2.66 (m, 24H), 3.01 (t,6H), 3.71 (s, 4H), 4.11-4.14 (m, 4H), 5.34-5.37 (m, 2H), 7.10-7.20 (m, 8H)

[Example 131] Synthesis of compound 131

Using intermediate 47 and intermediate 1-7 and in the same manner as in the synthesis of compound 1 described in Example 1-1, compound 131 was synthesized.

[Chem. 313]

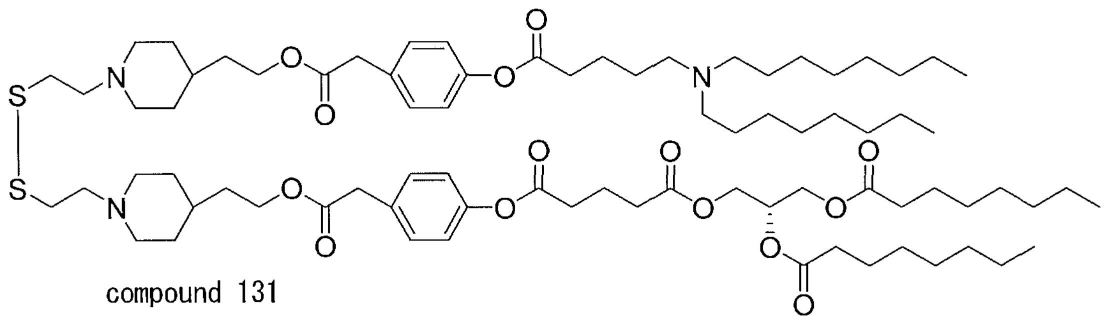

compound 131

<$^1$H-NMR (600 MHz, DMSO-$d_6$) of compound 131>

δ: 0.88-0.90 (m, 12H), 1.20-1.65 (m, 62H), 2.29-2.66 (m, 28H), 3.01 (t, 6H), 3.71 (s, 4H), 4.17-4.45 (m, 8H), 5.85 (m, 1H), 7.10-7.20 (m, 8H)

[Example 132] Synthesis of compound 132

Using intermediate 47 and intermediate 1-2 and in the same manner as in the synthesis of compound 1 described in Example 1-1, compound 132 was synthesized.

[Chem. 314]

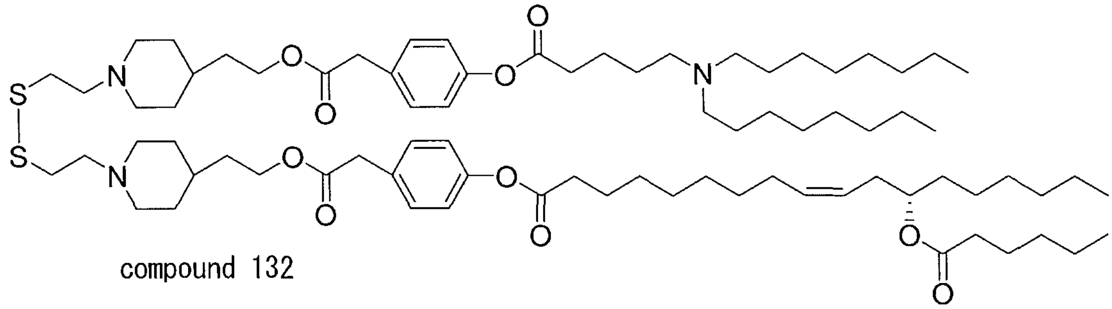

compound 132

<$^1$H-NMR (600 MHz, DMSO-$d_6$) of compound 132>

δ: 0.88 (m, 16H), 1.26-1.56 (m, 66H), 2.41-2.66 (m, 24H), 3.01 (t, 6H), 3.71 (s, 4H), 4.11-4.14 (m, 4H), 4.60-4.65 (m, 1H), 5.34-5.37 (m, 2H), 7.10-7.20 (m, 8H)

[Example 133] Synthesis of compound 133a and compound 133

<Synthesis of intermediate 48>

Intermediate 48 was synthesized by the following synthesis pathway.

[Chem. 315]

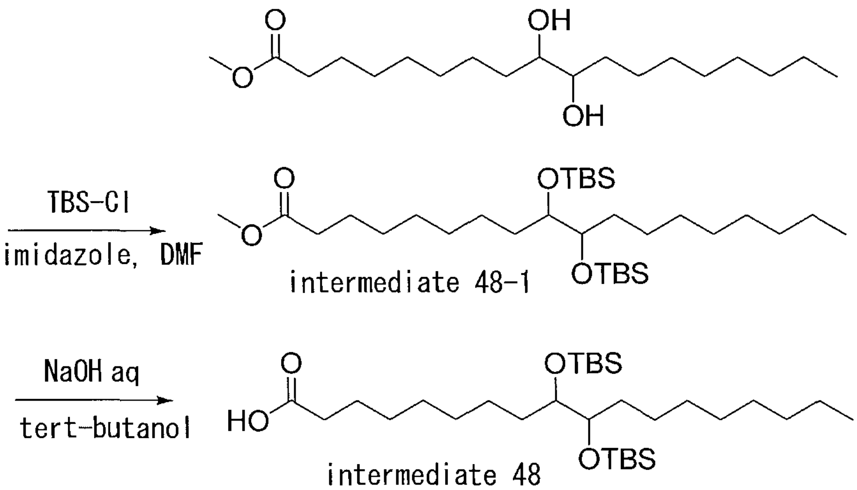


<Synthesis of intermediate 48-1>

Methyl 9,10-dihydroxystearate (1.00 g, 3.03 mmol), and imidazole (824 mg, 12.1 mmol) were dissolved in dimethylformamide (10.5 mL) at room temperature. To the obtained mixture was added TBS-Cl (1.46 g, 9.68 mmol) and the mixture was reacted at room temperature for 1 hr. Thereafter, to the reaction solution was added chloroform, and the mixture was washed with 5 wt% sodium hydrogen phosphate aqueous solution, and ion-exchanged water, and dehydrated by adding sodium sulfate. Sodium sulfate was removed by filtration, and the filtrate was concentrated by an evaporator to give 1.52 g of intermediate 48-1.

<Synthesis of intermediate 48>

Intermediate 48-1 (1.50 g) was dissolved in tert-butanol (11.3 g) at room temperature. To the obtained mixture was added 2 M NaOH aqueous solution (2.37 g) and the mixture was reacted at room temperature for 8 hr. Thereafter, to the reaction solution was added ethyl acetate, and the mixture was washed with 7 wt% sodium hydrogen carbonate aqueous solution, and 20 wt% saline, and dehydrated by adding sodium sulfate. Sodium sulfate was removed by filtration, and the filtrate was concentrated by an evaporator to give 1.29 g of intermediate 48.

<Synthesis of compound 133a>

Using intermediate 48 and intermediate 1-1 and in the same manner as in the synthesis of compound 1 described in Example 1-1, compound 133a was synthesized.

[Chem. 316]

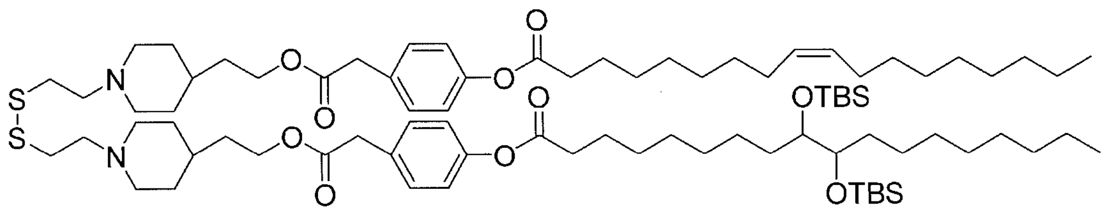

compound 133a

<$^1$H-NMR (600 MHz, DMSO-$d_6$) of compound 133a>

δ: 0.21 (s, 12H), 0.86-0.89 (m, 6H), 0.98 (s, 18H), 1.22-1.42 (m, 50H), 1.54-1.76 (m, 12H), 1.91-1.98 (m, 4H), 1.99-2.10 (m, 4H), 2.52-2.56 (t, 4H), 2.62-2.66 (m, 4H), 2.78-2.92 (m, 8H), 3.35-3.45 (m, 2H).3.59 (s, 4H), 4.11-4.13 (t, 4H), 5.30-5.38 (m, 2H), 7.02-7.04 (m, 4H), 7.26-7.29 (m, 4H)

<Synthesis of compound 133>

Compound 133a (400 mg, 0.278 mmol) was dissolved in isopropanol (6.40 mL) at room temperature. To the obtained mixture was added p-toluenesulfonic acid monohydrate (132 mg, 0.696 mmol) and the mixture was reacted at room temperature for 7 hr. Thereafter, to the reaction solution was added chloroform (6.40 mL), and the mixture was washed with 5 wt% sodium hydrogen carbonate aqueous solution, and 20 wt% saline, and dehydrated by adding sodium sulfate. Sodium sulfate was removed by filtration, and the filtrate was concentrated by an evaporator. The obtained residue was purified by silica gel column chromatography to give 188 mg of compound 133.

[Chem. 317]

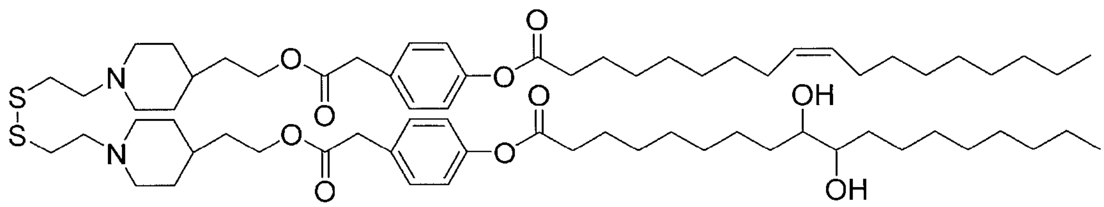

compound 133

<$^1$H-NMR (600 MHz, DMSO-$d_6$) of compound 133> δ: 0.88-0.90 (m, 6H), 1.20-1.61 (m, 62H), 2.29-2.66 (m, 24H), 3.40-3.42 (m, 2H), 3.71 (s, 4H), 4.13-4.15 (m, 4H), 5.34 (m, 2H), 7.10-7.20 (m, 8H)

[Example 134] Synthesis of compound 134a and compound 134

<Synthesis of compound 134a>

Using intermediate 48 and intermediate 1-7 and in the same manner as in the synthesis of compound 1 described in Example 1-1, compound 134a was synthesized.

[Chem. 318]

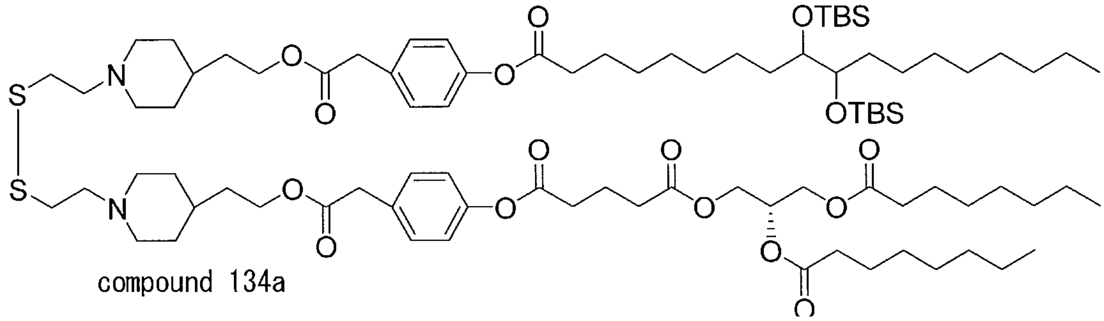

compound 134a

<$^1$H-NMR (600 MHz, DMSO-$d_6$) of compound 134a>

δ: 0.21 (s, 12H), 0.86-0.89 (m, 9H), 0.98 (s, 18H), 1.22-1.42 (m, 46H), 1.54-1.76 (m, 14H), 1.91-1.98 (m, 4H), 1.99-2.10 (m, 2H), 2.27-2.35 (m, 4H), 2.46-2.53 (m, 4H), 2.61-2.67 (m, 6H), 2.78-2.95 (m, 8H), 3.35-3.45 (m, 2H), 3.59 (s, 4H), 4.09-4.21 (m, 6H), 4.27-4.35 (m, 2H), 5.25-5.30 (m, 1H), 7.02-7.04 (m, 4H), 7.26-7.29 (m, 4H)

<Synthesis of compound 134>

Using compound 134a and in the same manner as in the synthesis of compound 133 described in Example 133, compound 134 was synthesized.

[Chem. 319]

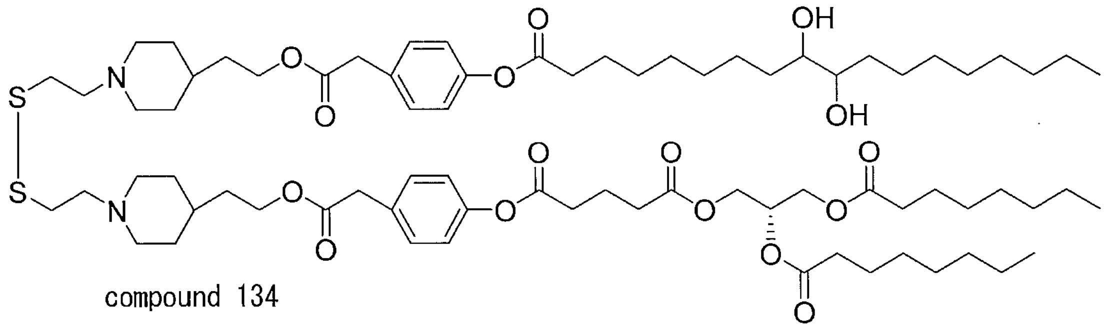

compound 134

<$^1$H-NMR (600 MHz, DMSO-$d_6$) of compound 134>

δ: 0.88 (m, 9H), 1.20-1.53 (m, 50H), 1.62-1.68 (m, 10H), 2.20-2.57 (m, 24H), 2.66 (t, 4H), 3.38-3.42 (m, 2H), 3.71 (s, 4H), 4.12-4.14 (m, 4H), 4.40-4.43 (m, 4H), 5.82-5.87 (m, 1H), 7.12-.7.20 (m, 8H)

[Example 135] Synthesis of compound 135

<Synthesis of compound 135a>

Using intermediate 48 and intermediate 1-2 and in the same manner as in the synthesis of compound 1 described in Example 1-1, compound 135a was synthesized.

[Chem. 320]

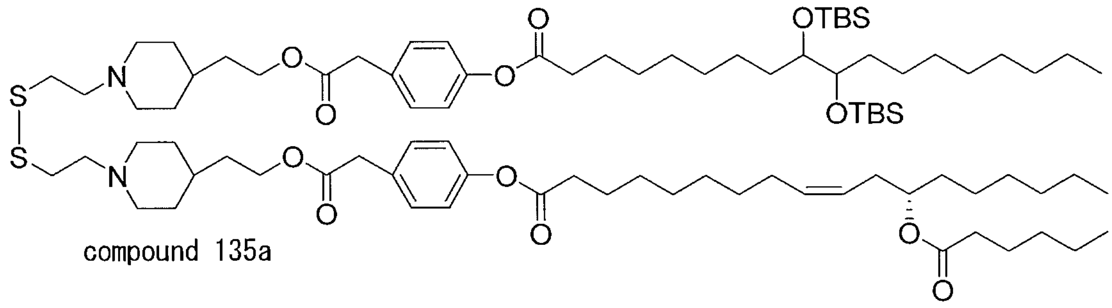

compound 135a

<$^1$H-NMR (600 MHz, DMSO-$d_6$) of compound 135a>

δ: 0.21 (s, 12H), 0.86-0.90 (m, 9H), 0.98 (s, 18H), 1.22-1.42 (m, 50H), 1.54-1.76 (m, 16H), 1.91-1.98 (m, 4H), 2.00-2.06 (m, 2H), 2.23-2.33 (m, 4H), 2.52-2.56 (m, 4H), 2.61-2.67 (m, 4H), 2.79-2.92 (m, 8H), 3.35-3.45 (m, 2H), 3.59 (s, 4H), 4.11-4.14 (t, 4H), 4.85-4.90 (m, 1H), 5.31-5.40 (m, 1H), 5.44-5.49 (m, 1H), 7.02-7.04 (m, 4H), 7.26-7.30 (m, 4H)

<Synthesis of compound 135>

Using compound 135a and in the same manner as in the synthesis of compound 133 described in Example 133, compound 135 was synthesized.

[Chem. 321]

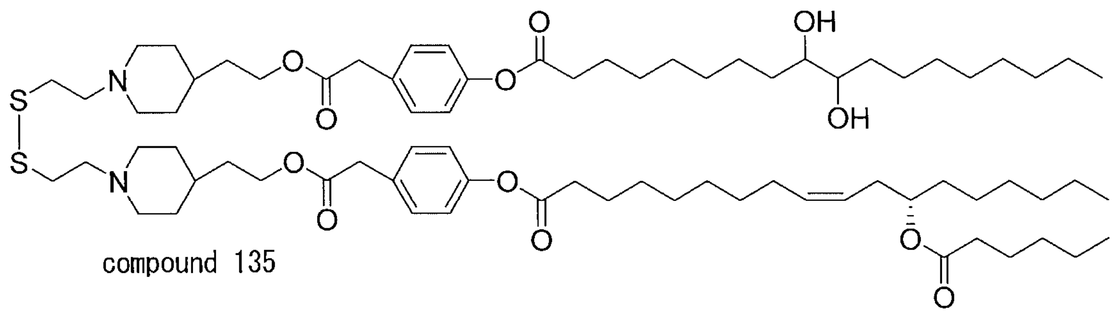

compound 135

<$^1$H-NMR (600 MHz, DMSO-$d_6$) of compound 135>

δ: 0.88 (m, 9H), 1.20-1.60 (m, 66H), 2.01-2.57 (m, 22H), 2.66 (t, 4H), 3.39-3.41 (m, 2H), 3.71 (s, 4H), 4.12-4.14 (m, 4H), 4.59-4.61 (m, 1H), 5.32-5.45 (m, 2H), 7.12-.7.20 (m, 8H)

[Example 136] Synthesis of compound 136

<Synthesis of intermediate 49>

Intermediate 49 was synthesized by the following synthesis pathway.

[Chem. 322]

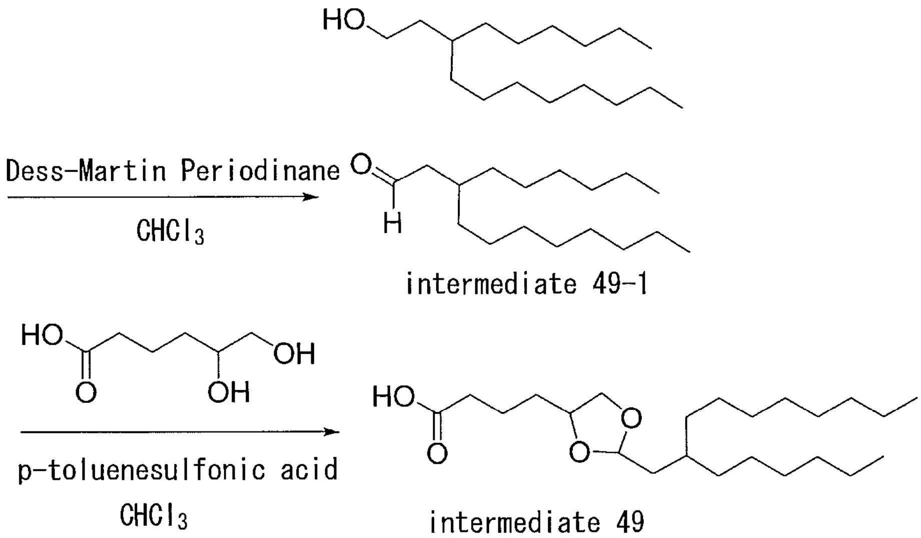


<Synthesis of intermediate 49-1>

3-Hexylundecan-1-ol (500 mg, 1.95 mmol) was dissolved in chloroform (10.0 mL) at room temperature. To the obtained mixture was added Dess-Martin periodinane (1.24 g, 2.92 mmol) and the mixture was reacted at room temperature for 2 hr. Thereafter, the reaction solution was washed with 5 wt% sodium hydrogen carbonate aqueous solution, and 20 wt% saline, and dehydrated by adding sodium sulfate. Sodium sulfate was removed by filtration, and the filtrate was concentrated by an evaporator. The obtained residue was purified by silica gel column chromatography to give 471 mg of intermediate 49-1.

<Synthesis of intermediate 49>

Intermediate 49-1 (471 mg, 1.85 mmol) and 5,6-dihydroxy hexanoic acid (329 mg, 2.22 mmol) were dissolved in chloroform (10.0 mL) at room temperature. To the obtained mixture was added p-toluenesulfonic acid monohydrate (105 mg, 0.555 mmol) and the mixture was reacted at room temperature for 7 hr. Thereafter, to the reaction solution was added chloroform (6.40 mL), and the mixture was washed with 5 wt% sodium hydrogen carbonate aqueous solution, and 20 wt% saline, and dehydrated by adding sodium sulfate. Sodium sulfate was removed by filtration, and the filtrate was concentrated by an evaporator. The obtained residue was purified by silica gel column chromatography to give 354 mg of intermediate 49.

<Synthesis of compound 136>

Using intermediate 49 and intermediate 1-1 and in the same manner as in the synthesis of compound 1 described in Example 1-1, compound 136 was synthesized.

[Chem. 323]

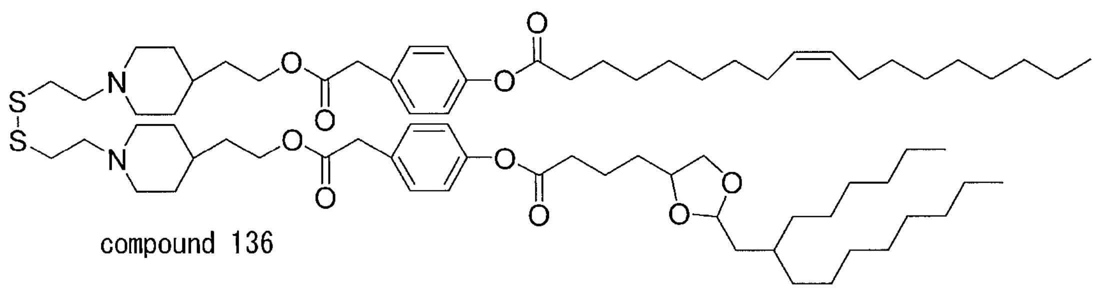

<$^1$H-NMR (600 MHz, DMSO-$d_6$) of compound 136>

δ: 0.88 (m, 9H), 1.20-1.68 (m, 67H), 2.01-2.57 (m, 20H), 2.66 (t, 4H), 3.60-3.64 (m, 2H), 3.71 (s, 4H), 3.85-3.89 (m, 1H), 4.12-4.14 (m, 4H), 5.34-5.47 (m, 3H), 7.12-.7.20 (m, 8H)

[Example 137] Synthesis of compound 137

Using intermediate 49 and intermediate 1-7 and in the same manner as in the synthesis of compound 1 described in Example 1-1, compound 137 was synthesized.

[Chem. 324]

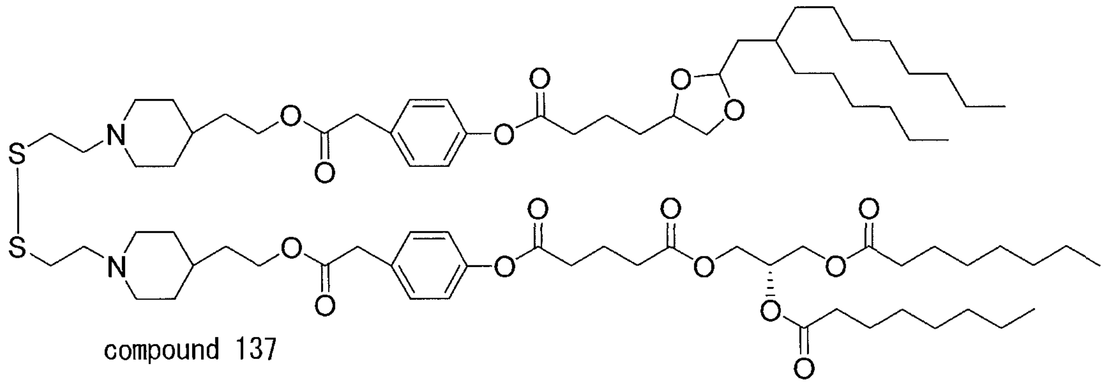

compound 137

<$^1$H-NMR (600 MHz, DMSO-$d_6$) of compound 137>

δ: 0.88 (m, 12H), 1.17-1.68 (m, 65H), 2.30-2.57 (m, 24H), 2.66 (t, 4H), 3.60-3.64 (m, 2H), 3.71 (s, 4H), 3.85-3.89 (m, 1H), 4.12-4.14 (m, 4H), 4.30 (d, 4H), 5.46 (t, 1H), 5.85 (dd, 1H), 7.12-.7.20 (m, 8H)

[Example 138] Synthesis of compound 138

Using intermediate 49 and intermediate 1-2 and in the same manner as in the synthesis of compound 1 described in Example 1-1, compound 138 was synthesized.

[Chem. 325]

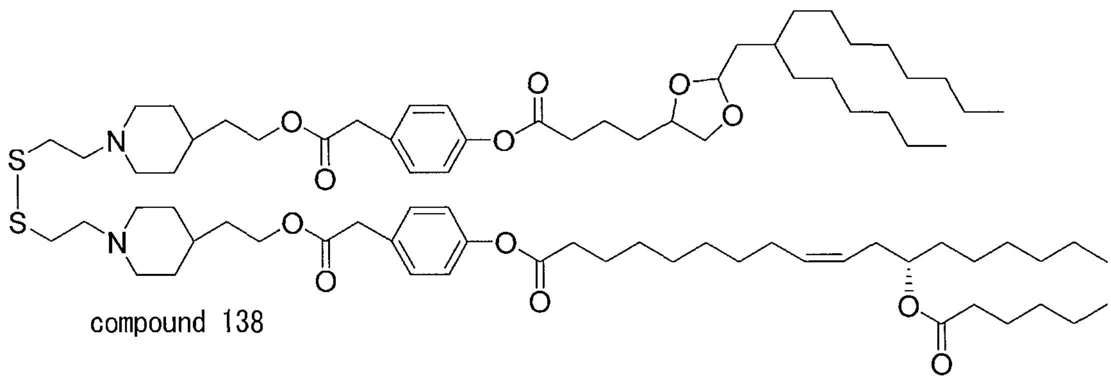

compound 138

<$^1$H-NMR (600 MHz, DMSO-$d_6$) of compound 138>

5: 0.86-0.90 (m, 12H), 1.22-1.42 (m, 53H), 1.51-1.76 (m, 18H), 1.91-1.98 (m, 4H), 2.00-2.06 (m, 2H), 2.23-2.33 (m, 4H), 2.52-2.56 (m, 4H), 2.61-2.67 (m, 4H), 2.79-2.92 (m, 8H), 3.59 (s, 4H), 3.62-3.90 (s, 3H), 4.11-4.14 (t, 4H), 4.85-4.90 (m, 1H), 5.31-5.40 (m, 2H), 5.44-5.49 (m, 1H), 7.02-7.04 (m, 4H), 7.26-7.30 (m, 4H)

[Example 139] Synthesis of compound 139a and compound 139

<Synthesis of intermediate 50>

Intermediate 50 was synthesized by the following synthesis pathway.

[Chem. 326]

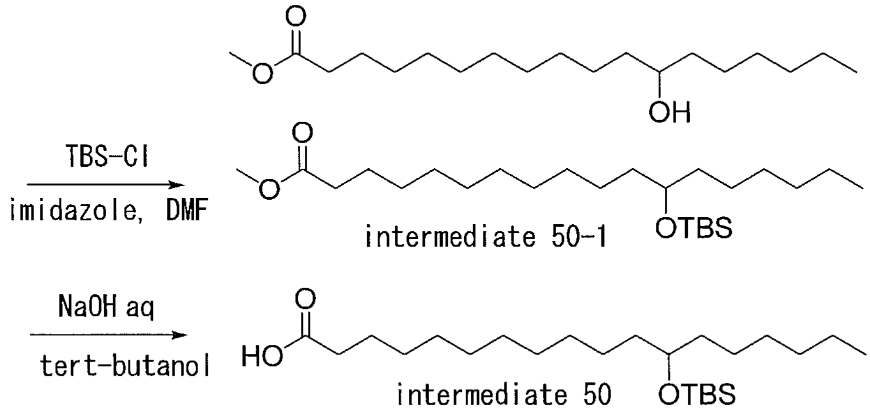


<Synthesis of intermediate 50-1>

Methyl 12-hydroxystearate (1.00 g, 3.18 mmol), and imidazole (433 mg, 6.36 mmol) were dissolved in dimethylformamide (10.5 mL) at room temperature. To the obtained mixture was added TBS-Cl (767 mg, 5.09 mmol) and the mixture was reacted at room temperature for 1 hr. Thereafter, to the reaction solution was added chloroform, and the mixture was washed with 5 wt% sodium hydrogen phosphate aqueous solution, and ion-exchanged water, and dehydrated by adding sodium sulfate. Sodium sulfate was removed by filtration, and the filtrate was concentrated by an evaporator to give 1.12 g of intermediate 50-1.

<Synthesis of intermediate 50>

Intermediate 50-1 (1.09 g) was dissolved in tert-butanol (5.51 mL) at room temperature. To the obtained mixture was added 2 M NaOH aqueous solution (2.04 g) and the mixture was reacted at room temperature for 8 hr. Thereafter, to the reaction solution was added ethyl acetate, and the mixture was washed with 7 wt% sodium hydrogen carbonate aqueous solution, and 20 wt% saline, and dehydrated by adding sodium sulfate. Sodium sulfate was removed by filtration, and the filtrate was concentrated by an evaporator to give 0.716 g of intermediate 50.

<Synthesis of compound 139a>

Using intermediate 50 and intermediate 1-1 and in the same manner as in the synthesis of compound 1 described in Example 1-1, compound 139a was synthesized.

[Chem. 327]

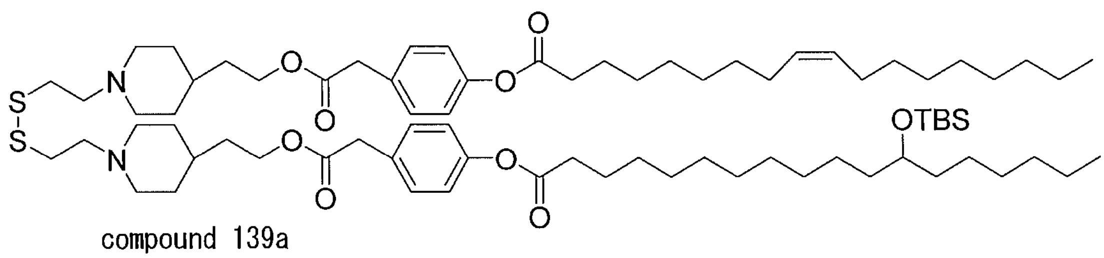

compound 139a

<$^1$H-NMR (600 MHz, DMSO-$d_6$) of compound 139a>

δ: 0.21 (s, 6H), 0.86-0.89 (m, 6H), 0.98 (s, 9H), 1.22-1.42 (m, 52H), 1.54-1.76 (m, 12H), 1.91-1.98 (m, 4H), 1.99-2.10 (m, 4H), 2.52-2.56 (m, 4H), 2.62-2.66 (m, 4H), 2.78-2.92 (m, 8H), 3.35-3.45 (m, 1H), 3.59 (s, 4H), 4.11-4.13 (t, 4H), 5.30-5.38 (m, 2H), 7.02-7.04 (m, 4H), 7.26-7.29 (m, 4H)

<Synthesis of compound 139>

Using compound 139a and in the same manner as in the synthesis of compound 127 described in Example 127, compound 139 was synthesized.

[Chem. 328]

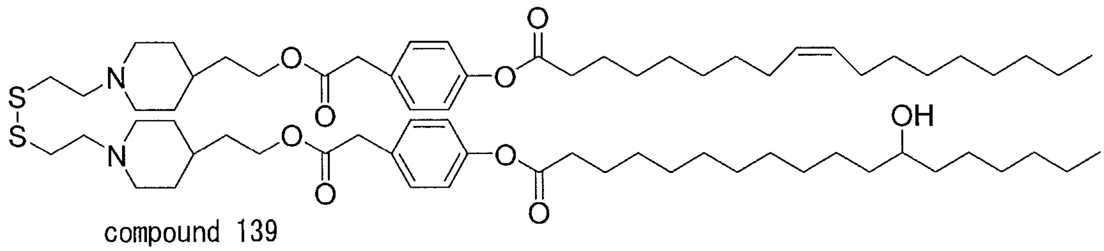

compound 139

<$^1$H-NMR (600 MHz, DMSO-$d_6$) of compound 139>

δ: 0.86-0.89 (m, 6H), 1.22-1.42 (m, 46H), 1.54-1.76 (m, 18H), 1.99-2.16 (m, 4H), 2.41-2.56 (m, 16H), 2.62-2.66 (m, 4H), 3.40-3.59 (s, 1H), 3.71 (s, 4H), 4.11-4.13 (t, 4H), 4.80 (brs, 1H), 5.30-5.38 (m, 2H), 7.02-7.04 (m, 4H), 7.26-7.29 (m, 4H)

[Example 140] Synthesis of compound 140a and compound 140

<Synthesis of compound 140a>

Using intermediate 50 and intermediate 1-7 and in the same manner as in the synthesis of compound 1 described in Example 1-1, compound 140a was synthesized.

[Chem. 329]

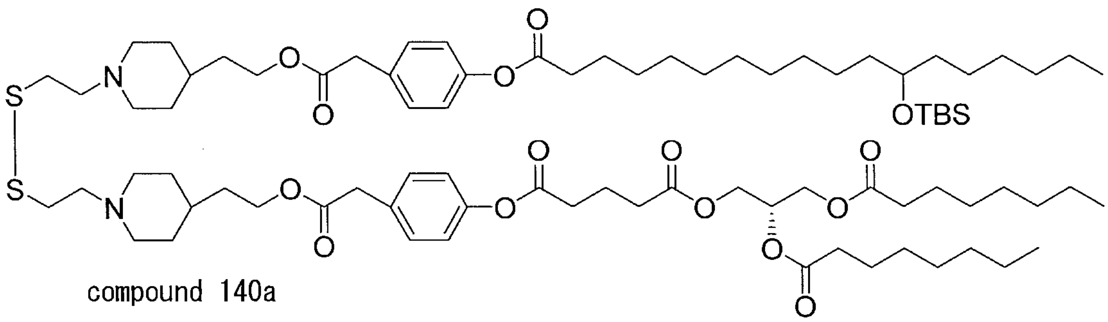

compound 140a

<$^1$H-NMR (600 MHz, DMSO-$d_6$) of compound 140a>

δ: 0.21 (s, 6H), 0.86-0.89 (m, 9H), 0.98 (s, 9H), 1.22-1.42 (m, 48H), 1.54-1.76 (m, 14H), 1.91-1.98 (m, 4H), 1.99-2.10 (m, 2H), 2.27-2.35 (m, 4H), 2.46-2.51 (m, 4H), 2.61-2.67 (m, 6H), 2.78-2.95 (m, 8H), 3.35-3.45 (m, 1H),3.59 (s, 4H), 4.09-4.21 (m, 6H), 4.27-4.35 (m, 2H), 5.25-5.30 (m, 1H), 7.02-7.04 (m, 4H), 7.26-7.29 (m, 4H)

<Synthesis of compound 140>

Using compound 140a and in the same manner as in the synthesis of compound 127 described in Example 127, compound 140 was synthesized.

[Chem. 330]

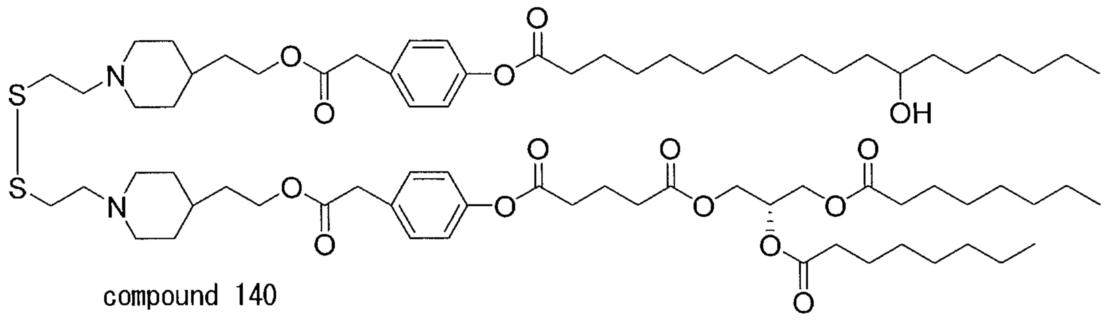

compound 140

<$^1$H-NMR (600 MHz, DMSO-$d_6$) of compound 140>

δ: 0.86-0.89 (m, 9H), 1.22-1.42 (m, 42H), 1.54-1.76 (m, 20H), 2.30-2.56 (m, 24H), 2.62-2.66 (m, 4H), 3.40-3.59 (m, 1H), 3.59 (s, 4H), 4.11-4.40 (t, 8H), 4.80 (brs, 1H), 5.81-5.86 (m, 1H), 7.02-7.04 (m, 4H), 7.26-7.29 (m, 4H)

[Example 141] Synthesis of compound 141a and compound 141

<Synthesis of compound 141a>

Using intermediate 50 and intermediate 1-2 and in the same manner as in the synthesis of compound 1 described in Example 1-1, compound 141a was synthesized.

[Chem. 331]

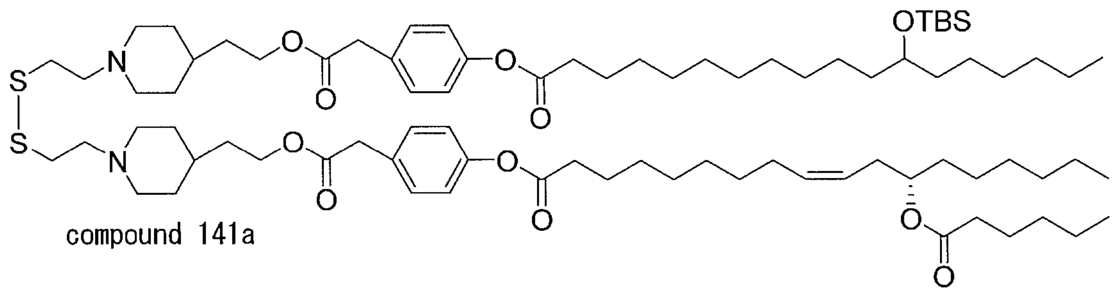

compound 141a

<$^1$H-NMR (600 MHz, DMSO-$d_6$) of compound 141a>

δ: 0.21 (s, 6H), 0.86-0.90 (m, 9H), 0.98 (s, 9H), 1.22-1.42 (m, 52H), 1.54-1.76 (m, 16H), 1.91-1.98 (m, 4H), 2.00-2.06 (m, 2H), 2.23-2.33 (m, 4H), 2.52-2.56 (m, 4H), 2.61-2.67 (m, 4H), 2.79-2.92 (m, 8H), 3.35-3.45 (m, 1H), 3.59 (s, 4H), 4.11-4.14 (t, 4H), 4.85-4.90 (m, 1H), 5.31-5.40 (m, 1H), 5.44-5.49 (m, 1H), 7.02-7.04 (m, 4H), 7.26-7.30 (m, 4H)

<Synthesis of compound 141>

Using compound 141a and in the same manner as in the synthesis of compound 127 described in Example 127, compound 141 was synthesized.

[Chem. 332]

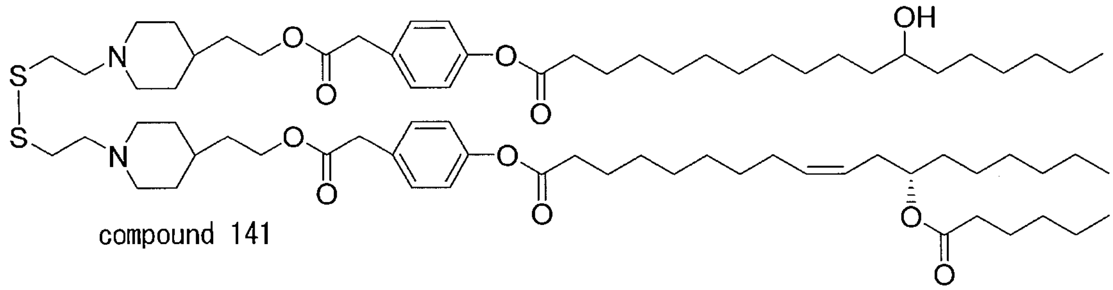

compound 141

<$^1$H-NMR (600 MHz, DMSO-$d_6$) of compound 141>

δ: 0.86-0.89 (m, 9H), 1.22-1.49 (m, 48H), 1.54-1.76 (m, 20H), 2.16-2.56 (m, 22H), 2.62-2.66 (m, 4H), 3.40-3.59 (m, 1H), 3.71 (s, 4H), 4.13 (t, 4H), 4.60-4.81 (m, 1H), 5.81-5.86 (m, 2H), 7.02-7.04 (m, 4H), 7.26-7.29 (m, 4H)

[Example 142] Synthesis of compound 142

<Synthesis of intermediate 51-1>

Methyl 12-hydroxystearate (2.42 g, 7.68 mmol), acetic acid (507 mg, 8.44 mmol), and DMAP (188 mg, 1.54 mmol) were dissolved in chloroform (36.0 g) at room temperature. To the obtained mixture was added EDC hydrochloride (2.94 g, 15.3 mmol) and the mixture was reacted at room temperature for 1 hr. The subsequent steps were performed in the same manner as in the synthesis of intermediate 2-1 described in Example 2-1 to obtain 2.64 g of intermediate 51-1.

[Chem. 333]

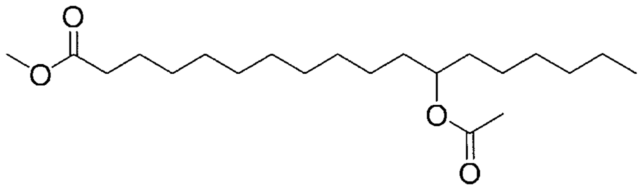

intermediate 51-1

<Synthesis of intermediate 51>

Intermediate 51-1 (2.64 g, 7.41 mmol) was dissolved in tert-butanol to 0.3 M at room temperature. To the obtained mixture was added 2 M NaOH aqueous solution (3.89 g), and the mixture was reacted at room temperature for 8 hr. The subsequent steps were performed in the same manner as in the synthesis of intermediate 2 described in Example 2-1 to obtain 2.44 g of intermediate 51.

[Chem. 334]

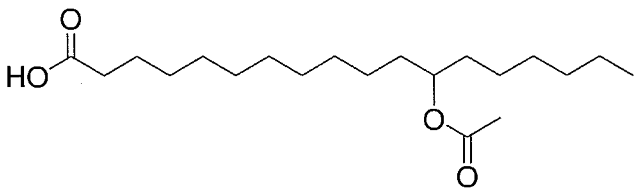

intermediate 51

<Synthesis of compound 142>

Using intermediate 51 and intermediate 1-1 and in the same manner as in the synthesis of compound 1 described in Example 1-1, compound 142 was synthesized.

[Chem. 335]

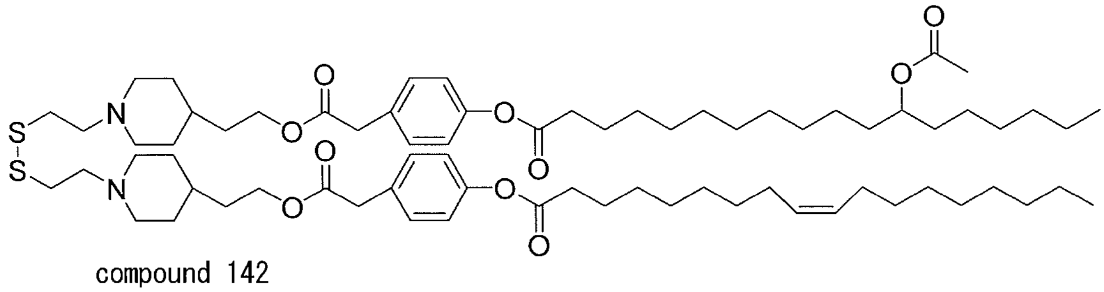

compound 142

<$^1$H-NMR (600 MHz, DMSO-$d_6$) of compound 142>

δ: 0.86-0.89 (m, 6H), 1.22-1.42 (m, 48H), 1.54-1.76 (m, 16H), 1.91-1.98 (m, 4H), 1.99-2.10 (m, 7H), 2.52-2.56 (t, 4H), 2.62-2.66 (m, 4H), 2.78-2.92 (m, 8H), 3.59 (s, 4H), 4.11-4.13 (t, 4H), 4.45-4.49 (m, 1H), 5.30-5.38 (m, 2H), 7.02-7.04 (m, 4H), 7.26-7.29 (m, 4H)

[Example 143] Synthesis of compound 143

Using intermediate 51 and intermediate 1-7 and in the same manner as in the synthesis of compound 1 described in Example 1-1, compound 143 was synthesized.

[Chem. 336]

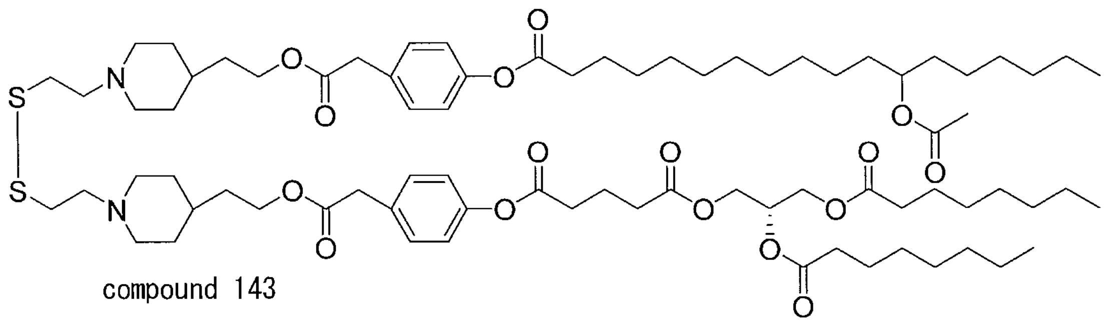

compound 143

<$^1$H-NMR (600 MHz, DMSO-$d_6$) of compound 143>

δ: 0.86-0.89 (m, 9H), 1.22-1.42 (m, 44H), 1.54-1.76 (m, 18H), 1.91-1.98 (m, 4H), 1.99-2.10 (m, 5H), 2.27-2.35 (m, 4H), 2.46-2.51 (m, 4H), 2.61-2.67 (m, 6H), 2.78-2.95 (m, 8H), 3.59 (s, 4H), 4.09-4.21 (m, 6H), 4.27-4.35 (m, 2H), 4.45-4.49 (m, 1H), 5.25-5.30 (m, 1H), 7.02-7.04 (m, 4H), 7.26-7.29 (m, 4H)

[Example 144] Synthesis of compound 144

Using intermediate 51 and intermediate 1-2 and in the same manner as in the synthesis of compound 1 described in Example 1-1, compound 144 was synthesized.

[Chem. 337]

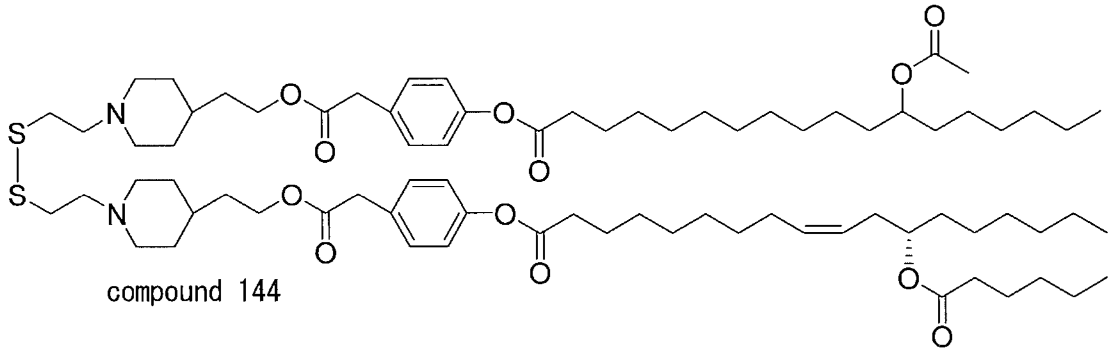

compound 144

<$^1$H-NMR (600 MHz, DMSO-$d_6$) of compound 144>

δ: 0.86-0.90 (m, 9H), 1.22-1.42 (m, 48H), 1.54-1.76 (m, 20H), 1.91-1.98 (m, 4H), 2.00-2.06 (m, 5H), 2.23-2.33 (m, 4H), 2.52-2.56 (m, 4H), 2.61-2.67 (m, 4H), 2.79-2.92 (m, 8H), 3.59 (s, 4H), 4.11-4.14 (t, 4H), 4.45-4.49 (m, 1H), 4.85-4.90 (m, 1H), 5.31-5.40 (m, 1H), 5.44-5.49 (m, 1H), 7.02-7.04 (m, 4H), 7.26-7.30 (m, 4H)

[Example 145] Synthesis of compound 145

<Synthesis of intermediate 52-1>

Methyl 12-hydroxystearate (2.42 g, 7.68 mmol), octanoic acid (1.22 g, 8.44 mmol), and DMAP (188 mg, 1.54 mmol) were dissolved in chloroform (36.0 g) at room temperature. To the obtained mixture was added EDC hydrochloride (2.94 g, 15.3 mmol) and the mixture was reacted at room temperature for 1 hr. The subsequent steps were performed in the same manner as in the synthesis of intermediate 2-1 described in Example 2-1 to obtain 3.27 g of intermediate 52-1.

[Chem. 338]

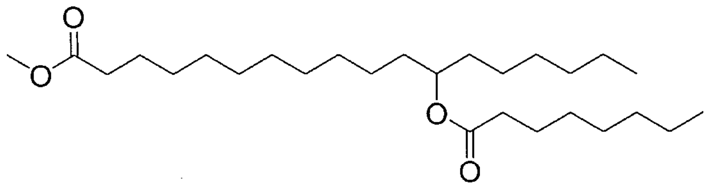

intermediate 52-1

<Synthesis of intermediate 52>

Intermediate 52-1 (3.27 g, 7.41 mmol) was dissolved in tert-butanol to 0.3 M at room temperature. To the obtained mixture was added 2 M NaOH aqueous solution (3.89 g) and the mixture was reacted at room temperature for 8 hr. The subsequent steps were performed in the same manner as in the synthesis of intermediate 2 described in Example 2-1 to obtain 3.04 g of intermediate 52.

[Chem. 339]

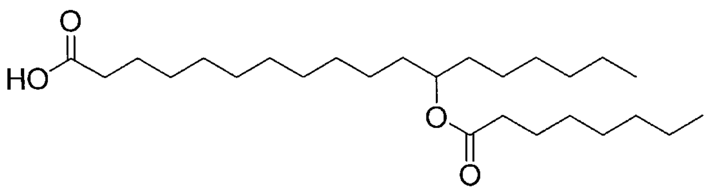

intermediate 52

<Synthesis of compound 145>

Using intermediate 52 and intermediate 1-1 and in the same manner as in the synthesis of compound 1 described in Example 1-1, compound 145 was synthesized.

[Chem. 340]

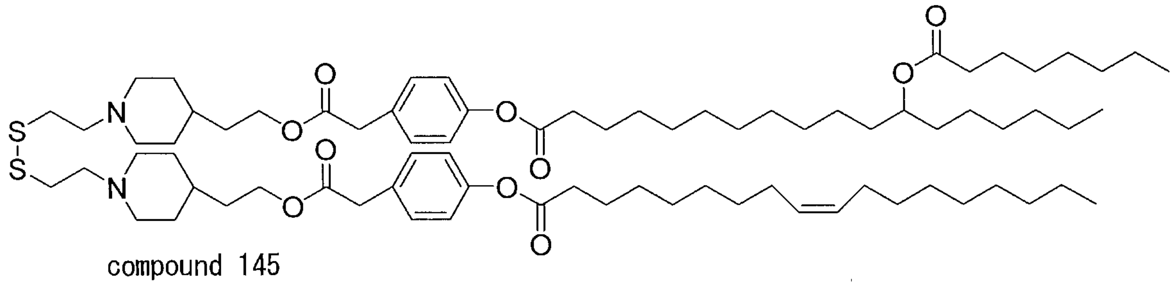

compound 145

<$^1$H-NMR (600 MHz, DMSO-$d_6$) of compound 145>

δ: 0.86-0.89 (m, 9H), 1.22-1.42 (m, 56H), 1.54-1.76 (m, 18H), 1.91-1.98 (m, 4H), 1.99-2.10 (m, 4H), 2.33-2.56 (m, 6H), 2.62-2.66 (m, 4H), 2.78-2.92 (m, 8H), 3.59 (s, 4H), 4.11-4.13 (t, 4H), 4.45-4.49 (m, 1H), 5.30-5.38 (m, 2H), 7.02-7.04 (m, 4H), 7.26-7.29 (m, 4H)

[Example 146] Synthesis of compound 146

Using intermediate 52 and intermediate 1-7 and in the same manner as in the synthesis of compound 1 described in Example 1-1, compound 146 was synthesized.

[Chem. 341]

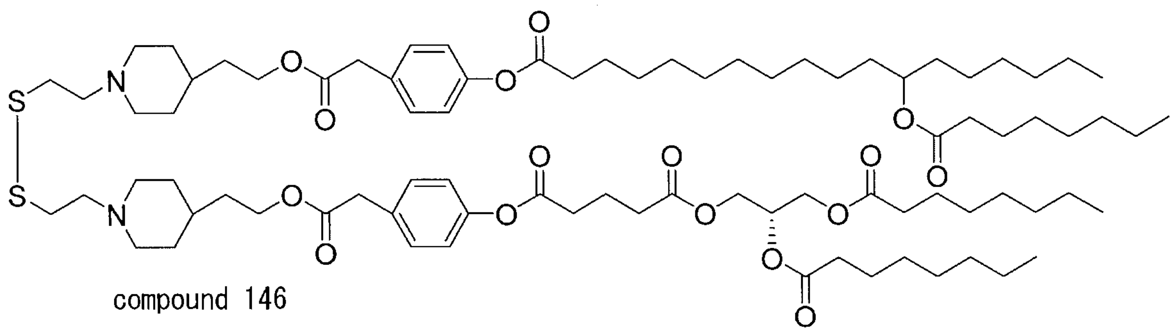

compound 146

<$^1$H-NMR (600 MHz, DMSO-$d_6$) of compound 146>

δ: 0.86-0.89 (m, 12H), 1.22-1.42 (m, 52H), 1.54-1.76 (m, 20H), 1.91-1.98 (m, 4H), 1.99-2.10 (m, 2H), 2.27-2.67 (m, 16H), 2.78-2.95 (m, 8H), 3.59 (s, 4H), 4.09-4.21 (m, 6H), 4.27-4.35 (m, 2H), 4.45-4.49 (m, 1H), 5.25-5.30 (m, 1H), 7.02-7.04 (m, 4H), 7.26-7.29 (m, 4H)

[Example 147] Synthesis of compound 147

Using intermediate 52 and intermediate 1-2 and in the same manner as in the synthesis of compound 1 described in Example 1-1, compound 147 was synthesized.

[Chem. 342]

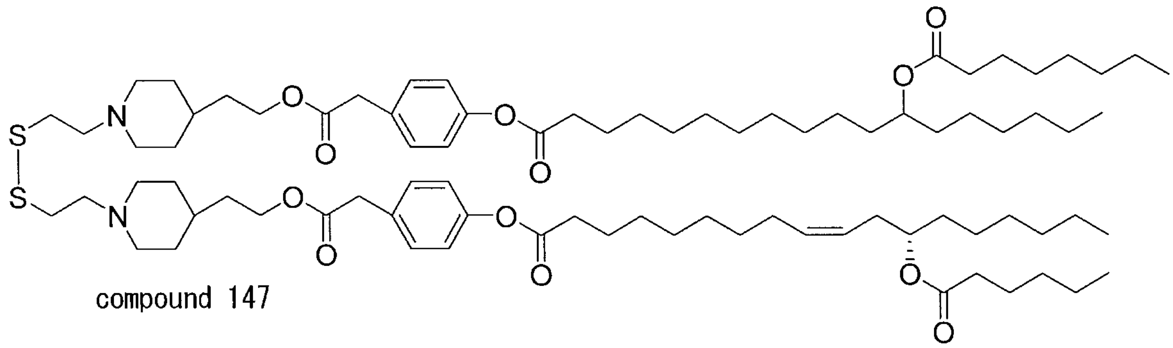

compound 147

<$^{1}$H-NMR (600 MHz, DMSO-$d_6$) of compound 147>

δ: 0.86-0.90 (m, 12H), 1.22-1.42 (m, 56H), 1.54-1.76 (m, 22H), 1.91-1.98 (m, 4H), 2.00-2.06 (m, 2H), 2.23-2.33 (m, 4H), 2.52-2.56 (m, 6H), 2.61-2.67 (m, 4H), 2.79-2.92 (m, 8H), 3.59 (s, 4H), 4.11-4.14 (t, 4H), 4.45-4.49 (m, 1H), 4.85-4.90 (m, 1H), 5.31-5.40 (m, 1H), 5.44-5.49 (m, 1H), 7.02-7.04 (m, 4H), 7.26-7.30 (m, 4H)

[Example 148] Synthesis of compound 148

Using intermediate 1-10 and intermediate 53 and in the same manner as in the synthesis of compound 1 described in Example 1-1, compound 148 was synthesized.

[Chem. 343]

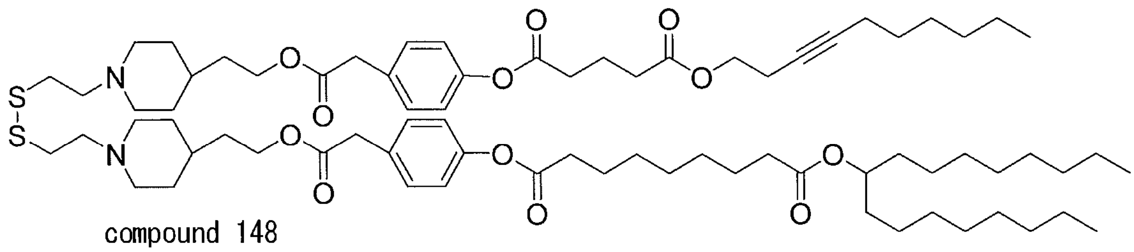

compound 148

<$^{1}$H-NMR (600 MHz, $CDCl_3$) of compound 148>

δ: 0.84-0.90 (m, 9H), 1.18-1.70 (m, 66H), 1.71-1.78 (m, 2H), 1.91-1.99 (m, 2H), 2.04-2.10 (m, 2H), 2.10-2.15 (m, 2H), 2.26-2.31 (m, 2H), 2.46-2.56 (m, 6H), 2.61-2.67 (m, 4H), 2.79-2.92 (m, 4H), 3.59 (s, 4H), 4.09-4.18 (m, 6H), 4.83-4.89 (m, 1H), 7.02-7.04 (m, 4H), 7.26-7.29 (m, 4H)

[Example 149] Synthesis of compound 149

Using intermediate 22 and intermediate 1-1 and in the same manner as in the synthesis of compound 1 described in Example 1-1, compound 149 was synthesized.

[Chem. 344]

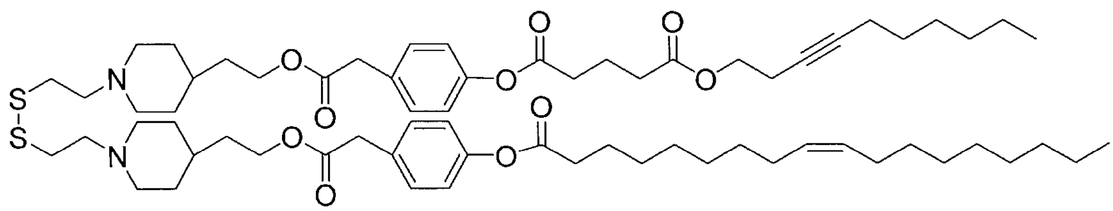

compound 149

<$^1$H-NMR (600 MHz, $CDCl_3$) of compound 149>

δ: 0.86-0.91 (m, 6H), 1.20-1.44 (m, 32H), 1.45-1.49 (m, 2H), 1.50-1.60 (m, 6H), 1.60-1.66 (m, 4H), 1.70-1.77 (m, 2H), 1.91-1.98 (m, 2H), 1.99-2.16 (m, 8H), 2.45-2.53 (m, 4H), 2.5.3-2.56 (m, 2H), 2.60-2.66 (m, 6H), 2.78-2.92 (m, 8H), 3.59 (s, 4H), 4.10-4.18 (m, 6H), 5.33-5.40 (m, 2H), 7.02-7.04 (m, 4H), 7.26-7.29 (m, 4H)

[Example 150] Synthesis of compound 150

Using intermediate 1-10 and an ester of tocopherol and glutaric acid obtained by the synthesis described in Example 46 and in the same manner as in the synthesis of compound 1 described in Example 1-1, compound 150 was synthesized.

[Chem. 345]

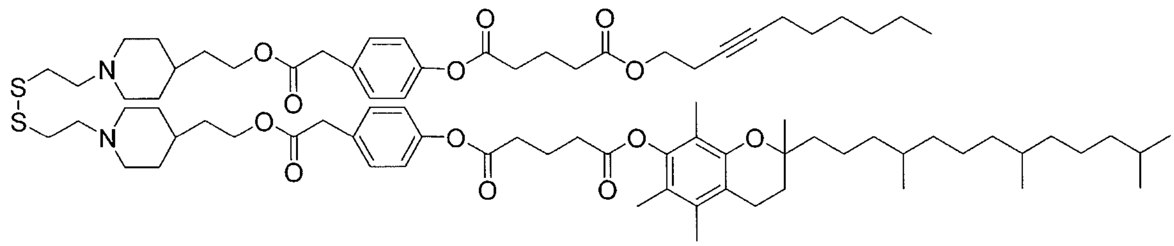

[1294]

<$^1$H-NMR (600 MHz, $CDCl_3$) of compound 150>

δ: 0.84-0.90 (m, 15H), 1.03-1.58 (m, 44H), 1.61-1.66 (m, 4H), 1.71-1.83 (m, 2H), 1.90-2.25 (m, 14H), 2.44-2.52 (m, 4H), 2.68-2.88 (m, 23H), 3.59 (s, 4H), 4.10-4.18 (m, 6H), 7.02-7.04 (m, 4H), 7.26-7.29 (m, 4H)

[Example 151] Synthesis of compound 151

Using intermediate 9 and intermediate 1-1 and in the same manner as in the synthesis of compound 1 described in Example 1-1, compound 151 was synthesized.

[Chem. 346]

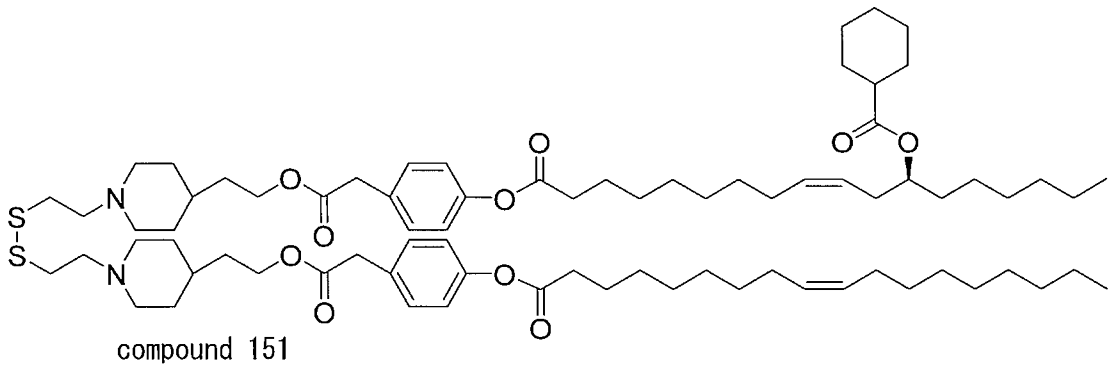

compound 151

<$^1$H-NMR (600 MHz, $CDCl_3$) of compound 151>

δ: 0.85-0.90 (t, 6H), 1.19-1.48 (m, 48H), 1.49-1.59 (m, 14H), 1.60-1.66 (m, 4H), 1.69-1.78 (m, 6H), 184-1.92 (m, 2H), 1.93-2.06 (m, 2H), 2.22-2.33 (m, 3H), 2.52-2.57 (m, 4H), 2.61-2.66 (m, 4H), 2.79-2.84 (m, 4H), 2.85-2.91 (m, 4H), 3.59 (s, 4H), 4.09-4.14 (t, 4H), 4.84-4.90 (m, 1H), 5.29-5.33 (m, 3H), 5.43-5.48 (m, 1H), 7.02-7.04 (m, 4H), 7.26-7.29 (m, 4H)

[Example 152] Synthesis of compound 152

<Synthesis of intermediate 1-11>

Using intermediate 9 and intermediate 1 and in the same manner as in the synthesis of compound 1 described in Example 1-1, intermediate 1-11 was synthesized.

[Chem. 347]

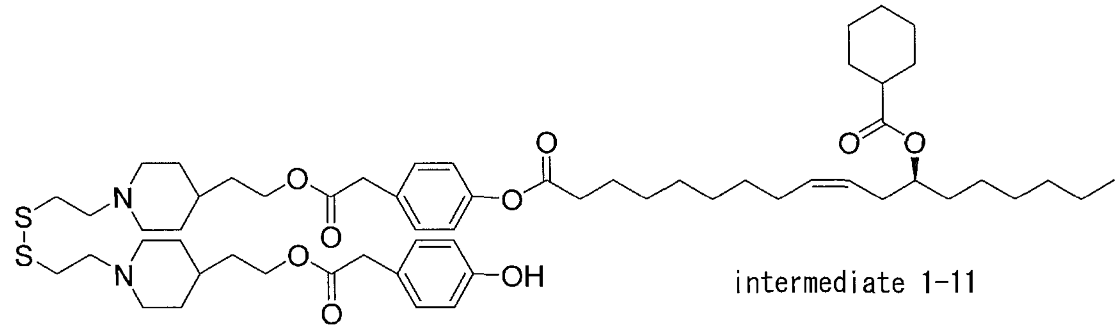

intermediate 1-11

<Synthesis of compound 152>

Using intermediate 1-11 and intermediate 53 and in the same manner as in the synthesis of compound 1 described in Example 1-1, compound 152 was synthesized.

[Chem. 348]

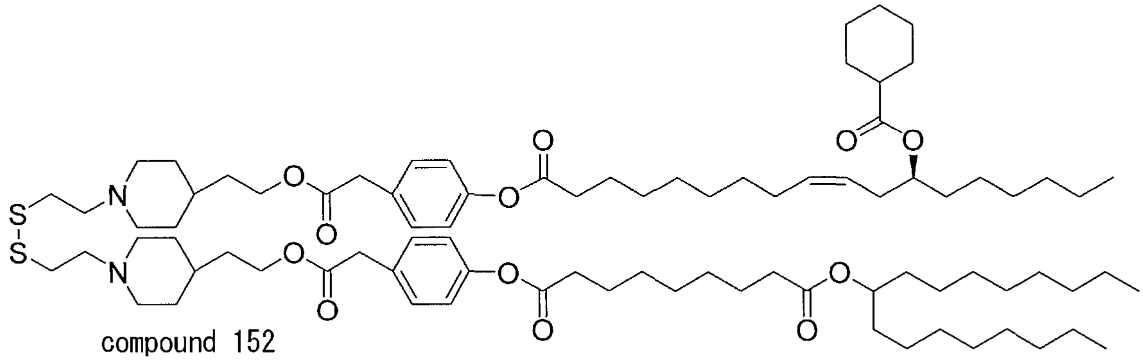

compound 152

<$^1$H-NMR (600 MHz, $CDCl_3$) of compound 152>

δ: 0.89-0.89 (m, 9H), 1.18-1.45 (m, 56H), 1.46-1.58 (m, 12H), 1.72-1.77 (m, 6H), 1.69-1.67 (m, 7H), 1.85-1.91 (m, 2H), 1.93-1.97 (m, 4H), 2.00-2.06 (m, 2H), 2.22-2.31 (m, 4H), 2.51-2.56 (m, 4H), 2.61-2.66 (m, 4H), 2.79-2.84 (m, 4H), 2.85-2.90 (m, 4H), 3.59 (s, 4H), 4.09-4.14 (t, 4H), 4.84-4.90 (m, 2H), 5.30-5.36 (m, 1H), 5.43-5.48 (m, 1H), 7.02-7.04 (m, 4H), 7.26-7.29 (m, 4H)

[Example 153] Synthesis of compound 153

Using intermediate 1-11 and intermediate 10 and in the same manner as in the synthesis of compound 1 described in Example 1-1, compound 153 was synthesized.

[Chem. 349]

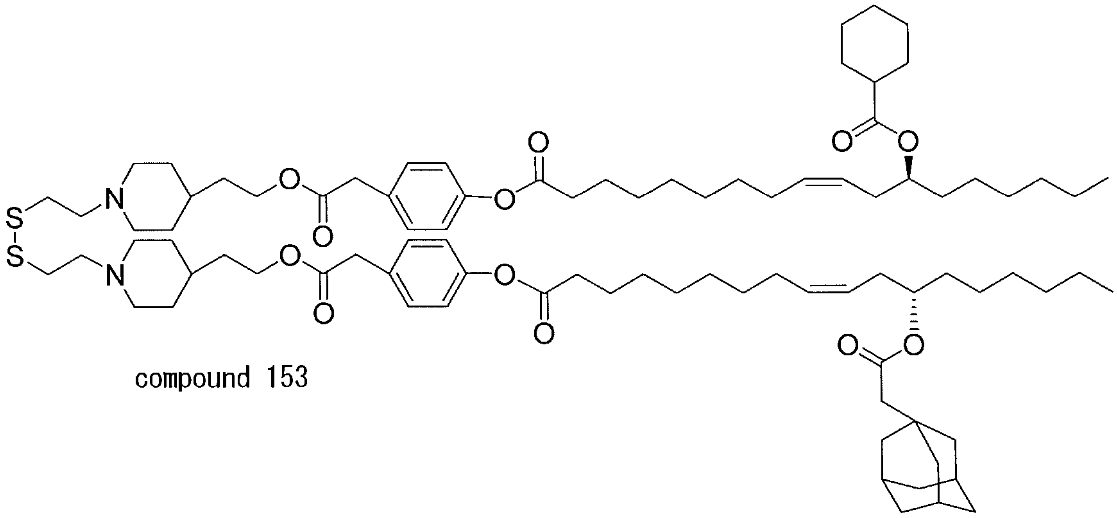

compound 153

<¹H-NMR (600 MHz, $CDCl_3$) of compound 153>

δ: 0.85-0.88 (t, 6H), 1.19-1.47 (m, 44H), 1.49-1.67 (m, 24H), 1.69-1.77 (m, 8H), 1.85-1.91 (m, 2H), 192-1.97 (m, 6H), 1.99-2.06 (m, 6H), 2.22-2.35 (m, 4H), 2.50-2.56 (m, 4H), 2.61-2.66 (m, 4H), 2.79-2.84 (m, 4H), 2.85-2.91 (m, 4H), 3.59 (s, 4H), 4.09-4.14 (t, 4H), 4.84-4.90 (m, 2H), 5.29-5.38 (m, 2H), 5.43-5.50 (m, 2H), 7.02-7.04 (m, 4H), 7.26-7.29 (m, 4H)

[Example 154] Synthesis of compound 154

Using intermediate 1-11 obtained by the synthesis described in Example 152 and cholesterol hydrogen succinate and in the same manner as in the synthesis of compound 1 described in Example 1-1, compound 154 was synthesized.

[Chem. 350]

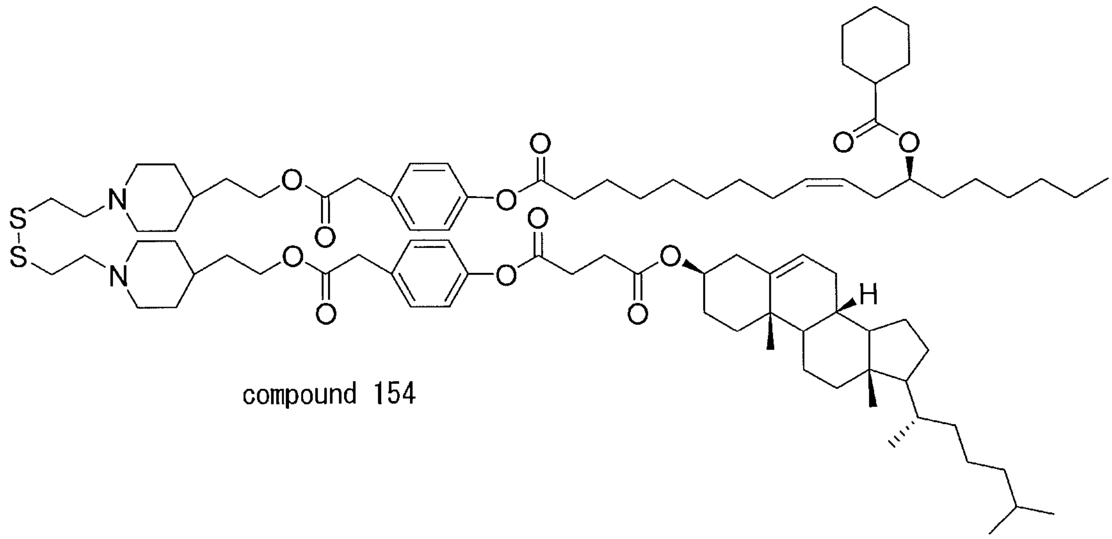

compound 154

<¹H-NMR (600 MHz, $CDCl_3$) of compound 154>

δ: 0.64-0.78 (m, 3H), 0.83-0.88 (m, 9H), 0.89-1.02 (m, 7H), 1.03-1.17 (m, 3H), 1.19-1.46 (m, 22H), 1.47-1.67 (m, 38H), 1.69-1.77 (m, 4H), 1.78-1.91 (m, 4H), 192-2.08 (m, 6H), 2.22-2.35 (m, 4H), 2.51-2.56 (m, 2H), 2.61-2.66 (m, 4H), 2.69-2.74 (m, 2H), 2.78-2.84 (m, 3H), 2.85-2.91 (m, 6H), 3.59 (s, 4H), 4.09-4.14 (t, 4H), 4.62-4.69 (m, 1H), 4.84-4.90 (m, 1H), 5.29-5.38 (m, 2H), 5.43-5.50 (m, 1H), 7.02-7.04 (m, 4H), 7.26-7.29 (m, 4H)

[Example 155] Synthesis of compound 155

Using intermediate 1-6 obtained by the synthesis described in Example 30 and ester of tocopherol and glutaric acid obtained by the synthesis described in Example 46 and in the same manner as in the synthesis of compound 1 described in Example 1-1, compound 155 was synthesized.

[Chem. 351]

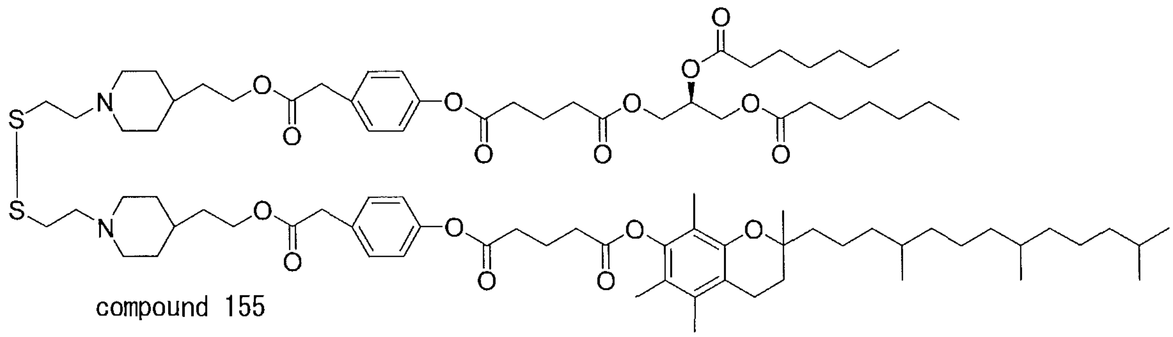

compound 155

<$^1$H-NMR (600 MHz, $CDCl_3$) of compound 155>

δ: 0.83-0.90 (m, 18H), 1.02-1.44 (m, 36H), 1.48-1.68 (m, 16H), 1.71-1.83 (m, 2H), 1.91-1.98 (m, 7H), 1.99-2.03 (m, 3H), 2.04-2.10 (m, 5H), 2.18-2.24 (m, 2H), 2.29-2.34 (m, 4H), 2.45-2.50 (m, 2H), 2.57-2.60 (m, 2H), 2.61-2.66 (m, 6H), 2.71-2.78 (m, 4H), 2.79-2.84 (m, 4H), 2.85-2.90 (m, 4H), 3.59 (s, 4H), 3.69-2.76 (m, 2H), 4.10-4.18 (m, 6H), 4.29-4.36 (m, 2H), 5.24-5.30 (m, 1H), 7.02-7.04 (m, 4H), 7.26-7.29 (m, 4H)

[Example 156] Synthesis of compound 156

<Synthesis of intermediate 54>

Intermediate 54 was synthesized by the following synthesis pathway.

[Chem. 352]

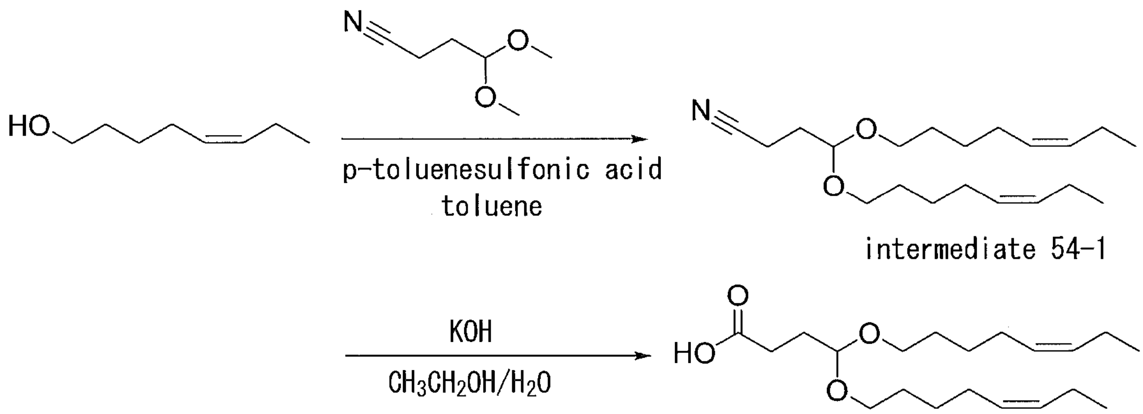


intermediate 54-1

intermediate 54

<Synthesis of intermediate 54-1>

cis-5-Octen-1-ol (2.48 g, 19.4 mmol) and 3-cyanopropionaldehyde dimethyl acetal (500 mg, 3.87 mmol) were dissolved in toluene (5.00 mL) at room temperature. To the obtained mixture was added p-toluenesulfonic acid monohydrate (736 mg, 3.87 mmol), and the mixture was reacted at 100°C for 40 hr. Thereafter, the reaction solution was washed with 5 wt% sodium hydrogen carbonate aqueous solution, and 20 wt% saline, and dehydrated by adding sodium sulfate. Sodium sulfate was removed by filtration, and the filtrate was concentrated by an evaporator. The obtained residue was

purified by silica gel column chromatography to give 946 mg of intermediate 54-1.

<Synthesis of intermediate 54>

Intermediate 54-1 (946 mg, 2.94 mmol) was dissolved in ethanol (3. 00 mL) and ion-exchanged water (3.00 mL) at room temperature. To the obtained mixture was added potassium hydroxide (495 mg, 8.82 mmol), and the mixture was reacted at 100°C for 20 hr. Thereafter, the reaction solution was washed with 5 wt% sodium dihydrogen phosphate aqueous solution, and 20 wt% saline, and dehydrated by adding sodium sulfate. Sodium sulfate was removed by filtration, and the filtrate was concentrated by an evaporator. The obtained residue was purified by silica gel column chromatography to give 951 mg of intermediate 54.

<Synthesis of compound 156>

Using intermediate 54 and intermediate 1-1 and in the same manner as in the synthesis of compound 1 described in Example 1-1, compound 156 was synthesized.

[Chem. 353]

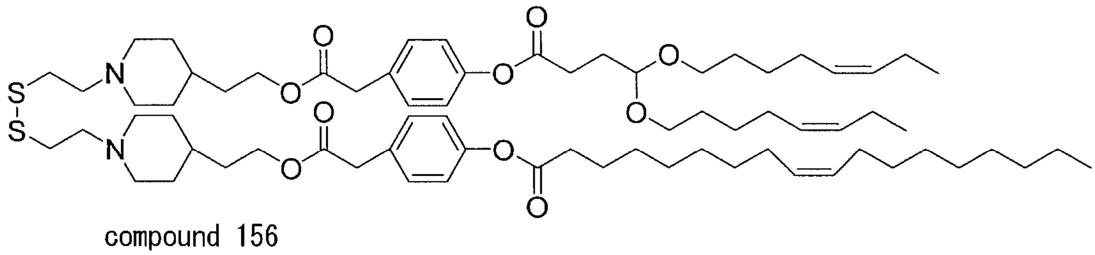

compound 156

<$^1$H-NMR (600 MHz, DMSO-$d_6$) of compound 156>

δ: 0.86-0.89 (m, 3H), 0.94 (t, 6H), 1.22-1.44 (m, 30H), 1.54-1.76 (m, 14H), 1.81 (q, 2H), 1.91-2.10 (m, 16H), 2.30 (t, 2H), 2.52-2.56 (t, 2H), 2.62-2.66 (m, 4H), 2.78-2.92 (m, 8H), 3.39 (dt, 2H), 3.51 (dt, 2H), 3.59 (s, 4H), 4.11-4.13 (t, 4H), 4.45 (t, 1H), 5.24-5.40 (m, 6H), 7.02-7.04 (m, 4H), 7.26-7.29 (m, 4H)

[Example 157] Synthesis of compound 157

Using intermediate 54 and intermediate 1-7 and in the same manner as in the synthesis of compound 1 described in Example 1-1, compound 157 was synthesized.

[Chem. 354]

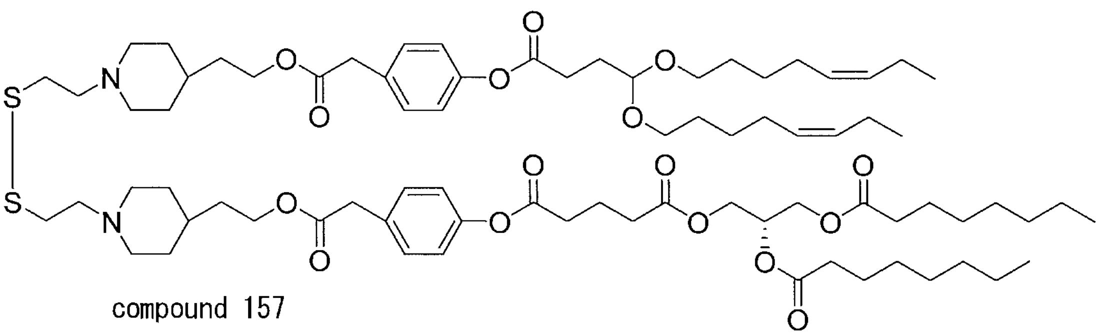

compound 157

<$^1$H-NMR (600 MHz, DMSO-$d_6$) of compound 157>

δ: 0.86-0.89 (m, 6H), 0.94 (t, 6H), 1.22-1.44 (m, 26H), 1.52-1.76 (m, 16H), 1.81 (q, 2H), 1.91-2.10 (m, 14H), 2.27-2.35 (m, 6H), 2.46-2.51 (t, 2H), 2.61-2.67 (m, 6H), 2.78-2.95 (m, 8H), 3.39 (dt, 2H), 3.51 (dt, 2H), 3.59 (s, 4H), 4.09-4.21 (m, 6H), 4.27-4.35 (m, 2H), 4.45 (t, 1H), 5.24-5.41 (m, 5H), 7.02-7.04 (m, 4H), 7.26-7.29 (m, 4H)

[Example 158] Synthesis of compound 158

Using intermediate 54 and intermediate 1-2 and in the same manner as in the synthesis of compound 1 described in Example 1-1, compound 158 was synthesized.

[Chem. 355]

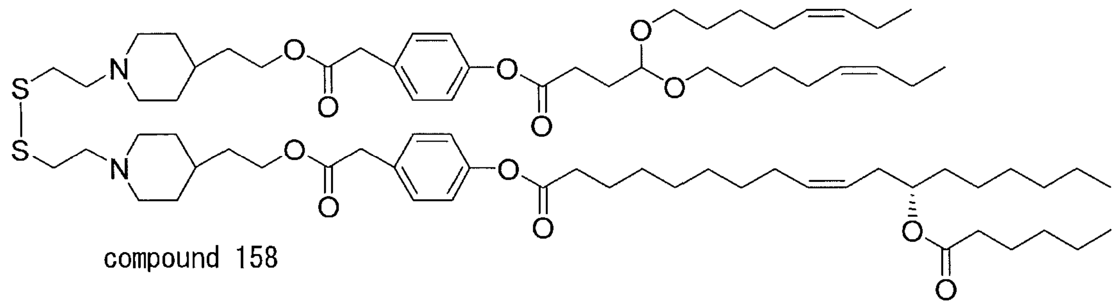

compound 158

<$^1$H-NMR (600 MHz, DMSO-$d_6$) of compound 158>

δ: 0.86-0.90 (m, 6H), 0.94 (t, 6H), 1.22-1.44 (m, 26H), 1.52-1.76 (m, 18H), 1.81 (q, 2H), 1.91-2.06 (m, 18H), 2.23-2.33 (m, 6H), 2.52-2.56 (m, 2H), 2.61-2.67 (m, 4H), 2.79-2.92 (m, 8H), 3.39 (dt, 2H), 3.51 (dt, 2H), 3.59 (s, 4H), 4.11-4.14 (t, 4H), 4.45 (t, 1H), 4.85-4.90 (m, 1H), 5.24-5.41 (m, 5H), 5.44-5.49 (m, 1H), 7.02-7.04 (m, 4H), 7.26-7.30 (m, 4H)

[Example 159] Synthesis of compound 159

Using intermediate 5 and intermediate 1 and in the same manner as in the synthesis of compound 2 described in Example 2-1, compound 159 was synthesized.

[Chem. 356]

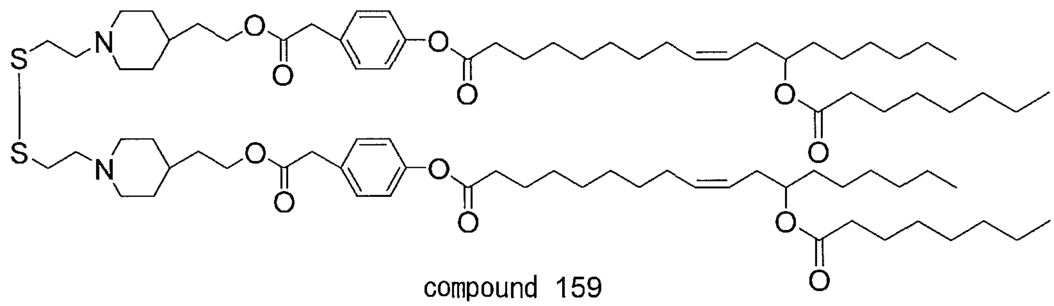

compound 159

<$^1$H-NMR (600 MHz, $CDCl_3$) of compound 159>

δ: 0.86-0.89 (m, 12H), 1.22-1.42 (m, 46H), 1.54-1.76 (m, 16H), 1.91-1.98 (m, 4H), 2.00-2.06 (m, 8H), 2.23-2.33 (m, 8H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 2H), 5.31-5.39 (m, 2H), 5.44-5.49 (m, 2H), 7.02-7.04 (m, 4H), 7.26-7.30 (m, 4H)

[Example 160] Synthesis of compound 160

<Synthesis of intermediate 60>

Azelaic acid (2.00 g, 10.6 mmol), 1-nonan-3-ol (756 mg, 5.31 mmol), and DMAP (649 mg, 5.31 mmol) were dissolved in chloroform (50.0 g) at room temperature. To the obtained mixture was added EDC hydrochloride (1.22 g, 6.36 mmol) and the mixture was reacted at room temperature for 1 hr. The subsequent steps were performed in the same manner as in the synthesis of intermediate 2-1 described in Example 2-1 to obtain 2.63 g of intermediate 60.

[Chem. 357]

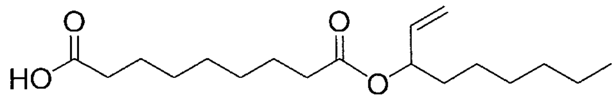

intermediate 60

<Synthesis of compound 160>

Using intermediate 60 and intermediate 1-1 and in the same manner as in the synthesis of compound 1 described in Example 1-1, compound 160 was synthesized.

[Chem. 358]

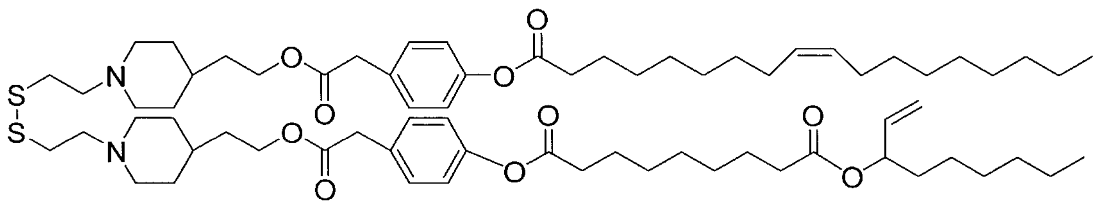

compound 160

<$^1$H-NMR (600 MHz, $CDCl_3$) of compound 160>

δ: 0.86-0.89 (m, 6H), 1.22-1.42 (m, 32H), 1.54-1.76 (m, 16H), 1.91-1.98 (m, 4H), 2.00-2.06 (m, 8H), 2.23-2.33 (m, 2H), 2.52-2.67 (m, 16H), 2.79-2.92 (m, 4H), 3.59 (s, 4H), 4.11-4.14 (t, 4H), 5.31-5.39 (m, 4H), 5.44-5.49 (m, 1H), 5.89-5.91 (m, 1H), 7.02-7.04 (m, 4H), 7.26-7.30 (m, 4H)

<Synthesis of compound 161>

Using intermediate 3 and intermediate 1 and in the same manner as in the synthesis of compound 2 described in Example 2-1, compound 161 was synthesized.

[Chem. 359]

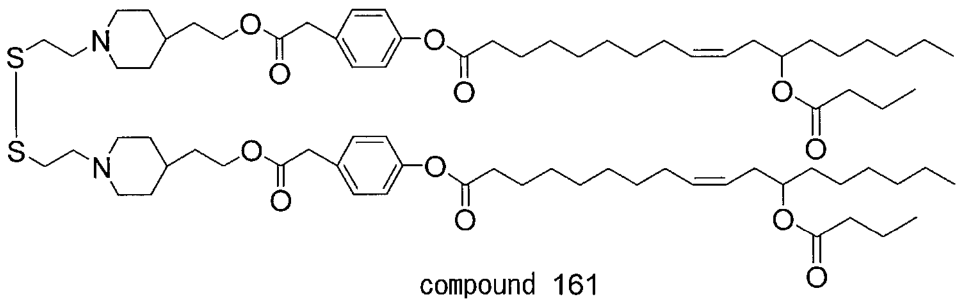

compound 161

<$^1$H-NMR (600 MHz, $CDCl_3$) of compound 161>

δ: 0.86-0.89 (m, 12H), 1.22-1.42 (m, 30H), 1.54-1.76 (m, 16H), 1.91-1.98 (m, 4H), 2.00-2.06 (m, 8H), 2.23-2.33 (m, 8H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 2H), 5.31-5.39 (m, 2H), 5.44-5.49 (m, 2H), 7.02-7.04 (m, 4H), 7.26-7.30 (m, 4H)

<Synthesis of compound 162>

Using intermediate 4 and intermediate 1 and in the same manner as in the synthesis of compound 2 described in Example 2-1, compound 162 was synthesized.

[Chem. 360]

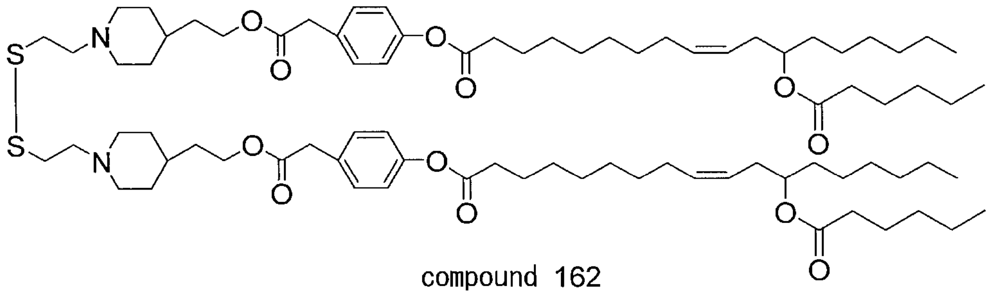

compound 162

<$^1$H-NMR (600 MHz, $CDCl_3$) of compound 162>

δ: 0.86-0.89 (m, 12H), 1.22-1.42 (m, 38H), 1.54-1.76 (m, 16H), 1.91-1.98 (m, 4H), 2.00-2.06 (m, 8H), 2.23-2.33 (m, 8H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 2H), 5.31-5.39 (m, 2H), 5.44-5.49 (m, 2H), 7.02-7.04 (m, 4H), 7.26-7.30 (m, 4H)

<Synthesis of compound 163>

Using intermediate 6 and intermediate 1 and in the same manner as in the synthesis of compound 2 described in Example 2-1, compound 163 was synthesized.

[Chem. 361]

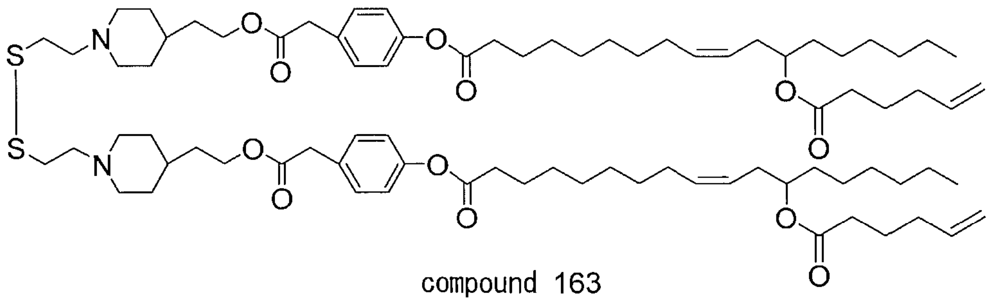

compound 163

<$^1$H-NMR (600 MHz, $CDCl_3$) of compound 163>

δ: 0.86-0.89 (m, 12H), 1.22-1.42 (m, 32H), 1.54-1.76 (m, 20H), 1.91-1.98 (m, 4H), 2.00-2.06 (m, 8H), 2.23-2.33 (m, 8H), 2.52-2.56 (m, 4H), 2.61-2.67 (m, 4H), 2.79-2.92 (m, 8H), 3.59 (s, 4H), 4.11-4.14 (t, 4H), 4.85-4.92 (m, 4H), 5.28-5.39 (m, 4H), 5.44-5.52 (m, 4H), 7.02-7.04 (m, 4H), 7.26-7.30 (m, 4H)

[Example 164] Synthesis of compound 164

<Synthesis of intermediate 61-1>

Methyl ricinoleate (4.00 g, 12.8 mmol), decanoic acid (2.32 g, 13.5 mmol), and DMAP (313 mg, 2.56 mmol) were dissolved in chloroform (40.0 g) at room temperature. To the obtained mixture was added EDC hydrochloride (3.68 g, 19.2 mmol) and the mixture was reacted at room temperature for 1 hr. The subsequent steps were performed in the same manner as in the synthesis of intermediate 2-1 described in Example 2-1 to obtain 6.08 g of intermediate 61-1.

[Chem. 362]

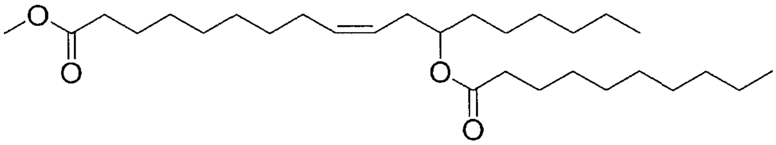

intermediate 61-1

<Synthesis of intermediate 61>

Intermediate 61 (6.08 g, 13.0 mmol) was dissolved in tert-butanol to 0.3 M at room temperature. To the obtained mixture was added 2 M NaOH aqueous solution (1.82 g) and the mixture was reacted at room temperature for 8 hr. The subsequent steps were performed in the same manner as in the synthesis of intermediate 2 described in Example 2-1 to obtain 4.92 g of intermediate 61.

[Chem. 363]

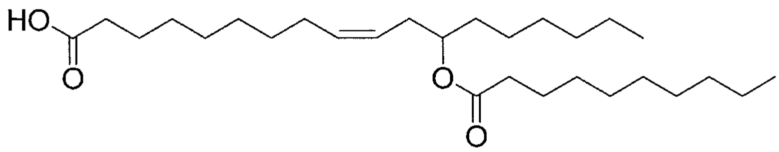

intermediate 61

<Synthesis of compound 164>

Using intermediate 61 and intermediate 1-1 and in the same manner as in the synthesis of compound 1 described in Example 1-1, compound 164 was synthesized.

[Chem. 364]

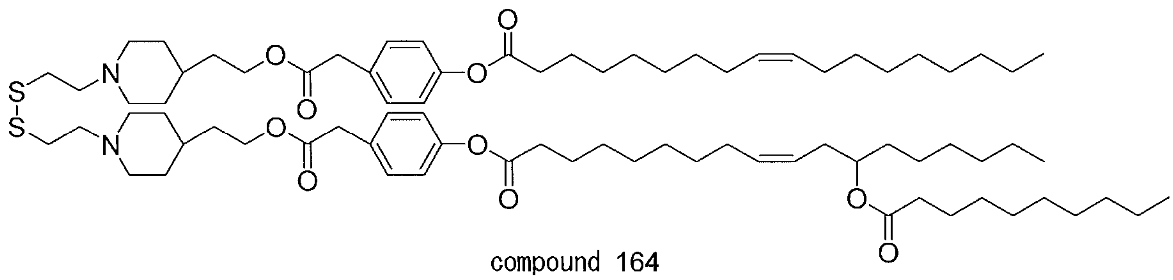

compound 164

<$^1$H-NMR (600 MHz, $CDCl_3$) of compound 164>

δ: 0.86-0.90 (m, 9H), 1.22-1.42 (m, 54H), 1.54-1.76 (m, 16H), 1.91-1.98 (m, 4H), 2.00-2.06 (m, 6H), 2.23-2.33 (m, 4H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 1H), 5.31-5.40 (m, 3H), 5.44-5.49 (m, 1H), 7.02-7.04 (m, 4H), 7.26-7.30 (m, 4H)

<Synthesis of compound 165>

Using intermediate 61 and intermediate 1 and in the same manner as in the synthesis of compound 2 described in Example 2-1, compound 165 was synthesized.

[Chem. 365]

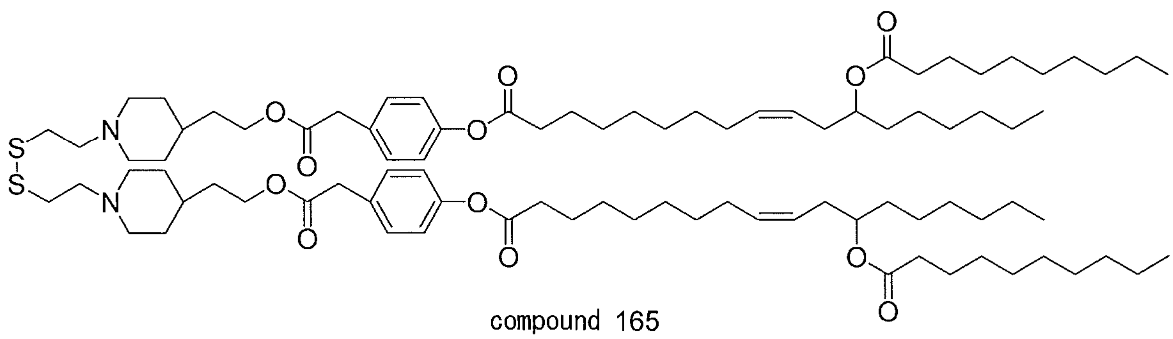

compound 165

<$^1$H-NMR (600 MHz, $CDCl_3$) of compound 165>

δ: 0.86-0.89 (m, 12H), 1.22-1.42 (m, 62H), 1.54-1.76 (m, 16H), 1.91-1.98 (m, 4H), 2.00-2.06 (m, 8H), 2.23-2.33 (m, 8H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 2H), 5.31-5.39 (m, 2H), 5.44-5.49 (m, 2H), 7.02-7.04 (m, 4H), 7.26-7.30 (m, 4H)

[Example 166] Synthesis of compound 166

<Synthesis of intermediate 62-1>

Methyl ricinoleate (4.00 g, 12.8 mmol), ethylhexanoyl acid (1.94 g, 13.5 mmol), and DMAP (313 mg, 2.56 mmol) were dissolved in chloroform (40.0 g) at room temperature. To the obtained mixture was added EDC hydrochloride (3.68 g, 19.2 mmol) and the mixture was reacted at room temperature for 1 hr. The subsequent steps were performed in the same manner as in the synthesis of intermediate 2-1 described in Example 2-1 to obtain 4.12 g of intermediate 62-1.

[Chem. 366]

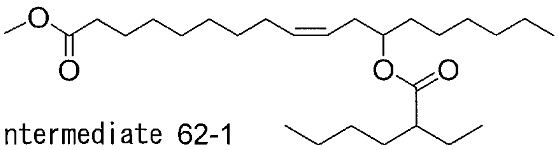

intermediate 62-1

<Synthesis of intermediate 62>

Intermediate 62-1 (2.00 g, 4.56 mmol) was dissolved in tert-butanol to 0.3 M at room temperature. To the obtained mixture was added 2 M NaOH aqueous solution (0.64 g) and the mixture was reacted at room temperature for 8 hr. The subsequent steps were performed in the same manner as in the synthesis of intermediate 2 described in Example 2-1 to obtain 1.73 g of intermediate 62.

[Chem. 367]

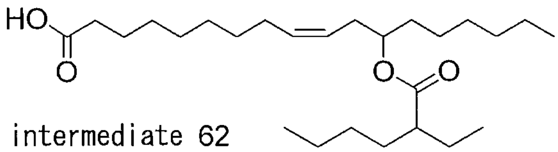

intermediate 62

<Synthesis of compound 166>

Using intermediate 62 and intermediate 1-1 and in the same manner as in the synthesis of compound 1 described in Example 1-1, compound 166 was synthesized.

[Chem. 368]

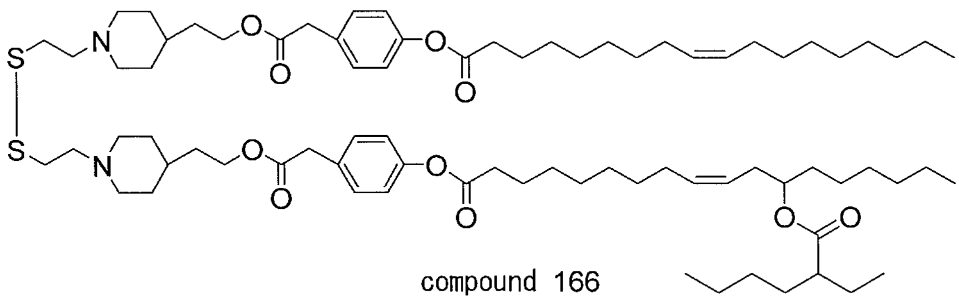

compound 166

<$^1$H-NMR (600 MHz, $CDCl_3$) of compound 166>

δ: 0.86-0.90 (m, 12H), 1.22-1.42 (m, 48H), 1.54-1.76 (m, 16H), 1.91-1.98 (m, 4H), 2.00-2.06 (m, 5H), 2.23-2.33

(m, 4H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 1H), 5.31-5.40 (m, 3H), 5.44-5.49 (m, 1H), 7.02-7.04 (m, 4H), 7.26-7.30 (m, 4H)

<Synthesis of compound 167>

Using intermediate 62 and intermediate 1 and in the same manner as in the synthesis of compound 2 described in Example 2-1, compound 167 was synthesized.

[Chem. 369]

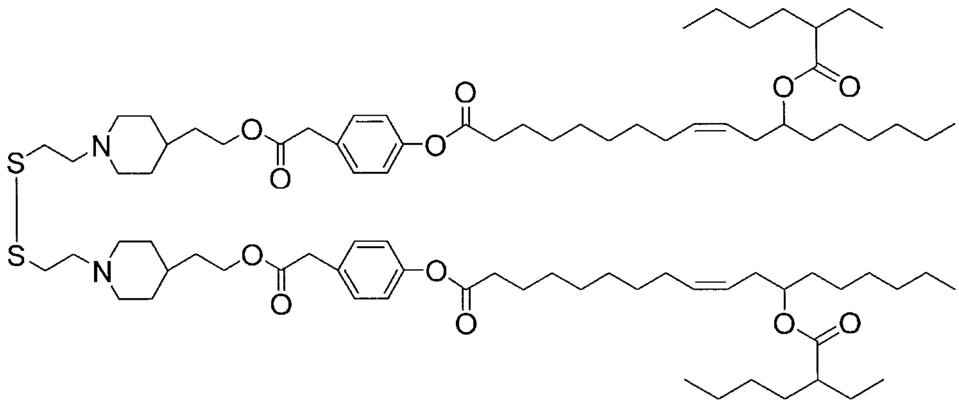

compound 167

<$^1$H-NMR (600 MHz, $CDCl_3$) of compound 167>

δ: 0.86-0.89 (m, 18H), 1.22-1.42 (m, 48H), 1.54-1.76 (m, 16H), 1.91-1.98 (m, 4H), 2.00-2.06 (m, 8H), 2.23-2.33 (m, 8H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 2H), 5.31-5.39 (m, 2H), 5.44-5.49 (m, 2H), 7.02-7.04 (m, 4H), 7.26-7.30 (m, 4H)

[Example 168] Synthesis of compound 168

<Synthesis of intermediate 63-1>

Methyl ricinoleate (4.00 g, 16.0 mmol), cyclohexanepropanoic acid (2.10 g, 13.4 mmol), and DMAP (313 mg, 2.56 mmol) were dissolved in chloroform (40.0 g) at room temperature. To the obtained mixture was added EDC hydrochloride (3.68 g, 19.2 mmol) and the mixture was reacted at room temperature for 1 hr. The subsequent steps were performed in the same manner as in the synthesis of intermediate 2-1 described in Example 2-1 to obtain 5.53 g of intermediate 63-1.

[Chem. 370]

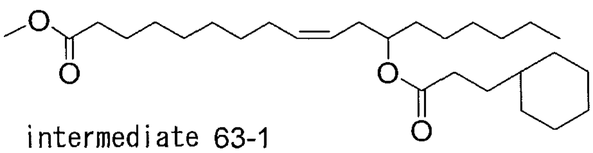

intermediate 63-1

<Synthesis of intermediate 63>

Intermediate 63-1 (5.53 g, 12.3 mmol) was dissolved in tert-butanol to 0.3 M at room temperature. To the obtained mixture was added 2 M NaOH aqueous solution (1.71 g) and the mixture was reacted at room temperature for 8 hr. The subsequent steps were performed in the same manner as in the synthesis of intermediate 2 described in Example 2-1 to obtain 2.93 g of intermediate 63.

[Chem. 371]

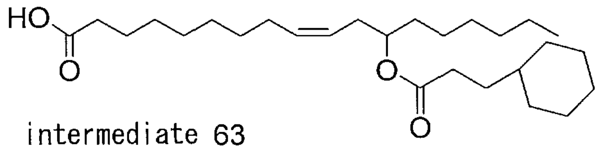

intermediate 63

<Synthesis of compound 168>

Using intermediate 63 and intermediate 1-1 and in the same manner as in the synthesis of compound 1 described in Example 1-1, compound 168 was synthesized.

[Chem. 372]

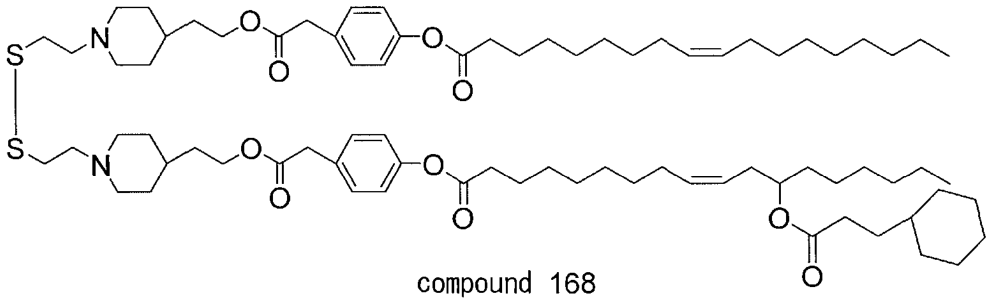

compound 168

<$^1$H-NMR (600 MHz, $CDCl_3$) of compound 168>

δ: 0.86-0.90 (m, 6H), 1.22-1.42 (m, 54H), 1.54-1.76 (m, 16H), 1.91-1.98 (m, 4H), 2.00-2.06 (m, 5H), 2.23-2.33 (m, 4H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 1H), 5.31-5.40 (m, 3H), 5.44-5.49 (m, 1H), 7.02-7.04 (m, 4H), 7.26-7.30 (m, 4H)

<Synthesis of compound 169>

Using intermediate 63 and intermediate 1 and in the same manner as in the synthesis of compound 2 described in Example 2-1, compound 169 was synthesized.

[Chem. 373]

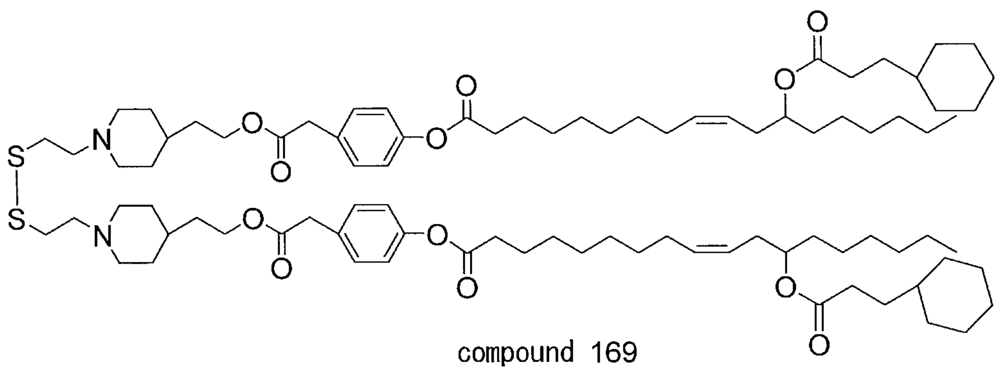

compound 169

<$^1$H-NMR (600 MHz, $CDCl_3$) of compound 169>

δ: 0.86-0.89 (m, 6H), 1.22-1.42 (m, 60H), 1.54-1.76 (m, 16H), 1.91-1.98 (m, 4H), 2.00-2.06 (m, 8H), 2.23-2.33 (m, 8H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 2H), 5.31-5.39 (m, 2H), 5.44-5.49 (m, 2H), 7.02-7.04 (m, 4H), 7.26-7.30 (m, 4H)

[Example 170] Synthesis of compound 170

<Synthesis of intermediate 64-1>

Methyl ricinoleate (4.00 g, 12.8 mmol), lipoic acid (2.77 g, 13.4 mmol), and DMAP (313 mg, 2.56 mmol) were dissolved in chloroform (40.0 g) at room temperature. To the obtained mixture was added EDC hydrochloride (3.68 g, 19.1 mmol) and the mixture was reacted at room temperature for 1 hr. The subsequent steps were performed in the same manner as in the synthesis of intermediate 2-1 described in Example 2-1 to obtain 6.34 g of intermediate 64-1.

[Chem. 374]

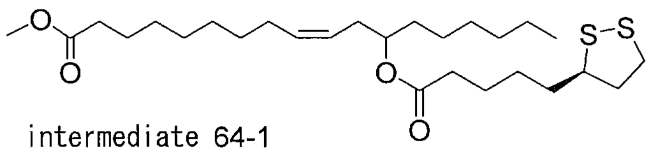

intermediate 64-1

<Synthesis of intermediate 64>

Intermediate 64-1 (6.34 g, 12.7 mmol) was dissolved in tert-butanol to 0.3 M at room temperature. To the obtained mixture was added 2 M NaOH aqueous solution (7.09 g) and the mixture was reacted at room temperature for 8 hr. The subsequent steps were performed in the same manner as in the synthesis of intermediate 2 described in Example 2-1 to obtain 5.52 g of intermediate 64.

[Chem. 375]

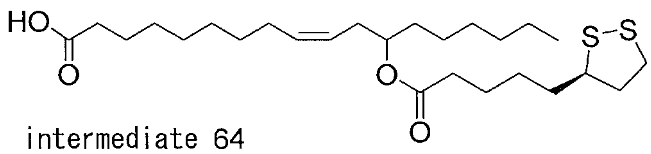

intermediate 64

<Synthesis of compound 170>

Using intermediate 64 and intermediate 1-1 and in the same manner as in the synthesis of compound 1 described in Example 1-1, compound 170 was synthesized.

[Chem. 376]

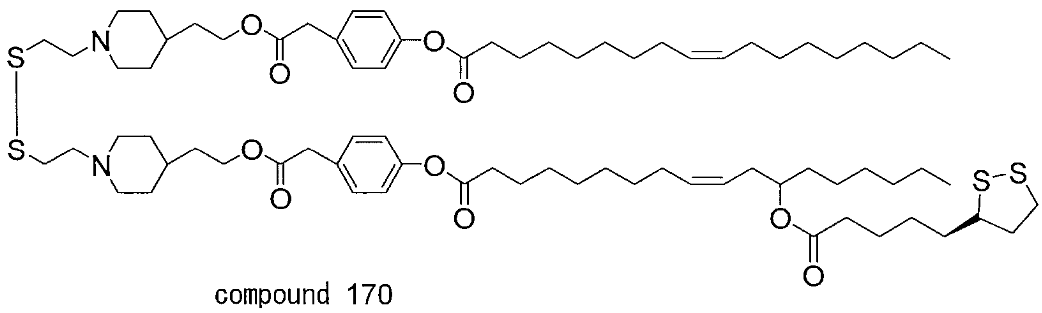

compound 170

<$^1$H-NMR (600 MHz, $CDCl_3$) of compound 170>

δ: 0.86-0.90 (m, 6H), 1.22-1.42 (m, 49H), 1.54-1.76 (m, 16H), 1.91-1.98 (m, 4H), 2.00-2.06 (m, 5H), 2.23-2.33 (m, 6H), 2.52-2.56 (m, 5H), 2.61-2.67 (m, 4H), 2.79-2.92 (m, 8H), 3.59 (s, 4H), 4.11-4.14 (t, 4H), 4.85-4.90 (m, 1H), 5.31-5.40 (m, 3H), 5.44-5.49 (m, 1H), 7.02-7.04 (m, 4H), 7.26-7.30 (m, 4H)

<Synthesis of compound 171>

Using intermediate 64 and intermediate 1 and in the same manner as in the synthesis of compound 2 described in

Example 2-1, compound 171 was synthesized.

[Chem. 377]

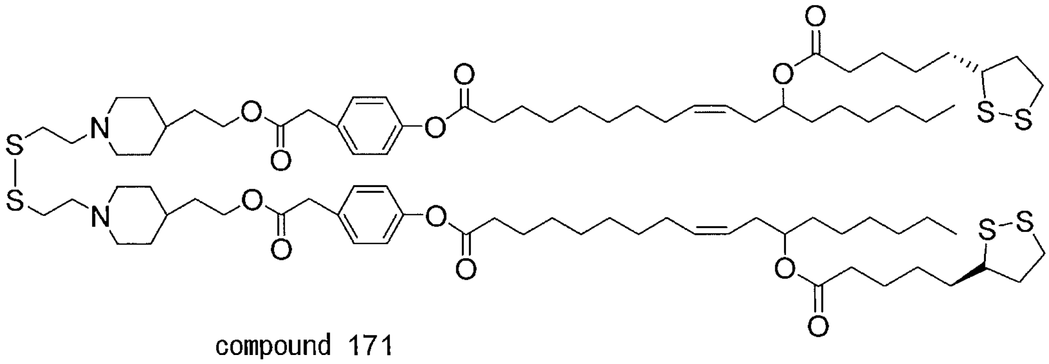

compound 171

<$^1$H-NMR (600 MHz, $CDCl_3$) of compound 171>

δ: 0.86-0.89 (m, 6H), 1.22-1.42 (m, 50H), 1.54-1.76 (m, 16H), 1.91-1.98 (m, 4H), 2.00-2.06 (m, 8H), 2.23-2.33 (m, 12H), 2.52-2.56 (m, 6H), 2.61-2.67 (m, 4H), 2.79-2.92 (m, 8H), 3.59 (s, 4H), 4.11-4.14 (t, 4H), 4.85-4.90 (m, 2H), 5.31-5.39 (m, 2H), 5.44-5.49 (m, 2H), 7.02-7.04 (m, 4H), 7.26-7.30 (m, 4H)

[Example 172] Synthesis of compound 172

<Synthesis of intermediate 65-1>

Methyl ricinoleate (4.00 g, 12.8 mmol), 3,4-diethoxybenzoic acid (2.83 g, 13.5 mmol), and DMAP (313 mg, 2.56 mmol) were dissolved in chloroform (40.0 g) at room temperature. To the obtained mixture was added EDC hydrochloride (3.68 g, 19.1 mmol) and the mixture was reacted at room temperature for 1 hr. The subsequent steps were performed in the same manner as in the synthesis of intermediate 2-1 described in Example 2-1 to obtain 5.34 g of intermediate 65-1.

[Chem. 378]

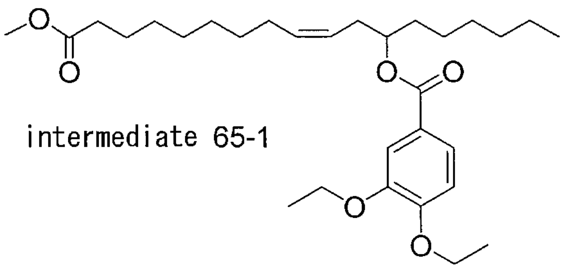

intermediate 65-1

<Synthesis of intermediate 65>

Intermediate 65-1 (2.00 g, 3.96 mmol) was dissolved in tert-butanol to 0.3 M at room temperature. To the obtained mixture was added 2 M NaOH aqueous solution (2.63 g) and the mixture was reacted at room temperature for 8 hr. The subsequent steps were performed in the same manner as in the synthesis of intermediate 2 described in Example 2-1 to obtain 1.65 g of intermediate 65.

[Chem. 379]

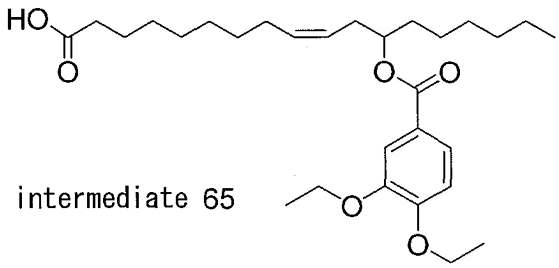

intermediate 65

<Synthesis of compound 172>

Using intermediate 65 and intermediate 1-1 and in the same manner as in the synthesis of compound 1 described in Example 1-1, compound 172 was synthesized.

[Chem. 380]

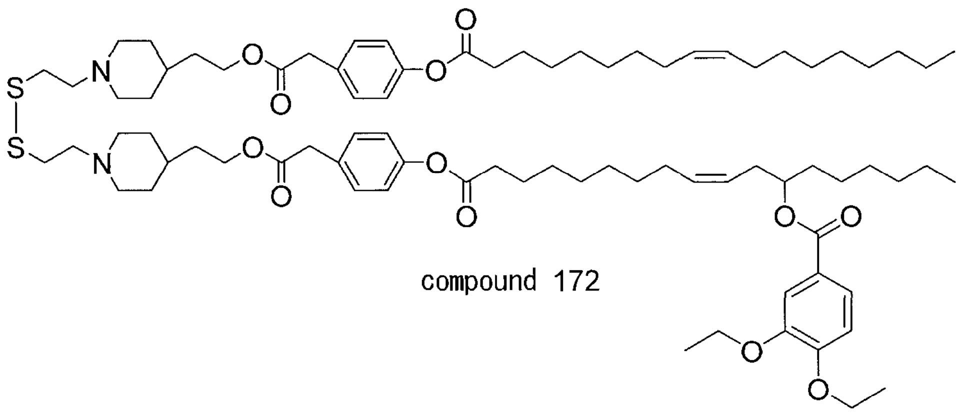

compound 172

<$^1$H-NMR (600 MHz, $CDCl_3$) of compound 172>

δ: 0.86-0.90 (m, 12H), 1.22-1.42 (m, 40H), 1.54-1.76 (m, 16H), 1.91-1.98 (m, 4H), 2.00-2.06 (m, 4H), 2.23-2.33 (m, 4H), 2.52-2.56 (m, 4H), 2.61-2.67 (m, 4H), 2.79-2.92 (m, 12H), 3.59 (s, 4H), 4.11-4.14 (t, 4H), 4.85-4.90 (m, 1H), 5.31-5.40 (m, 3H), 5.44-5.49 (m, 1H), 7.02-7.04 (m, 4H), 7.10-7.14 (m, 2H), 7.26-7.30 (m, 4H), 8.31 (s, 1H)

<Synthesis of compound 173>

Using intermediate 65 and intermediate 1 and in the same manner as in the synthesis of compound 2 described in Example 2-1, compound 173 was synthesized.

[Chem. 381]

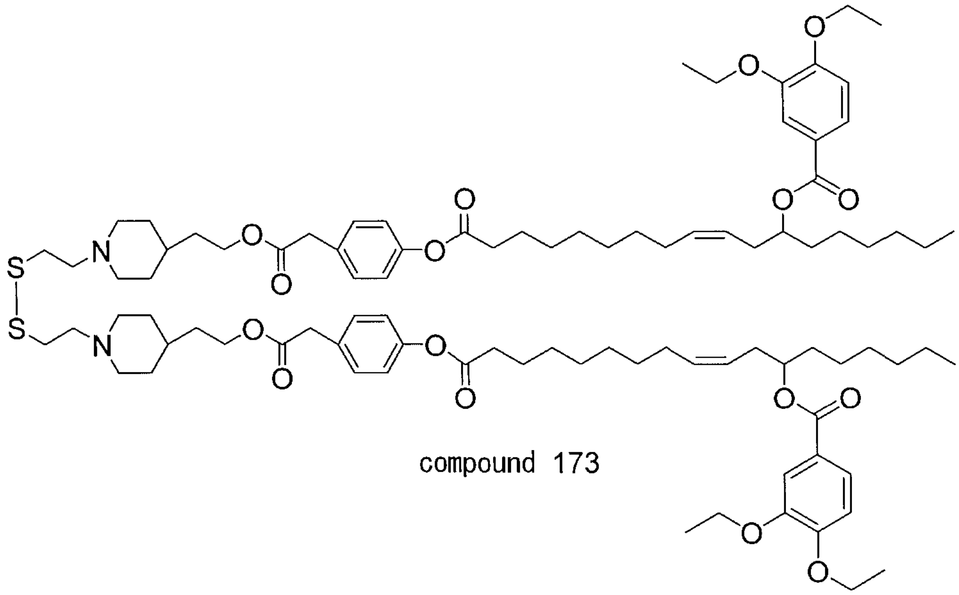

compound 173

<$^1$H-NMR (600 MHz, $CDCl_3$) of compound 173>

δ: 0.86-0.89 (m, 18H), 1.22-1.42 (m, 38H), 1.54-1.76 (m, 16H), 1.91-1.98 (m, 4H), 2.00-2.06 (m, 8H), 2.23-2.33 (m, 8H), 2.52-2.56 (m, 4H), 2.61-2.67 (m, 4H), 2.79-2.92 (m, 8H), 3.59 (s, 4H), 4.11-4.14 (t, 4H), 4.85-4.90 (m, 2H), 5.31-5.39 (m, 2H), 5.44-5.49 (m, 2H), 7.02-7.04 (m, 4H), 7.10-7.14 (m, 4H)7.26-7.30 (m, 4H), 8.31 (s, 2H)

[Example 174] Synthesis of compound 174

<Synthesis of intermediate 66-1>

Methyl ricinoleate (4.00 g, 12.8 mmol), N-octanoylglycine (2.71 g, 13.5 mmol), and DMAP (313 mg, 2.56 mmol) were dissolved in chloroform (40.0 g) at room temperature. To the obtained mixture was added EDC hydrochloride (3.68 g, 19.1 mmol) and the mixture was reacted at room temperature for 1 hr. The subsequent steps were performed in the same manner as in the synthesis of intermediate 2-1 described in Example 2-1 to obtain 5.06 g of intermediate 66-1.

[Chem. 382]

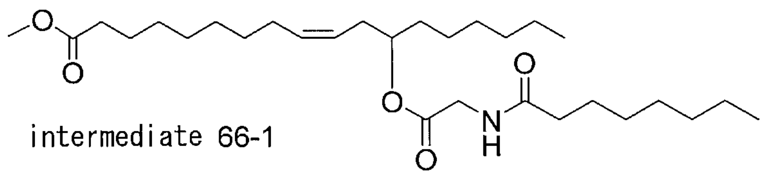

intermediate 66-1

<Synthesis of intermediate 66>

Intermediate 66-1 (2.00 g, 4.03 mmol) was dissolved in tert-butanol to 0.3 M at room temperature. To the obtained mixture was added 2 M NaOH aqueous solution (2.63 g) and the mixture was reacted at room temperature for 8 hr. The subsequent steps were performed in the same manner as in the synthesis of intermediate 2 described in Example 2-1 to obtain 1.86 g of intermediate 66.

[Chem. 383]

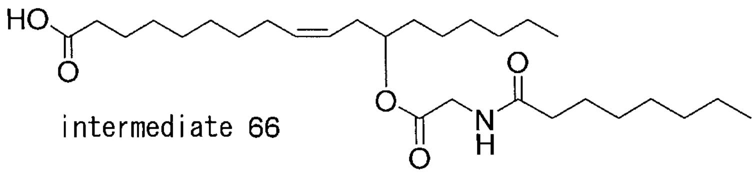

intermediate 66

<Synthesis of compound 174>

Using intermediate 66 and intermediate 1-1 and in the same manner as in the synthesis of compound 1 described in Example 1-1, compound 174 was synthesized.

[Chem. 384]

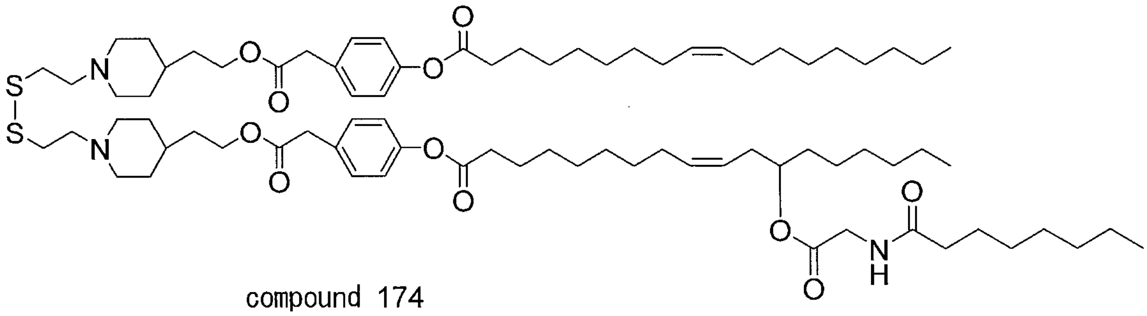

compound 174

<$^1$H-NMR (600 MHz, $CDCl_3$) of compound 174>

δ: 0.86-0.90 (m, 9H), 1.22-1.42 (m, 52H), 1.54-1.76 (m, 16H), 1.91-1.98 (m, 4H), 2.00-2.06 (m, 6H), 2.23-2.33 (m, 4H), 2.52-2.56 (m, 4H), 2.61-2.67 (m, 4H), 2.79-2.92 (m, 8H), 3.28 (d, 2H), 3.59 (s, 4H), 4.11-4.14 (t, 4H), 5.31-5.40 (m, 3H), 5.44-5.49 (m, 1H), 5.87-5.89 (m, 1H), 6.7.02-7.04 (m, 4H), 7.26-7.30 (m, 4H)

<Synthesis of compound 175>

Using intermediate 66 and intermediate 1-1 and in the same manner as in the synthesis of compound 1 described in Example 1-1, compound 175 was synthesized.

[Chem. 385]

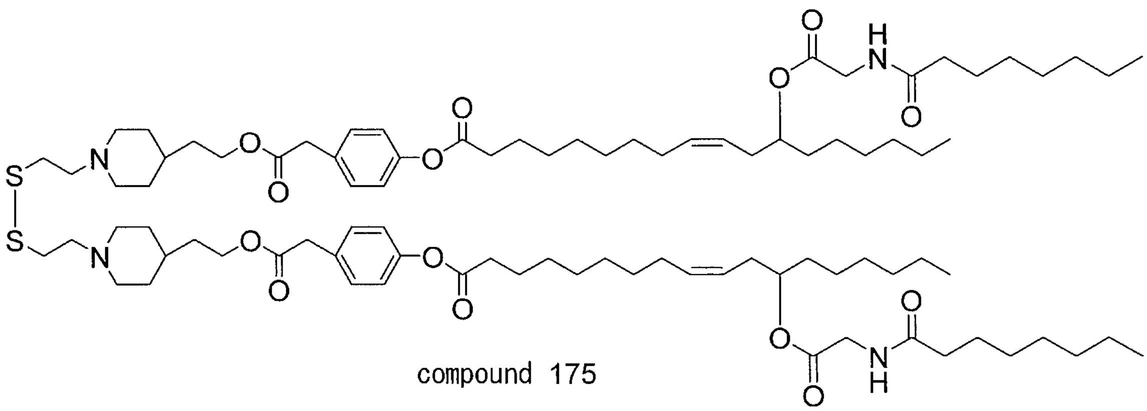

compound 175

<$^1$H-NMR (600 MHz, DMSO-$d_6$) of compound 175>

δ: 0.86-0.90 (m, 12H), 1.22-1.42 (m, 62H), 1.54-1.76 (m, 22H), 1.91-1.98 (m, 4H), 2.00-2.06 (m, 2H), 2.23-2.33 (m, 4H), 2.52-2.56 (m, 6H), 2.61-2.67 (m, 4H), 2.79-2.92 (m, 8H), 3.28 (d, 4H), 3.59 (s, 4H), 4.11-4.14 (t, 4H), 4.45-4.49 (m,

1H), 4.85-4.90 (m, 1H), 5.31-5.40 (m, 1H), 5.44-5.49 (m, 1H), 5.87-5.89 (m, 2H), 7.02-7.04 (m, 4H), 7.26-7.30 (m, 4H)

[Example 176] Synthesis of compound 176

<Synthesis of intermediate 67>

Using intermediate 40 and intermediate 1 and in the same manner as in the synthesis of intermediate 1-1 described in Example 1-1, intermediate 67 was synthesized.

[Chem. 386]

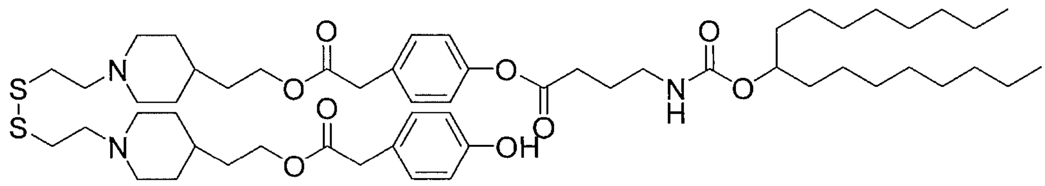

intermediate 67

<Synthesis of compound 176>

Using intermediate 67 and intermediate 44 and in the same manner as in the synthesis of compound 1 described in Example 1-1, compound 176 was synthesized.

[Chem. 387]

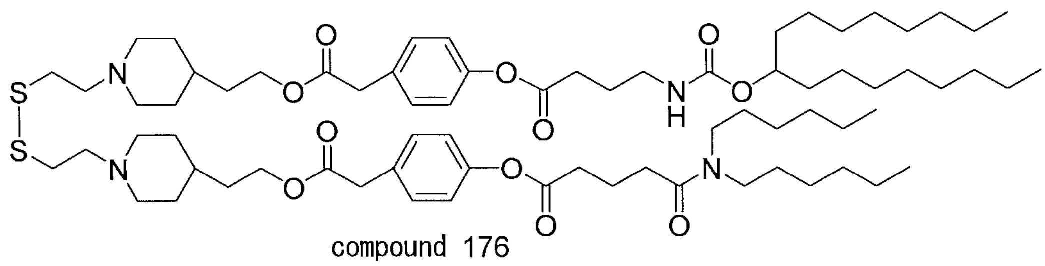

compound 176

<$^1$H-NMR (600 MHz, $CDCl_3$) of compound 176>

δ: 0.86-0.89 (m, 12H), 1.22-1.42 (m, 38H), 1.54-1.76 (m, 16H), 2.00-2.06 (m, 14H), 2.23-2.33 (m, 8H), 2.52-2.56 (m, 4H), 2.61-2.67 (m, 4H), 3.30-3.39 (m, 6H), 3.59 (s, 4H), 4.11-4.14 (t, 4H), 4.43-4.52 (m, 1H), 6.83-6.84 (m, 1H), 7.02-7.04 (m, 4H), 7.26-7.30 (m, 4H)

[Example 177] Synthesis of compound 177

<Synthesis of compound 177>

Using intermediate 67 and intermediate 52 and in the same manner as in the synthesis of compound 1 described in Example 1-1, compound 177 was synthesized.

[Chem. 388]

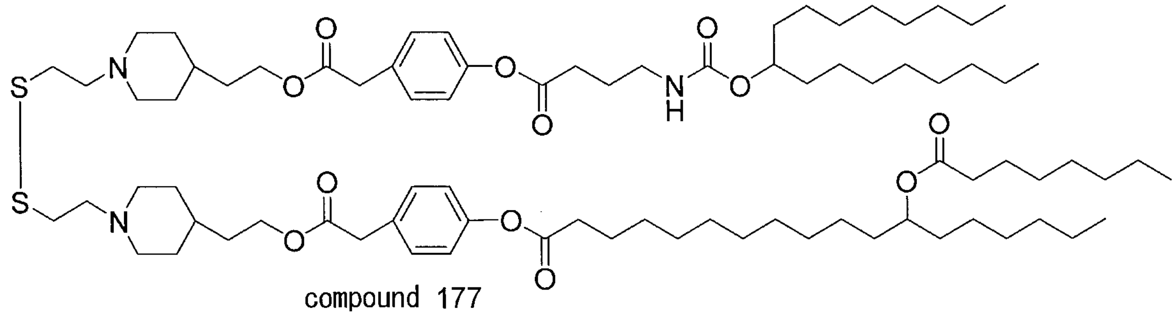

compound 177

<$^1$H-NMR (600 MHz, $CDCl_3$) of compound 177>

δ: 0.86-0.89 (m, 12H), 1.22-1.42 (m, 78H), 1.54-1.76 (m, 4H), 1.91-1.98 (m, 2H), 2.00-2.06 (m, 8H), 2.23-2.33 (m, 4H), 2.52-2.56 (m, 4H), 2.61-2.67 (m, 4H), 3.30-3.33 (m, 2H), 3.59 (s, 4H), 4.11-4.14 (t, 4H), 4.43-4.52 (m, 2H), 6.83-6.84 (m, 1H), 7.02-7.04 (m, 4H), 7.26-7.30 (m, 4H)

[Example 178] Synthesis of compound 178

<Synthesis of compound 178>

Using intermediate 67 and ricinoleic acid and in the same manner as in the synthesis of compound 1 described in Example 1-1, compound 178 was synthesized.

[Chem. 389]

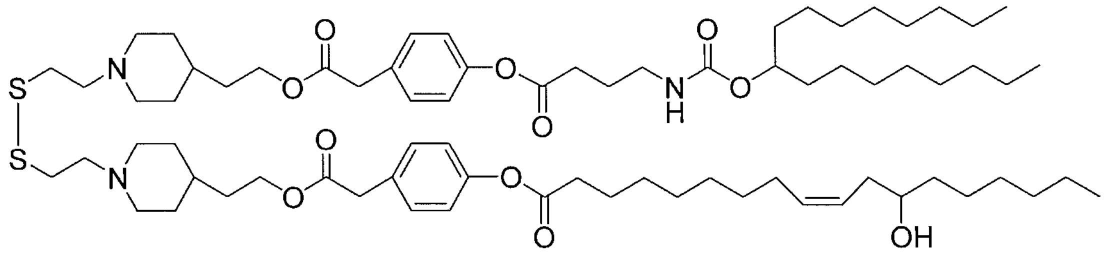

compound 178

<$^1$H-NMR (600 MHz, $CDCl_3$) of compound 178>

δ: 0.86-0.89 (m, 9H), 1.22-1.42 (m, 56H), 1.54-1.76 (m, 6H), 1.91-1.98 (m, 4H), 2.00-2.06 (m, 10H), 2.23-2.33 (m, 4H), 2.52-2.56 (m, 4H), 2.61-2.67 (m, 4H), 3.30-3.33 (m, 2H), 3.59 (s, 4H), 4.11-4.14 (m, 5H), 4.43-4.52 (m, 1H), 5.31-5.40 (m, 1H), 5.44-5.49 (m, 1H), 5.72-5.73 (m, 1H), 6.83-6.84 (m, 1H), 7.02-7.04 (m, 4H), 7.26-7.30 (m, 4H)

[Example 179] Synthesis of compound 179

<Synthesis of intermediate 68>

Using intermediate 44 and intermediate 1 and in the same manner as in the synthesis of intermediate 1-1 described in Example 1-1, intermediate 68 was synthesized.

[Chem. 390]

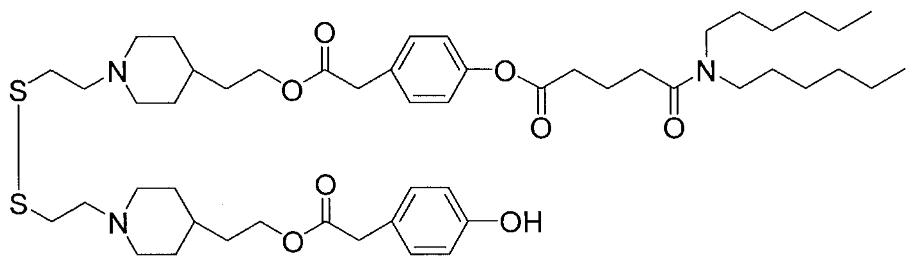

intermediate 68

<Synthesis of compound 179>

Using intermediate 68 and intermediate 52 and in the same manner as in the synthesis of compound 1 described in Example 1-1, compound 179 was synthesized.

[Chem. 391]

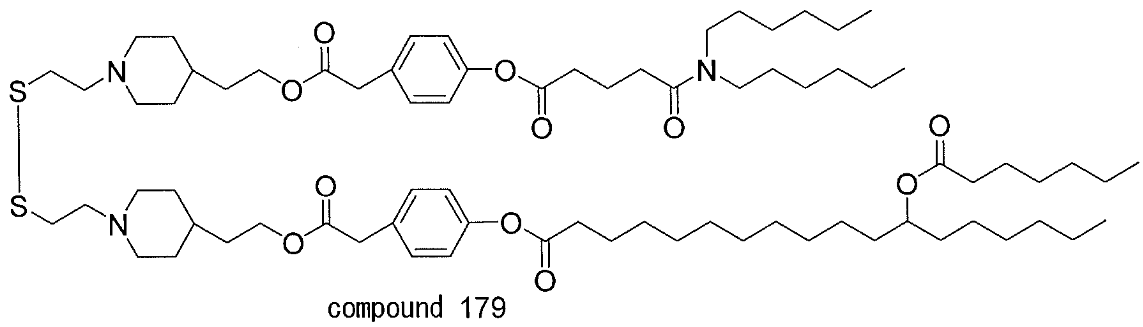

compound 179

<$^1$H-NMR (600 MHz, $CDCl_3$) of compound 180>

δ: 0.86-0.89 (m, 12H), 1.22-1.42 (m, 58H), 1.54-1.76 (m, 12H), 1.91-1.98 (m, 2H), 2.00-2.06 (m, 10H), 2.23-2.33 (m, 4H), 2.52-2.56 (m, 4H), 2.61-2.67 (m, 4H), 3.30-3.39 (m, 4H), 3.59 (s, 4H), 4.11-4.14 (t, 4H), 4.40-4.48 (m, 1H), 7.02-7.04 (m, 4H), 7.26-7.30 (m, 4H)

[Example 180] Synthesis of compound 180

<Synthesis of compound 180>

Using intermediate 68 and ricinoleic acid and in the same manner as in the synthesis of compound 1 described in Example 1- 1, compound 180 was synthesized.

[Chem. 392]

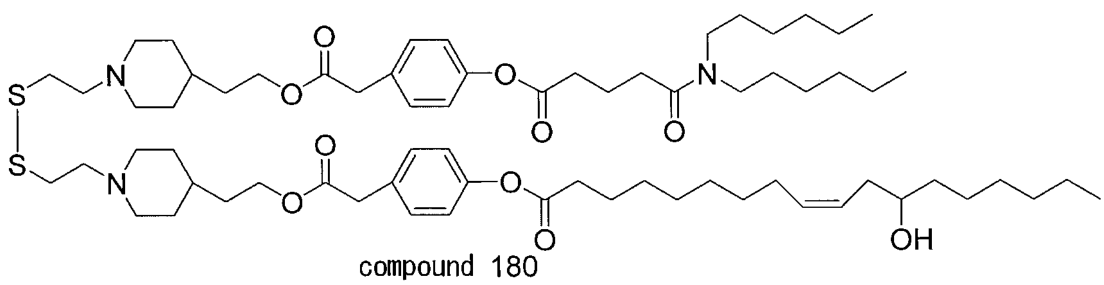

compound 180

<$^1$H-NMR (600 MHz, $CDCl_3$) of compound 180>

δ: 0.86-0.89 (m, 9H), 1.22-1.42 (m, 32H), 1.54-1.76 (m, 20H), 1.91-1.98 (m, 4H), 2.00-2.06 (m, 10H), 2.23-2.33 (m, 4H), 2.52-2.56 (m, 4H), 2.61-2.67 (m, 4H), 3.30-3.39 (m, 4H), 3.59 (s, 4H), 4.11-4.14 (t, 4H), 4.40-4.48 (m, 1H), 5.31-5.40 (m, 1H), 5.44-5.49 (m, 1H), 5.72-5.73 (m, 1H), 7.02-7.04 (m, 4H), 7.26-7.30 (m, 4H)

<Synthesis of compound 181 to compound 187>

Compound 181 to compound 187 were synthesized by the following synthesis pathways.

[Chem. 393]

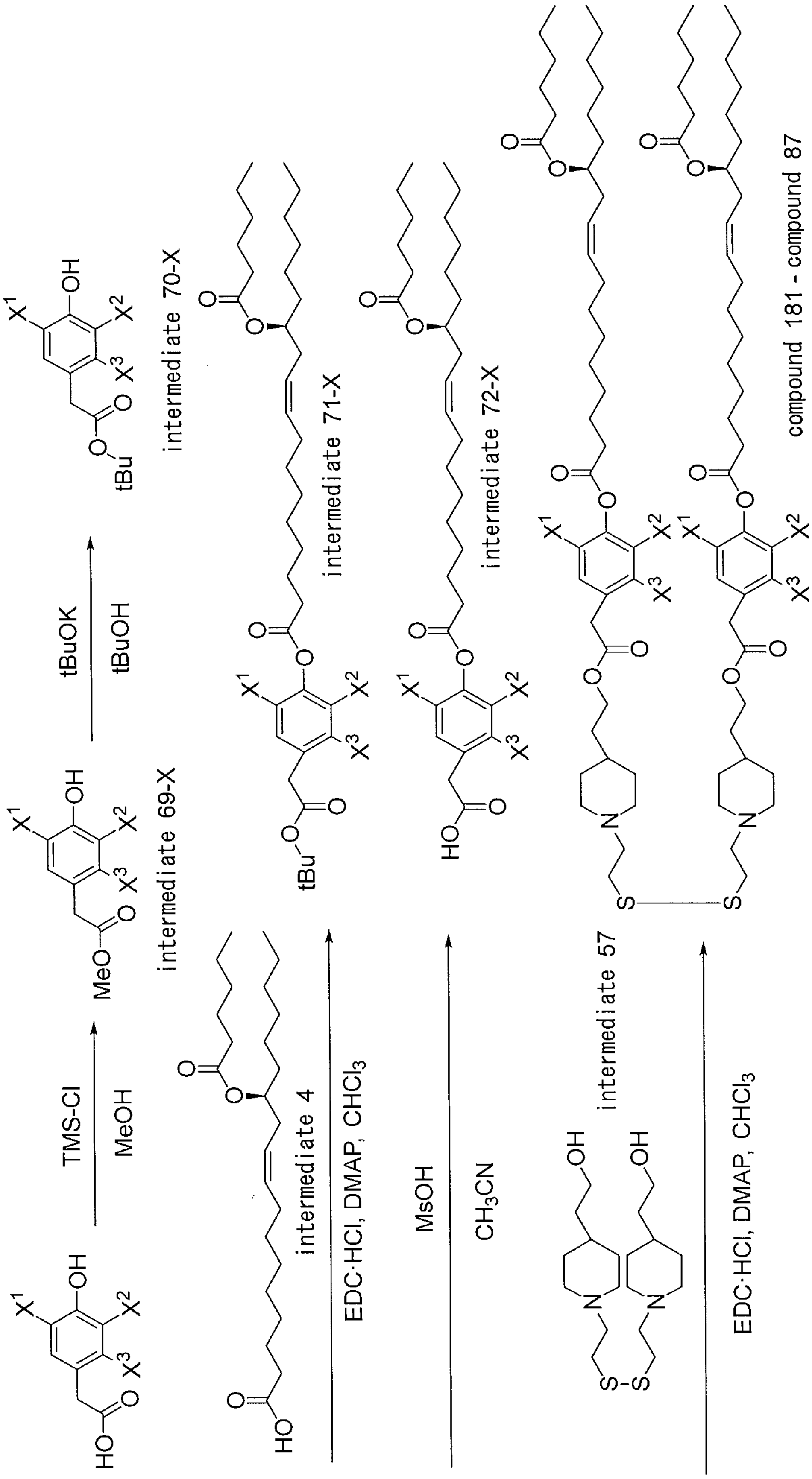


intermediate 69-X

intermediate 70-X

intermediate 4

intermediate 71-X

intermediate 72-X

intermediate 57

compound 181 - compound 87

In the above-mentioned synthesis pathway, "intermediate 69-X" shows any of intermediate 69-1 to intermediate 69-7, "intermediate 70-X" shows any of intermediate 70-1 to intermediate 70-7, "intermediate 71-X" shows any of intermediate 71-1 to intermediate 71-7, and "intermediate 72-X" shows any of intermediate 72-1 to intermediate 72-7.

In intermediate 69-1, intermediate 70-1, intermediate 71-1, intermediate 72-1, and compound 181, $X^1$ is OMe, $X^2$ is H, and $X^3$ is H, in the above-mentioned synthesis pathway.

In intermediate 69-2, intermediate 70-2, intermediate 71-2, intermediate 72-2, and compound 182, $X^1$ is Cl, $X^2$ is H, and $X^3$ is H, in the above-mentioned synthesis pathway.

In intermediate 69-3, intermediate 70-3, intermediate 71-3, intermediate 72-3, and compound 183, $X^1$ is OMe, $X^2$ is OMe, and $X^3$ is H, in the above-mentioned synthesis pathway.

In intermediate 69-4, intermediate 70-4, intermediate 71-4, intermediate 72-4, and compound 184, $X^1$ is Me, $X^2$ is H, and $X^3$ is H, in the above-mentioned synthesis pathway.

In intermediate 69-5, intermediate 70-5, intermediate 71-5, intermediate 72-5, and compound 185, $X^1$ is F, $X^2$ is H, and $X^3$ is H, in the above-mentioned synthesis pathway.

In intermediate 69-6, intermediate 70-6, intermediate 71-6, intermediate 72-6, and compound 186, $X^1$ is Br, $X^2$ is H, and $X^3$ is H, in the above-mentioned synthesis pathway.

In intermediate 69-7, intermediate 70-7, intermediate 71-7, intermediate 72-7, and compound 187, $X^1$ is H, $X^2$ is H, and $X^3$ is Br, in the above-mentioned synthesis pathway.

[Example 181] Synthesis of compound 181

<Synthesis of intermediate 69-1>

4-Hydroxy-3-methoxyphenylacetic acid (4.00 g, 22.0 mmol) was dissolved in methanol (50.5 mL) at room temperature. To the obtained mixture was added chlorotrimethylsilane (4.77 g) and the mixture was reacted at room temperature for 2 hr. Thereafter, to the reaction solution was added ethyl acetate (88.9 mL), and the mixture was washed with 7 wt% sodium hydrogen carbonate aqueous solution, and 20 wt% saline, and dehydrated by adding sodium sulfate. Sodium sulfate was removed by filtration, and the filtrate was concentrated by an evaporator to give 3.80 g of intermediate 69-1.

[Chem. 394]

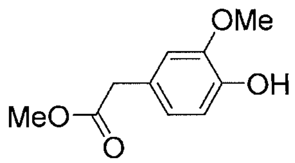

intermediate 69-1

<Synthesis of intermediate 70-1>

Intermediate 69-1 (2.00 g, 10.2 mmol) was dissolved in tert-butyl alcohol (25.6 mL) at room temperature. To the obtained mixture was added potassium tert-butoxide (3.43 g, 30.6 mmol), and the mixture was reacted at 45°C for 5 hr. Thereafter, to the reaction solution were added tert-butyl alcohol (11.5 mL) and dichloromethane (4.51 mL), and then phosphoric acid (3.00 g). The precipitated solid was removed by filtration, the filtrate was extracted with 5 wt% sodium hydrogen carbonate aqueous solution and dichloromethane, and the organic layer was concentrated by an evaporator to give 956 mg of intermediate 70-1.

[Chem. 395]

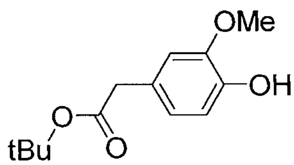

intermediate 70-1

<Synthesis of intermediate 71-1>

Intermediate 70-1 (278 mg, 1.17 mmol) was dissolved in chloroform (2.78 g), intermediate 4 (491 mg, 1.29 mmol), DMAP (28.5 mg, 0.234 mmol) and EDC hydrochloride (335 mg, 1.76 mmol) were added and the mixture was reacted at

room temperature for 2 hr. The solution after the reaction was washed with water, 5 wt% sodium dihydrogen phosphate aqueous solution, and 20 wt% saline, and dehydrated by adding sodium sulfate. Sodium sulfate was removed by filtration, and the filtrate was concentrated by an evaporator to give 673 mg of intermediate 71-1.

[Chem. 396]

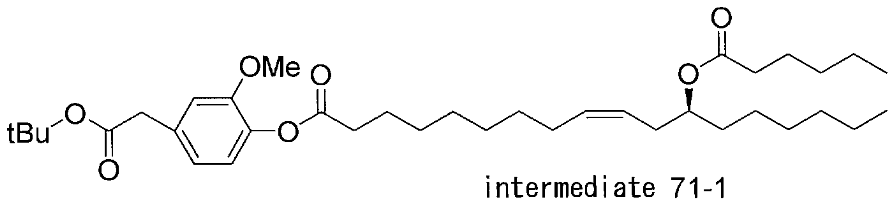

intermediate 71-1

<Synthesis of intermediate 72-1>

Intermediate 71-1 (673 mg, 1.09 mmol) was dissolved in acetonitrile (3.36 g), mesylic acid (168 mg, 1.70 mmol) was added and the mixture was reacted at room temperature for 2 hr. Thereafter, to the reaction solution was added chloroform (3.37 g), and the mixture was washed with reverse osmosis water. To the obtained solution was added DMAP and the mixture was stirred for 30 min, washed with 0.1 M hydrochloric acid aqueous solution, and 20 wt% saline, and dehydrated by adding sodium sulfate. Sodium sulfate was removed by filtration, and concentration by an evaporator and silica gel column purification using heptane/ethyl acetate were performed to give 340 mg of intermediate 72-1.

[Chem. 397]

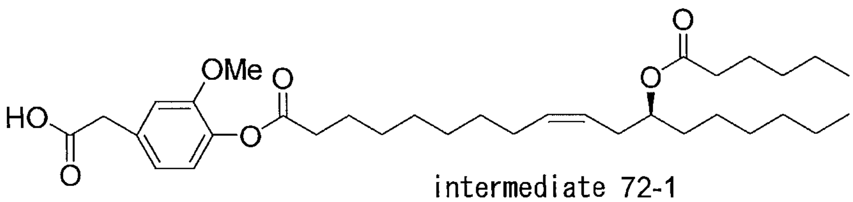

intermediate 72-1

<Synthesis of compound 181>

Intermediate 72-1 (337 mg, 0.601 mmol) was dissolved in chloroform (3.48 g), intermediate 57 (116 mg, 0.308 mmol), DMAP (15.1 mg, 0.124 mmol) and EDC hydrochloride (177 mg, 0.923 mmol) were added and the mixture was reacted at room temperature for 2 hr. The solution after the reaction was washed with 5 wt% sodium dihydrogen phosphate aqueous solution, 5 wt% sodium hydrogen carbonate aqueous solution, and 20 wt% saline, and dehydrated by adding sodium sulfate. Sodium sulfate was removed by filtration, and the mixture was concentrated by an evaporator and purified by silica gel column using chloroform/ethanol to give 334 mg of compound 181.

[Chem. 398]

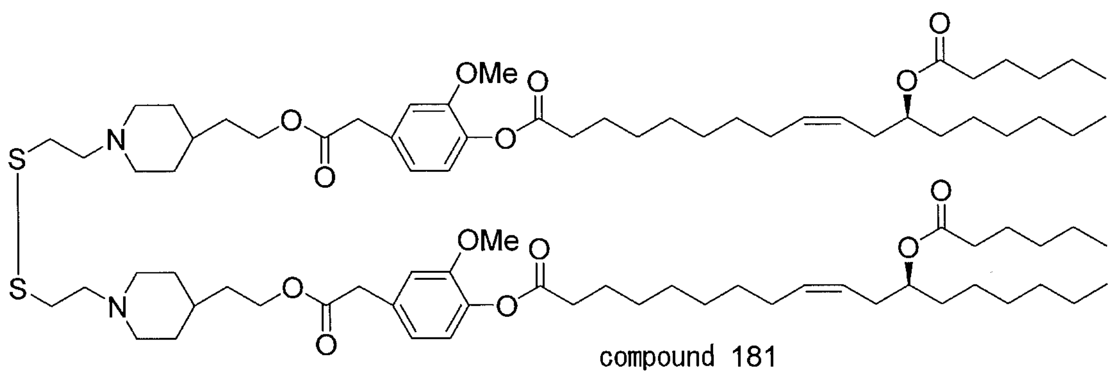

compound 181

<$^1$H-NMR (400 MHz, $CDCl_3$) of compound 181>

δ: 0.84-0.92 (m, 12H), 1.22-1.45 (m, 46H), 1.45-1.70 (m, 16H), 1.79 (dt, 4H), 1.92-2.07 (m, 8H), 2.22-2.36 (t, 8H), 2.62-2.68 (m, 8H), 2.80-2.93 (m, 8H), 3.58 (s, 4H), 3.81 (s, 6H), 4.13 (t, 4H), 4.87-4.90 (m, 2H), 5.32-5.36 (m, 2H), 5.44-5.48 (m, 2H), 6.86 (dd, 2H), 6.90 (d, 2H), 6.96 (d, 2H)

[Example 182] Synthesis of compound 182

<synthesis of intermediate 69-2>

Using 3-chloro-4-hydroxyphenylacetic acid and in the same manner as in the synthesis of intermediate 69-1 described in Example 181, intermediate 69-2 was synthesized.

[Chem. 399]

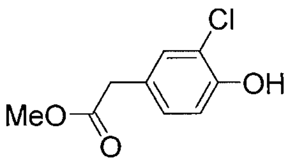

intermediate 69-2

<Synthesis of intermediate 70-2>

Using intermediate 69-2 and in the same manner as in the synthesis of compound Y-1 described in Example 181, intermediate 70-2 was synthesized.

[Chem. 400]

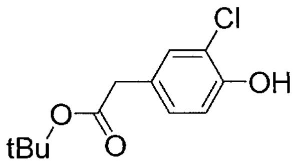

intermediate 70-2

<Synthesis of intermediate 71-2>

Using intermediate 70-2 and intermediate 4 and in the same manner as in the synthesis of intermediate 61-1 described in Example 181, intermediate 71-2 was synthesized.

[Chem. 401]

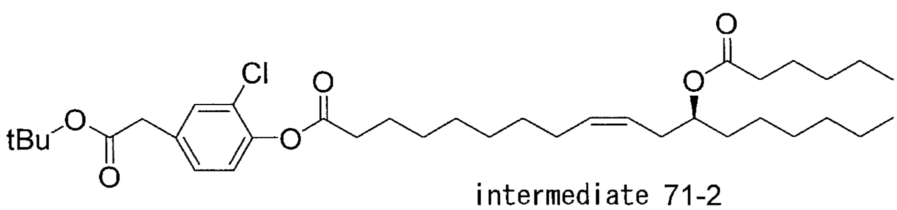

intermediate 71-2

<Synthesis of intermediate 72-2>

Using intermediate 71-2 and in the same manner as in the synthesis of intermediate 62-1 described in Example 181, intermediate 72-2 was synthesized.

[Chem. 402]

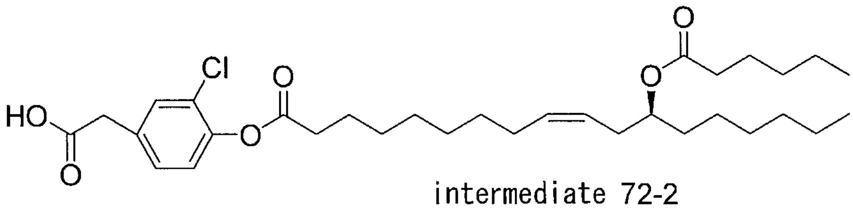

intermediate 72-2

<Synthesis of compound 182>

Using intermediate 72-2 and intermediate 57 and in the same manner as in the synthesis of compound 181 described in Example 181, compound 182 was synthesized.

[Chem. 403]

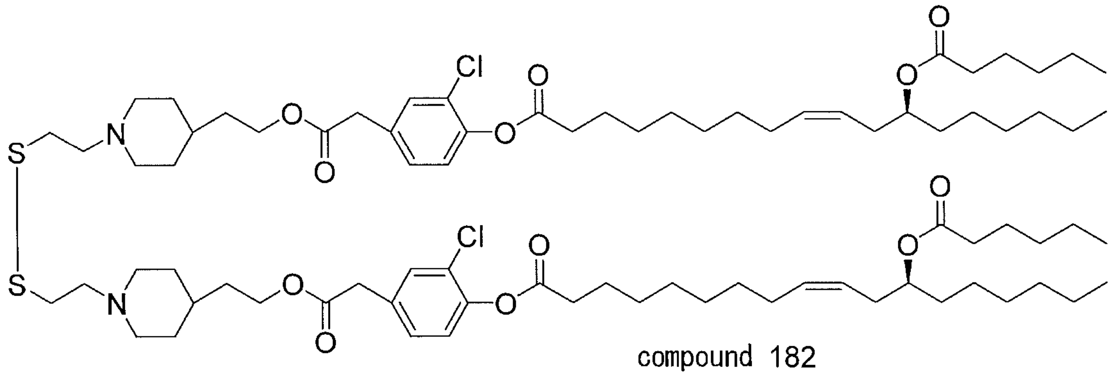

compound 182

<$^1$H-NMR (400 MHz, $CDCl_3$) of compound 182>

δ: 0.84-0.92 (m, 12H), 1.22-1.45 (m, 46H), 1.45-1.70 (m, 16H), 1.79 (dt, 4H), 1.92-2.07 (m, 8H), 2.22-2.36 (t, 8H), 2.62-2.68 (m, 8H), 2.80-2.93 (m, 8H), 3.58 (s, 4H), 4.14 (t, 4H), 4.87-4.90 (m, 2H), 5.32-5.36 (m, 2H), 5.44-5.48 (m, 2H), 7.07 (d, 2H), 7.19 (dd, 2H), 7.38 (d, 2H)

[Example 183] Synthesis of compound 183

<Synthesis of intermediate 69-3>

Using 4-hydroxy-3,5-dimethoxyphenylacetic acid and in the same manner as in the synthesis of intermediate 69-1 described in Example 181, intermediate 69-3 was synthesized.

[Chem. 404]

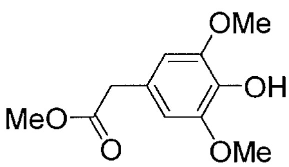

intermediate 69-3

<Synthesis of intermediate 70-3>

Using intermediate 69-3 and in the same manner as in the synthesis of compound 69-1 described in Example 181, intermediate 70-3 was synthesized.

[Chem. 405]

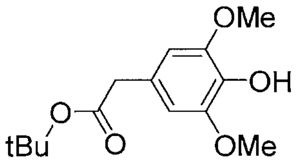

intermediate 70-3

<Synthesis of intermediate 71-3>

Using intermediate 70-3 and intermediate 4 and in the same manner as in the synthesis of intermediate 71-1 described in Example 181, intermediate 71-3 was synthesized.

[Chem. 406]

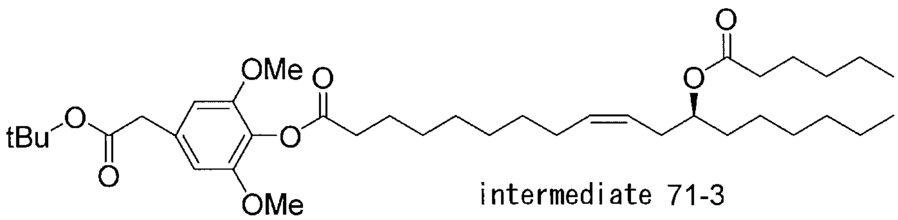

intermediate 71-3

<Synthesis of intermediate 72-3>

Using intermediate 71-3 and in the same manner as in the synthesis of intermediate 72-1 described in Example 181, intermediate 72-3 was synthesized.

[Chem. 407]

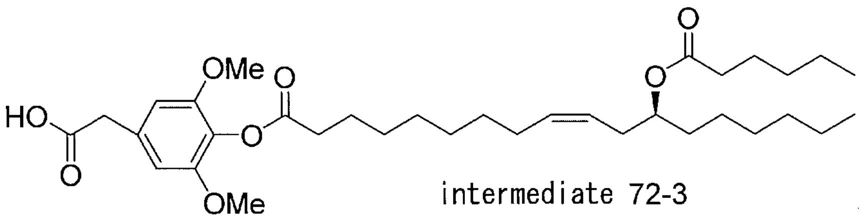

intermediate 72-3

<Synthesis of compound 183>

Using intermediate 72-3 and intermediate 57 and in the same manner as in the synthesis of compound 181 described in Example 181, compound 183 was synthesized.

[Chem. 408]

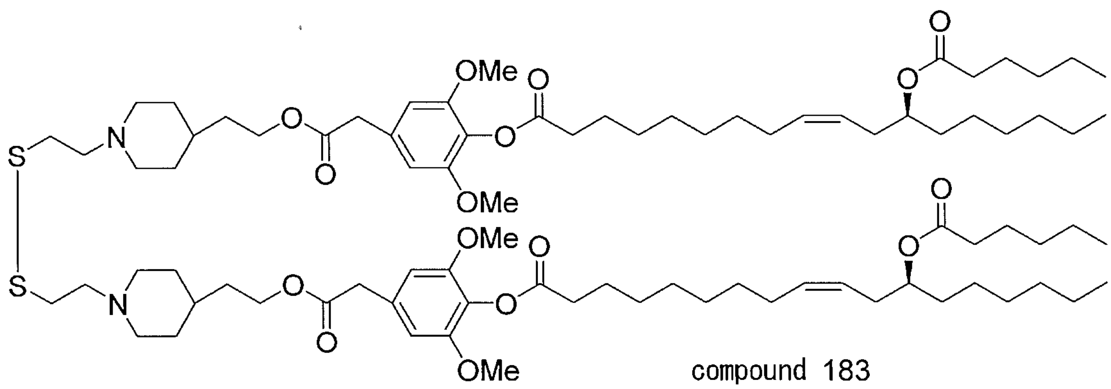

compound 183

<$^1$H-NMR (400 MHz, $CDCl_3$) of compound 183>

δ: 0.84-0.92 (m, 12H), 1.22-1.45 (m, 46H), 1.45-1.70 (m, 16H), 1.79 (dt, 4H), 1.92-2.07 (m, 8H), 2.22-2.36 (t, 8H), 2.62-2.68 (m, 8H), 2.80-2.93 (m, 8H), 3.58 (s, 4H), 3.80 (s, 12H), 4.14 (t, 4H), 4.87-4.90 (m, 2H), 5.32-5.36 (m, 2H), 5.44-5.48 (m, 2H), 6.53 (s, 4H)

[Example 184] Synthesis of compound 184

<Synthesis of intermediate 69-4>

Using 4-hydroxy-3-methylphenylacetic acid and in the same manner as in the synthesis of intermediate 69-1 described in Example 181, intermediate 69-4 was synthesized.

[Chem. 409]

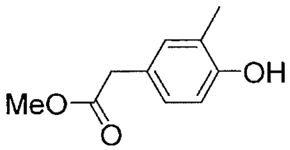

intermediate 69-4

<Synthesis of intermediate 70-4>

Using intermediate 69-4 and in the same manner as in the synthesis of compound 70-1 described in Example 181, intermediate 70-4 was synthesized.

[Chem. 410]

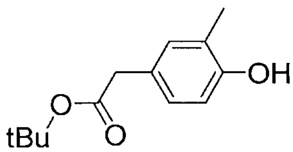

intermediate 70-4

<Synthesis of intermediate 71-4>

Using intermediate 70-4 and intermediate 4 and in the same manner as in the synthesis of intermediate 71-1 described in Example 181, intermediate 71-4 was synthesized.

[Chem. 411]

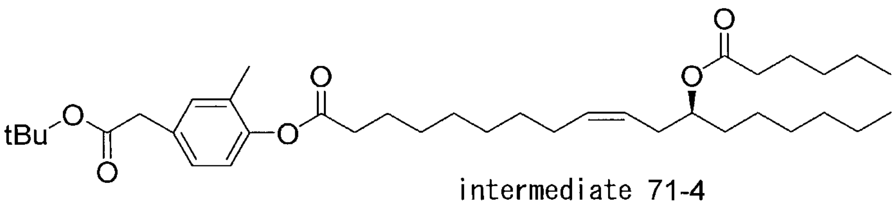

intermediate 71-4

<Synthesis of intermediate 72-4>

Using intermediate 71-4 and in the same manner as in the synthesis of intermediate 72-1 described in Example 181, intermediate 72-4 was synthesized.

[Chem. 412]

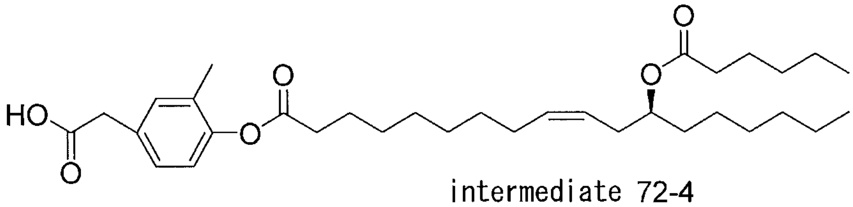

intermediate 72-4

<Synthesis of compound 184>

Using intermediate 72-4 and intermediate 57 and in the same manner as in the synthesis of compound 181 described in Example 181, compound 184 was synthesized.

[Chem. 413]

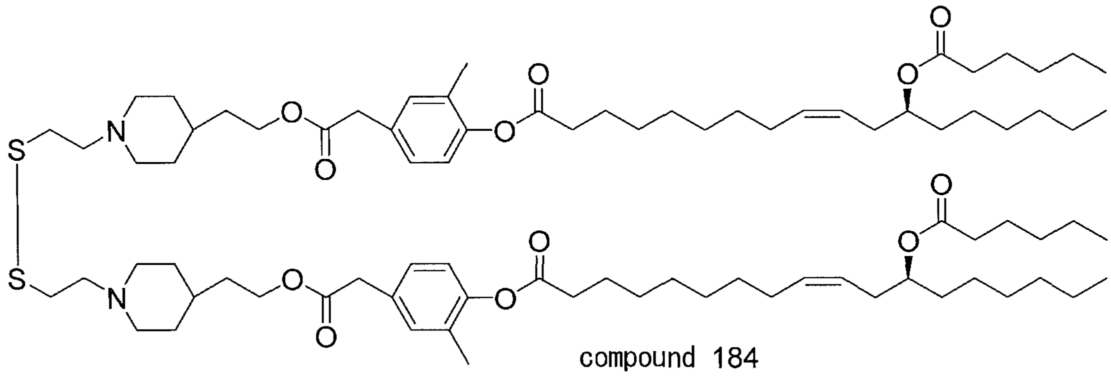

compound 184

<$^1$H-NMR (400 MHz, $CDCl_3$) of compound 184>

δ: 0.84-0.92 (m, 12H), 1.22-1.45 (m, 46H), 1.45-1.70 (m, 16H), 1.79 (dt, 4H), 1.92-2.07 (m, 8H), 2.15 (s, 6H), 2.22-2.36 (t, 8H), 2.62-2.68 (m, 8H), 2.80-2.93 (m, 8H), 3.58 (s, 4H), 4.14 (t, 4H), 4.87-4.90 (m, 2H), 5.32-5.36 (m, 2H), 5.44-5.48 (m, 2H), 7.04 (dd, 2H), 7.10 (d, 2H), 7.35 (d, 2H)

[Example 185] Synthesis of compound 185

<Synthesis of intermediate 69-5>

Using 3-fluoro-4-hydroxyphenylacetic acid and in the same manner as in the synthesis of intermediate 69-1 described in Example 181, intermediate 69-5 was synthesized.

[Chem. 414]

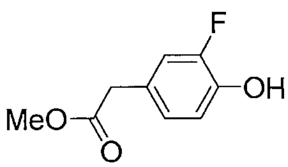

intermediate 69-5

<Synthesis of intermediate 70-5>

Using intermediate 69-5 and in the same manner as in the synthesis of compound 69-1 described in Example 181, intermediate 70-5 was synthesized.

[Chem. 415]

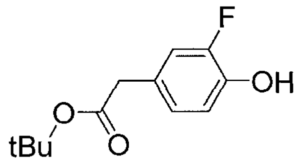

intermediate 70-5

<Synthesis of intermediate 71-5>

Using intermediate 70-5 and intermediate 4 and in the same manner as in the synthesis of intermediate 71-1 described in Example 181, intermediate 71-5 was synthesized.

[Chem. 416]

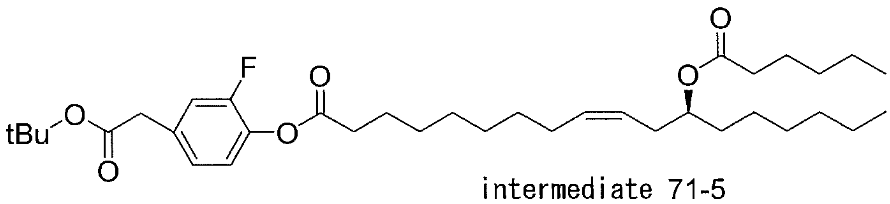

intermediate 71-5

<Synthesis of intermediate 72-5>

Using intermediate 71-5 and in the same manner as in the synthesis of intermediate 72-1 described in Example 181, intermediate 72-5 was synthesized.

[Chem. 417]

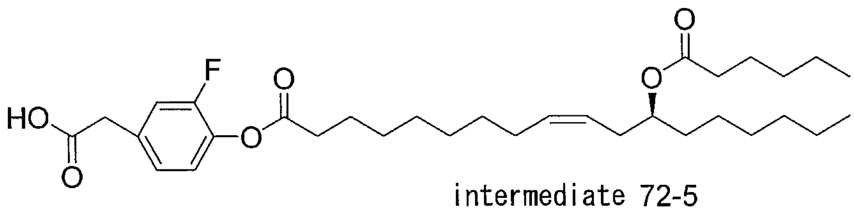

intermediate 72-5

<Synthesis of compound 185>

Using intermediate 72-5 and intermediate 57 and in the same manner as in the synthesis of compound 181 described in Example 181, compound 185 was synthesized.

[Chem. 418]

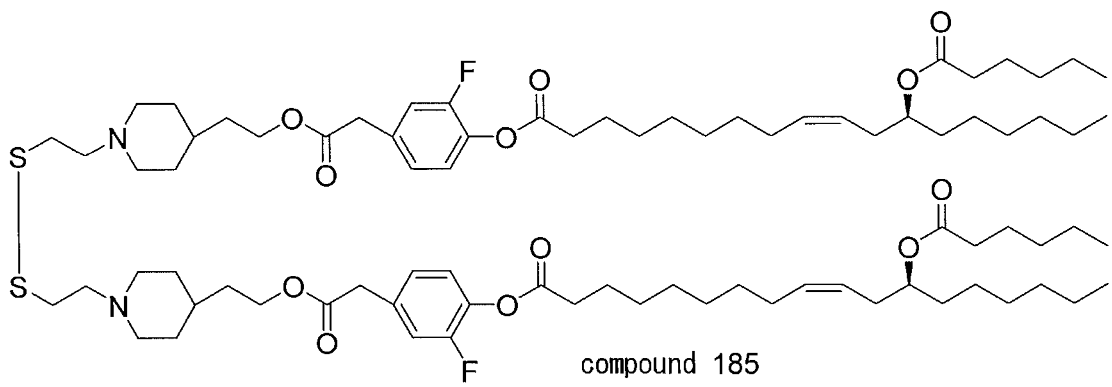

compound 185

<$^1$H-NMR (400 MHz, $CDCl_3$) of compound 185>

δ: 0.84-0.92 (m, 12H), 1.22-1.45 (m, 46H), 1.45-1.70 (m, 16H), 1.79 (dt, 4H), 1.92-2.07 (m, 8H), 2.22-2.36 (t, 8H), 2.62-2.68 (m, 8H), 2.80-2.93 (m, 8H), 3.58 (s, 4H), 4.14 (t, 4H), 4.87-4.90 (m, 2H), 5.32-5.36 (m, 2H), 5.44-5.48 (m, 2H), 6.91 (dd, 2H), 7.12-7.15 (m, 4H)

[Example 186] Synthesis of compound 186

<Synthesis of intermediate 69-6>

Using 3-bromo-4-hydroxyphenylacetic acid and in the same manner as in the synthesis of intermediate 69-1 described in Example 181, intermediate 69-6 was synthesized.

[Chem. 419]

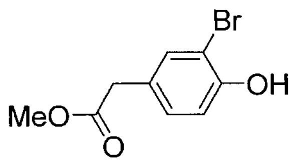

intermediate 69-6

<Synthesis of intermediate 70-6>

Using intermediate 69-6 and in the same manner as in the synthesis of compound 70-1 described in Example 181, intermediate 70-6 was synthesized.

[Chem. 420]

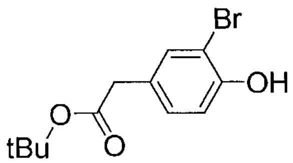

intermediate 70-6

<Synthesis of intermediate 71-6>

Using intermediate 70-6 and intermediate 4 and in the same manner as in the synthesis of intermediate 71-1 described in Example 181, intermediate 71-6 was synthesized.

[Chem. 421]

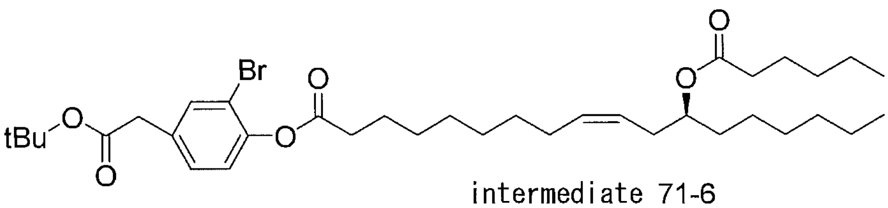

intermediate 71-6

<Synthesis of intermediate 72-6>

Using intermediate 71-6 and in the same manner as in the synthesis of intermediate 72-1 described in Example 181, intermediate 72-6 was synthesized.

[Chem. 422]

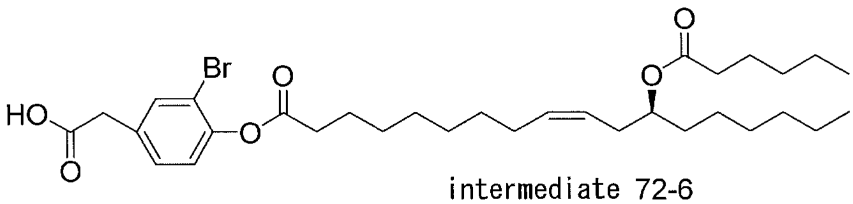

intermediate 72-6

<Synthesis of compound 186>

Using intermediate 72-6 and intermediate 57 and in the same manner as in the synthesis of compound 181 described in Example 181, compound 186 was synthesized.

[Chem. 423]

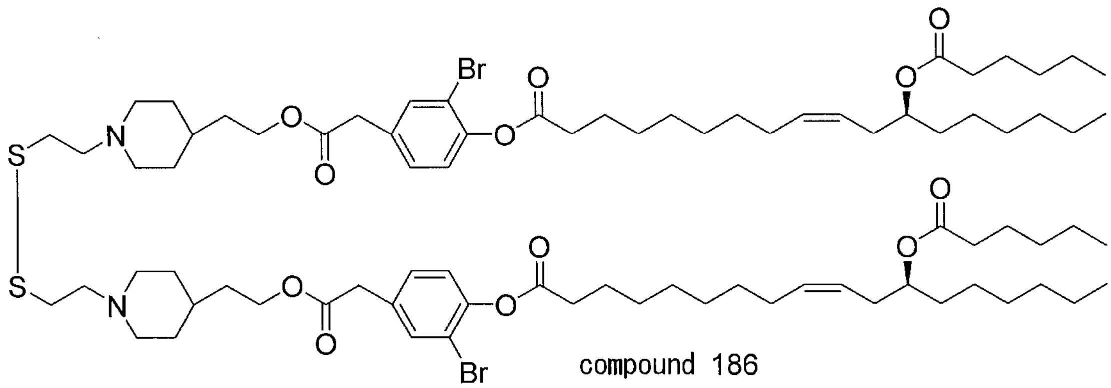

compound 186

<$^1$H-NMR (400 MHz, $CDCl_3$) of compound 186>

δ: 0.84-0.92 (m, 12H), 1.22-1.45 (m, 46H), 1.45-1.70 (m, 16H), 1.79 (dt, 4H), 1.92-2.07 (m, 8H), 2.22-2.36 (t, 8H), 2.62-2.68 (m, 8H), 2.80-2.93 (m, 8H), 3.58 (s, 4H), 4.14 (t, 4H), 4.87-4.90 (m, 2H), 5.32-5.36 (m, 2H), 5.44-5.48 (m, 2H), 7.04 (d, 2H), 7.08 (dd, 2H), 7.60 (d, 2H)

[Example 187] Synthesis of compound 187

<Synthesis of intermediate 69-7>

Using 2-bromo-4-hydroxyphenylacetic acid and in the same manner as in the synthesis of intermediate 69-1 described in Example 181, intermediate 69-7 was synthesized.

[Chem. 424]

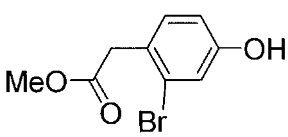

intermediate 69-7

<Synthesis of intermediate 70-7>

Using intermediate 69-7 and in the same manner as in the synthesis of compound 69-1 described in Example 181, intermediate 70-7 was synthesized.

[Chem. 425]

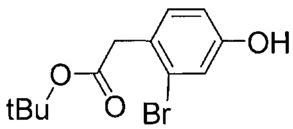

intermediate 70-7

<Synthesis of intermediate 71-7>

Using intermediate 70-7 and intermediate 4 and in the same manner as in the synthesis of intermediate 71-1 described in Example 181, intermediate 71-7 was synthesized.

[Chem. 426]

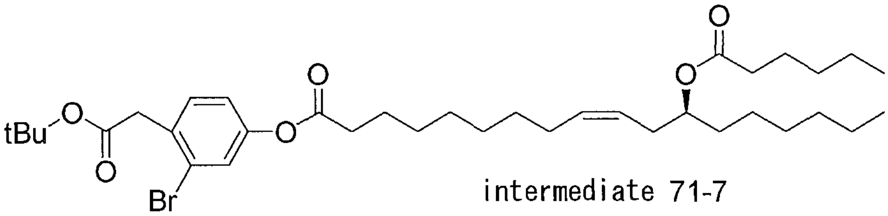

intermediate 71-7

<Synthesis of intermediate 72-7>

Using intermediate 71-7 and in the same manner as in the synthesis of intermediate 72-1 described in Example 181, intermediate 72-7 was synthesized.

[Chem. 427]

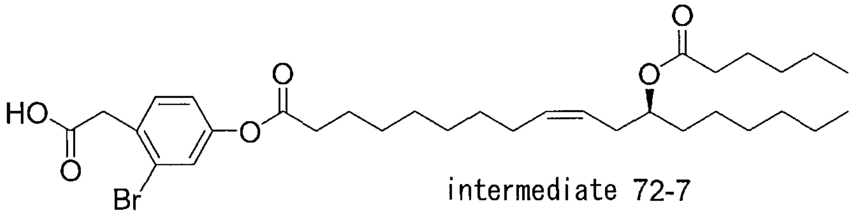

intermediate 72-7

<Synthesis of compound 187>

Using intermediate 72-7 and intermediate 57 and in the same manner as in the synthesis of compound 181 described in Example 181, compound 187 was synthesized.

[Chem. 428]

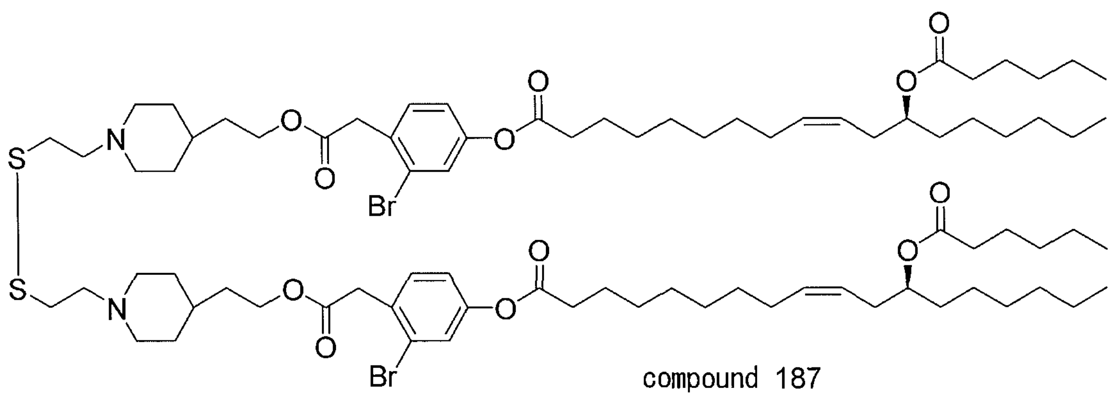

compound 187

<$^1$H-NMR (400 MHz, $CDCl_3$) of compound 187>

δ: 0.84-0.92 (m, 12H), 1.22-1.45 (m, 46H), 1.45-1.70 (m, 16H), 1.79 (dt, 4H), 1.92-2.07 (m, 8H), 2.22-2.36 (t, 8H), 2.62-2.68 (m, 8H), 2.80-2.93 (m, 8H), 3.58 (s, 4H), 4.14 (t, 4H), 4.87-4.90 (m, 2H), 5.32-5.36 (m, 2H), 5.44-5.48 (m, 2H), 7.12 (dd, 2H), 7.33 (d, 2H), 7.49 (d, 2H)

<Synthesis of compound 188 to compound 190>

Compound 188 to compound 190 were synthesized by the following synthesis pathways.

[Chem. 429]

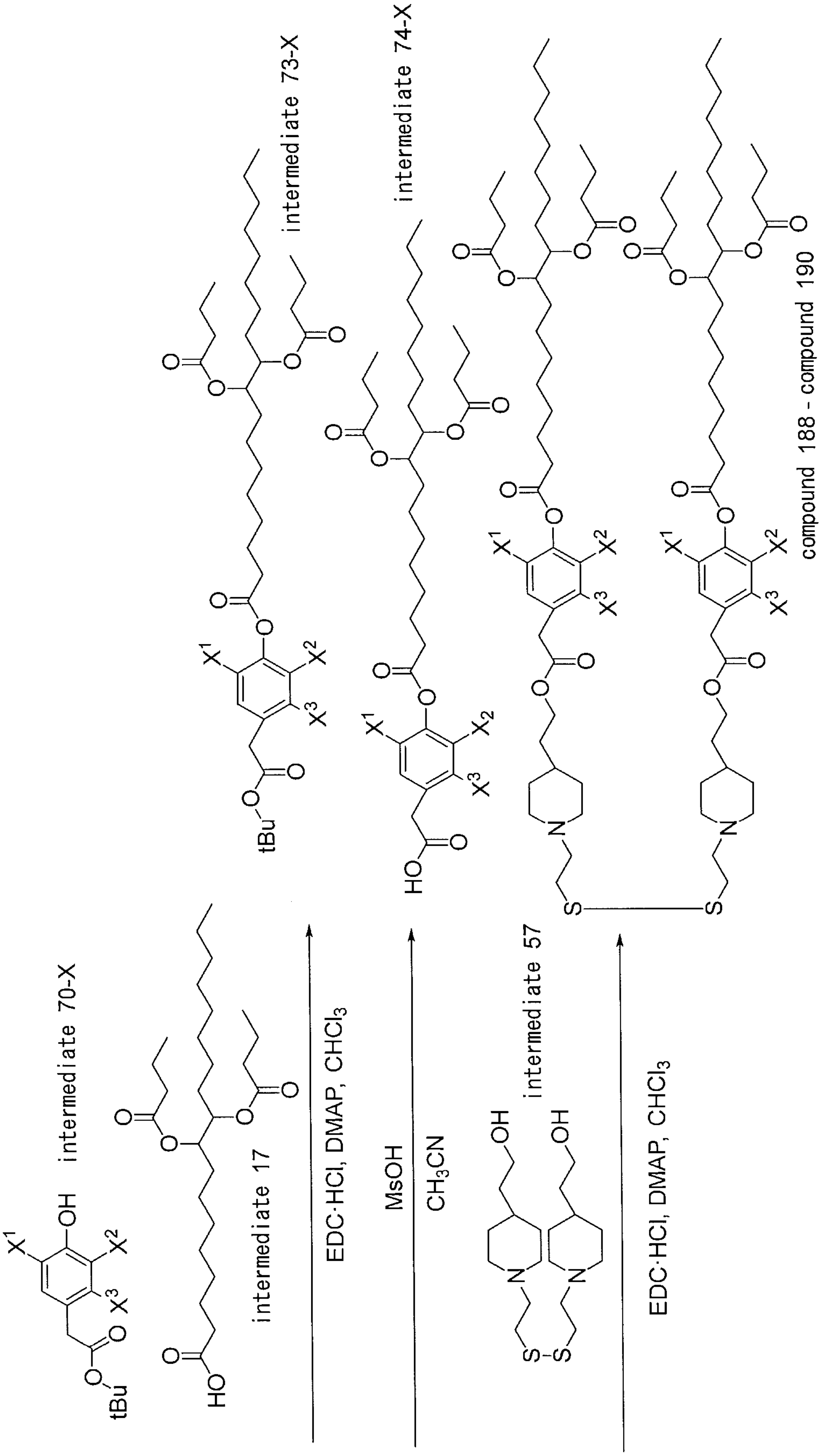
intermediate 70-X
intermediate 17
EDC·HCl, DMAP, $CHCl_3$
intermediate 73-X
MsOH
$CH_3CN$
intermediate 74-X
intermediate 57
EDC·HCl, DMAP, $CHCl_3$
compound 188 - compound 190

In the above-mentioned synthesis pathway, "intermediate 70-X" shows any of intermediate 70-1 to intermediate 70-3, "intermediate 73-X" shows any of intermediate 73-1 to intermediate 73-3, and "intermediate 74-X" shows any of intermediate 74-1 to intermediate 74-3.

In intermediate 70-1, intermediate 73-1, intermediate 74-1, and compound 188, $X^1$ is OMe, $X^2$ is H, and $X^3$ is H, in the above-mentioned synthesis pathway.

In intermediate 70-2, intermediate 73-2, intermediate 74-2, and compound 189, $X^1$ is Cl, $X^2$ is H, and $X^3$ is H, in the above-mentioned synthesis pathway.

In intermediate 70-3, intermediate 73-3, intermediate 74-3, and compound 190, $X^1$ is OMe, $X^2$ is OMe, and $X^3$ is H, in the above-mentioned synthesis pathway.

[Example 188] Synthesis of compound 188

<Synthesis of intermediate 73-1>

Using intermediate 70-1 and intermediate 17 and in the same manner as in the synthesis of intermediate 71-1 described in Example 181, intermediate 73-1 was synthesized.

[Chem. 430]

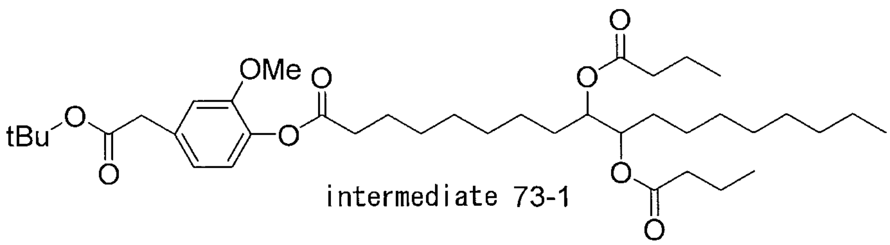

intermediate 73-1

<Synthesis of intermediate 74-1>

Using intermediate 73-1 and in the same manner as in the synthesis of intermediate 72-1 described in Example 181, intermediate 74-1 was synthesized.

[Chem. 431]

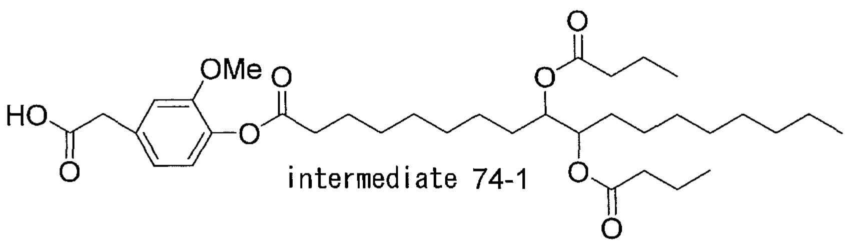

intermediate 74-1

<Synthesis of compound 188>

Using intermediate 74-1 and intermediate 57 and in the same manner as in the synthesis of compound 181 described in Example 181, compound 188 was synthesized.

[Chem. 432]

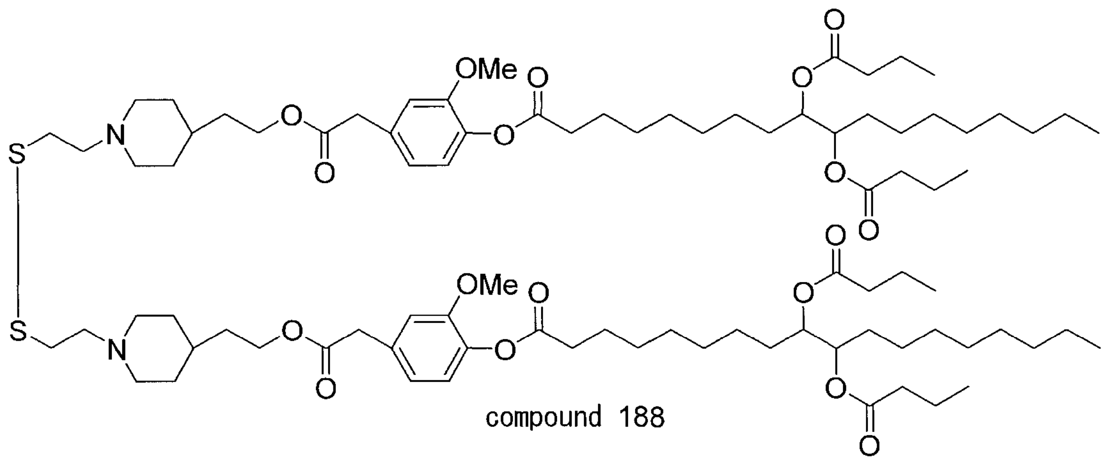

compound 188

<$^1$H-NMR (400 MHz, $CDCl_3$) of compound 188>

δ: 0.88 (t, 6H), 0.95 (t, 12H), 1.22-1.45 (m, 46H), 1.45-1.76 (m, 28H), 1.92-2.00 (m, 4H), 2.27 (t, 8H), 2.56 (t, 4H), 2.62-2.68 (m, 4H), 2.80-2.93 (m, 8H), 3.58 (s, 4H), 3.81 (s, 6H), 4.13 (t, 4H), 5.00 (m, 4H), 6.85 (d, 2H), 6.90 (d, 2H), 6.98 (d, 2H)

[Example 189] Synthesis of compound 189

<Synthesis of intermediate 73-2>

Using intermediate 70-2 and intermediate 17 and in the same manner as in the synthesis of intermediate 71-1 described in Example 181, intermediate 73-2 was synthesized.

[Chem. 433]

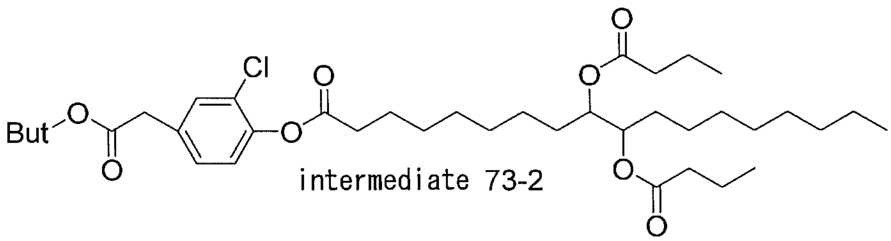

intermediate 73-2

<Synthesis of intermediate 74-2>

Using intermediate 73-2 and in the same manner as in the synthesis of intermediate 72-1 described in Example 181, intermediate 74-2 was synthesized.

[Chem. 434]

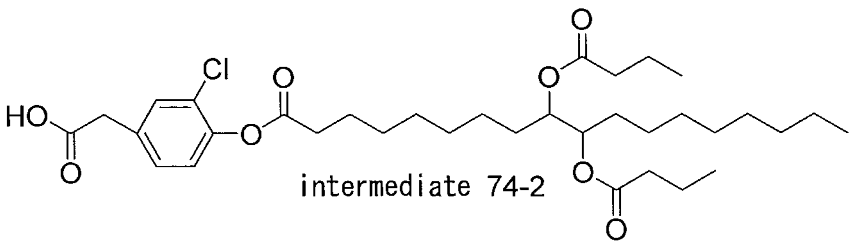

intermediate 74-2

<Synthesis of compound 189>

Using intermediate 74-2 and intermediate 57 and in the same manner as in the synthesis of compound 181 described in Example 181, compound 189 was synthesized.

[Chem. 435]

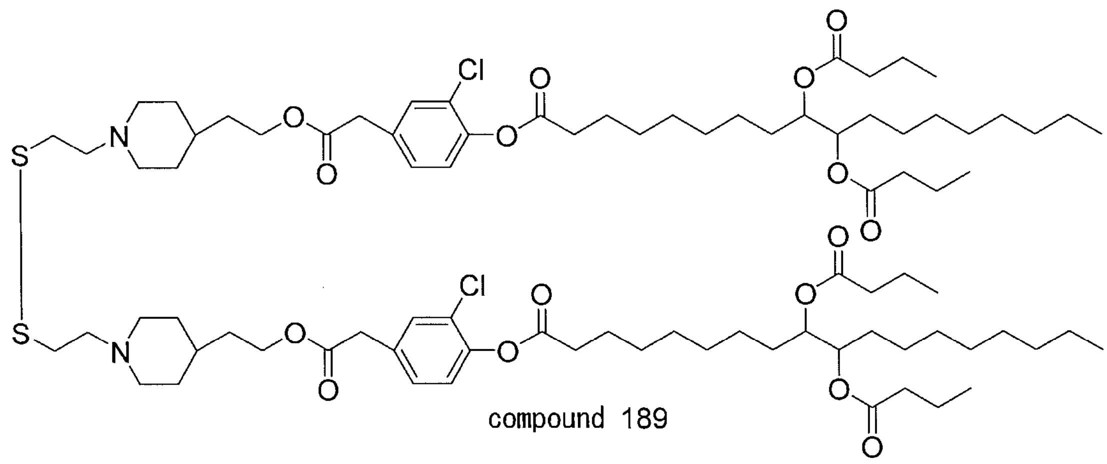

compound 189

<$^1$H-NMR (600 MHz, $CDCl_3$) of compound 189>

δ: 0.88 (t, 6H), 0.95 (t, 12H), 1.22-1.45 (m, 46H), 1.45-1.76 (m, 28H), 1.92-2.00 (m, 4H), 2.27 (t, 8H), 2.56 (t, 4H), 2.62-2.68 (m, 4H), 2.80-2.93 (m, 8H), 3.57 (s, 4H), 4.13 (t, 4H), 5.00 (m, 4H), 7.07 (d, 2H), 7.18 (dd, 2H), 7.38 (d, 2H)

[Example 190] Synthesis of compound 190

<Synthesis of intermediate 73-3>

Using intermediate 70-3 and intermediate 17 and in the same manner as in the synthesis of intermediate 71-1 described in Example 181, intermediate 73-3 was synthesized.

[Chem. 436]

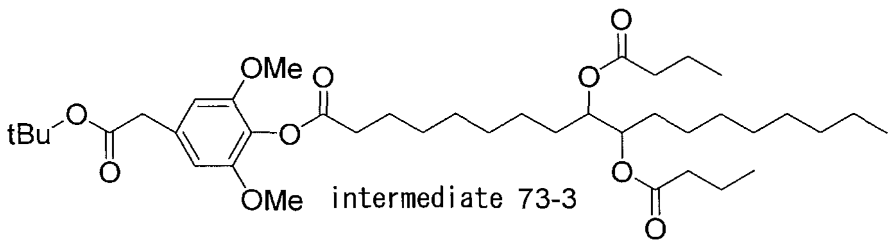

intermediate 73-3

<Synthesis of intermediate 74-3>

Using intermediate 73-3 and in the same manner as in the synthesis of intermediate 72-1 described in Example 181, intermediate 74-3 was synthesized.

[Chem. 437]

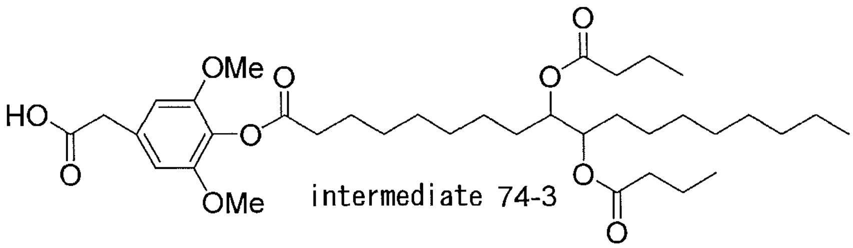

intermediate 74-3

<Synthesis of compound 190>

Using intermediate 74-4 and intermediate 57 and in the same manner as in the synthesis of compound 181 described in Example 181, compound 190 was synthesized.

[Chem. 438]

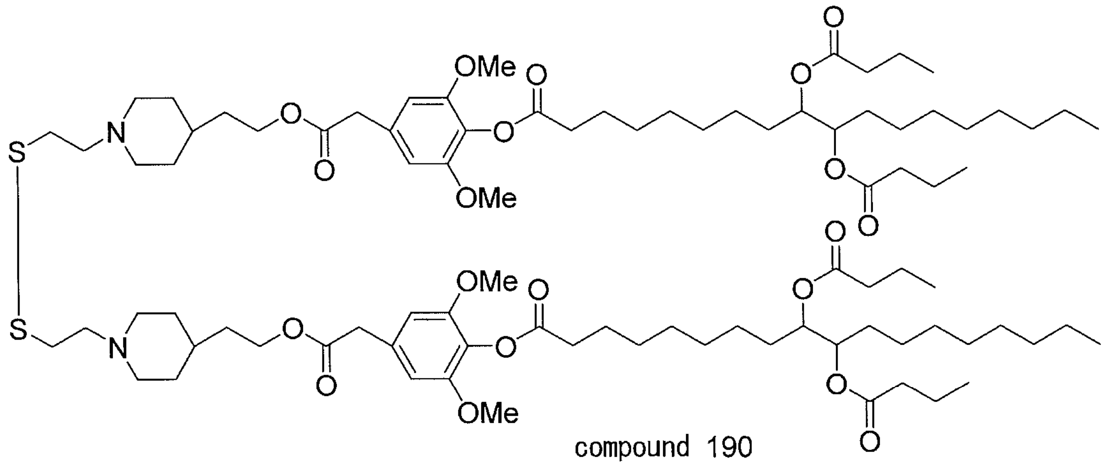

compound 190

<$^1$H-NMR (400 MHz, $CDCl_3$) of compound 190>

5: 0.88 (t, 6H), 0.95 (t, 12H), 1.22-1.45 (m, 46H), 1.45-1.76 (m, 28H), 1.92-2.00 (m, 4H), 2.27 (t, 8H), 2.56 (t, 4H), 2.62-2.68 (m, 4H), 2.80-2.93 (m, 8H), 3.55 (s, 4H), 3.80 (s, 12H), 4.13 (t, 4H), 5.00 (m, 4H), 6.53 (s, 4H)

<Synthesis of compound 191 to compound 193>

Compound 191 to compound 193 were synthesized by the following synthesis pathways.

[Chem. 439]

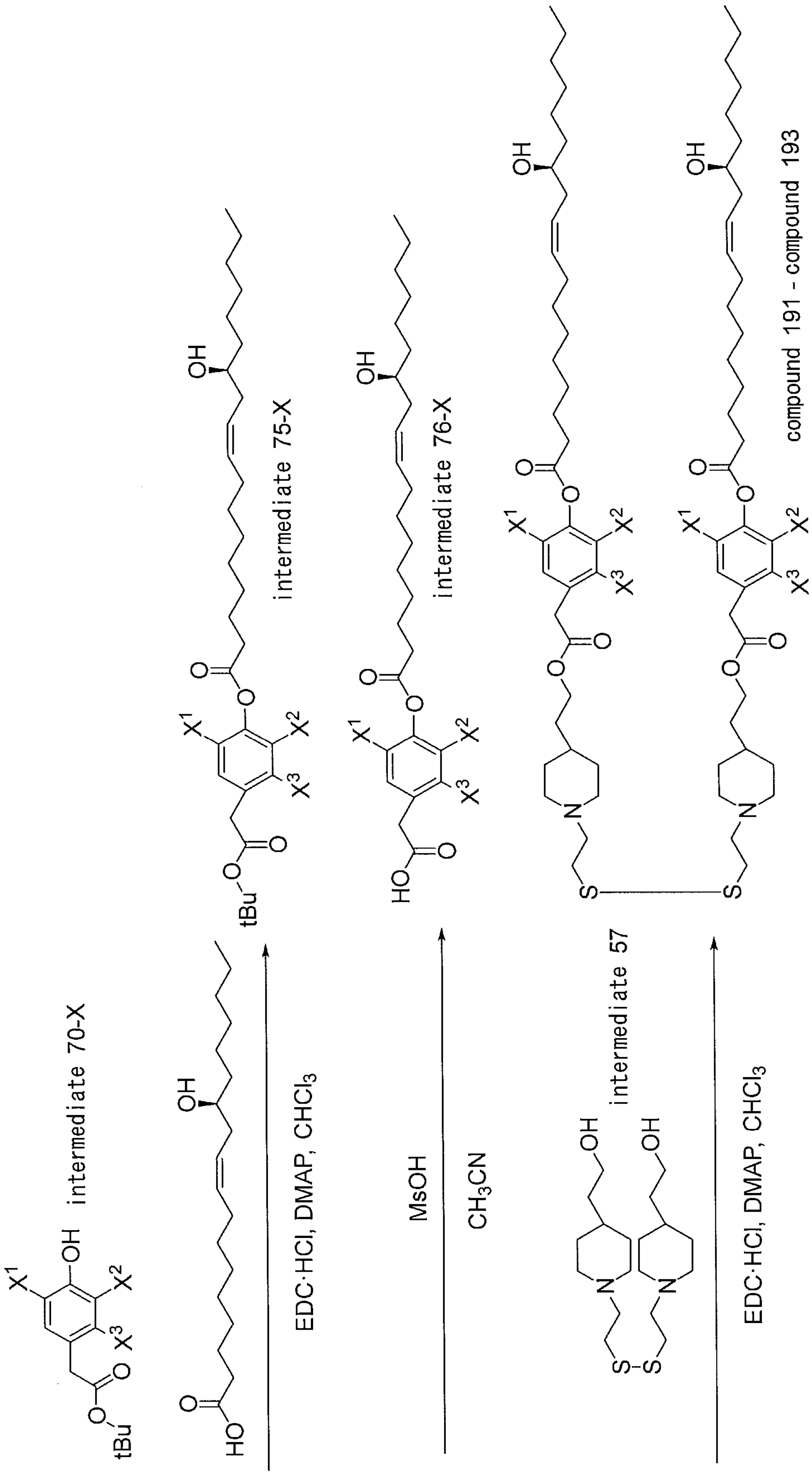


intermediate 70-X

intermediate 75-X

intermediate 76-X

intermediate 57

compound 191 - compound 193

In the above-mentioned synthesis pathway, "intermediate 70-X" shows any of intermediate 70-1 to intermediate

70-3, "intermediate 75-X" shows any of intermediate 75-1 to intermediate 75-3, and "intermediate 76-X" shows any of intermediate 76-1 to intermediate 76-3.

In intermediate 70-1, intermediate 75-1, intermediate 76-1, and compound 191, $X^1$ is OMe, $X^2$ is H, and $X^3$ is H, in the above-mentioned synthesis pathway.

In intermediate 70-2, intermediate 75-2, intermediate 76-2, and compound 192, $X^1$ is Cl, $X^2$ is H, and $X^3$ is H, in the above-mentioned synthesis pathway.

In intermediate 70-3, intermediate 75-3, intermediate 76-3, and compound 193, $X^1$ is OMe, $X^2$ is OMe, and $X^3$ is H, in the above-mentioned synthesis pathway.

[Example 191] Synthesis of compound 191

<Synthesis of intermediate 75-1>

Intermediate 70-1 (700 mg, 2.94 mmol) was dissolved in chloroform (7.00 g), ricinoleic acid (921 mg, 3.08 mmol), DMAP (71.8 mg, 0.588 mmol) and EDC hydrochloride (845 mg, 4.41 mmol) were added and the mixture was reacted at room temperature for 2 hr. The solution after the reaction was washed with water, 5 wt% sodium dihydrogen phosphate aqueous solution, and 20 wt% saline, and dehydrated by adding sodium sulfate. Sodium sulfate was removed by filtration, and the mixture was concentrated by an evaporator and purified by silica gel column using heptane/ethyl acetate to give 1090 mg of intermediate 75-1.

[Chem. 440]

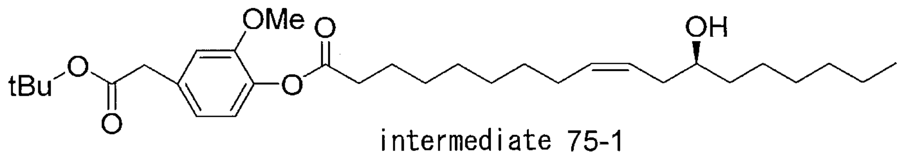

intermediate 75-1

<Synthesis of intermediate 76-1>

Intermediate 75-1 (1067 mg, 2.06 mmol) was dissolved in acetonitrile (5.33 g), mesylic acid (316 mg, 3.29 mmol) was added and the mixture was reacted at room temperature for 2 hr. Thereafter, to the reaction solution was added chloroform (5.33 g), and the mixture was washed with reverse osmosis water. To the obtained solution was added DMAP and the mixture was stirred for 30 min, washed with 0.1 M hydrochloric acid aqueous solution, and 20 wt% saline, and dehydrated by adding sodium sulfate. Sodium sulfate was removed by filtration, and the mixture was concentrated by an evaporator and purified by silica gel column using chloroform/ethanol to give 580 mg of intermediate 76-1.

[Chem. 441]

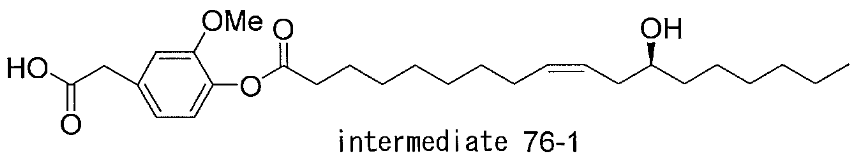

intermediate 76-1

<Synthesis of compound 191>

Intermediate 76-1 (571 mg, 1.23 mmol) was dissolved in chloroform (5.71 g), intermediate 57 (232 mg, 0.617 mmol), DMAP (30.1 mg, 0.247 mmol), and EDC hydrochloride (355 mg, 1.85 mmol) were added and the mixture was reacted at room temperature for 2 hr. The solution after the reaction was washed with 5 wt% sodium dihydrogen phosphate aqueous solution, 5 wt% sodium hydrogen carbonate aqueous solution, and 20 wt% saline, and dehydrated by adding sodium sulfate. Sodium sulfate was removed by filtration, and the mixture was concentrated by an evaporator and purified by silica gel column using chloroform/ethanol to give 234 mg of compound 191.

[Chem. 442]

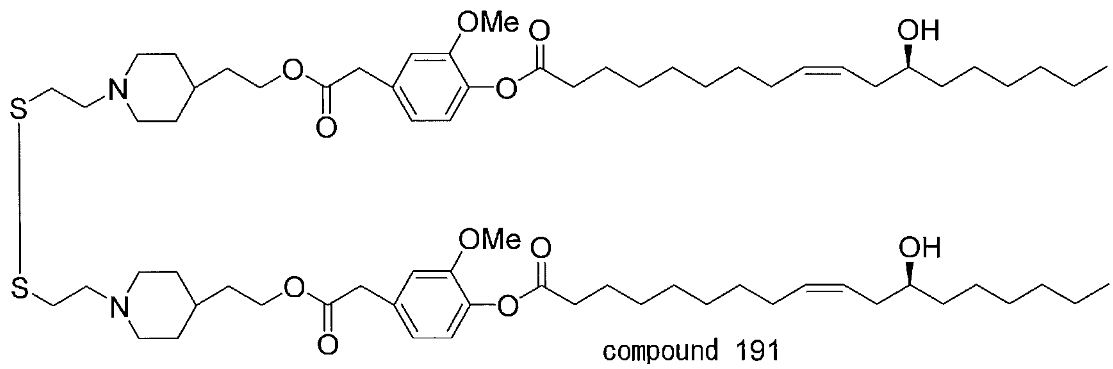

compound 191

<$^1$H-NMR (400 MHz, $CDCl_3$) of compound 191>

δ: 0.88 (m, 8H), 1.22-1.60 (m, 46H), 1.62-1.65 (m, 4H), 1.71-1.75 (m, 4H), 1.92-2.00 (m, 4H), 2.04-2.07 (m, 4H), 2.21 (dd, 4H)2.54 (t, 4H), 2.63-2.66 (m, 4H), 2.80-2.83 (m, 4H), 2.86-2.88 (m, 4H), 3.59-3.62 (m, 6H), 3.81 (s, 6H), 4.12 (t, 4H), 5.41 (m, 2H), 5.56 (m, 2H), 6.85 (d, 2H), 6.90 (d, 2H), 6.98 (d, 2H)

[Example 192] Synthesis of compound 192

<Synthesis of intermediate 75-2>

Using intermediate 70-2 and ricinoleic acid and in the same manner as in the synthesis of intermediate 75-1 described in Example 191, intermediate 75-2 was synthesized.

[Chem. 443]

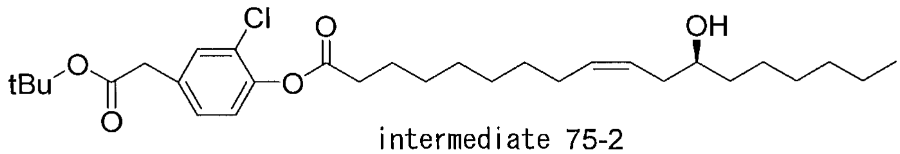

intermediate 75-2

<Synthesis of intermediate 76-2>

Using intermediate 75-2 and in the same manner as in the synthesis of intermediate 76-1 described in Example 191, intermediate 76-2 was synthesized.

[Chem. 444]

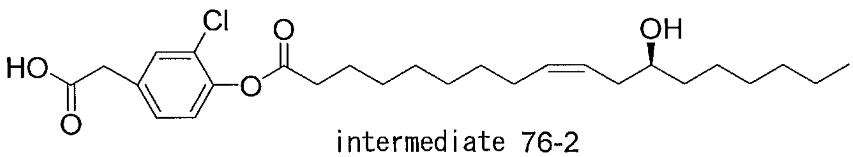

intermediate 76-2

<Synthesis of compound 192>

Using intermediate 76-2 and intermediate 57 and in the same manner as in the synthesis of compound 191 described in Example 191, compound 192 was synthesized.

[Chem. 445]

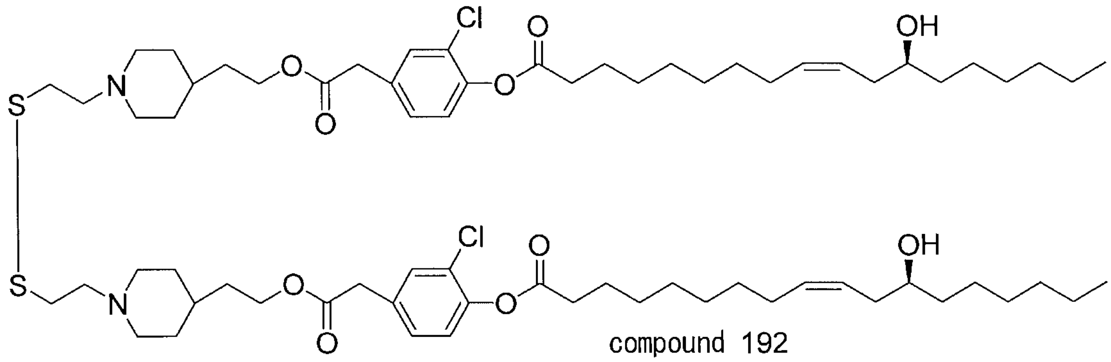

compound 192

<$^1$H-NMR (400 MHz, $CDCl_3$) of compound 192>

δ: 0.88 (m, 8H), 1.22-1.60 (m, 46H), 1.62-1.65 (m, 4H), 1.71-1.75 (m, 4H), 1.92-2.00 (m, 4H), 2.04-2.07 (m, 4H), 2.21 (dd, 4H)2.54 (t, 4H), 2.63-2.66 (m, 4H), 2.80-2.83 (m, 4H), 2.86-2.88 (m, 4H), 3.57-3.62 (m, 6H), 4.12 (t, 4H), 5.41 (m, 2H), 5.56 (m, 2H), 7.07 (d, 2H), 7.18 (dd, 2H), 7.38 (d, 2H)

[Example 193] Synthesis of compound 193

<Synthesis of intermediate 75-3>

Using intermediate 70-3 and ricinoleic acid and in the same manner as in the synthesis of intermediate 75-1 described in Example 191, intermediate 75-3 was synthesized.

[Chem. 446]

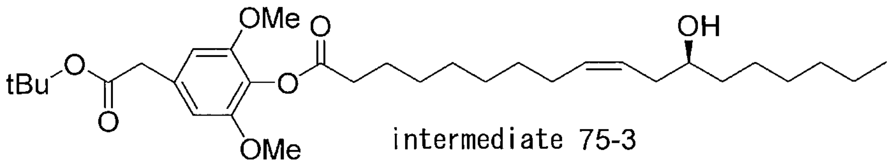

intermediate 75-3

<Synthesis of intermediate 76-3>

Using intermediate 75-3 and in the same manner as in the synthesis of intermediate 76-1 described in Example 191, intermediate 76-3 was synthesized.

[Chem. 447]

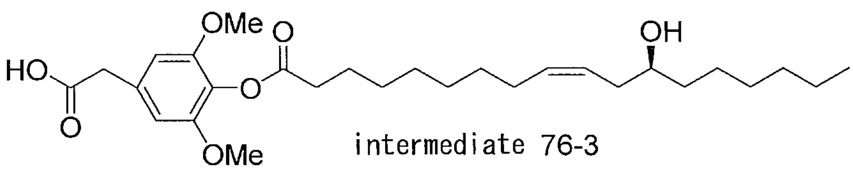

intermediate 76-3

<Synthesis of compound 193>

Using intermediate 76-3 and intermediate 57 and in the same manner as in the synthesis of compound 191 described in Example 191, compound 193 was synthesized.

[Chem. 448]

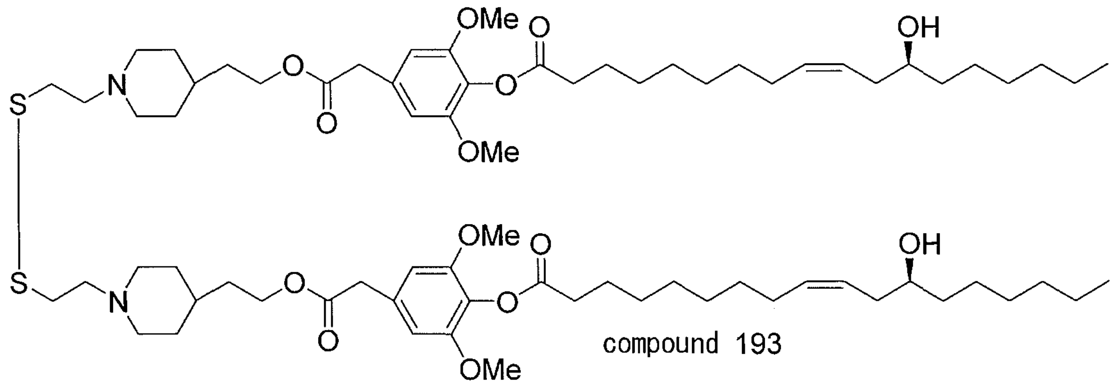

compound 193

<$^1$H-NMR (400 MHz, $CDCl_3$) of compound 193>

δ: 0.88 (m, 8H), 1.22-1.60 (m, 46H), 1.62-1.65 (m, 4H), 1.71-1.75 (m, 4H), 1.92-2.00 (m, 4H), 2.04-2.07 (m, 4H), 2.21 (dd, 4H)2.54 (t, 4H), 2.63-2.66 (m, 4H), 2.80-2.83 (m, 4H), 2.86-2.88 (m, 4H), 3.55 (s, 4H), 3.59-3.62 (m, 2H), 3.80 (s, 12H), 4.13 (t, 4H), 5.41 (m, 2H), 5.56 (m, 2H), 6.53 (s, 4H)

<Synthesis of compound 194>

Using ricinoleic acid and intermediate 1-1 and in the same manner as in the synthesis of compound 1 described in Example 1-1, compound 194 was synthesized.

[Chem. 449]

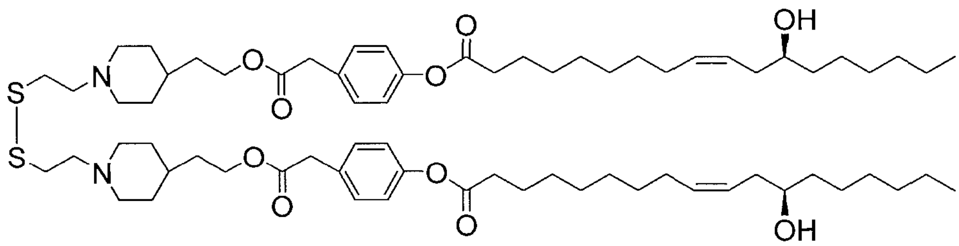

compound 194

<$^1$H-NMR (600 MHz, DMSO-$d_6$) of compound 194>

δ: 0.86-0.90 (m, 6H), 1.22-1.42 (m, 46H), 1.54-1.76 (m, 12H), 1.91-1.98 (m, 4H), 2.00-2.06 (m, 4H), 2.23-2.33 (m, 4H), 2.52-2.56 (m, 8H), 2.61-2.67 (m, 4H), 3.54-3.56 (m, 2H), 3.59 (s, 4H), 4.11-4.14 (t, 4H), 4.45-4.49 (m, 2H), 5.31-5.40 (m, 2H), 5.44-5.49 (m, 2H), 7.02-7.04 (m, 4H), 7.26-7.30 (m, 4H)

<Synthesis of compound 195>

Using ricinoleic acid and intermediate 1-2 and in the same manner as in the synthesis of compound 1 described in Example 1-1, compound 195 was synthesized.

[Chem. 450]

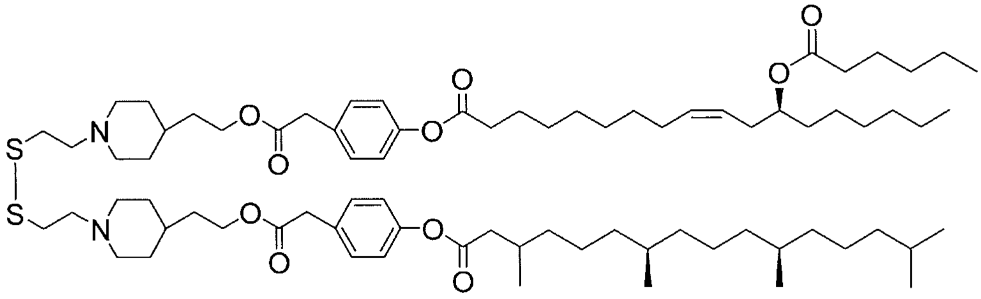

compound 195

<$^1$H-NMR (600 MHz, DMSO-$d_6$) of compound 195>

δ: 0.86-0.90 (m, 21H), 1.22-1.42 (m, 58H), 1.54-1.76 (m, 12H), 1.91-1.98 (m, 4H), 2.00-2.06 (m, 2H), 2.23-2.33 (m, 4H), 2.52-2.56 (m, 6H), 2.61-2.67 (m, 4H), 3.54-3.56 (m, 2H), 3.59 (s, 4H), 4.11-4.14 (t, 4H), 4.45-4.49 (m, 1H), 5.31-5.40 (m, 1H), 5.44-5.49 (m, 1H), 7.02-7.04 (m, 4H), 7.26-7.30 (m, 4H)

[Experimental Example 1] Measurement of Liposomal pKa

Lipid nano particles (LNP) without containing nucleic acid were used for the evaluation of Liposomal pKa.

1. Preparation of LNP by micro flow path

(1) Preparation of ethanol solution of lipid

A 10 mM ethanol solution of cationic lipid, a 5 mM ethanol solution of DOPC, and a 10 mM ethanol solution of Chol were mixed at desired ratio (cationic lipid:DOPC:Chol = 52.5:7.5:40 (molar ratio)) in an Eppendorf tube to achieve the total lipid amount of 720 nmol. To the obtained mixture was further added DMG-PEG2k (2 mM ethanol solution) in an amount of about 1.5 mol with respect to the total amount 100 mol of cationic lipid, DOPC, and Chol, and then ethanol was added to prepare an ethanol solution of the lipid (total amount: 360 μL).

(2) Preparation of LNP using micro flow path

An acidic malic acid buffer (20 mM, pH 3.0) (1080 μL) containing NaCl at a final concentration of 30 mM and an ethanol solution (360 μL) of lipid were each weighed in a syringe. Using an ultra high-speed nanomedicament producing apparatus NanoAssmblr (manufactured by Precision NanoSystems), LNP was prepared under the conditions of addition rate of acidic buffer solution: 3 mL/min, addition rate of ethanol solution of lipid: 1 mL/min, and syringe holder temperature: 25°C, and collected in a 15 mL tube. MES buffer (pH 6.5) (3000 μL) was added to the 15 mL tube, the obtained mixture was transferred to Amicon Ultra 4, and ultrafiltration was performed under centrifugation conditions (25°C, 1000 g, 6 min) to concentrate the mixture to about 100 μL. The obtained concentrate was diluted with PBS to 4 mL, and the mixture was concentrated again under centrifugation conditions (25°C, 1000 g, 6 min), and this operation was performed twice. The obtained concentrate was diluted with PBS to a lipid concentration of 0.5 mM to give a dispersion containing LNP.

2. Measurement of Liposomal pKa

20 mM Citrate buffer, sodium phosphate buffer, and tris HCl buffer, each containing NaCl at a final concentration of 150 mM and adjusted to various pHs within the range of pH 3.0 to 10.0, were prepared. TNS (manufactured by Sigma) solution was diluted with ultrapure water to 0.6 mM. TNS solution (2 μL), dispersions (12 μL) containing LNP prepared in [Experimental Example 1], 1., and buffers adjusted to various pHs (186 μL) were added to a black 96 well plate. The plate was protected from light and shaken at 400 rpm for 10 min. The fluorescence intensity (excitation: 321 nm/emission: 447 nm) was measured using a plate reader (manufactured by TECAN). The relative fluorescence intensity was calculated as a percentage, with the maximum value of the fluorescence intensity in each LNP being 100% and the minimum value being 0%. Furthermore, the pH at which the relative fluorescence intensity was 50% was taken as Liposomal pKa. The Liposomal pKas of the cationic lipids and LNPs used are shown in Table 4. In Comparative Example, the cationic lipid

described in Table 2 was used.

[Table 4]

| cationic lipid used | Liposomal pKa |
|---|---|
| compound 1 | 6.41 |
| compound 2 | 6.33 |
| compound 3 | 6.34 |
| compound 4 | 6.25 |
| compound 5 | 6.33 |
| compound 6 | 6.32 |
| compound 7 | 6.38 |
| compound 8 | 6.23 |
| compound 17 | 6.51 |
| compound 31 | 6.33 |
| compound 32 | 6.30 |
| compound 38 | 6.37 |
| Comparative Example | 6.50 |

3. Results

As shown in Table 4, all LNPs showed Liposomal pKa within the pKa range (5.0 to 9.5) preferable for endosomal escape.

[Experimental Example 2] Preparation of mRNA-encapsulating particles and property evaluation

1. Preparation of LNP by micro flow path method

(1) Preparation of ethanol solution of lipid

A 10 mM ethanol solution of cationic lipid, a 5 mM ethanol solution of DOPC, and a 10 mM ethanol solution of Chol were mixed at desired ratio (cationic lipid:DOPC:Chol = 50:10:40 (molar ratio)) in an Eppendorf tube to achieve the total lipid amount of 720 nmol. To the obtained mixture was further added DMG-PEG2k (2 mM ethanol solution) in an amount of about 1 mol with respect to the total amount 100 mol of cationic lipid, DOPC, and Chol, and then ethanol was added to prepare an ethanol solution of the lipid (total amount: 360 μL).

(2) Preparation of acidic buffer solution of nucleic acid

An acidic buffer solution (total amount: 1080 μL) of nucleic acid was prepared by weighing 7.2 μg of mRNA solution (0.6 mg/mL) in a 5 mL tube and adding acidic malate buffer (20 mM, pH 3.0).

(3) Preparation of LNP using micro flow path

An acidic buffer solution of nucleic acid and an ethanol solution of lipid were each weighed in a syringe. Using an ultra high-speed nanomedicament producing apparatus NanoAssmblr (manufactured by Precision NanoSystems), LNP was prepared under the conditions of addition rate of acidic buffer solution of nucleic acid: 3 mL/min, addition rate of ethanol solution of lipid: 1 mL/min, and syringe holder temperature: 25°C, and collected in a 15 mL tube. MES buffer (pH 6.5) (3000 μL) was added to the 15 mL tube, the obtained mixture was transferred to Amicon Ultra 4, and ultrafiltration was performed under centrifugation conditions (25°C, 1000 g, 6 min) to concentrate the mixture to about 500 μL. The obtained concentrate was diluted with PBS to 4 mL, and the mixture was concentrated again under centrifugation conditions (25°C, 1000 g, 6 min), and this operation was performed twice. The obtained concentrate was diluted with PBS to a lipid concentration of 2 mM to give a dispersion containing LNP.

2. Measurement of particle size, PdI and zeta potential of mRNA-encapsulating LNPs

The particle size, PdI (Polydispersity Index), and zeta potential of the mRNA-encapsulating LNP prepared by the method of the above-mentioned 1 were measured by a dynamic light scattering method (Zetasizer Nano; Malvern). The cationic lipids used and the results are shown in Table 5-1 to Table 5-3. In Comparative Example, the cationic lipid described in Table 2 was used.

[Table 5-1]

| cationic lipid used | particle size (nm) | PdI | zeta potential (mV) |
|---|---|---|---|
| compound 1 | 96.63 | 0.070 | -3.79 |
| compound 2 | 100.40 | 0.101 | -3.71 |
| compound 3 | 95.12 | 0.064 | -7.04 |
| compound 5 | 106.10 | 0.112 | -4.68 |
| compound 6 | 94.72 | 0.038 | -11. 60 |
| compound 7 | 97.79 | 0.082 | -4.15 |
| compound 8 | 106.10 | 0.039 | -3.41 |
| compound 9 | 89.18 | 0.078 | -2.88 |
| compound 10 | 92.75 | 0.114 | -3.07 |
| compound 11 | 86.63 | 0.070 | -3.32 |
| compound 12 | 99.68 | 0.102 | -3.30 |
| compound 13 | 94.42 | 0.073 | -3.63 |
| compound 14 | 92.18 | 0.073 | -2.80 |
| compound 16 | 147.80 | 0.082 | -3.63 |
| compound 17 | 127.90 | 0.061 | -5.19 |
| compound 18 | 117.10 | 0.080 | -4.20 |
| compound 19 | 148.30 | 0.105 | -4.94 |
| compound 20 | 173.80 | 0.224 | -3.85 |
| compound 21 | 122.40 | 0.058 | -5.13 |
| compound 22 | 111. 60 | 0.094 | -5.85 |
| compound 23 | 104.60 | 0.084 | -4.98 |
| compound 24 | 111.00 | 0.069 | -5.08 |
| compound 25 | 156.50 | 0.059 | -5.88 |
| compound 26 | 108.70 | 0.058 | -3.71 |
| compound 27 | 94.69 | 0.063 | -3.12 |

[Table 5-2]

| cationic lipid used | particle size (nm) | PdI | zeta potential (mV) |
|---|---|---|---|
| compound 28 | 96.27 | 0.084 | -2.37 |
| compound 29 | 105.80 | 0.038 | -3.36 |
| compound 30 | 96.18 | 0.064 | -3.29 |
| compound 31 | 95.95 | 0.058 | -3.05 |
| compound 32 | 93.18 | 0.061 | -2.54 |
| compound 33 | 85.78 | 0.091 | -2.72 |

(continued)

| cationic lipid used | particle size (nm) | PdI | zeta potential (mV) |
|---|---|---|---|
| compound 34 | 96.83 | 0.062 | -4.23 |
| compound 35 | 116.00 | 0.055 | -4.16 |
| compound 36 | 132.00 | 0.096 | -4.20 |
| compound 38 | 143.90 | 0.055 | -3.94 |
| compound 40 | 101.50 | 0.071 | -3.94 |
| compound 41 | 123.30 | 0.067 | -2.30 |
| compound 42 | 92.06 | 0.065 | -3.86 |
| compound 43 | 90.46 | 0.072 | -3.98 |
| compound 44 | 89.35 | 0.064 | -3.25 |
| compound 45 | 97.92 | 0.077 | -5.81 |
| compound 48 | 140.80 | 0.049 | -2.00 |
| compound 148 | 92.96 | 0.063 | -4.31 |
| compound 149 | 97.44 | 0.059 | -6.03 |
| compound 150 | 94.66 | 0.071 | -2.14 |
| compound 151 | 86.75 | 0.095 | -4.56 |
| compound 152 | 89.59 | 0.042 | -3.85 |
| compound 153 | 92.99 | 0.059 | -3.66 |
| compound 155 | 92.52 | 0.065 | -5.54 |

[Table 5-3]

| cationic lipid used | particle size (nm) | PdI | zeta potential (mV) |
|---|---|---|---|
| compound 52 | 86.9 | 0.052 | -3.05 |
| compound 58 | 96. 6 | 0.065 | -3.5 |
| compound 94 | 88.0 | 0.048 | -2.40 |
| compound 112 | 96.42 | 0.069 | -6.41 |
| compound 124 | 96.63 | 0.111 | -5.07 |
| compound 127a | 82.9 | 0.041 | -2.99 |
| compound 127 | 92.2 | 0.039 | -2.53 |
| compound 145 | 83.1 | 0.034 | -2.39 |
| compound 159 | 90.4 | 0.112 | -4.66 |
| compound 160 | 88.8 | 0.064 | -2.82 |
| compound 161 | 91.0 | 0.091 | -3.90 |
| compound 162 | 91.2 | 0.041 | -5.04 |
| compound 163 | 88.8 | 0.017 | -4.5 |
| compound 164 | 88.3 | 0.051 | -3.5 |
| compound 166 | 89.3 | 0.047 | -2.5 |
| compound 168 | 84.8 | 0.070 | -3.2 |
| compound 170 | 90.7 | 0.032 | -2.8 |
| compound 173 | 99.5 | 0.070 | -4.4 |

(continued)

| cationic lipid used | particle size (nm) | PdI | zeta potential (mV) |
|---|---|---|---|
| compound 175 | 97.7 | 0.052 | -4.0 |
| compound 194 | 107.4 | 0.061 | -4.02 |
| Comparative Example | 108.70 | 0.099 | -4.47 |

3. Results

As shown in Table 5-1 to Table 5-3, all mRNA-encapsulating LNPs showed a particle size of 30 to 300 nm, which is a preferable form, and the electric charge (zeta potential) thereof at physiological pH was within a preferable range (-15 to +15 mV).

[Experimental Example 3] Evaluation of in vitro gene expression in HeLa cells

1. Preparation of mRNA-encapsulating LNP

LNP encapsulating mRNA that expresses luciferase (cationic lipid:DOPC:Chol:DMG-PEG2k = 50:10:40:1 (molar ratio)) was prepared by the method described in [Experimental Example 2], 1.

2. Time-course evaluation of gene expression in HeLa cells

HeLa cells, which are human cervical cancer cells, were seeded in a 3.5 cm dish at $5.0\times10^4$ cells/2 mL/Dish 24 hours before transfection. After 24 hr, the medium was changed to a culture medium (D-MEM) containing 0.1 mM D-luciferin. The prepared mRNA-encapsulating LNP was diluted with PBS to an mRNA concentration of about 1 μg/mL. The diluted mRNA-encapsulating LNP solution (about 200 μL, mRNA: 0.2 μg) was added to a 3.5 cm dish, and set in an incubator luminometer KronosDio. The luminescence intensity of luciferase was measured for 2 min every one hour. The cumulative luciferase luminescence intensity in 24 hr was calculated from the obtained time change of expression. Cationic lipids used, cumulative luciferase luminescence intensity in 24 hr, and relative luminescence intensity (= cumulative luciferase luminescence intensity in 24 hr when cationic lipid of Example is used/cumulative luciferase luminescence intensity in 24 hr when cationic lipid of Comparative Example is used) are shown in Table 6-1 and Table 6-2. "E+0a" (a: integer) indicated in Table 6-1 and Table 6-2 means "$10^a$". For example, "1.0E+06" means "$1.0\times10^6$". In Comparative Example, the cationic lipid described in Table 2 was used.

[Table 6-1]

| cationic lipid used | cumulative luciferase luminescence intensity (RLU) in 24 hr | relative luminescence intensity |
|---|---|---|
| compound 1 | 2.61E+07 | 1. 42 |
| compound 2 | 2.98E+07 | 1.61 |
| compound 3 | 2.03E+07 | 1.10 |
| compound 5 | 4.16E+07 | 2.25 |
| compound 7 | 2.60E+08 | 14.10 |
| compound 9 | 1.88E+08 | 10.19 |
| compound 10 | 1.96E+08 | 10.64 |
| compound 11 | 1.85E+08 | 10.04 |
| compound 12 | 2.08E+08 | 11.28 |
| compound 13 | 1.24E+08 | 6.72 |
| compound 14 | 1.56E+08 | 8.44 |
| compound 16 | 6.57E+07 | 3.56 |
| compound 17 | 5.18E+07 | 2.81 |
| compound 18 | 6.51E+07 | 3.53 |

(continued)

| cationic lipid used | cumulative luciferase luminescence intensity (RLU) in 24 hr | relative luminescence intensity |
|---|---|---|
| compound 19 | 4.64E+07 | 2.51 |
| compound 21 | 1.01E+08 | 5.50 |
| compound 22 | 8.98E+07 | 4.87 |

Table 6-2]

| cationic lipid used | cumulative luciferase lumines-cence intensity (RLU) in 24 hr | relative luminescence intensity |
|---|---|---|
| compound 23 | 8.99E+07 | 4.87 |
| compound 24 | 7.58E+07 | 4.11 |
| compound 25 | 8.72E+07 | 4.72 |
| compound 26 | 1.08E+08 | 5.87 |
| compound 27 | 1.23E+08 | 6.66 |
| compound 28 | 1.37E+08 | 7.41 |
| compound 29 | 1.50E+08 | 8.13 |
| compound 30 | 1.90E+08 | 10.29 |
| compound 31 | 2.32E+08 | 12.56 |
| compound 32 | 2.10E+08 | 11.38 |
| compound 33 | 1.19E+08 | 6.43 |
| compound 34 | 1.65E+08 | 8.92 |
| compound 35 | 2.40E+08 | 13.01 |
| compound 36 | 1.80E+08 | 9.75 |
| compound 38 | 1.18E+08 | 6.37 |
| compound 40 | 2.16E+07 | 1.17 |
| compound 45 | 7.94E+07 | 4.30 |
| Comparative Example | 1.85E+07 | 1.00 |

3. Results

The higher the cumulative luciferase luminescence intensity in 24 hr, i.e., the higher the total luciferase activity is, the higher the gene expression is. The LNPs containing the cationic lipids of the Examples shown in Tables 6-1 and 6-2 exhibited superior gene expression activity compared to the LNP containing the cationic lipid of the Comparative Example. Therefore, it was clarified that the LNP containing the cationic lipid of the present invention is beneficial as an LNP capable of promoting the expression of mRNA.

[Experimental Example 4] Evaluation of in vivo gene expression

1. Preparation of mRNA-encapsulating LNP

LNP encapsulating mRNA that expresses luciferase (cationic lipid:DOPC:Chol:DMG-PEG2k = 52.5:7.5:40:1.5 (molar ratio)) was prepared by the method described in [Experimental Example 2], 1.

2. Evaluation of in vivo gene expression

The prepared each mRNA-encapsulating LNP dispersion was administered from the tail vein of BALB/c mouse (male, 5-week-old). The dose of mRNA was set to 0.05 mg per 1 kg body weight of the mouse, and the dose of the mRNA-encapsulating LNP dispersion was set to 10 μL per 1 g body weight of the mouse. After 4.5 hr from the administration of the mRNA-encapsulating LNP dispersion, a PBS solution of D-luciferin potassium was administered into the abdominal cavity of the mouse. The dose of D-luciferin potassium was set to 3 mg per mouse, and the dose of the PBS solution of D-luciferin potassium was set to 200 μL per mouse. After 15 min from the administration of the PBS solution of D-luciferin potassium, the mouse was euthanized, and the liver was isolated. The luminescence in the liver was quantified using an in vivo imaging system (IVIS) with an exposure of 10 sec. The amount of luminescence in the liver was quantified by image analysis using Live Imaging software attached to IVIS. The amount of luminescence (photons/sec) was calculated from the acquired image and used as an index of gene expression activity. The cationic lipids used, the luminescence intensity in the liver, and the relative luminescence intensity (= luminescence intensity in the liver when cationic lipid of Example was used/luminescence intensity in the liver when cationic lipid of Comparative Example was used) are shown in Table 7-1 to Table 7-3. "E+0a" (a: integer) indicated in Table 7-1 to Table 7-3 means "$10^a$". For example, "3.94E+09" means "$3.94\times10^9$". In Comparative Example, the cationic lipid described in Table 2 was used.

[Table 7-1]

| cationic lipid used | amount of luminescence (photons/sec) in liver | relative amount of luminescence |
|---|---|---|
| compound 1 | 3.94E+09 | 2.59 |
| compound 2 | 4.02E+09 | 2.64 |
| compound 3 | 2.26E+09 | 1. 48 |
| compound 5 | 3.76E+09 | 2.48 |
| compound 6 | 2.96E+09 | 1.94 |
| compound 7 | 3.89E+09 | 2.56 |
| compound 9 | 6.61E+09 | 4.35 |
| compound 10 | 3.66E+09 | 2.41 |
| compound 11 | 2.42E+09 | 1.59 |
| compound 12 | 3.07E+09 | 2.02 |
| compound 14 | 6.12E+09 | 4.03 |
| compound 17 | 3.73E+09 | 2.45 |
| compound 18 | 3.15E+09 | 2.07 |
| compound 19 | 2.12E+09 | 1. 40 |
| compound 21 | 3.33E+09 | 2.19 |
| compound 22 | 4.50E+09 | 2.96 |
| compound 23 | 1.73E+09 | 1.14 |
| compound 25 | 2.56E+09 | 1. 68 |
| compound 26 | 2.91E+09 | 1. 91 |

[Table 7-2]

| cationic lipid used | amount of luminescence (photons/sec) in liver | relative amount of luminescence |
|---|---|---|
| compound 27 | 5.76E+09 | 3.79 |
| compound 28 | 4.49E+09 | 2.95 |
| compound 29 | 1.82E+09 | 1.20 |
| compound 30 | 4.03E+09 | 2.65 |
| compound 31 | 4.91E+09 | 3.23 |
| compound 32 | 3.79E+09 | 2.49 |
| compound 33 | 6.21E+09 | 4.08 |
| compound 34 | 2.76E+09 | 1.81 |

(continued)

| cationic lipid used | amount of luminescence (photons/sec) in liver | relative amount of luminescence |
|---|---|---|
| compound 35 | 3.62E+09 | 2.38 |
| compound 36 | 1.72E+09 | 1.13 |
| compound 41 | 1.73E+09 | 1.14 |
| compound 42 | 2.03E+09 | 1.34 |
| compound 44 | 2.38E+09 | 1.56 |
| compound 112 | 9.26E+09 | 6.09 |
| compound 124 | 5.12E+09 | 3.37 |
| compound 151 | 2.73E+09 | 1. 79 |
| compound 152 | 1.97E+09 | 1.30 |
| compound 155 | 4.12E+09 | 2.71 |

[Table 7-3]

| cationic lipid used | amount of luminescence (photons/sec) in liver | relative amount of luminescence |
|---|---|---|
| compound 94 | 9.40E+09 | 6.18 |
| compound 127a | 6.50E+09 | 4.27 |
| compound 127 | 1.07E+10 | 7.04 |
| compound 145 | 6.65E+09 | 4.37 |
| compound 160 | 1.09E+09 | 7.19 |
| compound 161 | 3.28E+09 | 2.16 |
| compound 162 | 4.43E+09 | 2.85 |
| compound 163 | 5.85E+09 | 3.85 |
| compound 164 | 5.12E+09 | 3.37 |
| compound 168 | 7.01E+09 | 4.61 |
| compound 170 | 5.30E+09 | 3.49 |
| compound 194 | 8.82E+09 | 5.80 |
| Comparative Example | 1.52E+09 | 1.00 |

3. Results

The higher the amount of luminescence in organ, i.e., the higher the total luciferase activity is, the higher the gene expression is. As shown in Table 7-1 to Table 7-3, in liver, the LNPs containing the cationic lipids of the Examples exhibited superior gene expression activity compared to the LNP containing the cationic lipid of the Comparative Example. Therefore, it was clarified that the LNP containing the cationic lipid of the present invention is beneficial as an LNP capable of promoting the expression of mRNA.

[Experimental Example 5] Evaluation of hemolysis activity (membrane fusion ability) at pH 7.4 and 5.5

1. Preparation of various LNPs

LNPs encapsulating mRNA that expresses luciferase (cationic lipid:DOPC:Chol:DMG-PEG2k=52.5:7.5:40:1.5 (molar ratio)) were prepared using the method described in [Experimental Example 2] 1. As cationic lipid, the cationic lipid of Comparative Example, compound 9, compound 14, compound 27, compound 33, or compound 42 was used.

2. Obtaining mouse red blood cells

6 to 7-week-old male ICR mice were euthanized, and about 1000 μL of blood was collected from the inferior vena cava. The collected blood was immediately mixed with 1.0 μL of heparin solution (5000 U/5 mL). About 9 mL of PBS was added to the blood to make the total volume 10 mL. The mixture was then mixed by inversion and centrifuged (4°C, 400 g, 10 min). The supernatant containing plasma components was removed using a Pasteur pipette. About 9 mL of PBS was added to the blood cell components to make the total volume 10 mL, and the mixture was centrifuged again. A similar washing procedure was repeated 4 times to obtain mouse red blood cells.

3. Evaluation of hemolysis activity

2, 4, 6, 8, and 10 μL of mouse red blood cells were weighed and diluted with PBS containing 1 w/v% Triton-X100. The entire diluted solution was transferred to a clear 96-well plate, and the absorbance at 545 nm was measured using a plate reader. A calibration curve was created using these samples, and the point where the absorbance reached 1 was determined as the amount of blood cells to be used in the hemolysis assay. Empty LNP solution was weighed into an Eppendorf tube and diluted with malic acid-PBS buffer (pH 5.5, pH 7.4), followed by addition of mouse red blood cells. The final lipid concentration was 100 μM at pH 7.4 and 33.3 μM at pH 5.5, and the final volume of the solution was 250 μL. Each tube was shaken at 1900 rpm for 15 min. Each sample was centrifuged under centrifugation conditions (4°C, 400 g, 5 min), and 100 μL of the supernatant was transferred to a clear 96-well plate and the absorbance at 545 nm was measured. Untreated red blood cells were used as the negative control. 1 w/v% Triton-X was used as the positive control. The hemolysis activity of each sample was calculated using the following formula:
Hemolysis activity (%) = 100×(absorbance of each sample - absorbance of negative control)/(absorbance of positive control - absorbance of negative control). The hemolysis activity of LNP at pH 7.4 is shown in Fig. 1, and the hemolysis activity of LNP at pH 5.5 is shown in Fig. 2.

4. Results

As shown in Fig. 1, LNPs containing compound 9, compound 14, compound 27, compound 33, compound 42, or the cationic lipid of Comparative Example did not exhibit hemolysis activity at physiological pH (7.4). On the other hand, as shown in Fig. 2, at the pH of the endosomal environment (5.5), LNPs containing compound 9, compound 14, compound 27, compound 33, or compound 42 exhibited superior hemolysis activity compared to LNP containing the cationic lipid of Comparative Example. Therefore, LNP containing the cationic lipid of the present invention is superior in the membrane fusion ability and therefore is superior in nucleic acid delivery.

[Experimental Example 6] Preparation of mRNA-encapsulating particles and property evaluation

1. Preparation of LNP by micro flow path method

(1) Preparation of ethanol solution of lipid

A 5 mM ethanol solution of cationic lipid, a 5 mM ethanol solution of DOPC, a 10 mM ethanol solution of Chol, and a 0.5 mM ethanol solution of DMG-PEG 2k were mixed at desired ratio (cationic lipid:DOPC:Chol=49:7.5:42.5:1 (molar ratio)) in an Eppendorf tube to achieve the total lipid amount of 720 nmol. Then, ethanol was added to prepare an ethanol solution of the lipid (total amount: 500 μL).

(2) Preparation of acidic buffer solution of nucleic acid

An acidic buffer solution (total amount: 1300 μL) of nucleic acid was prepared by weighing 6.5 μg of mRNA solution (0.6 mg/mL) in a 5 mL tube and adding acidic citrate buffer (20 mM, pH 5.0).

(3) Preparation of LNP using micro flow path

An acidic buffer solution of nucleic acid and an ethanol solution of lipid were each weighed in a syringe. Using an ultra high-speed nanomedicament producing apparatus NanoAssmblr (manufactured by Precision NanoSystems), LNP was prepared under the conditions of addition rate of acidic buffer solution of nucleic acid: 9 mL/min, addition rate of ethanol solution of lipid: 3 mL/min, and syringe holder temperature: 25°C, and collected in a 15 mL tube. TBS buffer (pH 6.5) (3000 μL) was added to the 15 mL tube, the obtained mixture was transferred to Amicon Ultra 4, and ultrafiltration was performed under centrifugation conditions (25°C, 1000 g, 6 min) to concentrate the mixture to about 500 μL. The obtained

concentrate was diluted with TBS to 4 mL, and the mixture was concentrated again under centrifugation conditions (25°C, 1000 g, 6 min), and this operation was performed twice. The obtained concentrate was diluted with TBS to a lipid concentration of 2 mM to give a dispersion containing LNP.

2. Measurement of particle size, PdI and zeta potential of mRNA-encapsulating LNPs

The particle size, PdI (Polydispersity Index), and zeta potential of the mRNA-encapsulating LNP prepared by the method of the above-mentioned 1 were measured by a dynamic light scattering method (Zetasizer Nano; Malvern). The cationic lipids used and the results are shown in Table 8-1 and Table 8-2. In Comparative Example, the cationic lipid described in Table 2 was used.

[Table 8-1]

| cationic lipid used | particle size (nm) | PdI | zeta potential (mV) |
|---|---|---|---|
| compound 9 | 124.8 | 0.054 | -2.59 |
| compound 10 | 128.8 | 0.118 | -3.1 |
| compound 11 | 120.4 | 0.085 | -2.12 |
| compound 12 | 122.7 | 0.066 | -2.84 |
| compound 33 | 123.1 | 0.12 | -5.6 |
| compound 43 | 128.2 | 0.17 | -5.9 |
| compound 48 | 121.6 | 0.12 | -5.8 |
| compound 52 | 130.8 | 0.051 | -3.73 |
| compound 58 | 90.0 | 0.071 | -4.2 |
| compound 94 | 119.7 | 0.121 | -4.45 |
| compound 127a | 118.9 | 0.065 | -2.71 |
| compound 145 | 122.1 | 0.082 | -3.79 |
| compound 148 | 133.6 | 0.15 | -7.4 |
| compound 149 | 123.1 | 0.15 | -6.2 |
| compound 150 | 130.2 | 0.11 | -8.1 |
| compound 151 | 126.4 | 0.10 | -4.0 |
| compound 152 | 122.4 | 0.14 | -5.6 |

[Table 8-2]

| cationic lipid used | particle size (nm) | PdI | zeta potential (mV) |
|---|---|---|---|
| compound 153 | 123.7 | 0.12 | -6.2 |
| compound 155 | 147.9 | 0.14 | -7.3 |
| compound 159 | 126.8 | 0.14 | -5.8 |
| compound 161 | 125 | 0.098 | -4.29 |
| compound 162 | 126 | 0.079 | -3.44 |
| compound 163 | 127.9 | 0.088 | -3.85 |
| compound 164 | 138.2 | 0.052 | -2.86 |
| compound 166 | 130.5 | 0.09 | -2.99 |
| compound 168 | 129.6 | 0.099 | -2.93 |
| compound 170 | 129.8 | 0.063 | -3.57 |
| compound 173 | 126.1 | 0.035 | -4.83 |
| compound 175 | 123 | 0.059 | -3.68 |

(continued)

| cationic lipid used | particle size (nm) | PdI | zeta potential (mV) |
|---|---|---|---|
| compound 181 | 90.7 | 0.072 | -5.6 |
| compound 182 | 92.0 | 0.084 | -5.4 |
| compound 188 | 92.8 | 0.051 | -6.0 |
| compound 194 | 117.5 | 0.12 | -5.1 |
| Comparative Example | 123.80 | 0.075 | -2.68 |

[Experimental Example 7] Evaluation of in vivo gene expression

1. Preparation of mRNA-encapsulating LNP

LNP encapsulating mRNA that expresses luciferase (cationic lipid:DOPC:Chol:DMG-PEG2k = 52.5:7.5:40:1.5 (molar ratio)) was prepared by the method described in [Experimental Example 6], 1.

2. Evaluation of in vivo gene expression

The prepared each mRNA-encapsulating LNP dispersion was administered from the tail vein of BALB/c mouse (male, 5-week-old). The dose of mRNA was set to 0.05 mg per 1 kg body weight of the mouse, and the dose of the mRNA-encapsulating LNP dispersion was set to 10 μL per 1 g body weight of the mouse. After 4.5 hr from the administration of the mRNA-encapsulating LNP dispersion, a PBS solution of D-luciferin potassium was administered into the abdominal cavity of the mouse. The dose of D-luciferin potassium was set to 3 mg per mouse, and the dose of the PBS solution of D-luciferin potassium was set to 200 μL per mouse. After 15 min from the administration of the PBS solution of D-luciferin potassium, the mouse was euthanized, and the liver was isolated. The luminescence in the liver was quantified using an in vivo imaging system (IVIS) with an exposure of 10 sec. The amount of luminescence in the liver was quantified by image analysis using Live Imaging software attached to IVIS. The amount of luminescence (photons/sec) was calculated from the acquired image and used as an index of gene expression activity. The cationic lipids used, the luminescence intensity in the liver, and the relative luminescence intensity (= luminescence intensity in the liver when cationic lipid of Example was used/luminescence intensity in the liver when cationic lipid of Comparative Example was used) are shown in Table 9. "E+0a" (a: integer) indicated in Table 9 means "$10^a$". For example, "3.94E+09" means "$3.94 \times 10^9$". In Comparative Example, the cationic lipid described in Table 2 was used.

Table 9]

| cationic lipid used | amount of luminescence (photons/sec) in liver | relative amount of luminescence |
|---|---|---|
| compound 58 | 1.72E+10 | 8.40 |
| compound 166 | 8.49E+09 | 4.15 |
| compound 173 | 9.37E+09 | 4.57 |
| compound 175 | 1.14E+10 | 5.55 |
| compound 181 | 1.76E+10 | 12.3 |
| compound 182 | 1.4E+10 | 8.61 |
| compound 188 | 2.52E+10 | 6.85 |
| Comparative Example | 2.05E+09 | 1.00 |

[Experimental Example 8] Evaluation of hemolysis activity (membrane fusion ability) at pH 7.4 and 5.5

1. Preparation of various LNPs

LNP encapsulating mRNA that expresses luciferase (cationic lipid:DOPC:Chol:DMG-PEG2k = 52.5:7.5:40:1.5 (molar ratio)) was prepared by the method described in [Experimental Example 3], 1. As cationic lipid, the cationic lipid of Comparative Example, compound 94, compound 112, compound 162, compound 181, or compound 194 was used.

2. Obtaining mouse red blood cells

6 to 7-week-old male ICR mice were euthanized, and about 1000 μL of blood was collected from the inferior vena cava. The collected blood was immediately mixed with 1.0 μL of heparin solution (5000 U/5 mL). About 9 mL of PBS was added to the blood to make the total volume 10 mL. The mixture was then mixed by inversion and centrifuged (4°C, 400 g, 10 min). The supernatant containing plasma components was removed using a Pasteur pipette. About 9 mL of PBS was added to the blood cell components to make the total volume 10 mL, and the mixture was centrifuged again. A similar washing procedure was repeated 4 times to obtain mouse red blood cells.

3. Evaluation of hemolysis activity

2, 4, 6, 8, and 10 μL of mouse red blood cells were weighed and diluted with PBS containing 1 w/v% Triton-X100. The entire diluted solution was transferred to a clear 96-well plate, and the absorbance at 545 nm was measured using a plate reader. A calibration curve was created using these samples, and the point where the absorbance reached 1 was determined as the amount of blood cells to be used in the hemolysis assay. Empty LNP solution was weighed into an Eppendorf tube and diluted with malic acid-PBS buffer (pH 5.5, pH 7.4), followed by addition of mouse red blood cells. The final lipid concentration was 100 μM at pH 7.4 and 33.3 μM at pH 5.5, and the final volume of the solution was 250 μL. Each tube was shaken at 1900 rpm for 15 min. Each sample was centrifuged under centrifugation conditions (4°C, 400 g, 5 min), and 100 μL of the supernatant was transferred to a clear 96-well plate and the absorbance at 545 nm was measured. Untreated red blood cells were used as the negative control. 1 w/v% Triton-X was used as the positive control. The hemolysis activity of each sample was calculated using the following formula:
Hemolysis activity (%) = 100×(absorbance of each sample - absorbance of negative control)/(absorbance of positive control - absorbance of negative control). The hemolysis activity of LNP at pH 7.4 is shown in Fig. 3, and the hemolysis activity of LNP at pH 5.5 is shown in Fig. 4.

4. Results

As shown in Fig. 3, none of the LNPs containing compound 94, compound 112, compound 162, compound 181, compound 194, or the cationic lipid of Comparative Example exhibited hemolysis activity at physiological pH (7.4). On the other hand, as shown in Fig. 4, at the pH of the endosomal environment (5.5), the LNPs containing compound 94, compound 112, compound 162, compound 181, and compound 194 exhibited superior hemolysis activity compared to the LNP containing the cationic lipid of Comparative Example. Therefore, the LNP containing the cationic lipid of the present invention is superior in the membrane fusion ability and is therefore superior in nucleic acid delivery.

[Experimental Example 9] Evaluation of gene expression in vivo

1. Preparation of mRNA-encapsulated LNP

(1) Preparation of lipid ethanol solution and nucleic acid buffer solution

(a) L/R ratio (= total lipid (nmol)/nucleic acid amount (μg))=33 nmol/μg

A 20 mM ethanol solution of cationic lipid, a 20 mM ethanol solution of DOPC, a 40 mM ethanol solution of Chol, and a 2 mM ethanol solution of DMG-PEG 2k were mixed in a ratio of cationic lipid:DOPC:Chol=48.5:7.5:42.5:1.5 (molar ratio) to obtain a total lipid amount of 660 nmol to prepare a lipid ethanol solution (total volume: 82.5 μL). As cationic lipid, the cationic lipid of Comparative Example, compound 94, or compound 162 was used.
CleanCap (registered trademark) Fluc-mRNA (manufactured by TriLink BioTechnologies) (hereinafter referred to as "mRNA") (20.0 μg) was weighed and added to acidic citrate buffer (20 mM, pH 5.0, 227.5 μL) to prepare an acidic buffer solution of nucleic acid (total volume: 247.5 μL).

(b) L/R ratio (=total lipid amount (nmol)/nucleic acid amount (μg))=50 nmol/μg

A 20 mM ethanol solution of cationic lipid, a 20 mM ethanol solution of DOPC, a 40 mM ethanol solution of Chol, and a 2 mM ethanol solution of DMG-PEG 2k were mixed in a ratio of cationic lipid:DOPC:Chol=48.5:7.5:42.5:1.5 (molar ratio) to give a total lipid amount of 1000 nmol, and a lipid ethanol solution (total volume: 125 μL) was prepared. As cationic lipid, the cationic lipid of Comparative Example, compound 94, or compound 162 was used.
mRNA (20.0 μg) was weighed and added to to acidic citrate buffer (20 mM, pH 5.0, 355 μL) to prepare an acidic buffer solution of nucleic acid (total amount: 375 μL).

(c) L/R ratio (=total lipid amount (nmol)/nucleic acid amount (μg))=100 nmol/μg

A 20 mM ethanol solution of cationic lipid, a 20 mM ethanol solution of DOPC, a 40 mM ethanol solution of Chol, and a 2 mM ethanol solution of DMG-PEG 2k were mixed in a ratio of cationic lipid:DOPC:Chol=48.5:7.5:42.5:1.5 (molar ratio) to give a total lipid amount of 4000 nmol, and a lipid ethanol solution (total volume: 500 μL) was prepared. As cationic lipid, the cationic lipid of Comparative Example, compound 94, or compound 162 was used.
mRNA (20.0 μg) was weighed and added to to acidic citrate buffer (20 mM, pH 5.0, 1480 μL) to prepare an acidic buffer solution of nucleic acid (total amount: 1500 μL).

(2) Preparation of mRNA-encapsulating LNP using micro flow path

The acidic buffer solution of nucleic acid and the ethanol solution of lipid, obtained in the above-mentioned (1) (a) to (c), were each weighed in a syringe. Using an ultra high-speed nanomedicament producing apparatus NanoAssmblr (manufactured by Precision NanoSystems), LNP was prepared under the conditions of addition rate of acidic buffer solution of nucleic acid: 9 mL/min, addition rate of ethanol solution of lipid: 3 mL/min, and syringe holder temperature: 25°C, and collected in a 15 mL tube. TBS buffer (50 mM, pH 7.6) was added to the 15 mL tube until the total volume was four times the volume of the mixed solution, the obtained mixture was transferred to Amicon Ultra 4, and ultrafiltration was performed under centrifugation conditions (25°C, 1000 g, 6 min) to concentrate the mixture to about 500 μL. The obtained concentrate was diluted with TBS to 4 mL, and the mixture was concentrated again under centrifugation conditions (25°C, 1000 g, 6 min), and this operation was performed twice. The obtained concentrate was diluted with PBS to a lipid concentration of 10 μg/mL to give a dispersion containing mRNA-encapsulating LNP.

2. Evaluation of in vivo gene expression

The prepared each mRNA-encapsulating LNP dispersion was administered from the tail vein of BALB/c mouse (male, 5-week-old). The dose of mRNA was set to 0.10 mg per 1 kg body weight of the mouse, and the dose of the mRNA-encapsulating LNP dispersion was set to 10 μL per 1 g body weight of the mouse. After 4.5 hr from the administration of the mRNA-encapsulating LNP dispersion, a PBS solution of D-luciferin potassium was administered into the abdominal cavity of the mouse. The dose of D-luciferin potassium was set to 3 mg per mouse, and the dose of the PBS solution of D-luciferin potassium was set to 200 μL per mouse. After 15 min from the administration of the PBS solution of D-luciferin potassium, the mouse was euthanized, and the liver was isolated. The luminescence in the liver was quantified using an in vivo imaging system (IVIS) with an exposure of 10 sec. The amount of luminescence in the liver was quantified by image analysis using Live Imaging software attached to IVIS. The amount of luminescence (photons/sec) was calculated from the acquired image and used as an index of gene expression activity. The cationic lipids used and the total amount of luminescence (Total Flux(p/s)) in the liver are shown in Fig. 5 (L/R ratio = 33 nmol/μg), Fig. 6 (L/R ratio = 50 mmol/μg), and Fig. 7 (L/R ratio = 100 nmol/μg). "E+a" or "E+0a" (a: integer) indicated in Fig. 5 to Fig. 7 means "$10^a$". For example, "1.5E+10" means "$1.5\times10^{10}$".

3. Results

The higher the total amount of luminescence (Total Flux(p/s)) in the liver is, the higher the gene expression is. As shown in Fig. 5 to Fig. 7, the LNPs containing the cationic lipids of the Examples exhibited superior gene expression activity compared to the LNP containing the cationic lipid of the Comparative Example. Therefore, it was clarified that the LNP containing the cationic lipid of the present invention is beneficial as an LNP capable of promoting the expression of mRNA.

[Industrial Applicability]

The cationic lipid of the present invention is useful for nucleic acid medicaments, gene therapy, biochemical experiments, and the like.

## Claims

**1.** A cationic lipid represented by the formula (1):

[Chem. 1]

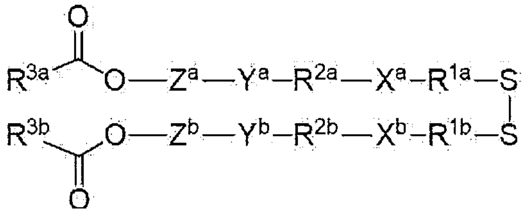

(1)

(in the formula (1), $R^{1a}$ and $R^{1b}$ are each independently an alkylene group having 1 to 6 carbon atoms,

$X^a$ and $X^b$ are each independently an acyclic alkyl tertiary amino group having 1 to 6 carbon atoms and 1 tertiary amino group, or a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms or an oxydialkylene group having 2 to 8 carbon atoms,
$Y^a$ and $Y^b$ are each independently an ester bond, an amide bond, a carbamate bond, an ether bond, or a urea bond,
$Z^a$ and $Z^b$ are each independently a divalent group derived from an aromatic compound having 3 to 16 carbon atoms, at least one aromatic ring, and optionally having a heteroatom,
$R^{3a}$ is
(ia) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group),
(iia) a monovalent group having 10 to 50 carbon atoms and at least two carbonyl groups (excluding a monovalent group containing a residue of a liposoluble vitamin having a hydroxy group and a residue of a sterol derivative having a hydroxy group),
(iiia) a monovalent group represented by the formula (2):

$$*\text{-}R^4\text{-}X^1\text{-}R^5 \qquad (2)$$

(in the formula (2), * is a bonding position,

$R^4$ is an alkylene group having 1 to 10 carbon atoms,
$X^1$ is a carbamate bond, a carbonate bond, or an amide bond, and
$R^5$ is an alkyl group having 1 to 25 carbon atoms, and $R^5$ is optionally substituted by a substituent selected from the group consisting of a halogen atom and a hydroxy group),

(iva) a monovalent group represented by the formula (3):

$$*\text{-}R^6\text{-CO-O-}R^7 \qquad (3)$$

(in the formula (3), * is a bonding position,

$R^6$ is an alkylene group having 1 to 10 carbon atoms, and
$R^7$ is an alkyl group having 1 to 25 carbon atoms and substituted by at least one halogen atom),

(va) a monovalent group represented by the formula (4):

[Chem. 2]

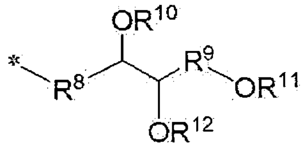

(4)

(in the formula (4), * is a bonding position,

$R^8$ and $R^9$ are each independently an alkylene group having 1 to 10 carbon atoms, an alkenediyl group having 2 to 10 carbon atoms, or an alkynediyl group having 2 to 10 carbon atoms,
$R^{10}$ to $R^{12}$ are each independently a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group)),

(via) a monovalent group represented by the formula (5):

[Chem. 3]

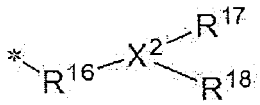

(5)

(in the formula (5), * is a bonding position,
$X^2$ is a nitrogen atom or a trivalent group represented by the formula (6):

[Chem. 4]

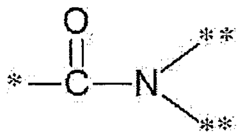

(6)

(in the formula (6), * is a bonding position with $R^{16}$, and

** is a bonding position with $R^{17}$ or $R^{18}$),
when $X^2$ is a nitrogen atom, $R^{16}$ is an alkylene group having 1 to 10 carbon atoms, an alkenediyl group having 2 to 10 carbon atoms, or an alkynediyl group having 2 to 10 carbon atoms, and $R^{16}$ is optionally substituted by a substituent selected from the group consisting of a halogen atom and a hydroxy group,
when $X^2$ is a trivalent group represented by the formula (6), $R^{16}$ is an alkylene group having 1 to 10 carbon atoms, and $R^{16}$ is optionally substituted by a substituent selected from the group consisting of a halogen atom and a hydroxy group,
when $X^2$ is a nitrogen atom, $R^{17}$ and $R^{18}$ are each independently an alkyl group having 1 to 10 carbon atoms, an alkenyl group having 2 to 10 carbon atoms, or an alkynyl group having 2 to 10 carbon atoms, and $R^{17}$ and $R^{18}$ are each independently optionally substituted by a substituent selected from the group consisting of a halogen atom and a hydroxy group, and
when $X^2$ is a trivalent group represented by the formula (6), $R^{17}$ and $R^{18}$ are each independently an alkyl group having 1 to 10 carbon atoms, and $R^{17}$ and $R^{18}$ are each independently optionally substituted by a substituent selected from the group consisting of a halogen atom and a hydroxy group),

(viia) a monovalent group represented by the formula (7):

[Chem. 5]

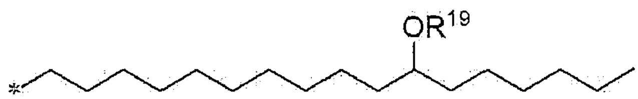

(7)

(in the formula (7), * is a bonding position, and
$R^{19}$ is a hydrogen atom, a benzyl group, or a *-Si ($R^{13}$)($R^{14}$)($R^{15}$) group (wherein * is a bonding position, and $R^{13}$ to $R^{15}$ are each independently an alkyl group having 1 to 4 carbon atoms or a phenyl group), or a *-CO-$R^{20}$ group (wherein * is a bonding position, and $R^{20}$ is an alkyl group having 1 to 9 carbon atoms)),
(viiia) a monovalent group represented by the formula (8):

[Chem. 6]

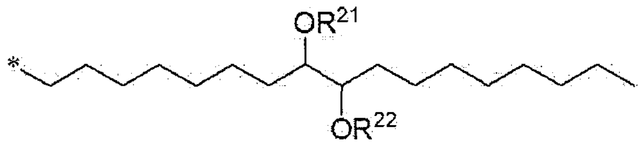
(8)

(in the formula (8), * is a bonding position, and
$R^{21}$ and $R^{22}$ are each independently a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group)),
(ixa) a monovalent group represented by the formula (9):

[Chem. 7]

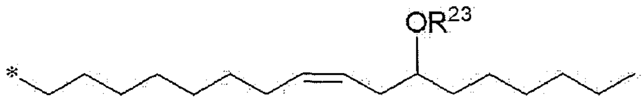
(9)

(in the formula (9), * is a bonding position, and
$R^{23}$ is a hydrogen atom, a benzyl group, or a *-Si($R^{13}$)($R^{14}$)($R^{15}$) group (wherein * is a bonding position, and $R^{13}$ to $R^{15}$ are each independently an alkyl group having 1 to 4 carbon atoms or a phenyl group)),
(xa) a monovalent group represented by the formula (10):

[Chem. 8]

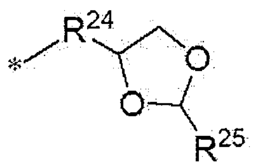
(10)

(in the formula (10), * is a bonding position,

$R^{24}$ is an alkylene group having 1 to 10 carbon atoms, an alkenediyl group having 2 to 10 carbon atoms, or an alkynediyl group having 2 to 10 carbon atoms, and
$R^{25}$ is an alkyl group having 1 to 30 carbon atoms, an alkenyl group having 2 to 30 carbon atoms, or an alkynyl group having 2 to 30 carbon atoms),

(xia) a monovalent group represented by the formula (11):

[Chem. 9]

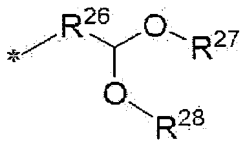
(11)

(in the formula (11), * is a bonding position, and

$R^{26}$ is an alkylene group having 1 to 10 carbon atoms, an alkenediyl group having 2 to 10 carbon atoms, or an alkynediyl group having 2 to 10 carbon atoms, and

$R^{27}$ and $R^{28}$ are each independently an alkyl group having 1 to 10 carbon atoms, an alkenyl group having 2 to 10 carbon atoms, or an alkynyl group having 2 to 10 carbon atoms), or (xiia) a monovalent group represented by the formula (12):

[Chem. 10]

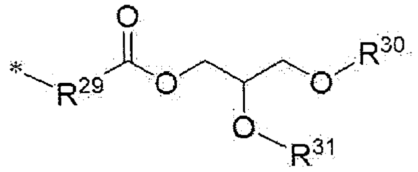

(12)

(in the formula (12), * is a bonding position,
$R^{29}$ is an alkylene group having 1 to 10 carbon atoms, and
$R^{30}$ and $R^{31}$ are each independently an alkyl group having 1 to 10 carbon atoms),
$R^{3b}$ is

(ib) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group),
(iib) a monovalent group having 10 to 50 carbon atoms and at least two carbonyl groups (excluding a monovalent group containing a residue of a liposoluble vitamin having a hydroxy group and a residue of a sterol derivative having a hydroxy group),
(iiib) a monovalent group represented by the formula (2), (ivb) a monovalent group represented by the formula (3), (vb) a monovalent group represented by the formula (4), (vib) a monovalent group represented by the formula (5), (viib) a monovalent group represented by the formula (7), (viiib) a monovalent group represented by the formula (8), (ixb) a monovalent group represented by the formula (9), (xb) a monovalent group represented by the formula (10), (xib) a monovalent group represented by the formula (11), (xiib) a monovalent group represented by the formula (12), (xiiib) a monovalent group which is an alkyl group having 1 to 30 carbon atoms, an alkenyl group having 2 to 20 carbon atoms, or an alkynyl group having 2 to 20 carbon atoms, in which one ethylene group in the alkyl group is optionally replaced by one ester bond, or
(xivb) an $R^{3c}$-CO-$(CH_2)_p$- group (wherein $R^{3c}$ is a residue of a liposoluble vitamin having a hydroxy group or a residue of a sterol derivative having a hydroxy group, and p is an integer of 1 to 8), and
$R^{3a}$ and $R^{3b}$ may be the same or different).

**2.** The cationic lipid according to claim 1, wherein $R^{3a}$ is

(ia) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group), or
(iia) a monovalent group having 10 to 50 carbon atoms and at least two carbonyl groups (excluding a monovalent group containing a residue of a liposoluble vitamin having a hydroxy group and a residue of a sterol derivative having a hydroxy group).

**3.** The cationic lipid according to claim 1, wherein $R^{3a}$ is

(ia) a monovalent group having 10 to 50 carbon atoms, one carbonyl group, and at least one unsaturated bond selected from the group consisting of an olefinic carbon-carbon double bond and a carbon-carbon triple bond (excluding a monovalent group containing a residue of a liposoluble vitamin having a hydroxy group and a residue of a sterol derivative having a hydroxy group), or
(iia) a monovalent group having 10 to 50 carbon atoms and at least two carbonyl groups (excluding a monovalent group containing a residue of a liposoluble vitamin having a hydroxy group and a residue of a sterol derivative having a hydroxy group),
$R^{3b}$ is
(ib) a monovalent group having 10 to 50 carbon atoms, one carbonyl group, and at least one unsaturated bond selected from the group consisting of an olefinic carbon-carbon double bond and a carbon-carbon triple bond

(excluding a monovalent group containing a residue of a liposoluble vitamin having a hydroxy group and a residue of a sterol derivative having a hydroxy group),

(iib) a monovalent group having 10 to 50 carbon atoms and at least two carbonyl groups (excluding a monovalent group containing a residue of a liposoluble vitamin having a hydroxy group and a residue of a sterol derivative having a hydroxy group),

(xiiib) a monovalent group which is an alkyl group having 1 to 30 carbon atoms, an alkenyl group having 2 to 20 carbon atoms, or an alkynyl group having 2 to 20 carbon atoms, in which one ethylene group in the alkyl group is optionally replaced by one ester bond, or

(xivb) a $R^{3c}$-CO-$(CH_2)_p$- group (wherein $R^{3c}$ is a residue of a liposoluble vitamin having a hydroxy group or a residue of a sterol derivative having a hydroxy group, and p is an integer of 1 to 8), and

$R^{3a}$ and $R^{3b}$ may be the same or different.

**4.** The cationic lipid according to claim 1, wherein $R^{3a}$ is

(iiia) a monovalent group represented by the formula (2),
(iva) a monovalent group represented by the formula (3),
(va) a monovalent group represented by the formula (4),
(via) a monovalent group represented by the formula (5),
(viia) a monovalent group represented by the formula (7),
(viiia) a monovalent group represented by the formula (8),
(ixa) a monovalent group represented by the formula (9),
(xa) a monovalent group represented by the formula (10),
(xia) a monovalent group represented by the formula (11), or
(xiia) a monovalent group represented by the formula (12),
$R^{3b}$ is
(iiib) a monovalent group represented by the formula (2),
(ivb) a monovalent group represented by the formula (3),
(vb) a monovalent group represented by the formula (4),
(vib) a monovalent group represented by the formula (5),
(viib) a monovalent group represented by the formula (7),
(viiib) a monovalent group represented by the formula (8),
(ixb) a monovalent group represented by the formula (9),
(xb) a monovalent group represented by the formula (10),
(xib) a monovalent group represented by the formula (11),
(xiib) a monovalent group represented by the formula (12),
(xiiib) a monovalent group which is an alkyl group having 1 to 30 carbon atoms, an alkenyl group having 2 to 20 carbon atoms, or an alkynyl group having 2 to 20 carbon atoms, in which one ethylene group in the alkyl group is optionally replaced by one ester bond, or
(xivb) a $R^{3c}$-CO-$(CH_2)_p$- group (wherein $R^{3c}$ is a residue of a liposoluble vitamin having a hydroxy group or a residue of a sterol derivative having a hydroxy group, and p is an integer of 1 to 8), and
$R^{3a}$ and $R^{3b}$ may be the same or different.

**5.** The cationic lipid according to any one of claims 1 to 4, wherein $Z^a$ and $Z^b$ are each independently a divalent group represented by the formula (13):

[Chem. 11]

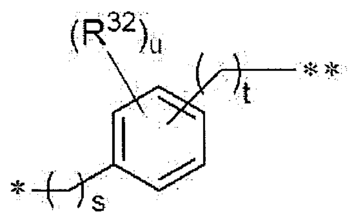

(13)

(in the formula (13), * is a bonding position with O in the formula (1),

** is a bonding position with $Y^a$ or $Y^b$ in the formula (1),

s is an integer of 0 to 3,
t is an integer of 0 to 3,
u is an integer of 0 to 4, and
$R^{32}$ in the number of u are each independently a substituent).

**6.** The cationic lipid according to claim 5, wherein s is 0.

**7.** The cationic lipid according to any one of claims 1 to 4, wherein $X^a$ and $X^b$ are each independently a cyclic alkylene tertiary amino group having 2 to 5 carbon atoms and 1 or 2 tertiary amino groups.

**8.** The cationic lipid according to claim 1 or 2, wherein $R^{3a}$ is

(ia-1) a monovalent group having 10 to 50 carbon atoms and represented by the formula (14):

$$*\text{-}R^{33}\text{-CO-}X^{3}\text{-}R^{34} \quad (14)$$

(in the formula (14), * is a bonding position,

$R^{33}$ is an alkylene group having 2 to 10 carbon atoms, an alkenediyl group having 2 to 10 carbon atoms, or an alkynediyl group having 2 to 10 carbon atoms, and $R^{33}$ is optionally substituted by a substituent selected from the group consisting of a halogen atom, a hydroxy group, and a hydrocarbon ring group having 3 to 12 carbon atoms,
$R^{34}$ is an alkyl group having 1 to 40 carbon atoms, an alkenyl group having 2 to 40 carbon atoms, or an alkynyl group having 2 to 40 carbon atoms, and $R^{34}$ is optionally substituted by a substituent selected from the group consisting of a halogen atom, a hydroxy group, and a hydrocarbon ring group having 3 to 12 carbon atoms, at least one of $R^{33}$ and $R^{34}$ has at least one unsaturated bond selected from the group consisting of an olefinic carbon-carbon double bond and a carbon-carbon triple bond, and
$X^3$ is an oxygen atom, NH, or a sulfur atom),

(ia-2) a monovalent group having 50 or less carbon atoms and represented by the formula (15):

[Chem. 12]

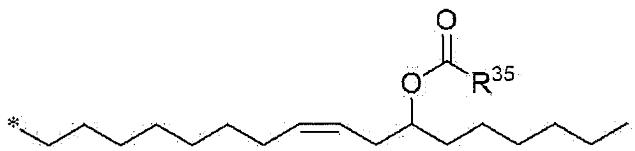

(15)

(in the formula (15), * is a bonding position, and
$R^{35}$ is an alkyl group having 1 to 20 carbon atoms, an alkenyl group having 2 to 20 carbon atoms, an alkynyl group having 2 to 20 carbon atoms, or hydrocarbon ring group having 3 to 12 carbon atoms, at least one ethylene group or at least one trimethylene group in $R^{35}$ is optionally replaced by at least one bond selected from the group consisting of an ester bond, an amide bond, a carbamate bond, and a carbonate bond, and $R^{35}$ is optionally substituted by a substituent selected from the group consisting of an alkoxy group having 1 to 4 carbon atoms, a 3- to 14-membered heterocyclic group, and a hydrocarbon ring group having 3 to 12 carbon atoms),
(iia-1) a monovalent group having 50 or less carbon atoms and represented by the formula (16):

$$*\text{-}R^{36}\text{-CO-}X^{4}\text{-}R^{37} \quad (16)$$

(in the formula (16), * is a bonding position,

$R^{36}$ is an alkylene group having 2 to 9 carbon atoms, an alkenediyl group having 2 to 9 carbon atoms, or an alkynediyl group having 2 to 9 carbon atoms, and $R^{36}$ is optionally substituted by a substituent selected from the group consisting of a halogen atom, a hydroxy group, and a hydrocarbon ring group having 3 to 12 carbon atoms,
$R^{37}$ is an alkyl group having 7 to 45 carbon atoms, an alkenyl group having 7 to 45 carbon atoms, or an alkynyl group having 7 to 45 carbon atoms, at least one ethylene group or at least one trimethylene group in $R^{37}$ is

replaced by at least one bond selected from the group consisting of an ester bond, an amide bond, a carbamate bond, and a carbonate bond, and $R^{37}$ is optionally substituted by a substituent selected from the group consisting of a halogen atom, a hydroxy group, and a hydrocarbon ring group having 3 to 12 carbon atoms, and
$X^4$ is an oxygen atom, NH, or a sulfur atom),

(iia-2) a monovalent group having 10 to 50 carbon atoms and represented by the formula (17):

$$*\text{-}R^{33}\text{-O-}R^{39} \quad (17)$$

(in the formula (17), * is a bonding position, and

$R^{38}$ is an alkylene group having 2 to 10 carbon atoms, an alkenediyl group having 2 to 10 carbon atoms, or an alkynediyl group having 2 to 10 carbon atoms, at least one ethylene group or at least one trimethylene group in $R^{38}$ is optionally replaced by at least one bond selected from the group consisting of an ester bond, an amide bond, a carbamate bond, and a carbonate bond, and $R^{38}$ is optionally substituted by a substituent selected from the group consisting of a halogen atom, a hydroxy group, and a hydrocarbon ring group having 3 to 12 carbon atoms, and
$R^{39}$ is an alkyl group having 3 to 30 carbon atoms, an alkenyl group having 4 to 30 carbon atoms, or an alkenyl group having 4 to 30 carbon atoms, at least two methylene groups in $R^{39}$ are replaced by at least two carbonyl groups, and at least one methylene group in $R^{39}$ is optionally replaced by at least one ether bond),

(iia-3) a monovalent group having 50 or less carbon atoms and represented by the formula (18):

[Chem. 13]

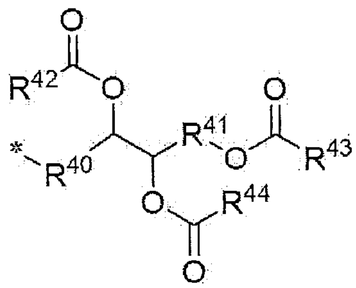

(18)

(in the formula (18), * is a bonding position,

$R^{40}$ and $R^{41}$ are each independently an alkylene group having 3 to 10 carbon atoms, and
$R^{42}$ to $R^{44}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{42}$ is optionally replaced by at least one bond selected from the group consisting of an ester bond, an amide bond, a carbamate bond, and a carbonate bond, at least one ethylene group or at least one trimethylene group in $R^{43}$ is optionally replaced by at least one bond selected from the group consisting of an ester bond, an amide bond, a carbamate bond, and a carbonate bond, at least one ethylene group or at least one trimethylene group in $R^{44}$ is optionally replaced by at least one bond selected from the group consisting of an ester bond, an amide bond, a carbamate bond, and a carbonate bond, and $R^{42}$ to $R^{44}$ are each independently optionally substituted by a substituent selected from the group consisting of a halogen atom, a hydroxy group, and a hydrocarbon ring group having 3 to 12 carbon atoms), or

(iia-4) a monovalent group having 50 or less carbon atoms and represented by the formula (19):

[Chem. 14]

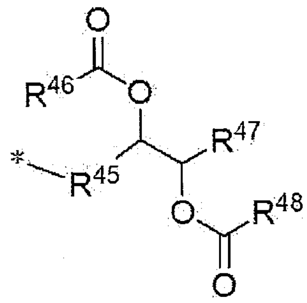

(19)

(in the formula (19), * is a bonding position,

$R^{45}$ is an alkylene group having 5 to 10 carbon atoms, and
$R^{46}$ to $R^{48}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{46}$ is optionally replaced by at least one bond selected from the group consisting of an ester bond, an amide bond, a carbamate bond, and a carbonate bond, at least one ethylene group or at least one trimethylene group in $R^{47}$ is optionally replaced by at least one bond selected from the group consisting of an ester bond, an amide bond, a carbamate bond, and a carbonate bond, at least one ethylene group or at least one trimethylene group in $R^{48}$ is optionally replaced by at least one bond selected from the group consisting of an ester bond, an amide bond, a carbamate bond, and a carbonate bond, and $R^{46}$ to $R^{48}$ are each independently optionally substituted by a substituent selected from the group consisting of a halogen atom, a hydroxy group, and a hydrocarbon ring group having 3 to 12 carbon atoms),
$R^{3b}$ is

(ib-1) a monovalent group having 10 to 50 carbon atoms and represented by the formula (14),
(ib-2) a monovalent group having 50 or less carbon atoms and represented by the formula (15),
(iib-1) a monovalent group having 50 or less carbon atoms and represented by the formula (16),
(iib-2) a monovalent group having 10 to 50 carbon atoms and represented by the formula (17),
(iib-3) a monovalent group having 50 or less carbon atoms and represented by the formula (18),
(iib-4) a monovalent group having 50 or less carbon atoms and represented by the formula (19),
(iiib) a monovalent group represented by the formula (2),
(ivb) a monovalent group represented by the formula (3),
(vb) a monovalent group represented by the formula (4),
(vib) a monovalent group represented by the formula (5),
(viib) a monovalent group represented by the formula (7),
(viiib) a monovalent group represented by the formula (8),
(ixb) a monovalent group represented by the formula (9),
(xb) a monovalent group represented by the formula (10),
(xib) a monovalent group represented by the formula (11),
(xiib) a monovalent group represented by the formula (12),
(xiiib) a monovalent group which is an alkyl group having 1 to 30 carbon atoms, an alkenyl group having 2 to 20 carbon atoms, or an alkynyl group having 2 to 20 carbon atoms, in which one ethylene group in the alkyl group is optionally replaced by one ester bond, or
(xivb) $R^{3c}$-CO-$(CH_2)_p$- group (wherein $R^{3c}$ is a residue of a liposoluble vitamin having a hydroxy group or a residue of a sterol derivative having a hydroxy group, and p is an integer of 1 to 8), and
$R^{3a}$ and $R^{3b}$ may be the same or different.

**9.** The cationic lipid according to claim 8, wherein $R^{35}$ in the formula (15) is an alkyl group having 1 to 20 carbon atoms, an unsubstituted alkenyl group having 2 to 20 carbon atoms, an unsubstituted alkynyl group having 2 to 20 carbon atoms, or a hydrocarbon ring group having 3 to 12 carbon atoms, and the alkyl group is optionally substituted by a hydrocarbon ring group having 3 to 12 carbon atoms.

**10.** The cationic lipid according to claim 8, wherein $R^{37}$ in the formula (16) is

(iia-1-1) a monovalent group represented by the formula (20):

[Chem. 15]

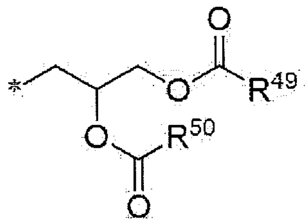

(20)

(in the formula (20), * is a bonding position, and
$R^{49}$ and $R^{50}$ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in $R^{49}$ is optionally replaced by at least one bond selected from the group consisting of an ester bond, an amide bond, a carbamate bond, and a carbonate bond, at least one ethylene group or at least one trimethylene group in $R^{50}$ is optionally replaced by at least one bond selected from the group consisting of an ester bond, an amide bond, a carbamate bond, and a carbonate bond, and $R^{49}$ and $R^{50}$ are each independently optionally substituted by a substituent selected from the group consisting of a halogen atom, a hydroxy group, and a hydrocarbon ring group having 3 to 12 carbon atoms),
(iia-1-2) a monovalent group represented by the formula (21):

[Chem. 16]

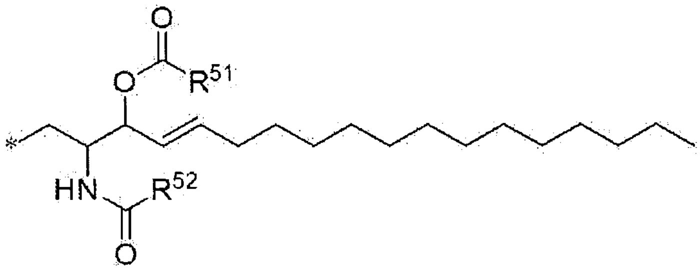

(21)

(in the formula (21), * is a bonding position, and
$R^{51}$ and $R^{52}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{51}$ is optionally replaced by at least one bond selected from the group consisting of an ester bond, an amide bond, a carbamate bond, and a carbonate bond, at least one ethylene group or at least one trimethylene group in $R^{52}$ is optionally replaced by at least one bond selected from the group consisting of an ester bond, an amide bond, a carbamate bond, and a carbonate bond, and $R^{51}$ and $R^{52}$ are each independently optionally substituted by a substituent selected from the group consisting of a halogen atom, a hydroxy group, and a hydrocarbon ring group having 3 to 12 carbon atoms), or
(iia-1-3) a monovalent group represented by the formula (22):

[Chem. 17]

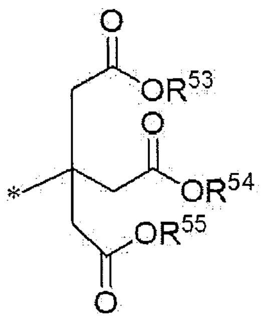

(22)

(in the formula (22), * is a bonding position, and
$R^{53}$ to $R^{55}$ are each independently an alkyl group having 1 to 10 carbon atoms, an alkenyl group having 2 to 10 carbon atoms, or an alkynyl group having 2 to 10 carbon atoms, at least one ethylene group or at least one

trimethylene group in $R^{53}$ is optionally replaced by at least one bond selected from the group consisting of an ester bond, an amide bond, a carbamate bond, and a carbonate bond, at least one ethylene group or at least one trimethylene group in $R^{54}$ is optionally replaced by at least one bond selected from the group consisting of an ester bond, an amide bond, a carbamate bond, and a carbonate bond, at least one ethylene group or at least one trimethylene group in $R^{55}$ is optionally replaced by at least one bond selected from the group consisting of an ester bond, an amide bond, a carbamate bond, and a carbonate bond, and $R^{53}$ to $R^{55}$ are each independently optionally substituted by a substituent selected from the group consisting of a halogen atom, a hydroxy group, and a hydrocarbon ring group having 3 to 12 carbon atoms).

**11.** The cationic lipid according to claim 8, wherein $R^{39}$ in the formula (17) is

(iia-2-1) a monovalent group represented by the formula (23):

[Chem. 18]

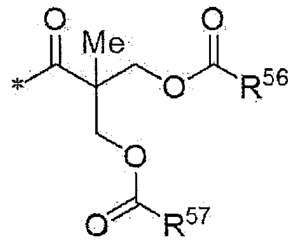

(23)

(in the formula (23), * is a bonding position,

Me is a methyl group, and
$R^{56}$ and $R^{57}$ are each independently an alkyl group having 1 to 10 carbon atoms, an alkenyl group having 2 to 10 carbon atoms, or an alkynyl group having 2 to 10 carbon atoms, at least one ethylene group or at least one trimethylene group in $R^{56}$ is optionally replaced by at least one bond selected from the group consisting of an ester bond, an amide bond, a carbamate bond, and a carbonate bond, at least one ethylene group or at least one trimethylene group in $R^{57}$ is optionally replaced by at least one bond selected from the group consisting of an ester bond, an amide bond, a carbamate bond, and a carbonate bond, and $R^{56}$ and $R^{57}$ are each independently optionally substituted by a substituent selected from the group consisting of a halogen atom, a hydroxy group, and a hydrocarbon ring group having 3 to 12 carbon atoms), or

(iia-2-2) a monovalent group represented by the formula (24):

[Chem. 19]

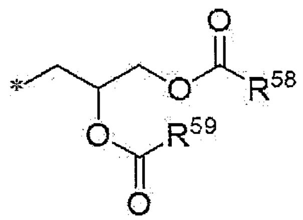

(24)

(in the formula (24), * is a bonding position, and
$R^{58}$ and $R^{59}$ are each independently an alkyl group having 1 to 17 carbon atoms, an alkenyl group having 2 to 17 carbon atoms, or an alkynyl group having 2 to 17 carbon atoms, at least one ethylene group or at least one trimethylene group in $R^{58}$ is optionally replaced by at least one bond selected from the group consisting of an ester bond, an amide bond, a carbamate bond, and a carbonate bond, at least one ethylene group or at least one trimethylene group in $R^{59}$ is optionally replaced by at least one bond selected from the group consisting of an ester bond, an amide bond, a carbamate bond, and a carbonate bond, and $R^{58}$ and $R^{59}$ are each independently optionally substituted by a substituent selected from the group consisting of a halogen atom, a hydroxy group, and a hydrocarbon ring group having 3 to 12 carbon atoms).

**12.** A lipid membrane structure comprising the cationic lipid according to any one of claims 1 to 4 as a constituent lipid of the membrane.

**13.** The lipid membrane structure according to claim 12, further comprising a nucleic acid.

**14.** A nucleic acid-introducing agent comprising the cationic lipid according to any one of claims 1 to 4.

**15.** The nucleic acid-introducing agent according to claim 14, further comprising a nucleic acid.

**16.** A pharmaceutical composition comprising the cationic lipid according to any one of claims 1 to 4.

**17.** The pharmaceutical composition according to claim 16, further comprising a nucleic acid.

**18.** A method for introducing a nucleic acid in a nucleic acid-introducing agent into a cell in vitro, comprising bringing the nucleic acid-introducing agent according to claim 15 into contact with the cell.

**19.** A method for introducing a nucleic acid in a nucleic acid-introducing agent into a target cell in a living organism, comprising administering the nucleic acid-introducing agent according to claim 15 to the living organism.

**20.** A method for producing a cellular medicine comprising a cell expressing a gene in a nucleic acid, comprising introducing the nucleic acid in a nucleic acid-introducing agent into a cell by bringing the nucleic acid-introducing agent according to claim 15 into contact with the cell.

[Fig. 1]

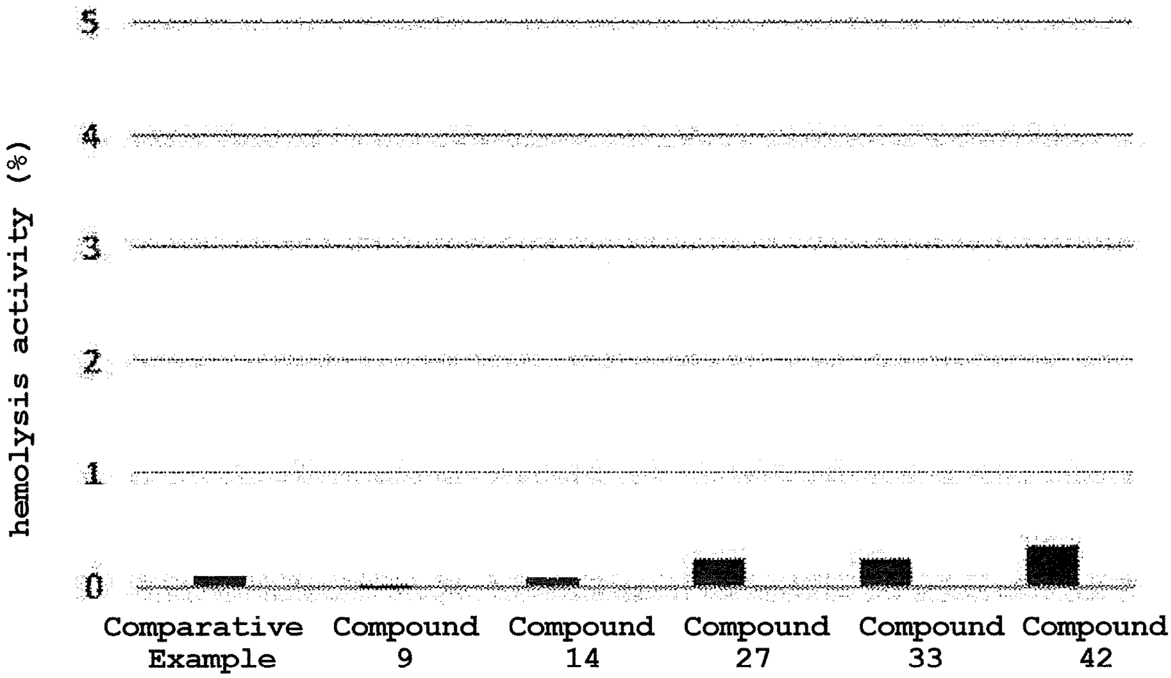
hemolysis activity (%)
5
4
3
2
1
0
Comparative Example
Compound 9
Compound 14
Compound 27
Compound 33
Compound 42

[Fig. 2]

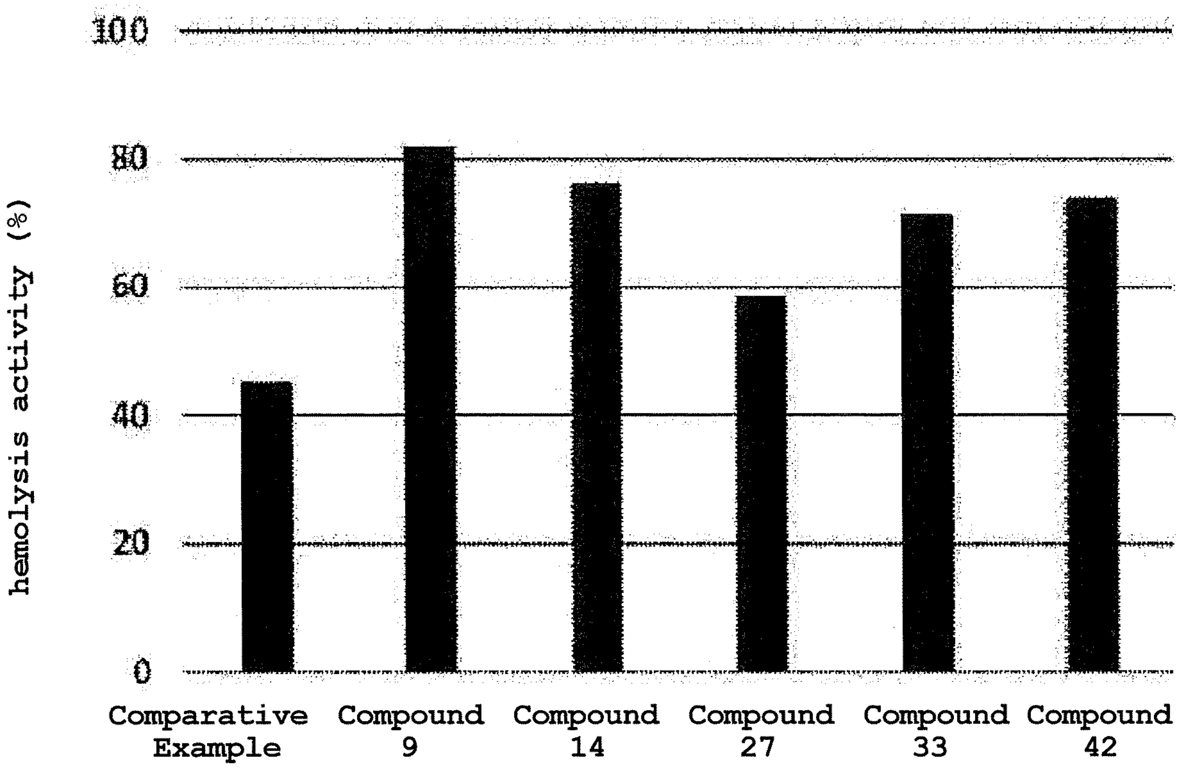
hemolysis activity (%)
100
80
60
40
20
0
Comparative Example
Compound 9
Compound 14
Compound 27
Compound 33
Compound 42

[Fig. 3]

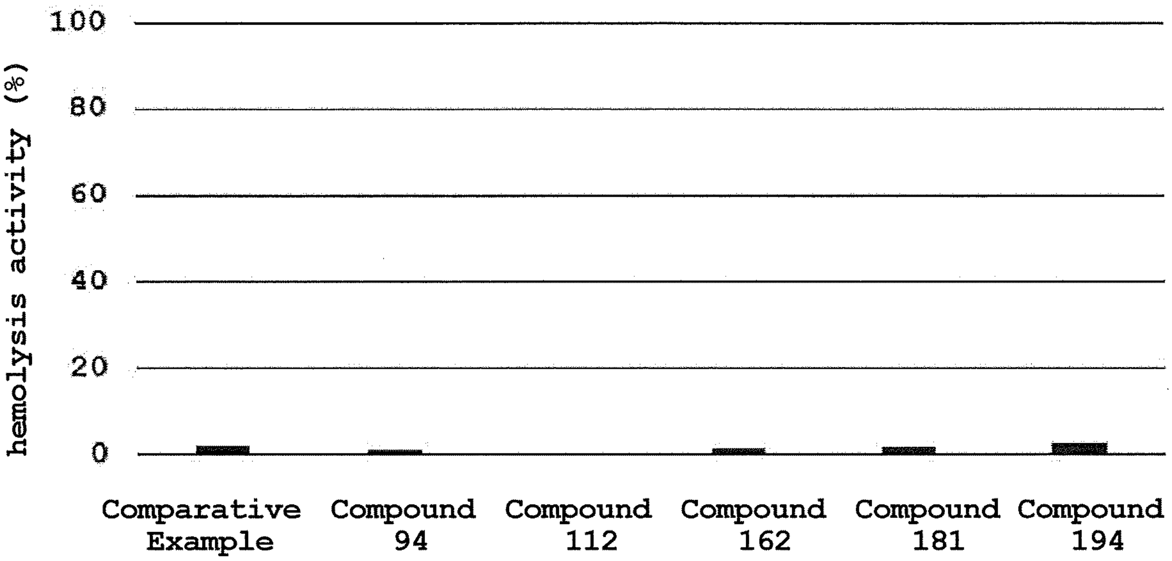
hemolysis activity (%)
100
80
60
40
20
0
Comparative Example
Compound 94
Compound 112
Compound 162
Compound 181
Compound 194

[Fig. 4]

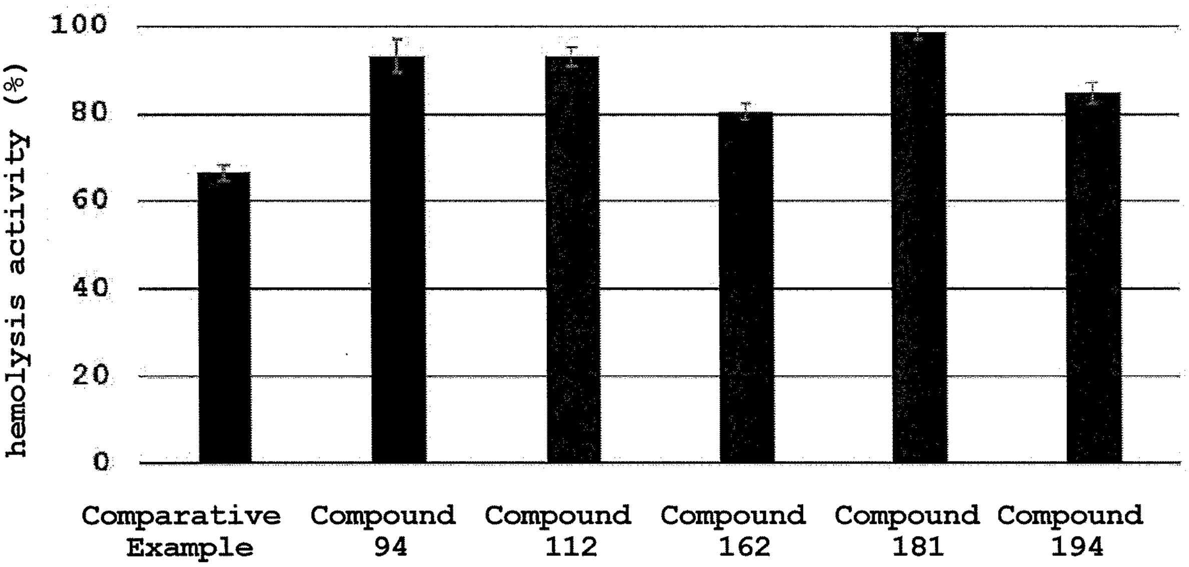
hemolysis activity (%)
100
80
60
40
20
0
Comparative Example
Compound 94
Compound 112
Compound 162
Compound 181
Compound 194

[Fig. 5]

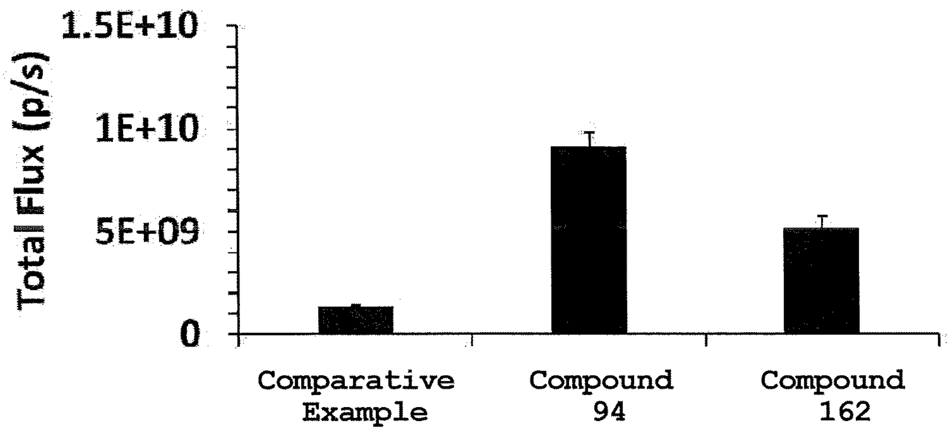


[Fig. 6]

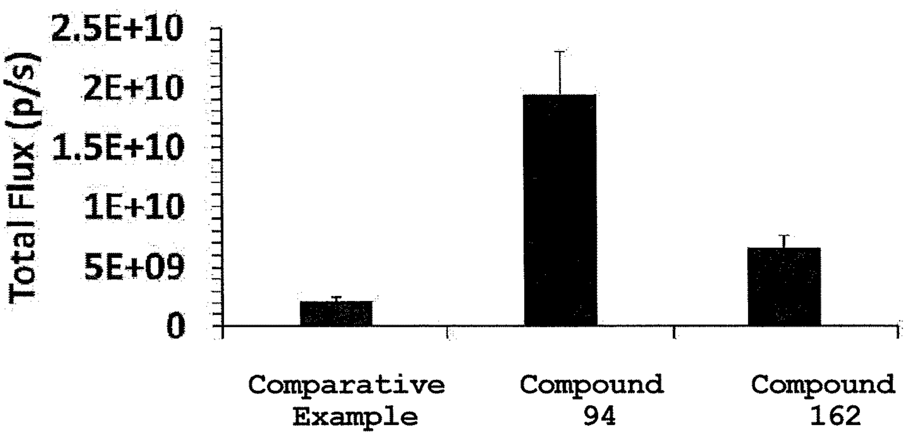


[Fig. 7]

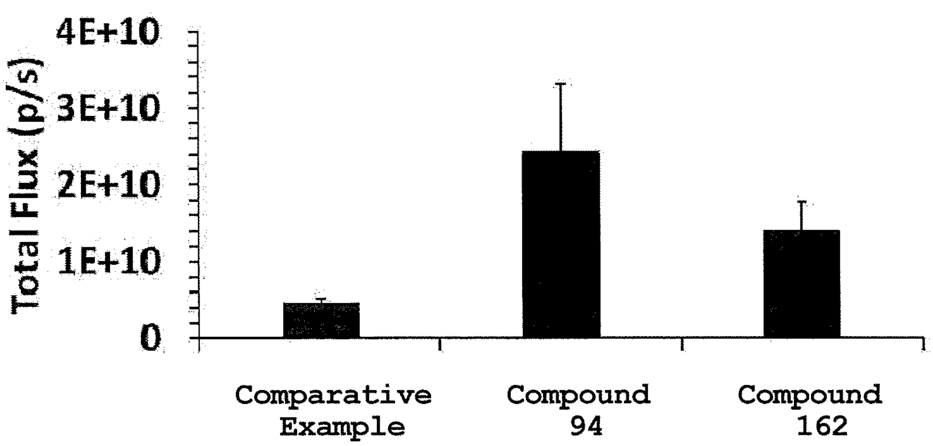

## INTERNATIONAL SEARCH REPORT

International application No.
**PCT/JP2024/010667**

**A. CLASSIFICATION OF SUBJECT MATTER**

***C07D 211/22***(2006.01)i; ***A61K 9/127***(2006.01)i; ***A61K 31/7088***(2006.01)i; ***A61K 47/22***(2006.01)i; ***A61K 48/00***(2006.01)i; ***A61P 43/00***(2006.01)i; ***C07F 7/10***(2006.01)i
FI: C07D211/22; A61K47/22; A61K9/127; A61K31/7088; A61K48/00; A61P43/00 105; C07F7/10 S CSP

According to International Patent Classification (IPC) or to both national classification and IPC

**B. FIELDS SEARCHED**

Minimum documentation searched (classification system followed by classification symbols)

C07D211/22; A61K9/127; A61K31/7088; A61K47/22; A61K48/00; A61P43/00; C07F7/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2019/188867 A1 (NOF CORPORATON) 03 October 2019 (2019-10-03) claims, table 1-1, examples, paragraphs [0048]-[0050] | 1-20 |
| Y | JP 2022-552774 A (INTEGRATED NANOTHERAPEUTICS INC.) 20 December 2022 (2022-12-20) claims, paragraphs [0006]-[0017], [0032]-[0042], table 1, examples | 1-3, 5-20 |
| Y | JP 2022-530043 A (INTELLIA THERAPEUTICS, INC.) 27 June 2022 (2022-06-27) claims, table 1, compounds 34, 52-61, examples, fig. 8A-8C | 1-3, 5-20 |
| Y | WO 2020/039631 A1 (KABUSHIKI KAISHA TOSHIBA) 27 February 2020 (2020-02-27) claims, paragraphs [0018]-[0030], examples | 1-20 |
| A | HASHIBA, K. et al. Branching Ionizable Lipids Can Enhance the Stability, Fusogenicity, and Functional Delivery of mRNA. Small Science. 2023, 3(1), p. 2200071, DOI: 10.1002/smsc.202200071 entire text | 1-20 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 May 2024** | **04 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No. **PCT/JP2024/010667**

**C. DOCUMENTS CONSIDERED TO BE RELEVANT**

| Category* | Citation of document, with indication, where appropriate, of the relevant passages | Relevant to claim No. |
|---|---|---|
| A | WO 2022/136641 A1 (ETHERNA IMMUNOTHERAPIES NV) 30 June 2022 (2022-06-30) entire text | 1-20 |
| P, X | WO 2024/014512 A1 (NOF CORPORATON) 18 January 2024 (2024-01-18) claims, ionic characteristics PiP9, PiP15, examples | 1-20 |
| P, X | WO 2023/121964 A1 (BEAM THERAPEUTICS INC.) 29 June 2023 (2023-06-29) claims, table 1, compounds 7-8 to 7-19, examples 7-3, 7-8, 7-9, 7-14 | 1, 4-7, 12-20 |

Form PCT/ISA/210 (second sheet) (July 2022)

**INTERNATIONAL SEARCH REPORT**
**Information on patent family members**

International application No.
**PCT/JP2024/010667**

| Patent document cited in search report | Publication date (day/month/year) | Patent family member(s) | Publication date (day/month/year) |
|---|---|---|---|
| WO 2019/188867 A1 | 03 October 2019 | US 2021/0023008 A1<br>claims, table 1-1, examples, paragraphs [0060], [0061]<br>US 2022/0192981 A1<br>EP 3778572 A1<br>CN 111902397 A<br>KR 10-2020-0136441 A<br>CA 3094057 A | |
| JP 2022-552774 A | 20 December 2022 | US 2022/0304929 A1<br>claims, paragraphs [0006]-[0017], [0062]-[0076], table 1, examples<br>WO 2021/026647 A1<br>EP 4013740 A1<br>KR 10-2022-0044816 A<br>AU 2020328474 A<br>CA 3150779 A<br>CN 114945555 A | |
| JP 2022-530043 A | 27 June 2022 | US 2022/0402862 A1<br>claims, table 1, compounds 34, 52-61, examples, fig. 8A-8C<br>WO 2020/219876 A1<br>EP 3959191 A1<br>TW 202106662 A<br>KR 10-2022-0005039 A<br>CN 114127044 A<br>AU 2020261419 A<br>CA 3137956 A<br>IL 287421 A<br>SG 11202111154W A<br>BR 112021021313 A<br>EA 202192918 A<br>MX 2021012934 A | |
| WO 2020/039631 A1 | 27 February 2020 | US 2020/0270217 A1<br>claims, paragraphs [0033]-[0058], examples<br>JP 2022-25076 A<br>JP 2023-16905 A<br>US 2023/0099139 A1<br>EP 3842412 A1<br>CN 111164068 A | |
| WO 2022/136641 A1 | 30 June 2022 | US 2024/0050574 A1<br>entire text<br>JP 2024-500918 A<br>EP 4267550 A1<br>CN 115362143 A<br>CA 3205455 A<br>AU 2021405781 A<br>IL 303659 A<br>KR 10-2023-0148325 A<br>TW 202233207 A | |
| WO 2024/014512 A1 | 18 January 2024 | (Family: none) | |

Form PCT/ISA/210 (patent family annex) (July 2022)

**INTERNATIONAL SEARCH REPORT**
**Information on patent family members**

International application No.
**PCT/JP2024/010667**

| Patent document cited in search report | Publication date (day/month/year) | Patent family member(s) | Publication date (day/month/year) |
|---|---|---|---|
| WO 2023/121964 A1 | 29 June 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 9708628 B
- WO 2016121942 A
- WO 2019188867 A
- WO 2021193397 A


### Non-patent literature cited in the description


- *Biomaterials*, 2008, vol. 29 (24-25), 3477-3496
- *Molecular Therapy*, 2016, vol. 24 (4), 788-795
- *Angewante Chemie International*, 2012, vol. 51, 8529-8533
- *Molecular Therapy*, 2006, vol. 13 (4), 786-794
- *Angewante Chemie International Edition*, 2012, vol. 51, 8529-8533
- *Molecular Therapy*, 2016, vol. 24 (4), 786-795
- *Proceedings of the National Academy of Science*, 2013, vol. 110 (32), 12881-12886